# FASCICULE DE BREVET EUROPEEN

(11) **EP 2 699 692 B1**
(45) Date de publication et mention de la délivrance du brevet: **14.09.2016**
(21) Numéro de dépôt: 12717661.8
(22) Date de dépôt: 20.04.2012
(51) Int. Cl.: C12Q 1/04, G01N 33/68

(54) **PROCEDE DE DETECTION D'AU MOINS UN MECANISME DE RESISTANCE AUX CEPHALOSPORINES PAR SPECTROMETRIE DE MASSE**
VERFAHREN ZUR ERFASSUNG VON MINDESTENS EINEM CEPHALOSPORIN-RESISTENZMECHANISMUS MITTELS MASSENSPEKTROMETRIE
METHOD FOR DETECTING AT LEAST ONE CEPHALOSPORIN RESISTANCE MECHANISM BY MEANS OF MASS SPECTROMETRY

(30) Priorité: 21.04.2011 US 201161477907 P
(43) Date de publication de la demande: 26.02.2014
(73) Titulaire: Biomerieux, Inc, Durham, NC 27712 (US)
(72) Inventeur: CHARRETIER, Yannick, F-69690 Courzieu (FR); CHARRIER, Jean-Philippe, F-69160 Tassin La Demi-lune (FR); FRANCESCHI, Christine, F-01800 Meximieux (FR); ZAMBARDI, Gilles, 38230 Tignieu Jameyzieu (FR); CECCHINI, Tiphaine, 69290 Saint-Genis les Ollières (FR); DEGOUT-CHARMETTE, Elodie, 01600 Toussieux (FR)
(74) Mandataire: Sprugnoli, Claude Louis Victor
(86) Numéro de dépôt international: PCT/EP2012/057322
(87) Numéro de publication internationale: WO 2012/143534

(56) Documents cités:
- N. A. LEDEBOER ET AL: "Molecular Detection of Resistance Determinants", JOURNAL OF CLINICAL MICROBIOLOGY, vol. 49, no. 9 Supplement, 1 septembre 2011 (2011-09-01), pages S20-S24, XP055173120, ISSN: 0095-1137, DOI: 10.1128/JCM.00771-11
- IKRYANNIKOVA L N ET AL: "A MALDI TOF MS-based minisequencing method for rapid detection of TEM-type extended-spectrum beta-lactamases in clinical strains of Enterobacteriaceae", JOURNAL OF MICROBIOLOGICAL METHODS, ELSEVIER, AMSTERDAM, NL, vol. 75, no. 3, 1 décembre 2008 (2008-12-01), pages 385-391, XP025631850, ISSN: 0167-7012, DOI: 10.1016/J.MIMET.2008.07.005 [extrait le 2008-07-16]
- I. WYBO ET AL: "Differentiation of cfiA-Negative and cfiA-Positive Bacteroides fragilis Isolates by Matrix-Assisted Laser Desorption Ionization-Time of Flight Mass Spectrometry", JOURNAL OF CLINICAL MICROBIOLOGY, vol. 49, no. 5, 23 février 2011 (2011-02-23), pages 1961-1964, XP055036513, ISSN: 0095-1137, DOI: 10.1128/JCM.02321-10
- J.-L. MAINARDI ET AL: "Resistance to cefotaxime and peptidoglycan composition in Enterococcus faecalis are influenced by exogenous sodium chloride", MICROBIOLOGY, vol. 144, no. 10, 1 octobre 1998 (1998-10-01), pages 2679-2685, XP055036332, ISSN: 1350-0872, DOI: 10.1099/00221287-144-10-2679
- K. BUSH ET AL: "Updated Functional Classification of -Lactamases", ANTIMICROBIAL AGENTS AND CHEMOTHERAPY, vol. 54, no. 3, 7 décembre 2009 (2009-12-07), pages 969-976, XP055036576, ISSN: 0066-4804, DOI: 10.1128/AAC.01009-09 cité dans la demande
- DATABASE MEDLINE [Online] US NATIONAL LIBRARY OF MEDICINE (NLM), BETHESDA, MD, US; 2010, SAVINOVA T A ET AL: "[A mass-spectrometric analysis of genetic markers of S. pneumoniae resistance to beta-lactam antibiotics].", XP002682431, Database accession no. NLM20882772
- N. A. LEDEBOER ET AL: "Molecular Detection of Resistance Determinants", JOURNAL OF CLINICAL MICROBIOLOGY, vol. 49, no. 9 Supplement, 1 September 2011 (2011-09-01), pages S20-S24, XP055173120, ISSN: 0095-1137, DOI: 10.1128/JCM.00771-11
- IKRYANNIKOVA L N ET AL: "A MALDI TOF MS-based minisequencing method for rapid detection of TEM-type extended-spectrum beta-lactamases in clinical strains of Enterobacteriaceae", JOURNAL OF MICROBIOLOGICAL METHODS, ELSEVIER, AMSTERDAM, NL, vol. 75, no. 3, 1 December 2008 (2008-12-01), pages 385-391, XP025631850, ISSN: 0167-7012, DOI: 10.1016/J.MIMET.2008.07.005 [retrieved on 2008-07-16]
- N. A. LEDEBOER ET AL: "Molecular Detection of Resistance Determinants", JOURNAL OF CLINICAL MICROBIOLOGY, vol. 49, no. 9 Supplement, 1 September 2011 (2011-09-01), pages S20-S24, XP055173120, ISSN: 0095-1137, DOI: 10.1128/JCM.00771-11
- IKRYANNIKOVA L N ET AL: "A MALDI TOF MS-based minisequencing method for rapid detection of TEM-type extended-spectrum beta-lactamases in clinical strains of Enterobacteriaceae", JOURNAL OF MICROBIOLOGICAL METHODS, ELSEVIER, AMSTERDAM, NL, vol. 75, no. 3, 1 December 2008 (2008-12-01), pages 385-391, XP025631850, ISSN: 0167-7012, DOI: 10.1016/J.MIMET.2008.07.005 [retrieved on 2008-07-16]

## Description

La présente invention concerne le domaine de la microbiologie. Plus précisément, l'invention concerne la détection d'au moins un mécanisme de résistance aux céphalosporines d'au moins un microorganisme issu d'un échantillon en utilisant la spectrométrie de masse.

Depuis la découverte des microbes par Pasteur, les microorganismes sont étudiés par microscopie et analyses biochimiques. Ces méthodes traditionnelles sont souvent longues et fastidieuses et des alternatives analytiques ont très tôt été recherchées. C'est ainsi que l'analyse de bactéries par spectrométrie de masse a été initiée dès 1975 par J. Anhalt et C. Fenselau [1].

Ces travaux préliminaires ont été suivis par l'étude en chromatographie en phase gazeuse couplée à la spectrométrie de masse (GC-MS) d'acides gras de la paroi des microorganismes [2]. Cette méthode a été popularisée sous l'appellation anglo-saxonne de FAME pour Fatty Acid Methyl Ester. Elle constitue actuellement une méthode de référence pour les études taxonomiques. Son utilisation reste cependant limitée à certains laboratoires spécialisés maîtrisant le traitement de l'échantillon par saponification, hydrolyse et dérivation.

En 1996, les travaux de M. Claydon et al [3] ainsi que de T. Krishnamurthy et P. Ross [4] ont montré la possibilité d'identifier différentes espèces bactériennes avec un spectromètre de masse de type MALDI-TOF (acronyme de l'anglais Matrix Assisted Laser Desorption lonization - Time Of Flight). L'analyse associe l'acquisition d'un spectre de masse et l'interprétation d'un logiciel expert. Elle est extrêmement simple et peut être effectuée en quelques minutes. Cependant elle ne se diffuse que depuis peu de temps dans les laboratoires d'analyses médicales [5]. Son utilisation clinique est actuellement limitée à l'identification d'espèces bactériennes et de levures. Elle n'est pas utilisée en routine pour l'identification des résistances aux antimicrobiens.

Or l'identification de résistances aux antimicrobiens tels que les antibiotiques est un élément essentiel pour assurer une prise en charge optimale des patients.

D'autres procédés de spectrométrie de masse, notamment en tandem, ont été proposés pour répondre à ces besoins. A titre d'exemple, il est possible de citer les travaux de C. Fenselau et al. pour l'identification de β-Lactamase avec un quadripôle-TOF (Q-TOF) [6].

Cependant ces résultats de recherche ne sont pas applicables à une utilisation clinique de routine. Ils ont été obtenus avec des instruments de recherche nécessitant un personnel hautement qualifié. Les temps d'analyse, souvent supérieurs à une heure par échantillon, sont incompatibles avec la charge de travail d'un laboratoire d'analyse microbiologique.

Plus récemment S. Hofstadler et al. [7] ont proposé un procédé associant une amplification du génome microbien par PCR à une détection des produits de PCR par électrospray-TOF (ESI-TOF). Ce procédé est maintenant totalement automatisé [8]. Toutefois, il nécessite une amplification par PCR avec les défauts inhérents à la biologie moléculaire, à savoir rendement d'extraction, coût des sondes, etc.

Dans ce contexte, l'objectif de la présente invention est de proposer un procédé de détection de mécanismes de résistance aux céphalosporines, qui permette de palier les inconvénients des procédés de l'art antérieur, à savoir fournir un procédé peu coûteux, sans réactifs spécifiques à chaque espèce, notamment par rapport aux procédés de biologie moléculaire, donnant un résultat en un temps court, inférieur à une heure, et utilisable en clinique de routine, sans nécessiter un personnel hautement qualifié.

A cette fin, l'invention propose un nouveau procédé de détection, par spectrométrie de masse, d'au moins un mécanisme de résistance à au moins un antimicrobien d'au moins un microorganisme issu d'un échantillon, caractérisé en ce que l'antimicrobien est une céphalosporine et en ce que des protéines et/ou des peptides sont détectés en tant que marqueurs dudit mécanisme de résistance à au moins un antibiotique de la classe des céphalosporines.

Avantageusement, des marqueurs de résistance à plusieurs antimicrobiens différents peuvent être détectés simultanément.

Comme indiqué dans la demande PCT/FR2010/052181, des marqueurs de typage et/ou de virulence desdits microorganismes peuvent être détectés, de la même façon, par spectrométrie de masse, préalablement ou simultanément à la détection des marqueurs de mécanisme de résistance.

les inconvénients des procédés de l'art antérieur, à savoir fournir un procédé peu coûteux, sans réactifs spécifiques à chaque espèce, notamment par rapport aux procédés de biologie moléculaire, donnant un résultat en un temps court, inférieur à une heure, et utilisable en clinique de routine, sans nécessiter un personnel hautement qualifié.

A cette fin, l'invention propose un nouveau procédé de détection, par spectrométrie de masse, d'au moins un mécanisme de résistance à au moins un antimicrobien d'au moins un microorganisme issu d'un échantillon, caractérisé en ce que l'antimicrobien est une céphalosporine et en ce que des protéines et/ou des peptides sont détectés en tant que marqueurs dudit mécanisme de résistance à au moins un antibiotique de la classe des céphalosporines, lesdites protéines et/ou lesdits peptides étant de type TEM.

Avantageusement, des marqueurs de résistance à plusieurs antimicrobiens différents peuvent être détectés simultanément.

Comme indiqué dans la demande PCT/FR2010/052181, des marqueurs de typage et/ou de virulence desdits microorganismes peuvent être détectés, de la même façon, par spectrométrie de masse, préalablement ou simultanément à la détection des marqueurs de mécanisme de résistance.

Par marqueurs de la résistance à au moins un antimicrobien de la classe des céphalosporines, on entend des molécules d'origine protéique qui sont caractéristiques desdites propriétés.

Les céphalosporines sont des antibiotiques appartenant à la famille des béta-lactamines. Elles sont habituellement classées en plusieurs sous-classes:
- les céphalosporines de première génération, tels que cefazoline, cephalotine, cefaclor, cephalexine, qui sont dégradées par les beta-lactamases des groupes 1 et 2b, 2br, 2be, 2ber
- les céphalosporines de seconde génération, tels que cefamandole, cefpodoxime, cefuroxime, qui sont dégradées par les béta-lactamases des groupes 1, 2be, 2ber
- les céphalosporines de troisième génération, tels que cefotaxime, ceftazidime, ceftriaxone, cefixime, qui sont dégradées par les béta-lactamases des groupes 1, 2be, 2ber bien qu'étant plus stables que les céphalosporines de première et seconde génération
- les céphalosporines de quatrième génération, tels que cefepime et cefpirome, qui sont dégradées par les béta-lactamases des groupes 2be et 2 ber
- les céphalosporines avec activité anti-MRSA tels que ceftaroline et ceftobiprole, qui sont dégradées par les béta-lactamases des groupes 2be et 2 ber
- les céphamycines, tels que cefoxitine, cefotetan, cefmetazole, qui sont dégradées par les béta-lactamases des groupes 1

Par détermination de la résistance à au moins un antimicrobien, on entend la détermination de la susceptibilité d'un microorganisme à être détruit par un antimicrobien. Les protéines impliquées dans les mécanismes de résistance vont différer selon la famille et l'espèce.

La nomenclature des béta-lactamases, enzymes bactériennes de résistance aux béta-lactamines, n'est pas standardisée. Elles sont soit classées en quatre classes moléculaires (A à D) sur la base de leur structure primaire, soit en groupes fonctionnels sur la base des substrats ciblés et de leur résistance aux inhibiteurs (pour un revue, voir [9] Bush and Jacoby, Antimicrobial Agents and Chemotherapy, 2010; 54 (3): 969-976). Pour la classification moléculaire, les techniques de séquençage ont permis une classification plus précise : par exemple, 183 variants de la protéine TEM ont été décrits (notés TEM-i, i étant compris entre 1 et 183). Pour la classification fonctionnelle, Bush et Jacoby *(supra)* ont proposé de nouveaux sous-groupes fonctionnels :
- les enzymes du groupe 1 sont des céphalosporinases appartenant à la classe moléculaire C. ACC, ACT, MIR, MOX, DHA, CMY et FOX sont des enzymes, portées par des plasmides, appartenant à ce sous-groupe.
- les enzymes du groupe 2 appartiennent aux classes moléculaires A et D. Ce groupe est lui-même subdivisé en sous-groupes : 2a, 2b, 2be, 2br, 2ber, 2c, 2ce, 2d, 2de, 2df, 2f, etc. CTX-M (2be), SHV (2b, 2be ou 2br), PER (2be), VEB (2be) et TEM (2b, 2be, 2br ou 2ber) sont des enzymes appartenant à ce groupe.
   ∘ Le sous groupe 2a correspond à des béta-lactamases hydrolysant la benzylpénicilline et les dérivées de la pénicilline, mais n'hydrolysant pas les céphalosporines, les carbapénèmes ou les monobactames.
   ∘ Le sous-groupe 2b correspond à des béta-lactamases à large spectre, hydrolysant les pénicillines et les céphalosporines de première génération telles que la céphaloridine et la céphalothine, et qui sont inhibées par l'acide clavulanique, le sulfobactame ou le tazobactame. Les variants TEM-1, TEM-2 et SHV-1 appartiennent à ce sous-groupe.
      ▪ Le sous-groupe 2be correspond à des beta-lactamases à spectre étendu (BLSE), également inhibées par l'acide clavulanique, le sulfobactame ou le tazobactame. Ces enzymes, en plus des propriétés du sous-groupe 2b, hydrolysent au moins un oxyimino-béta-lactame tel que le cefotaxime ou le ceftazidime, et les monobactames comme l'aztreonam. Ce sous-groupe contient de nombreux variants de TEM et SHV, des variants de PER et VEB, ainsi que CTX-M, BEL-1, BES-1, SFO-1, TAL-1 et TAL-2.
      ▪ Le sous-groupe 2br correspond aux béta-lactamases du sous-groupe 2b qui sont insensibles à l'inhibition par l'acide clavulanique, le sulfobactame ou le tazobactame. Ce sous-groupe contient des variants des enzymes TEM et SHV.
      ▪ Le sous groupe 2ber correspond aux béta-lactamases du sous-groupe 2be qui sont insensibles à l'inhibition par l'acide travaux de C. Fenselau et al. pour l'identification de β-Lactamase avec un quadripôle-TOF (Q-TOF) [6].

Cependant ces résultats de recherche ne sont pas applicables à une utilisation clinique de routine. Ils ont été obtenus avec des instruments de recherche nécessitant un personnel hautement qualifié. Les temps d'analyse, souvent supérieurs à une heure par échantillon, sont incompatibles avec la charge de travail d'un laboratoire d'analyse microbiologique.
I. Wybo et al. [34] ont décrit une analyse de différenciation entre des isolats cfiA-positifs et cfiA-négatifs de Bacteroides fragilis. Cette analyse est réalisée en spectrométrie de masse du type MALDI-TOF.

J.-L. Mainardi et al. [35] ont présenté l'étude de l'influence du NaCI sur la sensibilité au céfotaxime d'Enterococcus faecalis. A cette fin, une analyse du peptidoglycane JH2-2 est réalisée par HPLC, couplé à une analyse en spectrométrie de masse. Plus particulièrement, c'est la composition en muropeptides du peptidoglycane JH2-2 qui a été analysée en HPLC inverse et en MS. Les structures de muropeptides ont été analysées en spectrométrie de masse simple et confirmées en utilisant une analyse en MS/MS.

Plus récemment S. Hofstadler et al. [7] ont proposé un procédé associant une amplification du génome microbien par PCR à une détection des produits de PCR par électrospray-TOF (ESI-TOF). Ce procédé est maintenant totalement automatisé [8]. Toutefois, il nécessite une amplification par PCR avec les défauts inhérents à la biologie moléculaire, à savoir rendement d'extraction, coût des sondes, etc.

Enfin, N.A. Ledeboer et al. [36] ont décrit dans un rapport de 2011 les différentes alternatives en termes de tests de sensibilité aux antibiotiques de routine et notamment l'utilisation de la spectrométrie de masse de type MALDI-TOF pour détecter les métabolites des béta-lactames hydrolysés par réaction avec les béta-lactamases bactériennes.

Dans ce contexte, l'objectif de la présente invention est de proposer un procédé de détection de mécanismes de résistance aux céphalosporines, qui permette de palier les inconvénients des procédés de l'art antérieur, à savoir fournir un procédé peu Le procédé de l'invention peut être mis en oeuvre pour détecter des mécanismes de résistance aux céphalosporines chez des bactéries. Ainsi, par exemple, à titre de bactéries chez lesquelles il est possible de rechercher un mécanisme de résistance aux céphalosporines selon le procédé de l'invention, on peut citer, de façon non exhaustive :
- les Enterobacteriaceae, en utilisant des protéines et peptides du groupe 1 et du groupe 2 comme marqueur de résistance ;
- les bactéries non fermentantes *(Pseudomonas aeruginosa, Acinetobacter baumannii*)
- etc.

Il est à noter par ailleurs que les souches connues comme résistantes aux carbapénèmes sont également résistantes aux céphalosporines et aux pénicillines Ainsi, un procédé de détection d'un mécanisme de résistance aux carbapénèmes permet également de détecter un mécanisme de résistance aux céphalosporines et aux pénicillines.

L'échantillon sur lequel le procédé de l'invention peut être mis en oeuvre est tout échantillon susceptible de contenir un microorganisme cible. L'échantillon peut être d'origine biologique, soit animale, végétale ou humaine. Il peut alors correspondre à un prélèvement de fluide biologique (sang total, sérum, plasma, urine, liquide céphalo-rachidien, sécrétion organique, par exemple), un prélèvement tissulaire ou des cellules isolées. Ce prélèvement peut être utilisé tel quel dans la mesure où les marqueurs des mécanismes de résistance des bactéries aux béta-lactamines sont disponibles dans l'échantillon testé, ou bien il peut subir préalablement à l'analyse, une préparation de type enrichissement, extraction, concentration, purification, culture, selon des méthodes connues de l'homme du métier.

L'échantillon peut être d'origine industrielle, soit, selon une liste non exhaustive un prélèvement d'air, un prélèvement d'eau, un prélèvement effectué sur une surface, une pièce ou un produit manufacturé, un produit d'origine alimentaire. Parmi les échantillons d'origine alimentaire, on peut citer de façon non exhaustive un échantillon de produit lacté (yaourts, fromages), de viande, de poisson, d'oeuf, de fruit, de légume, d'eau, de boisson (lait, jus de fruits, soda, etc). Ces échantillons d'origine alimentaire peuvent aussi provenir de sauces ou de plats élaborés. Un échantillon alimentaire peut enfin être issu d'une alimentation destinée aux animaux, telle que notamment des farines animales.

En amont de la détection par spectrométrie de masse, l'échantillon à analyser est préférentiellement traité au préalable pour générer des peptides à partir de l'ensemble des protéines présentes dans l'échantillon pour fragmenter ces protéines en peptides, par exemple par digestion avec une enzyme protéolytique (protéase), ou par action d'un réactif chimique. En effet, le clivage des protéines peut être fait par un traitement physico-chimique, par un traitement biologique ou par une combinaison des deux traitements. Parmi les traitements utilisables, on peut citer le traitement par des radicaux hydroxyle, notamment avec de l'H₂O₂. Le traitement par les radicaux hydroxyle provoque une coupure des liaisons peptidiques qui se fait de manière aléatoire sur n'importe quelle liaison peptidique de la protéine. La concentration en radicaux hydroxyle conditionne le nombre de clivages opérés et donc la longueur des fragments peptidiques obtenus. D'autres traitements chimiques peuvent également être utilisés comme, par exemple, le traitement au bromure de cyanogène (CNBr) qui scinde spécifiquement les liaisons peptidiques au niveau du groupe carboxylique des résidus méthionyle. Il est également possible de réaliser un clivage acide partiel au niveau des résidus aspartyle par chauffage à 1000°C d'une solution de protéines dans de l'acide trifluoroacétique.

Le traitement des protéines par digestion enzymatique est néanmoins préféré par rapport au traitement physico-chimique car il préserve davantage la structure des protéines, et est plus facile à contrôler. Par « digestion enzymatique », on entend l'action simple ou combinée d'une ou de plusieurs enzymes dans des conditions de réaction appropriées. Les enzymes effectuant la protéolyse, appelées protéases, coupent les protéines à des endroits spécifiques. Chaque protéase reconnaît généralement une séquence d'acides aminés au sein desquels elle effectue toujours la même coupure. Certaines protéases reconnaissent un seul acide aminé ou une séquence de deux acides aminés entre lesquels elles opèrent un clivage, d'autres protéases ne reconnaissent que des séquences plus longues. Ces protéases peuvent être des endoprotéases ou des exoprotéases. Parmi les protéases connues on peut citer, comme décrit dans WO2005/098071 :
- les enzymes spécifiques comme la trypsine qui scinde la liaison peptidique au niveau du groupe carboxylique des résidus Arg et Lys, l'endolysine qui clive la liaison peptidique du groupe -CO des lysines, la chymotrypsine qui hydrolyse la liaison peptidique au niveau du groupe carboxylique des résidus aromatiques (Phe, Tyr et Trp), la pepsine qui coupe au niveau du groupe NH₂ des résidus aromatiques (Phe, Tyr et Trp), la protéase V8 de la souche V8 de *Staphylococcus aureus* qui clive la liaison peptidique au niveau du groupe carboxylique du résidu Glu ;
- les enzymes non-spécifiques comme la thermolysine provenant de la bactérie *Bacillus thermoproteolyticus* qui hydrolyse la liaison peptidique du groupe NH₂ des acides aminés hydrophobes (Xaa-Leu, Xaa-Ile, Xaa-Phe), la subtilisine et la pronase qui sont des protéases bactériennes qui hydrolysent pratiquement toutes les liaisons et peuvent transformer les protéines en oligopeptides dans des conditions de réaction contrôlées (concentration en enzyme et durée de réaction).

Plusieurs protéases peuvent être utilisées de façon simultanée, si leurs modes d'action sont compatibles, ou elles peuvent être utilisées de façon successive. Dans le cadre de l'invention, la digestion de l'échantillon est, de préférence, réalisée par action d'une enzyme protéase, par exemple la trypsine.

La génération de peptides à l'aide d'un réactif chimique ou d'une protéase, peut être obtenu par simple réaction en solution. Elle peut également être mise en oeuvre avec un four à micro-ondes [10], ou sous pression [11], ou bien encore avec un dispositif à ultrasons [12]. Dans ces trois derniers cas, le protocole sera beaucoup plus rapide.

Parmi les peptides ainsi obtenus, les peptides spécifiques de la protéine, sont nommés peptides protéotypiques. Ce sont eux qui seront dosés par spectrométrie de masse.

Selon l'invention, les marqueurs des mécanismes de résistance des bactéries aux céphalosporines sont des protéines de la bactérie chez laquelle les mécanismes de résistance aux céphalosporines sont à rechercher. En particulier, lesdites protéines sont digérées en peptides, de préférence par une enzyme, de préférence encore par la trypsine.

De même, l'échantillon contenant des marqueurs protéiques de caractérisation des mécanismes de résistance des bactéries aux céphalosporines peut également être préalablement traité à des fins de purification. Ce traitement préalable de purification peut être mis en oeuvre avant ou après l'étape de génération de peptides tels que décrits précédemment.

Le traitement préalable de purification d'échantillon est largement connu de l'homme du métier et pourra notamment mettre en oeuvre des techniques de centrifugation, de filtration, d'électrophorèse ou de chromatographie. Ces techniques séparatives peuvent être utilisées seules ou combinées entre elles pour obtenir une séparation multidimensionnelle. Par exemple, une chromatographie multidimensionnelle peut être utilisée en associant une séparation par chromatographie d'échange d'ions à une chromatographie en phase inverse, comme décrit par T. Fortin et al. [13], ou H. Keshishian et al. [14]. Dans ces publications, le milieu chromatographique peut être en colonne ou en cartouche (extraction en phase solide).

La fraction électrophorétique ou chromatographique (ou le temps de rétention en chromatographie mono ou multidimensionnelle) des peptides protéotypiques est caractéristique de chaque peptide et la mise en oeuvre de ces techniques permet donc de sélectionner le ou les peptides protéotypiques à doser. Un tel fractionnement des peptides générés permet d'accroître la spécificité du dosage ultérieur par spectrométrie de masse.

Une alternative aux techniques d'électrophorèse ou de chromatographie, pour le fractionnement des peptides, consiste à purifier spécifiquement les N-glycopeptides ([15] et demande de brevet WO 2008/066629). Néanmoins, une telle purification ne permet que la quantification des peptides ayant subi une modification post-traductionnelle de type N-glycosylation. Or toutes les protéines ne sont pas glycosylées, ce qui limite donc son utilisation.

La spectrométrie de masse à mettre en oeuvre dans le procédé de l'invention est largement connue de l'homme du métier comme un outil puissant pour l'analyse et la détection de différents types de molécules. De façon générale, tout type de molécule pouvant être ionisée peut être détecté en fonction de sa masse moléculaire à l'aide d'un spectromètre de masse. Selon la nature de la molécule à détecter, d'origine protéique ou métabolique, certaines technologies de spectrométrie de masse peuvent être plus adaptées. Néanmoins, quelque soit la méthode de spectrométrie de masse utilisée pour la détection, cette dernière comprend une étape d'ionisation de la molécule cible en ions dits moléculaires, dans le cas présent une étape d'ionisation des marqueurs de caractérisation, et une étape de séparation des ions moléculaires obtenus en fonction de leur masse.

Tous les spectromètres de masse comportent donc :
- une source d'ionisation destinée à ioniser les marqueurs présents dans l'échantillon à analyser, c'est-à-dire à conférer une charge positive ou négative à ces marqueurs;
- un analyseur de masse destiné à séparer les marqueurs ionisés, ou ions moléculaires, en fonction de leur ratio masse sur charge (m /z) ;
- un détecteur destiné à mesurer le signal produit soit directement par les ions moléculaires, soit par des ions produits à partir des ions moléculaires, comme détaillés ci-après.

L'étape d'ionisation nécessaire pour la mise en oeuvre d'une spectrométrie de masse peut être mise en oeuvre par tout procédé connu de l'homme du métier. La source d'ionisation permet d'amener les molécules à doser sous un état gazeux et ionisé. Une source d'ionisation peut être utilisée soit en mode positif pour étudier les ions positifs, soit en mode négatif pour étudier les ions négatifs. Plusieurs types de sources existent et seront utilisés en fonction du résultat recherché et des molécules analysées. On peut citer, notamment :
- l'ionisation électronique (EI), l'ionisation chimique (CI) et la désorption-ionisation chimique (DCI)
- le bombardement par atomes rapides (FAB), atomes métastables (MAB) ou ions (SIMS, LSIMS)
- le couplage plasma inductif (ICP)
- l'ionisation chimique à pression atmosphérique (APCI) et la photoionisation à pression atmosphérique (APPI)
- l'électronébulisation ou électrospray (ESI)
- la désorption-ionisation laser assistée par matrice (MALDI), activée par une surface (SELDI) ou sur silicium (DIOS)
- l'ionisation-désorption par interaction avec espèces métastables (DART) Notamment, l'ionisation peut être mise en oeuvre comme suit : l'échantillon contenant les molécules cibles est introduit dans une source d'ionisation, où les molécules sont ionisées à l'état gazeux et ainsi transformées en ions moléculaires qui correspondent aux molécules initiales. Une source d'ionisation de type électrospray (ESI pour ElectroSpray Ionisation) permet d'ioniser une molécule tout en la faisant passer d'un état liquide à un état gazeux. Les ions moléculaires obtenus correspondent alors aux molécules présentes à l'état liquide, avec en mode positif un, deux, voire trois protons supplémentaires ou plus et sont donc porteurs de une, deux, voire trois charges ou plus. Par exemple, lorsque la molécule cible est une protéine, une ionisation des peptides protéotypiques obtenus après fractionnement de la protéine cible, grâce à une source de type électrospray fonctionnant en mode positif, conduit à des ions polypeptidiques à l'état gazeux, avec un, deux, voire trois protons supplémentaires ou plus et qui sont donc porteurs de une, deux, voire trois charges ou plus, et permet un passage d'un état liquide à un état gazeux [16]. Ce type de source est particulièrement bien adapté, lorsque les molécules cibles ou peptides protéotypiques obtenus sont préalablement séparées par chromatographie liquide en phase inverse. Néanmoins, le rendement d'ionisation des molécules présentes dans l'échantillon peut varier en fonction de la concentration et de la nature des différentes espèces en présence. Ce phénomène se traduit par un effet matrice bien connu de l'homme de l'art.

Une source d'ionisation MALDI permettra d'ioniser des molécules, à partir d'un échantillon à l'état solide.

L'analyseur de masse dans lequel est mis en oeuvre l'étape de séparation des marqueurs ionisés en fonction de leur rapport masse/charge (m/z) est tout analyseur de masse connu de l'homme du métier. On peut citer les analyseurs basse résolution, du type quadripôle ou quadrupôle (Q), piège à ions 3D (IT) ou linéaire (LIT), également appelés trappe ionique, et les analyseurs haute résolution, permettant de mesurer la masse exacte des analytes et qui utilisent notamment le secteur magnétique couplé à un secteur électrique, le temps de vol (TOF), la résonance cyclotronique ionique à transformé de fourier (FT-ICR), l'orbitrappe.

La séparation des ions moléculaires en fonction de leur ratio m/z peut être mise en oeuvre une seule fois (spectrométrie de masse simple ou MS), ou bien plusieurs séparations MS successives peuvent être menées. Lorsque deux séparations MS successives sont réalisées, l'analyse est appelée MS/MS ou MS². Lorsque trois séparations MS successives sont réalisées, l'analyse est appelée MS/MS/MS ou MS³ et plus généralement, lorsque n séparations MS successives sont réalisées, l'analyse est appelée MSⁿ.

Parmi les techniques mettant en oeuvre plusieurs séparations successives, les modes SRM (Selected Reaction Monitoring) en cas de détection ou dosage d'une seule molécule cible, ou bien MRM (Multiple Reaction Monitoring) en cas de détection ou dosage de plusieurs molécules cibles, sont des utilisations particulières de séparation MS². De même le mode MRM³ est une utilisation particulière de séparation en MS/MS/MS. On parle alors de spectrométrie de masse ciblée.

Dans le cas d'une détection en mode MS simple, c'est le rapport masse/charge des ions moléculaires obtenus qui est corrélé à la molécule cible à détecter.

Dans le cas d'une détection en mode MS/MS, essentiellement deux étapes sont ajoutées, par rapport à un dosage MS qui sont :
- une fragmentation des ions moléculaires, alors appelés ions précurseurs, pour donner des ions dit ions fragments de 1^{ère} génération, et
- une séparation des ions dit ions fragments de 1^{ère} génération en fonction de leur masse (m/z)₂, le rapport (m/z)₁ correspondant au rapport (m/z) des ions précurseurs.

C'est alors le rapport masse/charge des ions fragments de 1^{ère} génération ainsi obtenus qui est corrélé à la molécule cible à détecter. Par ion fragment de première génération, on entend un ion issu de l'ion précurseur, suite à une étape de fragmentation et dont le rapport masse sur charge m/z est différent de l'ion précurseur.

Les couples (m/z)₁ et (m/z)₂ sont baptisés transitions et sont représentatifs des ions caractéristiques à détecter.

Le choix des ions caractéristiques qui sont détectés pour être corrélés à la molécule cible est effectué par l'homme du métier selon les méthodes standards. Leur sélection conduira avantageusement aux dosages les plus sensibles, les plus spécifiques et les plus robustes possibles, en termes de reproductibilité et fiabilité.

Dans les méthodes développées pour la sélection de peptides protéotypiques (m/z)₁, et de fragment de première génération (m/z)₂, le choix est essentiellement basé sur l'intensité de la réponse. Pour plus de détails, on pourra se référer à V. Fusaro et al. [17]. Des logiciels commerciaux, tels que les logiciels MIDAS et MRM Pilote d'Applied Biosytems ou encore MRMaid [18] pourront être utilisés par l'homme de l'art pour lui permettre de prédire tous les couples de transitions possibles. Il pourra également être fait appel à une base de données nommée PeptideAtlas, construite par F. Desiere et al. [19] pour compiler l'ensemble des transitions MRM de peptides décrites par la communauté scientifique. Cette base PeptideAtlas est disponible en accès libre sur internet. Pour des molécules non protéiques, il est également possible d'utiliser des bases de données, telles que par exemple celle accessible au travers du logiciel Cliquid de la société Applied Biosytems (Etats Unis d'Amérique).

Une approche alternative pour sélectionner les peptides protéotypiques, (m/z)₁ et (m/z)₂, consiste à utiliser les spectres de fragmentation MS/MS obtenus à l'occasion d'autres travaux. Ces travaux peuvent être, par exemple, les phases de découverte et d'identification des biomarqueurs par analyse protéomique. Cette approche a été proposée par Thermo Scientific lors de réunions utilisateurs [18]. Elle permet de générer une liste de transitions candidates à partir des peptides identifiés expérimentalement par le logiciel SIEVE (Thermo Scientific). Certains critères ont été détaillés par J. Mead et al. [18] pour le choix des ions (m/z)₁ et (m/z)₂ et sont détaillés ci-après :
- les peptides avec des sites de clivage interne, c'est-à-dire avec de la Lysine ou de l'Arginine interne, doivent être évités, sauf si la Lysine ou l'Arginine est suivie par de la Proline,
- les peptides avec de l'Asparagine ou de la Glutamine doivent être évités car ils peuvent se désaminer,
- les peptides avec de la Glutamine ou de l'Acide Glutamique en N-terminal doivent être évités car ils peuvent se cycliser spontanément,
- les peptides avec de la Méthionine doivent être évités car ils peuvent être oxydés,
- les peptides avec de la Cystéine doivent être évités car ils peuvent être modifiés de façon non reproductible lors d'une éventuelle étape de dénaturation, réduction et blocage des fonctions thiols,
- les peptides avec de la Proline peuvent être considérés comme favorables parce qu'ils produisent généralement des fragments intenses en MS/MS avec un seul pic très majoritaire. Cependant, un seul fragment très majoritaire ne permet pas de valider l'identité de la transition dans un mélange complexe. En effet, seule la présence simultanée de plusieurs fragments caractéristiques permet de vérifier que l'ion précurseur recherché est bien détecté,
- les peptides ayant une Proline adjacente au C-terminal (position n-1) ou en seconde position par rapport au C-terminal (position n-2) sont à éviter car, dans ce cas, la taille du peptide fragment de première génération est généralement considérée comme trop petite pour être suffisamment spécifique,
- la sélection de fragments ayant une masse supérieure au précurseur est à privilégier pour favoriser la spécificité. Pour cela, il faut sélectionner un ion précurseur dichargé et sélectionner l'ion fragment de première génération le plus intense ayant une masse supérieure au précurseur, c'est à dire un ion fragment de première génération monochargé.

La fragmentation des ions précurseurs sélectionnés est mise en oeuvre dans une cellule de fragmentation telle que les modèles de type triple quadripôle [20], ou de type trappe ionique [21], ou encore de type temps de vol (TOF) [22], lesquels permettent également la séparation des ions. La ou les fragmentations seront classiquement réalisées par collision avec un gaz inerte tel que l'argon ou l'azote, au sein d'un champ électrique, par photo-excitation ou photodissociation à l'aide d'une source lumineuse intense, collision avec des électrons ou espèces radicalaires, par application d'une différence de potentiel, par exemple dans un tube de temps de vol, ou par tout autre mode d'activation. Les caractéristiques du champ électrique conditionnent l'intensité et la nature de la fragmentation. Ainsi, le champ électrique appliqué en présence d'un gaz inerte, par exemple dans un quadripôle, conditionne l'énergie de collision apportée aux ions. Cette énergie de collision sera optimisée, par l'homme du métier, pour accroître la sensibilité de la transition à doser. A titre d'exemple, il est possible de faire varier l'énergie de collision entre 5 et 180 e⁻V en q2 dans un spectromètre de masse AB SCIEX QTRAP® 5500 de la société Applied Biosystems (Foster City, Etats Unis d'Amérique). De même, la durée de l'étape de collision et l'énergie d'excitation au sein, par exemple, d'une trappe ionique seront optimisées, par l'homme du métier, pour conduire au dosage le plus sensible. A titre d'exemple, il est possible de faire varier cette durée, baptisée temps d'excitation, entre 0,010 et 50 ms et l'énergie d'excitation entre 0 et 1 (unité arbitraire) en Q3 dans un spectromètre de masse AB SCIEX QTRAP® 5500 de la société Applied Biosystems.

Enfin, la détection des ions caractéristiques sélectionnés se fait de façon classique, notamment grâce à un détecteur et à un système de traitement. Le détecteur collecte les ions et produit un signal électrique dont l'intensité dépend de la quantité d'ions collectée. Le signal obtenu est ensuite amplifié pour qu'il puisse être traité informatiquement. Un ensemble informatique de traitement des données permet de transformer les informations reçues par le détecteur en spectre de masse.

Le principe du mode SRM, ou encore du mode MRM, est de sélectionner spécifiquement un ion précurseur, de le fragmenter, puis de sélectionner spécifiquement l'un de ses ions fragments. Pour de telles applications, des dispositifs du type triple quadripôle ou des hybrides triple quadripôle à trappe ionique sont généralement utilisés.

Dans le cas d'un dispositif triple quadripôle (Q1q2Q3) utilisé en mode MS², en vue du dosage ou de la détection d'une protéine cible, le premier quadripôle (Q1) permet de filtrer les ions moléculaires, correspondant aux peptides protéotypiques caractéristiques de la protéine à doser et obtenus lors d'une étape antérieure de digestion, en fonction de leur ratio masse sur charge (m/z). Seuls les peptides ayant le ratio masse/charge du peptide protéotypique recherché, ratio appelé (m/z)₁, sont transmis dans le deuxième quadripôle (q2) et jouent le rôle d'ions précurseurs pour la fragmentation ultérieure. L'analyseur q2 permet de fragmenter les peptides de ratio masse/charge (m/z)₁ en ions fragments de première génération. La fragmentation est généralement obtenue par collision des peptides précurseurs avec un gaz inerte, comme de l'azote ou de l'argon dans q2. Les ions fragments de première génération sont transmis dans un troisième quadripôle (Q3) qui filtre les ions fragments de première génération en fonction d'un ratio masse sur charge spécifique, ratio appelé (m/z)₂. Seuls les ions fragments de première génération ayant le ratio masse/charge d'un fragment caractéristique du peptide protéotypique recherché (m/z)₂ sont transmis dans le détecteur pour être détectés, voire quantifiés. Ce mode de fonctionnement présente une double sélectivité, en relation avec la sélection de l'ion précurseur d'une part et de la sélection de l'ion fragment de première génération d'autre part. La spectrométrie de masse en mode SRM ou MRM est donc avantageuse pour la quantification

La spectrométrie de masse mise en oeuvre dans le procédé de l'invention est une spectrométrie de masse en tandem (MS², MS³, MS⁴ ou MS⁵). Ainsi, plusieurs analyseurs de masse peuvent être couplés entre eux. Par exemple, un premier analyseur sépare les ions, une cellule de collision permet de fragmenter les ions, et un second analyseur sépare les ions fragments. Certains analyseurs, comme les pièges à ions ou le FT-ICR, constituent plusieurs analyseurs en un et permettent de fragmenter les ions et d'analyser les fragments directement.

Le procédé de l'invention comprend une ou plusieurs des caractéristiques suivantes :
- la spectrométrie de masse, mise en oeuvre pour les propriétés de résistance potentielle à au moins un antimicrobien, est une spectrométrie de type MS/MS, ce qui a pour avantage de générer un fragment spécifique de la molécule à détecter ou à quantifier, et ainsi d'apporter une grande spécificité à la méthode de dosage ;
- la spectrométrie MS/MS est de la MRM, ce qui a pour avantage d'utiliser un temps de cycle d'analyse dans le spectromètre de masse de quelques dizaines de millisecondes, ce qui permet de détecter ou de quantifier avec une grande sensibilité, et de façon multiplexée, un grand nombre de molécules différentes ;
- le cas échéant, la détermination des propriétés de typage, et du facteur de virulence est mise en oeuvre dans le même appareil de spectrométrie de masse que la détermination des marqueurs de résistance à au moins un antimicrobien, de préférence simultanément, ce qui a pour avantage de réduire le temps d'analyse et le coût de l'instrument, cela facilite également le traitement et le rendu des résultats.

Outre la détermination de la résistance à un antibiotique, il convient d'identifier le ou les microorganismes présents dans l'échantillon à tester.

Les procédés d'identification de microorganismes sont largement connus de l'homme du métier, comme décrit par exemple par Murray P. R. el al. dans Manuel of Clinical Microbiology, 2007, 9ème édition, et en particulier dans le Vol. I, Section III, chapitres 15 et 16 pour les bactéries et levures, Vol. II, Section VI, chapitre 82 pour les virus, et Vol. II, Section X, chapitre 135 pour les protozoaires. A titre d'exemple de procédés classiques d'identification, on peut citer la détermination du profil biologique, en utilisant par exemple les cartes d'identification du Vitek 2 (bioMérieux), ou bien encore en utilisant des techniques de biologie moléculaire avec des critères d'identification basés sur l'étude de la présence de certains gènes, sur l'étude de leur séquence.

L'identification peut être mise en oeuvre directement à partir de l'échantillon dans lequel on effectue l'identification, ou bien les microorganismes contenus dans l'échantillon peuvent être cultivés par des méthodes bien connues de l'homme du métier avec des milieux de culture et des conditions de culture optimales adaptées en fonction des espèces de microorganismes à rechercher, comme décrit par Murray P.R. et al. dans Manuel of Clinical Microbiology, 2007, 9ème édition, Vol. I, Section III, chapitre 14, et en particulier dans le Vol. I, Section IV, chapitre 21 pour les bactéries, Vol. II, Section VI, chapitre 81 pour les virus, Vol. II, Section VIII, chapitre 117 pour les levures, et Vol. II, Section X, chapitre 134 pour les protozoaires.

Ainsi, de façon générale, dans le cas d'une identification par une méthode biochimique d'une bactérie dans un prélèvement, il faut d'abord l'obtenir en culture pure, par exemple après ensemencement sur gélose. La biologie moléculaire (PCR) peut dans certains cas s'appliquer directement à l'échantillon à analyser.

Au lieu de cultiver les microorganismes, ces derniers peuvent être concentrés par capture directement dans l'échantillon au moyen de surfaces actives. Un tel procédé a été décrit par W.-J. Chen et al. [11] qui ont capturé différentes espèces bactériennes à l'aide de billes magnétiques avec une surface activée au Fe₃O₄/TiO₂.

Une capture par d'autres moyens est également possible, telle qu'une capture par des lectines [23], ou par des anticorps [24], ou encore par de la Vancomycine [25]. La capture permet de concentrer les microorganismes et ainsi de réduire ou même de supprimer l'étape de culture. Il s'en suit un gain de temps considérable.

L'identification peut également être mise en oeuvre par spectrométrie de masse, selon les techniques décrites précédemment, de préférence par MS, par MS/MS, ou bien par MS suivie d'une spectrométrie de type MS/MS, ce qui constitue un mode de réalisation de l'invention. Dans ce cas également, l'échantillon peut être soumis préalablement à une étape de culture telle qu'un ensemencement sur gélose.

L'utilisation d'un procédé d'identification par MS est avantageux en ce qu'il peut être réalisé en quelques minutes et en ce qu'il nécessite un spectromètre de masse avec un seul analyseur, c'est à dire un instrument moins complexe qu'un spectromètre de masse en tandem utilisé en MS/MS.

L'utilisation d'un procédé d'identification par MS suivi d'une spectrométrie de type MS/MS est également avantageuse. Elle permet de s'assurer de l'identité des ions observée en MS, ce qui augmente la spécificité de l'analyse.

L'utilisation d'un procédé d'identification par MS/MS de type MRM présente l'avantage d'être plus sensible et plus simple que les approches MS puis MS/MS traditionnelle. Ce procédé ne nécessite ni logiciel performant pour traiter l'information entre l'acquisition du spectre MS et du spectre MS/MS, ni changement du réglage des paramètres machines pour enchaîner des spectres MS puis MS/MS.

Le procédé d'identification par MS peut être mis en oeuvre avec une source électrospray sur échantillon brut, comme décrit par S. Vaidyanathan et al. [26] ou encore par R. Everley et al. [27] après séparation chromatographique. Différentes gammes de m/z permettent alors d'identifier les microorganismes. S. Vaidyanathan et al. ont utilisé une fenêtre entre 200 et 2000 Th, et R. Everley et al. une fenêtre entre 620 et 2450 Th. Les spectres de masse peuvent également être déconvolués pour accéder à la masse des protéines indépendamment de leur état de charge. R. Everley et al. ont ainsi exploité les masses entre environ 5 000 et 50 000 Da. De façon alternative, le procédé d'identification par MS peut être également mis en oeuvre à l'aide d'un MALDI-TOF, comme décrit par Claydon et al [3] et T. Krishnamurthy et P. Ross [4]. L'analyse associe l'acquisition d'un spectre de masse et l'interprétation d'un logiciel expert. Elle est extrêmement simple et peut être effectuée en quelques minutes. Ce procédé d'identification se répand actuellement dans les laboratoires d'analyse médicale [28].

L'identification de bactéries par MS puis MS/MS via leurs protéines présentes dans l'échantillon, a été largement appliquée par de nombreuses équipes. A titre d'exemple, il est possible de citer les travaux récents de Manes N. et al. [29] qui ont étudié le peptidome de *Salmonella enterica,* ou les travaux de R. Nandakumar et al. [30] ou de L. Hernychova et al. [31] qui ont étudié le protéome de bactéries après digestion des protéines avec de la trypsine. L'approche classique consiste à i) acquérir un spectre MS, ii) sélectionner successivement chaque ion précurseur observé sur le spectre MS avec un signal intense, iii) fragmenter successivement chaque ion précurseur et acquérir son spectre MS/MS, iv) interroger des bases de données protéiques telles que SWISS-PROT ou NCBI, aux travers de logiciels tels que Mascot (Matrix Science, Londres, Royaume-Uni) ou SEQUEST (Thermo Scientific, Waltham, Etats-Unis d'Amérique), pour identifier le peptide ayant une forte probabilité de correspondre au spectre MS/MS observé. Cette méthode peut conduire à l'identification d'un microorganisme si une protéine ou un peptide caractéristique de l'espèce est identifié.

Un des avantages de l'utilisation de la spectrométrie de masse réside en ce qu'elle est particulièrement utile pour quantifier des molécules, dans le cas présent les marqueurs des mécanismes de résistance des bactéries aux beta-lactamines. Pour ce faire, on utilise l'intensité de courant détectée, laquelle est proportionnelle à la quantité de molécule cible. L'intensité de courant ainsi mesurée pourra servir de mesure quantitative permettant de déterminer la quantité de molécule cible présente, laquelle est caractérisée par son expression en unités du Système International (SI) de type mol/m³ ou kg/m³, ou par les multiples ou sous-multiples de ces unités, ou par les dérivées usuelles des unités SI, y compris leurs multiples ou sous-multiples. A titre d'exemple non limitatif, les unités telles que ng/ml ou fmol/l sont des unités caractérisant une mesure quantitative.

Un calibrage est néanmoins nécessaire pour pouvoir corréler l'aire du pic mesurée, correspondant à l'intensité de courant induit par les ions détectés, à la quantité de molécule cible à doser. Pour cela, les calibrages classiquement utilisés en spectrométrie de masse pourront être mis en oeuvre, dans le cadre de l'invention. Les dosages MRM sont classiquement calibrés à l'aide de standards externes ou, de préférence, à l'aide de standards internes tels que décrits par T. Fortin et al. [13].

Dans le cas où la molécule cible est un peptide protéotypique, permettant de doser une protéine d'intérêt, la corrélation entre la mesure quantitative et la quantité de peptide protéotypique cible, et par la suite de protéine d'intérêt, est obtenue en étalonnant le signal mesuré par rapport à un signal étalon pour lequel la quantité à doser est connue. L'étalonnage peut être réalisé au moyen d'une courbe d'étalonnage, par exemple obtenue par injections successives de peptide protéotypique étalon à différentes concentrations (étalonnage externe), ou de façon préférentielle, par étalonnage interne en utilisant un peptide lourd, comme standard interne, par exemple conformément aux méthodes AQUA, QconCAT ou PSAQ détaillées ci-après. Par « peptide lourd », on entend un peptide correspondant au peptide protéotypique, mais dans lequel un ou plusieurs atomes de carbone 12 (¹²C) est (sont) remplacé(s) par du carbone 13 (¹³C), et/ou un ou plusieurs atomes d'azote 14 (¹⁴N) est (sont) remplacé(s) par de l'azote 15 (¹⁵N).

L'utilisation de peptides lourds, comme standards internes (AQUA), a également été proposée dans la demande de brevet US 2004/0229283. Le principe est de synthétiser artificiellement des peptides protéotypiques avec des acides aminés comportant des isotopes plus lourds que les isotopes naturels usuels. De tels acides aminés sont obtenus, par exemple, en remplaçant certains des atomes de carbone 12 (¹²C) par du carbone 13 (¹³C), ou en replaçant certains des atomes d'azote 14 (¹⁴N) par de l'azote 15 (¹⁵N). Le peptide artificiel (AQUA) ainsi synthétisé a rigoureusement les mêmes propriétés physicochimiques que le peptide naturel (à l'exception d'une masse plus élevée). Il est généralement ajouté, à une concentration donnée, à l'échantillon, en amont du dosage par spectroscopie de masse, par exemple entre le traitement entrainant le clivage des protéines de l'échantillon d'intérêt et le fractionnement des peptides obtenus après l'étape de traitement. De ce fait, le peptide AQUA est co-purifié avec le peptide naturel à doser, lors du fractionnement des peptides. Les deux peptides sont donc injectés simultanément dans le spectromètre de masse, pour le dosage. Ils subissent alors les mêmes rendements d'ionisation dans la source. La comparaison des aires de pic des peptides naturels et AQUA, dont la concentration est connue, permet de calculer la concentration du peptide naturel et de remonter ainsi à la concentration de la protéine à doser. Une variante de la technique AQUA a été proposée par J.-M. Pratt et al. [32] sous le nom de QconCat. Cette variante est également décrite dans la demande de brevet WO 2006/128492. Elle consiste à concaténer différents peptides AQUA et à produire le polypeptide artificiel sous forme de protéine recombinante lourde. La protéine recombinante est synthétisée avec des acides aminés comportant des isotopes lourds. De cette façon, il est possible d'obtenir un standard pour calibrer le dosage simultané de plusieurs protéines à moindre coût. Le standard QconCAT est ajouté dès le début, en amont du traitement entrainant le clivage des protéines et avant les étapes de fractionnement des protéines, de dénaturation, de réduction puis de blocage des fonctions thiols des protéines, si celles-ci sont présentes. Le standard QconCAT subit donc le même cycle de traitement entrainant le clivage des protéines que la protéine naturelle, ce qui permet de tenir compte du rendement de l'étape de traitement entrainant le clivage des protéines. En effet, le traitement, notamment par digestion, de la protéine naturelle peut ne pas être complet. Dans ce cas l'utilisation d'un standard AQUA conduirait à sous-estimer la quantité de protéine naturelle. Pour un dosage absolu, il peut donc être important de tenir compte des rendements de traitement entrainant le clivage des protéines.

Cependant, V. Brun et al. [33] ont montré que, parfois, les standards QconQAT ne reproduisaient pas exactement le rendement de traitement notamment par digestion de la protéine naturelle, sans doute du fait d'une conformation tridimensionnelle différente de la protéine QconCAT.

V. Brun et al. [33] ont alors proposé d'utiliser une méthode baptisée PSAQ et décrite dans la demande de brevet WO 2008/145763. Dans ce cas, le standard interne est une protéine recombinante, ayant la même séquence que la protéine naturelle mais synthétisée avec des acides aminés lourds. La synthèse est réalisée ex-vivo avec des acides aminés lourds. Ce standard a rigoureusement les mêmes propriétés physicochimiques que la protéine naturelle (à l'exception d'une masse plus élevée). Il est ajouté dès le début, avant l'étape de fractionnement des protéines, lorsque cette dernière est présente. Il est donc co-purifié avec la protéine native, lors de l'étape de fractionnement des protéines. Il présente le même rendement de traitement, notamment par digestion, que la protéine native. Le peptide lourd obtenu après clivage est également co-purifié avec le peptide naturel, si une étape de fractionnement des peptides est réalisée. Les deux peptides sont donc injectés simultanément dans le spectromètre de masse, pour être dosé quantitativement. Ils subissent alors les mêmes rendements d'ionisation dans la source. La comparaison des aires de pic des peptides naturels et des peptides de référence dans la méthode PSAQ permet de calculer la concentration de la protéine à doser en tenant compte de la totalité des étapes du procédé de dosage.

L'ensemble de ces techniques, à savoir AQUA, QconCAT ou PSAQ ou toute autre technique de calibrage, utilisée dans des dosages par spectrométrie de masse et en particulier dans les dosages MRM ou MS, pourront être mises en oeuvre pour effectuer le calibrage, dans le cadre de l'invention.

La spectrométrie de masse utilisée dans le procédé de détection selon l'invention est de type MS/MS. Plus préférentiellement, la spectrométrie de masse est la MRM.

Le procédé de l'invention permet la détection de résistances aux céphalosporines, caractérisée par la détection d'au moins un peptide en tant que marqueur de résistance. De façon préférentielle, ledit peptide marqueur de résistance appartient aux protéines TEM, CMY, CTX-M, SHV, FOX, ACC, ACT, CARB, DHA, MIR, MOX, PER, VEB, OXA ou GES.

En particulier, la détection d'un mécanisme de résistance aux céphalosporines induit par l'expression de la protéine TEM est caractérisée par la détection d'au moins un peptide appartenant à la protéine TEM et à ses différents variants de séquences **SEQ ID** N **1** à **SEQ ID N°165** et **SEQ ID N°1836** à **SEQ ID N°1843.**
**SEQ ID N° 1** :
**SEQ** ID **N° 2** :
**SEQ ID N° 2** : 
MSIQHFRVALIPFFAAFCLPVFAHPETLVKVKDAEDKLGARVGYIELDLNSGKILESFR
PEERFPMMSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDRWEPELNEAI
PNDERDTTMPAAMATTLRKLLTGELLTLASRQQLIDWMEADKVAGPLLRSALPAGW
FIADKSGAGERGSRGIIAALGPDGKPSRIVVIYTTGSQATMDERNRQIAEIGASLIKH
W
**SEQ** ID **N°** 3 : 
MSIQHFRVALIPFFAAFCLPVFAHPETLVKVKDAEDKLGARVGYIELDLNSGKILESFR
PEERFPMMSTFKVLLCGAVLSRVDAGQEQLGRRIHYSQNDLVKYSPVTEKHLTDG
MTVRELCSAAITMSDNTAANLLLTTIGGPKELTAFLHNMGDHVTRLDRWEPELNEAI
PNDERDTTMPAAMATTLRKLLTGELLTLASRQQLIDWMEADKVAGPLLRSALPAGW
FIADKSGASERGSRGIIAALGPDGKPSRIVVIYTTGSQATMDERNRQIAEIGASLIKHW
**SEQ** ID **N° 4** : 
MSIQHFRVALIPFFAAFCFPVFAHPETLVKVKDAEDQLGARVGYIELDLNSGKILESF
RPEERFPMMSTFKVLLCGAVLSRVDAGQEQLGRRIHYSQNDLVKYSPVTEKHLTDG
MTVRELCSAAITMSDNTAANLLLTTIGGPKELTAFLHNMGDHVTRLDRWEPELNEAI
PNDERDTTMPAAMATTLRKLLTGELLTLASRQQLIDWMEADKVAGPLLRSALPAGW
FIADKSGASERGSRGIIAALGPDGKPSRIVVIYMTGSQATMDERNRQIAEIGASLIKH
W
**SEQ** ID **N°** 5 : 
MSIQHFRVALIPFFAAFCLPVFAHPETLVKVKDAEDQLGARVGYIELDLNSGKILESF
RPEERFPMMSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDSWEPELNEAI
PNDERDTTMPAAMATTLRKLLTGELLTLASRQQLIDWMEADKVAGPLLRSALPAGW
FIADKSGTGKRGSRGIIAALGPDGKPSRIVVIYTTGSQATMDERNRQIAEIGASLIKH
W
**SEQ** ID **N°** 6 : 
MSIQHFRVALIPFFAAFCLPVFAHPETLVKVKDAEDQLGARVGYIELDLNSGKILESF
RPEERFPMMSTFKVLLCGAVLSRVDAGQEQLGRRIHYSQNDLVKYSPVTEKHLTDG
MTVRELCSAAITMSDNTAANLLLTTIGGPKELTAFLHNMGDHVTRLDHWEPELNEAI
PNDERDTTMPAAMATTLRKLLTGELLTLASRQQLIDWMEADKVAGPLLRSALPAGW
FIADKSGAGERGSRGIIAALGPDGKPSRIVVIYTTGSQATMDERNRQIAEIGASLIKH
W
**SEQ** ID **N°** 7 : 
MSIQHFRVALIPFFAAFCLPVFAHPETLVKVKDAEDKLGARVGYIELDLNSGKILESFR
PEERFPMMSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDSWEPELNEAI
PNDERDTTMPAAMATTLRKLLTGELLTLASRQQLIDWMEADKVAGPLLRSALPAGW
FIADKSGAGERGSRGIIAALGPDGKPSRIVVIYTTGSQATMDERNRQIAEIGASLIKH
W
**SEQ** ID **N°** 8 : 
MSIQHFRVALIPFFAAFCLPVFAHPETLVKVKDAEDKLGARVGYIELDLNSGKILESFR
PEERFPMMSTFKVLLCGAVLSRVDAGQEQLGRRIHYSQNDLVKYSPVTEKHLTDG
MTVRELCSAAITMSDNTAANLLLTTIGGPKELTAFLHNMGDHVTRLDSWEPELNEAI
PNDERDTTMPAAMATTLRKLLTGELLTLASRQQLIDWMEADKVAGPLLRSALPAGW
FIADKSGASERGSRGIIAALGPDGKPSRIVVIYTTGSQATMDERNRQIAEIGASLIKHW
**SEQ** ID **N° 9** : 
MSIQHFRVALIPFFAAFCFPVFAHPETLVKVKDAEDQLGARVGYIELDLNSGKILESF
RPEERFPMMSTFKVLLCGAVLSRVDAGQEQLGRRIHYSQNDLVKYSPVTEKHLTDG
MTVRELCSAAITMSDNTAANLLLTTIGGPKELTAFLHNMGDHVTRLDSWEPELNEAI
PNDERDTTMPAAMATTLRKLLTGELLTLASRQQLIDWMEADKVAGPLLRSALPAGW
FIADKSGAGERGSRGIIAALGPDGKPSRIVVIYMTGSQATMDERNRQIAEIGASLIKH
W
**SEQ** ID **N°** 10 : 
MSIQHFRVALIPFFAAFCLPVFAHPETLVKVKDAEDQLGARVGYIELDLNSGKILESF
RPEERFPMMSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDSWEPELNEAI
PNDERDTTMPAAMATTLRKLLTGELLTLASRQQLIDWMEADKVAGPLLRSALPAGW
FIADKSGAGKRGSRGIIAALGPDGKPSRIVVIYTTGSQATMDERNRQIAEIGASLIKH
W
**SEQ** ID **N°** 11 : 
MSIQHFRVALIPFFAAFCLPVFAHPETLVKVKDAEDKLGARVGYIELDLNSGKILESFR
PEERFPMMSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDHWEPELNEAI
PNDERDTTMPAAMATTLRKLLTGELLTLASRQQLIDWMEADKVAGPLLRSALPAGW
FIADKSGAGERGSRGIIAALGPDGKPSRIVVIYTTGSQATMDERNRQIAEIGASLIKH
W
**SEQ** ID **N°** 12 : 
MSIQHFRVALIPFFAAFCLPVFAHPETLVKVKDAEDQLGARVGYIELDLNSGKILESF
RPEERFPMMSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDSWEPELNEAI
PNDERDTTMPAAMATTLRKLLTGELLTLASRQQLIDWMEADKVAGPLLRSALPAGW
FIADKSGAGERGSRGIIAALGPDGKPSRIVVIYTTGSQATMDERNRQIAEIGASLIKH
W
**SEQ** ID **N°** 13 : 
MSIQHFRVALIPFFAAFCLPVFAHPETLVKVKDAEDKLGARVGYIELDLNSGKILESFR
PEERFPMMSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDRWEPELNEAI
PNDERDTTMPAAMATTLRKLLTGELLTLASRQQLIDWMEADKVAGPLLRSALPAGW
FIADKSGAGERGSRGIIAALGPDGKPSRIVVIYMTGSQATMDERNRQIAEIGASLIKH
W
**SEQ** ID **N°** 14 : 
MSIQHFRVALIPFFAAFCLPVFAHPETLVKVKDAEDQLGARVGYIELDLNSGKILESF
RPEERFPMMSTFKVLLCGAVLSRVDAGQEQLGRRIHYSQNDLVKYSPVTEKHLTDG
MTVRELCSAAITMSDNTAANLLLTTIGGPKELTAFLHNMGDHVTRLDRWEPELNEAI
PNDERDTTMPAAMATTLRKLLTGELLTLASRQQLIDWMEADKVAGPLLRSALPAGW
FIADKSGASERGSRGIIAALGPDGKPSRIVVIYTTGSQATMDERNRQIAEIGASLIKHW
**SEQ** ID **N°** 15 : 
MSIQHFRVALIPFFAAFCLPVFAHPETLVKVKDAEDKLGARVGYIELDLNSGKILESFR
PEERFPMMSTFKVLLCGAVLSRVDAGQEQLGRRIHYSQNDLVKYSPVTEKHLTDG
MTVRELCSAAITMSDNTAANLLLTTIGGPKELTAFLHNMGDHVTRLDHWEPELNEAI
PNDERDTTMPAAMATTLRKLLTGELLTLASRQQLIDWMEADKVAGPLLRSALPAGW
FIADKSGAGERGSRGIIAALGPDGKPSRIVVIYTTGSQATMDERNRQIAEIGASLIKH
W
**SEQ** ID **N°** 16 : 
MSIQHFRVALIPFFAAFCLPVFAHPETLVKVKDAEDQLGARVGYIELDLNSGKILESF
RPEERFPMMSTFKVLLCGAVLSRVDAGQEQLGRRIHYSQNDLVKYSPVTEKHLTDG
MTVRELCSAAITMSDNTAANLLLTTIGGPKELTAFLHNMGDHVTRLDRWEPELNEAI
PNDERDTTMPAAMATTLRKLLTGELLTLASRQQLIDWMEADKVAGPLLRSALPAGW
FIADKSGAGERGSRGIIAALGPDGKPSRIVVIYTTGSQATMDERNRQIAEIGASLIKH
W
**SEQ** ID **N°** 17 : 
MSIQHFRVALIPFFAAFCLPVFAHPETLVKVKDAEDKLGARVGYIELDLNSGKILESFR
PEERFPMMSTFKVLLCGAVLSRVDAGQEQLGRRIHYSQNDLVKYSPVTEKHLTDG
MTVRELCSAAITMSDNTAANLLLTTIGGPKELTAFLHNMGDHVTRLDRWEPELNEAI
PNDERDTTMPAAMATTLRKLLTGELLTLASRQQLIDWMEADKVAGPLLRSALPAGW
FIADKSGAGERGSRGIIAALGPDGKPSRIVVIYTTGSQATMDERNRQIAEIGASLIKH
W
**SEQ** ID **N°** 18 : 
MSIQHFRVALIPFFAAFCLPVFAHPETLVKVKDAEDQLGARVGYIELDLNSGKILESF
RPEERFPMMSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDRWEPELNEAI
PNDERDTTMPAAMATTLRKLLTGELLTLASRQQLIDWMEADKVAGPLLRSALPAGW
FIADKSGASERGSRGIIAALGPDGKPSRIVVIYTTGSQATMDERNRQIAEIGASLIKHW
**SEQ** ID **N°** 19 : 
MSIQHFRVALIPFFAAFCLPVFAHPETLVKVKDAEDQLGARVGYIELDLNSGKILESF
RPEERFPMMSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDRWEPELNEAI
PNDERDTTTPAAMATTLRKLLTGELLTLASRQQLIDWMEADKVAGPLLRSALPAGW
FIADKSGASERGSRGIIAALGPDGKPSRIVVIYTTGSQATMDERNRQIAEIGASLIKHW
**SEQ** ID **N° 20** : 
MSIQHFRVALIPFFAAFCLPVFAHPETLVKVKDAEDKLGARVGYIELDLNSGKILESFR
PEERFPMMSTFKVLLCGAVLSRVDAGQEQLGRRIHYSQNDLVKYSPVTEKHLTDG
MTVRELCSAAITMSDNTAANLLLTTIGGPKELTAFLRNMGDHVTRLDRWEPELNEAI
PNDERDTTMPAAMATTLRKLLTGELLTLASRQQLIDWMEADKVAGPLLRSALPAGW
FIADKSGASERGSRGIIAALGPDGKPSRIVVIYTTGSQATMDERNRQIAEIGASLIKHW
**SEQ** ID **N°** 21 : 
MSIQHFRVALIPFFAAFCLPVFAHPETLVKVKDAEDKLGARVGYIELDLNSGKILESFR
PEERFPMMSTFKVLLCGAVLSRVDAGQEQLGRRIHYSQNDLVKYSPVTEKHLTDG
MTVRELCSAAITMSDNTAANLLLTTIGGPKELTAFLHNMGDHVTRLDRWEPELNEAI
PNDERDTTMPAAMATTLRKLLTGELLTLASRQQLIDWMEADKVAGPLLRSALPAGW
FIADKSGGSERGSRGIIAALGPDGKPSRIVVIYTTGSQATMDERNRQIAEIGASLIKH
**SEQ** ID **N° 22** : 
MSIQHFRVALIPFFAAFCLPVFAHPETLVKVKDAEDKLGARVGYIELDLNSGKILESFR
PEERFPMMSTFKVLLCGAVLSRVDAGQEQLGRRIHYSQNDLVKYSPVTEKHLTDG
 
MTVRELCSAAITMSDNTAANLLLTTIGGPKELTAFLHNMGDHVTRLDSWEPELNEAI
PNDERDTTMPAAMATTLRKLLTGELLTLASRQQLIDWMEADKVAGPLLRSALPAGW
FIADKSGTGKRGSRGIIAALGPDGKPSRIVVIYTTGSQATMDERNRQIAEIGASLIKH
W
**SEQ** ID **N°** 23 : 
MSIQHFRVALIPFFAAFCFPVFAHPETLVKVKDAEDQLGARVGYIELDLNSGKILESF
RPEERFPMMSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDRWEPELNEAI
PNDERDTTMPAAMATTLRKLLTGELLTLASRQQLIDWMEADKVAGPLLRSALPAGW
FIADKSGASERGSRGIIAALGPDGKPSRIVVIYMTGSQATMDERNRQIAEIGASLIKH
W
**SEQ** ID **N° 24 :** 
MSIQHFRVALIPFFAAFCLPVFAHPETLVKVKDAEDQLGARVGYIELDLNSGKILESF
RPEERFPMMSTFKVLLCGAVLSRVDAGQEQLGRRIHYSQNDLVKYSPVTEKHLTDG
MTVRELCSAAITMSDNTAANLLLTTIGGPKELTAFLHNMGDHVTRLDSWEPELNEAI
PNDERDTTMPAAMATTLRKLLTGELLTLASRQQLIDWMEADKVAGPLLRSALPAGW
FIADKSGAGERGSRGIIAALGPDGKPSRIVVIYTTGSQATMDERNRQIAEIGASLIKH
W
**SEQ** ID **N° 25 :** 
MSIQHFRVALIPFFAAFCLPVFAHPETLVKVKDAEDQLGARVGYIELDLNSGKILESF
RPEERFPMMSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDHWEPELNEAI
PNDERDTTMPAAMATTLRKLLTGELLTLASRQQLIDWMEADKVAGPLLRSALPAGW
FIADKSGAGKRGSRGIIAALGPDGKPSRIVVIYMTGSQATMDERNRQIAEIGASLIKH
W
**SEQ** ID **N°** 26 : 
MSIQHFRVALIPFFAAFCLPVFAHPETLVKVKDAEDQLGARVGYIELDLNSGKILESF
RPEERFPMMSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDHWEPELNEAI
PNDERDTTMPAAMATTLRKLLTGELLTLASRQQLIDWMEADKVAGPLLRSALPAGW
FIADKSGAGKRGSRGIIAALGPDGKPSRIVVIYTTGSQATMDERNRQIAEIGASLIKH
W
**SEQ** ID **N°** 27 : MSIQHFRVALIPFFAAFCLPVFAHPETLVKVKDAEDQLGARVGYIELDLNSGKILESF 
RPEERFPMMSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDHWEPELNEAI
PNDERDTTMPAAMATTLRKLLTGELLTLASRQQLIDWMEADKVAGPLLRSALPAGW
FIADKSGAGERGSRGIIAALGPDGKPSRIVVIYTTGSQATMDERNRQIAEIGASLIKH
W
**SEQ** ID **N°** 28 : 
MSIQHFRVALIPFFAAFCLPVFAHPETLVKVKDAEDQLGARVGYIELDLNSGKILESF
RPEERFPMMSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDRWEPELNEAI
PNDERDTTMPAAMATTLRKLLTGELLTLASRQQLIDWMEADKVAGPLLRSALPAGW
FIADKSGAGERGSSGIIAALGPDGKPSRIVVIYTTGSQATMDERNRQIAEIGASLIKH
W
**SEQ** ID **N° 29** : 
MSIQHFRVALIPFFAAFCLPVFAHPETLVKVKDAEDQLGARVGYIELDLNSGKILESF
RPEERFPMMSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDRWEPELNEAI
PNDERDTTMPAAMATTLRKLLTGELLTLASRQQLIDWMEADKVAGPLLRSALPAGW
FIADKSGAGERGSCGIIAALGPDGKPSRIVVIYTTGSQATMDERNRQIAEIGASLIKH
W
**SEQ** ID **N°** 30 : 
MSIQHFRVALIPFFAAFCLPVFAHPETLVKVKDAEDQLGARVGYIELDLNSGKILESF
RPEERFPMISTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDRWEPELNEAI
PNDERDTTTPAAMATTLRKLLTGELLTLASRQQLIDWMEADKVAGPLLRSALPAGW
FIADKSGAGERGSRGIIAALGPDGKPSRIVVIYTTGSQATMDERNRQIAEIGASLIKH
W
**SEQ** ID **N°** 31 : 
MSIQHFRVALIPFFAAFCLPVFAHPETLVKVKDAEDQLGARVGYIELDLNSGKILESF
RPEERFPMLSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDRWEPELNEAI
PNDERDTTMPAAMATTLRKLLTGELLTLASRQQLIDWMEADKVAGPLLRSALPAGW
FIADKSGAGERGSRGIIAALGPDGKPSRIVVIYTTGSQATMDERNRQIAEIGASLIKH
W
**SEQ** ID **N°** 32 : 
MSIQHFRVALIPFFAAFCLPVFAHPETLVKVKDAEDQLGARVGYIELDLNSGKILESF
RPEERFPMVSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDRWEPELNEAI
PNDERDTTMPAAMATTLRKLLTGELLTLASRQQLIDWMEADKVAGPLLRSALPAGW
FIADKSGAGERGSRGIIAALGPDGKPSRIVVIYTTGSQATMDERNRQIAEIGASLIKH
W
**SEQ ID N° 33 :** 
MSIQHFRVALIPFFAAFCLPVFAHPETLVKVKDAEDQLGARVGYIELDLNSGKILESF
RPEERFPMLSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDRWEPELNEAI
PNDERDTTMPAAMATTLRKLLTGELLTLASRQQLIDWMEADKVAGPLLRSALPAGW
FIADKSGAGERGSRGIIAALGPDGKPSRIVVIYTTGSQATMDERDRQIAEIGASLIKH
W
**SEQ ID N° 34 :** 
MSIQHFRVALIPFFAAFCLPVFAHPETLVKVKDAEDQLGARVGYIELDLNSGKILESF
RPEERFPMVSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDRWEPELNEAI
PNDERDTTMPAAMATTLRKLLTGELLTLASRQQLIDWMEADKVAGPLLRSALPAGW
FIADKSGAGERGSRGIIAALGPDGKPSRIVVIYTTGSQATMDERDRQIAEIGASLIKH
W
**SEQ ID N° 35 :** 
MSIQHFRVALIPFFAAFCLPVFAHPETLVKVKDAEDQLGARVGYIELDLNSGKILESF
RPEERFPMISTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDRWEPELNEAI
PNDERDTTMPAAMATTLRKLLTGELLTLASRQQLIDWMEADKVAGPLLRSALPAGW
FIADKSGAGERGSRGIIAALGPDGKPSRIVVIYTTGSQATMDERDRQIAEIGASLIKH
W
**SEQ ID N° 36 :** 
MSIQHFRVALIPFFAAFCLPVFAHPETLVKVKDAEDQLGARVGYIELDLNSGKILESF
RPEERFPMVSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDRWEPELNEAI
PNDERDTTMPAAMATTLRKLLTGELLTLASRQQLIDWMEADKVAGPLLRSALPAGW
FIADKSGAGERGSRGIIAALGPDGKPSRIVVIYTTGSQATMDELNRQIAEIGASLIKHW
**SEQ ID N° 37 :** 
MSIQHFRVALIPFFAAFCLPVFAHPETLVKVKDAEDQLGARVGYIELDLNSGKILESF
RPEERFPMLSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDRREPELNEAIP
NDERDTTMPAAMATTLRKLLTGELLTLASRQQLIDWMEADKVAGPLLRSALPAGWF
IADKSGAGERGSRGIIAALGPDGKPSRIVVIYTTGSQATMDERDRQIAEIGASLIKHW
**SEQ** ID **N°** 38 : 
MSIQHFRVALIPFFAAFCLPVFAHPETLVKVKDAEDQLGARVGYIELDLNSGKILESF
RPEERFPMISTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDRWEPELNEAI
PNDERDTTMPAAMATTLRKLLTGELLTLASRQQLIDWMEADKVAGPLLRSALPAGW
FIADKSGAGERGSRGIIAALGPDGKPSRIVVIYTTGSQATMDERNRQIAEIGASLIKH
W
**SEQ** ID **N°** 39 : 
MSIQHFRVALIPFFAAFCLPVFAHPETLVKVKDAEDQLGARVGYIELDLNSGKILESF
RPEERFPMMSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDRWEPELNEAI
PNDERDTTMPAAMATTLRKLLTGELLTLASRQQLIDWMEADKVAGPLLRSALPAGW
FIADKSGAGERGSTGIIAALGPDGKPSRIVVIYTTGSQATMDERNRQIAEIGASLIKHW
**SEQ** ID **N° 40** : 
MSIQHFRVALIPFFAAFCLPVFAHPETLVKVKDAEDKLGVRVGYIELDLNSGKILESFR
PEERFPMMSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDRWEPELNEAI
PNDERDTTMPAAMATTLRKLLTGELLTLASRQQLIDWMEADKVAGPLLRSALPAGW
FIADKSGASKRGSRGIIAALGPDGKPSRIVVIYMTGSQATMDERNRQIAEIGASLIKH
W
**SEQ** ID **N°** 41 : 
MSIQHFRVALIPFFAAFCLPVFAHPETLVKVKDAEDQLGARVGYIELDLNSGKILESF
RPEERFPMMSTFKVLLCGAVLSRVDAGQEQLGRRIHYSQNDLVKYSPVTEKHLTDG
MTVRELCSAAITMSDNTAANLLLTTIGGPKELTAFLHNMGDHVTRLDHWEPELNEAI
PNDERDTTTPAAMATTLRKLLTGELLTLASRQQLIDWMEADKVAGPLLRSALPAGW
FIADKSGAGERGSRGIIAALGPDGKPSRIVVIYTTGSQATMDERNRQIAEIGASLIKH
W
**SEQ** ID **N° 42** : MSIQHFRVALIPFFAAFCLPVFAHPETLVKVKDAEDKLGARVGYIELDLNSGKILESFR 
PEERFPMMSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDRWEPELNEAI
PNDERDTTMPAAMATTLRKLLTGELLTLASRQQLIDWMEADKVAGPLLRSALPAGW
FIADKSGAGERGSSGIIAALGPDGKPSRIVVIYTTGSQATMDERNRQIAEIGASLIKH
W
**SEQ ID N° 43 :** 
MSIQHFRVALIPFFAAFCLPVFAHPETLVKVKDAEDQLGARVGYIELDLNSGKILESF
RPEERFPMLSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDRWEPELNEAI
PNDERDTTMPAAMATTLRKLLTGELLTLASRQQLIDWMEADKVAGPLLRSALPAGW
FIADKSGAGERGSRGIIAALGPDGKPSRIVVIYTTGSQATMDEQNRQIAEIGASLIKH
W
**SEQ ID N° 44 :** 
MSIQHFRVALIPFFAAFCLPVFAHPETLVKVKDAEDKLGARVGYIELDLNSGKILESFR
PEERFPMMSTFKVLLCGAVLSRVDAGQEQLGRRIHYSQNDLVKYSPVTEKHLTDG
MTVRELCSAAITMSDNTAANLLLTTIGGPKELTAFLHNMGDHVTRLDSWEPELNEAI
PNDERDTTMPAAMATTLRKLLTGELLTLASRQQLIDWMEADKVAGPLLRSALPAGW
FIADKSGAGKRGSRGIIAALGPDGKPSRIVVIYTTGSQATMDERNRQIAEIGASLIKH
W
**SEQ ID N° 45 :** 
MSIQHFRVALIPFFAAFCLPVFAHPETLVKVKDAEDQLGARVGYIELDLNSGKILESF
RPEERFPMMSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDRWEPELNEAI
PNDERDTTMPAAMATTLRKLLTGELLTLASRQQLIDWMEADKVAGPLLRSALPAGW
FIADKSGASKRGSRGIIAALGPDGKPSRIVVIYMTGSQATMDERNRQIAEIGASLIKH
W
**SEQ ID N° 46 :** 
MSIQHFRVALIPFFAAFCFPVFAHPETLVKVKDAEDQLGARVGYIELDLNSGKILESF
RPEERFPMMSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDRWEPELNEAI
PNDERDTTMPAAMATTLRKLLTGELLTLASRQQLIDWMEADKVAGPLLRSALPAGW
FIADKSGASKRGSRGIIAALGPDGKPSRIVVIYMTGSQATMDERNRQIAEIGASLIKH
W
**SEQ ID N° 47 :** 
MSIQHFRVALIPFFAAFCFPVFAHPETLVKVKDAEDQLGARVGYIELDLNSGKILESF
RPEERFPMMSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDRWEPELNEAI
PNDERDTTMPAAMATTLRKLLTGELLTLASRQQLIDWMEADKVAGPLLRSALPAGW
FIADKSGASKRGSRGIIAALGPDGKPSRIVVIYMTGGQATMDERNRQIAEIGASLIKH
W
**SEQ ID N° 48 :** 
MSIQHFRVALIPFFAAFCLPVFAHPETLVKVKDAEDQLGARVGYIELDLNSGKILESF
RPEERFPMLSTFKVLLCGAVLSRVDAGQEQLGRRIHYSQNDLVKYSPVTEKHLTDG
MTVRELCSAAITMSDNTAANLLLTTIGGPKELTAFLHNMGDHVTRLDRWEPELNEAI
PNDERDTTMPAAMATTLRKLLTGELLTLASRQQLIDWMEADKVAGPLLRSALPAGW
FIADKSGASERGSRGIIAALGPDGKPSRIVVIYTTGSQATMDERDRQIAEIGASLIKHW
**SEQ ID N° 49 :** 
MSIQHFRVALIPFFAAFCLPVFAHPETLVKVKDAEDQLGARVGYIELDLNSGKILESF
RPEERFPMMSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDRWEPELNEAI
PNDERDTTMPAAMATTLRKLLTGELLTLASRQQLIDWMEADKVAGPLLRSALPAGW
FIADKSGAGERGSHGIIAALGPDGKPSRIVVIYTTGSQATMDERNRQIAEIGASLIKH
W
**SEQ ID N° 50 :** 
MSIQHFRVALIPFFAAFCLPVFAHPETLVKVKDAEDQLGARVGYIELDLNSGKILESF
RPEERFPMMSTFKVLLCGAVLSRVDAGQEQLGRRIHYSQNDLVKYSPVTEKHLTDG
MTVRELCSAAITMSDNTAANLLLTTIGGPKELTAFLHNMGDHVTRLDRWEPELNEAI
PNDERDTTTPAAMATTLRKLLTGELLTLASRQQLIDWMEADKVAGPLLRSALPAGW
FIADKSGASERGSRGIIAALGPDGKPSRIVVIYTTGSQATMDERNRQIAEIGASLIKHW
**SEQ ID N° 51 :** 
MSIQHFRVALIPFFAAFCFPVFAHPETLVKVKDAEDQLGARVGYIELDLNSGKILESF
RPEERFPMMSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDSWEPELNEAI
PNDERDTTMPAAMATTLRKLLTGELLTLASRQQLIDWMEADKVAGPLLRSALPAGW
FIADKSGAGERGSRGIIAALGPDGKPSRIVVIYTTGSQATMDERNRQIAEIGASLIKH
W
**SEQ ID N° 52 :** MSIQHFRVALIPFFAAFCLPVFAHPETLVKVKDAEDQLGARVGYIELDLNSGKILESF 
RPEERFPMMSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDRWEPELNEAI
PNDERDTTMPAAMATTLRKLLTGELLTLASRQQLIDWMEADKVAGPLLRSALPAGW
FIADKSGAGERGSLGIIAALGPDGKPSRIVVIYTTGSQATMDERNRQIAEIGASLIKHW
**SEQ ID N° 53 :** 
MSIQHFRVALIPFFAAFCLPVFAHPETLVKVKDAEDQLGARVGYIELDLNSGKILESF
RPEERFPMMSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDRWEPELNEAI
PNDERDTTMPAAMATTLRKLLTGELLTLASRQQLIDWMEADKVAEPLLRSALPAGW
FIADKSGAGERGSRGIIAALGPDGKPSRIVVIYTTGSQATMDERNRQIAEIGASLIKH
W
**SEQ ID N° 54 :** 
MSIQHFRVALIPFFAAFCLPVFAHPETLVKVKDAEDKLGARVGYIELDLNSGKILESFR
PEERFPMMSTFKVLLCGAVLSRVDAGQEQLGRRIHYSQNDLVKYSPVTEKHLTDG
MTVRELCSAAITMSDNTAANLLLTTIGGPKELTAFLRNMGDHVTRLDRWEPELNEAI
PNDERDTTMPAAMATTLRKLLTGELLTLASRQQLIDWMEADKVAGPLLRSALPAGW
FIADKSGAGERGSRGIIAALGPDGKPSRIVVIYTTGSQATMDERNRQIAEIGASLIKH
W
**SEQ ID N° 55 :** 
MSIQHFRVALIPFFAAFCLPVFAHPETLVKVKDAEDQLGARVGYIELDLNSGKILESF
RPEERFPMMSTFKVLLCGAVLSRVDAGQEQLDRRIHYSQNDLVEYSPVTEKHLTDG
MTVRELCSAAITMSDNTAANLLLTTIGGPKELTAFLHNMGDHVTRLDRWEPELNEAI
PNDERDTTMPAAMATTLRKLLTGELLTLASRQQLIDWMEADKVAGPLLRSALPAGW
FIADKSGAGERGSRGIIAALGPDGKPSRIVVIYTTGSQATMDERNRQIAEIGASLIKH
W
**SEQ ID N° 56 :** 
MSIQHFRVALIPFFAAFCLPVFAHPETLVKVKDAEDQLGARVGYIELDLNSGKILESF
RPEERFPMMSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDRWEPELNEAI
PNDERDTTMPAAMATTLRKLLTGELLTLASRQQLIDWMEADKVAGPLLRSALPAGW
FIADKSGAGERGSSGIIAALGPDGKPSRIVIIYTTGSQATMDERNRQIAEIGASLIKHW
**SEQ ID N° 57 :** 
MSIQHFRVALIPFFAAFCLPVFAHPETLVKVKDAEDKLGARVGYIELDLNSGKILESFR
PEERFPMMSTFKVLLCGAVLSRVDAGQEQLGRRIHYSQNDLVEYSPVTEKHLTDG
 
MTVRELCSAAITMGDNTAANLLLTTIGGPKELTAFLHNMGDHVTRLDRWEPELNEAI
PNDERDTTMPAAMATTLRKLLTGELLTLASRQQLIDWMEADKVAGPLLRSALPAGW
FIADKSGAGERGSRGIIAALGPDGKPSRIVVIYTTGSQATMDERNRQIAEIGASLIKH
W
**SEQ ID N° 58 :** 
MSIQHFRVALIPFFAAFCLPVFAHPETLVKVKDAEDKLGARVGYIELDPNSGKILESF
RPEERFPMMSTFKVLLCGAVLSRVDAGQEQLGRRIHYSQNDLVKYSPVTEKHLTDG
MTVRELCSAAITMSDNTAANLLLTTIGGPKELTAFLHNMGDHVTRLDSWEPELNEAI
PNDERDTTMPAAMATTLRKLLTGELLTLASRQQLIDWMEADKVAGPLLRSALPAGW
FIADKSGAGERGSRGIIAALGPDGKPSRIVVIYTTGSQATMDERNRQIAEIGASLIKH
W
**SEQ ID N° 59 :** 
MSIQHFRVALIPFFAAFCLPVFAHPETLVKVKDAEDKLGARVGYIELDLNSGKILESFR
PEERFPMMSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDHWEPELNEAI
PNDERDTTMPAAMATTLRKLLTGELLTLASRQQLIDWMEADKVAGPLLRSALPAGW
FIADKSGAGKRGSRGIIAALGPDGKPSRIVVIYTTGSQATMDERNRQIAEIGASLIKH
W
**SEQ ID N° 60 :** 
MSIQHFRVALIPFFAAFCFPVFAHPETLVKVKDAEDQLGARVGYIELDLNSGKILESF
RPEERFPMMSTFKVLLCGAVLSRVDAGQEQLGRRIHYSQNDLVKYSPVTEKHLTDG
MTVRELCSAAITMSDNTAANLLLTTIGGPKELTAFLHNMGDHVTRLDSWEPELNEAI
PNDERDTTTPAAMATTLRKLLTGELLTLASRQQLIDWMEADKVAGPLLRSALPAGW
FIADKSGAGERGSRGIIAALGPDGKPSRIVVIYTTGSQATMDERNRQIAEIGASLIKH
W
**SEQ ID N° 61 :** 
MSIQHFRVALIPFFAAFCLPVFAHPETLVKVKDAEDKLGARVGYIELDLNSGKILESFR
PEERFPMMSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDRWEPELNEAI
PNDERDTTMPAAMATTLRKLLTGELLTLASRQQLIDWMEADKVAGPLLRSALPAGW
FIADKSGAGERGSCGIIAALGPDGKPSRIVVIYTTGSQATMDERNRQIAEIGASLIKH
W
**SEQ ID N° 62 :** MSIQHFRVALIPFFAAFCLPVFAHPETLVKVKDAEDKLGARVGYIELDLNSGKILESFR 
PEERFPMMSTFKVLLCGAVLSRVDAGQEQLDRRIHYSQNDLVKYSPVTEKHLTDG
MTVRELCSAAITMSDNTAANLLLTTIGGPKELTAFLHNMGDHVTRLDRWEPELNEAI
PNDERDTTMPAAMATTLRKLLTGELLTLASRQQLIDWMEADKVAGPLLRSALPAGW
FIADKSGASERGSRGIIAALGPDGKPSRIVVIYTTGSQATMDERNRQIAEIGASLIKHW
**SEQ ID N° 63 :** 
MSIQHFRVALIPFFAAFCIPVFAHPETLVKVKDAEDKLGARVGYIELDLNSGKILESFR
PEERFPMMSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDRWEPELNEAI
PNDERDTTMPAAMATTLRKLLTGELLTLASRQQLIDWMEADKVAGPLLRSALPAGW
FIADKSGAGERGSCGIIAALGPDGKPSRIVVIYTTGSQATMDERNRQIAEIGASLIKH
W
**SEQ ID N° 64 :** 
MSIQHFRVALIPFFAAFCLPVFAHPETLVKVKDAEDQLGARVGYIELDLNSGKILESF
RPEERFPMMSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDRWEPELNEAI
PNDERDTTMPAAMATTLRKLLTGELLTLASRQQLIDWMEADKVAGPLLRSALPAGW
FIADKSGASKRGSRGIIAALGPDGKPSRIVVIYMTGSQATMDELNRQIAEIGASLIKH
W
**SEQ ID N° 65 :** 
MSIQHFRVALIPFFAAFCLPVFAHPETLVKVKDAEDQLGARVGYIELDLNSGKILESF
RPEERFPMMSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDRWEPELNEAI
PNDERDTTMPAAMATTLRKLLTGELLTLASQQQLIDWMEADKVAGPLLRSALPAGW
FIADKSGAGERGSRGIIAALGPDGKPSRIVVIYTTGSQATMDERNRQIAEIGASLIKH
W
**SEQ ID N° 66 :** 
MSIQHFRVALIPFFAAFCLPVFAHPETLVKVKDAEDQLGARVGYIELDLNSGKILESF
RPEERFPMMSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDRWEPELNEAI
PNDERDTTMPAAMATTLRKLLTGELLTLASRQQLIDWMEADKVAGPLLRSALPAGW
FIADKSGASKRGSRGIIAALGPDGKPSRIVVIYTTGSQATMDERNRQIAEIGASLIKHW
**SEQ ID N° 67 :** 
MSIQHFRVALIPFFAAFCLPVFAHPETLVKVKDAEDKLGARVGYIELDLNSGKILESFR
PEERFPMMSTFKVLLCGAVLSRVDAGQEQLGRRIHYSQNDLVEYSPVTEKHLTDG
 
MTVRELCSAAITMSDNTAANLLLTTIGGPKELTAFLHNMGDHVTRLDRWEPELNEAI
PNDERDTTTPAAMATTLRKLLTGELLTLASRQQLIDWMEADKVAGPLLRSALPAGW
FIADKSGASKRGSRGIIAALGPDGKPSRIVVIYTTGSQATMDERNRQIAEIGASLIKHW
**SEQ ID N° 68 :** 
MSIQHFRVALIPFFAAFCFPVFAHPETLVKVKDAEDQLGARVGYIELDLNSGKILESF
RPEERFPMMSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDRWEPELNEAI
PNDERDTTMPAAMATTLRKLLTGELLTLASRQQLIDWMEADKVAGPLLRSALPAGW
FIADKSGAGERGSCGIIAALGPDGKPSRIVVIYMTGSQATMDERNRQIAEIGASLIKH
W
**SEQ ID N° 69 :** 
MSIQHFRVALIPFFAAFCFPVFAHPETLVKVKDAEDQLGARVGYIELDLNSGKILESF
RPEERFPMMSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDRWEPELNEAI
PNDERDTTMPAAMATTLRKLLTGELLTLASRQQLIDWMEADKVAGPLLRSALPAGW
FIADKSGAGERGSSGIIAALGPDGKPSRIVVIYMTGSQATMDERNRQIAEIGASLIKH
W
**SEQ ID N° 70 :** 
MSIQHFRVALIPFFAAFCFPVFAHPETLVKVKDAEDQLGARVGYIELDLNSGKILESF
RPEERFPMMSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDHWEPELNEAI
PNDERDTTMPAAMATTLRKLLTGELLTLASRQQLIDWMEADKVAGPLLRSALPAGW
FIADKSGAGERGSRGIIAALGPDGKPSRIVVIYMTGSQATMDERNRQIAEIGASLIKH
W
**SEQ ID N° 71 :** 
MSIQHFRVALIPFFAAFCLPVFAHPETLVKVKDAEDQLGARVGYIELDLNSGKILESF
RPEERFPMMSTFKVLLCGAVLSRVDAGQEQLGRRIHYSQNDLVEYSPVTEKHLTDG
MTVRELCSAAITMGDNTAANLLLTTIGGPKELTAFLHNMGDHVTRLDRWEPELNEAI
PNDERDTTMPAAMATTLRKLLTGELLTLASRQQLIDWMEADKVAGPLLRSALPAGW
FIADKSGAGERGSRGIIAALGPDGKPSRIVVIYTTGSQATMDERNRQIAEIGASLIKH
W
**SEQ ID N° 72 :** 
MSIQHFRVALIPFFAAFCLPVFAHPETLVKVKDAEDQLGARVGYIELDLNSGKILESF
RPEERFPMLSTFKVLLCGAVLSRVDAGQEQLGRRIHYSQNDLVEYSPVTEKHLTDG
 
MTVRELCSAAITMSDNTAANLLLTTIGGPKELTAFLHNMGDHVTRLDRWEPELNEAI
PNDERDTTMPAAMATTLRKLLTGELLTLASRQQLIDWMEADKVAGPLLRSALPAGW
FIADKSGAGERGSSGIIAALGPDGKPSRIVVIYTTGSQATMDERNRQIAEIGASLIKH
W
**SEQ ID N° 73 :** 
MSIQHFRVALIPFFAAFCLPVFAHPETLVKVKDAEDQLGARVGYIELDLNSGKILESF
RPEERFPMVSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDRREPELNEAIP
NDERDTTMPAAMATTLRKLLTGELLTLASRQQLIDWMEADKVAGPLLRSALPAGWF
IADKSGAGERGSRGIIAALGPDGKPSRIVVIYTTGSQATMDERDRQIAEIGASLIKHW
**SEQ ID N° 74 :** 
MSIQHFRVALIPFFAAFCLPVFAHPETLVKVKDAEDQLGARVGYIELDLNSGKILESF
RPEERFPMMSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDRWEPELNEAI
PNDERDTTMPAAMATTLRKLLTGELLTLASRQQLIDWMEADKVAGPLLRSALPAGW
FIADKSGAGERGSGGIIAALGPDGKPSRIVVIYTTGSQATMDERNRQIAEIGASLIKH
W
**SEQ ID N° 75 :** 
MSIQHFRVALIPFFAAFCLPVFAHPETLVKVKDAEDQLGARVGYIELDLNSGKILESF
RPEERFPMLSTFKVLLCGAVLSRVDAGQEQLGRRIHYSQNDLVEYSPVTEKHLTDG
MTVRELCSAAVTMSDNTAANLLLTTIGGPKELTAFLHNMGDHVTRLDRWEPELNEAI
PNDERDTTMPAAMATTLRKLLTGELLTLASRQQLIDWMEADKVAGPLLRSALPAGW
FIADKSGAGERGSRGIIAALGPDGKPSRIVVIYTTGSQATMDERDRQIAEIGASLIKH
W
**SEQ ID N° 76 :** 
MSIQHFRVALIPFFAAFCLPVFAHPETLVKVKDAEDQLGARVGYIELDLNSGKILESF
RPEERFPMLSTFKVLLCGAVLSRVDAGQEQLGRRIHYSQNDLVEYSPVTEKHLTDG
MTVRELCSAAVTMSDNTAANLLLTTIGGPKELTAFLHNMGDHVTRLDRWEPELNEAI
PNDERDTTMPAAMATTLRKLLTGELLTLASRQQLIDWMEADKVAGPLLRSALPAGW
FIADKSGAGERGSRGIIAALGPDGKPSRIVVIYTTGSQATMDERNRQIAEIGASLIKH
W
**SEQ ID N° 77 :** 
MSIQHFRVALIPFFAAFCLPVFAHPETLVKVKDAEDQLGARVGYIELDLNSGKILESF
RPEERFPMVSTFKVLLCGAVLSRVDAGQEQLGRRIHYSQNDLVEYSPVTEKHLTDG
 
MTVRELCSAAITMSDNTAANLLLTTIGGPKELTAFLHNMGDHVTRLDRWEPELNEAI
PNDERDTTMPAAMATTLRKLLTGELLTLASRQQLIDWMEADKVAGPLLRSALPAGW
FIADKSGAGERGSRGIIAALGPDGKPSRIVVIYTTGSQATMDEQNRQIAEIGASLIKH
W
**SEQ ID N° 78 :** 
MSIQHFRVALIPFFAAFCLPVFAHPETLVKVKDAEDQLGARVGYIELDLNSGKILESF
RPEERFPMLSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDRCEPELNEAIP
NDERDTTMPAAMATTLRKLLTGELLTLASRQQLIDWMEADKVAGPLLRSALPAGWF
IADKSGAGERGSRGIIAALGPDGKPSRIVVIYTTGSQATMDEQNRQIAEIGASLIKHW
**SEQ ID N° 79 :** 
MSIQHFRVALIPFFAAFCLPVFAHPETLVKVKDAEDQLGARVGYIELDLNSGKILESF
RPEERFPMMSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDRWEPELNEAI
PNDERDTTMPAAMATTLRKLLTGELLTLASRQQLIDWMEADKVAGPLLRSALPAGW
FIADKSGAGERGSRGIIAALGPDGKPSRIVVIYTTGSQATMDERDRQIAEIGASLIKH
W
**SEQ ID N° 80 :** 
MSIQHFRVALIPFFAAFCFPVFAHPETLVKVKDAEDQLGARVGYIELDLNSGKILESF
RPEERFPMMSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDSWEPELNEAI
PNDERDTTMPAAMATTLRKLLTGELLTLASRQQLIDWMEADKVAGPLLRSALPAGW
FIADKSGAGKRGSRGIIAALGPDGKPSRIVVIYMTGSQATMDERNRQIAEIGASLIKH
W
**SEQ ID N° 81 :** 
MSIQHFRVALIPFFAAFCFPVFAHPETLVKVKDAEDQLGARVGYIELDLNSGKILESF
RPEERFPMMSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDSWEPELNEAI
PNDERDTTMPAAMATTLRKLLTGELLTLASRQQLIDWMEADKVAGPLLRSALPAGW
FIADKSGTGKRGSRGIIAALGPDGKPSRIVVIYMTGSQATMDERNRQIAEIGASLIKH
W
**SEQ ID N° 82 :** 
MSIKHFRVALIPFFAAFCLPVFAHPETLVKVKDAEDQLGARVGYIELDLNSGKILESFR
PEERFPMMSTFKVLLCGAVLSRVDAGQEQLGRRIHYSQNDLVKYSPVTEKHLTDG
 
MTVRELCSAAITMSDNTAANLLLTTIGGPKELTAFLHNMGDHVTRLHCWEPELNEAI
PNDERDTTTPAAMATTLRKLLTGELLTLASRQQLIDWMEADKVAGPLLRSALPAGW
FIADKSGAGERGSRGIIAALGPDGKPSRIVVIYTTGSQATMDERNRQIAEIGASLIKH
W
**SEQ ID N° 83 :** 
MSIQHFRVALIPFFAAFCLPVFAHPETLVKVKDAEDQLGARVGYIELDLNSGKILESF
RPEERFPMMSTFKVLLCGAVLSRVDAGQEQLGRRIHYSQNDLVKYSPVTEKHLTDG
MTVRELCSAAITMSDNTAANLLLTTIGGPKELTAFLHNMGDHVTRLDRWEPELNEAI
PNDERDTTTPAAMATTLRKLLTDELLTLASRQQLIDWMEADKVAGPLLRSALPAGW
FIADKSGASERGSRGIIAALGPDGKPSRIVVIYTTGSQATMDERNRQIAEIGASLIKHW
**SEQ ID N° 84 :** 
MSIQHFRVALIPFFAAFCLPVFAHPETLVKVKDAEDKLGARVGYIELDLNSGKILESFR
PEERFPMMSTFKVLLCGAVLSRVDAGQEQLGRRIHYSQNDLVKYSPVTEKHLTDG
MTVRELCSAAITMGDNTAANLLLTTIGGPKELTAFLHNMGDHVTRLDRWEPELNEAI
PNDERDTTMPAAMATTLRKLLTGELLTLASRQQLIDWMEADKVAGPLLRSALPAGW
FIADKSGASERGSRGIIAALGPDGKPSRIVVIYTTGSQATMDERNRQIAEIGASLIKHW
**SEQ ID N° 85 :** 
MSIQHFRVALIPFFAAFCLPVFAHPETLVKVKDAEDQLGARVGYIELDLNSGKILESF
RPEERFPMMSTFKVLLCGAVLSRVDAGQEQLGRRIHYSQNDLVEYSPVTEKHLTG
GMTVRELCSAAITMSDNTAANLLLTTIGGPKELTAFLHNMGDHVTRLDRWEPELNE
AIPNDERDTTMPAAMATTLRKLLTGELLTLASRQQLIDWMEADKVAGPLLRSALPAG
WFIADKSGAGERGSRGIIAALGPDGKPSRIVVIYTTGSQATMDERNRQIAEIGASLIK
HW
**SEQ ID N° 86 :** 
MSIQHFRVALIPFFAAFCLPVFAHPETLVKVKDAEDQLGARVGYIELDLNSGKILESF
RPEERFPMMSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDCWEPELNEAI
PNDERDTTTPAAMATTLRKLLTGELLTLASRQQLIDWMEADKVAGPLLRSALPAGW
FIADKSGAGKRGSRGIIAALGPDGKPSRIVVIYTTGSQATMDERNRQIAEIGASLIKH
W
**SEQ ID N° 87 :** 
MSIKHFRVALIPFFAAFCLPVFAHPETLVKVKDAEDQLGARVGYIELDLNSGKILESFR
PEERFPMMSTFKVLLCGAVLSRVDAGQEQLGRRIHYSQNDLVKYSPVTEKHLTDG
MTVRELCSAAITMSDNTAANLLLTTIGGPKELTAFLHNMGDHVTRLDRWEPELNEAI
 
PNDERDTTTPAAMATTLRKLLTGELLTLASRQQLIDWMEADKVAGPLLRSALPAGW
FIADKSGASERGSRGIIAALGPDGKPSRIVVIYTTGSQATMDERNRQIAEIGASLIKHW
**SEQ ID N° 88 :** 
MSIQHFRVALIPFFAAFCLPVFAHPETLVKVKDAEDQLGARVGYIELDLNSGKILESF
RPEERFPMMSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDRWEPELNEAI
PNDERDTTTPAAMATTLRKLLTGELLTLASRQQLIDWMEADKVAGPLLRSALPAGW
FIADKSGASKRGSRGIIAALGPDGKPSRIVVIYTTGSQATMDERNRQIAEIGASLIKHW
**SEQ ID N° 89 :** 
MSIQHFRVALIPFFAAFCFPVFAHPETLVKVKDAEDQLGARVGYIELDLNSGKILESF
RPEERFPMMSTFKVLLCGAVLSRVDAGQEQLGRRIHYSQNDLVKYSPVTEKHLTDG
MTVRELCSAAITMSDNTAANLLLTTIGGPKELTAFLHNMGDHVTRLDRWEPELNEAI
PNDERDTTTPAAMATTLRKLLTGELLTLASRQQLIDWMEADKVAGPLLRSALPAGW
FIADKSGASERGSRGIIAALGPDGKPSRIVVIYMTGSQATMDERNRQIAEIGASLIKH
W
**SEQ ID N° 90 :** 
MSIQHFRVALIPFFAAFCLPVFAHPETLVKVKDAEDQLGARVGYIELDLNSGKILESF
RPEERFPMMSTFKVLLCGAVLSRVDAGQEQLGRRIHYSQNDLVEYSPVTEKHLTDG
MTVRELCSAAITMSDNTAANLLLTTIGGAKELTAFLHNMGDHVTRLDRWEPELNEAI
PNDERDTTMPAAMATTLRKLLTGELLTLASRQQLIDWMEADKVAGPLLRSALPAGW
FIADKSGAGERGSRGIIAALGPDGKPSRIVVIYTTGSQATMDERNRQIAEIGASLIKH
W
**SEQ ID N° 91 :** 
MSIQHFRVALIPFFAAFCLPVFAHPETLVKVKDAEDQLGARVGYIELDLNSGKILESF
RPEERFPMMSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLGRWEPELNEAI
PNDERDTTMPAAMATTLRKLLTGELLTLASRQQLIDWMEADKVAGPLLRSALPAGW
FIADKSGAGERGSRGIIAALGPDGKPSRIVVIYTTGSQATMDERNRQIAEIGASLIKH
W
**SEQ ID N° 92 :** 
MSIQHFRVALIPFFAAFCLPVFAHPETLVKVKDAEDKLGARVGYIELDLNSGKILESFR
PEERFPMMSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDRWEPELNEAI
PNDERDTTMPAAMATTLRKLLTGELLTLASRQQLIDWMEADKVAGPLLRSALPAGW
 FIADKSGASKRGSRGIIAALGPDGKPSRIVVIYTTGSQATMDERNRQIVEIGASLIKHW
**SEQ ID N° 93 :** 
MSIQHFRVALIPFFAAFCFPVFAHPETLVKVKDAEDQLGARVGYIELDLNSGKILESF
RPEERFPMMSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDSWEPELNEAI
PNDERDTTMPAAMATTLRKLLTGELLTLASRQQLIDWMEADKVAGPLLRSALPAGW
FIADKSGAGERGSRGIIAALGPDGKPSRIVVIYMTGSQATMDERNRQIAEIGASLIKH
W
**SEQ ID N° 94 :** 
MSIQHFRVALIPFFAAFCLPVFAHPETLVKVKDAEDQLGARVGYIELDLNSGKILESF
RPEERFPMMSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDRWEPELNEAI
PNDERDTTMPAAMATTLRKLLTGELLTLASRQQLIDWMEADKVAGPLLRSALPAGW
FIADKSGAGERGSRGIIAALGPDGKPSRIVVIYTTGSQATMDELNRQIAEIGASLIKHW
**SEQ ID N° 95 :** 
MSIQHFRVALIPFFAAFCLPVFAHPETLVKVKDAEDQLGARVGYIELDLNSGKILESF
RPEERFPMMSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDRWEPELNEAI
PNDERDTTMPAAMATTLRKLLTGELLTLASRQQLIDWMEADKVAGPLLRSALPAGW
FIADKSGAGERGSRGIIAALGPDGKPSRIVVIYTTGSQATMDERNRQIVEIGASLIKH
W
**SEQ ID N° 96 :** 
MSIQHFRVALIPFFAAFCLPVFAHPETLVKVKDAEDQLGARVGYIELDLNSGKILESF
RPEERFPMMSTFKVLLCGAVLSRVDAGQEQLGRRIHYSQNDLVEYSPVTEKHLTDG
MTVRELCNAAITMSDNTAANLLLTTIGGPKELTAFLHNMGDHVTRLDRWEPELNEAI
PNDERDTTMPAAMATTLRKLLTGELLTLASRQQLIDWMEADKVAGPLLRSALPAGW
FIADKSGAGERGSRGIIAALGPDGKPSRIVVIYTTGSQATMDERNRQIAEIGASLIKH
W
**SEQ ID N° 97 :** 
MSIQHFRVALIPFFAAFCLPVFAHPETLVKVKDAEDQLGARVGYIELDLNSGKILESF
RPEERFPMMSTFKVLLCGAVLSRVDAGQEQLGRRIHYSQNDLVKYSPVTEKHLTDG
MTVRELCSAAITMSDNTAANLLLTTIGGPKELTAFLHNMGDHVTRLDRWEPELNEAI
PNDERDTTTPAAMATTLRKLLTGELLTLASRQQLIDWMEADKVAGPLLRSALPAGW
FIADKSGAGERGSRGIIAALGPDGKPSRIVVIYTTGSQATMDERNRQIAEIGASLIKH
 W
**SEQ ID N° 98 :** 
MSIQHFRVALIPFFAAFCLPVFAHPETLVKVKDAEDQLGARVGYIELDLNSGKILESF
RPEERFPMMSTFKVLLCGAVLSRVDAGQEQLGRRIHYSQNDLVKYSPVTEKHLTDG
MTVRELCSAAITMSDNTAANLLLTTIGGPKELTAFLHNMGDHVTRLDHWEPELNEAI
PNDERDTTTPAAMATTLRKLLTGELLTLASRQQLIDWMEADKVAGPLLRSALPAGW
FIADKSGASERGSRGIIAALGPDGKPSRIVVIYTTGSQATMDERNRQIAEIGASLIKHW
**SEQ ID N° 99 :** 
MSIQHFRVALIPFFAAFCLPVFAHPETLVKVKDAEDQLGARVGYIELDLNSGKILESF
RPEERFPMMSTFKVLLCGAELSRVDAGQEQLGRRIHYSQNDLVEYSPVTEKHLTDG
MTVRELCSAAITMSDNTAANLLLTTIGGPKELTAFLHNMGDHVTRLDRWEPELNEAI
PNDERDTTMPAAMATTLRKLLTSELLTLASRQQLIDWMEADKVAGPLLRSALPAGW
FIADKSGAGERGSRGIIAALGPDGKPSRIVVIYTTGSQATMDERSRQIAEIGASLIKH
W
**SEQ ID N° 100 :** 
MSIQHFRVALIPFFAAFCLPVFAHPETLVKVKDAEDQLGARVGYIELDLNSGKILESF
RPEERFPMLSTFKVLLCGAVLSRVDAGQEQLGRRIHYSQNDLVKYSPVTEKHLTDG
MTVRELCSAAITMSDNTAANLLLTTIGGPKELTAFLHNMGDHVTRLDHWEPELNEAI
PNDERDTTMPAAMATTLRKLLTGELLTLASRQQLIDWMEADKVAGPLLRSALPAGW
FIADKSGAGERGSRGIIAALGPDGKPSRIVVIYTTGSQATMDERNRQIAEIGASLIKH
W
**SEQ ID N° 101 :** 
MSIQHFRVALIPFFAAFCFPVFAHPETLVKVKDAEDQLGARVGYIELDLNSGKILESF
RPEERFPMMSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDRWEPELNEAI
PNDERDTTMPAAMATTLRKLLTGELLTLASRQQLIDWMEADKVAGPLLRSALPAGW
FIADKSGAGERGSRGIIAALGPDGKPSRIVVIYMTGSQATMDERNRQIAEIGASLIKH
W
**SEQ ID N° 102 :** 
MSIQHFRVALIPFFAAFCLPVFAHPETLVKVKDAEDQLGARVGYIELDLNSGKILESF
RPEERFPMMSTFKVLLCGAVLSRVDAGQEQLGRRIHYSQNDLVKYSPVTEKHLTDG
MTVRELCSAAITMSDNTAANLLLTTIGGPKELTAFLHNMGDHVTRLDRWEPELNEAI
PNDERDTTMPAAMATTLRKLLTGELLTLASRQQLIDWMEADKVAGPLLRSALPAGW
FIADKSGADERGSRGIIAALGPDGKPSRIVVIYMTGSQATMDERNRQIAEIGASLIKH
 W
**SEQ ID N° 103 :** 
MSIQHFRVALIPFFAAFCLPVFAHPETLVKVKDAEDQLGARVGYIELDLNSGKILESF
RPEERFPMMSTFKVLLCGAVLSRVDAGQEQLGRRIHYSQNDLVEYSPVTEKHLTDG
MTVRELCSAAITMSDNTAANLLLTTIGGPKELTAFLRNMGDHVTRLDRWEPELNEAI
PNDERDTTMPAAMATTLRKLLTGELLTLASRQQLIDWMEADKVAGPLLRSALPAGW
FIADKSGASERGSRGIIAALGPDGKPSRIVVIYTTGSQATMDERNRQIAEIGASLIKHW
**SEQ ID N° 104 :** 
MSIQHFRVALIPFFAAFCLPVFAHPETLVKVKDAEDKLGARVGYIELDLNSGKILESFR
PEERFPMMSTFKVLLCGAVLSRVDAGQEQLGRRIHYSQNDLVKYSPVTEKHLTDG
MTVRELCSAAITMSDNTAANLLLTTIGGPKELTAFLHNMGDHVTRLDRWEPELNEAI
PNDERDTTTPAAMATTLRKLLTGELLTLASRQQLIDWMEADKVAGPLLRSALPAGW
FIADKSGASERGSRGIIAALGPDGKPSRIVVIYTTGSQATMDERNRQIAEIGASLIKHW
**SEQ ID N° 105 :** 
MSIQHFRVALIPFFAAFCLPVFAHPETLVKVKDAEDKLGARVGYIELDLNSGKILESFR
PEERFPMMSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDSWEPELNEAI
PNDERDTTMPAAMATTLRKLLTGELLTLASRQQLIDWMEADKVAGPLLRSALPAGW
FIADKSGTGKRGSRGIIAALGPDGKPSRIVVIYTTGSQATMDERNRQIAEIGASLIKH
W
**SEQ ID N° 106 :** 
MSIQHFRVALIPFFAAFCFPVFAHPETLVKVKDAEDQLGARVGYIELDLNSGKILESF
RPEERFPMMSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDHWEPELNEAI
PNDERDTTMPAAMATTLRKLLTGELLTLASRQQLIDWMEADKVAGPLLRSALPAGW
FIADKSGAGERGSRGIIAALGPDGKPSRIVVIYTTGSQATMDERNRQIAEIGASLIKH
W
**SEQ ID N° 107 :** 
MSIQHFRVALIPFFAAFCLPVFAHPETLVKVKDAEDQLGARVGYIELDLNSGKILESF
RPEERFPMMSTFKVLLCGAVLSRIDAGQEQLGRRIHYSQNDLVEYSPVTEKHLTDG
MTVRELCSAAITMSDNTAANLLLTTIGGPKELTAFLHNMGDHVTRLDRWEPELNEAI
PNDERDTTMPVAMATTLRKLLTGELLTLASRQQLIDWMEADKVAGPLLRSALPAGW
FIADKSGAGERGSRGIIAALGPDGKPSRIVVIYTTGSQATMDERNRQIAEIGASLIKH
W
**SEQ ID N° 108 :** 
MSIQHFRVALIPFFAAFCFPVFAHPETLVKVKDAEDQLGARVGYIELDLNSGKILESF
RPEERFPMMSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDRWEPELNEAI
PNDERDTTMPAAMATTLRKLLTGELLTLASRQQLIDWMEADKVAGPLLRSALPAGW
FIADKSGAGERGSRGIIAALGPDGKPSRIVVIYTTGSQATMDERNRQIAEIGASLIKH
W
**SEQ ID N° 109 :** 
MSIQHFRVALIPFFAAFCLPVFAHPETLVKVKDAEDQLGARVGYIELDLNSGKILESF
RPEERFPMMSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDHWEPELNEAI
PNDERDTTMPAAMATTLRKLLTGELLTLASRQQLIDWMEADKVAGPLLRSALPAGW
FIADKSGAGERGSRGIIAALGPDGKPSRIVVIYMTGSQATMDERNRQIAEIGASLIKH
W
**SEQ ID N° 110 :** 
MSIQHFRVALIPFFAAFCFPVFAHPETLVKVKDAEDQLGARVGYIELDLNSGKILESF
RPEERFPMMSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDRWEPELNEAI
PNDERDTTMPAAMATTLRKLLTGELLTLASRQQLIDWMEADKVAGPLLRSALPAGW
FIADKSGASERGSRGIIAALGPDGKPSRIVVIYTTGSQATMDERNRQIAEIGASLIKHW
**SEQ ID N° 111 :** 
MSIQHFRVALIPFFAAFCLPVFAHPETLVKVKDAEDKLGARVGYIELDLNSGKILESFR
PEERFPMMSTFKVLLCGAVLSRVDAGQEQLGRRIHYSQNDLVKYSPVTEKHLTDG
MTVRELCSAAITMSDNTAANLLLTTIGGPKELTAFLHNMGDHVTRLDSWEPELNEAI
PNDERDTTMPAAMATTLRKLLTGELLTLASRQQLIDWMEADKVAGPLLRSALPAGW
FIADKSGTGKRGSSGIIAALGPDGKPSRIVVIYTTGSQATMDERNRQIAEIGASLIKHW
**SEQ ID N° 112 :** 
MSIQHFRVALIPFFAAFCLPVFAHPETLVKVKDAEDQLGARVGYIELDLNSGKILESF
RPEERFPMMSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDRWEPELNEAI
PNDERDTTMPAAMATTLRKLLTGELLTLASRQQLIDWMEADKVAGPLLRSALPAGW
FIADKSGAGERGSRGIIAALGPDGKPSRIVVIYTTGSQATMDEQNRQIAEIGASLIKH
W
**SEQ ID N° 113 :** 
MSIKHFRVALIPFFAAFCLPVFAHPETLVKVKDAEDQLGARVGYIELDLNSGKILESFR
PEERFPMMSTFKVLLCGAVLSRVDAGQEQLGRRIHYSQNDLVKYSPVTEKHLTDG
MTVRELCSAAITMSDNTAANLLLTTIGGPKELTAFLHNMGDHVTRLDRWEPELNEAI
PNDERDTTMPAAMATTLRKLLTGELLTLASRQQLIDWMEADKVAGPLLRSALPAGW
FIADKSGASERGSRGIIAALGPDGKPSRIVVIYTTGSQATMDERNRQIAEIGASLIKHW
**SEQ ID N° 114 :** 
MSIKHFRVALIPFFAAFCLPVFAHPETLVKVKDAEDQLGARVGYIELDLNSGKILESFR
PEERFPMMSTFKVLLCGAVLSRVDAGQEQLGRRIHYSQNDLVKYSPVTEKHLTDG
MTVRELCSAAITMSDNTAANLLLTTIGGPKELTAFLHNMGDHVTRLDRWEPELNEAI
PNDERDTTTPAAMATTLRKLLTGELLTLASRQQLIDWMEADKVAGPLLRSALPAGW
FIADKSGAGERGSRGIIAALGPDGKPSRIVVIYTTGSQATMDERNRQIAEIGASLIKH
W
**SEQ ID N° 115 :** 
MSIQHFRVALIPFLAAFCLPVFAHPETLVKVKDAEDQLGARVGYIELDLNSGKILESF
RPEERFPMLSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDSREPELNEAIP
NDERDTTMPAAMATTLRKLLTGELLTLASRQQLIDWMEADKVAGPLLRSALPAGWF
IADKSGAGERGSRGIIAALGPDGKPSRIVVIYTTGSQATMDERDRQIAEIGASLIKHW
**SEQ ID N° 116 :** 
MSIQHFRVALIPFFAAFCLPVFAHPETLVKVKDAEDQLGARVGYIELDLNSGKILESF
RPEERFPMMSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDRWEPELNEAI
PNDERETTTPAAMATTLRKLLTGELLTLASRQQLIDWMEADKVAGPLLRSALPAGW
FIADKSGAGERGSRGIIAALGPDGKPSRIVVIYTTGSQATMDERNRQIAEIGASLIKH
W
**SEQ ID N° 117 :** 
MSIQHFRVALIPFFAAFCLPVFAHPETLVKVKDAEDQLGARVGYIELDLNSGKILESF
RPEERFPMMSTFKVLLCGAVLSRVDAGQEQLGRRIHYSQNDLVEYSPVTEKHLTDG
MTVRELCSAAITMSDNTAANLLLTTIGGPKELTAFLHNMGDNVTRLDRWEPELNEAI
PNDERDTTMPAAMATTLRKLLTGELLTLASRQQLIDWMEADKVAGPLLRSALPAGW
FIADKSGAGERGSRGIIAALGPDGKPSRIVVIYTTGSQATMDERNRQIAEIGASLIKH
W
**SEQ ID N° 118 :** MSIQHFRVALIPFFAAFCLPVFAHPETLVKVKDAEDQLGARVGYIELDLNSGKILESF 
RPEERFPMMSTFKVLLCGAVLSRVDAGQEQLGRRIHYSQNDLVEYSPVTEKHLTDG
MTVRELCSAAITMSDNTAANLLLTTIGGPKELTAFLHNMGEHVTRLDRWEPELNEAI
PNDERDTTMPAAMATTLRKLLTGELLTLASRQQLIDWMEADKVAGPLLRSALPAGW
FIADKSGAGERGSRGIIAALGPDGKPSRIVVIYTTGSQATMDERNRQIAEIGASLIKH
W
**SEQ ID N° 119 :** 
MSIQHFRVALIPFFAAFCLPVFAHPETLVKVKDAEDKLGARVGYIELDLNSGKILESFR
PEERFPMMSTFKVLLCGAVLSRVDAGQEQLGRRIHYSQNDLVKYSPVTEKHLTDG
MTVRELCSAAITMSDNTAANLLLTTIGGPKELTAFLHNMGDHVTRLDSWEPELNEAI
PNDERDTTMPAAMATTLRKLLTGELLTLASRQQLIDWMEADKVAGPLLRSALPAGW
FIADKSGAGERGSRGIIAALGPDGKPSRIVVIYTTGSQATMDERNRQIAEIGASLIKH
W
**SEQ ID N° 120 :** 
MSIQHFRVALIPFFAAFCLPVFAHPETLVKVKPAEDKLGARVGYIELDLNSGKILESFR
PEERFPMMSTFKVLLCGAVLSRVDAGQEQLGRRIHYSQNDLVKYSPVTEKHLTDG
MTVRELCSAAITMSDNTAANLLLTTIGGPKELTAFLHNMGDHVTRLDSWEPELNEAI
PNDERDTTMPAAMATTLRKLLTGELLTLASRQQLIDWMEADKVAGPLLRSALPAGW
FIADKSGTGERGSRGIIAALGPDGKPSRIVVIYTTGSQATMDERNRQIAEIGASLIKH
W
**SEQ ID N° 121 :** 
MSIQHFRVALIPFFAAFCFPVFAHPETLVKVKDAEDQLGARVGYIELDLNSGKILESF
RPEERFPMMSTFKVLLCGAVLSRVDAGQEQLGRRIHYSQNDLVKYSPVTEKHLTDG
MTVRELCSAAITMSDNTAANLLLTTIGGPKELTAFLHNMGDHVTRLDSWEPELNEAI
PNDERDTTTPAAMATTLRKLLTGELLTLASRQQLIDWMEADKVAGPLLRSALPAGW
FIADKSGTGERGSRGIIAALGPDGKPSRIVVIYTTGSQATMDERNRQIAEIGASLIKH
W
**SEQ ID N° 122 :** 
MSIQHFRVALIPFFAAFCLPVFAHPETLVKVKDAEDQLGARVGYIELDLNSGKILESF
RPEERFPMMSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDHWEPELNEAV
PNDERDTTMPAAMATTLRKLLTGELLTLASRQQLIDWMEADKVAGPLLRSALPAGW
FIADKSGAGKRGSRGIIAALGPDGKPSRIVVIYTTGSQATMDERNRQIAEIGASLIKH
W
**SEQ ID N° 123 :** 
MSIQHFRVALIPFFAAFCFPVFAHPETLVKVKDAEDQLGARVGYIELDLNSGKILESF
RPEERFPMMSTFKVLLCGAVLSRVDAGQEQLGRRIHYSQNDLVKYSPVTEKHLTDG
MTVRELCSAAITMSDNTAANLLLTTIGGPKELTAFLHNMGDHVTRLDSWEPELNEAI
PNDERDTTMPAAMATTLRKLLTGELLTLASRQQLIDWMEADKVAGPLLRSALPAGW
FIADKSGAGERGSRGIIAALGPDGKPSRIVVIYTTGSQATMDERNRQIAEIGASLIKH
W
**SEQ ID N° 124 :** 
MSIQHFRVALIPFFAAFCLPVFAHPETLVKVKDAEDKLGARVGYIELDLNSGKILESFR
PEERFPMMSTFKVLLCGAVLSRVDAGQEQLGRRIHYSQNDLVKYSPVTEKHLTDG
MTVRELCSAAITMSDNTAANLLLTTIGGPKELTAFLHNMGDHVTRLDHWEPELNEAI
PNDERDTTMPAAMATTLRKLLTGELLTLASRQQLIDWMEADKVAGPLLRSALPAGW
FIADKSGASERGSRGIIAALGPDGKPSRIVVIYTTGSQATMDERNRQIAEIGASLIKHW
**SEQ ID N° 125 :** 
MSIQHFRVALIPFFAAFCLPVFAHPETLVKVKDAEDQLGARVGYIELDLNSGKILESF
RPEERFPMMSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDRWEPELNEAI
PNDERDTTTPAAMATTLRKLLTGELLTLASRQQLIDWMEADKVAGPLLRSALPAGW
FIADKSGAGERGSRGIIAALGPDGKPSRIVVIYTTGSQATMDERNRQIAEIGASLIKH
W
**SEQ ID N° 126 :** 
MSIQHFRVALIPFFAAFCLPVFAHPETLVKVKDAEDQLGARVGYIELDLNSGKILESF
RPEERFPMMSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDSWEPELNEAI
PNDERDTTMPAAMATTLRKLLTGELLTLASRQQLIDWMEADKVAGPLLRSALPAGW
FIADKSGTGKRGSRGIIAALGPDGKPSRIVVIYTTGGQATMDERNRQIAEIGASLIKH
W
**SEQ ID N° 127 :** 
MSIQHFRVALIPFFAAFCLPVFAHPETLVKVKDAEDQLGARVGYIELDLNSGKILESF
RPEERFPMMSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDSWEPELNEAI
PNDERDTTMPAAMATTLRKLLTGELLTLASRQQLIDWMEADKVAGPLLRSALPAGW
FIADKSGAGRRGSRGIIAALGPDGKPSRIVVIYTTGSQATMDERNRQIAEIGASLIKH
W
**SEQ ID N° 128 :** 
MSIQHFRVALIPFFAAFCLPVFAHPETLVKVKDAEDQLGARVGYIELDLNSGKILESF
RPEERFPMMSTFKVLLCGAVLSRVDAGQEQLGRRIHYSQNDLVKYSPVTEKHLTDG
MTVRELCSAAITMSDNTAANLLLTTIGGPKELTAFLHNMGDHVTRLDRWEPELNEAI
PIDERDTTMPAAMATTLRKLLTGELLTLASRQQLIDWMEADKVAGPLLRSALPAGWF
IADKSGASERGSRGIIAALGPDGKPSRIVVIYTTGSQATMDERNRQIAEIGASLIKHW
**SEQ ID N° 129 :** 
MSIQHFRVALIPFFAAFCLPVFAHPETLVKVKDAEDKLGARVGYIEMDLNSGKILESF
RPEERFPMMSTFKVLLCGAVLSRVDAGQEQLGRRIHYSQNDLVKYSPVTEKHLTDG
MTVRELCSAAITMSDNTAANLLLTTIGGPKELTAFLHNMGDHVTRLDRWEPELNEAI
PNDERDTTMPAAMATTLRKLLTGELLTLASRQQLIDWMEADKVAGPLLRSALPAGW
FIADKSGASERGSRGIIAALGPDGKPSRIVVIYTTGSQATMDERNRQIAEIGASLIKHW
**SEQ ID N° 130 :** 
MSIQHFRVALIPFFAAFCLPVFAHPETLVKVEDAEDQLGARVGYIELDLNSGKILESF
RPEERFPMMSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDRWEPELNEAI
PNDERDTTMPAAMATTLRKLLTGELLTLASRQQLIDWMEADKVAGPLLRSALPAGW
FIADKSGAGERGSRGIIAALGPDGKPSRIVVIYTTGSQATMDERNRQIAEIGASLIKH
W
**SEQ ID N° 131 :** 
MSIQHFRVALIPFFAAFCLPVFAHPETLVKVKDAEDQLGARVGYIELDLNSGKILESF
RPEERFPMMSTFKVLLCGAVLSRVDAGQEQLGRRIHYSQNDLVKYSPVTEKHLTDG
MTVRELCSAAITMSDNTAANLLLTTIGGPKELTAFLHNMGDHVTRLDRWEPELNEAI
PNDERDTTMPAAMATTLRKLLTGELLTLASRQQLIDWMEADKVAGPLLRSALPAGW
FIADKSGANERGSRGIIAALGPDGKPSRIVVIYMTGSQATMDERNRQIAEIGASLIKH
W
**SEQ ID N° 132 :** 
MSIQHFRVALIPFFAAFCLPVFAHPETLVKVKDAEDQLGARVGYIELDLNSGKILESF
RPEERFPMMSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDCWEPELNEAI
PNDERDTTMPAAMATTLRKLLTGELLTLASRQQLIDWMEADKVAGPLLRSALPAGW
FIADKSGAGERGSRGIIAALGPDGKPSRIVVIYTTGSQATMDERNRQIAEIGASLIKH
W
**SEQ ID N° 133 :** MSIQHFRVALIPFFAAFCLPVFAHPETLVKVKDAEDQLGARVGYIELDLNSGKILESF 
RPEERFPMMSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDCWEPELNEAI
PNDERDTTMPAAMATTLRKLLTGELLTLASRQQLIDWMEADKVAGPLLRSALPAGW
FIADKSGAGKRGSRGIIAALGPDGKPSRIVVIYTTGSQATMDERNRQIAEIGASLIKH
W
**SEQ ID N° 134 :** 
MSIQHFRVALIPFFAAFCLPVFAHPETLVKVKDAEDQLGARVGYIELDLNSGKILESF
RPEERFPMMSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDRWEPELNEAI
PNDERDTTMPAAMATTLRKLLTGELLTLASRQQLIDWMEADKVAGPLMRSALPAG
WFIADKSGAGERGSHGIIAALGPDGKPSRIVVIYTTGSQATMDERNRQIAEIGASLIK
HW
**SEQ ID N° 135 :** 
MSIQHFRVALIPFFAAFCLPVFAHPETLVKVKDAEDQLGARVGYIELDLNSGKILESF
RPEERFPMMSTFKVLLCGAVLSRVDAGQEQLGRRIHYSQNDLVEYSPVTEKHLPD
GMTVRELCSAAITMSDNTAANLLLTTIGGPKELTAFLHNMGDHVTRLDRWEPELNE
AIPNDERDTTMPAAMATTLRKLLTGELLTLASRQQLIDWMEADKVAGPLLRSALPAG
WFIADKSGAGERGSHGIIAALGPDGKPSRIVVIYTTGSQATMDERNRQIAEIGASLIK
HW
**SEQ ID N° 136 :** 
MSIQHFRVALIPFFAAFCLPVFAHPETLVKVKDAEDQLGARVGYIELDLNSGKILESF
RPEERFPMMSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDHWEPELNEAI
PNDERDTTMPAAMATTLRKLLTGELLTLASRQQLIDWMEADKVAGPLLRSVLPAGW
FIADKSGAGERGSRGIIAALGPDGKPSRIVVIYTTGSQATMDERNRQIAEIGASLIKH
W
**SEQ ID N° 137 :** 
MSIQHFRVALIPFFAAFCLPVFAHPETLVKVKDAEDQLGARVGYIELDLNSGKILESF
RPEERFPMMSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDRWEPELNEAI
PNDERDTTMPAAMATKLRKLLTGELLTLASRQQLIDWMEADKVAGPLLRSALPAGW
FIADKSGAGERGSRGIIAALGPDGKPSRIVVIYTTGSQATMDERNRQIAEIGASLIKH
W
**SEQ ID N° 138 :** 
MSIQHFRVALIPFFAAFCLPVFAHPETLVKVKDAEDQLGARVGYIELDLNSGKILESF
RPEERFPMMSTFKVLLCGAVLSRVDAGQEQLGRRIHYSQNDLVKYSPVTEKHLTDG
MTVRELCSAAITMSDNTAANLLLTTIGGPKELTAFLHNMGDHVTRLDSWEPELNEAI
PNDERDTTTPAAMATTLRKLLTGELLTLASRQQLIDWMEADKVAGPLLRSALPAGW
FIADKSGAGVRGSRGIIAALGPDGKPSRIVVIYTTGSQATMDERNRQIAEIGASLIKH
W
**SEQ ID N° 139 :** 
MSIQHFRVALIPFFAAFCLPVFAHPDTLVKVKDAEDQLGARVGYIELDLNSGKILESF
RPEERFPMMSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDRWEPELNEAI
PNDERDTTMPAAMATTLRKLLTGELLTLASRQQLIDWMEADKVAGPLLRSALPAGW
FIADKSGAGERGSRGIIAALGPDGKPSRIVVIYTTGSQATMDERNRQIAEIGASLIKH
W
**SEQ ID N° 140 :** 
MSIQHFRVALIPFFAAFCLPVFAHPETLVKVKDAEDQLGARVGYIELDLNSGKILESF
RPEERFPMVSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDHWEPELNEAI
PNDERDTTMPAAMATTLRKLLTGELLTLASRQQLIDWMEADKVAGPLLRSALPAGW
FIADKSGAGERGSRGIIAALGPDGKPSRIVVIYTTGSQATMDERDRQIAEIGGSLIKH
W
**SEQ ID N° 141 :** 
MSIQHFRVALIPFFAAFCLPVFAHPETLVKVKDAEDQLGARVGYIELDLNSGKILESF
RPEERFPMVSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDHWEPELNEAI
PNDERDTTMPAAMATTLRKLLTGELLTLASRQQLIDWMEADKVAGPLLRSALPAGW
FIADKSGAGKRGSRGIIAALGPDGKPSRIVVIYTTGSQATMDERDRQIAEIGASLIKH
W
**SEQ ID N° 142 :** 
MSIQHFRVALIPFFAAFCLPVFAHPETLVKVKDAEDQLGARVGYIELDLNSGKILESF
RPEERFPMLSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDSWEPELNEAI
PNDERDTTMPAAMATTLRKLLTGELLTLASRQQLIDWMEADKVAGPLLRSALPAGW
FIADKSGAGERGSRGIIAALGPDGKPSRIVVIYTTGSQATMDERNRQIAEIGASLIKH
W
**SEQ ID N° 143 :** 
MSIQHFRVALIPFFAAFCLPVFAHPETLVKVKDAEDKLGARVGYIELDLNSGKILESFR
PEERFPMMSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDSWEPELNEAI
PNDERDTTMPAAMATTLRKLLTGELLTLASRQQLIDWMEADKVAGPLLRSALPAGW
FIADKSGAGKRGSRGIIAALGPDGKPSRIVVIYTTGSQATMDERNRQIAEIGASLIKH
W
**SEQ ID N° 144 :** 
MSIQHFRVALIPFFAAFCLPVFAHPETLVKVKDAEDQLGARVGYIELDLNSGKILESF
RPEERFPMMSTFKVLLCGAVLSRVDAGQEQLGRRIHYSQNDLVEYSPVTEKHLTDG
MTVRELCSAAITMSDNTAANLLLTTIGGPKELTAFLHNIGDHVTRLDRWEPELNEAIP
NDERDTTMPAAMATTLRKLLTGELLTLASRQQLIDWMEADKVAGPLLRSALPAGWF
IADKSGAGERGSRGIIAALGPDGKPSRIVVIYTTGSQATMDERNRQIAEIGASLIKHW
**SEQ ID N° 145 :** 
MSIQHFRVALIPFFAAFCLPVFAHPETLVKVKDAEDQLGARVGYIELDLNSGKILESF
RPEERFPMMSTFKVLLCGAVLSRIDAGQEQLGRRIHYSQSDLVEYSPVTEKHLTDG
MTVRELCSAAITMSDNTAANLLLTTIGGPKELTAFLHNMGDHVTRLDRWEPELNEAI
PNDERDTTMPVAMATTLRKLLTGELLTLASRQQLIDWMEADKVAGPLLRSALPAGW
FIADKSGAGERGSRGIIAALGPDGKPSRIVVIYTTGSQATMDERNRQIAEIGASLIKH
W
**SEQ ID N° 146 :** 
MSIQHFRVALIPFFAAFCLPVFAHPETLVKVKDAEDQLGARVGYIELDLNSGKILESF
RPEERFPMLSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDSWEPELNEAI
PNDERDTTMPAAMATTLRKLLTGELLTLASRQQLIDWMEADKVAGPLLRSALPAGW
FIADKSGAGERGSRGIIAALGPDGKPSRIVVIYTTGSQATMDERDRQIAEIGASLIKH
W
**SEQ ID N° 147 :** 
MSIQHFRVALIPFFAAFCFPVFAHPETLVKVKDAEDQLGARVGYIELDLNSGKILESF
RPEERFPMISTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDRWEPELNEAI
PNDERDTTTPAAMATTLRKLLTGELLTLASRQQLIDWMEADKVAGPLLRSALPAGW
FIADKSGAGERGSRGIIAALGPDGKPSRIVVIYTTGSQATMDERNRQIAEIGASLIKH
W
**SEQ ID N° 148 :** 
MSIQHFRVALIPFFAAFCLPVFAHPETLVKVKDAEDKLGARVGYIELDLNSGKILESFR
PEERFPMVSTFKVLLCGAVLSRVDAGQEQLGRRIHYSQNDLVEYSPVTEKHLTDGM
TVRELCSAAITMSDNTAANLLLTTIGGPKELTAFLHNMGDHVTRLDRWEPELNEAIP
NDERDTTMPAAMATTLRKLLTGELLTLASRQQLIDWMEADKVAGPLLRSALPAGWF
IADKSGAGERGSRGIIAALGPDGKPSRIVVIYTTGSQATMDERNRQIAEIGASLIKHW
**SEQ ID N° 149 :** 
MSIKHFRVALIPFFAAFCLPVFAHPETLVKVKDAEDQLGARVGYIELDLNSGKILESFR
PEERFPMMSTFKVLLCGAVLSRVDAGQEQLGRRIHYSQNDLVKYSPVTEKHLTDG
MTVRELCSAAITMSDNTAANLLLTTIGGPKELTAFLHNMGDHVTRLDHWEPELNEAI
PNDERDTTTPAAMATTLRKLLTGELLTLASRQQLIDWMEADKVAGPLLRSALPAGW
FIADKSGAGERGSRGIIAALGPDGKPSRIVVIYTTGSQATMDERNRQIAEIGASLIKH
W
**SEQ ID N° 150 :** 
MSIQHFRVALIPFFAAFCLPVFAHPKTLVKVKDAENQLGARVGYIELDLNSGKILESF
RPEKRFPMMSTFKVLLCGAVLSRIDAGQEQLGRRIHYSQSDVVEYSPVTEKHLTDG
MTVRELCSAAITMSDNTAANLLLTTIGGPKELTAFLHNMGDHVTRLDRWEPELNEAI
PNDERDTTMPVAMATTLRKLLTGELLTLASRQQLIDWMEADKVAGPLLRSALPAGW
FIADKSGAGERGSRGIIAALGPDGKPSRIVVIYTTGSQATMDERNRQIAEIGASLIKH
W
**SEQ ID N° 151 :** 
MSIQHFRVALIPFFAAFCLPVFAHPETLVKVKDAEDQLGARVGYIELDLNSGKILESF
RPEERFPMMSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDRWEPELNEAI
PNDERDTTMPAAMATTLRKLLTGELLTLASRQQLIDWMEADKVAGPLLRSALPAGW
FIADKSGAGERGSRGIIAALGPDGKPSRIVVIYTTGSQATMDEQNRQIAEIGASLIKLW
**SEQ ID N° 152 :** 
MSIQHFRVALIPFFAAFCLPVFAHPETLVKVKDAEDQVGARVGYIELDLNSGKILESF
RPEERFPMMSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDRWEPELNEAI
PNDERDTTMPAAMATTLRKLLTGELLTLASRQQLIDWMEADKVAGPLLRSALPAGW
FIADKSGAGERGSRGIIAALGPDGKPSRIVVIYTTGSQATMDERNRQTAEIGASLIKH
W
**SEQ ID N° 153 :** 
MSIQHFRVALIPFFAAFCLPVFAHPETLVKVKDAEDQLGARVGYIELDLNSGKILESF
RPEERFPMMSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDSWEPELNEAI
PNDERDTTTPAAMATTLRKLLTGELLTLASRQQLIDWMEADKVAGPLLRSALPAGW
FIADKSGAGERGSRGIIAALGPDGKPSRIVVIYTTGSQATMDERNRQIAEIGASLIKH
W
**SEQ ID N° 154 :** 
MSIQHFRVALIPFFAAFCLPVFAHPETLVKVKDAEDQLGARVGYIELDLNSGKILESF
RPEERFPMMSTFKVLLCGAVLSRVDAGQEQLGRRIHYSQNDLVEYSPVTEKHLTDG
MTVGELCSAAITMSDNTAANLLLTTIGGPKELTAFLHNMGDHVTRLDRWEPELNEAI
PNDERDTTMPAAMATTLRKLLTGELLTLASRQQLIDWMEADKVAGPLLRSALPAGW
FIADKSGAGERGSRGIIAALGPDGKPSRIVVIYTTGSQATMDERNRQIAEIGASLIKH
W
**SEQ ID N° 155 :** 
MSIQHFRVALIPFFAAFCFPVFAHPETLVKVKDAEDQLGARVGYIELDLNSGKILESF
RPEERFPMMSTFKVLLCGAVLSRVDAGQEQLGRRIHYSQNDLVKYSPVTEKHLTDG
MTVRELCSAAITMSDNTAANLLLTTIGGPKELTAFLHNMGDHVTRLDRWEPELNEAI
PNDERDTTMPAAMATTLRKLLTGELLTLASRQQLIDWMEADKVAGPLLRSVLPAGW
FIADKSGASERGSRGIIAALGPDGKPSRIVVIYMTGSQATMDERNRQIAEIGASLIKH
W
**SEQ ID N° 156 :** 
MSIQHFRVALIPFFAAFCLPVFAHPETLVKVKDAEDQLGARVGYIELDLNSGKILESF
RPEERFPMMSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDRWEPELNEAI
PNDERDTTMPAAMATTLRKLLTGELLTLASRQQLIDWMEADKVAGPLLRSALPAGW
FIADKSGAGERGSRGIIAALGPDGKPSRIVVIYMTGSQATMDERNRQIAEIGASLIKH
W
**SEQ ID N° 157 :** 
MSIQHFRVALIPFFAAFCLPVFAHPETLVKVKDAEDQLGARVGYIELDLNSGKILESF
RPEERFPMLSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDRGEPELNEAI
PNDERDTTMPAAMATTLRKLLTGELLTLASRQQLIDWMEADKVAGPLLRSALPAGW
FIADKSGAGERGSRGIIAALGPDGKPSRIVVIYTTGSQATMDERNRQIAEIGASLIKH
W
**SEQ ID N° 158 :** 
MSIQHFRVALIPFFAAFCLPVFAHPETLVKVKDAEDQLGARVGYIELDLNSGKILESF
RPEERFPMMSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDRWEPELNEAI
PNDERDTTMPAAMATTLRKLLTGELLTLASRQQLIDWMEADKVAGPLLRSALPAGW
FIADKSGAGERGSRGIIAALGPDGKPSRIVVIYTTGSQATMDERNRQIAEICASLIKH
W
**SEQ ID N° 159 :** 
MSIQHFRVALIPFFAAFCLPVFAHPETLVKVKDAEDQLGARVGYIELDLNSGKILESF
RPEERFPMMSTFKVLLCGAVLSRIDAGQEQLGRRIHYSQNDLVEYSPVTEKHLTDG
MTVRELCSAAITMSDNTAANLLLTTIGGPKELTAFLHNMGDHVTRLDRWEPELNEAI
PNDERDTTMPAAMATTLRKLLTGELLTLASRQQLIDWMEADKVAGPLLRSALPAGW
FIADKSGAGERGSRGIIAALGPDGKPSRIVVIYTTGSQATMDERNRQIAEIGASLIKH
W
**SEQ ID N°160** : 
MSIQHFRVALIPFFAAFCLPVFAHPETLVKVKDAEDQLGARVGYIELDLNSGKILESF
RPEERFPMMSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDRWEPELNEAI
PNDERDTTMPAAMATTLRKLLTGELLTLASRQQLIDWMEVDKVAGPLLRSALPAGW
FIADKSGAGERGSSGIIAALGPDGKPSRIVIIYTTGSQATMDERNRQIAEIGASLIKHW
**SEQ ID N°161** : 
MSIQHFRVALIPFFAAFCLPVFAHPETLVKVKDAEDQLGARVGYIELDLNSGKILESF
RPEERFPMMSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDRWEPELNEAI
PNDERDTTMPAAMATTLRKLLTGELLTLASRQQLIDWMEVDKVAGPLLRSALPAGW
FIADKSGAGERGSSGIIAALGPDGKPSRIVVIYTTGSQATMDERKRQIAEIGASLIKHW
**SEQ ID N°162** : 
MSIQHFRVALIPFFAAFCLPVFAHPETLVKVKDAEDQLGARVGYIELDLNSGKILESF
RPEERFPMMSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDRWEPELNEAI
PNDERDTTMPAAMATTLRKLLTGELLTLASRQQLIDWMEVDKVAGPLLRSALPAGW
FIADKSGAGERGSRGIIAALGPDGKPSRIVVIYTTGSQATMDERNRQIAEIGASLIKH
W
**SEQ ID N°163** : 
MSIQHFRVALIPFFAAFCLPVFAHPETLVKVKDAEDQLGARVGYIELDLNSGKILESF
RPEERFPMMSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDRWEPELNEAI
PNDERDTTMPAAMATTLRKLLTGELLTLASRQQLIDWMEADKVAGPLLRSVLPAGW
FIADKSGAGERGSRGIIAALGPDGKPSRIVVIYTTGSQATMDERNRQIAEIGASLIKH
W
**SEQ ID N°164** : 
MSIKHFRVALIPFFAAFCLPVFAHPETLVKVKDAEDKLGARVGYIELDLNSGKILESFR
PEERFPMMSTFKVLLCGAVLSRVDAGQEQLGRRIHYSQNDLVKYSPVTEKHLTDG
MTVRELCSAAITMSDNTAANLLLTTIGGPKELTAFLHNMGDHVTRLDSWEPELNEAI
PNDERDTTTPAAMATTLRKLLTGELLTLASRQQLIDWMEADKVAGPLLRSALPAGW
FIADKSGTGKRGSRGIIAALGPDGKPSRIVVIYTTGSQATMDERNRQIAEIGASLIKH
W
**SEQ ID N°165** : 
MSIQHFRVALIPFFAAFCLPVFAHPETLVKVKDAEDQLGSTSGYIELDLNSGKILESFR
PEERFPMMSTFKVLLCGAVLSRVDAGQEQLGRRIHYSQNDLVEYSPVTEKHLTDG
MTVRELCSAAITMSDNTAANLLLTTIGGSQELTAFLHNMGDHVTRLDRWEPELNEAI
PNDEADTTMPAAMATTLRKLLTGELLTLASRQQLIDWMADKVAGPLLRSALPAGWFI
ADKSGARERGSRGIIAALGPDGKPSRIVVIYTTGSQATMDERNRQIAEIGASLIKHW
**SEQ ID N°1836** :
**SEQ ID N°1837** : 
MSIQHFRVALIPFFAAFCLPVFAHPETLVKVKDAEDQLGARVGYIELDLNSGKILESF
RPEERFPMLSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDRWEPELNEAI
PNDERDTTMPAAMATTLRKLLTGELLTLASRQQLIDWMEADKVAGPLLRSALPAGW
FIADKSGAGERGSRGIIAALGPDGKPSRIVVIYTTGSQATMDERNRQIAEIGASLIKH
W
**SEQ ID N°1838** : 
MSIQHFRVALIPFFAAFCLPVFAHPETLVKVKDAEDKLGARVGYIELDLNSGKILESFR
PEERFPMMSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDRWEPELNEAI
PNDERDTTMPAAMATTLRKLLTGELLTLASRQQLIDWMEADKVAGPLLRSALPAGW
FIADKSGAGERGSRGIIAALGPDGKPSRIVVIYTTGSQATMDERNRQIAEIGASLIKH
W
**SEQ ID N°1839** : 
MSIQHFRVALIPFFAAFCLPVFAHPETLVKVKDAEDQLGARVGYIELDLNSGKILESF
RPEERFPMMSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDRWEPELNEAI
PNDERDTTMPAAMATTLRKLLTGELLTLASRQQLIDWMEADKVAGPLLRSALPAGW
FIADKSGASKRGSRGIIAALGPDGKPSRIVVIYMTGSQATMDERNRQIAEIGASLIKH
W
**SEQ ID N°1840** : 
MSIQHFRVALIPFFAAFCFPVFAHPETLVKVKDAEDQLGARVGYIELDLNSGKILESF
RPEERFPMMSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDRWEPELNEAI
PNDERDTTMPAAMATTLRKLLTGELLTLASRQQLIDWMEADKVAGPLLRSALPAGW
FIADKSGASKRGSRGIIAALGPDGKPSRIVVIYMTGSQATMDERNRQIAEIGASLIKH
W
**SEQ ID N°1841** : 
MSIQHFRVALIPFFAAFCFPVFAHPETLVKVKDAEDQLGARVGYIELDLNSGKILESF
RPEERFPMMSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDRWEPELNEAI
PNDERDTTMPAAMATTLRKLLTGELLTLASRQQLIDWMEADKVAGPLLRSALPAGW
FIADKSGASKRGSRGIIAALGPDGKPSRIVVIYMTGGQATMDERNRQIAEIGASLIKH
W
**SEQ ID N°1842** : 
MSIQHFRVALIPFFAAFCLPVFAHPETLVKVKDAEDQLGARVGYIELDLNSGKILESF
RPEERFPMMSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDHWEPELNEAI
PNDERDTTMPAAMATTLRKLLTGELLTLASRQQLIDWMEADKVAGPLLRSALPAGW
FIADKSGAGERGSRGIIAALGPDGKPSRIVVIYTTGSQATMDERNRQIAEIGASLIKH
W
**SEQ ID N°1843** : 
MSIQHFRVALIPFFAAFCLPVFAHPETLVKVKDAEDQLGARVGYIELDLNSGKILESF
RPEERFPMMSTFKVLLCGAVLSRVDAGQEQLGRRIHYSQNDLVEYSPVTEKHLTDG
MTVRELCSAAITMSDNTAANLLLTTIGGPKELTAFLHNMGDHVTRLDRWEPELNEAI
PNDERDTTMPAAMATTLRKLLTGELLTLASRQQLIDWMEADKVAGPLLRSALPAGW
FIADKSGAGERGSSGIIAALGPDGKPSRIVVIYTTGSQATMDERNRQIAEIGASLIKH
W
 lesdits peptides étant choisis, de préférence, parmi les peptides de séquence **SEQ ID N°166** à **SEQ ID 261 et SEQ ID N°1923** à **SEQ ID 1928** tels que définis ci-après :

| Peptide SEQ ID N° | Séquence d'acides aminés | Position du peptide dans la ou les protéines TEM | Intérêt clinique |
|---|---|---|---|
| SEQ ID N°166 | CEPELNEAIPNDER | 163-176 pour la protéine de SEQ N°78 | 2br |
| SEQ ID N°167 | DAEDQLGAR | 33-41 pour les protéines de SEQ N°1, 4, 5, 6, 9, 10, 12, 14, 16, 18, 19, 23, 24, 25, 26, 27, 28, 29, 30, 31, 32, 33, 34, 35, 36, 37, 38, 39, 41, 43, 45, 46, 47, 48, 49, 50, 51, 52, 53, 55, 56, 60, 64, 65, 66, 68, 69, 70, 71, 72, 73, 74, 75, 76, 77, 78, 79, 80, 81, 82, 83, 85, 86, 87, 88, 89, 90, 91, 93, 94, 95, 96, 97, 98, 99, 100, 101, 102, 103, 106, 107, 108, 109, 110, 112, 113, 114, 115, 116, 117, 118, 121, 122, 123, 125, 126, 127, 128, 130, 131, 132, 133, 134, 135, 136, 137, 138, 139, 140, 141, 142, 144, 145, 146, 147, 149, 151, 153, 154, 155, 156, 157, 158, 159, 160, 161, 162, 163, 1837, 1838, 1840, 1841, 1842, 1843, 1844 | TEM |
| SEQ ID N°168 | DAEDQLGSTSGYIELDLNSGK | 33-53 pour la protéine de SEQ N°165 | 2be |
| SEQ ID N°169 | DAEDQVGAR | 33-41 pour la protéine de SEQ N°152 | 2be |
| SEQ ID N°170 | DAENQLGAR | 33-41 pour la protéine de SEQ N°150 | TEM |
| SEQ ID N°171 | DTTMPAAMATK | 177-187 pour la protéine de SEQ N°137 | TEM |
| SEQ ID N°172 | DTTMPAAMATTLR | 177-189 pour les protéines de SEQ N°1, 2, 3, 4, 5, 6, 7, 8, 9, 10, 11, 12, 13, 14, 15, 16, 17, 18, 20, 21, 22, 23, 24, 25, 26, 27, 28, 29, 31, 32, 33, 34, 35, 36, 37, 38, 39, 40, 42, 43, 44, 45, 46, 47, 48, 49, 51, 52, 53, 54, 55, 56, 57, 58, 59, 61, 62, 63, 64, 65, 66, 68, 69, 70, 71, 72, 73, 74, 75, 76, 77, 78, 79, 80, 81, 84, 85, 90, 91, 92, 93, 94, 95, 96, 99, 100, 101, 102, 103, 105, 106, 108, 109, 110, 111, 112, 113, 115, 117, 118, 119, 120, 122, 123, 124, 126, 127, 128, 129, 130, 131, 132, 133, 134, 135, 136, 139, 140, 141, 142, 143, 144, 146, 148, 151, 152, 154, 155, 156, 157, 158, 159, 160, 161, 162, 163, 165, 1837, 1838, 1839, 1840, 1841, 1842, 1843, 1844 | TEM |
| SEQ ID N°173 | DTTMPVAMATTLR | 177-189 pour les protéines de SEQ N°107, 145, 150 | TEM |
| SEQ ID N°174 | DTTTPAAMATTLR | 177-189 pour les protéines de SEQ N°19, 30, 41, 50, 60, 67, 82, 83, 86, 87, 88, 89, 97, 98, 104, 114, 121, 125, 138, 147, 149, 153, 164 | TEM |
| SEQ ID N°175 | ELTAFLHNIGDHVTR | 145-159 pour la protéine de SEQ N°144 | TEM |
| SEQ ID N°176 | ELTAFLHNMGDHVTR | 145-159 pour les protéines de SEQ N°1, 2, 3, 4, 5, 6, 7, 8, 9, 10, 11, 12, 13, 14, 15, 16, 17, 18, 19, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 104, 105, 106, 107, 108, 109, 110, 111, 112, 113, 114, 115, 116, 119, 120,121, 122, 123; 124, 125, 126, 127, 128, 129, 130, 131, 132, 133, 134, 135, 136, 137, 138, 139, 140, 141, 142, 143, 145,146, 147, 148, 149, 150, 151, 152, 153, 154, 155, 156, 157, 158, 159, 160, 161, 162, 163, 164, 165, 1837, 1838, 1839, 1840, 1841, 1842, 1843, 1844 | TEM |
| SEQ ID N°177 | ELTAFLHNMGDNVTR | 145-159 pour la protéine de SEQ N°117 | 2b |
| SEQ ID N°178 | ELTAFLHNMGEHVTR | 145-159 pour la protéine de SEQ N°118 | 2b |
| SEQ ID N°179 | ELTAFLR | 145-151 pour les protéines de SEQ N°20, 54, 103 | 2be |
| SEQ ID N°180 | EPELNEAIPNDER | 164-176 pour les protéines de SEQ N°1, 2, 3, 4, 5, 6, 7; 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 8,69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 123, 124, 125, 126, 127, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 1837, 1838, 1839, 1840, 1841, 1842, 1843, 1844 | TEM |
| SEQ ID N°181 | ETTTPAAMATTLR | 177-189 pour la protéine de SEQ N°116 | 2be |
| SEQ ID N°182 | FPMISTFK | 64-71 pour les protéines de SEQ N°30, 35, 38, 147 | 2br |
| SEQ ID N°183 | FPMLSTFK | 64-71 pour les protéines de SEQ N°31, 33, 37, 43, 48, 72, 76, 78, 100, 115, 142, 146, 157, 1838 | TEM |
| SEQ ID N°184 | FPMMSTFK | 64-71 pour les protéines de SEQ N°1, 2, 3, 4, 5, 6, 7, 8, 9, 10, 11, 12, 13, 14, 15, 16, 17, 18, 19, 20, 21, 22, 23, 24, 25, 26, 27, 28, 29, 39, 40, 41, 42, 44, 45, 46, 47, 49, 50, 51, 52, 53, 54, 55, 56, 57, 58, 59, 60, 69, 62, 63, 64, 65, 66, 67, 68, 69, 70, 71, 74, 79, 80, 81, 82, 83, 84, 85, 86, 87, 88, 89, 90, 91, 92, 93, 94, 95, 96, 97, 98, 99, 101, 102, 103, 104, 105, 106, 107, 108, 109, 110, 111, 112, 113, 114, 116, 117, 118, 119, 120, 121, 122, 123, 124, 125, 126, 127, 128, 129, 130, 131, 132, 133, 134, 135, 136, 137, 138, 139, 143, 144, 145, 149, 150, 151, 152, 153, 154, 155, 156, 158, 159, 160, 161, 162, 163, 164, 165, 1837, 1839, 1840, 1841, 1842, 1843,1844 | TEM |
| SEQ ID N°185 | FPMVSTFK | 64-71 pour les protéines de SEQ N°32, 34, 36, 73, 77, 140, 141, 148 | TEM |
| SEQ ID N°186 | GEPELNEAIPNDER | 163-176 pour la protéine de SEQ N°157 | 2be |
| SEQ ID N°187 | GIIAALGPDGKPSR | 242-255 pour les protéines de SEQ N°1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 58, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124,125, 126, 127, 128, 929, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 1837, 1838, 1839, 1840, 1841, 1842, 1843, 1844 ; 241-254 pour la protéine de séquence SEQ ID N°165 | TEM |
| SEQ ID N°188 | GSCGIIAALGPDGKPSR | 239-255 pour les protéines de SEQ N°29, 61, 63, 68 | 2br |
| SEQ ID N°189 | GSGGIIAALGPDGKPSR | 239-255 pour la protéine de SEQ N°74 | 2br |
| SEQ ID N°190 | GSHGIIAALGPDGKPSR | 239-255 pour les protéines de SEQ N°49, 134, 135 | 2br |
| SEQ ID N°191 | GSLGIIAALGPDGKPSR | 239-255 pour la protéine de SEQ N°52 | 2br |
| SEQ ID N°192 | GSSGIIAALGPDGKPSR | 239-255 pour les protéines de SEQ N°28, 42, 56, 69, 72, 111, 160, 161, 1844 | TEM |
| SEQ ID N°193 | GSTGIIAALGPDGKPSR | 239-255 pour la protéine de SEQ N°39 | TEM |
| SEQ ID N°194 | HLPDGMTVR | 110-118 pour la protéine de SEQ N°135 | TEM |
| SEQ ID N°195 | HLTDGMTVR | 110-118 pour les protéines de SEQ N°1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 136, 137, 138, 139, 140, 141, 142, 143, 144, 145, 146, 147, 148, 149, 150, 151, 152, 153, 155, 156, 157, 158, 159, 160, 161, 162, 163, 164, 165, 1837, 1838, 1839, 1840, 1841, 1842, 1843, 1844 | TEM |
| SEQ ID N°196 | HLTGGMTVR | 110-118 pour la protéine de SEQ N°85 | 2b |
| SEQ ID N°197 | IDAGQEQLGR | 82-91 pour les protéines de SEQ N°107, 145, 150, 159 | TEM |
| SEQ ID N°198 | IHYSQNDLVEYSPVTEK | 93-109 pour les protéines de SEQ N°1, 2, 5, 7, 10, 11, 12, 13, 18, 19, 23, 25, 26, 27, 28, 29, 30, 31, 32, 33, 34, 35, 36, 37, 38, 39, 40, 42, 43, 45, 46, 47, 49, 51, 52, 53, 55, 56, 57, 59, 61, 63, 64, 65, 66, 67, 68, 69, 70, 71, 72, 73, 74, 75, 76, 77, 78, 79, 80, 81, 85, 86, 88, 90, 91, 92, 93, 94, 95, 96, 99, 101, 103, 105, 106, 107, 108, 109, 110, 112, 115, 116, 117, 118, 122, 125, 126, 127, 130, 132, 133, 134, 135, 136, 137, 139, 140, 141, 142, 143, 144, 146, 147, 148, 151, 152, 153, 154, 156, 157, 158, 159, 160, 161, 162, 163, 165, 1837, 1838, 1839, 1840, 1841, 1842, 1843, 1844 | TEM |
| SEQ ID N°199 | IHYSQNDLVK | 93-102 pour les protéines de SEQ N°3, 4, 6, 8, 9, 14, 15, 16, 17, 20, 21, 22, 24, 41, 44, 48, 50, 54, 58, 60, 62, 82, 83, 84, 87, 89, 97, 98, 100, 102, 104, 111, 113, 114, 119, 120, 121, 123, 124, 128, 129, 131, 138, 149, 155, 164 | 2be |
| SEQ ID N°200 | IHYSQSDLVEYSPVTEK | 93-109 pour la protéine de SEQ N°145 | 2be |
| SEQ ID N°201 | IHYSQSDWEYSPVTEK | 93-109 pour la protéine de SEQ N°150 | TEM |
| SEQ ID N°202 | ILESFRPEER | 54-63 pour les protéines de SEQ N°1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 3, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 151, 152, 153, 154, 155, 156, 157, 158, 159, 160, 161, 162, 163, 164, 165, 1837, 1838, 1839, 1840, 1841, 1842, 1843, 1844 | TEM |
| SEQ ID N°203 | ILESFRPEK | 54-62 pour la protéine de SEQ N°150 | TEM |
| SEQ ID N°204 | IVIIYTTGSQATMDER | 256-271 pour les protéines de SEQ N°56, 160 | 2br |
| SEQ ID N°205 | IVVIYMTGGQATMDER | 256-271 pour les protéines de SEQ N°47, 1842 | 2be |
| SEQ ID N°206 | IVVIYMTGSQATMDELNR | 256-273 pour la protéine de SEQ N°64 | 2be |
| SEQ ID N°207 | IVVIYMTGSQATMDER | 256-271 pour les protéines de SEQ N°4, 9, 13, 23, 25, 40, 45, 46, 68, 69, 70, 80, 81, 89, 93, 101, 102, 109, 131, 155, 156, 1840,1841 | TEM |
| SEQ ID N°208 | IVVIYTTGGQATMDER | 256-271 pour la protéine de SEQ N°126 | 2be |
| SEQ ID N°209 | IVVIYTTGSQATMDELNR | 256-273 pour les protéines de SEQ N°36, 94 | 2br |
| SEQ ID N°210 | IVVIYTTGSQATMDEQNR | 256-273 pour les protéines de SEQ N°43, 77, 78, 112, 151 | 2br |
| SEQ ID N°211 | IVVIYTTGSQATMDER | 256-271 pour les protéines de SEQ N°1, 2, 3, 5, 6, 7, 8, 10, 11, 12, 14, 15, 16, 17, 18, 19, 20, 21, 22, 24, 26, 27, 28, 29, 30, 31, 32, 33, 34, 35, 37, 38, 39, 41, 42, 44, 48, 49, 50, 51, 52, 53, 54, 55, 57, 58, 59, 60, 61, 62, 63, 65, 66, 67, 71, 72, 73, 74, 75, 76, 79, 82, 83, 84, 85, 86, 87, 88, 90, 91, 92, 95, 96, 97, 98, 99, 100, 103, 104, 105, 106, 107, 108, 110, 111, 113, 114, 115, 116, 117, 118, 119, 120, 121, 122, 123, 124, 125, 127, 128, 129, 130, 132, 133, 134, 135, 136, 137, 138, 139, 140, 141, 142, 143, 144, 145, 146, 147, 148, 149, 150, 152, 153, 154, 157, 158, 159, 161, 162, 163, 164, 1837, 1838, 1839, 1843,1844 | TEM |
| SEQ ID N°212 | LDCWEPELNEAIPNDER | 160-176 pour les protéines de SEQ N°86, 132, 133 | 2be |
| SEQ ID N°213 | LDHWEPELNEAIPNDER | 160-176 pour les protéines de SEQ N°6, 11, 15, 25, 26, 27, 41, 59, 70, 98, 100, 106, 109, 124, 136, 140, 141, 149, 1843 | 2be |
| SEQ ID N°214 | LDHWEPELNEAVPNDER | 160-176 pour la protéine de SEQ N°122 | 2be |
| SEQ ID N°215 | LDSWEPELNEAIPNDER | 160-176 pour les protéines de SEQ N°5, 7, 8, 9, 10, 12, 22, 24, 44, 51, 58, 60, 80, 81, 93, 105, 111, 119, 120, 121, 123, 126, 127, 138, 142, 143, 146, 153, 164 | 2be |
| SEQ ID N°216 | LHCWEPELNEAIPNDER | 160-176 pour la protéine de SEQ N°82 | 2be |
| SEQ ID N°217 | LLTDELLTLASR | 191-202 pour la protéine de SEQ N°83 | 2be |
| SEQ ID N°218 | LLTGELLTLASQQQLIDWMEADK | 191-213 pour la protéine de SEQ N°65 | TEM |
| SEQ ID N°219 | LLTGELLTLASR | 191-202 pour les protéines de SEQ N°1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 3, 64, 66, 67, 68, 69, 70, 71, 72, 73, 74, 75, 76, 77, 78, 79, 80, 81, 82, 84, 85, 86, 87, 88, 89, 90, 91, 92, 93, 94, 95, 96, 97, 98, 100, 101, 102, 103, 104, 105, 106, 107, 108, 149, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141,142, 143, 144, 145, 146, 147, 148, 149, 150, 151, 152, 153, 154, 155, 156, 157, 158, 159, 160, 161, 162, 163, 164, 165, 1837, 1838, 1839, 1840, 1841, 1842, 1843, 1844 | TEM |
| SEQ ID N°220 | LLTSELLTLASR | 191-202 pour la protéine de SEQ N°99 | TEM |
| SEQ ID N°221 | MSIQHFR | 1-7 pour les protéines de SEQ N°1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 83, 84, 85, 86, 88, 89, 90, 91, 92, 93, 94, 95, 96, 97, 98, 99, 100, 101, 102, 103, 104, 105, 106, 107, 108, 109, 110, 111, 112, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 150, 151, 152, 153, 154, 155, 156, 157, 158, 159, 160, 161, 162, 163, 165, 1837, 1838, 1839, 1840, 1841, 1842, 1843, 1844 | TEM |
| SEQ ID N°222 | NMGDHVTR | 152-159 pour les protéines de SEQ N°1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 119, 120, 121, 122, 123, 124, 125, 126, 127, 128, 129, 130, 131, 132, 133, 134, 135, 136, 137, 138, 139, 140, 141, 142, 143, 145, 146, 147, 148, 149, 150, 151, 152, 153, 154, 155, 156, 157, 158, 159, 160, 161, 162, 163, 164, 165, 1837, 1838,1839, 1840, 1841, 1842, 1843, 1844 | 2be |
| SEQ ID N°223 | QIAEICASLIK | 274-284 pour la protéine de SEQ N°158 | TEM |
| SEQ ID N°224 | QIAEIGASLIK | 274-284 pour les protéines de SEQ N°1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 93, 94, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 141, 142, 143, 144, 145, 146, 147, 148, 149, 150, 151, 153, 154, 155, 156, 157, 159, 160, 161, 162, 163, 164, 1837, 1838, 1839, 1840, 1841, 1842, 1843, 1844; 273-283 pour la protéine de séquence SEQ ID N°165 | TEM |
| SEQ ID N°225 | QIAEIGGSLIK | 274-284 pour la protéine de SEQ N°140 | 2be |
| SEQ ID N°226 | QIVEIGASLIK | 274-284 pour les protéines de SEQ N°92, 95 | 2be |
| SEQ ID N°227 | QQLIDWMADK | 203-212 pour la protéine de SEQ N°165 | 2be |
| SEQ ID N°228 | QQLIDWMEADK | 203-213 pour les protéines de SEQ N°1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 2, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151,152,153,154,155,156,157,158, 159, 163, 164, 1837, 1838, 1839, 1840, 1841, 1842, 1843, 1844 | TEM |
| SEQ ID N°229 | QQLIDWMEVDK | 203-213 pour les protéines de SEQ N°160, 161, 162 | TEM |
| SEQ ID N°230 | QTAEIGASLIK | 274-284 pour la protéine de SEQ N°152 | 2be |
| SEQ ID N°231 | SALPAGWFIADK | 221-232 pour les protéines de SEQ N°1, 2, 3, 4, 5, 6, 7, 8, 9, 10, 11, 12, ,13, 14, 15, 16, 17, 18, 19, 20, 21, 22, 23, 24 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 137, 138, 139, 140, 141, 142, 143, 144,145,146,147,148,149,150,151, 152, 153, 154, 156, 157, 158, 159, 160, 161, 162, 164, 1837, 1838, 1839, 1840, 1841, 1842, 1843, 1844; 220-231 pour la protéine de séquence SEQ ID N°165 | TEM |
| SEQ ID N°232 | SGADER | 233-238 pour la protéine de SEQ N°102 | TEM |
| SEQ ID N°233 | SGAGER | 233-238 pour les protéines de SEQ N°1, 2, 6, 7, 9, 11, 12, 13, 15, 16, 17, 24, 27, 28, 29, 30, 31, 32, 33, 34, 35, 36, 37, 38, 39, 41, 42, 43, 49, 51, 52, 53, 54, 55, 56, 57, 58, 60, 61, 63, 65, 68, 69, 70, 71, 72, 73, 74, 75, 76, 77, 78, 79, 82, 85, 90, 91, 93, 94, 95, 96, 97, 99, 100, 101, 106, 107, 108, 109, 112, 114, 115, 116, 117, 118, 119, 123, 125, 130, 132, 134, 135, 136, 137, 139, 140, 142, 144, 145, 146, 147, 148, 149, 150, 151, 152, 153, 154, 156, 157, 158, 159, 160, 161, 162, 163, 1837, 1838, 1839, 1843, 1844 | TEM |
| SEQ ID N°234 | SGAGVR | 233-238 pour la protéine de SEQ N°138 | 2be |
| SEQ ID N°235 | SGANER | 233-238 pour la protéine de SEQ N°131 | TEM |
| SEQ ID N°236 | SGASER | 233-238 pour les protéines de SEQ N°3, 4, 8, 14, 18, 19, 20, 23, 48, 50, 62, 83, 84, 87, 89, 98, 103, 104, 110, 113, 124, 128, 129, 155 | 2be |
| SEQ ID N°237 | SGASK | 233-237 pour les protéines de SEQ N°40, 45, 46, 47, 64, 66, 67, 88, 92, 1840, 1841,1842 | 2be |
| SEQ ID N°238 | SGGSER | 233-238 pour la protéine de SEQ N°21 | 2be |
| SEQ ID N°239 | SGTGER | 233-238 pour les protéines de SEQ N°120, 121 | 2be |
| SEQ ID N°240 | SVLPAGWFIADK | 221-232 pour les protéines de SEQ N°136, 155, 163 | 2be |
| SEQ ID N°241 | VAEPLLR | 214-220 pour la protéine de SEQ N°53 | 2b |
| SEQ ID N°242 | VAGPLLR | 214-220 pour les protéines de SEQ N°1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 1837, 1838, 1839, 1840, 1841, 1842, 1843, 1844; 213-219 pour la protéine de séquence SEQ ID N°165 | TEM |
| SEQ ID | VAGPLMR | 214-220 pour la protéine de SEQ N°134 | 2br |
| SEQ ID N°244 | VALIPFFAAFCFPVFAHPETLVK | 8-30 pour les protéines de SEQ N°4, 9, 23, 46, 47, 51, 60, 68, 69, 70, 80, 81, 89, 93, 101, 106, 108, 110, 121, 123, 147, 155, 1841, 1842 | TEM |
| SEQ ID N°245 | VALIPFFAAFCIPVFAHPETLVK | 8-30 pour la protéine de SEQ N°63 | 2br |
| SEQ ID N°246 | VALIPFFAAFCLPVFAHPDTLVK | 8-30 pour la protéine de SEQ N°139 | TEM |
| SEQ ID N°247 | VALIPFFAAFCLPVFAHPETLVK | 8-30 pour les protéines de SEQ N°1, 2, 3, 5, 6, 7, 8, 10, 11, 12, 13, 14, 15, 16, 17, 18, 19, 20, 21, 22, 24, 25, 26, 27, 28, 29, 30, 31, 32, 33, 34, 35, 36, 37, 38, 39, 40, 41, 42, 43, 44, 45, 48, 49, 50, 52, 53, 54, 55, 56, 57, 58, 59, 61, 62, 64, 65, 66, 67, 71, 72, 73, 74, 75, 76, 77, 78, 79, 82, 83, 84, 85, 86, 87, 88, 90, 91, 92, 94, 95, 96, 97, 98, 99, 100, 102, 103, 104, 105, 107, 109, 111, 112, 113, 114, 116, 117, 118, 119, 120, 122, 124, 125, 126, 127, 128, 129, 130, 131, 132, 133, 134, 135, 136, 137, 138, 140, 141, 142, 143, 144, 145, 146, 148, 149, 151, 152, 153, 154, 156, 157, 158, 159, 160, 161, 162, 163, 164, 165, 1837, 1838, 1839, 1840, 1843, 1844 | TEM |
| SEQ ID N°248 | VALIPFFAAFCLPVFAHPK | 8-26 pour la protéine de SEQ N°150 | TEM |
| SEQ ID N°249 | VALIPFLAAFCLPVFAHPETLVK | 8-30 pour la protéine de SEQ N°115 | 2be |
| SEQ ID N°250 | VDAGQEQLDR | 82-91 pour les protéines de SEQ N°55, 62 | TEM |
| SEQ ID N°251 | VDAGQEQLGR | 82-91 pour les protéines de SEQ N°1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 56, 57, 58, 59, 60, 61, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 146, 147, 148, 149, 151, 152, 153, 154, 155, 156, 157, 158, 160, 161, 162, 163, 164, 165, 1837, 1838, 1839, 1840, 1841, 1842, 1843, 1844 | TEM |
| SEQ ID N°252 | VEDAEDQLGAR | 31-41 pour la protéine de SEQ N°130 | 2b |
| SEQ ID N°253 | VGYIELDLNSGK | 42-53 pour les protéines de SEQ N°1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 1837, 1838, 1839, 1840, 1841, 1842, 1843, 1844 | TEM |
| SEQ ID N°254 | VGYIELDPNSGK | 42-53 pour la protéine de SEQ N°58 | 2be |
| SEQ ID N°255 | VGYIEMDLNSGK | 42-53 pour la protéine de SEQ N°129 | 2be |
| SEQ ID N°256 | VKPAEDK | 31-37 pour la protéine de SEQ N°120 | 2be |
| SEQ ID N°257 | VLLCGAELSR | 72-81 pour la protéine de SEQ N°99 | TEM |
| SEQ ID N°258 | VLLCGAVLSR | 72-81 pour les protéines de SEQ N°1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 1837, 1838, 1839, 1840, 1841, 1842, 1843, 1844 | TEM |
| SEQ ID N°259 | WEPELNEAIPIDER | 163-176 pour la protéine de SEQ N°128 | 2be |
| SEQ ID N°260 | WEPELNEAIPNDER | 163-176 pour les protéines de SEQ N°1, 2, 3, 4, 5, 6, 7, 8, 9, 10, 11, 12, 13, 14, 15, 16,17, 18, 19, 20, 21, 22, 23, 24, 25, 26, 27, 28, 29, 30, 31, 32, 33, 34, 35, 36, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 74, 75, 76, 77, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 116, 117, 118, 119, 120, 121, 123, 124, 125, 126, 127, 129, 130, 131, 132, 133, 134, 135, 136, 137, 138, 139, 140, 141, 142, 143, 144, 145, 146, 147, 148, 149, 150, 151, 152, 153, 154, 155, 156, 158, 159, 160, 161, 162, 163, 164, 1837,1838,1839,1840,1841,1842, 1843,1844 | TEM |
| SEQ ID N°261 | YSPVTEK | 103-109 pour les protéines de SEQ N°1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100,101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 1837, 1838, 1839, 1840, 1841, 1842, 1843, 1844 | 2be |
| SEQ ID N°1923 | DAEDK | 33-37 pour les protéines de SEQ N°2, 3, 7, 8, 11, 13, 15, 17, 20, 21, 22, 40, 42, 44, 54, 57, 58, 59, 61, 62, 63, 67, 84, 92, 104, 105, 111, 119, 124, 129, 143, 148, 164, 1839 | TEM |
| SEQ ID N°1924 | GIIAALGPDGK | 242-252 pour les protéines de SEQ N°1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 1, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 1837, 1838, 1839, 1840, 1841, 1842, 1843, 1844; 241-251 pour la protéine de séquence SEQ ID N°165 | TEM |
| SEQ ID N°1925 | GSSGIIAALGPOGK | 239-252 pour les protéines de SEQ N°28, 42, 56, 69, 72, 111, 160, 161, 1844 | TEM |
| SEQ ID N°1926 | ILESFR | 54-59 pour les protéines de SEQ N°1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 1837, 1838, 1839, 1840, 1841, 1842, 1843, 1844 | TEM |
| SEQ ID N°1927 | SGAGK | 233-237 pour les protéines de SEQ N°10, 25, 26, 44, 59, 80, 86, 122, 133, 141, 143 | 2be |
| SEQ ID N°1928 | SGTGK | 233-237 pour les protéines de SEQ N°5, 22,81,105,111,126,164 | 2be |

Dans la colonne intérêt clinique, les mentions 2b, 2br, 2be et 2ber correspondent aux sous-groupes fonctionnels des béta-lactamases TEM que permet de détecter le peptide correspondant. Ainsi la détection d'un peptide 2be signera la présence d'une béta-lactamase avec un spectre étendu (BLSE) capable d'hydrolyser les pénicillines, les céphalosporines de première génération telles que la céphaloridine et la céphalotine ainsi qu'au moins un antibiotique de la classe des oxyimino-béta-lactames tels que le cefotaxime, le ceftazidime ou les monobactames tels que l'aztreoname.

La mention TEM indique un peptide commun entre au moins deux des sous-groupes 2b, 2br et 2be ou 2ber. Le peptide correspondant signe la présence d'une beta-lactamase de type TEM et la présence d'un mécanisme de résistance au moins aux pénicillines et aux céphalosporines de première génération.

La détection d'un mécanisme de résistance aux céphalosporines de type BLSE (beta-lactamase à spectre étendu), induit par la protéine TEM, est caractérisée par la détection d'au moins un peptide marqueur de résistance, de type 2be, choisi parmi les séquences SEQ ID N°168, SEQ ID N°169, SEQ ID N°179, SEQ ID N°181, SEQ ID N°186, SEQ ID N°199, SEQ ID N°200, SEQ ID N°205, SEQ ID N°206, SEQ ID N°208, SEQ ID N°212, SEQ ID N°213, SEQ ID N°214, SEQ ID N°215, SEQ ID N°216, SEQ ID N°217, SEQ ID N°222, SEQ ID N°225, SEQ ID N°226, SEQ ID N°227, SEQ ID N°230, SEQ ID N°234, SEQ ID N°236, SEQ ID N°237, SEQ ID N°238, SEQ ID N°239, SEQ ID N°240, SEQ ID N°249, SEQ ID N°254, SEQ ID N°255, SEQ ID N°256, SEQ ID N°259, SEQ ID N°261, SEQ ID N°1927, SEQ ID N°1928.

La détection d'un mécanisme de résistance aux céphalosporines induit par l'expression de la protéine CMY est caractérisée par la détection d'au moins un peptide appartenant à la protéine CMY et à ses différents variants de séquences **SEQ ID N° 262** à **SEQ ID N°311** et **SEQ ID N°1844** à **SEQ ID N°1870.**
**SEQ ID N° 262 :** 
MQQRQSILWGAVATLMWAGLAHAGEASPVDPLRPVVDASIQPLLKEHRIPGMAVAV
LKDGKAHYFNYGVANRESGASVSEQTLFEIGSVSKTLTATLGAYAVVKGAMQLDDK
ASRHAPWLKGSVFDSITMGELATYSAGGLPLQFPEEVDSSEKMRAYYRQWAPVYS
PGSHRQYSNPSIGLFGHLAASSLKQPFAQLMEQTLLPGLGMHHTYVNVPKQAMAS
YAYGYSKEDKPIRVNPGMLADEAYGIKTSSADLLAFVKANIGGVDDKALQQAISLTH
KGHYSVGGMTQGLGWESYAYPVTEQTLLAGNSAKVILEANPTAAPRESGSQVLFN
KTGSTNGFGAYVAFVPARGIGIVMLANRNYPIPARVKAAHAILAQLAG
**SEQ ID N° 263 :** 
MQQRQSILWGAVATLMWAGLAHAGEASPVDPLRPVVDASIQPLLKEHRIPGMAVAV
LKDGKAHYFNYGVANRESGAGVSEQTLFEIGSVSKTLTATLGAYAVVKGAMQLDDK
ASRHAPWLKGSAFDSITMGELATYSAGGLPLQFPEEVDSSEKMRAYYRQWAPVYS
PGSHRQYSNPSIGLFGHLAASSLKQPFAPLMEQTLLPGLGMHHTYVNVPKQAMAS
YAYGYSKEDKPIRVNPGMLADEAYGIKTSSADLLRFVKANIGGVDDKALQQAISLTH
QGHYSVGGMTQGLGWESYAYPVTEQTLLAGNSAKVILEANPTAAPRESGSQVLFN
KTGSTNGFGAYVAFVPARGIGIVMLANRNYPIEARIKAAHAILAQLAG
**SEQ** ID **N° 264 :** 
MQQRQSILWGAVATLMWAGLAHAGEASPVDPLRPVVDASIQPLLKEHRIPGMAVAV
LKDGKAHYFNYGVANRESGAGVSEQTLFEIGSVSKTLTATLGAYAVVKGAMQLDDK
ASRHAPWLKGSAFDSITMGELATYSAGGLPLQFPEEVDSSEKMRAYYRQWAPVYS
PGSHRQYSNPSIGLFGHLAASSLKQPFAPLMEQTLLPGLGMHHTYVNVPKQAMAS
YAYGYSKEDKPIRVNPGMLADEAYGIKTSSADLLRFVKANIGGVDDKALQQAISLTH
QGHYSVGGMTQGLGWESYAYPVTEQTLLAGNSAKVSLEANPTAAPRESGSQVLFN
KTGSTNGFGAYVAFVPARGIGIVMLANRNYPIEARIKAAHAILAQLAG
**SEQ** ID **N° 265 :** 
MMKKSLCCALLLTASFSTFASAKTEQQIADIVNRTITPLMQEQAIPGMAVAIIYQGKPY
YFTWGKADIANNHPVTQQTLFELGSVSKTFNGVLGGDAIARGEIKLSDPVTKYWPEL
TGKQWQGISLLHLATYTAGGLPLQIPDDVTDKAALLRFYQNWQPQWAPGAKRLYA
NSSIGLFGALAVKPSGMSYEEAMTRRVLQPLKLAHTWITVPQNEQKDYAWGYREG
KPVHVSPGQLDAEAYGVKSNVTDMARWVQVNMDASRVQEKTLQQGIALAQSRYW
RIGDMYQGLGWEMLNWPLKADSIINGSDSKVALAALPAVEVNPPAPAVKASWVHKT
GSTGGFGSYVAFVPEKNLGIVMLANKSYPNPVRVEAAWRILEKLQ
**SEQ** ID **N° 266 :** 
MMKKSLCCALLLTASFSTFAAAKTEQQIADIVNRTITPLMQEQAIPGMAVAVIYQGKP
YYFTWGKADIANNHPVTQQTLFELGSVSKTFNGVLGGDAIARGEIKLSDPVTKYWP
ELTGKQWQGIRLLHLATYTAGGLPLQIPDDVRDKAALLHFYQNWQPQWTPGAKRL
YANSSIGLFGALAVKPSGMSYEEAMTRRVLQPLKLAHTWITVPQNEQKDYAWGYR
EGKPVHVSPGQLDAEAYGVKSSVIDMARWVQANMDASHVQEKTLQQGIALAQSRY
WRIGDMYQGLGWEMLNWPLKADSIINGSDSKVAFAALPAVEVNPPAPAVKASWVH
KTGSTGGFGSYVAFVPEKNLGIVMLANKSYPNPVRVEAAWRILEKLQ
**SEQ** ID **N° 267 :** 
MMKKSLCCALLLTASFSTFAAAKTEQQIADIVNRTITPLMQEQAIPGMAVAVIYQGKP
YYFTWGKADIANNHPVTQQTLFELGSVSKTFNGVLGGDAIARGEIKLSDPVTKYWP
 
ELTGKQWQGIRLLHLATYTAGGLPLQIPDDVRDKAALLHFYQNWQPQWTPGAKRL
YANSSIGLFGALAVKPSGMSYEEAMTRRVLQPLKLAHTWITVPQNEQKDYAWGYR
EGKPVHVSPGQLDAEAYGVKSSVIDMARWVQANMDASHVQEKTLQQGIALAQSRY
WRIGDMYQGLGWEMLNWPLKADSIINGNGSDSKVALAALPAVEVNPPAPAVKASW
VHKTGSTGGFGSYVAFVPEKNLGIVMLANKSYPNPVRVEAAWRILEKLQ
**SEQ ID N° 268 :** 
MKKSLCCALLLTASFSTFAAAKTEQQIADIVNRTITPLMQEQAIPGMAVAVIYQGKPY
YFTWGKADIANNHPVTQQTLFELGSVSKTFNGVLGGDAIARGEIKLSDPVTKYWPEL
TGKQWQGISLLHLATYTAGGLPLQIPDDVTDKAALLHFYQNWQPQWTPGAKRLYA
NSSIGLFGALAVKPSGMSYEEAMTRRVLQPLKLAHTWITVPQNEQKDYAWGYREG
KPVHVSPGQLDAEAYGVKSSVIDMARWVQANMDASHVQEKTLQQGIALAQSRYW
RIGDMYQGLGWEMLNWPLKADSIINGSDNKVALAALPAVEVNPPAPAVKASWVHKT
GSTGGFGSYVAFVPEKNLGIVMLANKSYPNPVRVEAAWRILEKLQ
**SEQ ID N° 269 :** 
MMKKSLCCALLLTASFSTFAAAKTEQQIADIVNRTITPLMQEQAIPGMAVAVIYQGKP
YYFTWGKADIANNHPVTQQTLFELGSVSKTFNGVLGGDAIARGEIKLSDPVTKYWP
ELTGKQWQGIRLLHLATYTAGGLPLQIPDDVRDKAALLHFYQNWQPQWTPGAKRL
YANSSIGLFGELAVKPSGMSYEEAMTRRVLQPLKLAHTWITVPQNEQKDYAWGYR
EGKPVHVSPGKLDAEAYGVKSSVIDMARWVQANMDASHVQEKTLQQGIALAQSRY
WRIGDMYQGLGWEMLNWPLKADSIINGSDSKVALAALPAVEVNPPAPAVKASWVH
KTGSTGGFGSYVAFVPEKNLGIVMLANKSYPNPVRVEAAWRILEKLQ
**SEQ ID N° 270**: 
MMKKSLCCALLLTASFSTFAAAKTEQQIADIVNRTITPLMQEQAIPGMAVAVIYQGKP
YYFTWGKADIANNHPVTQQTLFELGSVSKTFNGVLGGDAIARGEIKLSDPVTKYWP
ELTGKQWQGIRLLHLATYTAGGLPLQIPDDVRDKAALLHFYQNWQPQWTPGAKRL
YANSSIGLFGALAVKPSGMSYEEAMTRRVLQPLKLAHTWITVPQNEQKDYAWGYR
EGKPVHVSPGQLDAEAYGVKSSVIDMARWVQANMDASHVQEKTLQQGIALAQSRY
WRIGDMYQGLGWEMLNWPLKADSIINGSDSKVALAALPAVEVNPPAPAVKASWVH
KTGSTGGFGSYVAFVPEKNLGIVMLANKSYPNPVRVEAYWRILEKLQ
**SEQ ID N° 271 :** 
MMKKSLCCALLLTASFSTFAAAKTEQQIADIVNRTITPLMQEQAIPGMAVAVIYQGKP
YYFTWGKADIANNHPVTQQTLFELGSVSKTFNGVLGGDAIARGEIKLSDPVTKYWP
ELTGKQWQGIRLLHLATYTAGGLPLQIPDDVRDKAALLHFYQNWQPQWTPGAKRL
YANSSIGLFGALAVKPSGMSYEEAMTRRVLQPLKLAHTWITVPQNEQKDYAWGYR
 
EGKPVHVSPGQLDAGAYGVKSSVIDMARWVQANMDASHVQEKTLQQGIALAQSRY
WRIGDMYQGLGWEMLNWPLKADSIINGSDSKVALAALPAVEVNPPAPAVKASWVH
KTGSTGGFGSYVAFVPEKNLGIVMLANKSYPNPVRVEAAWRILEKLQ
**SEQ ID N° 272:** 
MMKKSLCCALLLTASFSTFAAAKTEQQIADIVNRTITPLMQEQAIPGMAVAVIYQGKP
YYFTWGKADIANNHPVTQQTLFELGSVSKTFNGVLGGDAIARGEIKLSDPVTKYWP
ELTGKQWQGISLLHLATYTAGGLPLQIPDDVTDKAALLHFYQNWQPQWTPGAKRLY
ANSSIGLFGALAVKPSGMSYEEAMTRRVLQPLKLAHTWITVPQNEQKDYAWGYRE
GKPVHVSPGQLDAEAYGVKSSVIDMARWVQANMDASHVQEKTLQQGIALAQSRY
WRIGDMYQGLGWEMLNWPLKADSIINGSDSKVALAALPAVEVNPPAPAVKASWVH
KTGSTGGFGSYVAFVPEKNLGIVMLANKSYPNPVRVEAAWRILEKLQ
**SEQ ID N° 273 :** 
MMKKSLCCALLLTASFSTFAAAKTEQQIADIVNRTITPLMQEQAIPGMAVAVIYQGKP
YYFTWGKADIANNHPVTQQTLFELGSVSKTFNGVLGGDAIARGEIKLSDPVTKYWP
ELTGKQWQGIRLLHLATYTAGGLPLQIPDDVRDKAALLHFYQNWQPQWTPGAKRL
YANSSIGLFGALAVKPSGMSYEEAMTRRVLQPLKLAHTWITVPQNEQKDYAWGYR
EGKAVHVSPGQLDAEAYGVKSSVIDMARWVQVNMDASRVQEKTLQQGIALAQSRY
WRIGDMYQGLGWEMLNWPLKADSIINGSDSKVALAALPAVEVNPPAPAVKASWVH
KTGSTGGFGSYVAFVPEKNLGIVMLANKSYPNPVRVEAAWRILEKLQ
**SEQ ID N° 274:** 
MMKKSLCCALLLTASFSTFAAAKTEQQIADIVNRTITPLMQEQAIPGMAVAVIYQGKP
YYFTWGKADIANNHPVTQQTLFELGSVSKTFNGVLGGDAIARGEIKLSDPVTKYWP
ELTGKQWQGIRLLHLATYTAGGLPLQIPDDVRDKAALLHFYQNWQPQWTPGAKRL
YANSSIGLFGALAVKPSGMSYEEAMTRRVLQPLKLAHTWITVPQNEQKDYAWGYR
EGKPVYVSPGQLDAEAYGVKSSVIDMARWVQANMDASHVQEKTLQQGIALAQSRY
WRIGDMYQGLGWEMLNWPLKADSIINGSDSKVALAALPAVEVNPPAPAVKASWVH
KTGSTGGFGSYVAFVPEKNLGIVMLANKSYPNPVRVEAAWRILEKLQ
**SEQ ID N° 275 :** 
MMKKSLCCALLLTASFSTFAAAKTEQQIADIVNRTITPLMQEQAIPGMAVAVIYQGKP
YYFTWGKADIANNHPVTQQTLFELGSVSKTFNGVLGGDAIARGEIKLSDPVTKYWP
ELTGKQWQGIRLLHLATYTAGGLPLQIPDDVRDKAALLHFYQNWQPQWTPGAKRL
YANSSIGLFGELAVKPSGMSYEEAMTRRVLQPLKLAHTWITVPQNEQKDYAWGYR
EGKPVHVSPGQLDAEAYGVKSSVIDMARWVQANMDASHVQEKTLQQGIALAQSRY
WRIGDMYQGLGWEMLNWPLKADSIINGSDNKVALAALPAVEVNPPAPAVKASWVH
 KTGSTGGFGSYVAFVPEKNLGIVMLANKSYPNPVRVEAAWRILEKLQ
**SEQ ID N° 276 :** 
MMKKSLCCALLLTASFSTFAAAKTEQQIADIVNRTITPLMQEQAIPGMAVAVIYQGKP
YYFTWGKADIANNHPVTQQTLFELGSVSKTFNGVLGGDAIARGEIKLSDPVTKYWP
ELTGKQWQGIRLLHLATYTAGGLPLQIPDDVRDKAALLHFYQNWQPQWTPGAKRL
YANSSIGLFGALAVKPSGMSYEEAMTRRVLQPLKLAHTWITVPQNEQKDYAWGYR
EGKPVHVSPGRLDAEAYGVKSSVIDMARWVQANMDASHVQEKTLQQGIALAQSRY
WRIGDMYQGLGWEMLNWPLKADSIINGSDSKVALAALPAVEVNPPAPAVKASWVH
KTGSTGGFGSYVAFVPEKNLGIVMLANKSYPNPVRVEAAWRILEKLQ
**SEQ ID N° 277 :** 
MMKKSLCCALLLTASFSTFAAAKTEQQIADIVNRTITPLMQEQAIPGMAVAVIYQGKP
YYFTWGKADIANNHPVTQQTLFELGSVSKTFNGVLGGDAIARGEIKLSDPVTKYWP
ELTGKQWQGIRLLHLATYTAGGLPLQFPDDVRDKAALLHFYQNWQPQWTPGAKRL
YANSSIGLFGELAVKPSGMSYEEAMTRRVLQPLKLAHTWITVPQNEQKDYAWGYR
EGKPVHVSPGQLDAEAYGVKSSVIDMARWVQANMDASHVQEKTLQQGIALAQSRY
WRIGDMYQGLGWEMLNWPLKADSIINGSDSKVALAALPAVEVNPPAPAVKASWVH
KTGSTGGFGSYVAFVPEKNLGIVMLANKSYPNPVRVEAAWRILEKLQ
 
SEQ ID N° 278 :
MMKKSLCCALLLTASFSTFAAAKTEQQIADIVNRTITPLMQEQAIPGMAVAVIYQGKP
YYFTWGKADIANNHPVTQQTLFELGSVSKTFNGVLGGDAIARGEIKLSDPVTKYWP
ELTGKQWQGIRLLHLATYTAGGLPLQIPDDVRDKAALLHFYQNWQPQWTPGAKRL
YANSSIGLFGALAVKPSGMSYEEAMTRRVLQPLKLAHTWITVPQNEQKDYAWGYR
EGKPVHGSPGQLDAEAYGVKSSVIDMARWVQANMDASHVQEKTLQQGIALAQSRY
WRIGDMYQGLGWEMLNWPLKADSIINGSDSKVALAALPAVEVNPPAPAVKASWVH
KTGSTGGFGSYVAFVPEKNLGIVMLANKSYPNPVRVEAAWRILEKLQ
**SEQ ID N° 279 :** 
MMKKSLCCALLLTASFSTFAAAKTEQQIADIVNRTITPLMQEQAIPGMAVAVIYQGKP
YYFTWGKADIANNHPVTQQTLFELGSVSKTFNGVLGGDCIARGEIKLSDPVTKYWP
ELTGKQWQGIRLLHLATYTAGGLPLQIPDDVRDKAALLHFYQNWQPQWTPGAKRL
YANSSIGLFGALAVKPSGMSYEEAMTRRVLQPLKLAHTWITVPENEQKDYAWGYRE
GKPVHVSPGQLDAEAYGVKSSVIDMARWVQANMDASHVQEKTLQQGIALAQSRY
WRIGDMYQGLGWEMLNWPLKADSIINGSDSKVALAALPAVEVNPPAPAVKASWVH
KTGSTGGFGSYVAFVPEKNLGIVMLANKSYPNPVRVEAAWRILEKLQ
**SEQ ID N° 280 :** 
MMKKSLCCALLLTASFSTFAAAKTEQQIADIVNRTITPLMQEQAIPGMAVAVIYQGKP
YYFTWGKADIANNHPVTQQTLFELGSVSKTFNGVLGGDAIARGEIKLSDPVTKYWP
ELTGKQWQGIRLLHLATYTAGGLPLQIPDDVTDKAALLHFYQNWQPQWTPGAKRLY
ANSSIGLFGALAVKPSGMSYEEAMTRRVLQPLKLAHTWITVPQNEQKDYAWGYRE
GKPVHVSPGQLDAEAYGVKSSVIDMARWVQANMDASHVQEKTLQQGIALAQSRY
WRIGDMYQGLGWEMLNWPLKADSIINGSDSKVALAALPAVEVNPPAPAVKASWVH
KTGSTGGFGSYVAFVPEKNLGIVMLANKSYPNPVRVEAAWRILEKLQ
**SEQ ID N° 281 :** 
MMKKSLCCALLLTASFSTFAAAKTEQQIADIVNRTITPLMQEQAIPGMAVAVIYQGKP
YYFTWGKADIANNHPVTQQTLFELGSVSKTFNGVLGGDAIARGEIKLSDPVTKYWP
ELTGKQWQGIRLLHLATYTAGGLPLQIPDDVRDKAALLHFYQNWQPQWTPGAKRL
YANSSIGLFGALAVKPSGMSYEEAMTRRVLQPLKLAHTWITVPQNEQKDYACGYRE
GKPVHVSPGQLDAEAYGVKSSVIDMARWVQANMDASHVQEKTLQQGIALAQSRY
WRIGDMYQGLGWEMLNWPLKADSIINGSDSKVALAALPAVEVNPPAPAVKASWVH
KTGSTGGFGSYVAFVPEKNLGIVMLANKSYPNPVRVEAAWRILEKLQ
**SEQ ID N° 282** : 
MMKKSLCCALLLTASFSTFAAAKTEQQIADIVNRTITPLMQEQAIPGMAVAVIYQGKP
YYFTWGKADIANNHPVTQQTLFELGSVSKTFNGVLGGDAIARGEIKLSDPVTKYWP
ELTGKQWQGISLLHLATYTAGGLPLQIPDDVTDKAALLRFYQNWQPQWTPGAKRLY
ANSSIGLFGTLAVKPSGMSYEEAMTRRVLQPLKLAHTWITVPQSEQKDYAWGYRE
GKPVHVSPGQLDAEAYGVKSSVIDMAHWVQANMDASHVQEKTLQQGIELAQSRY
WRIGDMYQGLGWEMLNWPLKADSIINGSDSKVALAALPAVEVNPPAPAVKASWVH
KTGYTGGFGSYVAFVPEKNLGIVMLANKSYPNPVRVEAAWRILEKLQ
**SEQ ID N° 283 :** 
MMKKSLCCALLLTASFSTFAAAKTEQQIADIVNRTITPLMQEQAIPGMAVAVIYQGKP
YYFTWGKADIANNHPVTQQTLFELGSVSKTFNGVLGGDAIARGEIKLSDPVTKYWP
ELTGKQWQGIRLLHLATYTAGGLPLQIPDDVRDKAALLHFYQNWQPQWTPGAKRL
YANSSIGLFGALAVKPSGMSYEEAMTRRVLQPLKLAHTWITVPQNEQKDYAWGYR
EGKPVHVSPEQLDAEAYGVKSSVIDMARWVQANMDASHVQEKTLQQGIALAQSRY
WRIGDMYQGLGWEMLNWPLKADSIINGSDSKVALAALPAVEVNPPAPAVKASWVH
KTGSTGGFGSYVAFVPEKNLGIVMLANKSYPNPVRVEAAWRILEKLQ
**SEQ ID N° 284 :** 
MMKKSLCCALLLTASFSTFAAAKTEQQIADIVNRTITPLMQEQAIPGMAVAVIYQGKP
YYFTWGKADIANNHPVTQQTLFELGSVSKTFNGVLGGDAIARGEIKLSDPVTKYWP
 
ELTGKQWQGIRLLHLATYTAGGLPLQIPDDVRDKAALLHFYQNWQPQWTPGAKRL
YANSSIGLFGALAVKPSGMSYEEAMTRRVLQPLKLAHTWITVPQNEQKDYAWGYR
EGKPVHVSPGQLDAEAYGVKSSVIDMARWVQANMDASHVQEKTLQQGIALAQSRY
WRIGDMYQGLGWEMLNWPLKADSIINGSDSKVAALPAVEVNPPAPAVKASWVHKT
GSTGGFGSYVAFVPEKNLGIVMLANKSYPNPVRVEAAWRILEKLQ
**SEQ ID N° 285 :** 
MMKKSLCCALLLTASFSTFAAAKTEQQIADIVNRTITPLMQEQAIPGMAVAVIYQGKP
YYFTWGKADIANNHPVTQQTLFELGSVSKTFNGVLGGDAIARGEIKLSDPVTKYWP
ELTGKQWQGIRLLHLATYTAGGLPLQIPDDVRDKAALLHFYQNWQPQWTPGAKRL
YANSSIGLFAALAVKPSGMSYEEAMTRRVLQPLKLAHTWITVPQNEQKDYAWGYRE
GKPVHVSPGQLDAEAYGVKSSVIDMARWVQANMDASHVQEKTLQQGIALAQSRY
WRIGDMYQGLGWEMLNWPLKADSIINGSDSKVALAALPAVEVNPPAPAVKASWVH
KTGSTGGFGSYVAFVPEKNLGIVMLANKSYPNPVRVEAAWRILEKLQ
**SEQ ID N° 286 :** 
MMKKSLCCALLLTASFSTFAAAKTEQQIADIVNRTITPLMQEQAIPGMAVAVIYQGKP
YYFTWGKADIANNHPVTQQTLFELGSVSKTFNGVLGGDAIARGEIKLSDPVTKYWP
ELTGKQWQGIRLLHLATYTAGGLPLQIPDDVRDKAALLHFYQNWQPQWTPGAKRL
YANSSIGLFGALAVKPSGMSYEEAMTRRVLQPLKLAHTWITVPQNEQKDYAWGYR
EGKPVHVSPGQLDAEAYGVKSSVIDMARWVQANMDASHVQEKTLQQGIALAQSRY
WRIGDMYQGLGWEMLNWPLKADSIINGSDSKVAPAVEVNPPAPAVKASWVHKTGS
TGGFGSYVAFVPEKNLGIVMLANKSYPNPVRVEAAWRILEKLQ
**SEQ ID N° 287 :** 
MMKKSLCCALLLTASFSTFAAAKTEQQIADIVNRTITPLMQEQAIPGMAVAIIYQGKPY
YFTWGKADIANNHPVTQQTLFELGSVSKTFNGVLGGDAIARGEIKLSDPVTKYWPEL
TGKQWQGISLLHLATYTAGGLPLQIPDDVTDKAALLRFYQNWQPQWTPGAKRLYA
NSSIGLFGALAVKPSGMSYEEAMTRRVLQPLKLAHTWITVPQNEQKDYAWGYREG
KPVHVTPGQLDAEAYGVKSNVTDMARWIQVNMDASRVQEKTLQQGIALAQSRYW
RIGDMYQGLGWEMLNWPLKADSIINGSDSKVALAALHTVEVNPPAPAVKASWVHKT
GSTGGFGSYVAFIPEKNLGIVMLANKSYPNPVRVEAAWRILEKLQ
**SEQ ID N° 288 :** 
MMKKSICCALLLTASFSTFAAAKTEQQIADIVNRTITPLMQEQAIPGMAVAIIYEGKPY
YFTWGKADIANNHPVTQQTLFELGSVSKTFNGVLGGDAIARGEIKLSDPVTKYWPEL
TGKQWRGISLLHLATYTAGGLPLQIPDEVTDKAELLRFYQNWQPQWTPGAKRLYAN
SSIGLFGALVVKPSGMSYEEAMTRRVLQPLKLAHTWITVPQSEQKNYAWGYREGK
 
PVHVSPGQLDAEAYGVKSSVIDMARWVQANMDASHVQEKTLQQGIELAQSRYWRI
GDMYQGLGWEMLNWPLKADSIINGSDSKVALAALPAVEVNPPAPAVKASWVHKTG
STGGFGSYVAFVPEKNLGIVMLANKSYPNPARVEAAWRILEKLQ
**SEQ ID N° 289 :** 
MMKKSLCCALLLTASFSTFAAAKTEQQIADIVNRTITPLMQEQAIPGMAVAVIYQGKP
YYFTWGKADIANNHPVTQQTLFELGSVSKTFNGVLGGDAIARGEIKLSDPVTKYWP
ELTGKQWQGIRLLHLATYTAGGLPLQIPDDVRDKAALLHFYQNWQPQWTPGAKRL
YANSSIGLFGALAVKPSGMSYEEAMTRRVLQPLKLAHTWITVPQNEQKDYAWGYR
EGKPVHVSPGQLELDAEAYGVKSSVIDMARWVQANMDASHVQEKTLQQGIALAQS
RYWRIGDMYQGLGWEMLNWPLKADSIINGSDSKVALAALPAVEVNPPAPAVKASW
VHKTGSTGGFGSYVAFVPEKNLGIVMLANKSYPNPVRVEAAWRILEKLQ
**SEQ ID N° 290** : 
MMKKSLCCALLLTASFSTFAAAKTEQQIADIVNRTITPLMQEQAIPGMAVAVIYQGKP
YYFTWGKADIANNHPVTQQTLFELGSVSKTFNGVLGGEAIARGEIKLSDPVTKYWPE
LTGKQWQGIRLLHLATYTAGGLPLQIPDDVRDKAALLHFYQNWQPQWTPGAKRLY
ANSSIGLFGALAVKPSGMSYEEAMTRRVLQPLKLAHTWITVPQNEQKDYAWGYRE
GKPVHVSPGQLDAEAYGVKSSVIDMARWVQANMDASHVQEKTLQQGIALAQSRY
WRIGDMYQGLGWEMLNWPLKADSIINGSDSKVALAALPAVEVNPPAPAVKASWVH
KTGSTGGFGSYVAFVPEKNLGIVMLANKSYPNPVRVEAAWRILEKLQ
**SEQ ID N° 291 :** 
MMKKSLCCALLLTASFSTFAAAKTEQQIADIVNRTITPLMQEQAIPGMAVAVIYQGKP
YYFTWGKADIANNHPVTQQTLFELGSVSKTFNGVLGGDAIARGEIKLSDPVTKYWP
ELTGKQWQGIRLLHLATYTAGGLPLQIPDDVRDKAALLHFYQNWQPQWTPGAKRL
YANSSIGLFGALAVKPSGMSYEEAMTRRVLQPLKLAHTWITVPQNEQKDYAWGYR
EGKPVHVSPGQFDAEAYGVKSSVIDMARWVQANMDASHVQEKTLQQGIALAQSRY
WRIGDMYQGLGWEMLNWPLKADSIINGSDSKVALAALPAVEVNPPAPAVKASWVH
KTGSTGGFGSYVAFVPEKNLGIVMLANKSYPNPVRVEAAWRILEKLQ
**SEQ ID N° 292** : 
MMKKSLCCALLLTASFSTFAAAKTEQQIADIVNRTITPLMQEQAIPGMAVAVIYQGKP
YYFTWGKADIANNHPVTQQTLFELGSVSKTFNGVLGGDAIARGEIKLSDPVTKYWP
ELTGKQWQGIRLLHLATYTAGGLPLQIPDDVRDKAALLHFYQNWQPQWTPGAKRL
YANSSIGLFGALAVKPSGMSYEEAMTRRVLQPLKLAHTWITVPQNEQKDYAWGYR
EGKPVHVSPGQLDAEAYGVKSSVIDMARWVQANMDASHVQEKTLQQGIALAQSRY
WRIGDMYQGLGWEMLNWPLKTDSIINGSDSKVALAALPAVEVNPPAPAVKASWVH
 KTGSTGGFGSYVAFVPEKNLGIVMLANKSYPNPVRVEAAWRILEKLQ
**SEQ ID N° 293 :** 
MQQRQSILWGAVATLMWAGLAHAGEASPVDPLRPVVDASIQPLLKEHRIPGMAVAV
LKDGKAHYFNYGVANRESGASVSEQTLFDIGSVSKTLTATLGAYAVVKGAMQLDDK
ASRHAPWLKGSVFDSITMGELATYSAGGLPLQFPEEVDSSEKMRAYYRQWAPVYS
PGSHRQYSNPSIGLFGHLAASSLKQPFAQLMEQTLLPGLGMHHTYVNVPKQAMAS
YAYGYSKEDKPIRVNPGMLADEAYGIKTSSADLLAFVKANIGGVDDKALQQAISLTH
KGHYSVGGMTQGLGWESYAYPVTEQTLLAGNSAKVILEANPTAAPRESGSQVLFN
KTGSTNGFGAYVAFVPARGIGIVMLANRNYPIPARVKAAHAILAQLAG
**SEQ ID N° 294 :** 
MQQRQSILWGAVATLMWAGLAHAGEASPVDPLRPVVDASIQPLLKEHRIPGMAVAV
LKDGKAHYFNYGVANRESGASVSEQTLFDIGSVSKTLTATLGAYAVVKGAMQLDDK
ASRHAPWLKGSVFDSITMGELATYSAGGLPLQFPEEVDSSEKMRAYYRQWAPVYS
PGSHRQYSNPSIGLFGHLAASSLKQPFAQLMEQTLLPGLGMHHTYVNVPKQAMAS
YAYGYSKEDKPIRVNPGMLADEAYGIKTSSADLLAFVKANIGGVDDKALQQAISLTH
KGHYSVGGMTQGLGWESYAYPVTEQTLLAGNSAKVSLEANPTAAPRESGSQVLFN
KTGSTNGFGAYVAFVPARGIGIVMLANRNYPIPARVKAAHAILAQLAG
**SEQ ID N° 295 :** 
MMKKSLCCALLLTASFSTFAAAKTEQQIADIVNRTITPLMQEQAIPGMAVAVIYQGKP
YYFTWGKADIANNHPVTQQTLFELGSVSKTFNGVLGGDAIARGEIKLSDPVTKYWS
ELTGKQWQGISLLHLATYTAGGLPLQIPDDVTDKTALLHFYQNWQPQWAPGAKRLY
ANSSIGLFGALAVKPSGMSYEEAMTRRVLQPLKLAHTWITVPQSEQKDYAWGYRE
GKPVHVSPGQLDAEAYGVKSSVIDMARWVQANMDASRVQEKTLQQGIALAQSRY
WRIGDMYQGLGWEMLNWPLKADSIINGSDSKVALAAIPAVEVNPPAPAVKASWVHK
TGSTGGFGSYVAFVPEKNLGIVMLANKSYPNPIRVEAAWRILEKLQ
**SEQ ID N° 296 :** 
MMKKSLCCALLLTASFSTFASAKTEQQIADIVNRTITPLMQEQAIPGMAVAVIYQGKT
YYFTWGKADIANNHPVTQQTLFELGSVSKTFNGVLGGDAIARGEIKLSDPVTKYWP
ELTGKQWQDISLLHLATYTAGGLPLQIPDDVTDKTALLHFYQNWQPQWAPGAKRLY
ANSSIGLFGALAVKPSGMSYEEAMTRRVLQPLKLAHTWITVPQSEQKDYAWGYRE
GKPVHVSPGQLDAEAYGVKSNVTDMARWVQVNMDASRVQEKTLQQGIALAQSRY
WRIGDMYQGLGWEMLNWPLKADSIINGSDSKVALAALPAVEVNPPAPAVKASWVH
KTGSTGGFGSYVAFVPEKNLGIVMLANKSYPNPVRVEAAWRILEKLQ
**SEQ ID N° 297 :** 
MMKKSLCCALLLTAPLSTFAAAKTEQQIADIVNRTITPLMQEQAIPGMAVAVIYQGKP
YYFTWGKADITNNHPVTQQTLFELGSVSKTFNGVLGGDAIARGEIKLSDPVTKYWPE
LTGKQWQGISLLHLATYTAGGLPLQIPDDVTDKAALLRFYQNWQPQWAPGAKRLYA
NSSIGLFGALAVKPSGMSYEEAMTRRVLQPLKLAHTWIKVPQSEQKDYAWGYREG
KAVHVSPGQLDAEAYGVKSSVIDMARWVQVNMDASRVQEKTLQQGIALAQSRYW
RIGDMYQGLGWEMLNWPLKADSIINGSDSKVALAALPAVEVNPPAPAVKASWVHKT
GSTGGFGSYVAFVPEKNLGIVMLANKSYPNPVRVEAAWRILEKLQ
**SEQ ID N° 298 :** 
MMKKSICCALLLTASFSTFAATKTEQQIADIVNRTITPLMQEQAIPGMAVAIIYEEKPY
YFTWGKADIANNHPVTQQTLFELGSVSKTFNGVLGGDAIARGEIKLSDPVTKYWPEL
TGKQWRGISLLHLATYTAGGLPLQIPDEVTDKAALLRFYQNWQPQWTPGAKRLYAN
SSIGLFGALAVKPSGMSYEEAMTRRVLQPLKLAHTWITVPQSEQKNYAWGYREGK
PVHVSPGQLDAEAYGVKSSVIDMARWVQANMDASHVQEKTLQQGIELAQSRYWRI
GDMYQGLGWEMLNWPLKADSIINGSDSKVALAALPAVEVNPPAPAVKASWVHKTG
STGGFGSYVAFVPEKNLGIVMLANKSYPNPARVEAAWRILEKLQ
**SEQ ID N° 299** : 
GPGHLFAFNYGTDFMMKKSLCCALLLTASFSTFAAAKTEQQIADIVNRTITPLMQEQ
AIPGMAVAVIYQGKPYYFTWGKADIANNHPVTQQTLFELGSVSKTFNGVLGGDAIAR
GEIKLSDPVTKYWPELTGKQWQGIRLLHLATYTAGGLPLQIPDDVRDKAALLHFYQN
WQPQWTPGAKRLYANSSIGLFGALAVKPSGMSYEEAMTRRVLQPLKLAHTWITVP
QNEQKDYAWGYREGKPVHASPGQLDAEAYGVKSSVIDMARWVQANMDASHVQE
KTLQQGIALAQSRYWRIGDMYQGLGWEMLNWPLKADSIINGSDSKVALAALPAVEV
NPPAPAVKASWVHKTGSTGGFGSYVAFVPEKNLGIVMLANKSYPNPVRVEAAWRIL
EKLQ
**SEQ ID N° 300 :** 
MMKKSLCCALLLTASFSTFAAAKTEQQIADIVNRTITPLMQEQAIPGMAVAVIYQGKP
YYFTWGKADIANNHPVTQQTLFELGSVSKTFNGVLGGDAIARGEIKLSDPVTKYWP
ELTGKQWQGIRLLHLATYTAGGLPLQIPDDVRDKAALLHFYQNWQPQWTPGAKRL
YANSSIGLFGALAVKPSGMSYEEAMTRRVLQPLKLAHTWITVPQNEQKDYAWGYR
EGKPVHVSPGQLDAEAYGVKSSVIDMARWVQANMDASHVQEKTLQQGIALAQSRY
WRIGDMYQGLGWEMLNWPLKADSIINGSDSKVALAALPAVEVNPPAPAVKASWVH
KTGSTGGFGSYVAFVPEKNLGIVMLANKSYPNPVRVEAAWRILEKLQ
**SEQ ID N° 301 :** MQQRQSILWGAVATLMWAGLAHAGEASPVDPLRPVVDASIQPLLKEHRIPGMAVAV 
LKDGKAHYFNYGVANRESGAGVSEQTLFEIGSVSKTLTATLGAYAVVKGAMQLDDK
ASRHAPWLKGSAFDSITMGELATYSAGGLPLQFPEEVDSSEKMRAYYRQWAPVYS
PGSHRQYSNPSIGLFGHLAASSLKQPFAPLMEQTLLPGLGMHHTYVNVPKQAMAS
YAYGYSKEDKPIRVNPGMLADEAYGIKTSSADLLRFVKANIGGVDDKALQQAISLTH
QGHYSVGGMTQGLGWESYAYPVTEQTLLAGNSAKVILEANPTAAPRESGSQVLFN
KTGSTNGFGAYVAFVPARGIGIVMLANRNYPNEARIKAAHAILAQLAG
**SEQ ID N° 302 :** 
MMKKSLCCALLLTASFSTFAAAKTEQQIADIVNRTITPLMQEQAIPGMAVAVIYQGKP
YYFTWGKADIANNHPVTQQTLFELGSVSKTFNGVLGGDAIARGEIKLSDPVTKYWP
ELTGKQWQGIRLLHLATYTAGGLPLQIPDDVRDKAALLHFYQNWQPQWTPGAKRL
YANSSIGLFGALAVKPSGMSYEEAMTRRVLQPLKLAHTWITVPQNEQKDYARGYRE
GKPVHVSPGQLDAEAYGVKSSVIDMARWVQANMDASHVQEKTLQQGIALAQSRY
WRIGDMYQGLGWEMLNWPLKADSIINGSDSKVALAALPAVEVNPPAPAVKASWVH
KTGSTGGFGSYVAFVPEKNLGIVMLANKSYPNPVRVEAAWRILEKLQ
**SEQ ID N° 303 :** 
MMKKSLCCALLLTASFSTFAAAKTEQQIADIVNRTITPLMQEQAIPGMAVAVIYQGKP
YYFTWGKADIANNHPVTQQTLFELGSVSKTFNGVLGGDAIARGEIKLSDPVTKYWP
ELTGKQWQGIRLLHLATYTAGGLPLQIPDDVRDKAALLHFYQNWQPQWTPGAKRL
YANSSIGLFGALAVKPSGMSYEEAMTRRVLQPLKLAHTWITVPQNEQKDYARGYRE
GKPVHVSPGQLDAEAYGVKSSVIDMARWVQANMDASHVQEKTLQQGIALAQSRY
WRIGDMYQGLGWEMLNWPLKADSIINGSDSKVALAALPAVEVNPPAPAVKASWVH
KTGSTGGFGSYVAFVPEKNLGIVMLANKSYPNPVRVEAAWRILEKLQ
**SEQ ID N° 304 :** 
MMKKSLCCALLLTASFSTFAAAKTEQQIADIVNRTITPLMQEQAIPGMAVAVIYQGKP
YYFTWGKADIANNHPVTQQTLFELGSVSKTFNGVLGGDAIARGEIKLSDPVTKYWP
ELTGKQWQGIRLLHLATYTAGGLPLQIPDDVRDKAALLHFYQNWQPQWTPGAKRL
YSNSSIGLFGALAVKPSGMSYEEAMTRRVLQPLKLAHTWITVPQNEQKDYARGYRE
GKPVHVSPGQLDAEAYGVKSSVIDMARWVQANMDASHVQEKTLQQGIALAQSRY
WRIGDMYQGLGWEMLNWPLKADSIINGSDSKVALAALPAVEVNPPAPAVKASWVH
KTGSTGGFGSYVAFVPEKNLGIVMLANKNYPNPVRVEAAWRILEKLQ
**SEQ ID N° 305 :** 
MMKKSLCCALLLTASFSTFAAAKTEQQIADIVNRTITPLMQEQAIPGMAVAVIYQGKP
YYFTWGKADIANNHPVTQQTLFELGSVSKTFNGVLGGDAIARGEIKLSDPVTKYWP
ELTGKQWQGIRLLHLATYTAGGLPLQIPDDVRDKAALLHFYQNWQPQWTPGAKRL
 
YANSSIGLFGELAVKPSGMSYEEAMTRRVLQPLKLAHTWITVPQNEQKDYAWGYR
EGKPVHVSPGQLDAEAYGVKSSVIDMARWVQANMDASHVQEKTLQQGIALAQSRY
WRIGDMYQGLGWEMLNWPLKADSIINGSDSKVALAALPAVEVNPPAPAVKASWVH
KTGSTGGFGSYVAFVPEKNLGIVMLANKSYPNPVRVEAAWRILEKLQ
**SEQ ID N° 306 :** 
MMKKSLCCALLLTASFSTFAAAKTEQQIADIVNRTITPLMQEQAIPGMAVAVIYQGKP
YYFTWGKADIANNHPVTQQTLFELGSVSKTFNGVLGGDAIARGEIKLSDPVTKYWP
ELTGKQWQGIRLLHLATYTAGGLPLQIPDDVRDKAALLHFYQNWQPQWTPGAKRL
YANSSIGLFGALAVKPSGMSYEEAMTRRVLQPLKLAHTWITVPQNEQKDYALGYRE
GKPVHVSPGQLDAEAYGVKSSVIDMARWVQANMDASHVQEKTLQQGIALAQSRY
WRIGDMYQGLGWEMLNWPLKADSIINGSDSKVALAALPAVEVNPPAPAVKASWVH
KTGSTGGFGSYVAFVPEKNLGIVMLANKSYPNPVRVEAAWRILEKLQ
**SEQ ID N° 307 :** 
MMKKSLCCALLLTASFSTFAAAKTEQQIADIVNRTITPLMQEQAIPGMAVAVIYQGKP
YYFTWGKADIANNHPVTQQTLFELGSVSKTFNGVLGGDAIARGEIKFSDPVTKYWP
ELTGKQWQGIRLLHLATYTAGGLPLQIPDDVRDKAALLHFYQNWQPQWTPGAKRL
YANSSIGLFGALAVKPSGMSYEEAMTRRVLQPLKLAHTWITVPQNEQKDYAWGYR
EGKPVHVSPGQLDAEAYGVKSSVIDMARWVQANMDASHVQEKTLQQGIALAQSRY
WRIGDMYQGLGWEMLNWPLKADSIINGSDSKVALAALPAVEVNPPAPAVKASWVH
KTGSTGGFGSYVAFVPEKNLGIVMLANKSYPNPVRVEAAWRILEKLQ
**SEQ ID N° 308 :** 
MMKKSLCCALLLTASFSTFAAAKTEQQIADIVNRTITPLMQEQAIPGMAVAVIYQGKP
YYFTWGKADIANNHPVTQQTLFELGSVSKTFNGVLGGDAIARGEIKLSDPVTKYWP
ELTGKQWQGIRLLHLATYTAGGLPLQIPDDVRDKAALLHFYQNWQPQWTPGAKRL
YANSSIGLFGALAVKPSGMSYEEAMTRRVLQPLKLAHTWITVPQNEQKDYARGYRE
GKPVHVSPGQLDAEAYGVKSSVIDMARWVQANMDASHVQEKTLQQGIALAQSRY
WRIGDMYQGLGWEMLNWPLKADSIINGSDSKVALAALPAVEVNPPAPAVKASWVH
KTGSTGGFGSYVAFVPEKNLGIVMLANKNYPNPVRVEAAWRILEKLQ
**SEQ ID N° 309 :** 
MMKKSLCCALLLTASFSTFAAAKTEQQIADIVNRTITPLMQEQAIPGMAVAVIYQGKP
YYFTWGKADIANNHPVTQQTLFELGSVSKTFNGVLGGDAIARGEIKLSDPVTKYWP
ELTGKQWQGIRLLHLATYTAGGLPLQIPDDVRDKAALLHFYQNWQPQWTPGAKRL
YANSSIGLFGALAVKPSGMSYEEAMTRRVLQPLKLAHTWITVPQNEQKDYARGYRE
GKPVHVSPGQLDAEAYGVKSSVIDMARWVQANMDASHVQEKTLQQGIALAQSRY
 
WRIGDMYQGLGWEMLNWPLKADSIINGSDSKVALAALPAVEINPPAPAVKASWVHK
TGSTGGFGSYVAFVPEKNLGIVMLANKSYPNPVRVEAAWRILEKLQ
**SEQ ID N° 310 :** 
MMKKSLCCALLLTASFSTFAAAKTEQQIADIVNRTITPLMQEQAIPGMAVAVIYQGKP
YYFTWGKADIANNHPVTQQTLFELGSVSKTFNGVLGGDAIARGEIKLSDPVTKYWP
ELTGKQWQGIRLLHLATYTAGGLPLQIPDDVRDKAALLHFYQNWQPQWTPGAKRL
YSNSSIGLFGALAVKPSGMSYEEAMTRRVLQPLKLAHTWITVPQNEQKDYARGYRE
GKPVHVSPGQLDAEAYGVKSSVIDMARWVQANMDASHVQEKTLQQGIALAQSRY
WRIGDMYQGLGWEMLNWPLKADSIINGSDSKVALAALPAVEVNPPAPAVKASWVH
KTGSTGGFGSYVAFVPEKNLGIVMLANKSYPNPVRVEAAWRILEKLQ
**SEQ ID N° 311 :** 
MMKKSLCCALLLTASFSTFAAAKTEQQIADIVNRTITPLMQEQAIPGMAVAVIYQGKP
YYFTWGKADIANNHPVTQQTLFELGSVSKTFNGVLGGDAIARGEIKLSDPVTKYWP
ELTGKQWQGIRLLHLATYTAGGLPLQIPDDVRDKAALLHFYQNWQPQWTPGAKRL
YANSSIGLFGALAVKPSGMSYEEAMTRRVLQPLKLAHTWITVPQNEQKDYARGYRE
GKPVHVSPGQLNAEAYGVKSSVIDMARWVQANMDASHVQEKTLQQGIALAQSRY
WRIGDMYQGLGWEMLNWPLKADSIINGSDSKVALAALPAVEVNPPAPAVKASWVH
KTGSTGGFGSYVAFVPEKNLGIVMLANKNYPNPVRVEAAWRILEKLQ
**SEQ ID N°1844** : 
MQQRQSILWGAVATLMWAGLAHAGEASPVDPLRPVVDASIQPLLKEHRIPGMAVAV
LKDGKAHYFNYGVANRESGAGVSEQTLFEIGSVSKTLTATLGAYAVVKGAMQLDDK
ASRHAPWLKGSAFDSITMGELATYSAGGLPLQFPEEVDSSEKMRAYYRQWAPVYS
PGSHRQYSNPSIGLFGHLAASSLKQPFAPLMEQTLLPGLGMHHTYVNVPKQAMAS
YAYGYSKEDKPIRVNPGMLADEAYGIKTSSADLLRFVKANIGGVDDKALQQAISLTH
QGHYSVGGMTQGLGWESYAYPVTEQTLLAGNSAKVILEANPTAAPRESGSQVLFN
KTGSTNGFGAYVAFVPARGIGIVMLANRNYPIEARIKAAHAILAQLAG
**SEQ ID N°1845** : 
MQQRQSILWGAVATLMWAGLAHAGEASPVDPLRPVVDASIQPLLKEHRIPGMAVAV
LKDGKAHYFNYGVANRESGAGVSEQTLFEIGSVSKTLTATLGAYAVVKGAMQLDDK
ASRHAPWLKGSAFDSITMGELATYSAGGLPLQFPEEVDSSEKMRAYYRQWAPVYS
PGSHRQYSNPSIGLFGHLAASSLKQPFAPLMEQTLLPGLGMHHTYVNVPKQAMAS
YAYGYSKEDKPIRVNPGMLADEAYGIKTSSADLLRFVKANIGGVDDKALQQAISLTH
QGHYSVGGMTQGLGWESYAYPVTEQTLLAGNSAKVSLEANPTAAPRESGSQVLFN
KTGSTNGFGAYVAFVPARGIGIVMLANRNYPIEARIKAAHAILAQLAG
**SEQ ID N°1846** : 
MMKKSLCCALLLTASFSTFAAAKTEQQIADIVNRTITPLMQEQAIPGMAVAVIYQGKP
YYFTWGKADIANNHPVTQQTLFELGSVSKTFNGVLGGDAIARGEIKLSDPVTKYWP
ELTGKQWQGIRLLHLATYTAGGLPLQIPDDVRDKAALLHFYQNWQPQWTPGAKRL
YANSSIGLFGALAVKPSGMSYEEAMTRRVLQPLKLAHTWITVPQNEQKDYAWGYR
EGKPVHVSPGQLDAEAYGVKSSVIDMARWVQANMDASHVQEKTLQQGIALAQSRY
WRIGDMYQGLGWEMLNWPLKADSIINGSDSKVAFAALPAVEVNPPAPAVKASWVH
KTGSTGGFGSYVAFVPEKNLGIVMLANKSYPNPVRVEAAWRILEKLQ
**SEQ ID N°1847** : 
MMKKSLCCALLLTASFSTFAAAKTEQQIADIVNRTITPLMQEQAIPGMAVAVIYQGKP
YYFTWGKADIANNHPVTQQTLFELGSVSKTFNGVLGGDAIARGEIKLSDPVTKYWP
ELTGKQWQGIRLLHLATYTAGGLPLQIPDDVRDKAALLHFYQNWQPQWTPGAKRL
YANSSIGLFGALAVKPSGMSYEEAMTRRVLQPLKLAHTWITVPQNEQKDYAWGYR
EGKPVHVSPGQLDAEAYGVKSSVIDMARWVQANMDASHVQEKTLQQGIALAQSRY
WRIGDMYQGLGWEMLNWPLKADSIINGNGSDSKVALAALPAVEVNPPAPAVKASW
VHKTGSTGGFGSYVAFVPEKNLGIVMLANKSYPNPVRVEAAWRILEKLQ
**SEQ ID N°1848** : 
MMKKSLCCALLLTASFSTFAAAKTEQQIADIVNRTITPLMQEQAIPGMAVAVIYQGKP
YYFTWGKADIANNHPVTQQTLFELGSVSKTFNGVLGGDAIARGEIKLSDPVTKYWP
ELTGKQWQGIRLLHLATYTAGGLPLQIPDDVRDKAALLHFYQNWQPQWTPGAKRL
YANSSIGLFGALAVKPSGMSYEEAMTRRVLQPLKLAHTWITVPQNEQKDYAWGYR
EGKPVHVSPGQLDAEAYGVKSSVIDMARWVQANMDASHVQEKTLQQGIALAQSRY
WRIGDMYQGLGWEMLNWPLKADSIINGSDSKVALAALPAVEVNPPAPAVKASWVH
KTGSTGGFGSYVAFVPEKNLGIVMLANKSYPNPVRVEAYWRILEKLQ
**SEQ ID N°1849** : 
MMKKSLCCALLLTASFSTFAAAKTEQQIADIVNRTITPLMQEQAIPGMAVAVIYQGKP
YYFTWGKADIANNHPVTQQTLFELGSVSKTFNGVLGGDAIARGEIKLSDPVTKYWP
ELTGKQWQGIRLLHLATYTAGGLPLQIPDDVRDKAALLHFYQNWQPQWTPGAKRL
YANSSIGLFGELAVKPSGMSYEEAMTRRVLQPLKLAHTWITVPQNEQKDYAWGYR
EGKPVHVSPGQLDAEAYGVKSSVIDMARWVQANMDASHVQEKTLQQGIALAQSRY
WRIGDMYQGLGWEMLNWPLKADSIINGSDNKVALAALPAVEVNPPAPAVKASWVH
KTGSTGGFGSYVAFVPEKNLGIVMLANKSYPNPVRVEAAWRILEKLQ
**SEQ ID N°1850** : MMKKSLCCALLLTASFSTFAAAKTEQQIADIVNRTITPLMQEQAIPGMAVAVIYQGKP 
YYFTWGKADIANNHPVTQQTLFELGSVSKTFNGVLGGDAIARGEIKLSDPVTKYWP
ELTGKQWQGIRLLHLATYTAGGLPLQIPDDVRDKAALLHFYQNWQPQWTPGAKRL
YANSSIGLFGALAVKPSGMSYEEAMTRRVLQPLKLAHTWITVPQNEQKDYAWGYR
EGKPVHVSPGQLDAEAYGVKSSVIDMARWVQANMDASHVQEKTLQQGIALAQSRY
WRIGDMYQGLGWEMLNWPLKADSIINGSDSKVAALPAVEVNPPAPAVKASWVHKT
GSTGGFGSYVAFVPEKNLGIVMLANKSYPNPVRVEAAWRILEKLQ
**SEQ ID N°1851** : 
MMKKSLCCALLLTASFSTFAAAKTEQQIADIVNRTITPLMQEQAIPGMAVAVIYQGKP
YYFTWGKADIANNHPVTQQTLFELGSVSKTFNGVLGGDAIARGEIKLSDPVTKYWP
ELTGKQWQGIRLLHLATYTAGGLPLQIPDDVRDKAALLHFYQNWQPQWTPGAKRL
YANSSIGLFGALAVKPSGMSYEEAMTRRVLQPLKLAHTWITVPQNEQKDYAWGYR
EGKPVHVSPGQLDAEAYGVKSSVIDMARWVQANMDASHVQEKTLQQGIALAQSRY
WRIGDMYQGLGWEMLNWPLKADSIINGSDSKVAPAVEVNPPAPAVKASWVHKTGS
TGGFGSYVAFVPEKNLGIVMLANKSYPNPVRVEAAWRILEKLQ
**SEQ ID N°1852** : 
MMKKSLCCALLLTASFSTFAAAKTEQQIADIVNRTITPLMQEQAIPGMAVAVLYQGKP
YYFTWGKADIANNHPVTQQTLFELGSVSKTFNGVLGGDAIARGEIKLSDPVTKYLPE
LTGKQWQGISLLHLATYTAGGLPLQIPDDVTDKAALLRFYQNWQPQWTPGAKRLYA
NSSIGLFGALAVKPSGMSYEEAMTRRVLQPLKLAHTWITVPQNEQKDYAWGYREG
KPVHVTPGQLDAEAYGVKSNVTDMARWIQVNMDASRVQEKTLQQGIALAQSRYW
RIGDMYQGLGWEMLNWPLKADSIINGSDSKVALAALPTVEVNPPAPAVKASWVHKT
GSTGGFGSYVAFVPEKNLGIVMLANKSYPNPVRVEAAWRILEKLQ
**SEQ ID N°1853** : 
MMKKSICCALLLTASFSTFAAAKTEQQIADIVNRTITPLMQEQAIPGMAVAIIYEGKPY
YFTWGKADIANNHPVTQQTLFELGSVSKTFNGVLGGDAIARGEIKLSDPVTKYWPEL
TGKQWRGISLLHLATYTAGGLPLQIPDDVTDKAALLRFYQNWQPQWTPGAKRLYAN
SSIGLFGALAVKPSGMSYEEAMTRRVLQPLKLAHTWITVPQSEQKNYAWGYREGK
PVHVSPGQLDAEAYGVKSSVIDMARWVQANMDASHVQEKTLQQGIELAQSRYWRI
GDMYQGLGWEMLNWPLKADSIINGSDSKVALAALPAVEVNPPAPAVKASWVHKTG
STGGFGSYVAFVPEKNLGIVMLANKSYPNPARVEAAWRILEKLQ
**SEQ ID N°1854** : 
MMKKSLCCALLLTASFSTFAAAKTEQQIADIVNRTITPLMQEQAIPGMAVAVIYQGKP
YYFTWGKADIANNHPVTQQTLFELGSVSKTFNGVLGGDAIARGEIKLSDPVTKYWP
ELTGKQWQGIRLLHLATYTAGGLPLQIPDDVRDKAALLHFYQNWQPQWTPGAKRL
 
YANSSIGLFGALAVKPSGMSYEEAMTRRVLQPLKLAHTWITVPQNEQKDYAWGYR
EGKPVHVSPGQLDAEAYGVKSSVIDMARWVQANMDASHVQEKTLQQGIALAQSRY
WRIGDMYQGLGWEMLNWPLKTDSIINGSDSKVALAALPAVEVNPPAPAVKASWVH
KTGSTGGFGSYVAFVPEKNLGIVMLANKSYPNPVRVEAAWRILEKLQ
**SEQ ID N°1855** : 
MMKKSLCCALLLTASLSTFAAAKTEQQIADIVNRTITPLMQEQAIPGMAIAVIYQGKPY
YFTWGKADITNNHPVTQQTLFELGSVSKTFNGVLGGDAIARGEIKLSDPVTKYWPEL
TGKQWQGISLLHLATYTAGGLPLQIPDDVTDKAALLRFYQNWQPQWAPGAKRLYA
NSSIGLFGALAVKPSGMSYEEAMTRRVLQPLKLAHTWITVPQNEQKDYAWGYREG
KAVHVSPGQLDAEAYGVKSSVIDMARWVQVNMDASRVQEKTLQQGIALAQSRYW
RIGDMYQGLGWEMLNWPLKADSIINGSDSKVALAALPAVEVNPPAPAVKASWVHKT
GSTGGFGSYVAFVPEKNLGIVMLANKSYPNPVRVEAAWRILEKLQ
**SEQ ID N°1856** : 
MMKKSLCCALLLTASFSTFAAAKTEQQIADIVNRTITPLMQEQAIPGMAVAVIYQGKP
YYFTWGKADIANNHPVTQQTLFELGSVSKTFNGVLGGDAIARGEIKLSDPVTKYWP
ELTGKQWQGIRLLHLATYTAGGLPLQIPDDVRDKAALLHFYQNWQPQWTPGAKRL
YANSSIGLFGALAVKPSGMSYEEAMTRRVLQPLKLAHTWITVPQNEQKDYAWGYR
EGKPVHVSPGQLDAEAYCVKSSVIDMARWVQANMDASHVQEKTLQQGIALAQSRY
WRIGDMYQGLGWEMLNWPLKADSIINGSDSKVALAALPAVEVNPPAPAVKASWVH
KTGSTGGFGSYVAFVPEKNLGIVMLANKSYPNPVRVEAAWRILEKLQ
**SEQ ID N°1857** : 
MMKKSLCCALLLTASFSTFAAAKTEQQIADIVNRTITPLMQEQAIPGMAVAVIYQGKP
YYFTWGKADIANNHPVTQQTLFELGSVSKTFIGVLGGDAIARGEIKLSDPVTKYWPE
LTGKQWQGIRLLHLATYTAGGLPLQIPDDVRDKAALLHFYQNWQPQWTPGAKRLY
ANSSIGLFGALAVKPSGMSYEEAMTRRVLQPLKLAHTWITVPQNEQKDYAWGYRE
GKPVHVSPGQLDAEAYGVKSSVIDMARWVQANMDASHVQEKTLQQGIALAQSRY
WRIGDMYQGLGWEMLNWPLKADSIINGSDSKVALAALPAVEVNPPAPAVKASWVH
KTGSTGGFGSYVAFVPEKNLGIVMLANKSYPNPVRVEAAWRILEKLQ
**SEQ ID N°1858** : 
MMNRYAAALLLTASFSTFAAAKTEQQIADIVNRTITPLMQEQAIPGMAVAVIYQGKPY
YFTWGKADIANNHPVTQQTLFELGSVSKTFNGVLGGDAIARGEIKLSDPVTKYWPEL
TGKQWQGIRLLHLATYTAGGLPLQIPDDVRDKAALLHFYQNWQPQWTPGAKRLYA
NSSIGLFGALAVKPSGMSYEEAMTRRVLQPLKLAHTWITVPQNEQKDYAWGYREG
KPVHVSPGQLDAEAYGVKSSVIDMARWVQANMDASHVQEKTLQQGIALAQSRYW
 
RIGDMYQGLGWEMLNWPLKADSIINGSDSKVALAALPAVEVNPPAPAVKASWVHKT
GSTGGFGSYVAFVPEKNLGIVMLANKSYPNPVRVEAAWRILEKLQ
**SEQ ID N°1859** : 
MMKKSLCCALLLTASFSTFAAAKTEQQIADIVNRTITPLMQEQAIPGMAVAVIYQGKP
YYFTWGKADIANNHPVTQQTLFELGSVSKTFNGVLGGDAIARGEIKLSDPVTKYWP
ELTGKQWQGIRLLHLATYTAGGLPLQIPDDVRDKAALLHFYQNWQPQWTPGAKRL
YANSSIGLFSALAVKPSGMSYEEAMTRRVLQPLKLAHTWITVPQNEQKDYAWGYRE
GKPVHVSPGQLDAEAYGVKSSVIDMARWVQANMDASHVQEKTLQQGIALAQSRY
WRIGDMYQGLGWEMLNWPLKADSIINGSDSKVALAALPAVEVNPPAPAVKASWVH
KTGSTGGFGSYVAFVPEKNLGIVMLANKSYPNPVRVEAAWRILEKLQ
**SEQ ID N°1860** : 
MQQRQSILWGAVATLMWAGLAHAGEASPVDPLRPVVDASIQPLLKEHRIPGMAVAV
LKDGKAHYFNYGVANRESGASVSEQTLFDIGSVSKTLTATLGAYAVVKGAMQLDDK
ASRHAPWLKGSVFDSITMGELATYSAGGLPLQFPEEVDSSEKMRAYYRQWAPVYS
PGSHRQYSNPSIGLFGHLAASSLKQPFAQLMEQTLLPGLGMHHTYVNVPKQAMAS
YAYGYSKEDKPIRVNPGMLADEAYGIKTSSADLLAFVKANIGGVDDKALQQAISLTH
KGHYSVGGMTQGLGWESYAYPVTEQTLLAGNSAKVILEANPTAAPRESGSQVLFN
KTGSTNGFGAYVAFVPARGIGIVMLANRNYPIPARVKAAHAILAQLAG
**SEQ ID N°1861** : 
MQQRQSILWGAVATLMWAGLAHAGEASPVDPLRPVVDASIQPLLKEHRIPGMAVAV
LKDGKAHYFNYGVANRESGASVSEQTLFDIGSVSKTLTATLGAYAVVKGAMQLDDK
ASRHAPWLKGSVFDSITMGELATYSAGGLPLQFPEEVDSSEKMRAYYRQWAPVYS
PGSHRQYSNPSIGLFGHLAASSLKQPFAQLMEQTLLPGLGMHHTYVNVPKQAMAS
YAYGYSKEDKPIRVNPGMLADEAYGIKTSSADLLAFVKANIGGVDDKALQQAISLTH
KGHYSVGGMTQGLGWESYAYPVTEQTLLAGNSAKVSLEANPTAAPRESGSQVLFN
KTGSTNGFGAYVAFVPARGIGIVMLANRNYPIPARVKAAHAILAQLAG
**SEQ ID N°1862** : 
MMKKSLCCALLLTASFSTFAAAKTEQQIADIVNRTITPLMQEQAIPGMAVAVIYQGKP
YYFTWGKADIANNHPVTQQTLFELGSVSKTFNGVLGGDAIARGEIKLSDPVTKYWP
ELTGKQWQGIRLLHLATYTAGGLPLQIPDDVRDKAALLHFYQNWQPQWTPGAKRL
YANSSIGLFGALAVKPSGMSYEEAMTRRVLQPLKLAHTWITVPQNEQKDYAWGYR
EGKPVHVSPGQLDAEAYGVKSSVIDMARWVQANMDASHVQEKTLQQGIALAQSRY
WRIGDMYQGLGWEMLNWPLKADSIINGSDSKVALAALPAVEVNPPAPAVKASWVH
KTGSTGGFGSYVAFVPEKNLGIVMLANKSYPNPVRVEAAWRILEKLQ
**SEQ ID N°1863** : 
MQQRQSILWGAVATLMWAGLAHAGEASPVDPLRPVVDASIQPLLKEHRIPGMAVAV
LKDGKAHYFNYGVANRESGAGVSEQTLFEIGSVSKTLTATLGAYAVVKGAMQLDDK
ASRHAPWLKGSAFDSITMGELATYSAGGLPLQFPEEVDSSEKMRAYYRQWAPVYS
PGSHRQYSNPSIGLFGHLAASSLKQPFAPLMEQTLLPGLGMHHTYVNVPKQAMAS
YAYGYSKEDKPIRVNPGMLADEAYGIKTSSADLLRFVKANIGGVDDKALQQAISLTH
QGHYSVGGMTQGLGWESYAYPVTEQTLLAGNSAKVILEANPTAAPRESGSQVLFN
KTGSTNGFGAYVAFVPARGIGIVMLANRNYPNEARIKAAHAILAQLAG
**SEQ ID N°1864** : 
MMKKSLCCALLLTASFSTFAAAKTEQQIADIVNRTITPLMQEQAIPGMAVAVIYQGKP
YYFTWGKADIANNHPVTQQTLFELGSVSKTFNGVLGGDAIARGEIKLSDPVTKYWP
ELTGKQWQGIRLLHLATYTAGGLPLQIPDDVRDKAALLHFYQNWQPQWTPGAKRL
YANSSIGLFGALAVKPSGMSYEEAMTRRVLQPLKLAHTWITVPQNEQKDYARGYRE
GKPVHVSPGQLDAEAYGVKSSVIDMARWVQANMDASHVQEKTLQQGIALAQSRY
WRIGDMYQGLGWEMLNWPLKADSIINGSDSKVALAALPAVEVNPPAPAVKASWVH
KTGSTGGFGSYVAFVPEKNLGIVMLANKSYPNPVRVEAAWRILEKLQ
**SEQ ID N°1865** : 
MMKKSLCCALLLTASFSTFAAAKTEQQIADIVNRTITPLMQEQAIPGMAVAVIYQGKP
YYFTWGKADIANNHPVTQQTLFELGSVSKTFNGVLGGDAIARGEIKLSDPVTKYWP
ELTGKQWQGIRLLHLATYTAGGLPLQIPDDVRDKAALLHFYQNWQPQWTPGAKRL
YSNSSIGLFGALAVKPSGMSYEEAMTRRVLQPLKLAHTWITVPQNEQKDYARGYRE
GKPVHVSPGQLDAEAYGVKSSVIDMARWVQANMDASHVQEKTLQQGIALAQSRY
WRIGDMYQGLGWEMLNWPLKADSIINGSDSKVALAALPAVEVNPPAPAVKASWVH
KTGSTGGFGSYVAFVPEKNLGIVMLANKNYPNPVRVEAAWRILEKLQ
**SEQ ID N°1866** : 
MMKKSLCCALLLTASFSTFAAAKTEQQIADIVNRTITPLMQEQAIPGMAVAVIYQGKP
YYFTWGKADIANNHPVTQQTLFELGSVSKTFNGVLGGDAIARGEIKLSDPVTKYWP
ELTGKQWQGIRLLHLATYTAGGLPLQIPDDVRDKAALLHFYQNWQPQWTPGAKRL
YANSSIGLFGELAVKPSGMSYEEAMTRRVLQPLKLAHTWITVPQNEQKDYAWGYR
EGKPVHVSPGQLDAEAYGVKSSVIDMARWVQANMDASHVQEKTLQQGIALAQSRY
WRIGDMYQGLGWEMLNWPLKADSIINGSDSKVALAALPAVEVNPPAPAVKASWVH
KTGSTGGFGSYVAFVPEKNLGIVMLANKSYPNPVRVEAAWRILEKLQ
**SEQ ID N°1867** : MMKKSLCCALLLTASFSTFAAAKTEQQIADIVNRTITPLMQEQAIPGMAVAVIYQGKP 
YYFTWGKADIANNHPVTQQTLFELGSVSKTFNGVLGGDAIARGEIKLSDPVTKYWP
ELTGKQWQGIRLLHLATYTAGGLPLQIPDDVRDKAALLHFYQNWQPQWTPGAKRL
YANSSIGLFGALAVKPSGMSYEEAMTRRVLQPLKLAHTWITVPQNEQKDYARGYRE
GKPVHVSPGQLDAEAYGVKSSVIDMARWVQANMDASHVQEKTLQQGIALAQSRY
WRIGDMYQGLGWEMLNWPLKADSIINGSDSKVALAALPAVEVNPPAPAVKASWVH
KTGSTGGFGSYVAFVPEKNLGIVMLANKNYPNPVRVEAAWRILEKLQ
**SEQ ID N°1868** : 
MMKKSLCCALLLTASFSTFAAAKTEQQIADIVNRTITPLMQEQAIPGMAVAVIYQGKP
YYFTWGKADIANNHPVTQQTLFELGSVSKTFNGVLGGDAIARGEIKLSDPVTKYWP
ELTGKQWQGIRLLHLATYTAGGLPLQIPDDVRDKAALLHFYQNWQPQWTPGAKRL
YANSSIGLFGALAVKPSGMSYEEAMTRRVLQPLKLAHTWITVPQNEQKDYARGYRE
GKPVHVSPGQLDAEAYGVKSSVIDMARWVQANMDASHVQEKTLQQGIALAQSRY
WRIGDMYQGLGWEMLNWPLKADSIINGSDSKVALAALPAVEINPPAPAVKASWVHK
TGSTGGFGSYVAFVPEKNLGIVMLANKSYPNPVRVEAAWRILEKLQ
**SEQ ID N°1869** : 
MMKKSLCCALLLTASFSTFAAAKTEQQIADIVNRTITPLMQEQAIPGMAVAVIYQGKP
YYFTWGKADIANNHPVTQQTLFELGSVSKTFNGVLGGDAIARGEIKLSDPVTKYWP
ELTGKQWQGIRLLHLATYTAGGLPLQIPDDVRDKAALLHFYQNWQPQWTPGAKRL
YSNSSIGLFGALAVKPSGMSYEEAMTRRVLQPLKLAHTWITVPQNEQKDYARGYRE
GKPVHVSPGQLDAEAYGVKSSVIDMARWVQANMDASHVQEKTLQQGIALAQSRY
WRIGDMYQGLGWEMLNWPLKADSIINGSDSKVALAALPAVEVNPPAPAVKASWVH
KTGSTGGFGSYVAFVPEKNLGIVMLANKSYPNPVRVEAAWRILEKLQ
**SEQ ID N°1870** : 
MMKKSLCCALLLTASFSTFAAAKTEQQIADIVNRTITPLMQEQAIPGMAVAVIYQGKP
YYFTWGKADIANNHPVTQQTLFELGSVSKTFNGVLGGDAIARGEIKLSDPVTKYWP
ELTGKQWQGIRLLHLATYTAGGLPLKIPDDVRDKAALLHFYQNWQPQWTPGAKRLY
SNSSIGLFGALAVKPSGMSYEEAMTRRVLQPLKLAHTWITVPQNEQKDYARGYREG
KPVHVSPGQLDAEAYGVKSSVIDMARWVQANMDASHVQEKTLQQGIALAQSRYW
RIGDMYQGLGWEMLNWPLKADSIINGSDSKVALAALPAVEVNPPAPAVKASWVHKT
GSTVGFGSYVAFVPEKNLGIVMLANKSYPNPVRVEAAWRILEKLQ
lesdits peptides étant choisis, de préférence, parmi les peptides de séquence **SEQ ID N°312** à **SEQ ID N°350, SEQ ID N°734, SEQ ID N°735** et **SEQ ID N°1929 à** et **SEQ ID N°2007,** tels que définis ci-après :

| **Peptide SEQ ID N°** | Séquence d'acides aminés | **Position du peptide dans la protéine CMY** |
|---|---|---|
| **SEQ ID N°312** | AALLHFYQNWQPQWTPGAK | 149-167 pour les protéines de SEQ N°266, 267, 269, 270, 271, 272, 273, 274, 275, 276, 277, 278, 279, 280, 281, 283, 284, 285, 286, 289, 290, 291, 292, 300, 302, 303, 304, 305, 306, 307, 308, 309, 310, 311, 1846, 1847, 1848, 1849, 1850, 1851, 1854, 1856, 1857, 1859, 1862, 1864, 1865, 1866, 1867, 1868, 1869, 1870; 148-166 pour la protéine de séquence SEQ ID N°268; 163-181 pour la protéine de séquence SEQ ID N°299; 148-166 pour la protéine de séquence SEQ ID N°1858 |
| **SEQ ID N°313** | ADIANNHPVTQQTLFELGSVS K | 66-87 pour les protéines de SEQ N°265, 266, 267, 269, 270, 271, 272, 273, 274, 275, 276, 277, 278, 279, 280, 281, 282, 283, 284, 285, 286, 287, 288, 289, 290, 291, 292, 295, 296, 298, 300, 302, 303, 304, 305, 306, 307, 308, 309, 310, 311, 1846, 1847, 1848, 1849, 1850, 1851, 1852, 1853, 1854, 1856, 1857, 1859, 1862, 1864, 1865, 1866, 1867, 1868, 1869, 1870; 65-86 pour la protéine de séquence SEQ ID N°268; 80-101 pour la protéine de séquence SEQ ID N°299; 65-86 pour la protéine de séquence SEQ ID N°1858 |
| **SEQ ID N°314** | ADSIINGSDSK | 300-310 pour les protéines de SEQ N°265, 266, 269, 270, 271, 272, 273, 274, 276, 277, 278, 279, 280, 281, 282, 283, 284, 285, 286, 287, 288, 290, 291, 295, 296, 297, 298, 300, 302, 303, 304, 305, 306, 307, 308, 309, 310, 311, 1846, 1848, 1850, 1851, 1852, 1853, 1855, 1856, 1857, 1859, 1862, 1864, 1865, 1866, 1867, 1868, 1869, 1870; 302-312 pour la protéine de séquence SEQ ID N°289; 314-324 pour la protéine de séquence SEQ ID N°299; 299-309 pour la protéine de séquence SEQ ID N°1858 |
| **SEQ ID N°315** | ASWVHK | 330-335 pour les protéines de SEQ N°265, 266, 269, 270, 271, 272, 273, 274, 275, 276, 277, 278, 279, 280, 281, 282, 283, 285, 287, 288, 290, 291, 292, 295, 296, 297, 298, 300, 302, 303, 304, 305, 306, 307, 308, 309, 310, 311, 1846, 1848, 1849, 1852, 1853, 1854, 1855, 1856, 1857, 1859, 1862, 1864, 1865, 1866, 1867, 1868, 1869, 1870; 332-337 pour la protéine de séquence SEQ ID N°267; 329-334 pour la protéine de séquence SEQ ID N°268; 328-333 pour la protéine de séquence SEQ ID N°284; 326-331 pour la protéine de séquence SEQ ID N°286; 332-337 pour la protéine de séquence SEQ ID N°289; 344-349 pour la protéine de séquence SEQ ID N°299; 332-337 pour la protéine de séquence SEQ ID N°1847; 328-333 pour la protéine de séquence SEQ ID N°1850; 326-331 pour la protéine de séquence SEQ ID N°1851; 329-334 pour la protéine de séquence SEQ ID N°1858 |
| **SEQ ID N°316** | DYAWGYR | 218-224 pour les protéines de SEQ N°265, 266, 267, 269, 270, 271, 272, 273, 274, 275, 276, 277, 278, 279, 280, 282, 283, 284, 285, 286, 287, 289, 290, 291, 292, 295, 296, 297, 300, 305, 307, 1846, 1847, 1848, 1849, 1850, 1851, 1852, 1854, 1855, 1856, 1857, 1859, 1862, 1866; 217-223 pour la protéine de séquence SEQ ID N°268; 232-238 pour la protéine de séquence SEQ ID N°299; 217-223 pour la protéine de séquence SEQ ID N°1858 |
| **SEQ ID N°317** | IGDMYQGLGWEMLNWPLK | 282-299 pour les protéines de SEQ N°265, 266, 267, 269, 270, 271, 272, 273, 274, 275, 276, 277, 278, 279, 280, 281, 282, 283, 284, 285, 286, 287, 288, 290, 291, 292, 295, 296, 297, 298, 300, 302, 303, 304, 305, 306, 307, 308, 309, 310, 311, 1846, 1847, 1848, 1849, 1850, 1851, 1852, 1853, 1854, 1855, 1856, 1857, 1859, 1862, 1864, 1865, 1866, 1867, 1868, 1869, 1870; 281298 pour la protéine de séquence SEQ ID N°268; 284-301 pour la protéine de séquence SEQ ID N°289; 296-313 pour la protéine de séquence SEQ ID N°299; 281-298 pour la protéine de séquence SEQ ID N°1858 |
| **SEQ ID N°318** | LAHTWITVPQNEQK | 204-217 pour les protéines de SEQ N°265, 266, 267, 269, 270, 271, 272, 273, 274, 275, 276, 277, 278, 280, 281, 283, 284, 285, 286, 287, 289, 290, 291, 292, 300, 302, 303, 304, 305, 306, 307, 308, 309, 310, 311, 1846, 1847, 1848, 1849, 1850, 1851, 1852, 1854, 1855, 1856, 1857, 1859, 1862, 1864, 1865, 1866, 1867, 1868, 1869, 1870; 203-216 pour la protéine de séquence SEQ ID N°268; 218-231 pour la protéine de séquence SEQ ID N°299; 203-216 pour la protéine de séquence SEQ ID N°1858 |
| **SEQ ID N°319** | LLHLATYTAGGLPLQIPDDVR | 126-146 pour les protéines de SEQ N°266, 267, 269, 270, 271, 273, 274, 275, 276, 278, 279, 281, 283, 284, 285, 286, 289, 290, 291, 292, 300, 302, 303, 304, 305, 306, 307, 308, 309, 310, 311, 1846, 1847, 1848, 1849, 1850, 1851, 1854, 1856, 1857, 1859, 1862, 1864, 1865, 1866, 1867, 1868, 1869; 140-160 pour la protéine de séquence SEQ ID N°299; 125-145 pour la protéine de séquence SEQ ID N°1858 |
| **SEQ ID N°320** | LSDPVTK | 105-111 pour les protéines de SEQ N°265, 266, 267, 269, 270, 271, 272, 273, 274, 275, 276, 277, 278, 279, 280, 281, 282, 283, 284, 285, 286, 287, 288, 289, 290, 291, 292, 295, 296, 297, 298, 300, 302, 303, 304, 305, 306, 308, 309, 310, 311, 1846, 1847, 1848, 1849, 1850, 1851, 1852, 1853, 1854, 1855, 1856, 1857, 1859, 1862, 1864, 1865, 1866, 1867, 1868, 1869, 1870; 104-110 pour la protéine de séquence SEQ ID N°268; 119-125 pour la protéine de séquence SEQ ID N°299; 104-110 pour la protéine de séquence SEQ ID N°1858 |
| **SEQ ID N°321** | LYANSSIGLFGALAVK | 169-184 pour les protéines de SEQ N°265, 266, 267, 270, 271, 272, 273, 274, 276, 278, 279, 280, 281, 283, 284, 286, 287, 289, 290, 291, 292, 295, 296, 297, 298, 300, 302, 303, 306, 307, 308, 309, 311, 1846, 1847, 1848, 1850, 1851, 1852, 1853, 1854, 1855, 1856, 1857, 1862, 1864, 1867, 1868; 168-183 pour la protéine de séquence SEQ ID N°268; 183-198 pour la protéine de séquence SEQ ID N°299; 168-183 pour la protéine de séquence SEQ ID N°1858 |
| **SEQ ID N°322** | NLGIVMLANK | 353-362 pour les protéines de SEQ N°265, 266, 269, 270, 271, 272, 273, 274, 275, 276, 277, 278, 279, 280, 281, 282, 283, 285, 287, 288, 290, 291, 292, 295, 296, 297, 298, 300, 302, 303, 304, 305, 306, 307, 308, 309, 310, 311, 1846, 1848, 1849, 1852, 1853, 1854, 1855, 1856, 1857, 1859, 1862, 1864, 1865, 1866, 1867, 1868, 1869, 1870; 355-364 pour la protéine de séquence SEQ ID N°267; 352-361 pour la protéine de séquence SEQ ID N°268; 351-360 pour la protéine de séquence SEQ ID N°284; 349-358 pour la protéine de séquence SEQ ID N°286; 355-364 pour la protéine de séquence SEQ ID N°289; 367-376 pour la protéine de séquence SEQ ID N°299; 355-364 pour la protéine de séquence SEQ ID N°1847; 351-360 pour la protéine de séquence SEQ ID N°1850; 349-358 pour la protéine de séquence SEQ ID N°1851; 352-361 pour la protéine de séquence SEQ ID N°1858 |
| **SEQ ID N°323** | QWQGIR | 120-125 pour les protéines de SEQ N°266, 267, 269, 270, 271, 273, 274, 275, 276, 277, 278, 279, 280, 281, 283, 284, 285, 286, 289, 290, 291, 292, 300, 302, 303, 304, 305, 306, 307, 308, 309, 310, 311, 1846, 1847, 1848, 1849, 1850, 1851, 1854, 1856, 1857, 1859, 1862, 1864, 1865, 1866, 1867, 1868, 1869, 1870; 134-139 pour la protéine de séquence SEQ ID N°299; 119-124 pour la protéine de séquence SEQ ID N°1858 |
| **SEQ ID N°324** | SLCCALLLTASFSTFAAAK | 5-23 pour les protéines de SEQ N°266, 267, 269, 270, 271, 272, 273, 274, 275, 276, 277, 278, 279, 280, 281, 282, 283, 284, 285, 286, 287, 289, 290, 291, 292, 295, 300, 302, 303, 304, 305, 306, 307, 308, 309, 310, 311, 1846, 1847, 1848, 1849, 1850, 1851, 1852, 1854, 1856, 1857, 1859, 1862, 1864, 1865, 1866, 1867, 1868, 1869, 1870; 4-22 pour la protéine de séquence SEQ ID N°268; 19-37 pour la protéine de séquence SEQ ID N°299 |
| **SEQ ID N°325** | SSVIDMAR | 245-252 pour les protéines de SEQ N°266, 267, 269, 270, 271, 272, 273, 274, 275, 276, 277, 278, 279, 280, 281, 283, 284, 285, 286, 288, 290, 291, 292, 295, 297, 298, 300, 302, 303, 304, 305, 306, 307, 308, 309, 310, 311, 1846, 1847, 1848, 1849, 1850, 1851, 1853, 1854, 1855, 1856, 1857, 1859, 1862, 1864, 1865, 1866, 1867, 1868, 1869, 1870; 244-251 pour la protéine de séquence SEQ ID N°268; 247-254 pour la protéine de séquence SEQ ID N°289; 259-266 pour la protéine de séquence SEQ ID N°299; 244-251 pour la protéine de séquence SEQ ID N°1858 |
| **SEQ ID N°326** | SYPNPVR | 363-369 pour les protéines de SEQ N°265, 266, 269, 270, 271, 272, 273, 274, 275, 276, 277, 278, 279, 280, 281, 282, 283, 285, 287, 290, 291, 292, 296, 297, 300, 302, 303, 305, 306, 307, 309, 310, 1846, 1848, 1849, 1852, 1854, 1855, 1856, 1857, 1859, 1862, 1864, 1866, 1868, 1869, 1870; 365-371 pour la protéine de séquence SEQ ID N°267; 362-368 pour la protéine de séquence SEQ ID N°268; 361-367 pour la protéine de séquence SEQ ID N°284; 359-365 pour la protéine de séquence SEQ ID N°286; 365-371 pour la protéine de séquence SEQ ID N°289; 377-383 pour la protéine de séquence SEQ ID N°299; 365-371 pour la protéine de séquence SEQ ID N°1847; 361-367 pour la protéine de séquence SEQ ID N°1850; 359-365 pour la protéine de séquence SEQ ID N°1851; 362-368 pour la protéine de séquence SEQ ID N°1858 |
| **SEQ ID N°327** | TEQQIADIVNR | 24-34 pour les protéines de SEQ N°265, 266, 267, 269, 270, 271, 272, 273, 274, 275, 276, 277, 278, 279, 280, 281, 282, 283, 284, 285, 286, 287, 288, 289, 290, 291, 292, 295, 296, 297, 298, 300, 302, 303, 304, 305, 306, 307, 308, 309, 310, 311, 1846, 1847, 1848, 1849, 1850, 1851, 1852, 1853, 1854, 1855, 1856, 1857, 1859, 1862, 1864, 1865, 1866, 1867, 1868, 1869, 1870; 23-33 pour la protéine de séquence SEQ ID N°268; 38-48 pour la protéine de séquence SEQ ID N°299; 23-33 pour la protéine de séquence SEQ ID N°1858 |
| **SEQ ID N°328** | TFNGVLGGDAIAR | 88-100 pour les protéines de SEQ N°265, 266, 267, 269, 270, 271, 272, 273, 274, 275, 276, 277, 278, 280, 281, 282, 283, 284, 285, 286, 287, 288, 289, 291, 292, 295, 296, 297, 298, 300, 302, 303, 304, 305, 306, 307, 308, 309, 310, 311, 1846, 1847, 1848, 1849, 1850, 1851, 1852, 1853, 1854, 1855, 1856, 1859, 1862, 1864, 1865, 1866, 1867, 1868, 1869, 1870; 87-99 pour la protéine de séquence SEQ ID N°268; 102-114 pour la protéine de séquence SEQ ID N°299; 87-99 pour la protéine de séquence SEQ ID N°1858 |
| **SEQ ID N°329** | TGSTGGFGSYVAFVPEK | 336-352 pour les protéines de SEQ N°265, 266, 269, 270, 271, 272, 273, 274, 275, 276, 277, 278, 279, 280, 281, 283, 285, 288, 290, 291, 292, 295, 296, 297, 298, 300, 302, 303, 304, 305, 306, 307, 308, 309, 310, 311, 1846, 1848, 1849, 1852, 1853, 1854, 1855, 1856, 1857, 1859, 1862, 1864, 1865, 1866, 1867, 1868, 1869; 338-354 pour la protéine de séquence SEQ ID N°267; 335-351 pour la protéine de séquence SEQ ID N°268; 334-350 pour la protéine de séquence SEQ ID N°284; 332-348 pour la protéine de séquence SEQ ID N°286; 338-354 pour la protéine de séquence SEQ ID N°289; 350-366 pour la protéine de séquence SEQ ID N°299; 338-354 pour la protéine de séquence SEQ ID N°1847; 334-350 pour la protéine de séquence SEQ ID N°1850; 332-348 pour la protéine de séquence SEQ ID N°1851; 335-351 pour la protéine de séquence SEQ ID N°1858 |
| **SEQ ID N°330** | TITPLMQEQAIPGMAVAVIYQ GK | 35-57 pour les protéines de SEQ N°266, 267, 269, 270, 271, 272, 273, 274, 275, 276, 277, 278, 279, 280, 281, 282, 283, 284, 285, 286, 289, 290, 291, 292, 295, 296, 297, 300, 302, 303, 304, 305, 306, 307, 308, 309, 310, 311, 1846, 1847, 1848, 1849, 1850, 1851, 1854, 1856, 1857, 1859, 1862, 1864, 1865, 1866, 1867, 1868, 1869, 1870; 34-56 pour la protéine de séquence SEQ ID N°268; 49-71 pour la protéine de séquence SEQ ID N°299; 34-56 pour la protéine de séquence SEQ ID N°1858 |
| **SEQ ID N°331** | TLQQGIALAQSR | 267-278 pour les protéines de SEQ N°265, 266, 267, 269, 270, 271, 272, 273, 274, 275, 276, 277, 278, 279, 280, 281, 283, 284, 285, 286, 287, 290, 291, 292, 295, 296, 297, 300, 302, 303, 304, 305, 306, 307, 308, 309, 310, 311, 1846, 1847, 1848, 1849, 1850, 1851, 1852, 1854, 1855, 1856, 1857, 1859, 1862, 1864, 1865, 1866, 1867, 1868, 1869, 1870; 266-277 pour la protéine de séquence SEQ ID N°268; 269-280 pour la protéine de séquence SEQ ID N°289; 281-292 pour la protéine de séquence SEQ ID N°299; 266-277 pour la protéine de séquence SEQ ID N°1858 |
| **SEQ ID N°332** | VALAALPAVEVNPPAPAVK | 311-329 pour les protéines de SEQ N°265, 269, 270, 271, 272, 273, 274, 275, 276, 277, 278, 279, 280, 281, 282, 283, 285, 288, 290, 291, 292, 296, 297, 298, 300, 302, 303, 304, 305, 306, 307, 308, 310, 311, 1848, 1849, 1853, 1854, 1855, 1856, 1857, 1859, 1862, 1864, 1865, 1866, 1867, 1869, 1870; 313-331 pour la protéine de séquence SEQ ID N°267; 310-328 pour la protéine de séquence SEQ ID N°268; 313-331 pour la protéine de séquence SEQ ID N°289; 325-343 pour la protéine de séquence SEQ ID N°299; 313-331 pour la protéine de séquence SEQ ID N°1847; 310-328 pour la protéine de séquence SEQ ID N°1858 |
| **SEQ ID N°333** | VEAAWR | 370-375 pour les protéines de SEQ N°265, 266, 269, 271, 272, 273, 274, 275, 276, 277, 278, 279, 280, 281, 282, 283, 285, 287, 288, 290, 291, 292, 295, 296, 297, 298, 300, 302, 303, 304, 305, 306, 307, 308, 309, 310, 311, 1846, 1849, 1852, 1853, 1854, 1855, 1856, 1857, 1859, 1862, 1864, 1865, 1866, 1867, 1868, 1869, 1870; 372-377 pour la protéine de séquence SEQ ID N°267; 369-374 pour la protéine de séquence SEQ ID N°268; 368-373 pour la protéine de séquence SEQ ID N°284; 366-371 pour la protéine de séquence SEQ ID N°286; 372-377 pour la protéine de séquence SEQ ID N°289; 384-389 pour la protéine de séquence SEQ ID N°299; 372-377 pour la protéine de séquence SEQ ID N°1847; 368-373 pour la protéine de séquence SEQ ID N°1850; 366-371 pour la protéine de séquence SEQ ID N°1851; 369-374 pour la protéine de séquence SEQ ID N°1858 |
| **SEQ ID N°334** | VLQPLK | 198-203 pour les protéines de SEQ N°265, 266, 267, 269, 270, 271, 272, 273, 274, 275, 276, 277, 278, 279, 280, 281, 282, 283, 284, 285, 286, 287, 288, 289, 290, 291, 292, 295, 296, 297, 298, 300, 302, 303, 304, 305, 306, 307, 308, 309, 310, 311, 1846, 1847, 1848, 1849, 1850, 1851, 1852, 1853, 1854, 1855, 1856, 1857, 1859, 1862, 1864, 1865, 1866, 1867, 1868, 1869, 1870; 197-202 pour la protéine de séquence SEQ ID N°268; 212-217 pour la protéine de séquence SEQ ID N°299; 197-202 pour la protéine de séquence SEQ ID N°1858 |
| **SEQ ID N°335** | WVQANMDASHVQEK | 253-266 pour les protéines de SEQ N°266, 267, 269, 270, 271, 272, 274, 275, 276, 277, 278, 279, 280, 281, 282, 283, 284, 285, 286, 288, 290, 291, 292, 298, 300, 302, 303, 304, 305, 306, 307, 308, 309, 310, 311, 1846, 1847, 1848, 1849, 1850, 1851, 1853, 1854, 1856, 1857, 1859, 1862, 1864, 1865, 1866, 1867, 1868, 1869, 1870; 252-265 pour la protéine de séquence SEQ ID N°268; 255-268 pour la protéine de séquence SEQ ID N°289; 267-280 pour la protéine de séquence SEQ ID N°299; 252-265 pour la protéine de séquence SEQ ID N°1858 |
| **SEQ ID N°336** | YWPELTGK | 112-119 pour les protéines de SEQ N°265, 266, 267, 269, 270, 271, 272, 273, 274, 275, 276, 277, 278, 279, 280, 281, 282, 283, 284, 285, 286, 287, 288, 289, 290, 291, 292, 296, 297, 298, 300, 302, 303, 304, 305, 306, 307, 308, 309, 310, 311, 1846, 1847, 1848, 1849, 1850, 1851, 1853, 1854, 1855, 1856, 1857, 1859, 1862, 1864, 1865, 1866, 1867, 1868, 1869, 1870; 111-118 pour la protéine de séquence SEQ ID N°268; 126-133 pour la protéine de séquence SEQ ID N°299; 111-118 pour la protéine de séquence SEQ ID N°1858 |
| **SEQ ID N°337** | AHYFNYGVANR | 62-72 pour les protéines de SEQ N°262, 263, 264, 293, 294, 301, 1844, 1845, 1860, 1861, 1863 |
| **SEQ ID N°338** | ANIGGVDDK | 261-269 pour les protéines de SEQ N°262, 263, 264, 293, 294, 301, 1844, 1845, 1860, 1861, 1863 |
| **SEQ ID N°339** | ESGSQVLFNK | 326-335 pour les protéines de SEQ N°262, 263, 264, 293, 294, 301, 1844, 1845, 1860, 1861, 1863 |
| **SEQ ID N°340** | GAMQLDDK | 105-112 pour les protéines de SEQ N°262, 263, 264, 293, 294, 301, 1844, 1845, 1860, 1861, 1863 |
| **SEQ ID N°341** | GIGIVMLANR | 353-362 pour les protéines de SEQ N°262, 263, 264, 293, 294, 301, 1844, 1845, 1860, 1861, 1863 |
| **SEQ ID N°342** | HAPWLK | 116-121 pour les protéines de SEQ N°262, 263, 264, 293, 294, 301, 1844, 1845, 1860, 1861, 1863 |
| **SEQ ID N°343** | IPGMAVAVLK | 49-58 pour les protéines de SEQ N°262, 263, 264, 293, 294, 301, 1844, 1845, 1860, 1861, 1863 |
| **SEQ ID N°344** | PWDASIQPLLK | 34-45 pour les protéines de SEQ N°262, 263, 264, 293, 294, 301,1844,1845,1860,1861,1863 |
| **SEQ ID N°345** | QAMASYAYGYSK | 218-229 pour les protéines de SEQ N°262, 263, 264, 293, 294, 301, 1844, 1845, 1860, 1861, 1863 |
| **SEQ ID N°346** | QWAPVYSPGSHR | 161-172 pour les protéines de SEQ N°262, 263, 264, 293, 294, 301, 1844, 1845, 1860, 1861, 1863 |
| **SEQ ID N°347** | QYSNPSIGLFGHLAASSLK | 173-191 pour les protéines de SEQ N°262, 263, 264, 293, 294, 301, 1844, 1845, 1860, 1861, 1863 |
| **SEQ ID N°348** | TGSTNGFGAYVAFVPAR | 336-352 pour les protéines de SEQ N°262, 263, 264, 293, 294, 301, 1844, 1845, 1860, 1861, 1863 |
| **SEQ ID N°349** | TLTATLGAYAVVK | 92-104 pour les protéines de SEQ N°262, 263, 264, 293, 294, 301, 1844, 1845, 1860, 1861, 1863 |
| **SEQ ID N°350** | VNPGMLADEAYGIK | 236-249 pour les protéines de SEQ N°262, 263, 264, 293, 294, 301, 1844, 1845, 1860, 1861, 1863 |
| **SEQ ID N°734** | PSGMSYEEAMTR | 185-196 pour les protéines de SEQ N°265, 266, 267, 269, 270, 271, 272, 273, 274, 275, 276, 277, 278, 279, 280, 281, 282, 283, 284, 285, 286, 287, 288, 289, 290, 291, 292, 295, 296, 297, 298, 300, 302, 303, 304, 305, 306, 307, 308, 309, 310, 311, 1846, 1847, 1848, 1849, 1850, 1851, 1852, 1853, 1854, 1855, 1856, 1857, 1859, 1862, 1864, 1865, 1866, 1867, 1868, 1869, 1870; 184-195 pour la protéine de séquence SEQ ID N°268; 199-210 pour la protéine de séquence SEQ ID N°299; 184-195 pour la protéine de séquence SEQ ID N°1858 |
| **SEQ ID N°735** | PYYFTWGK | 58-65 pour les protéines de SEQ N°265, 266, 267, 269, 270, 271, 272, 273, 274, 275, 276, 277, 278, 279, 280, 281, 282, 283, 284, 285, 286, 287, 288, 289, 290, 291, 292, 295, 297, 298, 300, 302, 303, 304, 305, 306, 307, 308, 309, 310, 311, 1846, 1847, 1848, 1849, 1850, 1851, 1852, 1853, 1854, 1855, 1856, 1857, 1859, 1862, 1864, 1865, 1866, 1867, 1868, 1869, 1870; 57-64 pour la protéine de séquence SEQ ID N°268; 72-79 pour la protéine de séquence SEQ ID N°299; 57-64 pour la protéine de séquence SEQ ID N°1858 |
| **SEQ ID N°1929** | AALLR | 149-153 pour les protéines de SEQ N°265, 282, 287, 297, 298, 1852, 1853, 1855 |
| **SEQ ID N°1930** | ADSIINGNGSDSK | 300-312 pour les protéines de SEQ N°267, 1847 |
| **SEQ ID N°1931** | ADSIINGSDNK | 300-310 pour les protéines de SEQ N°275, 1849; 299-309 pour la protéine de séquence SEQ ID N°268 |
| **SEQ ID N°1932** | AELLR | 149-153 pour la protéine de SEQ N°288 |
| **SEQ ID N°1933** | ALQQAISLTHK | 270-280 pour les protéines de SEQ N°262, 293, 294, 1860, 1861 |
| **SEQ ID N°1934** | AVHVSPGQLDAEAYGVK | 228-244 pour les protéines de SEQ N°273, 297, 1855 |
| **SEQ ID N°1935** | DYACGYR | 218-224 pour la protéine de SEQ N°281 |
| **SEQ ID N°1936** | DYALGYR | 218-224 pour la protéine de SEQ N°306 |
| **SEQ ID N°1937** | EDKPIR | 230-235 pour les protéines de SEQ N°262, 263, 264, 293, 294, 301, 1844, 1845, 1860, 1861, 1863 |
| **SEQ ID N°1938** | EGKPVHASPGQLDAEAYGVK | 239-258 pour la protéine de SEQ N°299 |
| **SEQ ID N°1939** | EGKPVHGSPGQLDAEAYGVK | 225-244 pour la protéine de SEQ N°278 |
| **SEQ ID N°1940** | EGKPVHVSPEQLDAEAYGVK | 225-244 pour la protéine de SEQ N°283 |
| **SEQ ID N°1941** | EGKPVHVSPGK | 225-235 pour la protéine de SEQ N°269 |
| **SEQ ID N°1942** | EGKPVHVSPGQFDAEAYGVK | 225-244 pour la protéine de SEQ N°291 |
| **SEQ ID N°1943** | EGKPVHVSPGQLDAEAYCVK | 225-244 pour les protéines de SEQ N°1856 |
| **SEQ ID N°1944** | EGKPVHVSPGQLDAEAYGVK | 225-244 pour les protéines de SEQ N°265, 266, 267, 270, 272, 275, 277, 279, 280, 281, 282, 284, 285, 286, 288, 290, 292, 295, 296, 298, 300, 302, 303, 304, 305, 306, 307, 308, 309, 310, 1846, 1847, 1848, 1849, 1850, 1851, 1853, 1854, 1857, 1859, 1862, 1864, 1865, 1866, 1867, 1868, 1869, 1870; 224-243 pour la protéine de séquence SEQ ID N°268; 224-243 pour la protéine de séquence SEQ ID N°1858 |
| **SEQ ID N°1945** | EGKPVHVSPGQLDAGAYGVK | 225-244 pour la protéine de SEQ N°271 |
| **SEQ ID N°1946** | EGKPVHVSPGQLNAEAYGVK | 225-244 pour la protéine de SEQ N°311 |
| **SEQ ID N°1947** | EGKPVHVSPGR | 225-235 pour la protéine de SEQ N°276 |
| **SEQ ID N°1948** | EGKPVHVTPGQLDAEAYGVK | 225-244 pour les protéines de SEQ N°287, 1852 |
| **SEQ ID N°1949** | EGKPVYVSPGQLDAEAYGVK | 225-244 pour la protéine de SEQ N°274 |
| **SEQ ID N°1950** | ESGAGVSEQTLFEIGSVSK | 73-91 pour les protéines de SEQ N°263, 264, 301, 1844, 1845, 1863 |
| **SEQ ID N°1951** | ESGASVSEQTLFDIGSVSK | 73-91 pour les protéines de SEQ N°293, 294, 1860, 1861 |
| **SEQ ID N°1952** | ESGASVSEQTLFEIGSVSK | 73-91 pour la protéine de SEQ N°262 |
| **SEQ ID N°1953** | FSDPVTK | 105-111 pour la protéine de SEQ N°307 |
| **SEQ ID N°1954** | FYQNWQPQWAPGAK | 154-167 pour les protéines de SEQ N°265, 295, 296, 297, 1855 |
| **SEQ ID N°1955** | FYQNWQPQWTPGAK | 154-167 pour les protéines de SEQ N°266, 267, 269, 270, 271, 272, 273, 274, 275, 276, 277, 278, 279, 280, 281, 282, 283, 284, 285, 286, 287, 288, 289, 290, 291, 292, 298, 300, 302, 303, 304, 305, 306, 307, 308, 309, 310, 311, 1846, 1847, 1848, 1849, 1850, 1851, 1852, 1853, 1854, 1856, 1857, 1859, 1862, 1864, 1865, 1866, 1867, 1868, 1869, 1870; 153-166 pour la protéine de séquence SEQ ID N°268; 168-181 pour la protéine de séquence SEQ ID N°299; 153-166 pour la protéine de séquence SEQ ID N°1858 |
| **SEQ ID N°1956** | IPDDVR | 141-146 pour les protéines de SEQ N°266, 267, 269, 270, 271, 273, 274, 275, 276, 278, 279, 281, 283, 284, 285, 286, 289, 290, 291, 292, 300, 302, 303, 304, 305, 306, 307, 308, 309, 310, 311, 1846, 1847, 1848, 1849, 1850, 1851, 1854, 1856, 1857, 1859, 1862, 1864, 1865, 1866, 1867, 1868, 1869, 1870; 155-160 pour la protéine de séquence SEQ ID N°299; 140-145 pour la protéine de séquence SEQ ID N°1858 |
| **SEQ ID N°1957** | LAHTWIK | 204-210 pour la protéine de SEQ N°297 |
| **SEQ ID N°1958** | LAHTWITVPENEQK | 204-217 pour la protéine de SEQ N°279 |
| **SEQ ID N°1959** | LAHTWITVPQSEQK | 204-217 pour les protéines de SEQ N°282, 288, 295, 296, 298, 1853 |
| **SEQ ID N°1960** | LDAEAYGVK | 236-244 pour les protéines de SEQ N°265, 266, 267, 269, 270, 272, 273, 274, 275, 276, 277, 278, 279, 280, 281, 282, 283, 284, 285, 286, 287, 288, 290, 292, 295, 296, 297, 298, 300, 302, 303, 304, 305, 306, 307, 308, 309, 310, 1846, 1847, 1848, 1849, 1850, 1851, 1852, 1853, 1854, 1855, 1857, 1859, 1862, 1864, 1865, 1866, 1867, 1868, 1869, 1870; 235-243 pour la protéine de séquence SEQ ID N°268; 238-246 pour la protéine de séquence SEQ ID N°289; 250-258 pour la protéine de séquence SEQ ID N°299; 235-243 pour la protéine de séquence SEQ ID N°1858 |
| **SEQ ID N°1961** | LLHLATYTAGGLPLK | 126-140 pour les protéines de SEQ N°1870 |
| **SEQ ID N°1962** | LLHLATYTAGGLPLQFPDDVR | 126-146 pour la protéine de SEQ N°277 |
| **SEQ ID N°1963** | LYANSSIGLFAALAVK | 169-184 pour la protéine de SEQ N°285 |
| **SEQ ID N°1964** | LYANSSIGLFGALVVK | 169-184 pour la protéine de SEQ N°288 |
| **SEQ ID N°1965** | LYANSSIGLFGELAVK | 169-184 pour les protéines de SEQ N°269, 275, 277, 305, 1849, 1866 |
| **SEQ ID N°1966** | LYANSSIGLFGTLAVK | 169-184 pour la protéine de SEQ N°282 |
| **SEQ ID N°1967** | LYANSSIGLFSALAVK | 169-184 pour les protéines de SEQ N°1859 |
| **SEQ ID N°1968** | LYSNSSIGLFGALAVK | 169-184 pour les protéines de SEQ N°304, 310, 1865, 1869, 1870 |
| **SEQ ID N°1969** | NYAWGYR | 218-224 pour les protéines de SEQ N°288, 298, 1853 |
| **SEQ ID N°1970** | NYPIPAR | 363-369 pour les protéines de SEQ N°262, 293, 294, 1860, 1861 |
| **SEQ ID N°1971** | NYPNEAR | 363-369 pour les protéines de SEQ N°301, 1863 |
| **SEQ ID N°1972** | NYPNPVR | 363-369 pour les protéines de SEQ N°304, 308, 311, 1865, 1867 |
| **SEQ ID N°1973** | SICCALLLTASFSTFAAAK | 5-23 pour les protéines de SEQ N°288, 1853 |
| **SEQ ID N°1974** | SICCALLLTASFSTFAATK | 5-23 pour la protéine de SEQ N°298 |
| **SEQ ID N°1975** | SLCCALLLTAPLSTFAAAK | 5-23 pour la protéine de SEQ N°297 |
| **SEQ ID N°1976** | SLCCALLLTASFSTFASAK | 5-23 pour les protéines de SEQ N°265, 296 |
| **SEQ ID N°1977** | SLCCALLLTASLSTFAAAK | 5-23 pour les protéines de SEQ N°1855 |
| **SEQ ID N°1978** | SNVTDMAR | 245-252 pour les protéines de SEQ N°265, 287, 296, 1852 |
| **SEQ ID N°1979** | SYPNPIR | 363-369 pour la protéine de SEQ N°295 |
| **SEQ ID N°1980** | TALLHFYQNWQPQWAPGAK | 149-167 pour les protéines de SEQ N°295, 296 |
| **SEQ ID N°1981** | TDSIINGSDSK | 300-310 pour les protéines de SEQ N°292, 1854 |
| **SEQ ID N°1982** | TFIGVLGGDAIAR | 88-100 pour les protéines de SEQ N°1857 |
| **SEQ ID N°1983** | TFNGVLGGDCIAR | 88-100 pour la protéine de SEQ N°279 |
| **SEQ ID N°1984** | TFNGVLGGEAIAR | 88-100 pour la protéine de SEQ N°290 |
| **SEQ ID N°1985** | TGSTVGFGSYVAFVPEK | 336-352 pour les protéines de SEQ N°1870 |
| **SEQ ID N°1986** | TGYTGGFGSYVAFVPEK | 336-352 pour la protéine de SEQ N°282 |
| **SEQ ID N°1987** | TLQQGIELAQSR | 267-278 pour les protéines de SEQ N°282, 288, 298, 1853 |
| **SEQ ID N°1988** | TSSADLLAFVK | 250-260 pour les protéines de SEQ N°262, 293, 294, 1860, 1861 |
| **SEQ ID N°1989** | TSSADLLR | 250-257 pour les protéines de SEQ N°263, 264, 301, 1844, 1845, 1863 |
| **SEQ ID N°1990** | TYYFTWGK | 58-65 pour la protéine de SEQ N°296 |
| **SEQ ID N°1991** | VAALPAVEVNPPAPAVK | 311-327 pour les protéines de SEQ N°284, 1850 |
| **SEQ ID N°1992** | VAFAALPAVEVNPPAPAVK | 311-329 pour les protéines de SEQ N°266, 1846 |
| **SEQ ID N°1993** | VALAAIPAVEVNPPAPAVK | 311-329 pour la protéine de SEQ N°295 |
| **SEQ ID N°1994** | VALAALHTVEVNPPAPAVK | 311-329 pour la protéine de SEQ N°287 |
| **SEQ ID N°1995** | VALAALPAVEINPPAPAVK | 311-329 pour les protéines de SEQ N°309, 1868 |
| **SEQ ID N°1996** | VALAALPTVEVNPPAPAVK | 311-329 pour les protéines de SEQ N°1852 |
| **SEQ ID N°1997** | VAPAVEVNPPAPAVK | 311-325 pour les protéines de SEQ N°286, 1851 |
| **SEQ ID N°1998** | VEAYWR | 370-375 pour les protéines de SEQ N°270, 1848 |
| **SEQ ID N°1999** | VILEANPTAAPR | 314-325 pour les protéines de SEQ N°262, 263, 293, 301, 1844, 1860, 1863 |
| **SEQ ID N°2000** | VPQSEQK | 211-217 pour les protéines de SEQ N°282, 288, 295, 296, 297, 298, 1853 |
| **SEQ ID N°2001** | VSLEANPTAAPR | 314-325 pour les protéines de SEQ N°264, 294, 1845, 1861 |
| **SEQ ID N°2002** | WIQVNMDASR | 253-262 pour les protéines de SEQ N°287, 1852 |
| **SEQ ID N°2003** | WVQANMDASR | 253-262 pour la protéine de SEQ N°295 |
| **SEQ ID N°2004** | WVQVNMDASR | 253-262 pour les protéines de SEQ N°265, 273, 296, 297, 1855 |
| **SEQ ID N°2005** | YAAALLLTASFSTFAAAK | 5-22 pour les protéines de SEQ N°1858 |
| **SEQ ID N°2006** | YLPELTGK | 112-119 pour les protéines de SEQ N°1852 |
| **SEQ ID N°2007** | YWSELTGK | 112-119 pour la protéine de SEQ N°295 |

La détection d'un mécanisme de résistance aux céphalosporines induit par l'expression de la protéine CTX-M est caractérisée par la détection d'au moins un peptide appartenant à la protéine CTX-M et à ses différents variants de séquences **SEQ ID N° 351** à **SEQ ID N°445** et **SEQ ID N° 1871 à SEQ ID N°1908.**
**SEQ ID N° 351 :** 
MMTQSIRRSMLTVMATLPLLFSSATLHAQTNSVQQQLEALEKSSGGRLGVALINTAD
NSQILYRADERFAMCSTSKVMAAAAVLKQSESDKHLLNQRVEIKKSDLVNYNPIAEK
HVNGTMTLAELGAAALQYSDNTAMNKLIAHLGGPDKVTAFARSLGDETFRLDRTEP
TLNTAIPGDPRDTTTPLAMAQTLKNLTLGKALAETQRAQLVTWLKGNTTGSASIRAG
LPKSWGVGDKTGSGDYGTTNDIAVIWPANHAPLVLVTYFTQPEQKAESRRDVLAAA
AKIVTHGF
**SEQ ID N° 352** : 
MMRHRVKRMMLMTTACISLLLGSAPLYAQANDVQQKLAALEKSSGGRLGVALIDTA
DNAQTLYRADERFAMCSTSKVMAAAAVLKQSETQKKVLSQKVEIKSSDLINYNPITE
KHVNGTMTLAELSAAALQYSDNTAMNKLIAHLGGPDKVTAFARAIGDNTFRLDRTEP
TLNTAIPGDPRDTTTPLAMAQTLRNLTLGSALGETQRAQLVTWLKGNTTGAASIQAG
LPTSWVVGDKTGSGDYGTTNDIAVIWPEGRAPLILVTYFTQPEQKAESRRDVLAAAA
KIVTDGY
**SEQ ID N° 353 :** 
MVTKRVQRMMFAAAACIPLLLGSAPLYAQTSAVQQKLAALEKSSGGRLGVALIDTA
DNTQVLYRGDERFPMCSTSKVMAAAAVLKQSETQKQLLNQPVEIKPADLVNYNPIA
EKHVNGTMTLAELSAAALQYSDNTAMNKLIAQLGGPGGVTAFARAIGDETFRLDRT
EPTLNTAIPGDPRDTTTPRAMAQTLRQLTLGHALGETQRAQLVTWLKGNTTGAASI
RAGLPTSWTAGDKTGSGDYGTTNDIAVIWPQGRAPLVLVTYFTQPQQNAESRRDV
LASAARIIAEGL
**SEQ ID N° 354 :** 
MVKKSLRQFTLMATATVTLLLGSVPLYAQTVDVQQKLAELEQQSGGRLGVALINTA
DNSQILYRADERFAMCSTSKVMAAAAVLKKSESEPNLLNQRVEIKKSDLVNYNPIAE
KHVNGTMSLAELSAAALQYSDNVAMNKLIAHVGGPASVTAFARQLGDETFRLDRTE
PTLNTAIPGDPRDTTSPRAMAQTLRNLTLGKALGDSQRAQLVTWMKGNTTGAASIQ
AGLPASWVVGDKTGSGDYGTTNDIAVIWPKDRAPLILVTYFTQPQPKAESRRDVLA
SAAKIVTDGL
**SEQ ID N° 355 :** 
MVTKRVQRMMFAAAACIPLLLGSAPLYAQTSAVQQKLAALEKSSGGRLGVALIDTA
DNTQVLYRGDERFPMCSTSKVMAAAAVLKQSETQKQLLNQPVEIKPADLVNYNPIA
EKHVNGTMTLAELSAAALQYSDNTAMNKLIAQLGGPGGVTAFARAIGDETFRLDRT
EPTLNTAIPGDPRDTTTPRAMAQTLRQLTLGHALGETQRAQLVTWLKGNTTGAASI
RAGLPTSWTVGDKTGSGDYGTTNDIAVIWPQGRAPLVLVTYFTQPQQNAESRRDV
LASAARIIAEGL
**SEQ ID N° 356 :** 
MVTKRMQRMMFAAAACIPLLLGSAPLYAQTSAVQQKLAALEKSSGGRLGVALIDTK
DNTQVLYRGDERFPMCSTSKVMAAAAVLKQSETQKQLLNQPVEIKPADLVNYNPIA
EKHVNGTMTLAELSAAALQYSDNTAMNKLIAQLGGPGGVTAFAREIGDETFRLDRT
EPTLNTAIPGDPRDTTTPRAMAQTLRQLTLGHALGETQRAQLVTWLKGNTTGAASI
RAGLPTSWTVGDKTGSGDYGTTNDIAVIWPQGRAPLVLVTYFTQPQQNAESRRDV
LASAARIIAEGL
**SEQ ID N° 357** : 
SEQ ID N° [?]
MVKKSLRQFTLMATATVTLLLGSVPLYAQTADVQQKLAGLERQSGGRLGVALINTA
 
DNSQILYRADERFAMCSTSKVMAAAAVLKKSESEPNLLNQRVEIKKSDLVNYNPIAE
KHVNGTMSPAELSAAALQYSDNVAMNKLIAHVGGPASVTAFARQLGDETFRLDRTE
PTLNTAIPGDPRDTTSPRAMAQTLRNLTLGKALGDSQRAQLVTWMKGNTTGAASIQ
AGLPASWVVGDKTGSGDYGTTNDIAVIWPKDRAPLILVTYFTQPQPKAESRRHVLA
S
**SEQ ID N° 358** : 
MVKKSLRQFTLMATAAVTLLLGSVPLYAQTADVQQKLAELERQSGGRLGVALINTA
DNSQILYRADERFAMCSTSKVMAAAAVLKKSESEPSLLNQRVEIKKSDLVNYNPIAE
KHVNGTMSLAELSAAALQYSDNVAMNKLIAHVGGPASVTAFARQLGDETFRLDRTE
PTLNTAIPGDPRDTTSPRAMAQTLRNLTLGKALGDSQRAQLVTWMKGNTTGAASIQ
AGLPASWVVGDKTGSGDYGTTNDIAVIWPKDRAPLILVTYFTQPQPKAESRRDILAS
AAKIVTDGL
**SEQ ID N° 359** : 
MVTKRVQRMMFAAAACIPLLLGSAPLYAQTSAVQQKLAALEKSSGGRLGVALIDTA
DNTQVLYRGDERFPMCSTSKVMAAAAVLKQSETQKQLLNQPVEIKPADLVNYNPIA
EKHVNGTMTLAELSAAALQYSDNTAMNKLIAQLGGPGGVTAFARAIGDETFRLDRT
EPTLNTAIPGDPRDTTTPRAMAQTLRQLTLGHALGETQRAQLVTWLKGNTTGAASI
RAGLPTSWTAGDKTGSGGYGTTNDIAVIWPQGRAPLVLVTYFTQPQQNAESRRDV
LASAARIIAEGL
**SEQ ID N° 360** : 
MVTKRVQRMMFAAAACIPLLLGSAPLYAQTSAVQQKLAALEKSSGGRLGVALIDTA
DNTQVLYRGDERFPMCSTSKVMAAAAVLKQSETQKQLLNQPVEIKPADLVNYNPIA
EKHVNGTMTLAELSAAALQYSDNTAMNKLIAQLGGPGGVTAFARAIGDETFRLDRT
EPTLNTAIPGDPRDTTTPRAMAQTLRQLTLGHALGETQRAQLVTWLKGNTTGAASI
RAGLPTSWTVGDKTGSGDYGTTNDIAVIWPQGRAPLVLVTYFTQPQQNAESRRDV
LASAARIIAEGL
**SEQ ID N° 361** : 
MVTKRVQRMMFAAAACIPLLLGSAPLYAQTSAVQQKLAALEKSSGGRLGVALIDTA
DNTQVLYRGDERFPMCSTSKVMAAAAVLKQSETQKQLLNQPVEIKPADLVNYNPIA
EKHVNGTMTLAELSAAALQYSDNTAMNKLIAQLGGPGGVTAFARAIGDETFRLDRT
ESTLNTAIPGDPRDTTTPRAMAQTLRQLTLGHALGETQRAQLVTWLKGNTTGAASI
RAGLPTSWTVGDKTGSGDYGTTNDIAVIWPQGRAPLVLVTYFTQPQQNAESRRDV
LASAARIIAEGL
**SEQ ID N° 362 :** 
MVTKRVQRMMFAAAACIPLLLGSAPLYAQTSAVQQKLAALEKSSGGRLGVALIDTA
DNTQVLYRGDERFPMCSTSKVMAAAAVLKQSETQKQLLNQPVEIKPADLVNYNPIA
EKHVNGTMTLAELSAAALQYSDNTAMNKLIAQLGGPGGVTAFARAIGDETFRLDRT
EPTLNTAIPGDPRDTTTPRAMAQTLRQLTLGHALGETQRAQLVTWLKGNTTGAASI
RAGLPTSWTVGDKTGSGDYGTTNDIAVIWPQGRAPLVLVTYFTQPQQNAESRRDV
LASAARIIAKGL
**SEQ ID N° 363 :** 
MVKKSLRQFTLMATATVTLLLGSVPLYAQTADVQQKLAELERQSGGRLGVALINTAD
NSQILYRADERFAMCSTSKVMAAAAVLKKSESEPNLLNQRVEIKKSDLVNYNPIAEK
HVNGTMSLAELSAAALQYSDNVAMNKLIAHVGGPASVTAFARQLGDETFRLDRTEP
TLNTAIPGDPRDTTSPRAMAQTLRNLTLGKALGDSQRAQLVTWMKGNTTGAASIQA
GLPASWVVGDKTGSGGYGTTNDIAVIWPKDRAPLILVTYFTQPQPKAESRRDVLAS
AAKIVTDGL
**SEQ ID N° 364** : 
MVKKSLRQFTLMATATVTLLLGSVPLYAQTADVQQKLAELERQSGGRLGVALINTAD
NSQILYRADERFAMCSTSKVMAAAAVLKKSESEPNLLNQRVEIKKSDLVNYNPIAEK
 
HVNGTMSLAELSAAALQYSDNVAMNKLIAHVGGPASVTAFARQLGDETFRLDRTEP
TLNTAIPGDPRDTTSPRAMAQTLRNLTLGKALGDSQRAQLVTWMKGNTTGAASIQA
GLPASWVVGDKTGSGDYGTTNDIAVIWPKDRAPLILVTYFTQPQPKAESRRDVLAS
AAKIVTNGL
**SEQ ID N° 365 :** 
MVKKSLRQFTLMATATVTLLLGSVPLYAQTADVQQKLAELERQSGGRLGVALINTAD
NSQILYRADERFAMCSTSKVMAVAAVLKKSESEPNLLNQRVEIKKSDLVNYNPIAEK
HVNGTMSLAELSAAALQYSDNVAMNKLIAHVGGPASVTAFARQLGDETFRLDRTET
TLNTAIPGDPRDTTSPRAMAQTLRNLTLGKALGDSQRAQLVTWMKGNTTGAASIQA
GLPASWVVGDKTGSGDYGTTNDIAVIWPKDRAPLILVTYFTQPQPKAESRRDVLAS
AAKIVTNGL
**SEQ ID N° 366 :** 
MMRKSVRRAMLMTTACVSLLLASVPLCAQANDVQQKLAALEKSSGGRLGVALINTA
DNTQTLYRADERFAMCSTSKVMAVAAVLKQSETQKGLLSQRVEIKPSDLINYNPIAE
KHVNGTMTFGELSAAALQYSDNTAMNKLIAHLGGPDKVTAFARTIGDDTFRLDRTE
PTLNTAIPGDPRDTTTPLAMAQALRNLTLGNALGDTQRAQLVMWLKGNTTGAASIQ
AGLPTSWVVGDKTGSGGYGTTNDIAVIWPEGRAPLVLVTYFTQSEPKAESRRDVLA
AAARIVTDGY
**SEQ ID N° 367 :** 
MVTKRVQRMMFAAAACIPLLLGSAPLYAQTSAVQQKLAALEKSSGGRLGVALIDTA
DNTQVLYRGDERFPMCSTSKVMAAAAVLKQSETQKQLLNQPVEIKPADLVNYNPIA
EKHVNGTMTLAELSAAALQYSDNTAMNKLIAQLGGPGGVTAFARAIGDETFRLDRT
EPTLNTAIPGDPRDTTTPRAMAQTLRQLTLGHALGETQRAQLVTWLKGNTTGAASI
RAGLPTSWTVGDKTGSGDYGTTNDIAVIWPQGRAPLVLVTYFTQPQQNAERRRDV
LASAARIIAEGL
**SEQ ID N° 368 :** 
MMRKSVRRAMLMTTACVSLLLASVPLCAQANDVQQKLAALEKSSGGRLGVALINTA
DNTQTLYRADERFAMCSTSKVMAAAAVLKQSETQKGLLSQRVEIKPSDLINYNPIAE
KHVNGTMTFGELSAAALQYSDNTAMNKLIAHLGGPDKVTAFARTIGDDTFRLDRTE
PTLNTAIPGDPRDTTTPLAMAQALRNLTLGNALGDTQRAQLVMWLKGNTTGAASIR
AGLPTSWVVGDKTGSGDYGTTNDIAVIWPEGRAPLVLVTYFTQSEPKAESRRDVLA
AAARIVTDGY
**SEQ ID N° 369 :** 
MVTKRVQRMMFAAAACIPLLLGSAPLYAQTSAVQQKLAALEKSSGGRLGVALIDTA
DNTQVLYRGDERFPMCSTSKVMAAAAVLKQSETQKQLLNQPVEIKPADLVNYNPIA
EKHVNGTMTLAELSAAALQYSDNTAMNKLIAQLGGPGGVTAFARAIGDETFRLDRT
EPTLNTAIPGDPRDTTTPRAMAQTLRQLTLGHALGETQRAQLVTWLKGNTTGAASI
RAGLPTSWTVGDKTGSGGYGTTNDIAVIWPQGRAPLVLVTYFTQPQQNAESRRDV
LASAARIIAEGL
**SEQ ID N° 370 :** 
MVKKSLRQFTLMATATVTLLLGSVPLYAQTADVQQKLAELERQSGGRLGVALINTAD
NSQILYRADERFAMCSTSKVMAAAAVLKKSESEPNLLNQRVEIKKSDLVNYSPIAEK
HVNGTMSLAELSAAALQYSDNVAMNKLIAHVGGPASVTAFARQLGDETFRLDRTEP
TLNTAIPGDPRDTTSPRAMAQTLRNLTLGKALGDSQRAQLVTWMKGNTTGAASIQA
GLPASWVVGDKTGSGGYGTTNDIAVIWPKDRAPLILVTYFTQPQPKAESRRDVLAS
AAKIVTDGL
**SEQ ID N° 371 :** 
MVKKSLRQFTLMATAAVTLLLGSVPLYAQTADVQQKLAELERQSGGRLGVALINTA
DNSQILYRADERFAMCSTSKVMAAAAVLKKSESEPNLLNQRVEIKKSDLVNYNPIAE
KHVDGTMSLAELSAAALQYSDNVAMNKLIAHVGGPASVTAFARQLGDETFRLDRTE
 
PTLNTAIPGDPRDTTSPRAMAQTLRNLTLGKALGDSQRAQLVTWMKGNTTGAASIQ
AGLPASWVVGDKTGSGGYGTTNDIAVIWPKDRAPLILVTYFTQPQPKAESRRDVLA
SAAKIVTNGL
**SEQ ID N° 372 :** 
MVKKSLRQFTLMATAAVTLLLGSVPLYAQTADVQQKLAELERQSGGRLGVALINTA
DNSQILYRADERFAMCSTSKVMAAAAVLKKSESEPNLLNQRVEIKKSDLVNYNPIAE
KHVDGTMSLAELSAAALQYSDNVAMNKLIAHVGGPASVTAFARQLGDETFRLDRTE
PTLNTAIPGDPRDTTSPRAMAQTLRNLTLGKALGDSQRAQLVTWMKGNTTGAASIQ
AGLPASWVVGDKTGSGDYGTTNDIAVIWPKDRAPLILVTYFTQPQPKAESRRDVLA
SAAKIVTDGL
**SEQ ID N° 373 :** 
MVKKSLRQFTLMATATVTLLLGSVPLYAQTVDVQQKLAELEQQSGGRLGVALINTA
DNSQILYRADERFAMCSTSKVMAAAAVLKKSESEPNLLNQRVEIKKSDLVNYNPIAE
KHVNGTMSLAELSAAALQYSDNVAMNKLIAHVGGPASVTAFARQLGDETFRLDRTE
PTLNTAIPGDPRDTTSPRAMAQTLRNLTLGKALGDSQRAQLVTWMKGNTTGAASIQ
AGLPASWVVGDKTGSCDYGTTNDIAVIWPKDRAPLILVTYFTQPQPKAESRRDVLA
SAAKIVTDGL
**SEQ ID N° 374 :** 
MVKKSLRQFTLMATATVTLLLGSVPLHAQTADVQQKLAELEQQSGGRLGVALINTA
DNSQILYRADERFAMCSTSKVMAAAAVLKKSESEPNLLNQRVEIKKSDLVNYNPIAE
KHVDGTMSLAELSAAALQYSDNVAMNKLIAHVGGPASVTAFARQLGDETFRLDRTE
PTLNTAIPGDPRDTTSPRAMAQTLRNLTLGKALGDSQRAQLVTWMKGNTTGAASIQ
AGLPASWVVGDKTGSGDYGTTNDIAVIWPKDRAPLILVTYFTQPQPKAESRRDVLA
SAAKIVTDGL
**SEQ ID N° 375 :** 
MRHRVKRMMLMTTACISLLLGSAPLYAQANDVQQKLAALEKSSGGRLGVALIDTAD
NAQTLYRADERFAMCSTSKVMAAAAVLKQSETQKNVLSQKVEIKSSDLINYNPIAEK
HVNGTMTLAELSAAALQYSDNTAMNKLIAHLGGPDKVTAFARAIGDDTFRLDRTEPT
LNTAIPGDPRDTTTPLAMAQTLRHLTLGSALGETQRAQLVTWLKGNTTGAASIQAGL
PTSWVVGDKTGSGDYGTTNDIAVIWPEGRAPLILVTYFTQPEQKAESRRDVLAAAA
KIVTDGY
**SEQ ID N° 376 :** 
MVTKRVQRMMFAAAACIPLLLGSAPLYAQTSAVQQKLAALEKSSGGRLGVALIDTA
DNTQVLYRGDERFPMCSTSKVMAAAAVLKQSETQKQLLNQPVEIKPADLVNYNPIA
EKHVNGTMTLAELSAAALQYSDNTAMNKLIAQLGGPGGVTAFARAIGDETFRLDRT
EPTLNTAIPGDPRDTTTPRAMAQTLRQLTLGHALGETQRAQLVTWLKGNTTGAARI
RAGLPTSWTVGDKTGSGDYGTTNDIAVIWPQGRAPLVLVTYFTQPQQNAESRRDV
LASAARIIAEGL
**SEQ ID N° 377 :** 
MVTKRVQRMMFAAAACIPLLLGSAPLYAQTSAVQQKLAALEKSSGRRLGVALIDTAD
NTQVLYRGDERFPMCSTSKVMAAAAVLKQSETQKQLLNQPVEIKPADLVNYNPIAE
KHVNGTMTLAELSAAALQYSDNTAMNKLIAQLGGPGGVTAFARAIGDETFRLDRTE
PTLNTAIPGDPRDTTTPRAMAQTLRQLTLGHALGETQRAQLVTWLKGNTTGAASIR
AGLPTSWTVGDKTGSGDYGTTNDIAVIWPQGRAPLVLVTYFTQPQQNAESRRDVL
ASAARIIAEGL
**SEQ ID N° 378 :** 
MVTKRVQRMMFAAAACIPLLLGSAPLYAQTNAVQQKLAALEKSSGGRLGVALIDTA
DNTQVLYRGDERFPMCSTSKVMAAAAVLKQSETQKQLLNQPVEIKPADLVNYNPIA
EKHVNGTMTLAELSAAALQYSDNTAMNKLIAQLGGPGGVTAFARAIGDETFRLDRT
EPTLNTAIPGDPRDTTTPRAMAQTLRQLTLGHALGETQRAQLVTWLKGNTTGAASI
 
RAGLPTSWTVGDKTGSGDYGTTNDIAVIWPQGRAPLVLVTYFTQPQQNAESRRDV
LASAARIIAEGL
**SEQ ID N° 379** : 
MVTKRVQRMMFAAAACIPLLLGSAPLYAQTSAVQQKLAALEKSSGRRLGVPLIDTAD
NTQVLYRGDERFPMCSTSKVMAAAAVLKQSETQKQLLNQPVEIKPADLVNYNPIAE
KHVNGTMTLAELSAAALQYSDNTAMNKLIAQLGGPGGVTAFARAIGDETFRLDRTE
PTLNTAIPGDPRDTTTPRAMAQTLRQLTLGHALGETQRAQLVTWLKGNTTGAASIR
AGLPTSWTVGDKTGSGDYGTTNDIAVIWPQGRAPLVLVTYFTQPQQNAESRRDVL
ASAARIIAEGL
**SEQ ID N° 380** : 
MVTKRVQRMMFAAAACIPLLLGSAPLYAQTSAVQQKLAALEKSSGGRLGVPLIDTA
DNTQVLYRGDERFPMCSTSKVMAAAAVLKQSETQKQLLNQPVEIKPADLVNYNPIA
EKHVNGTMTLAELSAAALQYSDNTAMNKLIAQLGGPGGVTAFARAIGDETFRLDRT
EPTLNTAIPGDPRDTTTPRAMAQTLRQLTLGHALGETQRAQLVTWLKGNTTGAASI
RAGLPTSWTVGDKTGSGDYGTTNDIAVIWPQGRAPLVLVTYFTQPQQNAESRRDV
LASAARIIAEGL
**SEQ ID N° 381** : 
MVTKRVQRMMFAAAACIPLLLGSAPLYAQTNAVQQKLAALEKSSGGRLGVPLIDTA
DNTQVLYRGDERFPMCSTSKVMAAAAVLKQSETQKQLLNQPVEIKPADLVNYNPIA
EKHVNGTMTLAELSAAALQYSDNTAMNKLIAQLGGPGGVTAFARAIGDETFRLDRT
EPTLNTAIPGDPRDTTTPRAMAQTLRQLTLGHALGETQRAQLVTWLKGNTTGAASI
RAGLPTSWTVGDKTGSGDYGTTNDIAVIWPQGRAPLVLVTYFTQPQQNAESRRDV
LASAARIIAEGL
**SEQ ID N° 382 :** 
MVKKSLRQFTLMATATVTLLLGSVPLYAQTADVQQKLAELERQSGGRLGVALINTAD
NSQILYRADERFAMCSTSKVMAAAAVLKKSESEPNLLNQRVEIKKSDLVNYNPIAEK
HVNGTMSLAELSAAALQYSDNVAMNKLIAHVGGPASVTAFARQLGDETFRLDGTEP
TLNTAIPGDPRDTTSPRAMAQTLRNLTLGKALGDSQRAQLVTWMKGNTTGAASIQA
GLPASWVVGDKTGSGDYGTTNDIAVIWPKDRAPLILVTYFTQPQPKAESRRDVLAS
AAKIVTDGL
**SEQ ID N° 383 :** 
MMRKSVRRAMLMTTACVSLLLASVPLCAQANDVQQKLAALEKSSGGRLGVALINTA
DNTQTLYRADERFAMCSTSKVMAAAAVLKQSETQKGLLSQRVEIKPSDLINYNPIAE
KHVNGTMTFGELSAAALQYSDNTAMNKLIAHLGGPDKVTAFARTIGDDTFRLDRTE
PTLNTAIPGDPRDTTTPLAMAQALRNLTLGNALGDTQRAQLVMWLKGNTTGAASIQ
AGLPTSWVVGDKTGSGDYGTTNDIAVIWPEGRAPLVLVTYFTQSEPKAESRRDVLA
AAARIVTDGY
**SEQ ID N° 384 :** 
MMRKSVRRAMLMTTACVSLLLASVPLCAQANDVQQKLAALEKSSGGRLGVALINTA
DNTQTLYRADERFAMCSTSKVMAAAAVLKQSETQKGLLSQRVEIKPSDLVNYNPIA
EKHVNGTMTFGELIAAALQYSDNTAMNKLIAHLGGPDKVTAFARTIGDDTFRLDRTE
PTLNTAIPGDPRDTTTPLAMAQALRNLTLGNALGDTQRAQLVMWLKGNTTGAASIQ
AGLPTSWVVGDKTGSGGYGTTNDIAVIWPEGRAPLVLVTYFTQSEPKAESRRDVLA
AAARIVTDGY
**SEQ ID N° 385 :** 
MVKKSLRQFTLMATATVTLLLGSVPLYAQTADVQQKLAELERQSGGRLGVALINTAD
NSQILYRADERFAMCSTSKVMAAAAVLKKSESEPNLLNQRVEIKKSDLVNYNPIAEK
HVNGTMSLAELSAAALQYSDNVAMNKLIAHVGGPASVTAFARQLGDETFRLDRTET
TLNTAIPGDPRDTTSPRAMAQTLRNLTLGKALGDSQRAQLVTWMKGNTTGAASIQA
 
GLPASWVVGDKTGSGDYGTTNDIAVIWPKDRAPLILVTYFTQPQPKAESRRDVLAS
AAKIVTDGL
**SEQ ID N° 386** : 
MMTQSIRRSMLTVMATLPLLFSSATLHAQANSVQQQLEALEKSSGGRLGVALINTA
DNSQILYRADERFAMCSTSKVMAAAAVLKQSESDKHLLNQRVEIKKSDLVNYNPIAE
KHVNGTMTLAELGAAALQYSDNTAMNKLIAHLGGPDKVTAFARSLGDETFRLDRTE
PTLNTAIPGDPRDTTTPLAMAQTLKNLTLGKALAETQRAQLVTWLKGNTTGSASIRA
GLPKSWVVGDKTGSGGYGTTNDIAVIWPENHAPLVLVTYFTQPEQKAERRRDILAA
AAKIVTHGF
**SEQ ID N° 387 :** 
MVTKRVQRMMFAAAACIPLLLGSAPLYAQTSAVQQKLAALEKSSGGRLGVALIDTA
DNTQVLYRGDERFPMCSTSKVMAVAAVLKQSETQKQLLNQPVEIKPADLVNYNPIA
EKHVNGTMTLAELSAAALQYSDNTAMNKLIAQLGGPGGVTAFARAIGDETFRLDRT
EPTLNTAIPGDPRDTTTPRAMAQTLRQLTLGHALGETQRAQLVTWLKGNTTGAASI
RAGLPTSWTAGDKTGSGDYGTTNDIAVIWPQGRAPLVLVTYFTQPQQNAESRRDV
LASAARIIAEGL
**SEQ ID N° 388 :** 
MVKKSLRQFTLMATATVTLLLGSVPLYAQTADVQQKLAELERQSGGRLGVALINTAD
NSQILYRADERFAMCSTSKVMAVAAVLKKSESEPNLLNQRVEIKKSDLVNYNPIAEK
HVNGTMSLAELSAAALQYSDNVAMNKLIAHVGGPASVTAFARQLGDETFRLDRTES
TLNTAIPGDPRDTTSPRAMAQTLRNLTLGKALGDSQRAQLVTWMKGNTTGAASIQA
GLPASWVVGDKTGSGDYGTTNDIAVIWPKDRAPLILVTYFTQPQPKAESRRDVLAS
AAKIVTDGL
**SEQ ID N° 389 :** 
MVKKSLRQFTLMATATVTLLLGSVPLYAQTADVQQKLAELERQSGGRLGVALINTAD
NSQILYRADERFPMCSTSKVMAAAAVLKKSESEPNLLNQRVEIKKSDLVNYNPIAEK
HVNGTMSLAELSAAALQYSDNVAMNKLIAHVGGPASVTAFARQLGDETFRLDRTEP
TLNTAIPGDPRDTTSPRAMAQTLRNLTLGKALGDSQRAQLVTWMKGNTTGAASIQA
GLPASWVVGDKTGSGGYGTTNDIAVIWPKDRAPLILVTYFTQPQPKAESRRDVLAS
AAKIVTDGL
**SEQ ID N° 390 :** 
MVKKSLRQFTLMATATVTLLLGSVPLYAQTVDVQQKLAELEQQSGGRLGVALINTA
DNSQILYRADERFAMCSTSKVMAVAAVLKKSESEPNLLNQRVEIKKSDLVNYNPIAE
KHVNGTMSLAELSAAALQYSDNVAMNKLIAHVGGPASVTAFARQLGDETFRLDRTE
PTLNTAIPGDPRDTTSPRAMAQTLRNLTLGKALGDSQRAQLVTWMKGNTTGAASIQ
AGLPASWVVGDKTGSGGYGTTNDIAVIWPKDRAPLILVIYFTQPQPKAESRRDVLAS
AAKIVTDGL
 
SEQ ID N° 391 :
MVKKSLRQFTLMATATVTLLLGSVPLYAQTADVQQKLAELERQSGGRLGVALINTAD
NSQILYRADERFAMCSTSKVMAAAAVLKKSESEPNLLNQRVEIKKSDLVNYNPIAEK
HVNGTMSLAELSAAALQYSDNVAMNKLIAHVGGPASVTAFARQLGDETFRLDRTEQ
TLNTAIPGDPRDTTSPRAMAQTLRNLTLGKALGDSQRAQLVTWMKGNTTGAASIQA
GLPASWVVGDKTGSGDYGTTNDIAVIWPKDRAPLILVTYFTQPQPKAESRRDVLAS
AAKIVTDGL
**SEQ ID N° 392 :** 
MMTQSIRRSMLTVMATLPLLFSSATLHAQANSVQQQLEALEKSSGGRLGVALINTA
DNSQILYRADERFAMCSTSKVMAAAAVLKQSESDKLLLNQRVEIKKSDLVNYNPIAE
KHVNGTMTLAELGAAALQYSDNTAMNKLIAHLGGPDKVTAFARSLGDETFRLDRTE
PTLNTAIPGDPRDTTTPLAMAQTLKNLTLGKALAETQRAQLVTWLKGNTTGSASIRA
 
GLPKSWVVGDKTGSGDYGTTNDIAVIWPENHAPLVLVTYFTQPEQKAESRRDILAA
AAKIVTHGF
**SEQ ID N° 393 :** 
MVKKSLRQFTLMATATVTLLLGSVPLYAQTADVQQKLAELERQSGGRLGVALINTAD
NSQILYRADERFAMCSTSKVMAVAAVLKKSESEPNLLNQRVEIKKSDLVNYNPIAEK
HVNGTMSLAELSAAALQYSDNVAMNKLIAHVGGPASVTAFARQLGDETFRLDRTEP
TLNTAIPGDPRDTTSPRAMAQTLRNLTLGKALGDSQRAQLVTWMKGNTTGAASIQA
GLPASWVVGDKTGSGGYGTTNDIAVIWPKDRAPLILVTYFTQPQPKAESRRDVLAS
AAKIVTDGL
**SEQ ID N° 394 :** 
MVKKSLRQFTLMATATVTLLLGSVPLYAQTADVQQKLAELERQSGGRLGVALINTAD
NSQILYRADERFAMCSTSKVMAVAAVLKKSESEPNLLNQRVEIKKSDLVNYNPIAEK
HVNGTMSLAELSAAALQYSDNVAMNKLIAHVGGPASVTAFARQLGDETFRLDRTEP
TLNTAIPGDPRDTTSPRAMAQTLRNLTLGKALGDSQRAQLVTWMKGNTTGAASIQA
GLPASWVVGDKTGSGGYGTTNDIAVIWPKDRAPLILVTYFTQPQPKAESRRDVLAS
AAKIVTDGL
**SEQ ID N° 395 :** 
MVKKSLRQFTLMATATVTLLLGSVPLYAQTADVQQKLAELERQSGGRLGVALINTAD
NSQILYRADERFAMCSTSKVMAVAAVLKKSESEPNLLNQRVEIKKSDLVNYNPIAEK
HVDGTMSLAELSAAALQYSDNVAMNKLISHVGGPASVTAFARQLGDETFRLDRTET
TLNTAIPGDPRDTTSPRAMAQTLRNLTLGKALGDSQRAQLVTWMKGNTTGAASIQA
GLPASWVVGDKTGSGDYGTTNDIAVIWPKDRAPLILVTYFTQPQPKAESRRDVLAS
AAKIVTNGL
**SEQ ID N° 396 :** 
MVKKSLRQFTLMATATVTLLLGSVPLYAQTADVQQKLAELERQSGGRLGVALINTAD
NSQILYRADERFAMCSTSKVMAVAAVLKKSESEPNLLNQRVEIKKSDLVNYNPIAEK
HVDGTMSLAELSAAALQYSDNVAMNKLISHVGGPASVTAFARQLGDETFRLDRTEP
TLNTAIPGDPRDTTSPRAMAQTLRNLTLGKALGDSQRAQLVTWMKGNTTGAASIQA
GLPASWVVGDKTGSGDYGTTNDIAVIWPKDRAPLILVTYFTQPQPKAESRRDVLAS
AAKIVTDGL
**SEQ ID N° 397 :** 
MMTQSIRRSMLTVMATLPLLFSSATLHAQANSVQQQLEALEKSSGGRLGVALINTA
DNSQILYRADERFAMCSTSKVMAAAAVLKQSESDKHLLNQRVEIKKSDLVNYNPIAE
KHVNGTMTLAELGAAALQYSDNTAMNKLIAHLGGPDKVTAFARSLGDETFRLDRTE
PTLNTAIPGDPRDTTTPLAMAQTLKNLTLGKALAETQRAQLVTWLKGNTTGSASIRA
GLPKSWVVGDKTGSGDYGTTNDIAVIWPENHAPLVLVTYFTQPEQKAENRRDILAA
AAKIVTHGF
**SEQ ID N° 398 :** 
MVTKRVQRMMFAAAACIPLLLGSAPLYAQTSAVQQKLAALEKSSGGRLGVALIDTA
DNTQVLYRGDERFPMCSTSKVMAVAAVLKQSETQKQLLNQPVEIKPADLVNYNPIA
EKHVNGTMTLAELSAAALQYSDNTAMNKLIAQLGGPGGVTAFARAIGDETFRLDRT
EPTLNTAIPGDPRDTTTPRAMAQTLRQLTLGHALGETQRAQLVTWLKGNTTGAASI
RAGLPTSWTVGDKTGSGDYGTTNDIAVIWPQGRAPLVLVTYFTQPQQNAERRRDV
LASAARIIAEGL
**SEQ ID N° 399 :** 
MVKKSLRQFTLMATATVTLLLGSVPLYAQTADVQQKLAELERQSGGRLGVALINTAD
NSQILYRADERFAMCSTSKVMAVAAVLKKSESEPNLLNQRVEIKKSDLVNYNPIAEK
HVNGTMSLAELSAAALQYSDNVAMNKLIAHVGGPASVTAFARQLGDETFRLDRTEP
TLNTAIPGDPRDTTSPRAMAQTLRNLTLGKALGDSQRAQLVTWMKGNTTGAASIQA
 
GLPASWVVGDKTGSGGYGTTNDIAVIWPKDRAPLILVTYFTQPQPKAESRRDVLAS
AAKIVTNGL
**SEQ ID N° 400 :** 
MVKKSLRQFTLMATATVTLLLGSVPLYAQTADVQQKLAELERQSGGRLGVALINTAD
NSQILYRADERFAMCSTSKVMAAAAVLKKSESEPNLLNQRVEIKKSDLVNYNPIAEK
HVNGTMSLAELSAAALQYSDNVAMNKLIAHVGGPASVTAFARQLGDETFRLDRTES
TLNTAIPGDPRDTTSPRAMAQTLRNLTLGKALGDSQRAQLVTWMKGNTTGAASIQA
GLPASWVVGDKTGSGDYGTTNDIAVIWPKDRAPLILVTYFTQPQPKAESRRDVLAS
AAKIVTDGL
**SEQ ID N° 401** : 
MVKKSLRQFTLMATATVTLLLGNVPLYAQTADVQQKLAELERQSGGRLGVALINTA
DNSQILYRADERFAMCSTSKVMAAAAVLKKSESEPNLLNQRVEIKKSDLVNYNPIAE
KHVNGTMSLAELSAAALQYSDNVAMNKLIAHVGGPASVTAFARQLGDETFRLDRTE
PTLNTAIPGDPRDTTSPRAMAQTLRNLTLGKALGDSQRAQLVTWMKGNTTGAASIQ
AGLPASWVVGDKTGSGDYGTTNDIAVIWPKDRAPLILVTYFTQPQPKAESRRDVLA
SAAKIVTDGL
**SEQ ID N° 402** : 
MVTKRVQRMMFAAAACIPLLLGSAPLYAQTSAVQQKLAALEKSSGGRLGVALIDTA
DNTQVLYRGDERFPMCSTSKVMAAAAVLKQSETQKQLLNQPVEIKPADLVNYSPIA
EKHVNGTMTLAELSAAALQYSDNTAMNKLIAQLGGPGGVTAFARAIGDETFRLDRT
EPTLNTAIPGDPRDTTTPRAMAQTLRQLTLGHALGETQRAQLVTWLKGNTTGAASI
RAGLPTSWTVGDKTGSGDYGTTNDIAVIWPQGRAPLVLVTYFTQPQQNAESRRDV
LASAARIIAEGL
**SEQ ID N° 403** : 
MVTKRVQRMMFAAAACIPLLLGSAPLYAQTSAVQQKLAALEKSSGGRLGVALIDTA
DNTQVLYRGDERFPMCSTSKVMAAAAVLEQSETQKQLLNQPVEIQPADLVNYNPIA
EKHVNGTMTLAELSAAALQYSDHTAMNKLIAQLGGPGGVTAFARAIGDETFRLDRT
EPTLNTAIPGDPRDTTTPRAMAQTLRQLTLGHALGETQRAQLVTWLKGNTTGAASI
RAGLPTSWTVGDKTGSGDYGTTNDIAVIWPQGRAPLVLVTYFTQPQQNAESRRDV
LASAARIIAEGL
**SEQ ID N° 404** : 
MVKKSLRQFTLMATATVTLLLGSVPLHAQTVDVQQKLAELERQSGGRLGVALINTA
DNSQILYRADERFAMCSTSKVMAAAAVLKKSESEPNLLNQRVEIKKSDLVNYNPIAE
KHVNGTMSLAELSAAALQYSDNVAMNKLIAHVGGPASVTAFARQLGDDTFRLDRTE
PTLNTAIPGDPRDTTSPRAMAQTLRNLTLGKALGDSQRAQLVTWMKGNTTGAASIQ
AGLPASWVVGDKTGSGDYGTTNDIAVIWPKDRAPLILVTYFTQPQPKAESRRDVLA
SAAKIVTDGL
**SEQ ID N° 405** : 
MVKKSLRQFTLMATATVTLLLGSVPLYAQTVDVQQKLAELERQSGGRLGVALINTAD
NSQILYRADERFAMCSTSKVMAAAAVLKKSESEPNLLNQRVEIKKSDLVNYNPIAEK
HVNGTMSLAELSAAALQYSDNVAMNKLIAHVGGPASVTAFARQLGDETFRLDRTEP
TLNTAIPGDPRDTTSPRAMAQTLRNLTLGKALGDSQRAQLVTWMKGNTTGAASIQA
GLPASWVVGDKTGSGDYGTTNDIAVIWPKDRAPLILVTYFTQPQPKAESRRDVLAS
AAKIVTDGL
**SEQ ID N° 406** : 
MVKKSLRQFTLMATATVTLLLGSVPLYAQTADVQQKLAELERQSGGRLGVALINTAD
NSQILYRADERFAMCSTSKVMAVAAVLKKSESEPNLLNQRVEIKKSDLVNYNPIAEK
HVNGTMSLAELSAAALQYSDNVAMNKLIAHVGGPASVTAFARQLGDETFRLDRTEP
TLNTAIPGDPRDTTSPRAMAQTLRNLTLGKALGDSQRAQLVTWMKGNTTGAASIQA
 
GLPASWVVGDKTGSGGYGTTNDIAVIWPKDRAPLILVTYFTQPQPNAESRRDVLAS
AAKIVTNGL
**SEQ ID N° 407** : 
MVTKRVQRMMFAAAACIPLLLGSAPLYAQTSAVQQKLAALEKSSGGRLGVALIDTA
DNTQVLYRGDERFPMCSTSKVMAVAAVLKQSETQKQLLNQPVEIKPADLVNYNPIA
EKHVNGTMTLAELSAAALQYSDNTAMNKLIAQLGGPGGVTAFARAIGDETFRLDRT
ELTLNTAIPGDPRDTTTPRAMAQTLRQLTLGHALGETQRAQLVTWLKGNTTGAASIR
AGLPTSWTVGDKTGSGDYGTTNDIAVIWPQGRAPLVLVTYFTQPQQNAESRRDVL
ASAARIIAEGL
**SEQ ID N° 408** : 
MVTKRVQRMMFAAAACIPLLLGSAPLYAQTSAVQQKLAALEKSSGGRLGVALIDTA
DNTHVLYRGDERFPMCSTSKVMAAAAVLKQSETQKQLLNQPVEIKPADLVNYNPIA
EKHVNGTMTLAELSAAALQYSDNTAMNKLIAQLGGPGGVTAFARAIGDETFRLDRT
EPTLNTAIPGDPRDTTTPRAMAQTLRQLTLGHALGETQRAQLVTWLKGNTTGAASI
RAGLPTSWTVGDKTGSGDYGTTNDIAVIWPQGRAPLVLVTYFTQPQQNAESRRDV
LASAARIIAEGL
**SEQ ID N° 409** : 
MVTKRVQRMMFAAAACIPLLLGSAPLYAQTSAVQQKLAALEKSSGGRLGVALIDTA
DNTQVLYRGDERFPMCSTSKVMAAAAVLKQSETQKQLLNQPVEIKPADLVNYNPIA
EKHVNGTMTLAELSAAALQYSDNTAMNKLIAQLGGPGGVTAFARAIGDETFRLDRT
EPTLNTAIPGDPRDTTTPRAMAQTLRQLTLGHALGETQRAQLVAWLKGNTTGAASI
RAGLPTSWTVGDKTGSGDYGTTNDIAVIWPQGRAPLVLVTYFTQPQQNAESRRDV
LASAARIIAEGL
**SEQ ID N° 410** : 
MVTKRVQRMMFAAAACIPLLLGSAPLYAQTSAVQQKLAALEKSSGGRLGVALIDTA
DNTQVLYRGDERFPMCSTSKVMAAAAVLKQSETQKQLLNQPVEIKPADLVNYNPIA
EKHVNGTMTPAELSAAALQYSDNTAMNKLIAQLGGPGGVTAFARAIGDETFRLDRT
EPTLNTAIPGDPRDTTTPRAMAQTLRQLTLGHALGETQRAQLVTWLKGNTTGAASI
RAGLPTSWTVGDKTGSGDYGTTNDIAVIWPQGRAPLVLVTYFTQPQQNAESRRDV
LASAARIIAEGL
**SEQ ID N° 411** : 
MVTKRVQRMMFAAAACIPLLLGSAPLYAQTSAVQQKLAALEKSSGGRLGVALIDTA
DNTQVLYRGDERFPMCSTSKVMAAAAVLKQSETQKQLLNQPVEIKPADLVNYNPFA
EKHVNGTMTLAELSAAALQYSDNTAMNKLIAQLGGPGGVTAFARAIGDETFRLDRT
EPTLNTAIPGDPRDTTTPRAMAQTLRQLTLGHALGETQRAQLVTWLKGNTTGAASI
RAGLPTSWTVGDKTGSGDYGTTNDIAVIWPQGRAPLVLVTYFTQPQQNAESRRDV
LASAARIIAEGL
**SEQ ID N° 412** : 
MVKKSLRQFTLMATATVTLLLGSVPLYAQTADVQQKLAELERQSGGRLGVALINTAD
NSQILYRADERFAMCSTSKVMAAAAVLKKSESEPNLLNQRVEIKKSDLVNYNPIAEK
HVNGTMSLAELSAAALQYSDNVAMNKLIAHVGGPASVTAFARQLGDETFRLDRTEP
TLNTAIPGDPRDTTSPRAMAQTLRNLTLGKALGDSQRAQLVTWMKGNTTGAASIQA
GLPASWVVGDKTGSGGYGTTNDIAVIWPKDRAPLILVTYFTQPQPKAESRHDVLAS
AAKIVTDGL
**SEQ ID N° 413** : 
MVTKRVQRMMFAAAACIPLLLGSAPLYAQTSAVQQKLAALEKSSGGRLGVALIDTA
DNTQVLYRGDERFPMCSTSKVMAVAAVLKQSETQKQLLNQPVEIKPADLVNYNPIA
EKHVNGTMTLAELSAAALQYSDNTAMNKLIAQLGGPGGVTAFARAIGDETFRLDRT
EPTLNTAIPGDPRDTTTPRAMAQTLRQLTLGHALGETQRAQLVTWLKGNTTGAASI
 
RAGLPTSWTVGDKTGSGDYGTTNDIAVIWPQGRAPLVLVTYFTQPQQNAESRRDV
LASAARIIAEGL
**SEQ ID N° 414** : 
MMRKSVRRAMLMTTACVSLLLASVPLCAQANDVQQKLAALEKSSGGRLGVALINTA
DNTQTLYRADERFAMCSTSKVMAVAAVLKQSETQKGLLSQRVEIKPSDLINYNPIAE
KHVNGTMTFGELSAAALQYSDNTAMNKLIAHLGGPDKVTAFARTIGDDTFRLDRTE
PTLNTAIPGDPRDTTTPLAMAQALRNLTLGNALGDTQRAQLVMWLKGNTTGAASIQ
AGLPTSWVVGDKTGSGDYGTTNDIAVIWPEGRAPLVLVTYFTQSEPKAESRRDVLA
AAARIVTDGY
**SEQ ID N° 415** : 
MMTQSIRRSMLTVMATLPLLFSSATLHAQANSVQQQLEALEKSSGGRLGVALINTA
DNSQILYRADERFAMCSTSKVMAAAAVLKQSESDKHLLNQRVEIKKSDLVNYNPIAE
KHVNGTMTLAELGAAALQYSDNTAMNKLIAHLGGPDKVTAFARSLGDETFRLDRTE
TTLNTAIPGDPRDTTTPLAMAQTLKNLTLGKALAETQRAQLVTWLKGNTTGSASIRA
GLPKSWVVGDKTGSGDYGTTNDIAVIWPENHAPLVLVTYFTQPEQKAESRRDILAA
AAKIVTHGF
**SEQ ID N° 416** : 
MMTQSIRRSMLTVMATLSLLFSSATLHAQANSVQQQLEALEKSSGGRLGVALINTA
DNSQILYRADERFAMCSTSKVMAAAAVLKQSESDKHLLNQRVEIKKSDLVNYNPIAE
KHVNGTMTLAELGAAALQYSDNTAMNKLIAHLGGPDKVTAFARSLGDETFRLDRTE
PTLNTAIPGDPRDTTTPLAMAQTLKNLTLGKALAETQRAQLVTWLKGNTTGSASIRA
GLPKSWVVGDKTGSGDYGTTNDIAVIWPENHAPLVLVTYFTQPEQKAESRRDILAA
AAKIVTHGF
**SEQ ID N° 417** : 
MVKKSLRQFTLMATATVTLLLGSVPLYAQTADVQQKLAELERQSGGRLGVALINTAD
NSQILYRADERFAMCSTSKVMAAAAVLKKSESEPNLLNQRVEIKKSDLVNYNPIAEK
HVNGTMSLAELSAAALQYSDNVAMNKLIAHVGGPASVTAFARQLGDETFRLDRTEP
TLNTAIPGDPRDTTSPRAMAQTLRNLTLGKALGDSQRAQLVTWMKGNTTGAASIQA
GLPASWVVGDKTGSCGYGTTNDIAVIWPKDRAPLILVTYFTQPQPKAESRRDVLAS
AAKIVTDGL
**SEQ ID N° 418** : 
MMRKSVRRAMLMTTACVSLLLASVPLCAQANDVQQKLAALEKSSGGRLGVALINTA
DNTQTLYRADERFAMCSTSKVMAVAAVLKQSETQKGLLSQRVEIKPSDLINYNPIAE
KHVNGTMTFGELSAAALQYSDNTAMNKLIAHLGGPDKVTAFARTIGDDTFRLDRTE
PTLNTAIPGDPRDTTTPLAMAQSLRNLTLGNALGDTQRAQLVMWLKGNTTGAASIQ
AGLPTSWVVGDKTGSGDYGTTNDIAVIWPEGRAPLVLVTYFTQSEPKAESRRDVLA
AAARIVTDGY
**SEQ ID N° 419** : 
MMTQSIRRSMLTVMATLPLLFSSATLHAQANSVQQQLEALEKSSGGRLGVALINTA
DNSQILYRADERFAMCSTSKVMAAAAVLKQSESDKHLLNQRVEIKKSDLVNYNPIAE
KHVNGTMTLAELGAAALQYSDNTAMNKLIAHLGGPDKVTAFARSLGDETFRLDRTE
PTLNTAIPGDPRDTTTPLAMAQTLKNLTLGKALAETQRTQLVTWLKGNTTGSASIRA
GLPKSWVVGDKTGSGDYGTTNDIAVIWPENHAPLVLVTYFTQPEQKAESRRDILAA
AAKIVTHGF
**SEQ ID N° 420** : 
MMRKSVRRAMLMTTACVSLLLASVPLCAQANDVQQKLAALEKSSGGRLGVALINTA
DNTQTLYRADERFAMCSTSKVMAAAAVLKQSETQKGLLSQRVEIKPSDLINYNPIAE
KHVNGTMTLGELSAAALQYSDNTAMNKLIAHLGGPDKVTAFARTIGDDTFRLDRTEP
TLNTAIPGDPRDTTTPLAMAQALRNLTLGNALGDTQRAQLVMWLKGNTTGAASIQA
 
GLPTSWVVGDKTGSGGYGTTNDIAVIWPEGRAPLVLVTYFTQSEPKAESRRDVLAA
AARIVTDGY
**SEQ ID N° 421** : 
MVTKRVQRMMFAAAACIPLLLGSAPLYAQTSAVQQKLAALEKSSGGRLGVALIDTA
DNTQVLYRGDERFPMCSTSKVMAAAAVLKQSETQKQLLNQPVEIKPADLVNYNPIA
EKHVNGTMTLAELSAAALQYSDNTAMNKLIAQLGGPGGVTAFARAIGDETFRLDRT
ESTLNTAIPGDPRDTTTPRAMAQTLRQLTLGHALGETQRAQLVTWLKGNTTGAASI
RAGLPTSWTVGDKTGSGDYGTTNDIAVIWPQGRAPLVLVTYFTQPQQNAERRRDV
LASAARIIAEGL
**SEQ ID N° 422** : 
MMTQSIGRSMLTVMATLPLLFSSATLHAQANSVQQQLEALEKSSGGRLGVALINTA
DNSQILYRADERFAMCSTSKVMAAAAVLKQSESDKHLLNQRVEIKKSDLVNYNPIAE
KHVNGTMTLAELGAAALQYSDNTAMNKLIAHLGGPDKVTAFARSLGDETFRLDRTE
PTLNTAIPGDPRDTTTPLAMAQTLKNLTLGKALAETQRAQLVTWLKGNTTGSASIRA
GLPKSWVVGDKTGSGDYGTTNDIAVIWPENHAPLVLVTYFTQPEQKAESRRDILAA
AAKIVTHGF
**SEQ ID N° 423** : 
MVTKRVQRMMFAAAACIPLLLGSAPLYAQTSAVQQKLAALEKSSGGRLGVALIDTA
DNTQVLYRGDERFPMCSTSKVMAVAAVLKQSETQKQLLNQPVEIKPADLVNYNPIA
EKHVNGTMTLAELSAAALQYSDNTAMNKLIAQLGGPGGVTAFARAIGDETFRLDRT
EPTLNTAIPGDPRDTTTPRAMAQTLRQLTLGHALGETQRAQLVTWLKGNTTGAASI
RAGLPTSWTVGDKTGSGGYGTTNDIAVIWPQGRAPLVLVTYFTQPQQNAESRRDV
LASAARIIAEGL
**SEQ ID N° 424** : 
MMTQSIRRSMLTVMATLPLLFSSATLHAQANSVQQQLEALEKSSGGRLGVALINTA
DNSQILYRADERFAMCSTSKVMAAAAVLKQSESDKHLLNQRVEIKKSDLVNYNPIAE
KHVNGTMTLAELGAAALQYSDNTAMNKLIAHLGGPDKVTAFARSLGDETFRLDRTE
PTLNTAIPGDPRDTTTPLAMAQTLKNLTLGKALAETQRAQLVTWLKGNTTGSASIRA
GLPKSWVVGDKTGSGDYGTTNDIAVIWPENHAPLVLVTYFTQPEQKAERRRDILAA
AAKIVTHGF
**SEQ ID N° 425** : 
MVTKRVQRMMSAAAACIPLLLGSPTLYAQTSAVQQKLAALEKSSGGRLGVALIDTA
DNTQVLYRGDERFPMCSTSKVMAAAAVLKQSETQKQLLNQPVEIKPADLVNYNPIA
EKHVNGTMTLAELSAAALQYSDNTAMNKLIAQLGGPGGVTAFARAIGDETFRLDRT
EPTLNTAIPGDPRDTTTARAGADVASLRWVMRWAKPSGAVGDVAQRQYDRAAGIR
AGLPTSWTVGDKTGSGDYGTTNDIAVIWPQGRAPLVLVTYFTQPQQNAESRRDVL
ASAARIIAEGL
**SEQ ID N° 426** : 
MVKKSLRQFTLMATATVTLLLGSVPLYAQTADVQQKLAELERQSGGRLGVALINTAD
NSQILYRADERFAMCSTSKVMAAAAVLKKSESEPNLLNQRVEIKKSDLVNYNPIAEK
HVDGTMSLAELSAAALQYSDNVAMNKLISHVGGPASVTAFARQLGDETFRLDRTEP
TLNTAIPGDPRDTTSPRAMAQTLRNLTLGKALGDSQRAQLVTWMKGNTTGAASIQA
GLPASWVVGDKTGSGDYGTTNDIAVIWPKDRAPLILVTYFTQPQPKAESRRDVLAS
AAKIVTNGL
**SEQ ID N° 427** : 
MRHRVKRMMLMTTACISLLLGSAPLYAQANDVQQKLAALEKSSGGRLGVALIDTAD
NAQTLYRADERFAMCSTSKVMAAAAVLKQSETQKNVLSQKVEIKSSDLINYNPIAEK
HVNGTMTLAELSAAALQYSDNTAMNKLIAHLGGPDKVTAFARAIGDDTFRLDRTEPT
LNTAIPGDPRDTTTPLAMAQTLRHLTLGSALGETQRAQLVTWLKGNTTGAASIQAGL
 
PTSWVVGDKTGSGDYGTTNDIAVIWPEGRAPLILVTYFTQPEQKAENRRDVLAAAA
KIVTDGY
**SEQ ID N° 428 :** 
MVKKSLRQFTLMATATVTLLLGSVPLYAQTADVQQKLAELERQSGGRLGVALINTAD
NSQILYRADERFPMCSTSKVMAAAAVLKQSETQKQLLNQPVEIKPADLVNYNPIAEK
HVNGTMTLAELSAAALQYSDNTAMNKLIAQLGGPGGVTAFARAIGDETFRLDRTEP
TLNTAIPGDPRDTTTPRAMAQTLRQLTLGHALGETQRAQLVTWLKGNTTGAASIRA
GLPASWVVGDKTGSGGYGTTNDIAVIWPKDRAPLILVTYFTQPQPKAESRRDVLAS
AAKIVTDGL
**SEQ ID N° 429** : 
MMTQSIRRSMLTVMATLPLLFSSATLHAQANSVQQQLEALEKSSGGRLGVALINTA
DNSQILYLADERFAMCSTSKVMAAAAVLKQSESDKHLLNQRVEIKASDLVNYNPIAE
KHVNGTMTLAELGAGALQYSDNTAMNKLIAHLGGPDKVTAFARSLGDETFRLDRTE
PTLNSAIPGDPRDTTTPLAMAQTLKNLTLGKALAETQRAQLVTWLKGNTTGSASIRA
GLPKCWVVGDKTGSGDYGTTNDIAVIWPENHAPLVLVTYFTQPEQKAESRRDVLAA
AAKIVTHGF
**SEQ ID N° 430** : 
MMTQSIRRSMLTVMATLPLLFSSATLHAQANSVQQQLEALEKSSGGRLGVALINTA
DNSQILYVADERFAMCSTSKVMAAAAVLKQSESDKHLLNQRVEIRASDLVNYNPIAE
KHVNGTMTLAQLGAGALQYSDNTAMNKLIAHLGGPDKVTAFARSLGDETFRLDRTE
PTLNSAIPGDPRDTTTPLAMAQTLKNLTLGKALAETQRAQLVTWLKGNTTGSASIRA
GLPKSWGVGDKTGSGDYGTTNDIAVIWPENHAPLVLVTYFTQPEQKAESRRDVLAA
AAKIVTHGF
**SEQ ID N° 431** : 
MVKKSLRQFTLMATATVTLLLGSVPLYAQTADVQQKLAELERQSGGRLGVALINTAD
NSQILYRADERFAMCSTSKVMAVAAVLKKSESEPNLLNQRVEIKKSDLVNYNPIAEK
HVDGTMSLAELSAAALQYSDNVAMNKLISHVGGPASVTAFARQLGDETFRLDRTEP
TLNTAIPGDPRDTTSPRAMAQTLRNLTLGKALGDSQRAQLVTWMKGNTTGAASIQA
GLPASWVVGDKTGSGDYGTTNDIAVIWPKDRAPLILVTYFTQPQPKAESRRDVLAS
AAKIVTNGL
**SEQ ID N° 432** : 
MMTQSIRRSMLTVMATLPLLFSSATLHAQANSVQQQLEALEKSSGGRLGVALINTA
DNSQILYRADERFAMCSTSKVMAAAAVLKQSESDKHLLNQRVEIKKSDLVNYNPIAE
KHVNGTMTLAELGAAALQYSDNTAMNKLIAHLGGPDKVTAFARSLGDETFRLDRTE
PTLNTAIPGDPRDTTTPLAMAQTLKNLTLGKALAETQRAQLVTWLKGNTTGSASIRA
GLPKSWVVGDKTGSGDYGTTNDIAVIWPENHAPLVLVTYFTQPEQKAESRRDILAA
AAKIVTHGF
**SEQ ID N° 433** : 
MVKKSLRQFTLMATATVTLLLGSVPLYAQTADVQQKLAELERQSGGRLGVALINTAD
NSQILYRADERFAMCSTSKVMAAAAVLKKSESEPNLLNQRVEIKKSDLVNYNPIAEK
HVNGTMSLAELSAAALQYSDNVAMNKLIAHVGGPASVTAFARQLGDETFRLDRTEP
TLNTAIPGDPRDTTSPRAMAQTLRNLTLGKALGDSQRAQLVTWMKGNTTGAASIQA
GLPASWVVGDKTGSGDYGTTNDIAVIWPKDRAPLILVTYFTQPQPKAESRRDVLAS
AAKIVTDGL
**SEQ ID N° 434** : 
MMTQSIRRSMLTVMATLPLLFSSATLHAQANSVQQQLEALEKSSGGRLGVAQINTA
DNSQILYVADERFAMCSTSKVMAAAAVLKQSESDKHLLNQRVEIRASDLVNYNPIAE
KHVNGTMTLAELGAGALQYSDNTAMNKLIAHLGGPDKVTAFARSLGDETFRLDRTE
PTLNSAIPGDPRDTTTPLAMAQTLKNLTLGKALAETQRAQLVTWLKGNTTGSASIRA
 
GMPKSWGVGDKTGSGDYGTTNDIAVIWPENHAPLVLVTYFTQPEQKAESRRDILAA
AAKIVTHGF
**SEQ ID N° 435** : 
MMTQSIRRSMLTVMATLPLLFSSATLHAQANSVQQQLEALEKSSGGRLGVALINTA
DNSQILYRADERFAMCSTSKVMAAAAVLKQSESDKHLLNQRVEIKKSDLVNYNPIAE
KHVNGTMTLAELGAAALQYSDNTAMNKLIAHLGGPDKVTAFARSLGDETFRLDRTE
PTLNTAIPGDPRDTTTPLAMAQTLKNLTLGKALAETQRAQLVTWLKGNTTGSASIRA
GLPKSWVVGDKTGSGDYGTTNDIAVIWPENHAPLVLVTYFTQPEQKAESRRDFLAA
AAKIVTHGF
**SEQ ID N° 436** : 
MVTKRVQRMMFAGGAGIPLLLGSAPFYAQTSAGQQKLAALEKSSGGRLGVALIDTA
DNTQVLYRGDERFPMCSTSKVMAAAAVLKQSETQKQLLNQPVEIKPADLVNYNPIA
EKHVNGTMTLAELSAAALQYSDNTAMNKLIAQLGGPGGVTAFARAIGDETFRLDRT
EPTLNTAIPGDPRDTTTPRAMAQTLRQLTLGHALGETQRAQLVTWLKGNTTGAASI
RAGLPTSWTVGDKTGSGDYGTTNDIAVIWPQGRAPLVLVTYFTQPQQNAESRRDV
LASAARIIAEGL
**SEQ ID N° 437** : 
[?]
MVKKSLRQFTLMATATVTLLLGSVPLYAQTADVQQKLAELERQSGGRLGVALINTAD
NSQILYRADERFAMCSTSKVMAAAAVLKKSESEPNLLNQRVEIKKSDLVNYNPIAEK
HVNGTMSLAELSAAALQYSDNVAMNKLIAHVGGPASVTAFARQLGDETFRLDRTEP
TLNTAIPGDPRDTTSPRAMAQTLRNLTLGKALGDSQRAQLVTWMKGNTTGAASIQA
GLPASWVVGDKTGSGGYGTTNDIAVIWPKDRAPLILVTYFTQPQPKAESRRDVLAS
AAKIVTNGL
**SEQ ID N° 438** : 
MVKKSLRQFTLMATATVTLLLGSVPLYAQTADVQQKLAELERQSGGRLGVALINTAD
NSQILYRADERFAMCSTSKVMAVAAVLKKSESEPNLLNQRVEIKKSDLVNYNPIAEK
HVDGTMSLAELSAAALQYSDNVAMNKLISHVGGPASVTAFARQLGDETFRLDRTEP
TLNTAIPGDPRDTTSPRAMAQTLRNLTLGKALGDSQRAQLVTWMKGNTTGAASIQA
GLPASWVVGDKTGSGGYGTTNDIAVIWPKDRAPLILVTYFTQPQPKAESRRDVLAS
AAKIVTNGL
**SEQ ID N° 439** : 
MMTQSIRRSMLTVMATLPLLFSSATLHAQANSVQQQLEALEKSSGGRLGVALINTA
DNSQILYRADERFAMCSTSKVMAAAAVLKQSESDKHLLNQRVEIKKSDLVNYNPIAE
KHVNGTMTLAELGAAALQYSDNTAMNKLIAHLGGPDKVTAFARSLGDESFRLDRTE
PTLNTAIPGDPRDTTTPLAMAQTLKNLTLGKALAETQRAQLVTWLKGNTTGSASIRA
GLPKSWVVGDKTGSGDYGTTNDIAVIWPENHAPLVLVTYFTQPEQKAESRRDILAA
AAKIVTHGF
**SEQ ID N° 440** : 
MVKKSLRQFTLMATAAVTLLLGSVPLYAQTADVQQKLAELERQSGGRLGVALINTA
DNSQILYRADERFAMCSTSKVMAAAAVLKKSESEPSLLNQRVEIKKSDLVNYNPIAE
KHVNGTMSLAELSAAALQYSDNVAMNKLIAHVGGPASVTAFARQLGDETFRLDRTE
PTLNTAIPGDPRDTTSPRAMAQTLRNLTLGKALGDSQRAQLVTWMKGNTTGAASIQ
AGLPASWVVGDKTGSGGYGTTNDIAVIWPKDRAPLILVTYFTQPQPKAESRRDILAS
AAKIVTDGL
**SEQ ID N° 441** : 
MVKKSLRQFTLMATAAVTLLLGSVPLYAQTADVQQKLAELERQSGGRLGVALINTA
DNSQILYRADERFAMCSTSKVMAVAAVLKKSESEPSLLNQRVEIKKSDLVNYNPIAE
KHVNGTMSLAELSAAALQYSDNVAMNKLIAHVGGPASVTAFARQLGDETFRLDRTE
PTLNTAIPGDPRDTTSPRAMAQTLRNLTLGKALGDSQRAQLVTWMKGNTTGAASIQ
 
AGLPASWVVGDKTGSGDYGTTNDIAVIWPKDRAPLILVTYFTQPQPKAESRRDILAS
AAKIVTDGL
**SEQ ID N° 442** : 
MMTQSIRRSMLTVMATLPLLFSSATLHAQTNSVQQQLKALEKSSGGRLGVALINTAD
NSQILYRADERFAMCSTSKVMAAAAVLKQSESDKHLLNQRVEIKKSDLVNYNPIAEK
HVNGTMTLAELGAAALQYSDNTAMNKLIAHLGGPDKVTAFARSLGDETFRLDRTEP
TLNTAIPGDPRDTTTPLAMAQTLKNLTLGKALAETQRAQLVTWLKGNTTGSASIQAG
LPKSWVVGDKTGSGDYGTTNDIAIIWPENHAPLVLVTYFTQPEQKAESRRDVLAAAA
KIVTRGF
**SEQ ID N° 443** : 
MMRKSVRRAILMTTACVSLLLASVPLYAQANDIQQKLAALEKSSGGRLGVALINTAD
NTQTLYRADERFAMCSTSKVMAAAAVLKQSETQKDLLSQRVEIKSSDLINYNPIAEK
HVNGTMTLGELSAAALQYSDNTAMNKLIAHLGGPGKVTAFARVIGDDTFRLDRTEP
TLNTAIPGDPRDTTTPLAMAQTLRNLTLGNALGDTQRAQLVTWLKGNTTGAASIQA
GLPTSWVVGDKTGSGDYGTTNDIAVIWPEGRAPLVLVTYFTQPEPKAESRRDVLAA
AARIVTDGY
**SEQ ID N° 444** : 
MMTQSIRRSMLTVMATLPLLFSSATLHAQTNSVQQQLEALEKSSGGRLGVALINTAD
NSQILYRADERFAMCSTSKVMAAAAVLKQSESDKHLLNQRVEIKKSDLVNYNPIAEK
HVNGTMTLAELGAAALQYSDNTAMNKLIAHLGGPDKVTAFARSLGDETFRLDRTEP
TLNTAIPGDPRDTTTPLAMAQTLKNLTLGKALAETQRAQLVTWLKGNTTGSASIRAG
LPKSWGVGDKTGSGDYGTTNDIAVIWPENHAPLVLVTYFTQPEQKAESRRDVLAAA
AKIVTHGF
**SEQ ID N° 445** : 
MMTQSIRRSMLTVMATLPLLFSSATLHAQTNSVQQQLEALEKSSGGRLGVALINTAD
NSQILYRADERFAMCSTSKVMAAAALLKQSESDKHLLNQRVEIKKSDLVNYNPIAEK
HVNGTMTLAELGAAALQYSDNTAMNKLIAHLGGPDKVTAFARSLGDETFRLDRTEP
TLNTAIPGDPRDTTTPLAMAQTLKNLTLGKALAETQRAQLVTWLKGNTTGSASIQAG
LPKSWVVGDKTGSGDYGTTNDIAVIWPENHAPLVLVTYFTQPEQKAESRRDVLAAA
AKIVTHGF
**SEQ ID N°1871** : 
MVTKRVQRMMFAAAACIPLLLGSAPLYAQTSAVQQKLAALEKSSGGRLGVALIDTA
DNTQVLYRGDERFPMCSTSKVMAAAAVLKQSETQKQLLNQPVEIKPADLVNYNPIA
EKHVNGTMTLAELSAAALQYSDNTAMNKLIAQLGGPGGVTAFARAIGDETFRLDRT
EPTLNTAIPGDPRDTTTPRAMAQTLRQLTLGHALGETQRAQLVTWLKGNTTGAASI
RAGLPTSWTVGDKTGSGDYGTTNDIAVIWPQGRAPLVLVTYFTQPQQNAESRRDV
LASAARIIAEGL
**SEQ ID N°1872** : 
MVTKRVQRMMFAAAACIPLLLGSAPLYAQTSAVQQKLAALEKSSGGRLGVALIDTA
DNTQVLYRGDERFPMCSTSKVMAAAAVLKQSETQKQLLNQPVEIKPADLVNYNPIA
EKHVNGTMTLAELSAAALQYSDNTAMNKLIAQLGGPGGVTAFARAIGDETFRLDRT
ESTLNTAIPGDPRDTTTPRAMAQTLRQLTLGHALGETQRAQLVTWLKGNTTGAASI
RAGLPTSWTVGDKTGSGDYGTTNDIAVIWPQGRAPLVLVTYFTQPQQNAESRRDV
LASAARIIAEGL
**SEQ ID N°1873** : 
MVTKRVQRMMFAAAACIPLLLGSAPLYAQTSAVQQKLAALEKSSGGRLGVALIDTA
DNTQVLYRGDERFPMCSTSKVMAAAAVLKQSETQKQLLNQPVEIKPADLVNYNPIA
EKHVNGTMTLAELSAAALQYSDNTAMNKLIAQLGGPGGVTAFARAIGDETFRLDRT
EPTLNTAIPGDPRDTTTPRAMAQTLRQLTLGHALGETQRAQLVTWLKGNTTGAASI
 
RAGLPTSWTVGDKTGSGDYGTTNDIAVIWPQGRAPLVLVTYFTQPQQNAESRRDV
LASAARIIAKGL
**SEQ ID N°1874** : 
MVKKSLRQFTLMATATVTLLLGSVPLYAQTADVQQKLAELERQSGGRLGVALINTAD
NSQILYRADERFAMCSTSKVMAAAAVLKKSESEPNLLNQRVEIKKSDLVNYNPIAEK
HVNGTMSLAELSAAALQYSDNVAMNKLIAHVGGPASVTAFARQLGDETFRLDRTEP
TLNTAIPGDPRDTTSPRAMAQTLRNLTLGKALGDSQRAQLVTWMKGNTTGAASIQA
GLPASWVVGDKTGSGGYGTTNDIAVIWPKDRAPLILVTYFTQPQPKAESRRDVLAS
AAKIVTDGL
**SEQ ID N°1875** : 
MVKKSLRQFTLMATATVTLLLGSVPLYAQTADVQQKLAELERQSGGRLGVALINTAD
NSQILYRADERFAMCSTSKVMAAAAVLKKSESEPNLLNQRVEIKKSDLVNYNPIAEK
HVNGTMSLAELSAAALQYSDNVAMNKLIAHVGGPASVTAFARQLGDETFRLDRTEP
TLNTAIPGDPRDTTSPRAMAQTLRNLTLGKALGDSQRAQLVTWMKGNTTGAASIQA
GLPASWVVGDKTGSGDYGTTNDIAVIWPKDRAPLILVTYFTQPQPKAESRRDVLAS
AAKIVTNGL
**SEQ ID N°1876** : 
MVTKRVQRMMFAAAACIPLLLGSAPLYAQTSAVQQKLAALEKSSGGRLGVALIDTA
DNTQVLYRGDERFPMCSTSKVMAAAAVLKQSETQKQLLNQPVEIKPADLVNYNPIA
EKHVNGTMTLAELSAAALQYSDNTAMNKLIAQLGGPGGVTAFARAIGDETFRLDRT
EPTLNTAIPGDPRDTTTPRAMAQTLRQLTLGHALGETQRAQLVTWLKGNTTGAASI
RAGLPTSWTVGDKTGSGDYGTTNDIAVIWPQGRAPLVLVTYFTQPQQNAERRRDV
LASAARIIAEGL
**SEQ ID N°1877** : 
MRHRVKRMMLMTTACISLLLGSAPLYAQANDVQQKLAALEKSSGGRLGVALIDTAD
NAQTLYRADERFAMCSTSKVMAAAAVLKQSETQKNVLSQKVEIKSSDLINYNPIAEK
HVNGTMTLAELSAAALQYSDNTAMNKLIAHLGGPDKVTAFARAIGDDTFRLDRTEPT
LNTAIPGDPRDTTTPLAMAQTLRHLTLGSALGETQRAQLVTWLKGNTTGAASIQAGL
PTSWVVGDKTGSGDYGTTNDIAVIWPEGRAPLILVTYFTQPEQKAESRRDVLAAAA
KIVTDGY
**SEQ ID N°1878** : 
MVKKSLRQFTLMATATVTLLLGSVPLYAQTADVQQKLAELERQSGGRLGVALINTAD
NSQILYRADERFAMCSTSKVMAVAAVLKKSESEPNLLNQRVEIKKSDLVNYNPIAEK
HVNGTMSLAELSAAALQYSDNVAMNKLIAHVGGPASVTAFARQLGDETFRLDRTEP
TLNTAIPGDPRDTTSPRAMAQTLRNLTLGKALGDSQRAQLVTWMKGNTTGAASIQA
GLPASWVVGDKTGSGGYGTTNDIAVIWPKDRAPLILVTYFTQPQPKAESRRDVLAS
AAKIVTDGL
**SEQ ID N°1879** : 
MVKKSLRQFTLMATATVTLLLGSVPLYAQTADVQQKLAELERQSGGRLGVALINTAD
NSQILYRADERFAMCSTSKVMAVAAVLKKSESEPNLLNQRVEIKKSDLVNYNPIAEK
HVDGTMSLAELSAAALQYSDNVAMNKLISHVGGPASVTAFARQLGDETFRLDRTEP
TLNTAIPGDPRDTTSPRAMAQTLRNLTLGKALGDSQRAQLVTWMKGNTTGAASIQA
GLPASWVVGDKTGSGDYGTTNDIAVIWPKDRAPLILVTYFTQPQPKAESRRDVLAS
AAKIVTDGL
**SEQ ID N°1880** : 
MMTQSIRRSMLTVMATLPLLFSSATLHAQANSVQQQLEALEKSSGGRLGVALINTA
DNSQILYRADERFAMCSTSKVMAAAAVLKQSESDKHLLNQRVEIKKSDLVNYNPIAE
KHVNGTMTLAELGAAALQYSDNTAMNKLIAHLGGPDKVTAFARSLGDETFRLDRTE
PTLNTAIPGDPRDTTTPLAMAQTLKNLTLGKALAETQRAQLVTWLKGNTTGSASIRA
 
GLPKSWVVGDKTGSGDYGTTNDIAVIWPENHAPLVLVTYFTQPEQKAENRRDILAA
AAKIVTHGF
**SEQ ID N°1881** : 
MVTKRVQRMMFAAAACIPLLLGSAPLYAQTSAVQQKLAALEKSSGGRLGVALIDTA
DNTQVLYRGDERFPMCSTSKVMAVAAVLKQSETQKQLLNQPVEIKPADLVNYNPIA
EKHVNGTMTLAELSAAALQYSDNTAMNKLIAQLGGPGGVTAFARAIGDETFRLDRT
EPTLNTAIPGDPRDTTTPRAMAQTLRQLTLGHALGETQRAQLVTWLKGNTTGAASI
RAGLPTSWTVGDKTGSGDYGTTNDIAVIWPQGRAPLVLVTYFTQPQQNAERRRDV
LASAARIIAEGL
**SEQ ID N°1882** : 
MVKKSLRQFTLMATATVTLLLGSVPLYAQTADVQQKLAELERQSGGRLGVALINTAD
NSQILYRADERFAMCSTSKVMAVAAVLKKSESEPNLLNQRVEIKKSDLVNYNPIAEK
HVNGTMSLAELSAAALQYSDNVAMNKLIAHVGGPASVTAFARQLGDETFRLDRTEP
TLNTAIPGDPRDTTSPRAMAQTLRNLTLGKALGDSQRAQLVTWMKGNTTGAASIQA
GLPASWVVGDKTGSGGYGTTNDIAVIWPKDRAPLILVTYFTQPQPKAESRRDVLAS
AAKIVTNGL
**SEQ ID N°1883** : 
MVKKSLRQFTLMATATVTLLLGSVPLYAQTADVQQKLAELERQSGGRLGVALINTAD
NSQILYRADERFAMCSTSKVMAVAAVLKKSESEPNLLNQRVEIKKSDLVNYNPIAEK
HVNGTMSLAELSAAALQYSDNVAMNKLIAHVGGPASVTAFARQLGDETFRLDRTEP
TLNTAIPGDPRDTTSPRAMAQTLRNLTLGKALGDSQRAQLVTWMKGNTTGAASIQA
GLPASWVVGDKTGSGGYGTTNDIAVIWPKDRAPLILVTYFTQPQPNAESRRDVLAS
AAKIVTNGL
**SEQ ID N°1884** : 
MVKKSLRQFTLMATATVTLLLGSVPLYAQTADVQQKLAELERQSGGRLGVALINTAD
NSQILYRADERFAMCSTSKVMAAAAVLKKSESEPNLLNQRVEIKKSDLVNYNPIAEK
HVNGTMSLAELSAAALQYSDNVAMNKLIAHVGGPASVTAFARQLGDETFRLDRTEP
TLNTAIPGDPRDTTSPRAMAQTLRNLTLGKALGDSQRAQLVTWMKGNTTGAASIQA
GLPASWVVGDKTGSGGYGTTNDIAVIWPKDRAPLILVTYFTQPQPKAESRHDVLAS
AAKIVTDGL
**SEQ ID N°1885** : 
MVTKRVQRMMFAAAACIPLLLGSAPLYAQTSAVQQKLAALEKSSGGRLGVALIDTA
DNTQVLYRGDERFPMCSTSKVMAVAAVLKQSETQKQLLNQPVEIKPADLVNYNPIA
EKHVNGTMTLAELSAAALQYSDNTAMNKLIAQLGGPGGVTAFARAIGDETFRLDRT
EPTLNTAIPGDPRDTTTPRAMAQTLRQLTLGHALGETQRAQLVTWLKGNTTGAASI
RAGLPTSWTVGDKTGSGDYGTTNDIAVIWPQGRAPLVLVTYFTQPQQNAESRRDV
LASAARIIAEGL
**SEQ ID N°1886 :** 
MVKKSLRQFTLMATATVTLLLGSVPLYAQTADVQQKLAELERQSGGRLGVALINTAD
NSQILYRADERFAMCSTSKAMAVAAVLKKSESEPNLLNQRVEIKKSDLVNYNPIAEK
HVNGTMSLAELSAAALQYSDNVAMNKLIAHVGGPASVTAFARQLGDETFRLDRTEP
TLNTAIPGDPRDTTSPRAMAQTLRNLTLGKALGDSQRAQLVTWMKGNTTGAASIQA
GLPASWVVGDKTGSGGYGTTNDIAVIWPKDRAPLILVTYFTQPQPKAESRRDVLAS
AAKIVTDGL
**SEQ ID N°1887** : 
MVTKRVQRMMFAAAACIPLLLGSAPLYAQTSAVQQKLAALEKSSGGRLGVALIDTA
DNTQVLYRGDERFPMCSTSKVMAAAAVLKQSETQKQLLNQPVEIKPADLVNYNPIA
EKHVNGTMTLAELSAAALQYSDNTAMNKLIAQLGGPGGVTAFARAIGDETFRLDRT
ESTLNTAIPGDPRDTTTPRAMAQTLRQLTLGHALGETQRAQLVTWLKGNTTGAASI
 
RAGLPTSWTVGDKTGSGDYGTTNDIAVIWPQGRAPLVLVTYFTQPQQNAERRRDV
LASAARIIAEGL
**SEQ ID N°1888** : 
MVTKRVQRMMFAAAACIPLLLGSAPLYAQTSAVQQKLAALEKSSGGRLGVALIDTA
DNTQVLYRGDERFPMCSTSKVMAAAAVLKQSETQKQLLNQPVEIKPADLVNYNPIA
EKHVNGTMTLAELSAAALQYSDNTAMNKLIAQLGGPGGVTAFARAIGDETFRLDRT
EPTQNTAIPGDPRDTTTPRAMAQTLRQLTLGHALGETQRAQLVTWLKGNTTGAASI
RAGLPTSWTVGDKTGSGGYGTTNDIAVIWPQGRAPLVLVTYFTQPQQNAESRRDV
LASAARIIAEGL
**SEQ ID N°1889** : 
MVKKSLRQFTLMATATVTLLLGSVPLYAQTADVQQKLAELERQSGGRLGVALINTAD
NSQILYRADERFAMCSTSKVMAAAAVLKKSESEPNLLNQRVEIKKSDLVNYNPIAEK
HVNGTMSLAELIAAALQYSDNVAMNKLIAHVGGPASVTAFARQLGDETFRLDRTEPT
LNTAIPGDPRDTTSPRAMAQTLRNLTLGKALGDSQRAQLVTWMKGNTTGAASIQAG
LPASWVVGDKTGSGGYGTTNDIAVIWPKDRAPLILVTYFTQPQPKAESRRDVLASA
AKIVTDGL
**SEQ ID N°1890** : 
MVTKRVQRMMFAAAACIPLLLGSAPLYAQTSAVQQKLAALEKSSGGRLGVALIDTA
DNTQVLYRGDERFPMCSTSKVMAAAAVLKQSETQKQLLNQPVEIKPADLVNYNPIA
EKHVNGTMTLAELSAAALQYSDNTAMNKLIAQLGGPGGVTAFARAIGDETFRLDRT
EPTLNTAIPGDPRDTTTPRAMAQTLRQLTLGHALGETQREQLVTWLKGNTTGAASI
RAGLPTSWTVGDKTGSGGYGTTNDIAVIWPQGRAPLVLVTYFTQPQQNAESRRDV
LASAARIIAEGL
**SEQ ID N°1891** : 
MVTKRVQRMMFAAAACIPLLLGSAPLYAQTSAVQQKLAALEKSSGGRLGVALIDTA
DNTQVLYRGDERFPMCSTSKVMAVAAVLKQSETQKQLLNQPVEIKPADLVNYNPIA
EKHVNGTMTLAELSAAALQYSDNTAMNKLIAQLGGPGGVTAFARAIGDETFRLDRT
EPTLNTAIPGDPRDTTTPRAMAQTLRQLTLGHALGETQREQLVTWLKGNTTGAASI
RAGLPTSWTVGDKTGSGGYGTTNDIAVIWPQGRAPLVLVTYFTQPQQNAESRRDV
LASAARIIAEGL
**SEQ ID N°1892** : 
MVTKRVQRMMFAAAACIPLLLGSAPLYAQTSAVQQKLAALEKSSGGRLGVALIDTA
DNTQVLYRGDERFPMCSTSKVMAAAAVLKQSETQKQLLNQPVEIKPADLVNYNPIA
EKHVNGTMTLAELSAAALQYSDNTAMNKLIAQLGGPGGVTAFARAIGDETFRLDRT
EPTLNTAIPGDPRDTTTPRAMAQTLRQLTLGHALGETQRAQLVTWLKGNTTGAASI
RAGLPTSWTVGDKTGSGDYGTTNDIAVIWPQGRAPLVLVTYFTQPQQNAENRRDV
LASAARIIAEGL
**SEQ ID N°1893** : 
MVTKRVQRMMFAAAACIPLLLGSAPLYAQTSAVQQKLAALEKSSGGRLGVALIDTA
DNTQVLYRGDERFPMCSTSKVMAAAAVLKQSETQKQLLNQPVEIKPADLVNYNPIA
EKHVNGTMTLAELSAAALQYSDNTAMNKLIAQLGGPGGVTAFARAIGDETFRLDRT
EPTLNTAIPGDPRDTTTPRAMAQTLRQLTLGHALGETQRAQLVTWLKGNTTGAASI
RAGLPTSWTVGDRTGSGDYGTTNDIAVIWPQGRAPLVLVTYFTQPQQNAESRHDV
LASAARIIAEG
**SEQ ID N°1894** : 
MVKKSLRQFTLMATATVTLLLGSVPLYAQTADVQQKLAELERQSGGRLGVALINTAD
NSQILYRADERFAMCSTSKVMAVAAVLKKSESEPNLLNQRVEIKKSDLVNYNPIAEK
HVNGTMSLAELSAAALQYSDNVAMNKLIAHVGGPASVTAFARQLGDETFRLDRTEP
TLNTAIPGDPRDTTSPRAMAQTLRNLTLGKALGDSQRAQLVTWMKGNTTGAASIQA
 
GLPASWVVGDKTGSGDYGTTNDIAVIWPKDRAPLILVTYFTQPQPKAESRRDVLAS
AAKIVTNGL
**SEQ ID N°1895** : 
MVTKRVQRMMFAAAACIPLLLGSAPLYAQTSAVQQKLAALEKSSGGRLGVALIDTA
DNTQVLYRGDERFPMCSTSKVMAAAAVLKQSETQKQLLNQPVEIKPADLVNYNPIA
EEHVNGTMTLAELSAAALQYSDNTAMNKLIAQLGGPGGVTAFARAIGDETFRLDRT
EPTLNTAIPGDPRDTTTPRAMAQTLRQLTLGHALGETQRAQLVTWLKGNTTGAASI
RAGLPTSWTVGDKTGSGDYGTTNDIAVIWPQGRAPLVLVTYFTQPQQNAESRRDV
LASAARIIAEGNL
**SEQ ID N°1896** : 
MVTKRVQRMMFAAAACIPLLLGSAPLYAQTSAVQQKLAALEKSSGGRLGVALIDTA
DNTQVLYRGDERFPMCSTSKVMAAAAVLKQSETQKQLLNQPVEIKPADLVNYNPIA
EKHVNGTMTLAELSAAALQYSDNTAMNKLIAQLGGQGGVTAFARAIGDETFRLDRT
EPTLNTAIPGDPRDTTTPRAMAQTLRQLTLGHALGETQRAQLVTWLKGNTTGAASI
RAGLPTSWTVGDKTGSGDYGTTNDIAVIWPQGRAPLVLVTYFTQPQQNAESRRDV
LASAARIIAEGL
**SEQ ID N°1897** : 
VKKSLRQFTLMATATVTLLLGSVPLYAQTADVQQKLAELERQSGGRLGVALINTADN
SQILYRADERFAMCSTSKVMAAAAVLKKSESEPNLLNQRVEIKKSDLVNYNPIAEKH
VNGTMSLAELSAAALQYSDNVAMNKLIAHVGGPASVTAFARQLGDETFRLDRTEPT
LNTAIPGDPRDTTSPRAMAQTLRNLTLGKALGDSQRAQLVTWMKGNTTGAASIQAG
LPASWVVGDRTGSGGYGTTNDIAVIWPKDRAPLILVTYFTQPQPKAESRRDVLASA
AKIVT
**SEQ ID N°1898** : 
VKKSLRQFTLMATATVTLLLGSVPLYAQTADVQQKLAELERQSGGRLGVALINTADN
SQILYRADERFAMCSTSKVMAAAAVLKKSESEPNLLNQRAEIKKSDLVNYNPIAEKH
VNGTMSLAELSAAALQYSDNVAMNKLIAHVGGPASVTAFARQLGDETFRLDRTEPT
LNTAIPGDPRDTTSPRAMAQTLRNLTLGKALGDSQRAQLVTWMKGNTTGAASIQAG
LPASWVVGDKTGSGGYGTTNDIAVIWPKDRAPLILVTYFTQPQPKAESRRDVLASA
AKIVT
**SEQ ID N°1899** : 
MVTKRVQRMMFAAAACIPLLLGSAPLYAQTSAVQQKLAALEKSSGGRLGVALIDTA
DNTQVLYRGDERFPMCSTSKVMAAAAVLKQSETQKQLLNQPVEIKPADLVNYNPIA
EKHVNGTMTLAELGAAALQYSDNTAMNKLIAQLGGPGGVTAFARAIGDETFRLDRT
EPTLNTAIPGDPRDTTTPRAMAQTLRQLTLGHALGETQRAQLVTWLKGNTTGAASI
RAGLPTSWTVGDKTGSGDYGTTNDIAVIWPQGRAPLVLVTYFTQPQQNAESRRDV
LASAARIIAEGL
**SEQ ID N°1900** : 
MVTKRVQRMMFAAAACIPLLLGSAPLYAQTSAVQQKLAALEKSSGGRLGVALIDTA
DNTQVLYRGDERFPMCSTSKVMAAAAVLKRSETQKQLLNQPVEIKPADLVNYNPIA
EKHVNGTMTLAELSAAALQYSDNTAMNKLIAQLGGPGGVTAFARAIGDETFRLDRT
EPTLNTAIPGDPRDTTTPRAMAQTLRQLTLGHALGETQRAQLVTWLKGNTTGAASI
RAGLPTSWTVGDKTGSGDYGTTNDIAVIWPQGRAPLVLVTYFTQPQQNAESRRDV
LASAARIIAEGL
**SEQ ID N°1901** : 
VKKSLRQFTLMATATVTLLLGSVPLYAQTADVQQKLAELERQSGGRLGVALINTADN
SRILYRADERFAMCSTSKVMAAAAVLKKSESEPNLLNQRVEIKKSDLVNYNPIAEKH
VNGTMSLAELSAAALQYSDNVAMNKLIAHVGGPASVTAFARQLGDETFRLDRTEPT
LNTAIPGDPRDTTSPRAMAQTLRNLTLGKALGDSQRAQLVTWMKGNTTGAASIQAG
 
LPASWVVGDKTGSGGYGTTNDIAVIWPKDRAPLILVTYFTQPQPKAESRRDVLASA
AKIVTK
**SEQ ID N°1902** : 
MMTQSIRRSMLTVMATLPLLFSSATLHAQANSVQQQLEALEKSSGGRLGVALINTA
DNSQILYRADERFAMCSTSKVMAAAAVLKQSESDKHLLNQRVEIKKSDLVNYNPIAE
KHVNGTMTLAELGAAALQYSDNTAMNKLIAHLGGPDKVTAFARSLGDETFRLDRTE
PTLNTAIPGDPRDTTTPLAMAQTLKNLTLGKALAETQRAQLVTWLKGNTTGSASIRA
GLPKSWVVGDKTGSGDYGTTNDIAVIWPENHAPLVLVTYFTQPEQKAERRRDILAA
AAKIVTHGF
**SEQ ID N°1903** : 
MRHRVKRMMLMTTACISLLLGSAPLYAQANDVQQKLAALEKSSGGRLGVALIDTAD
NAQTLYRADERFAMCSTSKVMAAAAVLKQSETQKNVLSQKVEIKSSDLINYNPIAEK
HVNGTMTLAELSAAALQYSDNTAMNKLIAHLGGPDKVTAFARAIGDDTFRLDRTEPT
LNTAIPGDPRDTTTPLAMAQTLRHLTLGSALGETQRAQLVTWLKGNTTGAASIQAGL
PTSWVVGDKTGSGDYGTTNDIAVIWPEGRAPLILVTYFTQPEQKAENRRDVLAAAA
KIVTDGY
**SEQ ID N°1904** : 
MVKKSLRQFTLMATATVTLLLGSVPLYAQTADVQQKLAELERQSGGRLGVALINTAD
NSQILYRADERFAMCSTSKVMAVAAVLKKSESEPNLLNQRVEIKKSDLVNYNPIAEK
HVDGTMSLAELSAAALQYSDNVAMNKLISHVGGPASVTAFARQLGDETFRLDRTEP
TLNTAIPGDPRDTTSPRAMAQTLRNLTLGKALGDSQRAQLVTWMKGNTTGAASIQA
GLPASWVVGDKTGSGDYGTTNDIAVIWPKDRAPLILVTYFTQPQPKAESRRDVLAS
AAKIVTNGL
**SEQ ID N°1905** : 
MMTQSIRRSMLTVMATLPLLFSSATLHAQANSVQQQLEALEKSSGGRLGVALINTA
DNSQILYRADERFAMCSTSKVMAAAAVLKQSESDKHLLNQRVEIKKSDLVNYNPIAE
KHVNGTMTLAELGAAALQYSDNTAMNKLIAHLGGPDKVTAFARSLGDETFRLDRTE
PTLNTAIPGDPRDTTTPLAMAQTLKNLTLGKALAETQRAQLVTWLKGNTTGSASIRA
GLPKSWVVGDKTGSGDYGTTNDIAVIWPENHAPLVLVTYFTQPEQKAESRRDILAA
AAKIVTHGF
**SEQ ID N°1906** : 
MVKKSLRQFTLMATATVTLLLGSVPLYAQTADVQQKLAELERQSGGRLGVALINTAD
NSQILYRADERFAMCSTSKVMAAAAVLKKSESEPNLLNQRVEIKKSDLVNYNPIAEK
HVNGTMSLAELSAAALQYSDNVAMNKLIAHVGGPASVTAFARQLGDETFRLDRTEP
TLNTAIPGDPRDTTSPRAMAQTLRNLTLGKALGDSQRAQLVTWMKGNTTGAASIQA
GLPASWVVGDKTGSGDYGTTNDIAVIWPKDRAPLILVTYFTQPQPKAESRRDVLAS
AAKIVTDGL
**SEQ ID N°1907** : 
MMTQSIRRSMLTVMATLPLLFSSATLHAQANSVQQQLEALEKSSGGRLGVALINTA
DNSQILYRADERFAMCSTSKVMAAAAVLKQSESDKHLLNQRVEIKKSDLVNYNPIAE
KHVNGTMTLAELGAAALQYSDNTAMNKLIAHLGGPDKVTAFARSLGDETFRLDRTE
PTLNTAIPGDPRDTTTPLAMAQTLKNLTLGKALAETQRAQLVTWLKGNTTGSASIRA
GLPKSWVVGDKTGSGDYGTTNDIAVIWPENHAPLVLVTYFTQPEQKAESRRDFLAA
AAKIVTHGF
**SEQ ID N°1908** : 
MVKKSLRQFTLMATATVTLLLGSVPLYAQTADVQQKLAELERQSGGRLGVALINTAD
NSQILYRADERFAMCSTSKVMAAAAVLKKSESEPNLLNQRVEIKKSDLVNYNPIAEK
HVNGTMSLAELSAAALQYSDNVAMNKLIAHVGGPASVTAFARQLGDETFRLDRTEP
TLNTAIPGDPRDTTSPRAMAQTLRNLTLGKALGDSQRAQLVTWMKGNTTGAASIQA
GLPASWVVGDKTGSGGYGTTNDIAVIWPKDRAPLILVTYFTQPQPKAESRRDVLAS
 AAKIVTNGL
lesdits peptides étant choisis, de préférence, parmi les peptides de séquence **SEQ ID N°446** à **SEQ ID 495** et **SEQ ID N° 2008** à **SEQ ID N°2092** tels que définis ci-après :

| **Peptide SEQ ID N°** | **Séquence d'acides aminés** | **Position du peptide dans la protéine CTX-M** | **Intérêt clinique** |
|---|---|---|---|
| **SEQ ID N°446** | AGLPK | 226-230 pour les protéines de SEQ N°351, 386, 392, 397, 415, 416, 419, 422, 424, 429, 430, 432, 435, 439, 442, 444, 445, 1881, 1903, 1906, 1908 | 2be |
| **SEQ ID N°447** | AGLPTSWTVGDK | 226-237 pour les protéines de SEQ N°355, 356, 360, 361, 362, 367, 369, 376, 377, 378, 379, 380, 381, 398, 402, 403, 407, 408, 409, 410, 411, 413, 421, 423, 436, 1872, 1873, 1874, 1877, 1882, 1886, 1888, 1889, 1891, 1892, 1893, 1896, 1897, 1900, 1901; 224-235 pour la protéine de séquence SEQ ID N°425 | 2be |
| **SEQ ID N°448** | AIGDETFR | 157-164 pour les protéines de SEQ N°353, 355, 359, 360, 361, 362, 367, 369, 376, 377, 378, 379, 380, 381, 387, 398, 402, 403, 407, 408, 409, 410, 411, 413, 421, 423, 425, 428, 436, 1872, 1873, 1874, 1877, 1882, 1886, 1888, 1889, 1891, 1892, 1893, 1894, 1896, 1897, 1900, 1901 | 2be |
| **SEQ ID N°449** | ALAETQR | 201-207 pour les protéines de SEQ N°351, 386, 392, 397, 415, 416, 419, 422, 424, 429, 430, 432, 434, 435, 439, 442, 444, 445, 1881, 1903, 1906, 1908 | 2be |
| **SEQ ID N°450** | ALGDSQR | 201-207 pour les protéines de SEQ N°354, 357, 358, 363, 364, 365, 370, 371, 372, 373, 374, 382, 385, 388, 389, 390, 391, 393, 394, 395, 396, 399, 400, 401, 404, 405, 406, 412, 417, 426, 431, 433, 437, 438, 440, 441, 1875, 1876, 1879, 1880, 1883, 1884, 1885, 1887, 1890, 1895, 1905, 1907, 1909; 200-206 pour la protéine de séquence SEQ ID N°1898; 200-206 pour la protéine de séquence SEQ ID N°1899; 200-206 pour la protéine de séquence SEQ ID N° 1902 | 2be |
| **SEQ ID N°451** | AMAQTLR | 188-194 pour les protéines de SEQ N°352, 353, 354, 355, 356, 357, 358, 359, 360, 361, 362, 363, 364, 365, 367, 369, 370, 371, 372, 373, 374, 376, 377, 378, 379, 380, 381, 382, 385, 387, 388, 389, 390, 391, 393, 394, 395, 396, 398, 399, 400, 401, 402, 403, 404, 405, 406, 407, 408, 409, 410, 411, 412, 413, 417, 421, 423, 426, 428, 431, 433, 436, 437, 438, 440, 441, 443, 1872, 1873, 1874, 1875, 1876, 1877, 1879, 1880, 1882, 1883, 1884, 1885, 1886, 1887, 1888, 1889, 1890, 1891, 1892, 1893, 1894, 1895, 1896, 1897, 1900, 1901, 1905, 1907, 1909; 187-193 pour la protéine de séquence SEQ ID N°375; 187-193 pour la protéine de séquence SEQ ID N°427; 187-193 pour la protéine de séquence SEQ ID N°1878; 187-193 pour la protéine de séquence SEQ ID N°1898; 187-193 pour la protéine de séquence SEQ ID N°1899; 187-193 pour la protéine de séquence SEQ ID N°1902; 187-193 pour la protéine de séquence SEQ ID N°1904 | 2be |
| **SEQ ID N°452** | APLILVTYFTQPQPK | 258-272 pour les protéines de SEQ N°354, 357, 358, 363, 364, 365, 370, 371, 372, 373, 374, 382, 385, 388, 389, 391, 393, 394, 395, 396, 399, 400, 401, 404, 405, 412, 417, 426, 428, 431, 433, 437, 438, 440, 441, 1875, 1876, 1879, 1880, 1883, 1885, 1887, 1890, 1895, 1905, 1907, 1909; 257-271 pour la protéine de séquence SEQ ID N°1898; 257-271 pour la protéine de séquence SEQ ID N°1899; 257-271 pour la protéine de séquence SEQ ID N°1902 | 2be |
| **SEQ ID N°453** | APLVLVTYFTQPQQNAE SR | 258-276 pour les protéines de SEQ N°353, 355, 356, 359, 360, 361, 362, 369, 376, 377, 378, 379, 380, 381, 387, 402, 403, 407, 408, 409, 410, 411, 413, 423, 436, 1872, 1873, 1874, 1886, 1889, 1891, 1892, 1894, 1896, 1897, 1900, 1901; 256-274 pour la protéine de séquence SEQ ID N°425 | 2be |
| **SEQ ID N°454** | AQLVTWLK | 208-215 pour les protéines de SEQ N°351, 352, 353, 355, 356, 359, 360, 361, 362, 367, 369, 376, 377, 378, 379, 380, 381, 386, 387, 392, 397, 398, 402, 403, 407, 408, 410, 411, 413, 415, 416, 421, 422, 423, 424, 428, 429, 430, 432, 434, 435, 436, 439, 442, 443, 444, 445, 1872, 1873,1874,1877,1881,1882,1886,1888,1889,1893, 1894, 1896, 1897, 1900, 1901, 1903, 1906, 1908; 207-214 pour la protéine de séquence SEQ ID N°375; 207-214 pour la protéine de séquence SEQ ID N°427; 207-214 pour la protéine de séquence SEQ ID N°1878; 207-214 pour la protéine de séquence SEQ ID N°1904 | 2be |
| **SEQ ID N°455** | AQLVTWMK | 208-215 pour les protéines de SEQ N°354, 357, 358, 363, 364, 365, 370, 371, 372, 373, 374, 382, 385, 388, 389, 390, 391, 393, 394, 395, 396, 399, 400, 401, 404, 405, 406, 412, 417, 426, 431, 433, 437, 438, 440, 441, 1875, 1876, 1879, 1880, 1883, 1884, 1885, 1887, 1890, 1895, 1905, 1907, 1909; 207-214 pour la protéine de séquence SEQ ID N°1898; 207-214 pour la protéine de séquence SEQ ID N°1899; 207-214 pour la protéine de séquence SEQ ID N°1902 | 2be |
| **SEQ ID N°456** | DILAAAAK | 278-285 pour les protéines de SEQ N°386, 392, 397, 415, 416, 419, 422, 424, 432, 434, 439, 1881, 1903, 1906 | 2be |
| **SEQ ID N°457** | DTTSPR | 182-187 pour les protéines de SEQ N°354, 357, 358, 363, 364, 365, 370, 371, 372, 373, 374, 382, 385, 388, 389, 390, 391, 393, 394, 395, 396, 399, 400, 401, 404, 405, 406, 412, 417, 426, 431, 433, 437, 438, 440, 441, 1875, 1876, 1879, 1880, 1883, 1884, 1885, 1887, 1890, 1895, 1905, 1907, 1909; 181-186 pour la protéine de séquence SEQ ID N°1898; 181-186 pour la protéine de séquence SEQ ID N°1899; 181-186 pour la protéine de séquence SEQ ID N°1902 | 2be |
| **SEQ ID N°458** | DTTTPLAMAQTLK | 182-194 pour les protéines de SEQ N°351, 386, 392, 397, 415, 416, 419, 422, 424, 429, 430, 432, 434, 435, 439, 442, 444, 445, 1881, 1903, 1906, 1908 | 2be |
| **SEQ ID N°459** | DTTTPR | 182-187 pour les protéines de SEQ N°353, 355, 356, 359, 360, 361, 362, 367, 369, 376, 377, 378, 379, 380, 381, 387, 398, 402, 403, 407, 408, 409, 410, 411, 413, 421, 423, 428, 436, 1872, 1873, 1874, 1877, 1882, 1886, 1888, 1889, 1891, 1892, 1893, 1894, 1896, 1897, 1900, 1901 | 2be |
| **SEQ ID N°460** | DVLAAAAK | 277-284 pour les protéines de SEQ N°375, 427, 1878, 1904; 278-285 pour la protéine de séquence SEQ ID N°351; 278-285 pour la protéine de séquence SEQ ID N°352; 278-285 pour la protéine de séquence SEQ ID N°429; 278-285 pour la protéine de séquence SEQ ID N°430; 278-285 pour la protéine de séquence SEQ ID N°442; 278-285 pour la protéine de séquence SEQ ID N°444; 278-285 pour la protéine de séquence SEQ ID N°445 | 2be |
| **SEQ ID N°461** | DVLASAAK | 278-285 pour les protéines de SEQ N°354, 363, 364, 365, 370, 371, 372, 373, 374, 382, 385, 388, 389, 390, 391, 393, 394, 395, 396, 399, 400, 401, 404, 405, 406, 412, 417, 426, 428, 431, 433, 437, 438, 1875, 1876, 1879, 1880, 1883, 1884, 1885, 1887, 1890, 1895, 1905, 1907, 1909; 277-284 pour la protéine de séquence SEQ ID N°1898; 277-284 pour la protéine de séquence SEQ ID N°1899; 277-284 pour la protéine de séquence SEQ ID N°1902 | 2be |
| **SEQ ID N°462** | DVLASAAR | 278-285 pour les protéines de SEQ N°353, 355, 356, 359, 360, 361, 362, 367, 369, 376, 377, 378, 379, 380, 381, 387, 398, 402, 403, 407, 408, 409, 410, 411, 413, 421, 423, 436, 1872, 1873, 1874, 1877, 1882, 1886, 1888, 1889, 1891, 1892, 1893, 1894, 1896, 1897, 1900, 1901; 276-283 pour la protéine de séquence SEQ ID N°425 | 2be |
| **SEQ ID N°463** | FAMCSTSK | 69-76 pour les protéines de SEQ N°351, 352, 354, 357, 358, 363, 364, 365, 366, 368, 370, 371, 372, 373, 374, 382, 383, 384, 385, 386, 388, 390, 391, 392, 393, 394, 395, 396, 397, 399, 400, 401, 404, 405, 406, 412, 414, 415, 416, 417, 418, 419, 420, 422, 424, 426, 429, 430, 431, 432, 433, 434, 435, 437, 438, 439, 440, 441, 442, 443, 444, 445, 1875, 1876, 1879, 1880, 1881, 1883, 1884, 1885, 1887, 1890, 1895, 1903, 1905, 1906, 1907, 1908, 1909; 68-75 pour la protéine de séquence SEQ ID N°375; 68-75 pour la protéine de séquence SEQ ID N°427; 68-75 pour la protéine de séquence SEQ ID N°1878; 68-75 pour la protéine de séquence SEQ ID N°1898; 68-75 pour la protéine de séquence SEQ ID N°1899; 68-75 pour la protéine de séquence SEQ ID N°1902; 68-75 pour la protéine de séquence SEQ ID N°1904 | 2be |
| **SEQ ID N°464** | FPMCSTSK | 69-76 pour les protéines de SEQ N°353, 355, 356, 359, 360, 361, 362, 367, 369, 376, 377, 378, 379, 380, 381, 387, 389, 398, 402, 403, 407, 408, 409, 410, 411, 413, 421, 423, 425, 428, 436, 1872, 1873, 1874, 1877, 1882, 1886, 1888, 1889, 1891, 1892, 1893, 1894, 1896, 1897, 1900, 1901 | 2be |
| **SEQ ID N°465** | GNTTGAASIQAGLPASW VVGDK | 216-237 pour les protéines de SEQ N°354, 357, 358, 363, 364, 365, 370, 371, 372, 373, 374, 382, 385, 388, 389, 390, 391, 393, 394, 395, 396, 399, 400, 401, 404, 405, 406, 412, 417, 426, 431, 433, 437, 438, 440, 441, 1875, 1876, 1879, 1880, 1883, 1884, 1885, 1887, 1890, 1895, 1905, 1907, 1909; 215-236 pour la protéine de séquence SEQ ID N°1899; 215-236 pour la protéine de séquence SEQ ID N°1902 | 2be |
| **SEQ ID N°466** | GNTTGAASIQAGLPTSW VVGDK | 215-236 pour les protéines de SEQ N°375, 427, 1878, 1904; 216-237 pour la protéine de séquence SEQ ID N°352; 216-237 pour la protéine de séquence SEQ ID N°366; 216-237 pour la protéine de séquence SEQ ID N°383; 216-237 pour la protéine de séquence SEQ ID N°384; 216-237 pour la protéine de séquence SEQ ID N°414; 216-237 pour la protéine de séquence SEQ ID N°418; 216-237 pour la protéine de séquence SEQ ID N°420; 216-237 pour la protéine de séquence SEQ ID N°443 | 2be |
| **SEQ ID N°467** | GNTTGAASIR | 216-225 pour les protéines de SEQ N°353, 355, 356, 359, 360, 361, 362, 367, 368, 369, 377, 378, 379, 380, 381, 387, 398, 402, 403, 407, 408, 409, 410, 411, 413, 421, 423, 428, 436, 1872, 1873, 1874, 1877, 1882, 1886, 1888, 1889, 1891, 1892, 1893, 1894, 1896, 1897, 1900, 1901 | 2be |
| **SEQ ID N°468** | GNTTGSASIR | 216-225 pour les protéines de SEQ N°351, 386, 392, 397, 415, 416, 419, 422, 424, 429, 430, 432, 434, 435, 439, 444, 1881, 1903, 1906, 1908 | 2be |
| **SEQ ID N°469** | HLLNQR | 92-97 pour les protéines de SEQ N°351, 386, 397, 415, 416, 419, 422, 424, 429, 430, 432, 434, 435, 439, 442, 444, 445, 1881, 1903, 1906, 1908 | 2be |
| **SEQ ID N°470** | LAALEK | 36-41 pour les protéines de SEQ N°375, 427, 1878, 1904; 37-42 pour les protéine de séquence SEQ ID N°352, 353, 355, 356, 359, 360, 361, 362, 366, 367, 368, 369, 376, 377, 378, 379, 380, 381, 383, 384, 387, 398, 402, 403, 407, 408, 409, 410, 411, 413, 414, 418, 420, 421, 423, 425, 436, 443, 1872, 1873, 1874, 1877, 1882, 1886, 1888, 1889, 1891, 1892, 1893, 1894, 1896, 1897, 1900, 1901 | 2be |
| **SEQ ID N°471** | LAELER | 37-42 pour les protéines de SEQ N°358, 363, 364, 365, 370, 371, 372, 382, 385, 388, 389, 391, 393, 394, 395, 396, 399, 400, 401, 404, 405, 406, 412, 417, 426, 428, 431, 433, 437, 438, 440, 441, 1875, 1876, 1879, 1880, 1883, 1884, 1885, 1887, 1890, 1895, 1905, 1907, 1909; 36-41 pour la protéine de séquence SEQ ID N°1898; 36-41 pour la protéine de séquence SEQ ID N°1899; 36-41 pour la protéine de séquence SEQ ID N°1902 | 2be |
| **SEQ ID N°472** | LGVALIDTADNTQVLYR | 48-64 pour les protéines de SEQ N°353, 355, 359, 360, 361, 362, 367, 369, 376, 377, 378, 387, 398, 402, 403, 407, 409, 410, 411, 413, 421, 423, 425, 436, 1872, 1873, 1874, 1877, 1882, 1886, 1888, 1889, 1891, 1892, 1893, 1894, 1896, 1897, 1900, 1901 | 2be |
| **SEQ ID N°473** | LGVALINTADNSQILYR | 48-64 pour les protéines de SEQ N°351, 354, 357, 358, 363, 364, 365, 370, 371, 372, 373, 374, 382, 385, 386, 388, 389, 390, 391, 392, 393, 394, 395, 396, 397, 399, 400, 401, 404, 405, 406, 412, 415, 416, 417, 419, 422, 424, 426, 428, 431, 432, 433, 435, 437, 438, 439, 440, 441, 442, 444, 445, 1875, 1876, 1879, 1880, 1881, 1883, 1884, 1885, 1887, 1890, 1895, 1903, 1905, 1906, 1907, 1908, 1909; 47-63 pour la protéine de séquence SEQ ID N°1898; 47-63 pour la protéine de séquence SEQ ID N°1899 | 2be |
| **SEQ ID N°474** | LIAHLGGPDK | 141-150 pour les protéines de SEQ N°351, 352, 366, 368, 383, 384, 386, 392, 397, 414, 415, 416, 418, 419, 420, 422, 424, 429, 430, 432, 434, 435, 439, 442, 444, 445, 1881, 1903, 1906, 1908; 140-149 pour la protéine de séquence SEQ ID N°375; 140-149 pour la protéine de séquence SEQ ID N°427; 140-149 pour la protéine de séquence SEQ ID N°1878; 140-149 pour la protéine de séquence SEQ ID N°1904 | 2be |
| **SEQ ID N°475** | LIAHVGGPASVTAFAR | 141-156 pour les protéines de SEQ N°354, 357, 358, 363, 364, 365, 370, 371, 372, 373, 374, 382, 385, 388, 389, 390, 391, 393, 394, 399, 400, 401, 404, 405, 406, 412, 417, 433, 437, 440, 441, 1875, 1876, 1879, 1883, 1884, 1885, 1887, 1890, 1895, 1907, 1909; 140-155 pour la protéine de séquence SEQ ID N°1898; 140-155 pour la protéine de séquence SEQ ID N°1899; 140-155 pour la protéine de séquence SEQ ID N°1902 | 2be |
| **SEQ ID N°476** | LIAQLGGPGGVTAFAR | 141-156 pour les protéines de SEQ N°353, 355, 356, 359, 360, 361, 362, 367, 369, 376, 377, 378, 379, 380, 381, 387, 398, 402, 403, 407, 408, 409, 410, 411, 413, 421, 423, 425, 428, 436, 1872, 1873, 1874, 1877, 1882, 1886, 1888, 1889, 1891, 1892, 1893, 1894, 1896, 1900, 1901 | 2be |
| **SEQ ID N°477** | NLTLGK | 195-200 pour les protéines de SEQ N°351, 354, 357, 358, 363, 364, 365, 370, 371, 372, 373, 374, 382, 385, 386, 388, 389, 390, 391, 392, 393, 394, 395, 396, 397, 399, 400, 401, 404, 405, 406, 412, 415, 416, 417, 419, 422, 424, 426, 429, 430, 431, 432, 433, 434, 435, 437, 438, 439, 440, 441, 442, 444, 445, 1875, 1876, 1879, 1880, 1881, 1883, 1884, 1885, 1887, 1890, 1895, 1903, 1905, 1906, 1907, 1908, 1909; 194-199 pour la protéine de séquence SEQ ID N°1898; 194-199 pour la protéine de séquence SEQ ID N°1899; 194-199 pour la protéine de séquence SEQ ID N°1902 | 2be |
| **SEQ ID N°478** | QLGDETFR | 157-164 pour les protéines de SEQ N°354, 357, 358, 363, 364, 365, 370, 371, 372, 373, 374, 382, 385, 388, 389, 390, 391, 393, 394, 395, 396, 399, 400, 401, 405, 406, 412, 417, 426, 431, 433, 437, 438, 440, 441, 1875, 1876, 1879,1880,1883,1884,1885,1887,1890,1895,1905, 1907, 1909; 156-163 pour la protéine de séquence SEQ ID N°1898; 156-163 pour la protéine de séquence SEQ ID N°1899; 156-163 pour la protéine de séquence SEQ ID N°1902 | 2be |
| **SEQ ID N°479** | QLLNQPVEIK | 92-101 pour les protéines de SEQ N°353, 355, 356, 359, 360, 361, 362, 367, 369, 376, 377, 378, 379, 380, 381, 387, 398, 402, 407, 408, 409, 410, 411, 413, 421, 423, 425, 428, 436, 1872, 1873, 1874, 1877, 1882, 1886, 1888, 1889, 1891, 1892, 1893, 1894, 1896, 1897, 1900, 1901 | 2be |
| **SEQ ID N°480** | QLTLGHALGETQR | 195-207 pour les protéines de SEQ N°353, 355, 356, 359, 360, 361, 362, 367, 369, 376, 377, 378, 379, 380, 381, 387, 398, 402, 403, 407, 408, 409, 410, 411, 413, 421, 423, 428, 436, 1872, 1873, 1874, 1877, 1882, 1886, 1888, 1889, 1891, 1892, 1893, 1894, 1896, 1897, 1900, 1901 | 2be |
| **SEQ ID N°481** | QSESDK | 86-91 pour les protéines de SEQ N°351, 386, 392, 397, 415, 416, 419, 422, 424, 429, 430, 432, 434, 435, 439, 442, 444, 445, 1881, 1903, 1906, 1908 | 2be |
| **SEQ ID N°482** | QSETQK | 85-90 pour les protéines de SEQ N°375, 427, 1878, 1904; 86-91 pour la protéine de séquence SEQ ID N°352; 86-91 pour la protéine de séquence SEQ ID N°353; 86-91 pour la protéine de séquence SEQ ID N°355; 86-91 pour la protéine de séquence SEQ ID N°356; 86-91 pour la protéine de séquence SEQ ID N°359; 86-91 pour la protéine de séquence SEQ ID N°360; 86-91 pour la protéine de séquence SEQ ID N°361; 86-91 pour la protéine de séquence SEQ ID N°362; 86-91 pour la protéine de séquence SEQ ID N°366; 86-91 pour la protéine de séquence SEQ ID N°367; 86-91 pour la protéine de séquence SEQ ID N°368; 86-91 pour la protéine de séquence SEQ ID N°369; 86-91 pour la protéine de séquence SEQ ID N°376; 86-91 pour la protéine de séquence SEQ ID N°377; 86-91 pour la protéine de séquence SEQ ID N°378; 86-91 pour la protéine de séquence SEQ ID N°379; 86-91 pour la protéine de séquence SEQ ID N°380; 86-91 pour la protéine de séquence SEQ ID N°381; 86-91 pour la protéine de séquence SEQ ID N°383; 86-91 pour la protéine de séquence SEQ ID N°384; 86-91 pour la protéine de séquence SEQ ID N°387; 86-91 pour la protéine de séquence SEQ ID N°398; 86-91 pour la protéine de séquence SEQ ID N°402; 86-91 pour la protéine de séquence SEQ ID N°403; 86-91 pour la protéine de séquence SEQ ID N°407; 86-91 pour la protéine de séquence SEQ ID N°408; 86-91 pour la protéine de séquence SEQ ID N°409; 86-91 pour la protéine de séquence SEQ ID N°410; 86-91 pour la protéine de séquence SEQ ID N°411; 86-91 pour la protéine de séquence SEQ ID N°413; 86-91 pour la protéine de séquence SEQ ID N°414; 86-91 pour la protéine de séquence SEQ ID N°418; 86-91 pour la protéine de séquence SEQ ID N°420; 86-91 pour la protéine de séquence SEQ ID N°421; 86-91 pour la protéine de séquence SEQ ID N°423; 86-91 pour la protéine de séquence SEQ ID N°425; 86-91 pour la protéine de séquence SEQ ID N°428; 86-91 pour la protéine de séquence SEQ ID N°436; 86-91 pour la protéine de séquence SEQ ID N°443; 86-91 pour la protéine de séquence SEQ ID N°1872; 86-91 pour la protéine de séquence SEQ ID N°1873; 86-91 pour la protéine de séquence SEQ ID N°1874; 86-91 pour la protéine de séquence SEQ ID N°1877; 86-91 pour la protéine de séquence SEQ ID N°1882; 86-91 pour la protéine de séquence SEQ ID N°1886; 86-91 pour la protéine de séquence SEQ ID N°1888; 86-91 pour la protéine de séquence SEQ ID N°1889; 86-91 pour la protéine de séquence SEQ ID N°1891; 86-91 pour la protéine de séquence SEQ ID N°1892; 86-91 pour la protéine de séquence SEQ ID N°1893; 86-91 pour la protéine de séquence SEQ ID N°1894; 86-91 pour la protéine de séquence SEQ ID N°1896; 86-91 pour la protéine de séquence SEQ ID N°1897; 86-91 pour la protéine de séquence SEQ ID N°1900 | 2be |
| **SEQ ID N°483** | QSGGR | 43-47 pour les protéines de SEQ N°354, 357, 358, 363, 364, 365, 370, 371, 372, 373, 374, 382, 385, 388, 389, 390, 391, 393, 394, 395, 396, 399, 400, 401, 404, 405, 406, 412, 417, 426, 428, 431, 433, 437, 438, 440, 441, 1875, 1876, 1879, 1880, 1883, 1884, 1885, 1887, 1890, 1895, 1905, 1907, 1909; 42-46 pour la protéine de séquence SEQ ID N°1898; 42-46 pour la protéine de séquence SEQ ID N°1899; 42-46 pour la protéine de séquence SEQ ID N°1902 | 2be |
| **SEQ ID N°484** | SDLVNYNPIAEK | 103-114 pour les protéines de SEQ N°351, 354, 357, 358, 363, 364, 365, 371, 372, 373, 374, 382, 384, 385, 386, 388, 389, 390, 391, 392, 393, 394, 395, 396, 397, 399, 400, 401, 404, 405, 406, 412, 415, 416, 417, 419, 422, 424, 426, 429, 430, 431, 432, 433, 434, 435, 437, 438, 439, 440, 441, 442, 444, 445, 1875, 1876, 1879, 1880, 1881, 1883, 1884, 1885, 1887, 1890, 1895, 1903, 1905, 1906, 1907, 1908, 1909; 102-113 pour la protéine de séquence SEQ ID N°1898; 102-113 pour la protéine de séquence SEQ ID N°1899; 102-113 pour la protéine de séquence SEQ ID N°1902 | 2be |
| **SEQ ID N°485** | SESEPNLLNQR | 87-97 pour les protéines de SEQ N°354, 357, 363, 364, 365, 370, 371, 372, 373, 374, 382, 385, 388, 389, 390, 391, 393, 394, 395, 396, 399, 400, 401, 404, 405, 406, 412, 417, 426, 431, 433, 437, 438, 1875, 1876, 1879, 1880, 1883, 1884, 1885, 1887, 1890, 1895, 1905, 1907, 1909; 86-96 pour la protéine de séquence SEQ ID N°1898; 86-96 pour la protéine de séquence SEQ ID N°1899; 86-96 pour la protéine de séquence SEQ ID N°1902 | 2be |
| **SEQ ID N°486** | SLGDETFR | 157-164 pour les protéines de SEQ N°351, 386, 392, 397, 415, 416, 419, 422, 424, 429, 430, 432, 434, 435, 442, 444, 445, 1881, 1903, 1906, 1908 | 2be |
| **SEQ ID N°487** | SSGGR | 43-47 pour les protéines de SEQ N°351, 352, 353, 355, 356, 359, 360, 361, 362, 366, 367, 368, 369, 376, 378, 380, 381, 383, 384, 386, 387, 392, 397, 398, 402, 403, 407, 408, 409, 410, 411, 413, 414, 415, 416, 418, 419, 420, 421, 422, 423, 424, 425, 429, 430, 432, 434, 435, 436, 439, 442, 443, 444, 445, 1872, 1873, 1874, 1877, 1881,1882,1886,1888,1889,1891,1892,1893,1894, 1896, 1897, 1900, 1901, 1903, 1906, 1908; 42-46 pour la protéine de séquence SEQ ID N°375; 42-46 pour la protéine de séquence SEQ ID N°427; 42-46 pour la protéine de séquence SEQ ID N°1878; 42-46 pour la protéine de séquence SEQ ID N°1904 | 2be |
| **SEQ ID N°488** | SWVVGDK | 231-237 pour les protéines de SEQ N°352, 354, 357, 358, 363, 364, 365, 366, 368, 370, 371, 372, 373, 374, 382, 383, 384, 385, 386, 388, 389, 390, 391, 392, 393, 394, 395, 396, 397, 399, 400, 401, 404, 405, 406, 412, 414, 415, 416, 417, 418, 419, 420, 422, 424, 426, 428, 431, 432, 433, 435, 437, 438, 439, 440, 441, 442, 443, 445, 1875,1876,1879,1880,1881,1883,1884,1885,1887, 1890, 1895, 1903, 1905, 1906, 1907, 1908, 1909; 230-236 pour la protéine de séquence SEQ ID N°375; 230-236 pour la protéine de séquence SEQ ID N°427; 230-236 pour la protéine de séquence SEQ ID N°1878; 230-236 pour la protéine de séquence SEQ ID N°1899; 230-236 pour la protéine de séquence SEQ ID N°1902; 230-236 pour la protéine de séquence SEQ ID N°1904 | 2be |
| **SEQ ID N°489** | TEPTLNTAIPGDPR | 168-181 pour les protéines de SEQ N°351, 352, 353, 354, 355, 356, 357, 358, 359, 360, 362, 363, 364, 366, 367, 368, 369, 370, 371, 372, 373, 374, 376, 377, 378, 379, 380, 381, 382, 383, 384, 386, 387, 389, 390, 392, 393, 394, 396, 397, 398, 399, 401, 402, 403, 404, 405, 406, 408, 409, 410, 411, 412, 413, 414, 416, 417, 418, 419, 420, 422, 423, 424, 425, 426, 428, 431, 432, 433, 435, 436, 437, 438, 439, 440, 441, 442, 443, 444, 445, 1872, 1874, 1875, 1876, 1877, 1879, 1880, 1881, 1882, 1883, 1884, 1885, 1886, 1887, 1890, 1891, 1892, 1893, 1894, 1895, 1896, 1897, 1900, 1901, 1903, 1905, 1906, 1907, 1908, 1909; 167-180 pour la protéine de séquence SEQ ID N°375; 167-180 pour la protéine de séquence SEQ ID N°427; 167-180 pour la protéine de séquence SEQ ID N°1878; 167-180 pour la protéine de séquence SEQ ID N°1898; 167-180 pour la protéine de séquence SEQ ID N°1899; 167-180 pour la protéine de séquence SEQ ID N°1902; 167-180 pour la protéine de séquence SEQ ID N°1904 | 2be |
| **SEQ ID N°490** | TGSGDYGTTNDIAVIWPK | 238-255 pour les protéines de SEQ N°354, 357, 358, 364, 365, 372, 374, 382, 385, 388, 391, 395, 396, 400, 401, 404, 405, 426, 431, 433, 441, 1876, 1880, 1895, 1905, 1907 | 2be |
| **SEQ ID N°491** | TGSGDYGTTNDIAVIWP QGR | 238-257 pour les protéines de SEQ N°353, 355, 356, 360, 361, 362, 367, 376, 377, 378, 379, 380, 381, 387, 398, 402, 403, 407, 408, 409, 410, 411, 413, 421, 436, 1872, 1873, 1874, 1877, 1882, 1886, 1888, 1893, 1894, 1896, 1897, 1900, 1901; 236-255 pour la protéine de séquence SEQ ID N°425 | 2be |
| **SEQ ID N°492** | TGSGGYGTTNDIAVIWPK | 238-255 pour les protéines de SEQ N°363, 370, 371, 389, 390, 393, 394, 399, 406, 412, 428, 437, 438, 440, 1875, 1879, 1883, 1884, 1885, 1887, 1890, 1909; 237-254 pour la protéine de séquence SEQ ID N°1898; 237-254 pour la protéine de séquence SEQ ID N°1899; 237-254 pour la protéine de séquence SEQ ID N°1902 | 2be |
| **SEQ ID N°493** | VMAAAAVLK | 77-85 pour les protéines de SEQ N°351, 352, 353, 354, 355, 356, 357, 358, 359, 360, 361, 362, 363, 364, 367, 368, 369, 370, 371, 372, 373, 374, 376, 377, 378, 379, 380, 381, 382, 383, 384, 385, 386, 389, 391, 392, 397, 400, 401, 402, 404, 405, 408, 409, 410, 411, 412, 415, 416, 417, 419, 420, 421, 422, 424, 425, 426, 428, 429, 430, 432, 433, 434, 435, 436, 437, 439, 440, 442, 443, 444, 1872, 1873, 1874, 1875, 1876, 1877, 1881, 1885, 1888,1889,1890,1891,1893,1894,1896,1897,1900, 1901, 1903, 1906, 1907, 1908, 1909; 76-84 pour la protéine de séquence SEQ ID N°375; 76-84 pour la protéine de séquence SEQ ID N°427; 76-84 pour la protéine de séquence SEQ ID N°1878; 76-84 pour la protéine de séquence SEQ ID N°1898; 76-84 pour la protéine de séquence SEQ ID N°1899; 76-84 pour la protéine de séquence SEQ ID N°1902; 76-84 pour la protéine de séquence SEQ ID N°1904 | 2be |
| **SEQ ID N°494** | VMAVAAVLK | 77-85 pour les protéines de SEQ N°365, 366, 387, 388, 390, 393, 394, 395, 396, 398, 399, 406, 407, 413, 414, 418, 423, 431, 438, 441, 1879, 1880, 1882, 1883, 1884, 1886, 1892, 1895, 1905 | 2be |
| **SEQ ID N°495** | VTAFAR | 151-156 pour les protéines de SEQ N°351, 352, 353, 354, 355, 356, 357, 358, 359, 360, 361, 362, 363, 364, 365, 366, 367, 368, 369, 370, 371, 372, 373, 374, 376, 377, 378, 379, 380, 381, 382, 383, 384, 385, 386, 387, 388, 389, 390, 391, 392, 393, 394, 395, 396, 397, 398, 399, 400, 401, 402, 403, 404, 405, 406, 407, 408, 409, 410, 411, 412, 413, 414, 415, 416, 417, 418, 419, 420, 421, 422, 423, 424, 425, 426, 428, 429, 430, 431, 432, 433, 434, 435, 436, 437, 438, 439, 440, 441, 442, 443, 444, 445, 1872, 1873, 1874, 1875, 1876, 1877, 1879, 1880, 1881, 1882, 1883, 1884, 1885, 1886, 1887, 1888, 1889, 1890, 1891, 1892, 1893, 1894, 1895, 1896, 1897, 1900, 1901, 1903, 1905, 1906, 1907, 1908, 1909; 150-155 pour la protéine de séquence SEQ ID N°375; 150-155 pour la protéine de séquence SEQ ID N°427; 150-155 pour la protéine de séquence SEQ ID N°1878; 150-155 pour la protéine de séquence SEQ ID N°1898; 150-155 pour la protéine de séquence SEQ ID N°1899; 150-155 pour la protéine de séquence SEQ ID N°1902; 150-155 pour la protéine de séquence SEQ ID N°1904 | 2be |
| **SEQ ID N°2008** | AAGIR | 219-223 pour la protéine de SEQ N°425 | 2be |
| **SEQ ID N°2009** | AGADVASLR | 188-196 pour la protéine de SEQ N°425 | 2be |
| **SEQ ID N°2010** | AGLPASWVVGDK | 226-237 pour les protéines de SEQ N°354, 357, 358, 363, 364, 365, 370, 371, 372, 373, 374, 382, 385, 388, 389, 390, 391, 393, 394, 395, 396, 399, 400, 401, 404, 405, 406, 412, 417, 426, 428, 431, 433, 437, 438, 440, 441, 1875, 1876, 1879, 1880, 1883, 1884, 1885, 1887, 1890, 1895, 1905, 1907, 1909; 225-236 pour la protéine de séquence SEQ ID N°1899; 225-236 pour la protéine de séquence SEQ ID N°1902 | 2be |
| **SEQ ID N°2011** | AGLPTSWTAGDK | 226-237 pour les protéines de SEQ N°353, 359, 387 | 2be |
| **SEQ ID N°2012** | AGLPTSWTVGDR | 226-237 pour les protéines de SEQ N°1894 | 2be |
| **SEQ ID N°2013** | AGLPTSWWGDK | 225-236 pour les protéines de SEQ N°375, 427, 1878, 1904; 226-237 pour la protéine de séquence SEQ ID N°352; 226-237 pour la protéine de séquence SEQ ID N°366; 226-237 pour la protéine de séquence SEQ ID N°368; 226-237 pour la protéine de séquence SEQ ID N°383; 226-237 pour la protéine de séquence SEQ ID N°384; 226-237 pour la protéine de séquence SEQ ID N°414; 226-237 pour la protéine de séquence SEQ ID N°418; 226-237 pour la protéine de séquence SEQ ID N°420; 226-237 pour la protéine de séquence SEQ ID N°443 | 2be |
| **SEQ ID N°2014** | AGMPK | 226-230 pour la protéine de SEQ N°434 | 2be |
| **SEQ ID N°2015** | AIGDDTFR | 156-163 pour les protéines de SEQ N°375, 427, 1878, 1904 | 2be |
| **SEQ ID N°2016** | AIGDNTFR | 157-164 pour la protéine de SEQ N°352 | 2be |
| **SEQ ID N°2017** | AMAVAAVLK | 77-85 pour les protéines de SEQ N°1887 | 2be |
| **SEQ ID N°2018** | APLILVIYFTQPQPK | 258-272 pour la protéine de SEQ N°390 | 2be |
| **SEQ ID N°2019** | APLILVTYFTQPEQK | 257-271 pour les protéines de SEQ N°375, 427, 1878, 1904; 258-272 pour la protéine de séquence SEQ ID N°352 | 2be |
| **SEQ ID N°2020** | APLILVTYFTQPQPNAES R | 258-276 pour les protéines de SEQ N°406, 1884 | 2be |
| **SEQ ID N°2021** | APLVLVTYFTQPEPK | 258-272 pour la protéine de SEQ N°443 | 2be |
| **SEQ ID N°2022** | APLVLVTYFTQPQQNAE NR | 258-276 pour les protéines de SEQ N°1893 | 2be |
| **SEQ ID N°2023** | APLVLVTYFTQPQQNAE R | 258-275 pour les protéines de SEQ N°367, 398, 421, 1877,1882,1888 | 2be |
| **SEQ ID N°2024** | APLVLVTYFTQSEPK | 258-272 pour les protéines de SEQ N°366, 368, 383, 384, 414, 418, 420 | 2be |
| **SEQ ID N°2025** | AQLVAWLK | 208-215 pour la protéine de SEQ N°409 | 2be |
| **SEQ ID N°2026** | AQLVMWLK | 208-215 pour les protéines de SEQ N°366, 368, 383, 384, 414, 418, 420 | 2be |
| **SEQ ID N°2027** | ASDLVNYNPIAEK | 102-114 pour les protéines de SEQ N°429, 430, 434 | 2be |
| **SEQ ID N°2028** | CWVVGDK | 231-237 pour la protéine de SEQ N°429 | 2be |
| **SEQ ID N°2029** | DFLAAAAK | 278-285 pour les protéines de SEQ N°435, 1908 | 2be |
| **SEQ ID N°2030** | DILASAAK | 278-285 pour les protéines de SEQ N°358, 440, 441 | 2be |
| **SEQ ID N°2031** | DLLSQR | 92-97 pour la protéine de SEQ N°443 | 2be |
| **SEQ ID N°2032** | DNTQVLYR | 57-64 pour les protéines de SEQ N°353, 355, 356, 359, 360, 361, 362, 367, 369, 376, 377, 378, 379, 380, 381, 387, 398, 402, 403, 407, 409, 410, 411, 413, 421, 423, 425, 436, 1872, 1873, 1874, 1877, 1882, 1886, 1888, 1889, 1891, 1892, 1893, 1894, 1896, 1897, 1900, 1901 | 2be |
| **SEQ ID N°2033** | DTTTAR | 182-187 pour la protéine de SEQ N°425 | 2be |
| **SEQ ID N°2034** | DTTTPLAMAQALR | 182-194 pour les protéines de SEQ N°366, 368, 383, 384, 414,420 | 2be |
| **SEQ ID N°2035** | DTTTPLAMAQSLR | 182-194 pour la protéine de SEQ N°418 | 2be |
| **SEQ ID N°2036** | DTTTPLAMAQTLR | 181-193 pour les protéines de SEQ N°375, 427, 1878, 1904; 182-194 pour la protéine de séquence SEQ ID N°352; 182-194 pour la protéine de séquence SEQ ID N°443 | 2be |
| **SEQ ID N°2037** | DVLAAAAR | 278-285 pour les protéines de SEQ N°366, 368, 383, 384, 414, 418, 420, 443 | 2be |
| **SEQ ID N°2038** | EIGDETFR | 157-164 pour la protéine de SEQ N°356 | 2be |
| **SEQ ID N°2039** | EQLVTWLK | 208-215 pour les protéines de SEQ N°1891, 1892 | 2be |
| **SEQ ID N°2040** | GLLSQR | 92-97 pour les protéines de SEQ N°366, 368, 383, 384, 414, 418, 420 | 2be |
| **SEQ ID N°2041** | GNTTGAAR | 216-223 pour la protéine de SEQ N°376 | 2be |
| **SEQ ID N°2042** | GNTTGSASIQAGLPK | 216-230 pour les protéines de SEQ N°442, 445 | 2be |
| **SEQ ID N°2043** | HDVLASAAK | 277-285 pour les protéines de SEQ N°412, 1885 | 2be |
| **SEQ ID N°2044** | HDVLASAAR | 277-285 pour les protéines de SEQ N°1894 | 2be |
| **SEQ ID N°2045** | HLTLGSALGETQR | 194-206 pour les protéines de SEQ N°375, 427, 1878, 1904 | 2be |
| **SEQ ID N°2046** | LAELEQQSGGR | 37-47 pour les protéines de SEQ N°354, 373, 374, 390 | 2be |
| **SEQ ID N°2047** | LAGLER | 37-42 pour la protéine de SEQ N°357 | 2be |
| **SEQ ID N°2048** | LDGTEPTLNTAIPGDPR | 165-181 pour la protéine de SEQ N°382 | 2be |
| **SEQ ID N°2049** | LGVALIDTADNAQTLYR | 47-63 pour les protéines de SEQ N°375, 427, 1878, 1904; 48-64 pour la protéine de séquence SEQ ID N°352 | 2be |
| **SEQ ID N°2050** | LGVALIDTADNTHVLYR | 48-64 pour la protéine de SEQ N°408 | 2be |
| **SEQ ID N°2051** | LGVALIDTK | 48-56 pour la protéine de SEQ N°356 | 2be |
| **SEQ ID N°2052** | LGVALINTADNSQILYLAD ER | 48-68 pour la protéine de SEQ N°429 | 2be |
| **SEQ ID N°2053** | LGVALINTADNSQILYVA DER | 48-68 pour la protéine de SEQ N°430 | 2be |
| **SEQ ID N°2054** | LGVALINTADNSR | 47-59 pour les protéines de SEQ N°1902 | 2be |
| **SEQ ID N°2055** | LGVALINTADNTQTLYR | 48-64 pour les protéines de SEQ N°366, 368, 383, 384, 414, 418, 420, 443 | 2be |
| **SEQ ID N°2056** | LGVAQINTADNSQILYVA DER | 48-68 pour la protéine de SEQ N°434 | 2be |
| **SEQ ID** | LGVPLIDTADNTQVLYR | 48-64 pour les protéines de SEQ N°379, 380, 381 | 2be |
| **SEQ ID N°2058** | LIAHLGGPGK | 141-150 pour la protéine de SEQ N°443 | 2be |
| **SEQ ID N°2059** | LIAQLGGQGGVTAFAR | 141-156 pour les protéines de SEQ N°1897 | 2be |
| **SEQ ID N°2060** | LISHVGGPASVTAFAR | 141-156 pour les protéines de SEQ N°395, 396, 426, 431, 438, 1880, 1905 | 2be |
| **SEQ ID N°2061** | LLLNQR | 92-97 pour la protéine de SEQ N°392 | 2be |
| **SEQ ID N°2062** | NLTLGNALGDTQR | 195-207 pour les protéines de SEQ N°366, 368, 383, 384, 414, 418, 420, 443 | 2be |
| **SEQ ID N°2063** | NLTLGSALGETQR | 195-207 pour la protéine de SEQ N°352 | 2be |
| **SEQ ID N°2064** | NVLSQK | 91-96 pour les protéines de SEQ N°375, 427, 1878, 1904 | 2be |
| **SEQ ID N°2065** | QLGDDTFR | 157-164 pour la protéine de SEQ N°404 | 2be |
| **SEQ ID N°2066** | SDLVNYSPIAEK | 103-114 pour la protéine de SEQ N°370 | 2be |
| **SEQ ID N°2067** | SESEPSLLNQR | 87-97 pour les protéines de SEQ N°358, 440, 441 | 2be |
| **SEQ ID N°2068** | SETQK | 86-90 pour les protéines de SEQ N°375, 427, 1878, 1904; 87-91 pour la protéine de séquence SEQ ID N°352; 87-91 pour la protéine de séquence SEQ ID N°353; 87-91 pour la protéine de séquence SEQ ID N°355; 87-91 pour la protéine de séquence SEQ ID N°356; 87-91 pour la protéine de séquence SEQ ID N°359; 87-91 pour la protéine de séquence SEQ ID N°360; 87-91 pour la protéine de séquence SEQ ID N°361; 87-91 pour la protéine de séquence SEQ ID N°362; 87-91 pour la protéine de séquence SEQ ID N°366; 87-91 pour la protéine de séquence SEQ ID N°367; 87-91 pour la protéine de séquence SEQ ID N°368; 87-91 pour la protéine de séquence SEQ ID N°369; 87-91 pour la protéine de séquence SEQ ID N°376; 87-91 pour la protéine de séquence SEQ ID N°377; 87-91 pour la protéine de séquence SEQ ID N°378; 87-91 pour la protéine de séquence SEQ ID N°379; 87-91 pour la protéine de séquence SEQ ID N°380; 87-91 pour la protéine de séquence SEQ ID N°381; 87-91 pour la protéine de séquence SEQ ID N°383; 87-91 pour la protéine de séquence SEQ ID N°384; 87-91 pour la protéine de séquence SEQ ID N°387; 87-91 pour la protéine de séquence SEQ ID N°398; 87-91 pour la protéine de séquence SEQ ID N°402; 87-91 pour la protéine de séquence SEQ ID N°403; 87-91 pour la protéine de séquence SEQ ID N°407; 87-91 pour la protéine de séquence SEQ ID N°408; 87-91 pour la protéine de séquence SEQ ID N°409; 87-91 pour la protéine de séquence SEQ ID N°410; 87-91 pour la protéine de séquence SEQ ID N°411; 87-91 pour la protéine de séquence SEQ ID N°413; 87-91 pour la protéine de séquence SEQ ID N°414; 87-91 pour la protéine de séquence SEQ ID N°418; 87-91 pour la protéine de séquence SEQ ID N°420; 87-91 pour la protéine de séquence SEQ ID N°421; 87-91 pour la protéine de séquence SEQ ID N°423; 87-91 pour la protéine de séquence SEQ ID N°425; 87-91 pour la protéine de séquence SEQ ID N°428; 87-91 pour la protéine de séquence SEQ ID N°436; 87-91 pour la protéine de séquence SEQ ID N°443; 87-91 pour la protéine de séquence SEQ ID N°1872; 87-91 pour la protéine de séquence SEQ ID N°1873; 87-91 pour la protéine de séquence SEQ ID N°1874; 87-91 pour la protéine de séquence SEQ ID N°1877; 87-91 pour la protéine de séquence SEQ ID N°1882; 87-91 pour la protéine de séquence SEQ ID N°1886; 87-91 pour la protéine de séquence SEQ ID N°1888; 87-91 pour la protéine de séquence SEQ ID N°1889; 87-91 pour la protéine de séquence SEQ ID N°1891; 87-91 pour la protéine de séquence SEQ ID N°1892; 87-91 pour la protéine de séquence SEQ ID N°1893; 87-91 pour la protéine de séquence SEQ ID N°1894; 87-91 pour la protéine de séquence SEQ ID N°1896; 87-91 pour la protéine de séquence SEQ ID N°1897; 87-91 pour la protéine de séquence SEQ ID N°1900; 87-91 pour la protéine de séquence SEQ ID N°1901 | 2be |
| **SEQ ID N°2069** | SLGDESFR | 157-164 pour la protéine de SEQ N°439 | 2be |
| **SEQ ID N°2070** | SSDLINYNPIAEK | 101-113 pour les protéines de SEQ N°375, 427, 1878, 1904; 102-114 pour la protéine de séquence SEQ ID N°443 | 2be |
| **SEQ ID N°2071** | SSDLINYNPITEK | 102-114 pour la protéine de SEQ N°352 | 2be |
| **SEQ ID N°2072** | SWGVGDK | 231-237 pour les protéines de SEQ N°351, 430, 434, 444 | 2be |
| **SEQ ID N°2073** | TELTLNTAIPGDPR | 168-181 pour la protéine de SEQ N°407 | 2be |
| **SEQ ID N°2074** | TEPTLNSAIPGDPR | 168-181 pour les protéines de SEQ N°429, 430, 434 | 2be |
| **SEQ ID N°2075** | TEPTQNTAIPGDPR | 168-181 pour les protéines de SEQ N°1889 | 2be |
| **SEQ ID N°2076** | TEQTLNTAIPGDPR | 168-181 pour la protéine de SEQ N°391 | 2be |
| **SEQ ID N°2077** | TESTLNTAIPGDPR | 168-181 pour les protéines de SEQ N°361, 388, 400, 421, 1873,1888 | 2be |
| **SEQ ID n°2078** | TETTLNTAIPGDPR | 168-181 pour les protéines de SEQ N°365, 385, 395, 415 | 2be |
| **SEQ ID n°2079** | TGSCDYGTTNDIAVIWPK | 238-255 pour la protéine de SEQ N°373 | 2be |
| **SEQ ID n°2080** | TGSCGYGTTNDIAVIWPK | 238-255 pour la protéine de SEQ N°417 | 2be |
| **SEQ ID N°2081** | TGSGDYGTTNDIAVIWPE GR | 237-256 pour les protéines de SEQ N°375, 427, 1878, 1904; 238-257 pour la protéine de séquence SEQ ID N°352; 238-257 pour la protéine de séquence SEQ ID N°368; 238-257 pour la protéine de séquence SEQ ID N°383; 238-257 pour la protéine de séquence SEQ ID N°414; 238-257 pour la protéine de séquence SEQ ID N°418; 238-257 pour la protéine de séquence SEQ ID N°443 | 2be |
| **SEQ ID n°2082** | TGSGGYGTTNDIAVIWPE GR | 238-257 pour les protéines de SEQ N°366, 384, 420 | 2be |
| **SEQ ID n°2083** | TGSGGYGTTNDIAVIWP QGR | 238-257 pour les protéines de SEQ N°359, 369, 423, 1889,1891,1892 | 2be |
| **SEQ ID n°2084** | TIGDDTFR | 157-164 pour les protéines de SEQ N°366, 368, 383, 384, 414, 418, 420 | 2be |
| **SEQ ID n°2085** | TQLVTWLK | 208-215 pour la protéine de SEQ N°419 | 2be |
| **SEQ ID N°2086** | VEIKPSDLINYNPIAEK | 98-114 pour les protéines de SEQ N°366, 368, 383, 414, 418,420 | 2be |
| **SEQ ID n°2087** | VEIKPSDLVNYNPIAEK | 98-114 pour la protéine de SEQ N°384 | 2be |
| **SEQ ID n°2088** | VIGDDTFR | 157-164 pour la protéine de SEQ N°443 | 2be |
| **SEQ ID n°2089** | VLSQK | 92-96 pour les protéines de SEQ N°375, 427, 1878, 1904; 93-97 pour la protéine de séquence SEQ ID N°352 | 2be |
| **SEQ ID n°2090** | VMAAAALLK | 77-85 pour la protéine de SEQ N°445 | 2be |
| **SEQ ID n°2091** | VMAAAAVLEQSETQK | 77-91 pour la protéine de SEQ N°403 | 2be |
| **SEQ ID n°2092** | WAKPSGAVGDVAQR | 201-214 pour la protéine de SEQ N°425 | 2be |

Dans la colonne intérêt clinique, la mention 2be que l'ensemble des peptides CTX-M signe la présence d'une béta-lactamase avec un spectre étendu (BLSE) capable d'hydrolyser les pénicillines, les céphalosporines de première génération telles que la céphaloridine et la céphalotine ainsi qu'au moins un antibiotique de la classe des oxyimino-beta-lactames tels que le cefotaxime, le ceftazidime ou les monobactames tels que l'aztreoname.

La détection d'un mécanisme de résistance aux céphalosporines de type BLSE (béta-lactamase à spectre étendu), induit par la protéine CTX-M, est caractérisée par la détection d'au moins un peptide marqueur de résistance, de type 2be, choisi parmi les séquences **SEQ ID N°446** à **SEQ ID N°478, SEQ ID N°480** à **SEQ ID N°495** et **SEQ ID N° 2008 à SEQ ID N°2092**

La détection d'un mécanisme de résistance aux céphalosporines induit par l'expression de la protéine SHV est caractérisée par la détection d'au moins un peptide appartenant à la protéine SHV et à ses différents variants de séquences **SEQ ID N° 496** à **SEQ ID N°613** et **SEQ ID N° 1909** à **SEQ ID N°1919.**
**SEQ ID N° 496 :** 
KRYIRLCIISLLATLPLAVHASPQPLEQIKLSESQLSGRVGMIEMDLASGRTLTAWRA
DERFPMMSTFKVVLCGAVLARVDAGDEQLERKIHYRQQDLVDYSPVSEKHLADGM
TVGELCAAAITMSDNSAANLLLATVGGPAGLTAFLRQIGDNVTRLDRWETELNEALP
GDARDTTTPASMAATLRKLLTSQRLSARSQRQLLQWMVDDRVAGPLIRSVLPAGW
FIADKTGAGERGARGIVALLGPNNKAERIVVIYLRDTPASMAERNQQIAGIGAALIEH
WQR
**SEQ ID N° 497 :** 
LRYIRLCIISLLATLPLAVHASPQPLEQIKLSESQLSGRVGMIEMDLASGRTLTAWRAD
ERFPMMSTFKVVLCGAVLARVDAGDEQLERKIHYRQQDLVDYSPVSEKHLADGMT
VGELCAAAITMSDNSAANLLLATVGGPAGLTAFLRQIGDNVTRLDRWETELNEALPG
DARDTTTPASMAATLRKLLTSQRLSASSQRQLLQWMVDDRVAGPLIRSVLPAGWFI
ADKTGAGERGARGIVALLGPNNKAERIVVIYLRDTPASMAERNQQIAGIGAALIEHW
QR
**SEQ ID N° 498 :** 
MRFIRLCIISLLATLPLAVHASPQPLEQIKLSESQLSGRVGMIEMDLASGRTLTAWRA
DERFPMMSTFKVVLCGAVLARVDAGDEQLERKIHYRQQDLVDYSPVSEKHLADGM
TVGELCAAAITMSDNSAANLLLATVGGPAGLTAFLRQIGDNVTRLDRWETELNEALP
GDARDTTTPASMAATLRKLLTSQRLSARSQRQLLQWMVDDRVAGPLIRSVLPAGW
FIADKTGAGERGARGIVALLGPNNKAERIVVIYLRDTPASMAERNQQIAGIGAALIEH
WQR
**SEQ ID N° 499 :** 
MRFIRLCIISLLATLPLAVHASPQPLEQIKLSESQLSGRVGMIEMDLASGRTLTAWRA
DERFPMMSTFKVVLCGAVLARVDAGDEQLERKIHYRQQDLVDYSPVSEKHLADGM
TVGELCAAAITMSDNSAANLLLATVGGPAGLTAFLRQIGDNVTRLDRWETELNEALP
GDARDTTTPASMAATLRKLLTSQRLSARSQRQLLQWMVDDRVAGPLIRSVLPAGW
FIADKTGAGERGARGIVALLGPNNNAERMVVIYLRDTPASMAERNQQIAGIGAALIEH
WQR
**SEQ ID N° 500 :** 
MRFIRLCIISLLATLPLAVHASPQPLEQIKLSESQLSGRVGMIEMDLASGRTLTAWRA
DERFPMMSTFKVVLCGAVLARVDAGDEQLERKIHYRQQDLVDYSPVSEKHLADGM
 
TVGELCAAAITMSDNSAANLLLATVGGPAGLTAFLRQIGDNVTRLDRWETELNEALP
GDARDTTTPASMAATLRKLLTSQRLSARSQRQLLQWMVDDRVAGPLIRSVLPAGW
FIADKTGASKRGARGIVALLGPNNKAERIVVIYLRDTPASMAERNQQIAGIGAALIEH
WQR
**SEQ ID N° 501 :** 
MRIRLCIISLLATLPLAVHASPQPLEQIKLSESQLSGRVGMIEMDLASGRTLTAWRAD
ERFPMMSTFKVVLCGAVLARVDAGDEQLERKIHYRQQDLVDYSPVSEKHLADGMT
VGELCAAAITMSDNSAANLLLATVGGPVGLTAFLRQIGDNVTRLDRWETELNEALPG
DARDTTTPASMAATLRKLLTSQRLSARSQRQLLQWMVDDRVAGPLIRSVLPAGWFI
ADRTGAGERGARGIVALLGPNNKAERIVVIYLRDTPASMAERNQQIAGIGAALIEHW
QR
**SEQ ID N° 502 :** 
MRYARLCIISLLATLPLVVHASPQPLEQIKQSESQLSGRVGMIEMDLASGRTLTAWR
ADERFPMMSTFKVVLCGAVLARVDAGDEQLERKIHYRQQDLVDYSPVSEKHLADG
MTVGELCAAAITVSDNSAANLLLATVGGPAGLTAFLRQIGDNVTRLDRWETELNEAL
PGDARDTTTPASMAATLRKLLTSQRLSARSQRQLLQWMVDDRVAGPLIRSVLPAG
WFIADKTGAGERGARGIVALLGPNNKAERIVVIYLRDTPASMAERNQQIAGIGAALIE
HWQR
**SEQ ID N° 503 :** 
MRYFRLCIISLLATLPLAVHASPQPLEQIKLSESQLSGRVGMIEMDLASGRTLTAWRA
DERFPMMSTFKVVLCGAVLARVDAGDEQLERKIHYRQQDLVDYSPVSEKHLADGM
TVGELCAAAITMSDNSAANLLLATVGGPAGLTAFLRQIGDNVTRLDRWETELNEALP
GDARDTTTPASMAATLRKLLTSQRLSARSQRQLLQWMVDDRVAGPLIRSVLPAGW
FIADKTGASERGARGIVALLGPNNKAERIVVIYLRDTPASMAERNQQIAGIGAALIEH
WQR
**SEQ ID N° 504 :** 
MRYFRLCIISLLATLPLAVHASPQPLEQIKLSESQLSGRVGMIEMDLASGRTLTAWRA
DERFPMMSTFKVVLCGAVLARVDAGDEQLERKIHYRQQDLVDYSPVSEKHLADGM
TVGELCAAAITMSDNSAANLLLATVGGPVGLTAFLRQIGDNVTRLDRWETELNEALP
GDARDTTTPASMAATLRKLLTSQRLSARSQRQLLQWMVDDRVAGPLIRSVLPAGW
FIADRTGAGERGARGIVALLGPNNKAERIVVIYLRDTPASMAERNQQIAGIGAALIEH
WQR
**SEQ ID N° 505 :** 
MRYFRLCIISLLATLPLAVHASPQPLEQIKLSESQLSGSVGMIEMDLASGRTLTAWRA
DERFPMMSTFKVVLCGAVLARVDAGDEQLERKIHYRQQDLVDYSPVSEKHLADGM
TVGELCAAAITMSDNSAANLLLATVGGPAGLTAFLRQIDDNVTRLDRWETELNEALP
GDARDTTTPASMAATLRKLLTSQRLSARSQRQLLQWMVDDRVAGPLIRSVLPAGW
FIADKTGASKRGARGIVALLGPNNKAERIVVIYLRDTPASMAERNQQIAGIGAALIEH
WQR
**SEQ ID N° 506 :** 
MRYFRLCIISLLATLPLAVHASPQPLEQIKLSESQLSGSVGMIEMDLASGRTLTAWRA
DERFPMMSTFKVVLCGAVLARVDAGDEQLERKIHYRQQDLVDYSPVSEKHLADGM
TVGELCAAAITMSDNSAANLLLATVGGPAGLTAFLRQIGDNVTRLDRWETELNEALP
GDARDTTTPASMAATLRKLLTSQRLSARSQRQLLQWMVDDRVAGPLIRSVLPAGW
FIADKTGAAKRGARGIVALLGPNNKAERIVVIYLRDTPASMAERNQQIAGIGAALIEH
WQR
**SEQ ID N° 507 :** 
MRYFRLCIISLLATLPLAVHASPQPLEQIKLSESQLSGSVGMIEMDLASGRTLTAWRA
DERFPMMSTFKVVLCGAVLARVDAGDEQLERKIHYRQQDLVDYSPVSEKHLADGM
TVGELCAAAITMSDNSAANLLLATVGGPAGLTAFLRQIGDNVTRLDRWETELNEALP
 
GDARDTTTPASMAATLRKLLTSQRLSARSQRLLQWMVDDRVAGPLIRSVLPAGW
FIADKTGAGERGARGIVALLGPNNKAERIVVIYLRDTPASMAERNQQIAGIGAALIEH
WQR
**SEQ ID N° 508 :** 
MRYFRLCIISLLATLPLAVHASPQPLEQIKLSESQLSGSVGMIEMDLASGRTLTAWRA
DERFPMMSTFKVVLCGAVLARVDAGDEQLERKIHYRQQDLVDYSPVSEKHLADGM
TVGELCAAAITMSDNSAANLLLATVGGPAGLTAFLRQIGDNVTRLDRWETELNEALP
GDARDTTTPASMAATLRKLLTSQRLSARSQRQLLQWMVDDRVAGPLIRSVLPAGW
FIADKTGASERGARGIVALLGPNNKAERIVVIYLRDTPASMAERNQQIAGIGAALIEH
WQR
**SEQ ID N° 509 :** 
MRYFRLCIISLLATLPLAVHASPQPLEQIKLSESQLSGSVGMIEMDLASGRTLTAWRA
DERFPMMSTFKVVLCGAVLARVDAGDEQLERKIHYRQQDLVDYSPVSEKHLADGM
TVGELCAAAITMSDNSAANLLLATVGGPAGLTAFLRQIGDNVTRLDRWETELNEALP
GDARDTTTPASMAATLRKLLTSQRLSARSQRQLLQWMVDDRVAGPLIRSVLPAGW
FIADKTGASKRGARGIVALLGPNNKAERIVVIYLRDTPASMAERNQQIAGIGAALIEH
WQR
**SEQ ID N° 510 :** 
MRYFRLCIISLLATLPLAVHASPQPLEQIKLSESQLSGSVGMIEMDLASGRTLTAWRA
DGRFPMMSTFKVVLCGAVLARVDAGDEQLERKIHYRQQDLVDYSPVSEKHLADGM
TVGELCAAAITMSDNSAANLLLATVGGPAGLTAFLRQIGDNVTRLDRWETELNEALP
GDARDTTTPASMAATLRKLLTSQRLSARSQRQLLQWMVDDRVAGPLIRSVLPAGW
FIADKTGASERGARGIVALLGPNNKAERIVVIYLRDTPASMAERNQQIAGIGAALIEH
WQR
**SEQ ID N° 511 :** 
MRYIRLCIISLLAALPLAVHASPQPLEQIKQSESQLSGRVGMIEMDLASGRTLTAWRA
DERFPMISTFKVVLCGAVLARVDAGDEQLERKIHYRQQDLVDYSPVSEKHLADGMT
VGELCAAAITMSDNSAANLLLAIVGGPAGLTAFLRQIGDNVTRLDRWETELNEALPG
DARDTTTPASMAATLRKLLTSQRLSARSQRQLLQWMVDDRVAGPLIRSVLPAGWFI
ADKTGAGERGARGIVALLGPNNKAERIVVIYLRDTPASMAERNQQIAGIGAALIEHW
QR
**SEQ ID N° 512 :** 
MRYIRLCIISLLAALPLAVHASPQPLEQIKQSESQLSGRVGMIEMDLASGRTLTAWRA
DERFPMMSTFKVVLCGAVLARVDAGDEQLERKIHYRQQDLVDYSPVSEKHLADGM
TVGELCAAAITMSDNSAANLLLATVGGPAGLTAFLRQIGDNVTRLDRWETELNEALP
GDARDTTTPASMAATLRKLLTSQRLSARSQRQLLQWMVEDRVAGPLIRSVLPAGW
FIADKTGAGERGARGIVALLGPNNKAERIVVIYLRDTPASMAERNQQIAGIGAALIEH
WQR
**SEQ ID N° 513 :** 
MRYIRLCIISLLAALPLAVHASPQPLEQIKQSESQLSGRVGMIEMDLASGRTLTAWRA
DERFPMMSTFKVVLCGAVLARVDAGDEQLERKIHYRQQDLVDYSPVSEKHLADGM
TVGELCAAAITVSDNSAANLLLATVGGPAGLTAFLRQIGDNVTRLDRWETELNEALP
GDARDTTTPASMAATLRKLLTSQRLSARSQRQLLQWMVDDRVAGPLIRSVLPAGW
FIADKTGAGERGARGIVALLGPNNKAERIVVIYLRDTPASMAERNQHIAGIGAALIEH
WQR
**SEQ ID N° 514 :** 
MRYIRLCIISLLAALPLAVHASPQPLEQIKQSESQLSGRVGMIEMDLASGRTLTAWRA
DERFPMMSTFKVVLCGAVLARVDAGDEQLERKIHYRQQDLVDYSPVSEKHLADGM
TVGELCAAAITVSDNSAANLLLATVGGPAGLTAFLRQIGDNVTRLDRWETELNEALP
GDARDTTTPASMAATLRKLLTSQRLSARSQRQLLQWMVDDRVAGPLIRSVLPAGW
 
FIADKTGAGERGARGIVALLGPNNKAERIVVIYLRDTPASMAERNQQIAGIGAALIEH
WQR
**SEQ ID N° 515 :** 
MRYIRLCIISLLATLPLAVHASPQPLDQIKLSESQLSGRVGMIEMDLASGRTLTAWRA
DERFPMMSTFKVVLCGAVLARVDAGDEQLERKIHYRQQDLVDYSPVSEKHLADGM
TVGELCAAAITMSDNSAANLLLATVGGPAGLTAFLRQIGDNVTRLDRWETELNEALP
GDARDTTTPASMAATLRKLLTSQRLSARSQRQLLQWMVDDRVAGPLIRSVLPAGW
FIADKTGASKRGARGIVALLGPNNKAERIVVIYLRDTPASMAERNQQIAGIGAALIEH
WQR
**SEQ ID N° 516** : 
MRYIRLCIISLLATLPLAVHASPQPLEQIKLSESQLSGRVGMIEMDLASGRTLTAWHA
DERFPMMSTFKVVLCGAVLARVDAGDEQLERKIHYRQQDLVDYSPVSEKHLADGM
TVGELCAAAITMSDNSAANLLLATVGGPAGLTAFLRQIGDNVTRLDRWETELNEALP
GDARDTTTPASMAATLRKLLTSQRLSARSQRQLLQWMVDDRVAGPLIRSVLPAGW
FIADKTGAGERGARGIVALLGPNNKAERIVVIYLRDTPASMAERNQQIAGIGAALIEH
WQR
**SEQ ID N° 517 :** 
MRYIRLCIISLLATLPLAVHASPQPLEQIKLSESQLSGRVGMIEMDLASGRTLTAWHA
DERFPMMSTFKVVLCGAVLARVDAGDEQLERKIHYRQQDLVDYSPVSEKHLADGM
TVGELCAAAITMSDNSAANLLLATVGGPAGLTAFLRQIGDNVTRLDRWETELNEALP
GDARDTTTPASMAATLRKLLTSQRLSARSQRQLLQWMVDDRVAGPLIRSVLPAGW
FIADKTGAGKRGARGIVALLGPNNKAERIVVIYLRDTPASMAKRNQQIAGIGAALIEH
WQR
**SEQ ID N° 518 :** 
MRYIRLCIISLLATLPLAVHASPQPLEQIKLSESQLSGRVGMIEMDLASGRTLTAWRA
DERFPMISTFKVVLCGAVLARVDAGDEQLERKIHYRQQDLVDYSPVSEKHLADGMT
VGELCAAAITMSDNSAANLLLATVGGPAGLTAFLRQIGDNVTRLDRWETELNEALPG
DARDTTTPASMAATLRKLLTSQRLSARSQRQLLQWMVDDRVAGPLIRSVLPAGWFI
ADKTGAGERGARGIVALLGPNNKAERIVVIYLRDTPASMAERNQQIAGIGAALIEHW
QR
**SEQ ID N° 519 :** 
MRYIRLCIISLLATLPLAVHASPQPLEQIKLSESQLSGRVGMIEMDLASGRTLTAWRA
DERFPMMSTFKVVLCGAVLARVDAGDEQLERKIHYLQQDLVDYSPVSEKHLADGM
TVGELCAAAITMSDNSAANLLLATVGGPAGLTAFLRQIGDNVTRLDRWETELNEALP
GDARDTTTPASMAATLRKLLTSQRLSARSQRQLLQWMVDDRVAGPLIRSVLPAGW
FIADKTGAGERGARGIVALLGPNNKAERIVVIYLRDTPASMAERNQQIAGIGAALIEH
WQR
**SEQ ID N° 520 :** 
MRYIRLCIISLLATLPLAVHASPQPLEQIKLSESQLSGRVGMIEMDLASGRTLTAWRA
DERFPMMSTFKVVLCGAVLARVDAGDEQLERKIHYRQQDLVDYSPVSEKHFADGM
TVGELCAAAITMSDNSAANLLLATVGGPAGLSAFLRQIGDNVTRLDRWETELNEALP
GDARDTTTPASMAATLRKLLTSQRLSARSQRQLLQWMVDDRVAGPLIRSVLPAGW
FIADKTGAGERGARGIVALLGPNNKAERIVVIYLRDTPASMAERNQQIAGIGAALIEH
WQR
**SEQ ID N° 521 :** 
MRYIRLCIISLLATLPLAVHASPQPLEQIKLSESQLSGRVGMIEMDLASGRTLTAWRA
DERFPMMSTFKVVLCGAVLARVDAGDEQLERKIHYRQQDLVDYSPVSEKHLADGM
TIGELCAAAITMSDNSAANLLLATVGGPAGLTAFLRQIGDNVTRLDRWETELNEALP
GDARDTTTPASMAATLRKLLTSQRLSARSQRQLLQWMVDDRVAGPLIRSVLPAGW
 
FIADKTGAGERGARGIVALLGPNNKAERIVVIYLRDTPASMAERNQQIAGIGAALIEH
WQR
**SEQ ID N° 522 :** 
MRYIRLCIISLLATLPLAVHASPQPLEQIKLSESQLSGRVGMIEMDLASGRTLTAWRA
DERFPMMSTFKVVLCGAVLARVDAGDEQLERKIHYRQQDLVDYSPVSEKHLADGM
TVGEFCAAAITMSDNSAANLLLATVGGPAGLTAFLRQIGDNVTRLDRWETELNEAFP
GDARDTTTPASMAATLRKLLTSQRLSARSQRQLLQWMVDDRVAGPLIRSVLPAGW
FIADKTGASERGARGIVALLGPNNKAERIVVIYLRDTPASMAERNQQIAGIGAALIEH
WQR
**SEQ ID N° 523 :** 
MRYIRLCIISLLATLPLAVHASPQPLEQIKLSESQLSGRVGMIEMDLASGRTLTAWRA
DERFPMMSTFKVVLCGAVLARVDAGDEQLERKIHYRQQDLVDYSPVSEKHLADGM
TVGEFCAAAITMSDNSAANLLLATVGGPAGLTAFLRQIGDNVTRLDRWETELNEALP
GDARDTTTPASMAGTLRKLLTSQRLSARSQRQLLQWMVDDRVAGPLIRSVLPAGW
FIADKTGASKRGARGIVALLGPNNKAERIVVIYLRDTPASMAERNQQIAGIGAALIEH
WQR
**SEQ ID N° 524 :** 
MRYIRLCIISLLATLPLAVHASPQPLEQIKLSESQLSGRVGMIEMDLASGRTLTAWRA
DERFPMMSTFKVVLCGAVLARVDAGDEQLERKIHYRQQDLVDYSPVSEKHLADGM
TVGELCAAAITMSDNSAAKLLLATVGGPAGLTAFLRQIGDNVTRLDRWETELNEALP
GDARDTTTPASMAATLRKLLTSQRLSARSQRQLLQWMVDDRVAGPLIRSVLPAGW
FIADKTGAGERGARGIVALLGPNNKAERIVVIYLRDTPASMAERNQQIAGIGAALIEH
WQR
**SEQ ID N° 525 :** 
MRYIRLCIISLLATLPLAVHASPQPLEQIKLSESQLSGRVGMIEMDLASGRTLTAWRA
DERFPMMSTFKVVLCGAVLARVDAGDEQLERKIHYRQQDLVDYSPVSEKHLADGM
TVGELCAAAITMSDNSAANLLLATFGGPAGLTAFLRQIGDNVTRLDRWETELNEALP
GDARDTTTPASMAATLRKLLTSQRLSARSQRQLLQWMVDDRVAGPLIRSVLPAGW
FIADKTGAGERGARGIVALLGPNNKAERIVVIYLRDTPASMAERNQQIAGIGAALIEH
WQR
**SEQ ID N° 526 :** 
MRYIRLCIISLLATLPLAVHASPQPLEQIKLSESQLSGRVGMIEMDLASGRTLTAWRA
DERFPMMSTFKVVLCGAVLARVDAGDEQLERKIHYRQQDLVDYSPVSEKHLADGM
TVGELCAAAITMSDNSAANLLLATVGGLAGLTAFLRQIGDNVTRLDRWETELNEALP
ADARDTTTPASMAATLRKLLTSQRLSARSQRQLLQWMVDDRVAGPLIRSVLPAGW
FIADKTGAGERGARGIVALLGPNNKAERIVVIYLRDTPASMAERNQQIAGIGAALIEH
WQR
**SEQ ID N° 527 :** 
MRYIRLCIISLLATLPLAVHASPQPLEQIKLSESQLSGRVGMIEMDLASGRTLTAWRA
DERFPMMSTFKVVLCGAVLARVDAGDEQLERKIHYRQQDLVDYSPVSEKHLADGM
TVGELCAAAITMSDNSAANLLLATVGGPAGLTAFLRQIDDNVTRLDRWETELNEALP
GDARDTTTPASMAATLRKLLTSQRLSARSQRQLLQWMVDDRVAGPLIRSVLPAGW
FIADKTGAGERGARGIVALLGPNNKAERIVVIYLRDTPASMAERNQQIAGIGAALIEH
WQR
**SEQ ID N° 528 :** 
MRYIRLCIISLLATLPLAVHASPQPLEQIKLSESQLSGRVGMIEMDLASGRTLTAWRA
DERFPMMSTFKVVLCGAVLARVDAGDEQLERKIHYRQQDLVDYSPVSEKHLADGM
TVGELCAAAITMSDNSAANLLLATVGGPAGLTAFLRQIDDNVTRLDRWETELNEALP
GDARDTTTPASMAATLRKLLTSQRLSARSQRQLLQWMVDDRVAGPLIRSVLPAGW
 
FIADKTGASKRGARGIVALLGPNNKAERIVVIYLRDTPASMAERNQQIAGIGAALIEH
WQR
**SEQ ID N° 529 :** 
MRYIRLCIISLLATLPLAVHASPQPLEQIKLSESQLSGRVGMIEMDLASGRTLTAWRA
DERFPMMSTFKVVLCGAVLARVDAGDEQLERKIHYRQQDLVDYSPVSEKHLADGM
TVGELCAAAITMSDNSAANLLLATVGGPAGLTAFLRQIGDKVTRLDRWETELNEALP
GDARDTTTPASMAATLRKLLTSQRLSARSQRQLLQWMVDDRVAGPLIRSVLPAGW
FIADKTGASKRGARGIVALLGPNNKAERIVVIYLRDTPASMAERNQQIAGIGAALIEH
WQR
**SEQ ID N° 530 :** 
MRYIRLCIISLLATLPLAVHASPQPLEQIKLSESQLSGRVGMIEMDLASGRTLTAWRA
DERFPMMSTFKVVLCGAVLARVDAGDEQLERKIHYRQQDLVDYSPVSEKHLADGM
TVGELCAAAITMSDNSAANLLLATVGGPAGLTAFLRQIGDNVTRLDRWETDRWETE
LNEALPGDARDTTTPASMAATLRKLLTSQRLSARSQRQLLQWMVDDRVAGPLIRSV
LPAGWFIADKTGAGERGARGIVALLGPNNKAERIVVIYLRDTPASMAERNQQIAGIG
AALIEHWQR
**SEQ ID N° 531 :** 
MRYIRLCIISLLATLPLAVHASPQPLEQIKLSESQLSGRVGMIEMDLASGRTLTAWRA
DERFPMMSTFKVVLCGAVLARVDAGDEQLERKIHYRQQDLVDYSPVSEKHLADGM
TVGELCAAAITMSDNSAANLLLATVGGPAGLTAFLRQIGDNVTRLDRWETELNEAFP
GDARDTTTPASMAATLRKLLTSQRLSARSQRQLLQWMVDDRVAGPLIRSVLPAGW
FIADKTGAGERGARGIVALLGPNNKAERIVVIYLRDTPASMAERNQQIAGIGAALIEH
WQR
**SEQ ID N° 532 :** 
MRYIRLCIISLLATLPLAVHASPQPLEQIKLSESQLSGRVGMIEMDLASGRTLTAWRA
DERFPMMSTFKVVLCGAVLARVDAGDEQLERKIHYRQQDLVDYSPVSEKHLADGM
TVGELCAAAITMSDNSAANLLLATVGGPAGLTAFLRQIGDNVTRLDRWETELNEAFP
GDARDTTTPASMAATLRKLLTSQRLSARSQRQLLQWMVDDRVAGPLIRSVLPAGW
FIADKTGASERGARGIVALLGPNNKAERIVVIYLRDTPASMAERNQQIAGIGAALIEH
WQR
**SEQ ID N° 533 :** 
MRYIRLCIISLLATLPLAVHASPQPLEQIKLSESQLSGRVGMIEMDLASGRTLTAWRA
DERFPMMSTFKVVLCGAVLARVDAGDEQLERKIHYRQQDLVDYSPVSEKHLADGM
TVGELCAAAITMSDNSAANLLLATVGGPAGLTAFLRQIGDNVTRLDRWETELNEALP
GDARATTTPASMAATLRKLLTSQRLSARSQRQLLQWMVDDRVAGPLIRSVLPAGW
FIADKTGAGERGARGIVALLGPNNKAERIVVIYLRDTPASMAERNQQIAGIGAALIEH
WQR
**SEQ ID N° 534 :** 
MRYIRLCIISLLATLPLAVHASPQPLEQIKLSESQLSGRVGMIEMDLASGRTLTAWRA
DERFPMMSTFKVVLCGAVLARVDAGDEQLERKIHYRQQDLVDYSPVSEKHLADGM
TVGELCAAAITMSDNSAANLLLATVGGPAGLTAFLRQIGDNVTRLDRWETELNEALP
GDARDTTTPASMAATLRKLLNSQRLSARSQRQLLQWMVDDRVAGPLIRSVLPAGW
FIADKTGASKRGARGIVALLGPNNKAERIVVIYLRDTPASMAERNQQIAGIGAALIEH
WQR
**SEQ ID N° 535 :** 
MRYIRLCIISLLATLPLAVHASPQPLEQIKLSESQLSGRVGMIEMDLASGRTLTAWRA
DERFPMMSTFKVVLCGAVLARVDAGDEQLERKIHYRQQDLVDYSPVSEKHLADGM
TVGELCAAAITMSDNSAANLLLATVGGPAGLTAFLRQIGDNVTRLDRWETELNEALP
GDARDTTTPASMAATLRKLLTSQRLSARLQRQLLQWMVDDRVAGPLIRSVLPAGW
 
FIADKTGAGERGARGIVALLGPNNKAERIVVIYLRDTPASMAERNQQIAGIGAALIEH
WQR
**SEQ ID N° 536 :** 
MRYIRLCIISLLATLPLAVHASPQPLEQIKLSESQLSGRVGMIEMDLASGRTLTAWRA
DERFPMMSTFKVVLCGAVLARVDAGDEQLERKIHYRQQDLVDYSPVSEKHLADGM
TVGELCAAAITMSDNSAANLLLATVGGPAGLTAFLRQIGDNVTRLDRWETELNEALP
GDARDTTTPASMAATLRKLLTSQRLSARSQLQLLQWMVDDRVAGPLIRSVLPAGW
FIADKTGAGERGARGIVALLGPNNKAERIVVIYLRDTPASMAERNQQIAGIGAALIEH
WQR
**SEQ ID N° 537 :** 
MRYIRLCIISLLATLPLAVHASPQPLEQIKLSESQLSGRVGMIEMDLASGRTLTAWRA
DERFPMMSTFKVVLCGAVLARVDAGDEQLERKIHYRQQDLVDYSPVSEKHLADGM
TVGELCAAAITMSDNSAANLLLATVGGPAGLTAFLRQIGDNVTRLDRWETELNEALP
GDARDTTTPASMAATLRKLLTSQRLSARSQLQLLQWMVDDRVAGPLIRSVLPAGW
FIADKTGASERGARGIVALLGPNNKAERIVVIYLRDTPASMAERNQQIAGIGAALIEH
WQR
**SEQ ID N° 538 :** 
MRYIRLCIISLLATLPLAVHASPQPLEQIKLSESQLSGRVGMIEMDLASGRTLTAWRA
DERFPMMSTFKVVLCGAVLARVDAGDEQLERKIHYRQQDLVDYSPVSEKHLADGM
TVGELCAAAITMSDNSAANLLLATVGGPAGLTAFLRQIGDNVTRLDRWETELNEALP
GDARDTTTPASMAATLRKLLTSQRLSARSQLQLLQWMVDDRVAGPLIRSVLPAGW
FIADKTGASKRGARGIVALLGPNNKAERIVVIYLRDTPASMAERNQQIAGIGAALIEH
WQR
**SEQ ID N° 539 :** 
MRYIRLCIISLLATLPLAVHASPQPLEQIKLSESQLSGRVGMIEMDLASGRTLTAWRA
DERFPMMSTFKVVLCGAVLARVDAGDEQLERKIHYRQQDLVDYSPVSEKHLADGM
TVGELCAAAITMSDNSAANLLLATVGGPAGLTAFLRQIGDNVTRLDRWETELNEALP
GDARDTTTPASMAATLRKLLTSQRLSARSQRQLLQWMVDDGVAGPLIRSVLPAGW
FIADKTGAGERGARGIVALLGPNNKAERIVVIYLRDTPASMAERNQQIAGIGAALIEH
WQR
**SEQ ID N° 540** : 
MRYIRLCIISLLATLPLAVHASPQPLEQIKLSESQLSGRVGMIEMDLASGRTLTAWRA
DERFPMMSTFKVVLCGAVLARVDAGDEQLERKIHYRQQDLVDYSPVSEKHLADGM
TVGELCAAAITMSDNSAANLLLATVGGPAGLTAFLRQIGDNVTRLDRWETELNEALP
GDARDTTTPASMAATLRKLLTSQRLSARSQRQLLQWMVDDRVAGPLIRSVLPAGW
FIADKTGAAERGARGIVALLGPNNKAERIVVIYLRDTPASMAERNQQIAGIGAALIEH
WQR
**SEQ ID N° 541 :** 
MRYIRLCIISLLATLPLAVHASPQPLEQIKLSESQLSGRVGMIEMDLASGRTLTAWRA
DERFPMMSTFKVVLCGAVLARVDAGDEQLERKIHYRQQDLVDYSPVSEKHLADGM
TVGELCAAAITMSDNSAANLLLATVGGPAGLTAFLRQIGDNVTRLDRWETELNEALP
GDARDTTTPASMAATLRKLLTSQRLSARSQRQLLQWMVDDRVAGPLIRSVLPAGW
FIADKTGAGERGARGIVALLGPDNKAERIVVIYLRDTPASMAERNQQIAGIGAALIEH
WQR
**SEQ ID N° 542** : 
MRYIRLCIISLLATLPLAVHASPQPLEQIKLSESQLSGRVGMIEMDLASGRTLTAWRA
DERFPMMSTFKVVLCGAVLARVDAGDEQLERKIHYRQQDLVDYSPVSEKHLADGM
TVGELCAAAITMSDNSAANLLLATVGGPAGLTAFLRQIGDNVTRLDRWETELNEALP
GDARDTTTPASMAATLRKLLTSQRLSARSQRQLLQWMVDDRVAGPLIRSVLPAGW
 
FIADKTGAGERGARGIVALLGPNHKAERIVVIYLRDTPASMAERNQQIAGIGAALIEH
WQR
**SEQ ID N° 543 :** 
MRYIRLCIISLLATLPLAVHASPQPLEQIKLSESQLSGRVGMIEMDLASGRTLTAWRA
DERFPMMSTFKVVLCGAVLARVDAGDEQLERKIHYRQQDLVDYSPVSEKHLADGM
TVGELCAAAITMSDNSAANLLLATVGGPAGLTAFLRQIGDNVTRLDRWETELNEALP
GDARDTTTPASMAATLRKLLTSQRLSARSQRQLLQWMVDDRVAGPLIRSVLPAGW
FIADKTGAGERGARGIVALLGPNNKAERIVVIYLRDTPASMAERNQQIAGIGAALIEH
WQR
**SEQ ID N° 544 :** 
MRYIRLCIISLLATLPLAVHASPQPLEQIKLSESQLSGRVGMIEMDLASGRTLTAWRA
DERFPMMSTFKVVLCGAVLARVDAGDEQLERKIHYRQQDLVDYSPVSEKHLADGM
TVGELCAAAITMSDNSAANLLLATVGGPAGLTAFLRQIGDNVTRLDRWETELNEALP
GDARDTTTPASMAATLRKLLTSQRLSARSQRQLLQWMVDDRVAGPLIRSVLPAGW
FIADKTGAGERGARGIVALRGPNNKAERIVVIYLRDTPASMAERNQQIAGIGAALIEH
WQR
**SEQ ID N° 545 :** 
MRYIRLCIISLLATLPLAVHASPQPLEQIKLSESQLSGRVGMIEMDLASGRTLTAWRA
DERFPMMSTFKVVLCGAVLARVDAGDEQLERKIHYRQQDLVDYSPVSEKHLADGM
TVGELCAAAITMSDNSAANLLLATVGGPAGLTAFLRQIGDNVTRLDRWETELNEALP
GDARDTTTPASMAATLRKLLTSQRLSARSQRQLLQWMVDDRVAGPLIRSVLPAGW
FIADKTGAGKRGARGIVALLGPNNKAERIVVIYLRDSPASMAERNQQIAGIGAALIEH
WQR
**SEQ ID N° 546 :** 
MRYIRLCIISLLATLPLAVHASPQPLEQIKLSESQLSGRVGMIEMDLASGRTLTAWRA
DERFPMMSTFKVVLCGAVLARVDAGDEQLERKIHYRQQDLVDYSPVSEKHLADGM
TVGELCAAAITMSDNSAANLLLATVGGPAGLTAFLRQIGDNVTRLDRWETELNEALP
GDARDTTTPASMAATLRKLLTSQRLSARSQRQLLQWMVDDRVAGPLIRSVLPAGW
FIADKTGAGKRGARGIVALLGPNNKAERTVVIYLRDTPASMAERNQQIAGIGAALIEH
WQR
**SEQ ID N° 547 :** 
MRYIRLCIISLLATLPLAVHASPQPLEQIKLSESQLSGRVGMIEMDLASGRTLTAWRA
DERFPMMSTFKVVLCGAVLARVDAGDEQLERKIHYRQQDLVDYSPVSEKHLADGM
TVGELCAAAITMSDNSAANLLLATVGGPAGLTAFLRQIGDNVTRLDRWETELNEALP
GDARDTTTPASMAATLRKLLTSQRLSARSQRQLLQWMVDDRVAGPLIRSVLPAGW
FIADKTGASERGARGIVALLGPNNKAERIVVIYLRDSPASMAERNQQIAGIGAALIEH
WQR
 
SEQ ID N° 548 :
MRYIRLCIISLLATLPLAVHASPQPLEQIKLSESQLSGRVGMIEMDLASGRTLTAWRA
DERFPMMSTFKVVLCGAVLARVDAGDEQLERKIHYRQQDLVDYSPVSEKHLADGM
TVGELCAAAITMSDNSAANLLLATVGGPAGLTAFLRQIGDNVTRLDRWETELNEALP
GDARDTTTPASMAATLRKLLTSQRLSARSQRQLLQWMVDDRVAGPLIRSVLPAGW
FIADKTGASERGARGIVALLGPNNKAERIVVIYLRDTPASMAERNQQIAGIGAALIEH
WQR
**SEQ** ID **N°** 549 : 
MRYIRLCIISLLATLPLAVHASPQPLEQIKLSESQLSGRVGMIEMDLASGRTLTAWRA
DERFPMMSTFKVVLCGAVLARVDAGDEQLERKIHYRQQDLVDYSPVSEKHLADGM
TVGELCAAAITMSDNSAANLLLATVGGPAGLTAFLRQIGDNVTRLDRWETELNEALP
GDARDTTTPASMAATLRKLLTSQRLSARSQRQLLQWMVDDRVAGPLIRSVLPAGW
 
FIADKTGASKRGARGIVALLGPNNKAERIVVIYLRDTPASMAERNQQIAGIGAALIEH
WQR
**SEQ ID N° 550 :** 
MRYIRLCIISLLATLPLAVHASPQPLEQIKLSESQLSGRVGMIEMDLASGRTLTAWRA
DERFPMMSTFKVVLCGAVLARVDAGDEQLERKIHYRQQDLVDYSPVSEKHLADGM
TVGELCAAAITMSDNSAANLLLATVGGPAGLTAFLRQIGDNVTRLDRWETELNEALP
GDARDTTTPASMAATLRKLLTSQRLSARSQRQLLQWMVDDRVAGPLIRSVLPAGW
FIADKTGASKRGARGIVALLGPNNKAERIVVIYLRDTPASMAERNQQIAGLGAALIEH
WQR
**SEQ ID N°** 551 : 
MRYIRLCIISLLATLPLAVHASPQPLEQIKLSESQLSGRVGMIEMDLASGRTLTAWRA
DERFPMMSTFKVVLCGAVLARVDAGDEQLERKIHYRQQDLVDYSPVSEKHLADGM
TVGELCAAAITMSDNSAANLLLATVGGPAGLTAFLRQIGDNVTRLDRWETELNEALP
GDARDTTTPASMAATLRKLLTSQRLSARSQRQLLQWMVDDRVAGPLIRSVLPAGW
FIADRTGAGERGARGIVALLGPNNKAERIVVIYLRDTPASMAERNQQIAGIGAALIEH
WQR
**SEQ ID N° 552 :** 
MRYIRLCIISLLATLPLAVHASPQPLEQIKLSESQLSGRVGMIEMDLASGRTLTAWRA
DERFPMMSTFKVVLCGAVLARVDAGDEQLERKIHYRQQDLVDYSPVSEKHLADGM
TVGELCAAAITMSDNSAANLLLATVGGPAGLTAFLRQIGDNVTRLDRWETELNEALP
GDARDTTTPASMAATLRKLLTSQRLSARSQRQLLQWMVDDRVAGPLIRSVLSAGW
FIADKTGAGERGARGIVALLGPNNKAERIVVIYLRDTPASMAERNQQIAGIGAALIEH
WQR
**SEQ ID N° 553 :** 
MRYIRLCIISLLATLPLAVHASPQPLEQIKLSESQLSGRVGMIEMDLASGRTLTAWRA
DERFPMMSTFKVVLCGAVLARVDAGDEQLERKIHYRQQDLVDYSPVSEKHLADGM
TVGELCAAAITMSDNSAANLLLATVGGPAGLTAFLRQIGDNVTRLDRWETELNEALP
GDARDTTTPASMAATLRKLLTSQRLSARSQRQLLQWMVDGRVAGPLIRSVLPAGW
FIADKTGAGERGARGIVALLGPNNKAERIVVIYLRDTPASMAERNQQIAGIGAALIEH
WQR
**SEQ ID N° 554 :** 
MRYIRLCIISLLATLPLAVHASPQPLEQIKLSESQLSGRVGMIEMDLASGRTLTAWRA
DERFPMMSTFKVVLCGAVLARVDAGDEQLERKIHYRQQDLVDYSPVSEKHLADGM
TVGELCAAAITMSDNSAANLLLATVGGPAGLTAFLRQIGDNVTRLDRWETELNEALP
GDARDTTTPASMTATLRKLLTSQRLSARSQRHLLQWMVDDRVAGPLIRSVLPAGW
FIADKTGAGERGARGIVALLGPNNKAERIVVIYLRDTPASMAERNQQIAGIGAALIEH
WQR
**SEQ ID N° 555 :** 
MRYIRLCIISLLATLPLAVHASPQPLEQIKLSESQLSGRVGMIEMDLASGRTLTAWRA
DERFPMMSTFKVVLCGAVLARVDAGDEQLERKIHYRQQDLVDYSPVSEKHLADGM
TVGELCAAAITMSDNSAANLLLATVGGPAGLTAFLRQIGDNVTRLDRWETELNEALP
GDARDTTTPASMTATLRKLLTSQRLSARSQRQLLQWMVDDRVAGPLIRSVLPAGW
FIADKTGAGERGARGIVALLGPNNKAERIVVIYLRDTPASMAERNQQIAGIGAALIEH
WQR
**SEQ ID N° 556 :** 
MRYIRLCIISLLATLPLAVHASPQPLEQIKLSESQLSGRVGMIEMDLASGRTLTAWRA
DERFPMMSTFKVVLCGAVLARVDAGDEQLERKIHYRQQDLVDYSPVSEKHLADGM
TVGELCAAAITMSDNSAANLLLATVGGPAGLTAFLRQIGDNVTRLDRWETELNEALP
GDARDTTTPASMTATLRKLLTSQRLSARSQRQLLQWMVDDRVAGPLIRSVLPAGW
 
FIADKTGAGKRGARGIVALLGPNNKAERIVVIYLRDTPASMAERNQQIAGIGAALIEH
WQR
**SEQ ID N° 557 :** 
MRYIRLCIISLLATLPLAVHASPQPLEQIKLSESQLSGRVGMIEMDLASGRTLTAWRA
DERFPMMSTFKVVLCGAVLARVDAGDEQLERKIHYRQQDLVDYSPVSEKHLADGM
TVGELCAAAITMSDNSAANLLLATVGGPAGLTAFLRQIGDNVTRLDRWETELNEALP
GDARDTTTPASMTATLRKLLTSQRLSARSQRQLLQWMVDDRVAGPLIRSVLPAGW
FIADKTGASKRGARGIVALLGPNNKAERIVVIYLRDTPASMAERNQQIAGIGAALIEH
WQR
**SEQ ID N° 558 :** 
MRYIRLCIISLLATLPLAVHASPQPLEQIKLSESQLSGRVGMIEMDLASGRTLTAWRA
DERFPMMSTFKVVLCGAVLARVDAGDEQLERKIHYRQQDLVDYSPVSEKHLADGM
TVGELCAAAITMSDNSAANLLLATVGGPAGLTAFLRQIGDNVTRLDRWETELNEALP
GDARGTTTPASMAATLRKLLTSQRLSARSQRQLLQWMVDDRVAGPLIRSVLPAGW
FIADKTGAGERGARGIVALLGPNNKAERIVVIYLRDTPASMAERNQQIAGIGAALIEH
WQR
**SEQ ID N° 559 :** 
MRYIRLCIISLLATLPLAVHASPQPLEQIKLSESQLSGRVGMIEMDLASGRTLTAWRA
DERFPMMSTFKVVLCGAVLARVDAGDEQLERKIHYRQQDLVDYSPVSEKHLADGM
TVGELCAAAITMSDNSAANLLLATVGGPAGLTAFLRQIGDNVTRLDRWETELNEALP
GDARNTTTPASMAATLRKLLTSQRLSARSQRQLLQWMVDDRVAGPLIRSVLPAGW
FIADKTGAGERGARGIVALLGPNNKAERIVVIYLRDTPASMAERNQQIAGIGAALIEH
WQR
**SEQ ID N° 560 :** 
MRYIRLCIISLLATLPLAVHASPQPLEQIKLSESQLSGRVGMIEMDLASGRTLTAWRA
DERFPMMSTFKVVLCGAVLARVDAGDEQLERKIHYRQQDLVDYSPVSEKHLADGM
TVGELCAAAITMSDNSAANLLLATVGGPAGLTAFLRQIGDNVTRLDRWETELNEALS
GDARDTTTPASMAATLRKLLTSQRLSARSQRQLLQWMVDDRVAGPLIRSVLPAGW
FIADKTGAGERGARGIVALLGPNNKAERIVVIYLRDTPASMAERNQQIAGIGAALIEH
WQR
**SEQ ID N° 561 :** 
MRYIRLCIISLLATLPLAVHASPQPLEQIKLSESQLSGRVGMIEMDLASGRTLTAWRA
DERFPMMSTFKVVLCGAVLARVDAGDEQLERKIHYRQQDLVDYSPVSEKHLADGM
TVGELCAAAITMSDNSAANLLLATVGGPAGLTAFLRQIGDNVTRLDRWETERNEALP
GDARDTTTPASMAATLRKLLTSQRLSARSQRQLLQWMVDDRVAGPLIRSVLPAGW
FIADKTGAGERGARGIVALLGPNNKAERIVVIYLRDTPASMAERNQQIAGIGAALIEH
WQR
**SEQ ID N° 562 :** 
MRYIRLCIISLLATLPLAVHASPQPLEQIKLSESQLSGRVGMIEMDLASGRTLTAWRA
DERFPMMSTFKVVLCGAVLARVDAGDEQLERKIHYRQQDLVDYSPVSEKHLADGM
TVGELCAAAITMSDNSAANLLLATVGGPAGLTAFLRQIGENVTRLDRWETELNEALP
GDARDTTTPASMAATLRKLLTNQRLSARSQRQLLQWMVDDRVAGPLIRSVLPAGW
FIADKTGAGERGARGIVALLGPNNKAERIVVIYLRDTPASMAERNQQIAGIGAALIEH
WQR
**SEQ ID N° 563 :** 
MRYIRLCIISLLATLPLAVHASPQPLEQIKLSESQLSGRVGMIEMDLASGRTLTAWRA
DERFPMMSTFKVVLCGAVLARVDAGDEQLERKIHYRQQDLVDYSPVSEKHLADGM
TVGELCAAAITMSDNSAANLLLATVGGPVGLTAFLRQIGDNVTRLDRWETELNEALP
GDARDTTTPASMAATLRKLLTSQRLSARSQRQLLQWMVDDRVAGPLIRSVLPAGW
 
FIADKTGAGERGARGIVALLGPNNKAERIVVIYLRDTPASMAERNQQIAGIGAALIEH
WQR
**SEQ ID N° 564 :** 
MRYIRLCIISLLATLPLAVHASPQPLEQIKLSESQLSGRVGMIEMDLASGRTLTAWRA
DERFPMMSTFKVVLCGAVLARVDAGDEQLERKIHYRQQDLVDYSPVSEKHLADGM
TVGELCAAAITMSDNSVANLLLATVGGPAGLTAFLRQIGDNVTRLDRWETELNEALP
GDARDTTTPASMAATLRKLLTSQRLSARSQRQLLQWMVDDRVAGPLIRSVLPAGW
FIADKTGAGERGARGIVALLGPNNKAERIVVIYLRDTLASMAERNQQIAGIGAALIEH
WQR
**SEQ ID N° 565 :** 
MRYIRLCIISLLATLPLAVHASPQPLEQIKLSESQLSGRVGMIEMDLASGRTLTAWRA
DERFPMMSTFKVVLCGAVLARVDAGDEQLERKIHYRQQDLVDYSPVSEKHLADGM
TVGELCAAVITMSDNSAANLLLATVGGPAGLTAFLRQIDDNVTRLDRWETELNEALP
GDARDTTTPASMAATLRKLLTSQRLSARSQRQLLQWMVDDRVAGPLIRSVLPAGW
FIADKTGAGERGARGIVALLGPNNKAERIVVIYLRDTPASMAERNQQIAGIGAALIEH
WQR
**SEQ ID N° 566 :** 
MRYIRLCIISLLATLPLAVHASPQPLEQIKLSESQLSGRVGMIEMDLASGRTLTAWRA
DERFPMMSTFKVVLCGAVLARVDAGDEQLERKIHYRQQDLVDYSPVSEKHLADGM
TVGELCTAAITMSDNSAANLLLATVGGPAGLTAFLRQIGDNVTRLDRWETELNEALP
GDARDTTTPASMAATLRKLLTSQRLSARSQRQLLQWMVDDRVAGPLIRSVLPAGW
FIADKTGAGERGARGIVALLGPNNKAERIVVIYLRDTPASMAERNQQIAGIGAALIEH
WQR
**SEQ ID N° 567 :** 
MRYIRLCIISLLATLPLAVHASPQPLEQIKLSESQLSGRVGMIEMDLASGRTLTAWRA
DERFPMMSTFKVVLCGAVLARVDAGDEQLERKIHYRQQDLVDYSPVSEKYLADGM
TVGELCAAAITMSDNSAANLLLATVGGPVGLTAFLRQIGDNVTRLDRWETELNEALP
GDARDTTTPASMAATLRKLLTSQRLSARSQRQLLQWMVDDRVAGPLIRSVLPAGW
FIADKTGAGERGARGIVALLGPNNKAERIVVIYLRDTPASMAERNQQIAGIGAALIEH
WQR
**SEQ ID N° 568 :** 
MRYIRLCIISLLATLPLAVHASPQPLEQIKLSESQLSGRVGMIEMDLASRTLTAWRAD
ERFPMMSTFKVVLCGAVLARVDAGDEQLERKIHYRQQDLVDYSPVSEKHLADGMT
VGELCAAAITMGDNSAANLLLRTVGGPAGLTAFLRQIGDNVTRLDRWETELNEALP
GDARDTTTPASMAATLRNVLTSQRLSARSQRQLLQWMVDDRVAGPLIRSVLPAGW
FIADKTGASKRGARGIVALLGPNNKAERIVVIYLRDTPASMAERNQQIAGIGAALIEH
WQR
**SEQ ID N° 569 :** 
MRYIRLCIISLLATLPLAVHASPQPLEQIKLSESQLSGRVGMIEMDLASRTLTAWRAD
ERFPMMSTFKVVLCGAVLARVDAGDEQLERKIHYRQQDLVDYSPVSEKHLADGMT
VGELCAAAITMSDNSAANLLLRTVGGPAGLTAFLRQIGDNVTRLDRWETELNEALPG
DARDTTTPASMAATLRNVLTSQRLSARSQRQLLQWMVDDRVAGPLIRSVLPAGWFI
ADKTGASKRGARGIVALLGPNNKAERIVVIYLRDTPASMAERNQQIAGIGAALIEHW
QR
**SEQ ID N° 570 :** 
MRYIRLCIISLLATLPLAVHASPQPLEQIKLSESQLSGRVGMIEMDLASSRTLTAWRA
DERFPMMSTFKVVLCGAVLARVDAGDEQLERKIHYRQQDLVDYSPVSEKHLADGM
TVGELCAAAITMSDNSAANLLLATVGGPAGLTAFLRQIGDNVTRLDRWETELNEALP
GDARDTTTPASMAATLRKLLTSQRLSARSQRQLLQWMVDDRVAGPLIRSVLPAGW
 
FIADKTGAGERGARGIVALLGPNNKAERIVVIYLRDTPASMAERNQQIAGIGAALIEH
WQR
**SEQ ID N° 571 :** 
MRYIRLCIISLLATLPLAVHASPQPLEQIKQSESQLSGRVGMIEMDLASGRTLTAWCA
DERFPMMSTFKVVLCGAVLARVDAGDEQLERKIHYRQQDLVDYSPVSEKHLADGM
TVGELCAAAITMSDNSAANLLLATVGGPAGLTAFLRQIGDNVTRLDRWETELNEALP
GDARDTTTPASMAATLRKLLTSQRLSARSQRQLLQWMVDDRVAGPLIRSVLPAGW
FIADKTGAGERGARGIVALLGPNNKAERIVVIYLRDTPASMAERNQQIAGIGAALIEH
WQR
**SEQ ID N° 572 :** 
MRYIRLCIISLLATLPLAVHASPQPLEQIKQSESQLSGRVGMIEMDLASGRTLTAWRA
DERFPMISTFKVVLCGAVLARVDAGDEQLERKIHYRQQDLVDYSPVSEKHLADGMT
VGELCAAAITMSDNSAANLLLAIVGGPAGLTAFLRQIGDNVTRLDRWETELNEALPG
DARDTTTPASMAATLRKLLTSQRLSARSQRQLLQWMVDDRVAGPLIRSVLPAGWFI
ADKTGAGERGARGIVALLGPNNKAERIVVIYLRDTPASMAERNQQIAGIGAALIEHW
QR
**SEQ ID N° 573 :** 
MRYIRLCIISLLATLPLAVHASPQPLEQIKQSESQLSGRVGMIEMDLASGRTLTAWRA
DERFPMMSTFKVALCGAVLARVDAGDEQLERKIHYRQQDLVDYSPVSEKHLADGM
TVGELCAAAITMSDNSAANLLLATVGGPAGLTAFLRQIGDNVTRLDRWETELNEALP
GDARDTTTPASMAATLRKLLTSQRLSARSQRQLLQWMVDDRVAGPLIRSVLPAGW
FIADKTGAGERGARGIVALLGPNNKAERIVVIYLRDTPASMAERNQQIAGIGAALIEH
WQR
**SEQ ID N° 574** : 
MRYIRLCIISLLATLPLAVHASPQPLEQIKQSESQLSGRVGMIEMDLASGRTLTAWRA
DERFPMMSTFKVLLCGAVLARVDAGDEQLERKIHYRQQDLVDYSPVSEKHLADGM
TVGELCAAAITMSDNSAANLLLATVGGPAGLTAFLRQIGDNVTRLDRWETELNEALP
GDARDTTTPASMAATLRKLLTSQRLSARSQRQLLQWMVDDRVAGPLIRSVLPAGW
FIADKTGASKRGARGIVALLGPNNKAERIVVIYLRDTPASMAERNQQIAGIGAALIEH
WQR
**SEQ ID N° 575 :** 
MRYIRLCIISLLATLPLAVHASPQPLEQIKQSESQLSGRVGMIEMDLASGRTLTAWRA
DERFPMMSTFKVVLCGAMLARVDAGDKQLERKIHYRQQDLVDYSPVSEKHLADGM
TVGELCAAAITMSDNSAANLLLATVGGPAGLTAFLRQIGDNVTRLDRWETELNEALP
GDARDTTTPASMAATLRKLLTSQRLSARSQRQLLQWMVDDRVAGPLIRSVLPAGW
FIADKTGASKRGARGIVALLGPNNKAERIVVIYLRDTPASMAERNQQIAGIGAALIEH
WQR
**SEQ ID N° 576 :** 
MRYIRLCIISLLATLPLAVHASPQPLEQIKQSESQLSGRVGMIEMDLASGRTLTAWRA
DERFPMMSTFKVVLCGAVLARVDAGDEQLERKIHYRQQDLVDYSPVSEKHLADGM
TVGELCAAAITMSDNSAANLLLAIVGGPAGLTAFLRQIGDNVTRLDRWETELNEALP
GDARDTTTPASMAATLRKLLTSQRLSARSQRQLLQWMVDDRVAGPLIRSVLPAGW
FIADKTGAGERGARGIVALLGPNNKAERIVVIYLRDTPASMAERNQQIAGIGAALIEH
WQR
**SEQ ID N° 577 :** 
MRYIRLCIISLLATLPLAVHASPQPLEQIKQSESQLSGRVGMIEMDLASGRTLTAWRA
DERFPMMSTFKVVLCGAVLARVDAGDEQLERKIHYRQQDLVDYSPVSEKHLADGM
TVGELCAAAITMSDNSAANLLLATVGGPAGLTAFLRQIDDNVTRLDRWETELNEALP
GDARDTTTPASMAATLRKLLTSQRLSARSQRQLLQWMVDDRVAGPLIRSVLPAGW
 
FIADKTGAGERGARGIVALLGPNNKAERIVVIYLRDTPASMAERNQQIAGIGAALIEH
WQR
**SEQ ID N° 578 :** 
MRYIRLCIISLLATLPLAVHASPQPLEQIKQSESQLSGRVGMIEMDLASGRTLTAWRA
DERFPMMSTFKVVLCGAVLARVDAGDEQLERKIHYRQQDLVDYSPVSEKHLADGM
TVGELCAAAITMSDNSAANLLLATVGGPAGLTAFLRQIGDNVTRLDRWETELNEALP
GDARDTTTPASMAATLRKLLISQRLSARSQRQLLQWMVDDRVAGPLIRSVLPAGWF
IADKTGAGERGARGIVALLGPNNKAERIVVIYLRDTPASMAERNQQIAGIGAALIEHW
QR
**SEQ ID N° 579 :** 
MRYIRLCIISLLATLPLAVHASPQPLEQIKQSESQLSGRVGMIEMDLASGRTLTAWRA
DERFPMMSTFKVVLCGAVLARVDAGDEQLERKIHYRQQDLVDYSPVSEKHLADGM
TVGELCAAAITMSDNSAANLLLATVGGPAGLTAFLRQIGDNVTRLDRWETELNEALP
GDARDTTTPASMAATLRKLLTSQRLSARSQRQLLQWMVDARVAGPLIRSVLPAGW
FIADKTGAGERGARGIVALLGPNNKAERIVVIYLRDTPASMAERNQQIAGIGAALIEH
WQR
**SEQ ID N° 580 :** 
MRYIRLCIISLLATLPLAVHASPQPLEQIKQSESQLSGRVGMIEMDLASGRTLTAWRA
DERFPMMSTFKVVLCGAVLARVDAGDEQLERKIHYRQQDLVDYSPVSEKHLADGM
TVGELCAAAITMSDNSAANLLLATVGGPAGLTAFLRQIGDNVTRLDRWETELNEALP
GDARDTTTPASMAATLRKLLTSQRLSARSQRQLLQWMVDDRVAGPLIRSVLPAGW
FIADKAGAGERGARGIVALLGPNNKAERIVVIYLRDTPASMAERNQQIAGIGAALIEH
WQR
**SEQ ID N° 581 :** 
MRYIRLCIISLLATLPLAVHASPQPLEQIKQSESQLSGRVGMIEMDLASGRTLTAWRA
DERFPMMSTFKVVLCGAVLARVDAGDEQLERKIHYRQQDLVDYSPVSEKHLADGM
TVGELCAAAITMSDNSAANLLLATVGGPAGLTAFLRQIGDNVTRLDRWETELNEALP
GDARDTTTPASMAATLRKLLTSQRLSARSQRQLLQWMVDDRVAGPLIRSVLPAGW
FIADKTGAAERGARGIVALLGPNNKAERIVVIYLRDTPASMAERNQQIAGIGAALIEH
WQR
**SEQ ID N° 582 :** 
MRYIRLCIISLLATLPLAVHASPQPLEQIKQSESQLSGRVGMIEMDLASGRTLTAWRA
DERFPMMSTFKVVLCGAVLARVDAGDEQLERKIHYRQQDLVDYSPVSEKHLADGM
TVGELCAAAITMSDNSAANLLLATVGGPAGLTAFLRQIGDNVTRLDRWETELNEALP
GDARDTTTPASMAATLRKLLTSQRLSARSQRQLLQWMVDDRVAGPLIRSVLPAGW
FIADKTGAGERGARGIVALLGPNNKAERIVVIYLRDTPASMAERNQQIAGIGAALIEH
WQR
**SEQ ID N° 583 :** 
MRYIRLCIISLLATLPLAVHASPQPLEQIKQSESQLSGRVGMIEMDLASGRTLTAWRA
DERFPMMSTFKVVLCGAVLARVDAGDEQLERKIHYRQQDLVDYSPVSEKHLADGM
TVGELCAAAITMSDNSAANLLLATVGGPAGLTAFLRQIGDNVTRLDRWETELNEALP
GDARDTTTPASMAATLRKLLTSQRLSARSQRQLLQWMVDDRVAGPLIRSVLPAGW
FIADKTGAGERGGRGIVALLGPNNKAERIVVIYLRDTPASMAERNQQIAGIGAALIEH
WQR
**SEQ ID N° 584 :** 
MRYIRLCIISLLATLPLAVHASPQPLEQIKQSESQLSGRVGMIEMDLASGRTLTAWRA
DERFPMMSTFKVVLCGAVLARVDAGDEQLERKIHYRQQDLVDYSPVSEKHLADGM
TVGELCAAAITMSDNSAANLLLATVGGPAGLTAFLRQIGDNVTRLDRWETELNEALP
GDARDTTTPASMAATLRKLLTSQRLSARSQRQLLQWMVDDRVAGPLIRSVLPAGW
 
FIADKTGAGKRGARGIVALLGPNNKAERIVVIYLRDTPASMAERNQQIAGIGAALIEH
WQR
**SEQ ID N° 585 :** 
MRYIRLCIISLLATLPLAVHASPQPLEQIKQSESQLSGRVGMIEMDLASGRTLTAWRA
DERFPMMSTFKVVLCGAVLARVDAGDEQLERKIHYRQQDLVDYSPVSEKHLADGM
TVGELCAAAITMSDNSAANLLLATVGGPAGLTAFLRQIGDNVTRLDRWETELNEALP
GDARDTTTPASMAATLRKLLTSQRLSARSQRQLLQWMVDDRVAGPLIRSVLPAGW
FIADKTGASERGARGIVALLGPNNKAERIVVIYLRDTPASMAERNQQIAGIGAALIEH
WQR
**SEQ ID N° 586 :** 
MRYIRLCIISLLATLPLAVHASPQPLEQIKQSESQLSGRVGMIEMDLASGRTLTAWRA
DERFPMMSTFKVVLCGAVLARVDAGDEQLERKIHYRQQDLVDYSPVSEKHLADGM
TVGELCAAAITMSDNSAANLLLATVGGPAGLTAFLRQIGDNVTRLDRWETELNEALP
GDARDTTTPASMAATLRKLLTSQRLSARSQRQLLQWMVDDRVAGPLIRSVLPAGW
FIADKTGASKRGARGIVALLGGNIKAERIVVIYLRDTPASMAERNQQIAGIGAALIEHW
QR
**SEQ ID N° 587 :** 
MRYIRLCIISLLATLPLAVHASPQPLEQIKQSESQLSGRVGMIEMDLASGRTLTAWRA
DERFPMMSTFKVVLCGAVLARVDAGDEQLERKIHYRQQDLVDYSPVSEKHLADGM
TVGELCAAAITMSDNSAANLLLATVGGPAGLTAFLRQIGDNVTRLDRWETELNEALP
GDARDTTTPASMAATLRKLLTSQRLSARSQRQLLQWMVDDRVAGPLIRSVLPAGW
FIADKTGASKRGARGIVALLGPNNKAERIVVIYLRDTPASMAELDQQIAGIGAALIEHW
QR
**SEQ ID N° 588 :** 
MRYIRLCIISLLATLPLAVHASPQPLEQIKQSESQLSGRVGMIEMDLASGRTLTAWRA
DERFPMMSTFKVVLCGAVLARVDAGDEQLERKIHYRQQDLVDYSPVSEKHLADGM
TVGELCAAAITMSDNSAANLLLATVGGPAGLTAFLRQIGDNVTRLDRWETELNEALP
GDARDTTTPASMAATLRKLLTSQRLSARSQRQLLQWMVDDRVAGPLIRSVLPAGW
FIADKTGASKRGARGIVALLGPNNKAERIVVIYLRDTPASMAERNQQIAGIGAALIEH
WQR
**SEQ ID N° 589 :** 
MRYIRLCIISLLATLPLAVHASPQPLEQIKQSESQLSGRVGMIEMDLASGRTLTAWRA
DERFPMMSTFKVVLCGAVLARVDAGDEQLERKIHYRQQDLVDYSPVSEKHLADGM
TVGELCAAAITMSDNSAANLLLATVGGPAGLTAFLRQIGDNVTRLDRWETELNEALP
GDARDTTTPASMAATLRKLLTSQRLSARSQRQLLQWMVDDRVAGPLIRSVLPAGW
FIADKTGASRRGARGIVALLGPNNKAERIVVIYLRDTPASMAERNQQIAGIGAALIEH
WQR
**SEQ ID N° 590 :** 
MRYIRLCIISLLATLPLAVHASPQPLEQIKQSESQLSGRVGMIEMDLASGRTLTAWRA
DERFPMMSTFKVVLCGAVLARVDAGDEQLERKIHYRQQDLVDYSPVSEKHLADGM
TVGELCAAAITMSDNSAANLLLATVGGPAGLTAFLRQIGDNVTRLDRWETELNEALP
GDARDTTTPASMAATLRKLLTSQRLSARSQRQLLQWMVDDRVAGPLIRSVLPAGW
FIADRTGAGERGARGIVALLGPNNKAERIVVIYLRDTPASMAERNQQIAGIGAALIEH
WQR
**SEQ ID N° 591 :** 
MRYIRLCIISLLATLPLAVHASPQPLEQIKQSESQLSGRVGMIEMDLASGRTLTAWRA
DERFPMMSTFKVVLCGAVLARVDAGDEQLERKIHYRQQDLVDYSPVSEKHLADGM
TVGELCAAAITMSDNSAANLLLATVGGPAGLTAFLRQIGDNVTRLDRWETELNEALP
GDARDTTTPASMTATLRKLLTSQRLSARSQRQLLQWMVDDRVAGPLIRSVLPAGW
 
FIADKTGAGERGARGIVALLGPNNKAERIVVIYLRDTPASMAERNQQIAGIGAALIEH
WQR
**SEQ ID N° 592 :** 
MRYIRLCIISLLATLPLAVHASPQPLEQIKQSESQLSGRVGMIEMDLASGRTLTAWRA
DERFPMMSTFKVVLCGAVLARVDAGDEQLERKIHYRQQDLVDYSPVSEKHLADGM
TVGELCAAAITMSDNSAANLLLATVGGPAGLTAFLRQIGDNVTRLDRWETELNEVLP
GDARDTTTPASMAATLRKLLTSQRLSARSQRQLLQWMVDDRVAGPLIRSVLPAGW
FIADKTGAGERGARGIVALLGPNNKAERIVVIYLRDTPASMAERNQQIAGIGAALIEH
WQR
**SEQ ID N° 593 :** 
MRYIRLCIISLLATLPLAVHASPQPLEQIKQSESQLSGRVGMIEMDLASGRTLTAWRA
DERFPMMSTFKVVLCGAVLARVDAGDEQLERKIHYRQQDLVDYSPVSEKHLADGM
TVGELCAAAITMSDNSAANLLLATVGGPAGVTAFLRQIGDNVTRLDRWETELNEALP
GDARDTTTPASMAATLRKLLTSQRLSARSQRQLLQWMVDDRVAGPLIRSVLPAGW
FIADKTGAGERGARGIVALLGPNNKAERIVVIYLRDTPASMAERNQQIAGIGAALIEH
WQR
**SEQ ID N° 594 :** 
MRYIRLCIISLLATLPLAVHASPQPLEQIKQSESQLSGRVGMIEMDLASGRTLTAWRA
DERFPMMSTFKVVLCGAVLARVDAGDEQLERKIHYRQQDLVDYSPVSEKHLADGM
TVGELCAAAITMSDNSAANLLLATVGGPTGLTAFLRQIGDNVTRLDRWETELNEALP
GDARDTTTPASMAATLRKLLTSQRLSARSQRQLLQWMVDDRVAGPLIRSVLPAGW
FIADKTGAGERGARGIVALLGPNNKAERIVVIYLRDTPASMAERNQQIAGIGAALIEH
WQR
**SEQ ID N° 595 :** 
MRYIRLCIISLLATLPLAVHASPQPLEQIKQSESQLSGRVGMIEMDLASGRTLTAWRA
DERFPMMSTFKVVLCGAVLARVDAGDEQLERKIHYRQQDLVDYSPVSEKHLADGM
TVGELCAAAITMSDNSAANLLLATVGSPAGLTAFLRQIGDNVTRLDRWETELNEALP
GDARDTTTPASMAATLRKLLTSQRLSARSQRQLLQWMVDDRVAGPLIRSVLPAGW
FIADKTGAGERGARGIVALLGPNNKAERIVVIYLRDTPASMAERNQQIAGIGAALIEH
WQR
**SEQ ID N° 596 :** 
MRYIRLCIISLLATLPLAVHASPQPLEQIKQSESQLSGRVGMIEMDLASGRTLTAWRA
DERFPMMSTFKVVLCGAVLARVDAGDEQLERKIHYRQQDLVDYSPVSEKHLADGM
TVGELCAAAITVSDNSAANLLLATVGGPAGLTAFLRQIGDNVTRLDRWETELNEALP
GDARDTTTPASMAATLRKLLTSQRLSARSQRQLLQWMVDDRVAGPLIRSVLPAGW
FIADKTGAGERGARGIVALLGPNNKAERIVVIYLRDTPASMAERNQQIAGIGAALIEH
WQR
**SEQ ID N° 597 :** 
MRYIRLCIISLLATLPLAVHASPQPLEQIKQSESQLSGRVGMIEMDLASGRTLTAWRA
DERFPMMSTFKVVLCGAVLARVDAGDEQLERKIHYRQQDLVDYSPVSEKHLTDGM
TVGELCAAAITMSDNSAANLLLATVGGPAGLTAFLRQIGDNVTRLDRWETELNEALP
GDARDTTTPASMAATLRKLLTSQRLSARSQRQLLQWMVDDRVAGPLIRSVLPAGW
FIADKTGAGERGARGIVALLGPNNKAERIVVIYLRDTPASMAERNQQIAGIGAALIEH
WQR
**SEQ ID N° 598 :** 
MRYIRLCIISLLATLPLAVHASPQPLEQIKQSESQLSGRVGMIEMDLASGRTLTAWRA
DERFPMMSTFKVVLCGAVLARVDAGDEQLERKIHYRQQDLVDYSPVSEKYLADGM
TVGELCAAAITMSDNSAANLLLATVGGPAGLTAFLRQIGDNVTRLDRWETELNEALP
GDARDTTTPASMAATLRKLLTSQRLSARSQRQLLQWMVDDRVAGPLIRSVLPAGW
 
FIADKTGAGERGARGIVALLGPNNKAERIVVIYLRDTPASMAERNQQIAGIGAALIEH
WQR
**SEQ ID N° 599 :** 
MRYIRLCIISLLATLPLAVHASPQPLEQIKQSESQLSGRVGMIEMDLASGRTLTAWRA
DERFPMMSTFKVVLCGAVLARVDAGDEQLERKIHYRQQHLVDYSPVSEKHLADGM
TVGELCAAAITMSDNSAANLLLATVGGPAGLTAFLRQIGDNVTRLDRWETELNEALP
GDARDTTTPASMAATLRKLLTSQRLSARSQRQLLQWMVDDRVAGPLIRSVLPAGW
FIADKTGAGERGARGIVALLGPNNKAERIVVIYLRDTPASMAERNQQIAGIGAALIEH
WQR
**SEQ ID N° 600 :** 
MRYIRLCIISLLATLPLAVHASPQPLEQIKQSESQLSGRVGMIEMDLASGRTLTAWRA
DERFPMMSTFKVVLCGAVLARVDAGDKQLERKIHYRQQDLVDYSPVSEKHLADGM
TVGELCAAAITMSDNSAANLLLATVGGPAGLTAFLRQIGDNVTRLDRWETELNEALP
GDARDTTTPASMAATLRKLLTSQRLSARSQRQLLQWMVDDRVAGPLIRSVLPAGW
FIADKTGAGERGGRGIVALLGPNNKAERIVVIYLRDTPASMAERNQQIAGIGAALIEH
WQR
**SEQ ID N° 601 :** 
MRYIRLCIISLLATLPLAVHASPQPLEQIKQSESQLSGRVGMIEMDLASGRTLTAWRA
DERFPMMSTFKVVLCGTVLARVDAGDEQLERKIHYRQQDLVDYSPVSEKHLADGM
TVGELCAAAITMSDNSAANLLLATVGGPAGLTAFLRQIDDNVTRLDRWETELNEALP
GDARDTTTPASMAATLRKLLTSQRLSARSQRQLLQWMVDDRVAGPLIRSVLPAGW
FIADKTGAGERGARGIVALLGPNNKAERIVVIYLRDTPASMAERNQQIAGIGAALIEH
WQR
**SEQ ID N° 602** : 
MRYIRLCIISLLATLPLAVHASPQPLEQIKQSESQLSGRVGMIEMDLASGRTLTAWRA
DQRFPMMSTFKVVLCGAVLARVDAGDEQLERKIHYRQQDLVDYSPVSEKHLADGM
TVGELCAAAITMSDNSAANLLLATVGGPAGLTAFLRQIGDNVTRLDRWETELNEALP
GDARDTTTPASMAATLRKLLTSQRLSARSQRQLLQWMVDDRVAGPLIRSVLPAGW
FIADKTGASKRGARGIVALLGPNNKAERIVVIYLRDTPASMAERNQQIAGIGAALIEH
WQR
**SEQ** ID **N°** 603 : 
MRYIRLCIISLLATLPLAVHASPQPLEQIKQSESQLSGSVGMIEMDLASGRTLTAWRA
DERFPMMSTFKVVLCGAVLARVDAGDEQLERKIHYRQQDLVDYSPVSEKHLADGM
TVGELCAAAITMSDNSAANLLLATVGGPAGLTAFLRQIGDNVTRLDRWETELNEALP
GDARDTTTPASMAATLRKLLTSQRLSARSQRQLLQWMVDDRVAGPLIRSVLPAGW
FIADKTGAAERGARGIVALLGPNNKAERIVVIYLRDTPASMAERNQQIAGIGAALIEH
WQR
**SEQ** ID **N°** 604 : 
MRYIRLCIISLLATLPLAVHSSPQPLEQIKLSESQLSGRVGMIEMDLASGRTLTAWRA
DERFPMMSTFKVVLCGAVLARVDAGDEQLERKIHYRQQDLVDYSPVSEKHLADGM
TVGELCAAAITVSDNSAANLLLATVGGPAGLTAFLRQIGDNVTRLDRWETELNEALP
GDARDTTTPASMAATLRKLLTSQRLSARSQRQLLQWMVDDRVAGPLIRSVLPAGW
FIADKTGAGERGARGIVALLGPNNKAERIVVIYLRDTPASMAERNQQIAGIGAALIEH
WQR
**SEQ** ID **N°** 605 : 
MRYIRLCIISLLATLPLTVHASPQPLEQIKQSESQLSGRVGMIEMDLASGRTLTAWRA
DERFPMMSTFKVVLCGAVLARVDAGDEQLERKIHYRQQDLVDYSPVSEKHLADGM
TVGELCAAAITMSDNSAANLLLATVGGPAGLTAFLRQIGDNVTRLDRWETELNEALP
GDARDTTTPASMAATLRKLLTSQRLSARSQRQLLQWMVDDRVAGPLIRSVLPAGW
 
FIADKTGAGERGARGIVALLGPNNKAERIVVIYLRDTPASMAERNQQIAGIGAALIEH
WQR
**SEQ ID N° 606 :** 
MRYIRLCIISLLATLSLAVHASPQPLEQIKLSESQLSGRVGMIEMDLASGRTLTAWRA
DERFPMMSTFKVVLCGAVLARVDAGDEQLERKIHYRQQDLVDYSPVSEKHLADGM
TVGELCAAAITMSDNSAANLLLATVGGPAGLTAFLRQIGDNVTRLDRWETELNEALP
GDARDTTTPASMAATLRKLLTSQRLSARSQRQLLQWMVDDRVAGPLIRSVLPAGW
FIADKTGAGKRGARGIVALLGPNNKAERIVVIYLRDTPASMAERNQQIAGIGAALIEH
WQR
**SEQ ID N° 607 :** 
MRYIRLCIISLLATMPLAVHASPQPLEQIKQSESQLSGRVGMIEMDLASGRTLTAWR
ADERFPMMSTFKVVLCGAVLARVDAGDEQLERKIHYRQQDLVDYSPVSEKHLADG
MTVGELCAAAITMSDNSAANLLLATVGGPAGLTAFLRQIGDNVTRLDRWETELNEAL
PGDARDTTTPASMAATLRKLLTSQRLSARSQRQLLQWMVDDRVAGPLIRSVLPAG
WFIADKTGAGERGARGIVALLGPNNKAERIVVIYLRDTPASMAERNQQIAGIGAALIE
HWQR
**SEQ ID N° 608 :** 
MRYIRLCIISLLAVLPLAVHASPQPLEQIKQSESQLSGRVGMIEMDLASGRTLTAWRA
DERFPMMSTFKVVLCGAVLARVDAGDEQLERKIHYRQQDLVDYSPVSEKHLADGM
TVGELCAAAITVSDNSAANLLLATVGGPAGLTAFLRQIGDNVTRLDRWETELNEALP
GDARDTTTPASMAATLRKLLTSQRLSARSQRQLLQWMVDDRVAGPLIRSVLPAGW
FIADKTGAGERGARGIVALLGPNNKAERIVVIYLRDTPASMAERNQQIAGIGAALIEH
WQR
**SEQ ID N° 609 :** 
MRYIRLNIISLLATLPLAVHASPQPLEQIKLSESQLSGRVGMIEMDLASGRTLTAWRA
DERFPMMSTFKVVLCGAVLARVDAGDEQLERKIHYRQQDLVDYSPVSEKHLADGM
TVGELCAAAITMSDNSAANLLLATVGGPAGLTAFLRQIGDNVTRLDRWETELNEALP
GDARDTTTPASMAATLRKLLTSQRLSARSQRQLLQWMVDDRVAGPLIRSVLPAGW
FIADKTGAGERGARGIVALLGPNNKAERIVVIYLRDTPASMAERNQQIAGIGAALIEH
WQR
**SEQ** ID **N°** 610 : 
MRYIRRCIISLLATLPLAVHASPQPLEQIKQSESQLSGRVGMIEMDLASGRTLTAWRA
DERFPMMSTFKVVLCGAVLARVDAGDEQLERKIHYRQQDLVDYSPVSEKHLADGM
TVGELCAAAITMSDNSAANLLLATVGGPAGLTAFLRQIGDNVTRLDRWETELNEALP
GDARDTTTPASMAATLRKLLTSQRLSARSQRQLLQWMVDDRVAGPLIRSVLPAGW
FIADKTGAGERGARGIVALLGPNNKAERIVVIYLRDMPASMAERNQQIAGIGAALIEH
WQR
**SEQ** ID **N°** 611 : 
MRYIRRCIISLLATLPLAVHASPQPLEQIKQSESQLSGRVGMIEMDLASGRTLTAWRA
DERFPMMSTFKVVLCGAVLARVDAGDEQLERKIHYRQQDLVDYSPVSEKHLADGM
TVGELCAAAITMSDNSAANLLLATVGGPAGLTAFLRQIGDNVTRLDRWETELNEALP
GDARDTTTPASMAATLRKLLTSQRLSARSQRQLLQWMVDDRVAGPLIRSVLPAGW
FIADKTGAGERGARGIVALLGPNNKAERIVVIYLRDTPASMAERNQQIAGIGAALIEH
WQR
**SEQ** ID **N°** 612 : 
MRYVRLCIISLLATLPLAVHASPQPLEQIKQSESQLSGRVGMIEMDLASGRTLTAWR
ADERFPMMSTFKVVLCGAVLARVDAGDEQLERKIHYRQQDLVDYSPVSEKHLADG
MTVGELCAAAITMSDNSAANLLLATVGGPAGLTAFLRQIGDNVTRLDRWETELNEAL
PGDARDTTTPASMAATLRKLLTSQRLSARSQRQLLQWMVDDRVAGPLIRSVLPAG
 
WFIADKTGAGERGARGIVALLGPNNKAERIVVIYLRDTPASMAERNQQIAGIGAALIE
HWQR
**SEQ ID N° 613 :** 
MRYVRLCIISLLATLPLAVHTSPQPLEQIKQSESQLSGRVGMIEMDLASGRTLTAWR
ADERFPMMSTFKVVLCGAVLARVDAGDEQLERKIHYRQQDLVDYSPVSEKHLADG
MTVGELCAAAITMSDNSAANLLLATVGGPAGLTAFLRQIGDNVTRLDRWETELNEAL
PGDARDTTTPASMAATLRKLLTSQRLSARSQRQLLQWMVDDRVAGPLIRSVLPAG
WFIADKTGAGERGARGIVALLGPNNKAERIVVIYLRDTPASMAERNQQIAGIGAALIE
HWQR
**SEQ ID N°1909 :** 
MRYIRLCIISLLAALPLAVHASPQPLEQIKQSESQLSGRVGMIEMDLASGRTLTAWRA
DERFPMMSTFKVVLCGAVLARVDAGDEQLERKIHYRQQDLVDYSPVSEKHLADGM
TVGELCAAAITVSDNSAANLLLATVGGPAGLTAFLRQIGDNVTRLDRWETELNEALP
GDARDTTTPASMAATLRKLLTSQRLSARSQRQLLQWMVDDRVAGPLIRSVLPAGW
FIADKTGAGERGARGIVALLGPNNKAERIVVIYLRDTPASMAERNQHIAGIGAALIEH
WQR
**SEQ ID N°1910 :** 
ALPGDARDTTTPASMAATLRKLLTSQRLSARSQRQLLQWMVDDRVAGPLIRSVLPA
GWFIADKTGASERGARGIVALLGPNNKAERIVVIYLRDS
SEQ ID N°1911 :
MRYIRLCIISLLAALPLVVHASPQPLEQIKQSESQLSGRVGMIEMDLASGRTLTAWRA
DERFPMMSTFKVVLCGAVLARVDAGDEQLERKIHYRQQDLVDYSPVSEKHLADGM
TVGELCAAAITVSDNSAANLLLATVGGPAGLTAFLRQIGDNVTRLDRWETELNEALP
GDARDTTTPASMAATLRKLLTSQRLSARSQRQLLQWMVDDRVAGPLIRSVLPAGW
FIADKTGAGERGARGIVALLGPNNKAERIVVIYLRDTPASMAERNQQIAGIGAALIEH
WQR
**SEQ ID N°1912 :** 
MRYIRLCIISLLATLPLAVHASPQPLEQIKQSESQLSGRVGMIEMDLASGRTLTAWRA
DERFPMMSTFKVVLCGAVLARVDAGDEQLERKIHYRQQDLVDYSPVSEKHLADGM
TVGELCAAAITMSDNSAANLLLATVGGPAGLTAFLRQIGDNVTRLDRWETELNEALP
GDARDTTTPASMAATLRKLLTSQRLSARSQRQLLQWMVDDRVAGPLIRSVLPAGW
FIADKTGASKRGARGIVALLGPNNKAERIVVIYLRDTPASMAELDQQIAGIGAALIEHW
QR
**SEQ ID N°1913 :** 
MRYIRLCIISLLATLPLAVHASPQPLEQIKQSESQLSGRVGMIEMDLASGRTLTAWRA
DERFPMMSTFKVVLCGAVLARVDAGDEQLERKIHYRQQDLVDYSPVSEKHLADGM
TVGELRAAAITMSDNSAANLLLATVGGPAGLTAFLRQIGDNVTRLDRWETELNEALP
GDARDTTTPASMAATLRKLLTSQRLSARSQRQLLQWMVDDRVAGPLIRSVLPAGW
FIADKTGASKRGARGIVALLGPNNKAERIVVIYLRDTPASMAERNQQIAGTGAAPIEH
WQR
**SEQ ID N°1914 :** 
MRYIRLCIISLLATLPLAVHASPQPLEQIKQSESQLSGRVGMIEMDLASGRTLTAWRA
DERFPMMSTFKVVLCGAVLARVDAGDEQLERKIHYRQQDLVDYSPVSEKHLADGM
TVGELCAAAITMSDNSAANLLLATVGGPAGLTAFLREIGDNVTRLDRWETELNEALP
GDARDTTTPASMAATLRKLLTSQRLSARSQRQLLQWMVDDRVAGPLIRSVLPAGW
FIADKTGASKRGARGIVALLGPNNKAERIVVIYLRDTPASMAERNQQIAGIGAALIEH
WQR
**SEQ ID N°1915 :** 
MRYIRLCIISLLATLPLAVHASPQPLKQIKQSESQLSGRVGMIEMDLASGRTLTAWRA
DERFPMMSTFKVVLCGAVLARVDAGDEQLERKIHYRQQDLVDYSPVSEKHLADGM
 
TVGELCAAAITMSDNSAANLLLATVGGPAGLTAFLRQIGDNVTRLDRWETELNEALP
GDARDTTTPASMTATLRKLLTSQRLSARSQRQLLQWMVDDRVAGPLIRSVLPAGW
FIADKTGAGERGARGIVALLGPNNKAERIVVIYLRDTPASMAERNQQIAGIGAALIEH
WQR
**SEQ ID N°1916** : 
MRYIRLCIISLLATLPLAVHASPQPLEQIKLSESQLSGRVGMIEMDLASGRTLTAWRA
DERFPMMSTFKVVLCGAVLARVDAGDEQLERKIHYRQQDLVDYSPVSEKHLADGM
TVGELCAAAITMSDNSAANLLLATVGGPAALTAFLRQIGDNVTRLDRWETELNEALP
GDARDTTTPASMAATLRKLLTSQRLSARSQRQLLQWMVDDRVAGPLIRSVLPAGW
FIADKTGAGERGARGIVALLGPHNKAERIVVIYLRDTPASMAERNQQIAGIGAALIEH
WQR
**SEQ ID N°1917** : 
KRYIRLCIISLLATLPLAVHASPQPLEQIKLSESQLSGRVGMIEMDLASGRTLTAWRA
DERFPMMSTFKVVLCGAVLARVDAGDEQLERKIHYSQQDLVDYSPVSEKHLADGM
TVGELCAAAITMSDNSAANLLLATVGGPAGLTAFLRQIGDNVTRLDRWETELNEALP
GDARDTTTPASMAATLRKLLTSQRLSARSQRQLLQWMVDDRVAGPLIRSVLPAGW
FIADKTGAGERGARGIVALLGPNNKAERIVVIYLRDTPASMAERNQQIAGIGAALIEH
WQR
**SEQ** ID **N°1918** : 
MRYIRLCIISLLATLPLAVHASPQPLEQIKLSESQLSGRVGMIEMDLASGRTLTAWRA
DERFPMMSTFKVVLCGAVLARVDAGDEQLERKIHYRQQDLVDYSPVSEKHLADGM
TVGELCAAAITMSDNSAANLLLATVGGPAGLTAFLRQIGDNVTRLDRWETELNEALP
GDARDTTTPASMAATLRKLLTSQRLSARSQRQLLQWMVDDRVAGPLIRSVLPAGW
FIADKTGASKRGARGIVALLGPNNKAERIVVIYLRDTPASMAERNQQIAGIGAALIEH
WQR
**SEQ ID N°1919** : 
MRYIRLCIISLLATLPLAVHASPQPLEQIKQSESQLSGRVGMIEMDLASGRTLTAWRA
DERFPMMSTFKVVLCGAVLARVDAGDEQLERKIHYRQQDLVDYSPVSEKHLADGM
TVGELCAAAITMSDNSAANLLLATVGGPAGLTAFLRQIGDNVTRLDRWETELNEALP
GDARDTTTPASMAATLRKLLTSQRLSARSQRQLLQWMVDDRVAGPLIRSVLPAGW
FIADKTGASKRGARGIVALLGPNNKAERIVVIYLRDTPASMAERNQQIAGIGAA
lesdits peptides étant choisis, de préférence, parmi les peptides de séquence **SEQ ID N°614** à **SEQ ID N°711** et **SEQ ID N°** 2093 à **SEQ ID N°2096** tels que définis ci-après :

| **Peptide SEQ ID N°** | **Séquence d'acides aminés** | **Position du peptide dans la protéine SHV** | **Intérêt clinique** |
|---|---|---|---|
| **SEQ ID N°614** | AGAGER | 231-236 pour la protéine de SEQ N°580 | SHV |
| **SEQ ID N°615** | ATTTPASMAATL R | 175-187 pour la protéine de SEQ N°533 | 2be |
| **SEQ ID N°616** | CIISLLATLPLAVH ASPQPLEQIK | 7-30 pour les protéines de SEQ N°496, 497, 498, 499, 500, 503, 504, 505, 506, 507, 508, 509, 510, 516, 517, 518, 519, 520, 521, 522, 523, 524, 525, 526, 527, 528, 529, 530, 531, 532, 533, 534, 535, 536, 537, 538, 539, 540, 541, 542, 543, 544, 545, 546, 547, 548, 549, 550, 551, 552, 553, 554, 555, 556, 557, 558, 559, 560, 561, 562, 563, 564, 565, 566, 567, 568, 569, 570, 571, 572, 573, 574, 575, 576, 577, 578, 579, 580, 581, 582, 583, 584, 585, 586, 587, 588, 589, 590, 591, 592, 593, 594, 595, 596, 597, 598, 599, 600, 601, 602, 603, 610, 611, 612, 1913, 1914, 1915, 1917, 1918, 1919, 1920; 6-29 pour la protéine de séquence SEQ ID N°501 | SHV |
| **SEQ ID N°617** | DMPASMAER | 261-269 pour la protéine de SEQ N°610 | 2b |
| **SEQ ID N°618** | DSPASMAER | 261-269 pour les protéines de SEQ N°545, 547 | SHV |
| **SEQ ID N°619** | DTLASMAER | 261-269 pour la protéine de SEQ N°564 | SHV |
| **SEQ ID N°620** | DTPASMAER | 261-269 pour les protéines de SEQ N°496, 497, 498, 499, 500, 502, 503, 504, 505, 506, 507, 508, 509, 510, 511, 512, 513, 514, 515, 516, 518, 519, 520, 521, 522, 523, 524, 525, 526, 527, 528, 529, 531, 532, 533, 534, 535, 536, 537, 538, 539, 540, 541, 542, 543, 544, 546, 548, 549, 550, 551, 552, 553, 554, 555, 556, 557, 558, 559, 560, 561, 562, 563, 565, 566, 567, 570, 571, 572, 573, 574, 575, 576, 577, 578, 579, 580, 581, 582, 583, 584, 585, 586, 588, 589, 590, 591, 592, 593, 594, 595, 596, 597, 598, 599, 600, 601, 602, 603, 604, 605, 606, 607, 608, 609, 611, 612, 613, 1910, 1912, 1914, 1915, 1916, 1917, 1918, 1919, 1920; 260-268 pour la protéine de séquence SEQ ID N°501; 266-274 pour la protéine de séquence SEQ ID N°530; 260-268 pour la protéine de séquence SEQ ID N°568; 260-268 pour la protéine de séquence SEQ ID N°569 | SHV |
| **SEQ ID N°621** | DTPASMAK | 261-268 pour la protéine de SEQ N°517 | SHV |
| **SEQ ID N°622** | DTTTPASMAATL R | 175-187 pour les protéines de SEQ N°496, 497, 498, 499, 500, 502, 503, 504, 505, 506, 507, 508, 509, 510, 511, 512, 513, 514, 515, 516, 517, 518, 519, 520, 521, 522, 524, 525, 526, 527, 528, 529, 531, 532, 534, 535, 536, 537, 538, 539, 540, 541, 542, 543, 544, 545, 546, 547, 548, 549, 550, 551, 552, 553, 560, 561, 562, 563, 564, 565, 566, 567, 570, 571, 572, 573, 574, 575, 576, 577, 578, 579, 580, 581, 582, 583, 584, 585, 586, 587, 588, 589, 590, 592, 593, 594, 595, 596, 597, 598, 599, 600, 601, 602, 603, 604, 605, 606, 607, 608, 609, 610, 611, 612, 613, 1910, 1912, 1913, 1914, 1915, 1917, 1918, 1919, 1920; 174-186 pour la protéine de séquence SEQ ID N°501; 180-192 pour la protéine de séquence SEQ ID N°530; 174-186 pour la protéine de séquence SEQ ID N°568; 174-186 pour la protéine de séquence SEQ ID N°569; 8-20 pour la protéine de séquence SEQ ID N°1911 | SHV |
| **SEQ ID N°623** | DTTTPASMAGTL R | 175-187 pour la protéine de SEQ N°523 | 2b |
| **SEQ ID N°624** | DTTTPASMTATL R | 175-187 pour les protéines de SEQ N°554, 555, 556, 557, 591, 1916 | SHV |
| **SEQ ID N°625** | FPMISTFK | 62-69 pour les protéines de SEQ N°511, 518, 572 | 2br |
| **SEQ ID N°626** | FPMMSTFK | 62-69 pour les protéines de SEQ N°496, 497, 498, 499, 500, 502, 503, 504, 505, 506, 507, 508, 509, 510, 512, 513, 514, 515, 516, 517, 519, 520, 521, 522, 523, 524, 525, 526, 527, 528, 529, 530, 531, 532, 533, 534, 535, 536, 537, 538, 539, 540, 541, 542, 543, 544, 545, 546, 547, 548, 549, 550, 551, 552, 553, 554, 555, 556, 557, 558, 559, 560, 561, 562, 563, 564, 565, 566, 567, 570, 571, 573, 574, 575, 576, 577, 578, 579, 580, 581, 582, 583, 584, 585, 586, 587, 588, 589, 590, 591, 592, 593, 594, 595, 596, 597, 598, 599, 600, 601, 602, 603, 604, 605, 606, 607, 608, 609, 610, 611, 612, 613, 1910, 1912, 1913, 1914, 1915, 1916, 1917, 1918, 1919, 1920; 61-68 pour la protéine de séquence SEQ ID N°501; 61-68 pour la protéine de séquence SEQ ID N°568; 61-68 pour la protéine de séquence SEQ ID N°569 | SHV |
| **SEQ ID N°627** | GIVALLGGNIK | 240-250 pour la protéine de SEQ N°586 | 2b |
| **SEQ ID N°628** | GIVALLGPDNK | 240-250 pour la protéine de SEQ N°541 | SHV |
| **SEQ ID N°629** | GIVALLGPNHK | 240-250 pour la protéine de SEQ N°542 | SHV |
| **SEQ ID N°630** | GIVALLGPNNK | 240-250 pour les protéines de SEQ N°496, 497, 498, 500, 502, 503, 504, 505, 506, 507, 508, 509, 510, 511, 512, 513, 514, 515, 516, 517, 518, 519, 520, 521, 522, 523, 524, 525, 526, 527, 528, 529, 531, 532, 533, 534, 535, 536, 537, 538, 539, 540, 543, 545, 546, 547, 548, 549, 550, 551, 552, 553, 554, 555, 556, 557, 558, 559, 560, 561, 562, 563, 564, 565, 566, 567, 570, 571, 572, 573, 574, 575, 576, 577, 578, 579, 580, 581, 582, 583, 584, 585, 587, 588, 589, 590, 591, 592, 593, 594, 595, 596, 597, 598, 599, 600, 601, 602, 603, 604, 605, 606, 607, 608, 609, 610, 611, 612, 613, 1910, 1912, 1913, 1914, 1915, 1916, 1918, 1919, 1920; 239-249 pour la protéine de séquence SEQ ID N°501; 245-255 pour la protéine de séquence SEQ ID N°530; 239-249 pour la protéine de séquence SEQ ID N°568; 239-249 pour la protéine de séquence SEQ ID N°569; 73-83 pour la protéine de séquence SEQ ID N°1911 | SHV |
| **SEQ ID N°631** | GIVALLGPNNNAE R | 240-253 pour la protéine de SEQ N°499 | 2b |
| **SEQ ID N°632** | GIVALR | 240-245 pour la protéine de SEQ N°544 | SHV |
| **SEQ ID N°633** | GPNNK | 246-250 pour les protéines de SEQ N°496, 497, 498, 500, 502, 503, 504, 505, 506, 507, 508, 509, 510, 511, 512, 513, 514, 515, 516, 517, 518, 519, 520, 521, 522, 523, 524, 525, 526, 527, 528, 529, 531, 532, 533, 534, 535, 536, 537, 538, 539, 540, 543, 544, 545, 546, 547, 548, 549, 550, 551, 552, 553, 554, 555, 556, 557, 558, 559, 560, 561, 562, 563, 564, 565, 566, 567, 570, 571, 572, 573, 574, 575, 576, 577, 578, 579, 580, 581, 582, 583, 584, 585, 587, 588, 589, 590, 591, 592, 593, 594, 595, 596, 597, 598, 599, 600, 601, 602, 603, 604, 605, 606, 607, 608, 609, 610, 611, 612, 613, 1910, 1912, 1913, 1914, 1915, 1916, 1918, 1919, 1920; 245-249 pour la protéine de séquence SEQ ID N°501; 251-255 pour la protéine de séquence SEQ ID N°530; 245-249 pour la protéine de séquence SEQ ID N°568; 245-249 pour la protéine de séquence SEQ ID N°569; 79-83 pour la protéine de séquence SEQ ID N°1911 | SHV |
| **SEQ ID N°634** | GTTTPASMAATL R | 175-187 pour la protéine de SEQ N°558 | 2be |
| **SEQ ID N°635** | HLADGMTVGELC AAAITMSDNSAAK | 108-132 pour la protéine de SEQ N°524 | SHV |
| **SEQ ID N°636** | HLLQWMVDDR | 202-211 pour la protéine de SEQ N°554 | SHV |
| **SEQ ID N°637** | IHYLQQDLVDYSP VSEK | 91-107 pour la protéine de SEQ N°519 | SHV |
| **SEQ ID N°638** | IVVIYLR | 254-260 pour les protéines de SEQ N°496, 497, 498, 500, 502, 503, 504, 505, 506, 507, 508, 509, 510, 511, 512, 513, 514, 515, 516, 517, 518, 519, 520, 521, 522, 523, 524, 525, 526, 527, 528, 529, 531, 532, 533, 534, 535, 536, 537, 538, 539, 540, 541, 542, 543, 544, 545, 547, 548, 549, 550, 551, 552, 553, 554, 555, 556, 557, 558, 559, 560, 561, 562, 563, 564, 565, 566, 567, 570, 571, 572, 573, 574, 575, 576, 577, 578, 579, 580, 581, 582, 583, 584, 585, 586, 587, 588, 589, 590, 591, 592, 593, 594, 595, 596, 597, 598, 599, 600, 601, 602, 603, 604, 605, 606, 607, 608, 609, 610, 611, 612, 613, 1910, 1912, 1913, 1914, 1915, 1916, 1917, 1918, 1919, 1920; 253-259 pour la protéine de séquence SEQ ID N°501; 259-265 pour la protéine de séquence SEQ ID N°530; 253-259 pour la protéine de séquence SEQ ID N°568; 253-259 pour la protéine de séquence SEQ ID N°569; 87-93 pour la protéine de séquence SEQ ID N°1911 | SHV |
| **SEQ ID N°639** | LCIISLLAALPLAV HASPQPLEQIK | 6-30 pour les protéines de SEQ N°511, 512, 513, 514, 1910 | 2b |
| **SEQ ID N°640** | LCIISLLATLPLAV HASPQPLDQIK | 6-30 pour la protéine de SEQ N°515 | 2b |
| **SEQ ID N°641** | LCIISLLATLPLAV HASPQPLEQIK | 6-30 pour les protéines de SEQ N°496, 497, 498, 499, 500, 503, 504, 505, 506, 507, 508, 509, 510, 516, 517, 518, 519, 520, 521, 522, 523, 524, 525, 526, 527, 528, 529, 530, 531, 532, 533, 534, 535, 536, 537, 538, 539, 540, 541, 542, 543, 544, 545, 546, 547, 548, 549, 550, 551, 552, 553, 554, 555, 556, 557, 558, 559, 560, 561, 562, 563, 564, 565, 566, 567, 568, 569, 570, 571, 572, 573, 574, 575, 576, 577, 578, 579, 580, 581, 582, 583, 584, 585, 586, 587, 588, 589, 590, 591, 592, 593, 594, 595, 596, 597, 598, 599, 600, 601, 602, 603, 612, 1913, 1914, 1915, 1917, 1918, 1919, 1920; 5-29 pour la protéine de séquence SEQ ID N°501 | SHV |
| **SEQ ID N°642** | LCIISLLATLPLAV HSSPQPLEQIK | 6-30 pour la protéine de SEQ N°604 | SHV |
| **SEQ ID N°643** | LCIISLLATLPLAV HTSPQPLEQIK | 6-30 pour la protéine de SEQ N°613 | SHV |
| **SEQ ID N°644** | LCIISLLATLPLTV HASPQPLEQIK | 6-30 pour la protéine de SEQ N°605 | 2b |
| **SEQ ID N°645** | LCIISLLATLPLVV HASPQPLEQIK | 6-30 pour la protéine de SEQ N°502 | 2b |
| **SEQ ID N°646** | LCIISLLATLSLAV HASPQPLEQIK | 6-30 pour la protéine de SEQ N°606 | 2be |
| **SEQ ID N°647** | LCIISLLATMPLAV HASPQPLEQIK | 6-30 pour la protéine de SEQ N°607 | SHV |
| **SEQ ID N°648** | LCIISLLAVLPLAV HASPQPLEQIK | 6-30 pour la protéine de SEQ N°608 | 2b |
| **SEQ ID N°649** | LLISQR | 189-194 pour la protéine de SEQ N°578 | SHV |
| **SEQ ID N°650** | LLLATVGGPAGLT AFLR | 133-149 pour les protéines de SEQ N°496, 497, 498, 499, 500, 502, 503, 505, 506, 507, 508, 509, 510, 512, 513, 514, 515, 516, 517, 518, 519, 521, 522, 523, 524, 527, 528, 529, 530, 531, 532, 533, 534, 535, 536, 537, 538, 539, 540, 541, 542, 543, 544, 545, 546, 547, 548, 549, 550, 551, 552, 553, 554, 555, 556, 557, 558, 559, 560, 561, 562, 564, 565, 566, 570, 571, 573, 574, 575, 577, 578, 579, 580, 581, 582, 583, 584, 585, 586, 587, 588, 589, 590, 591, 592, 596, 597, 598, 599, 600, 601, 602, 603, 604, 605, 606, 607, 608, 609, 610, 611, 612, 613, 1910, 1912, 1913, 1914, 1915, 1916, 1918, 1919, 1920 | SHV |
| **SEQ ID N°651** | LLNSQR | 189-194 pour la protéine de SEQ N°534 | 2be |
| **SEQ ID N°652** | LLTNQR | 189-194 pour la protéine de SEQ N°562 | SHV |
| **SEQ ID N°653** | LLTSQR | 189-194 pour les protéines de SEQ N°496, 497, 498, 499, 500, 502, 503, 504, 505, 506, 507, 508, 509, 510, 511, 512, 513, 514, 515, 516, 517, 518, 519, 520, 521, 522, 523, 524, 525, 526, 527, 528, 529, 531, 532, 533, 535, 536, 537, 538, 539, 540, 541, 542, 543, 544, 545, 546, 547, 548, 549, 550, 551, 552, 553, 554, 555, 556, 557, 558, 559, 560, 561, 563, 564, 565, 566, 567, 570, 571, 572, 573, 574, 575, 576, 577, 579, 580, 581, 582, 583, 584, 585, 586, 587, 588, 589, 590, 591, 592, 593, 594, 595, 596, 597, 598, 599, 600, 601, 602, 603, 604, 605, 606, 607, 608, 609, 610, 611, 612, 613, 1910, 1912, 1913, 1914, 1915, 1916, 1917, 1918, 1919, 1920; 188-193 pour la protéine de séquence SEQ ID N°501; 194-199 pour la protéine de séquence SEQ ID N°530; 22-27 pour la protéine de séquence SEQ ID N°1911 | SHV |
| **SEQ ID N°654** | LNIISLLATLPLAV HASPQPLEQIK | 6-30 pour la protéine de SEQ N°609 | 2b |
| **SEQ ID N°655** | LSASSQR | 195-201 pour la protéine de SEQ N°497 | SHV |
| **SEQ ID N°656** | LSESQLSGR | 31-39 pour les protéines de SEQ N°496, 497, 498, 499, 500, 503, 504, 515, 516, 517, 518, 519, 520, 521, 522, 523, 524, 525, 526, 527, 528, 529, 530, 531, 532, 533, 534, 535, 536, 537, 538, 539, 540, 541, 542, 543, 544, 545, 546, 547, 548, 549, 550, 551, 552, 553, 554, 555, 556, 557, 558, 559, 560, 561, 562, 563, 564, 565, 566, 567, 568, 569, 570, 604, 606, 609, 1917, 1918, 1919; 30-38 pour la protéine de séquence SEQ ID N°501 | SHV |
| **SEQ ID N°657** | LSESQLSGSVGM IEMDLASGR | 31-51 pour les protéines de SEQ N°505, 506, 507, 508, 509, 510 | SHV |
| **SEQ ID N°658** | MVVIYLR | 254-260 pour la protéine de SEQ N°499 | 2b |
| **SEQ ID N°659** | NEALPGDAR | 166-174 pour les protéines de SEQ N°496, 497, 498, 499, 500, 502, 503, 504, 505, 506, 507, 508, 509, 510, 511, 512, 513, 514, 515, 516, 517, 518, 519, 520, 521, 523, 524, 525, 527, 528, 529, 533, 534, 535, 536, 537, 538, 539, 540, 541, 542, 543, 544, 545, 546, 547, 548, 549, 550, 551, 552, 553, 554, 555, 556, 557, 558, 559, 561, 562, 563, 564, 565, 566, 567, 570, 571, 572, 573, 574, 575, 576, 577, 578, 579, 580, 581, 582, 583, 584, 585, 586, 587, 588, 589, 590, 591, 593, 594, 595, 596, 597, 598, 599, 600, 601, 602, 603, 604, 605, 606, 607, 608, 609, 610, 611, 612, 613, 1910, 1912, 1913, 1914, 1915, 1916, 1917, 1918, 1919, 1920; 165-173 pour la protéine de séquence SEQ ID N°501; 171-179 pour la protéine de séquence SEQ ID N°530; 165-173 pour la protéine de séquence SEQ ID N°568; 165-173 pour la protéine de séquence SEQ ID N°569 | 2be |
| **SEQ ID N°660** | NQHIAGIGAALIE HWQR | 270-286 pour les protéines de SEQ N°513, 1910 | SHV |
| **SEQ ID N°661** | NQQIAGIGAALIE HWQR | 270-286 pour les protéines de SEQ N°496, 497, 498, 499, 500, 502, 503, 504, 505, 506, 507, 508, 509, 510, 511, 512, 514, 515, 516, 517, 518, 519, 520, 521, 522, 523, 524, 525, 526, 527, 528, 529, 531, 532, 533, 534, 535, 536, 537, 538, 539, 540, 541, 542, 543, 544, 545, 546, 547, 548, 549, 551, 552, 553, 554, 555, 556, 557, 558, 559, 560, 561, 562, 563, 564, 565, 566, 567, 570, 571, 572, 573, 574, 575, 576, 577, 578, 579, 580, 581, 582, 583, 584, 585, 586, 588, 589, 590, 591, 592, 593, 594, 595, 596, 597, 598, 599, 600, 601, 602, 603, 604, 605, 606, 607, 608, 609, 610, 611, 612, 613, 1912, 1915, 1916, 1917, 1918, 1919; 269-285 pour la protéine de séquence SEQ ID N°501; 275-291 pour la protéine de séquence SEQ ID N°530; 269-285 pour la protéine de séquence SEQ ID N°568; 269-285 pour la protéine de séquence SEQ ID N°569 | SHV |
| **SEQ ID N°662** | NQQIAGLGAALIE HWQR | 270-286 pour la protéine de SEQ N°550 | SHV |
| **SEQ ID N°663** | NTTTPASMAATL R | 175-187 pour la protéine de SEQ N°559 | 2be |
| **SEQ ID N°664** | NVLTSQR | 187-193 pour les protéines de SEQ N°568, 569 | SHV |
| **SEQ ID N°665** | QIDDNVTR | 150-157 pour les protéines de SEQ N°505, 527, 528, 565, 577, 601 | 2be |
| **SEQ ID N°666** | QIGDK | 150-154 pour la protéine de SEQ N°529 | 2b |
| **SEQ ID N°667** | QIGDNVTR | 150-157 pour les protéines de SEQ N°496, 497, 498, 499, 500, 502, 503, 504, 506, 507, 508, 509, 510, 511, 512, 513, 514, 515, 516, 517, 518, 519, 520, 521, 522, 523, 524, 525, 526, 530, 531, 532, 533, 534, 535, 536, 537, 538, 539, 540, 541, 542, 543, 544, 545, 546, 547, 548, 549, 550, 551, 552, 553, 554, 555, 556, 557, 558, 559, 560, 561, 563, 564, 566, 567, 570, 571, 572, 573, 574, 575, 576, 578, 579, 580, 581, 582, 583, 584, 585, 586, 587, 588, 589, 590, 591, 592, 593, 594, 595, 596, 597, 598, 599, 600, 602, 603, 604, 605, 606, 607, 608, 609, 610, 611, 612, 613, 1910, 1912, 1913, 1914, 1916, 1917, 1918, 1919, 1920; 149-156 pour la protéine de séquence SEQ ID N°501; 149-156 pour la protéine de séquence SEQ ID N°568; 149-156 pour la protéine de séquence SEQ ID N°569 | SHV |
| **SEQ ID N°668** | QIGENVTR | 150-157 pour la protéine de SEQ N°562 | 2b |
| **SEQ ID N°669** | QLLQWMVDAR | 202-211 pour la protéine de SEQ N°579 | SHV |
| **SEQ ID N°670** | QLLQWMVDDGV AGPLIR | 202-218 pour la protéine de SEQ N°539 | SHV |
| **SEQ ID N°671** | QLLQWMVDDR | 202-211 pour les protéines de SEQ N°496, 497, 498, 499, 500, 502, 503, 504, 505, 506, 507, 508, 509, 510, 511, 513, 514, 515, 516, 517, 518, 519, 520, 521, 522, 523, 524, 525, 526, 527, 528, 529, 531, 532, 533, 534, 535, 536, 537, 538, 540, 541, 542, 543, 544, 545, 546, 547, 548, 549, 550, 551, 552, 555, 556, 557, 558, 559, 560, 561, 562, 563, 564, 565, 566, 567, 570, 571, 572, 573, 574, 575, 576, 577, 578, 580, 581, 582, 583, 584, 585, 586, 587, 588, 589, 590, 591, 592, 593, 594, 595, 596, 597, 598, 599, 600, 601, 602, 603, 604, 605, 606, 607, 608, 609, 610, 611, 612, 613, 1910, 1912, 1913, 1914, 1915, 1916, 1917, 1918, 1919, 1920; 201-210 pour la protéine de séquence SEQ ID N°501; 207-216 pour la protéine de séquence SEQ ID N°530; 201-210 pour la protéine de séquence SEQ ID N°568; 201-210 pour la protéine de séquence SEQ ID N°569; 35-44 pour la protéine de séquence SEQ ID N°1911 | SHV |
| **SEQ ID N°672** | QLLQWMVDGR | 202-211 pour la protéine de SEQ N°553 | 2b |
| **SEQ ID N°673** | QLLQWMVEDR | 202-211 pour la protéine de SEQ N°512 | SHV |
| **SEQ ID N°674** | QQDLVDYSPVSE K | 95-107 pour les protéines de SEQ N°496, 497, 498, 499, 500, 502, 503, 504, 505, 506, 507, 508, 509, 510, 511, 512, 513, 514, 515, 516, 517, 518, 519, 520, 521, 522, 523, 524, 525, 526, 527, 528, 529, 530, 531, 532, 533, 534, 535, 536, 537, 538, 539, 540, 541, 542, 543, 544, 545, 546, 547, 548, 549, 550, 551, 552, 553, 554, 555, 556, 557, 558, 559, 560, 561, 562, 563, 564, 565, 566, 567, 570, 571, 572, 573, 574, 575, 576, 577, 578, 579, 580, 581, 582, 583, 584, 585, 586, 587, 588, 589, 590, 591, 592, 593, 594, 595, 596, 597, 598, 600, 601, 602, 603, 604, 605, 606, 607, 608, 609, 610, 611, 612, 613, 1910, 1912, 1913, 1914, 1915, 1916, 1917, 1918, 1919, 1920; 94-106 pour la protéine de séquence SEQ ID N°501; 94-106 pour la protéine de séquence SEQ ID N°568; 94-106 pour la protéine de séquence SEQ ID N°569 | SHV |
| **SEQ ID N°675** | QQHLVDYSPVSE K | 95-107 pour la protéine de SEQ N°599 | 2b |
| **SEQ ID N°676** | QSESQLSGR | 31-39 pour les protéines de SEQ N°502, 511, 512, 513, 514, 571, 572, 573, 574, 575, 576, 577, 578, 579, 580, 581, 582, 583, 584, 585, 586, 587, 588, 589, 590, 591, 592, 593, 594, 595, 596, 597, 598, 599, 600, 601, 602, 605, 607, 608, 610, 611, 612, 613, 1910, 1912, 1913, 1914, 1915, 1916, 1920 | SHV |
| **SEQ ID N°677** | QSESQLSGSVGM IEMDLASGR | 31-51 pour la protéine de SEQ N°603 | 2b |
| **SEQ ID N°678** | SQLQLLQWMVD DR | 199-211 pour les protéines de SEQ N°536, 537, 538 | 2be |
| **SEQ ID N°679** | SVLPAGWFIADK | 219-230 pour les protéines de SEQ N°496, 497, 498, 499, 500, 502, 503, 505, 506, 507, 508, 509, 510, 511, 512, 513, 514, 515, 516, 517, 518, 519, 520, 521, 522, 523, 524, 525, 526, 527, 528, 529, 531, 532, 533, 534, 535, 536, 537, 538, 539, 540, 541, 542, 543, 544, 545, 546, 547, 548, 549, 550, 553, 554, 555, 556, 557, 558, 559, 560, 561, 562, 563, 564, 565, 566, 567, 570, 571, 572, 573, 574, 575, 576, 577, 578, 579, 580, 581, 582, 583, 584, 585, 586, 587, 588, 589, 591, 592, 593, 594, 595, 596, 597, 598, 599, 600, 601, 602, 603, 604, 605, 606, 607, 608, 609, 610, 611, 612, 613, 1910, 1912, 1913, 1914, 1915, 1916, 1917, 1918, 1919, 1920; 224-235 pour la protéine de séquence SEQ ID N°530; 218-229 pour la protéine de séquence SEQ ID N°568; 218-229 pour la protéine de séquence SEQ ID N°569; 52-63 pour la protéine de séquence SEQ ID N°1911 | SHV |
| **SEQ ID N°680** | SVLPAGWFIADR | 219-230 pour les protéines de SEQ N°504, 551, 590; 218-229 pour la protéine de séquence SEQ ID N°501 | SHV |
| **SEQ ID N°681** | SVLSAGWFIADK | 219-230 pour la protéine de SEQ N°552 | 2b |
| **SEQ ID N°682** | TGAAER | 231-236 pour les protéines de SEQ N°540, 581, 603 | SHV |
| **SEQ ID N°683** | TGAAK | 231-235 pour la protéine de SEQ N°506 | 2be |
| **SEQ ID N°684** | TGAGER | 231-236 pour les protéines de SEQ N°496, 497, 498, 499, 502, 504, 507, 511, 512, 513, 514, 516, 518, 519, 520, 521, 524, 525, 526, 527, 531, 533, 535, 536, 539, 541, 542, 543, 544, 551, 552, 553, 554, 555, 558, 559, 560, 561, 562, 563, 564, 565, 566, 567, 570, 571, 572, 573, 576, 577, 578, 579, 582, 583, 590, 591, 592, 593, 594, 595, 596, 597, 598, 599, 600, 601, 604, 605, 607, 608, 609, 610, 611, 612, 613, 1910, 1912, 1916, 1917, 1918; 230-235 pour la protéine de séquence SEQ ID N°501; 236-241 pour la protéine de séquence SEQ ID N°530 | SHV |
| **SEQ ID N°685** | TGAGK | 231-235 pour les protéines de SEQ N°517, 545, 546, 556, 584, 606 | SHV |
| **SEQ ID N°686** | TGASER | 64-69 pour les protéines de SEQ N°1911; 231-236 pour la protéine de séquence SEQ ID N°503; 231-236 pour la protéine de séquence SEQ ID N°508; 231-236 pour la protéine de séquence SEQ ID N°510; 231-236 pour la protéine de séquence SEQ ID N°522; 231-236 pour la protéine de séquence SEQ ID N°532; 231-236 pour la protéine de séquence SEQ ID N°537; 231-236 pour la protéine de séquence SEQ ID N°547; 231-236 pour la protéine de séquence SEQ ID N°548; 231-236 pour la protéine de séquence SEQ ID N°585 | 2be |
| **SEQ ID N°687** | TGASK | 231-235 pour les protéines de SEQ N°500, 505, 509, 515, 523, 528, 529, 534, 538, 549, 550, 557, 574, 575, 586, 587, 588, 602, 1913, 1914, 1915, 1919, 1920; 230-234 pour la protéine de séquence SEQ ID N°568; 230-234 pour la protéine de séquence SEQ ID N°569 | SHV |
| **SEQ ID N°688** | TGASR | 231-235 pour la protéine de SEQ N°589 | SHV |
| **SEQ ID N°689** | TLTAWCADER | 52-61 pour la protéine de SEQ N°571 | SHV |
| **SEQ ID N°690** | TLTAWHADER | 52-61 pour les protéines de SEQ N°516, 517 | 2be |
| **SEQ ID N°691** | TLTAWR | 52-57 pour les protéines de SEQ N°496, 497, 498, 499, 500, 502, 503, 504, 505, 506, 507, 508, 509, 510, 511, 512, 513, 514, 515, 518, 519, 520, 521, 522, 523, 524, 525, 526, 527, 528, 529, 530, 531, 532, 533, 534, 535, 536, 537, 538, 539, 540, 541, 542, 543, 544, 545, 546, 547, 548, 549, 550, 551, 552, 553, 554, 555, 556, 557, 558, 559, 560, 561, 562, 563, 564, 565, 566, 567, 570, 572, 573, 574, 575, 576, 577, 578, 579, 580, 581, 582, 583, 584, 585, 586, 587, 588, 589, 590, 591, 592, 593, 594, 595, 596, 597, 598, 599, 600, 601, 602, 603, 604, 605, 606, 607, 608, 609, 610, 611, 612, 613, 1910, 1912, 1913, 1914, 1915, 1916, 1917, 1918, 1919, 1920; 51-56 pour la protéine de séquence SEQ ID N°501; 51-56 pour la protéine de séquence SEQ ID N°568; 51-56 pour la protéine de séquence SEQ ID N°569 | SHV |
| **SEQ ID N°692** | TVGGPAGLTAFL R | 137-149 pour les protéines de SEQ N°496, 497, 498, 499, 500, 502, 503, 505, 506, 507, 508, 509, 510, 512, 513, 514, 515, 516, 517, 518, 519, 521, 522, 523, 524, 527, 528, 529, 530, 531, 532, 533, 534, 535, 536, 537, 538, 539, 540, 541, 542, 543, 544, 545, 546, 547, 548, 549, 550, 551, 552, 553, 554, 555, 556, 557, 558, 559, 560, 561, 562, 564, 565, 566, 570, 571, 573, 574, 575, 577, 578, 579, 580, 581, 582, 583, 584, 585, 586, 587, 588, 589, 590, 591, 592, 596, 597, 598, 599, 600, 601, 602, 603, 604, 605, 606, 607, 608, 609, 610, 611, 612, 613, 1910, 1912, 1913, 1914, 1915, 1916, 1918, 1919, 1920; 136-148 pour la protéine de séquence SEQ ID N°568; 136-148 pour la protéine de séquence SEQ ID N°569 | SHV |
| **SEQ ID N°693** | TWIYLR | 254-260 pour la protéine de SEQ N°546 | SHV |
| **SEQ ID N°694** | VAGPLIR | 212-218 pour les protéines de SEQ N°496, 497, 498, 499, 500, 502, 503, 504, 505, 506, 507, 508, 509, 510, 511, 512, 513, 514, 515, 516, 517, 518, 519, 520, 521, 522, 523, 524, 525, 526, 527, 528, 529, 531, 532, 533, 534, 535, 536, 537, 538, 539, 540, 541, 542, 543, 544, 545, 546, 547, 548, 549, 550, 551, 552, 553, 554, 555, 556, 557, 558, 559, 560, 561, 562, 563, 564, 565, 566, 567, 570, 571, 572, 573, 574, 575, 576, 577, 578, 579, 580, 581, 582, 583, 584, 585, 586, 587, 588, 589, 590, 591, 592, 593, 594, 595, 596, 597, 598, 599, 600, 601, 602, 603, 604, 605, 606, 607, 608, 609, 610, 611, 612, 613, 1910, 1912, 1913, 1914, 1915, 1916, 1917, 1918, 1919, 1920; 211-217 pour la protéine de séquence SEQ ID N°501; 217-223 pour la protéine de séquence SEQ ID N°530; 211-217 pour la protéine de séquence SEQ ID N°568; 211-217 pour la protéine de séquence SEQ ID N°569; 45-51 pour la protéine de séquence SEQ ID N°1911 | SHV |
| **SEQ ID N°695** | VALCGAVLAR | 70-79 pour la protéine de SEQ N°573 | 2b |
| **SEQ ID N°696** | VDAGDEQLER | 80-89 pour les protéines de SEQ N°496, 497, 498, 499, 500, 502, 503, 504, 505, 506, 507, 508, 509, 510, 511, 512, 513, 514, 515, 516, 517, 518, 519, 520, 521, 522, 523, 524, 525, 526, 527, 528, 529, 530, 531, 532, 533, 534, 535, 536, 537, 538, 539, 540, 541, 542, 543, 544, 545, 546, 547, 548, 549, 550, 551, 552, 553, 554, 555, 556, 557, 558, 559, 560, 561, 562, 563, 564, 565, 566, 567, 570, 571, 572, 573, 574, 576, 577, 578, 579, 580, 581, 582, 583, 584, 585, 586, 587, 588, 589, 590, 591, 592, 593, 594, 595, 596, 597, 598, 599, 601, 602, 603, 604, 605, 606, 607, 608, 609, 610, 611, 612, 613, 1910, 1912, 1913, 1914, 1915, 1916, 1917, 1918, 1919, 1920; 79-88 pour la protéine de séquence SEQ ID N°501; 79-88 pour la protéine de séquence SEQ ID N°568; 79-88 pour la protéine de séquence SEQ ID N°569 | SHV |
| **SEQ ID N°697** | VDAGDK | 80-85 pour les protéines de SEQ N°575, 600 | SHV |
| **SEQ ID N°698** | VGMIEMDLASGR | 40-51 pour les protéines de SEQ N°496, 497, 498, 499, 500, 502, 503, 504, 505, 506, 507, 508, 509, 510, 511, 512, 513, 514, 515, 516, 517, 518, 519, 520, 521, 522, 523, 524, 525, 526, 527, 528, 529, 530, 531, 532, 533, 534, 535, 536, 537, 538, 539, 540, 541, 542, 543, 544, 545, 546, 547, 548, 549, 550, 551, 552, 553, 554, 555, 556, 557, 558, 559, 560, 561, 562, 563, 564, 565, 566, 567, 571, 572, 573, 574, 575, 576, 577, 578, 579, 580, 581, 582, 583, 584, 585, 586, 587, 588, 589, 590, 591, 592, 593, 594, 595, 596, 597, 598, 599, 600, 601, 602, 603, 604, 605, 606, 607, 608, 609, 610, 611, 612, 613, 1910, 1912, 1913, 1914, 1915, 1916, 1917, 1918, 1919, 1920; 39-50 pour la protéine de séquence SEQ ID N°501 | SHV |
| **SEQ ID N°699** | VGMIEMDLASR | 40-50 pour les protéines de SEQ N°568, 569 | SHV |
| **SEQ ID N°700** | VGMIEMDLASSR | 40-51 pour la protéine de SEQ N°570 | SHV |
| **SEQ ID N°701** | VLLCGAVLAR | 70-79 pour la protéine de SEQ N°574 | SHV |
| **SEQ ID N°702** | VVLCGAMLAR | 70-79 pour la protéine de SEQ N°575 | 2be |
| **SEQ ID N°703** | VVLCGAVLAR | 70-79 pour les protéines de SEQ N°496, 497, 498, 499, 500, 502, 503, 504, 505, 506, 507, 508, 509, 510, 511, 512, 513, 514, 515, 516, 517, 518, 519, 520, 521, 522, 523, 524, 525, 526, 527, 528, 529, 530, 531, 532, 533, 534, 535, 536, 537, 538, 539, 540, 541, 542, 543, 544, 545, 546, 547, 548, 549, 550, 551, 552, 553, 554, 555, 556, 557, 558, 559, 560, 561, 562, 563, 564, 565, 566, 567, 570, 571, 572, 576, 577, 578, 579, 580, 581, 582, 583, 584, 585, 586, 587, 588, 589, 590, 591, 592, 593, 594, 595, 596, 597, 598, 599, 600, 602, 603, 604, 605, 606, 607, 608, 609, 610, 611, 612, 613, 1910, 1912, 1913, 1914, 1915, 1916, 1917, 1918, 1919, 1920; 69-78 pour la protéine de séquence SEQ ID N°501; 69-78 pour la protéine de séquence SEQ ID N°568; 69-78 pour la protéine de séquence SEQ ID N°569 | SHV |
| **SEQ ID N°704** | VVLCGTVLAR | 70-79 pour la protéine de SEQ N°601 | 2b |
| **SEQ ID N°705** | WETDR | 161-165 pour la protéine de SEQ N°530 | 2be |
| **SEQ ID N°706** | WETELNEAFPGD AR | 161-174 pour les protéines de SEQ N°522, 531, 532 | SHV |
| **SEQ ID N°707** | WETELNEALPAD AR | 161-174 pour la protéine de SEQ N°526 | 2b |
| **SEQ ID N°708** | WETELNEALPGD AR | 161-174 pour les protéines de SEQ N°496, 497, 498, 499, 500, 502, 503, 504, 505, 506, 507, 508, 509, 510, 511, 512, 513, 514, 515, 516, 517, 518, 519, 520, 521, 523, 524, 525, 527, 528, 529, 533, 534, 535, 536, 537, 538, 539, 540, 541, 542, 543, 544, 545, 546, 547, 548, 549, 550, 551, 552, 553, 554, 555, 556, 557, 558, 559, 562, 563, 564, 565, 566, 567, 570, 571, 572, 573, 574, 575, 576, 577, 578, 579, 580, 581, 582, 583, 584, 585, 586, 587, 588, 589, 590, 591, 593, 594, 595, 596, 597, 598, 599, 600, 601, 602, 603, 604, 605, 606, 607, 608, 609, 610, 611, 612, 613, 1910, 1912, 1913, 1914, 1915, 1916, 1917, 1918, 1919, 1920; 160-173 pour la protéine de séquence SEQ ID N°501; 166-179 pour la protéine de séquence SEQ ID N°530; 160-173 pour la protéine de séquence SEQ ID N°568; 160-173 pour la protéine de séquence SEQ ID N°569 | SHV |
| **SEQ ID N°709** | WETELNEALSGD AR | 161-174 pour la protéine de SEQ N°560 | 2b |
| **SEQ ID N°710** | WETELNEVLPGD AR | 161-174 pour la protéine de SEQ N°592 | SHV |
| **SEQ ID N°711** | WETER | 161-165 pour la protéine de SEQ N°561 | SHV |
| **SEQ** ID **N°2093** | EIGDNVTR | 150-157 pour les protéines de SEQ N°1915 | SHV |
| **SEQ** ID **N°2094** | GIVALLGPHNK | 240-250 pour les protéines de SEQ N°1917 | SHV |
| **SEQ** ID **N°2095** | HLADGMTVGELR | 108-119 pour les protéines de SEQ N°1914 | 2be |
| **SEQ** ID **N°2096** | IHYSQQDLVDYS PVSEK | 91-107 pour les protéines de SEQ N°1918 | SHV |

Dans la colonne intérêt clinique, les mentions 2b, 2br, 2be et 2ber correspondent aux sous-groupes fonctionnels des béta-lactamases SHV que permet de détecter le peptide correspondant. Ainsi la détection d'un peptide 2be signera la présence d'une béta-lactamase avec un spectre étendu (BLSE) capable d'hydrolyser les pénicillines, les céphalosporines de première génération telles que la céphaloridine et la céphalotine ainsi qu'au moins un antibiotique de la classe des oxyimino-beta-lactames tels que le cefotaxime, le ceftazidime ou les monobactames tels que l'aztreoname.

La mention SHV indique un peptide commun entre au moins deux des sous-groupes 2b, 2br et 2be ou 2ber. Le peptide correspondant signe la présence d'une beta-lactamase de type SHV et la présence d'un mécanisme de résistance au moins aux pénicillines et aux céphalosporines de première génération.

La détection d'un mécanisme de résistance aux céphalosporines de type BLSE (beta-lactamase à spectre étendu), induit par la protéine SHV, est caractérisée par la détection d'au moins un peptide marqueur de résistance, de type 2be, choisi parmi les séquences SEQ ID N°616, SEQ ID N°617, SEQ ID N°627, SEQ ID N°629, SEQ ID N°634, SEQ ID N°647, SEQ ID N°648, SEQ ID N°653, SEQ ID N°654, SEQ ID N°656, SEQ ID N°660, SEQ ID N°661, SEQ ID N°662, SEQ ID N°663, SEQ ID N°664, SEQ ID N°665, SEQ ID N°670, SEQ ID N°673, SEQ ID N°674, SEQ ID N°675, SEQ ID N°678, SEQ ID N°682, SEQ ID N°684, SEQ ID N°685, SEQ ID N°686, SEQ ID N°687, SEQ ID N°688, SEQ ID N°697, SEQ ID N°702, SEQ ID N°703, SEQ ID N°704, SEQ ID N°707, SEQ ID N°708, SEQ ID N°709, SEQ ID N°711, SEQ ID N°2095.

La détection d'un mécanisme de résistance aux céphalosporines induit par l'expression de la protéine FOX est caractérisée par la détection d'au moins un peptide appartenant à la protéine FOX et à ses différents variants de séquences **SEQ ID N° 712** à **SEQ ID N°718 et SEQ ID N° 1920** à **SEQ ID N°1922.**
**SEQ ID** N° **712 :** 
MQQRRAFALLTLGSLLLAPCTYASGEAPLTAAVDGIIQPMLKAYRIPGMAVAVLKDG
KAHYFNYGVANRESGQRVSEQTLFEIGSVSKTLTATLGAYAAVKGGFELDDKVSQH
APWLKGSALDGVTMAELATYSAGGLPLQFPDEVDSNDKMRTYYRSWSPVYPAGT
HRQYSNPSIGLFGHLAANSLGQPFEQLMSQTLLPKLGLHHTYIQVPESAMVNYAYG
YSKEDKPVRVTPGVLAAEAYGIKTGSADLLKFAEANMGYQGDAAVKSAIALTHTGFY
SVGDMTQGLGWESYAYPVTEQTLLAGNAPAVSFQANPVTRFAVPKAMGEQRLYN
KTGSTGGFGAYVAFVPARGIAIVMLANRNYPIEARVKAAHAILSQLAE
**SEQ ID N° 713 :** 
MQQRRAFALLTLGSLLLAPCTYASGEAPLTAAVDGIIQPMLKAYRIPGMAVAVLKDG
KAHYFNYGVANRESGQRVSEQTLFEIGSVSKTLTATLGAYAAVKGGFELDDKVSQH
APWLKGSALDGVTMAELATYSAGGLPLQFPDEVDSNDKMRTYYRSWSPVYPAGT
HRQYSNPSIGLFGHLAANSLGQPFEQLMSQTLLPKLGLHHTYIQVPESAMVNYAYG
YSKEDKPVRVTPGVLAAEAYGIKTGSADLLKFAEANMGYQGDAAVKSAIALTHTGFY
SVGDMTQGLGWESYAYPVTEQTLLAGNAPAVSFQANPVTRFAVPKAMGEQRLYN
KTGSTGGFGAYVAFVPARGIAIVMLANRNYPIEARVKAAHAILSQLAE
**SEQ ID N° 14 :** 
MQQRRAFALLTLGSLLLAPCTYARGEAPLTAAVDGIIQPMLKEYRIPGMAVAVLKDG
KAHYFNYGVANRESGQRVSEQTLFEIGSVSKTLTATLGAYAAVKGGFELDDKVSQH
APWLKGSAFDGVTMAELATYSAGGLPLQFPDEVDSNDKMRTYYRHWSPVYPAGT
HRQYSNPSIGLFGHLAANSLGQPFEQLMSQTLLPKLGLHHTYIQVPESAIANYAYGY
SKEDKPVRVTPGVLAAEAYGIKTGSADLLKFTEANMGYQGDAALKTRIALTHTGFYS
VGDMTQGLGWESYAYPLTEQALLAGNSPAVSFQANPVTRFAVPKAMGEQRLYNKT
GSTGGFGAYVAFVPARGIAIVMLANRNYPIEARVKAAHAILSQLAE
**SEQ ID N° 715** : 
MQQRRALALLTLGSLLLAPCTYASGEAPLTAAVDGIIQPMLKEYRIPGMAVAVLKDG
KAHYFNYGVANRESGQRVSEQTLFEIGSVSKTLTATLGAYAAVKGGFELDDKVSHH
APWLKGSAFDGVTMAELATYSAGGLPLQFPDEVDSNDKMQTYYRSWSPVYPAGT
HRQYSNPSIGLFGHLAANSLGQPFEKLMSQTLLPKLGLHHTYIQVPESAMANYAYG
YSKEDKPIRVTPGVLAAEAYGIKTGSADLLKFVEANMGYQGDAALKSAIALTHTGFY
SVGDMTQGLGWESYAYPVTEQALLAGNSPAVSFQANPVTRFAVPKAMGEQRLYN
KTGSTGGFGAYVAFVPARGIAIVMLANRNYPIEARVKAAHAILSQLAE
**SEQ ID N°** 716 : 
MQQRRAFALLTLGSLLLAPCTYASGEAPLTATVDGIIQPMLKEYRIPGIAVAVLKDGK
AHYFNYGVANRESGQRVSEQTLFEIGSVSKTLTATLGAYAAVKGGFVLDDKVSQHA
PWLKGSALDGVTMAELATYSAGGLPLQFPDKVDSNDKMQTYYRSWSPVYPAGTH
RQYSNPSIGLFGHLAANSLGQPFEQLMSQTLLPKLGLHHTYIQVPESAMANYAYGY
SKEDKPIRVTPGVLAAEAYGIKTGSADLLKFAEANMGYQGDALVKSAIALTHTGFYS
VGEMTQGLGWESYDYPVTEQVLLAGNSPAVSFQANPVTRFAVPKAMGEQRLYNK
TGSTGGFGAYVAFVPARGIAIVMLANRNYPIEARVKAAHAILSQLAE
**SEQ ID N°** 717 : 
MQQRRALALLMLGSLLLAPCTYASGEAPLTATVDGIIQPMLKAYRIPGMAVAVLKDG
KAHYFNYGVANRESGQRVSEQTLFEIGSVSKTLTATLGAYAAVKGGFELDDKVSQH
APWLKGSAFDGVTMAELATYSAGGLPLQFPDEVDSNDKMQTYYRSWSPVYPAGT
HRQYSNPSIGLFGHLAANSLGQPFEQLMSQTLLPKLGLHHTYIQVPESAMANYAYG
YSKEDKPIRATPGVLAAEAYGIKTGSADLLKFVEANMGYQGDAALKSAIALTHTGFH
SVGEMTQGLGWESYDYPVTEQVLLAGNSPAVSFQANPVTRFAVPKAMGEQRLYN
KTGSTGGFGAYVAFVPARGIAIVMLANRNYPIEARVKAAHAILSQLAE
**SEQ ID N°** 718 : 
MQQRRAFALLTLGSLLLAPCTYARGEAPLTAAVDGIIQPMLKEYRIPGMAVAVLKDG
KAHYFNYGVANRESGQRVSEQTLFEIGSVSKTLTATLGAYAAVKGGFELDDKVSQH
APWLKGSAFDGVTMAELATYSAGGLPLQFPDEVDSNDKMRTYYRHWSPVYPAGT
HRQYSNPSIGLFGHLAANSLGQPFEQLMSQTLLPKLGLHHTYIQVPESAIANYAYGY
SKEDKPVRATPGVLAAEAYGIKTGSADLLKFTEANMGYQGDAALKSAIALTHTGFYS
VGDMTQGLGWESYAYPLTEQALLAGNSPAVSFQANPVTRFAVPKAMGEQRLYNKT
GSTGGFGAYVAFVPARGIAIVMLANRNYPIEARVKAAHAILSQLAE
**SEQ ID N°1920** : 
MQQRRAFALLTLGSLLLAPCTYASGEAPLTATVDGIIQPMLKEYRIPGIAVAVLKDGK
AHYFNYGVANRESGQRVSEQTLFEIGSVSKTLTATLGAYAAVKGGFVLDDKVSQHA
PWLKGSALDGVTMAELATYSAGGLPLQFPDKVDSNDKMQTYYRSWSPVYPAGTH
RQYSNPSIGLFGHLAANSLGQPFEQLMSQTLLPKLGLHHTYIQVPESAMANYAYGY
SKEDKPIRVTPGVLAAEAYGIKTGSADLLKFAEANMGYQGDALVKSAIALTHTGFYS
VGEMTQGLGWESYDYPVTEQVLLAGNSPAVSLQANPVTRFAVPKAMGEQRLYNKT
GSTGGFGAYVAFVPARGIAIVMLANRNYPIEARVKAAHAILSQLAE
**SEQ ID N°1921** : 
MQQRRAFALLTLGSLLLAPCTYASGEAPLTVTVDGIIQPMLKAYRIPGMAVAVLKDG
KAHYFNYGVANRESGQRVSEQTLFEIGSVSKTLTATLGAYAAVKGGFELDDKVSQH
APWLKGSAFDGVTMAELATYSAGGLPLQFPEEVDSNDKMRTYYRSWSPVYPAGT
HRQYANTSIGLFGYLAANSLGQSFEQLMSQTLLPKLGLHHTYIQVPESAMANYAYG
YSKEEKPIRVTPGMLAAEAYGIKTGSADLLKFAEANMGYQGDAAVKSAIALTHTGFY
SVGDMTQGLGWESYDYPVTEQVLLADNSPAVSFQANPVTRFAVPKAMGEQRLYN
KTGSTGGFGAYVAFVPARGIAIVMLANRNYPIEARVKAAHAILSQLAE
**SEQ ID N°1922** : MQQRRAFALLTLGSLLLAPCTYASGEAPLTATVDGIIQPMLKEYRIPGIAVAVLKDGK 
AHYFNYGVANRESGQRVSEQTLFEIGSVSKTLTATLGAYAAVKGGFVLDDKVSQHA
PWLKGSALDGVTMAELATYSAGGLPLQFPDKVDSNDKMQTYYRSWSPVYPAGTH
RQYSNPSIGLFGHLAANSLGQPFEQLMSQTLLPKLGLHHTYIQVPESAMANYAYGY
SKEDKPIRVTPGVLAAEAYGIKTGSADLLKFAEANMGYQGDALVKSAIALTHTGFYS
VGEMTQGLGWESYDYPVTEQVLLAGNSPAVSFQANPVTRFAVPKAMGEQRLYNK
TGSTGGFGAYVAFVPARGIAIVMLANRNYPIEARVKAAHAILSQLAE
lesdits peptides étant choisis, de préférence, parmi les peptides de séquence **SEQ ID N° 719** à **SEQ ID N° 733** et **SEQ ID N° 2097** à **SEQ ID N° 2113** tels que définis ci-après :

| **Peptide SEQ ID N°** | **Séquence d'acides aminés** | **Position du peptide dans la protéine FOX** |
|---|---|---|
| **SEQ ID N°719** | **AHYFNYGVANR** | 59-69 pour les protéines de SEQ N°712, 713, 714, 715, 716, 717, 718, 1920, 1921, 1922 |
| **SEQ I**D **N°720** | **AMGEQR** | 326-331 pour les protéines de SEQ N°712, 713, 714, 715, 716, 717, 718, 1920, 1921, 1922 |
| **SEQ ID N°721** | **ESGQR** | 70-74 pour les protéines de SEQ N°712, 713, 714, 715, 716, 717, 718, 1920, 1921, 1922 |
| **SEQ ID N°722** | **FAVPK** | 321-325 pour les protéines de SEQ N°712, 713, 714, 715, 716, 717, 718, 1920, 1921, 1922 |
| **SEQ ID N°723** | **GGFELDDK** | 102-109 pour les protéines de SEQ N°712, 713, 714, 715, 717, 718, 1921 |
| **SEQ** ID **N°724** | **GIAIVMLANR** | 353-362 pour les protéines de SEQ N°712, 713, 714, 715, 716, 717, 718, 1920, 1921, 1922 |
| **SEQ ID N°725** | **IPGMAVAVLK** | 46-55 pour les protéines de SEQ N°712, 713, 714, 715, 717, 718, 1921 |
| **SEQ ID N°726** | **NYPIEAR** | 363-369 pour les protéines de SEQ N°712, 713, 714, 715, 716, 717, 718, 1920, 1921, 1922 |
| **SEQ ID N°727** | **SWSPVYPAGTHR** | 158-169 pour les protéines de SEQ N°712, 713, 715, 716, 717, 1920, 1921, 1922 |
| **SEQ ID N°728** | **TGSADLLK** | 247-254 pour les protéines de SEQ N°712, 713, 714, 715, 716, 717, 718, 1920, 1921, 1922 |
| **SEQ ID N°729** | **TGSTGGFGAYVAFVPAR** | 336-352 pour les protéines de SEQ N°712, 713, 714, 715, 716, 717, 718, 1920, 1921, 1922 |
| **SEQ ID N°730** | **TLTATLGAYAAVK** | 89-101 pour les protéines de SEQ N°712, 713, 714, 715, 716, 717, 718, 1920, 1921, 1922 |
| **SEQ ID N°731** | **VSEQTLFEIGSVSK** | 75-88 pour les protéines de SEQ N°712, 713, 714, 715, 716, 717, 718, 1920, 1921, 1922 |
| **SEQ ID N°732** | **VSQHAPWLK** | 110-118 pour les protéines de SEQ N°712, 713, 714, 716, 717,718,1920,1921,1922 |
| **SEQ ID N°733** | **VTPGVLAAEAYGIK** | 233-246 pour les protéines de SEQ N°712, 713, 714, 715, 716, 1920, 1922 |
| **SEQ ID N°2097** | AFALLTLGSLLLAPCTYAR | 6-24 pour les protéines de SEQ N°714, 718 |
| **SEQ ID N°2098** | **ATPGVLAAEAYGIK** | 233-246 pour les protéines de SEQ N°717, 718 |
| **SEQ ID N°2099** | EDKPVR | 227-232 pour les protéines de SEQ N°712, 713,714,718 |
| **SEQ ID N°2100** | EEKPIR | 227-232 pour les protéines de SEQ N°1921 |
| **SEQ ID N°2101** | FAEANMGYQGDAAVK | 255-269 pour les protéines de SEQ N°712, 713, 1921 |
| **SEQ ID N°2102** | FAEANMGYQGDALVK | 255-269 pour les protéines de SEQ N°716, 1920, 1922 |
| **SEQ ID N°2103** | FTEANMGYQGDAALK | 255-269 pour les protéines de SEQ N°714, 718 |
| **SEQ ID N°2104** | FVEANMGYQGDAALK | 255-269 pour les protéines de SEQ N°715, 717 |
| **SEQ ID N°2105** | GEAPLTAAVDGIIQPMLK | 25-42 pour les protéines de SEQ N°712, 713, 714, 715, 718 |
| **SEQ ID N°2106** | GGFVLDDK | 102-109 pour les protéines de SEQ N°716, 1920, 1922 |
| SEQ **ID N°2107** | HWSPVYPAGTHR | 158-169 pour les protéines de SEQ N°714, 718 |
| **SEQ ID N°2108** | IPGIAVAVLK | 46-55 pour les protéines de SEQ N°716, 1920, 1922 |
| **SEQ ID N°2109** | LMSQTLLPK | 194-202 pour les protéines de SEQ N°712, 713,714,715,716,717,718,1920,1921, 1922 |
| **SEQ ID N°2110** | MQTYYR | 152-157 pour les protéines de SEQ N°715, 716, 717, 1920, 1922 |
| **SEQ ID N°2111** | VDSNDK | 146-151 pour les protéines de SEQ N°712, 713, 714, 715, 716, 717, 718, 1920, 1921, 1922 |
| **SEQ ID N°2112** | VSHHAPWLK | 110-118 pour la protéine de SEQ N°715 |
| **SEQ ID N°2113** | VTPGMLAAEAYGIK | 233-246 pour les protéines de SEQ N°1921 |

La détection d'un mécanisme de résistance aux céphalosporines induit par l'expression de la protéine ACC est caractérisée par la détection d'au moins un peptide appartenant à la protéine ACC et à ses différents variants de séquences **SEQ ID N° 736** à **SEQ ID N°739.**
**SEQ ID N°736:** 
MRKKMQNTLKLLSVITCLAATAQGAMAANIDESKIKDTVDGLIQPLMQKNNIPGMSV
AVTIRGRNYIYNYGLAAKQPQQPVTENTLFEVGSLSKTFAAILASYAQASGKLSLEQS
VSHYVPELRGSSFDHVSVLNVGTHTSGLQLFMPEDIKNTTQLMTYLKAWKPADAAG
THRVYSNIGTGLLGMIAAKSLGVSYEDAIEQTILPLLGMNQTYLKVPADQMENYAWG
YNKKDEPVHVNMEILGNEAYGIKTTSSDLLRYVQANMGQLKLDGNAKIQHALTATHT
GYFKSGEITQDLMWEQLPYPVSLPNLLTGNDMAMTKSVATPIVPPLPPQENVWINK
TGSTNGFGAYIAFVPAKKMGIVMLANKNYSIDQRVTVAYKILSSLEVNK
**SEQ ID N°737:** 
MRKKMQNTLKMLSVITCLALTAQGAMASEMDQAKIKDTVDSLIQPLMQKNNIPGMS
VAVTLNGKNYIYNYGLASKQPQQPVTDNTLFEVGSLSKTFAATLASYAQVSGKLSLD
KSISHYVPELRGSSFDHISVLNAGTHTTGLALFMPEEVKNTDQLMAYLKAWKPADPA
GTHRVYSNIGTGLLGMIAAQSMGMTYEDAIEKTLLPKLGMTHTYLNVPADQAENYA
WGYNKKNEPIHVNMEVLGNEAYGIRTNASDLIRYVQANMGQLKLDGNSTLQKALTD
THIGYFKSGKITQDLMWEQLPYPVSLPDLLTGNDMAMTKSVATPIVPPLPPQENVWI
NKTGSTNGFGAYIAFVPAKKMGIVMLANKNYSIDQRVT
**SEQ ID N°738:** 
MQNTLKLLSVITCLAATVQGALAANIDESKIKDTVDDLIQPLMQKNNIPGMSVAVTVN
GKNYIYNYGLAAKQPQQPVTENTLFEVGSLSKTFAATLASYAQVSGKLSLDQSVSH
YVPELRGSSFDHVSVLNVGTHTSGLQLFMPEDIKNTTQLMAYLKAWKPADAAGTHR
VYSNIGTGLLGMIAAKSLGVSYEDAIEKTLLPQLGMHHSYLKVPADQMENYAWGYN
KKDEPVHGNMEILGNEAYGIKTTSSDLLRYVQANMGQLKLDANAKMQQALTATHTG
YFKSGEITQDLMWEQLPYPVSLPNLLTGNDMAMTKSVATPIVPPLPPQENVWINKT
GSTNGFGAYIAFVPAKKMGIVMLANKNYSIDQRVTVAYKILSSLEGNK
**SEQ ID N°739:** 
MQNTLKLLSVITCLAATVQGALAANIDESKIKDTVDDLIQPLMQKNNIPGMSVAVTVN
GKNYIYNYGLAAKQPQQPVTENTLFEVGSLSKTFAATLASYAQVSGKLSLDQSVSH
YVPELRGSSFDHVSVLNVGTHTSGLQLFMPEDIKNTTQLMAYLKAWKPADAAGTHR
VYSNIGTGLLGMIAAKSLGVSYEDAIEKTLLPQLGMHHSYLKVPADQMENYAWGYN
KKDEPVHVNMEILGNEAYGIKTTSSDLLRYVQANMGQLKLDANAKMQQALTATHTG
YFKSGEITQDLMWEQLPYPVSLPNLLTGNDMAMTKSVATPIVPPLPPQENVWINKT
GSTNGFGAYIAFVPAKKMGIVMLANKNYSIDQRVTVAYKILSSLEGNK
lesdits peptides étant choisis, de préférence, parmi les peptides de séquence **SEQ ID N° 740** à **SEQ ID N° 787** tels que définis ci-après :

| **Peptide SEQ ID N°** | **Séquence d'acides aminés** | **Position du peptide dans la ou les protéines ACC** |
|---|---|---|
| **SEQ ID N°740** | ALTDTHIGYFK | 279-289 pour la protéine de SEQ N°737 |
| **SEQ ID N°741** | AWKPADAAGTHR | 159-170 pour les protéines de SEQ N°738, 739; 163-174 pour la protéine de séquence SEQ ID N°736 |
| **SEQ ID N°742** | AWKPADPAGTHR | 163-174 pour la protéine de SEQ N°737 |
| **SEQ ID N°743** | DEPVHGNMEILGNEAYGIK | 229-247 pour la protéine de SEQ N°738 |
| **SEQ ID N°744** | DEPVHVNMEILGNEAYGIK | 229-247 pour la protéine de SEQ N°739; 233-251 pour la protéine de séquence SEQ ID N°736 |
| **SEQ ID N°745** | DTVDDLIQPLMQK | 33-45 pour les protéines de SEQ N°738, 739 |
| **SEQ ID N°746** | DTVDGLIQPLMQK | 37-49 pour la protéine de SEQ N°736 |
| **SEQ ID N°747** | DTVDSLIQPLMQK | 37-49 pour la protéine de SEQ N°737 |
| **SEQ ID N°748** | IQHALTATHTGYFK | 276-289 pour la protéine de SEQ N°736 |
| **SEQ ID N°749** | LDANAK | 266-271 pour les protéines de SEQ N°738, 739 |
| **SEQ ID N°750** | LDGNAK | 270-275 pour la protéine de SEQ N°736 |
| **SEQ ID N°751** | LDGNSTLQK | 270-278 pour la protéine de SEQ N°737 |
| **SEQ ID N°752** | LGMTHTYLNVPADQAENYAWGYNK | 208-231 pour la protéine de SEQ N°737 |
| **SEQ ID N°753** | LLSVITCLAATAQGAMAANIDESK | 11-34 pour la protéine de SEQ N°736 |
| **SEQ ID N°754** | LLSVITCLAATVQGALAANIDESK | 7-30 pour les protéines de SEQ N°738, 739 |
| **SEQ ID N°755** | LSLDK | 110-114 pour la protéine de SEQ N°737 |
| **SEQ ID N°756** | LSLDQSVSHYVPELR | 106-120 pour les protéines de SEQ N°738, 739 |
| **SEQ ID N°757** | LSLEQSVSHYVPELR | 110-124 pour la protéine de SEQ N°736 |
| **SEQ ID N°758** | MGIVMLANK | 356-364 pour les protéines de SEQ N°738, 739; 360-368 pour les protéines de séquence SEQ ID N°736, 737 |
| **SEQ ID N°759** | MLSVITCLALTAQGAMASEMDQAK | 11-34 pour la protéine de SEQ N°737 |
| **SEQ ID N°760** | MQNTLK | 1-6 pour les protéines de SEQ N°738, 739; 5-10 pour les protéines de séquence SEQ ID N°736, 737 |
| **SEQ ID N°761** | MQQALTATHTGYFK | 272-285 pour les protéines de SEQ N°738, 739 |
| **SEQ ID N°762** | NEPIHVNMEVLGNEAYGIR | 233-251 pour la protéine de SEQ N°737 |
| **SEQ ID N°763** | NNIPGMSVAVTIR | 50-62 pour la protéine de SEQ N°736 |
| **SEQ ID N°764** | NNIPGMSVAVTLNGK | 50-64 pour la protéine de SEQ N°737 |
| **SEQ ID N°765** | NNIPGMSVAVTVNGK | 46-60 pour les protéines de SEQ N°738, 739 |
| **SEQ ID N°766** | NTDQLMAYLK | 153-162 pour la protéine de SEQ N°737 |
| **SEQ ID N°767** | NTTQLMAYLK | 149-158 pour les protéines de SEQ N°738, 739 |
| **SEQ ID N°768** | NTTQLMTYLK | 153-162 pour la protéine de SEQ N°736 |
| **SEQ ID N°769** | NYIYNYGLAAK | 61-71 pour les protéines de SEQ N°738, 739; 65-75 pour la protéine de séquence SEQ ID N°736 |
| **SEQ ID N°770** | NYIYNYGLASK | 65-75 pour la protéine de SEQ N°737 |
| **SEQ ID N°771** | NYSIDQR | 365-371 pour les protéines de SEQ N°738, 739; 369-375 pour la protéine de séquence SEQ ID N°736, 737 |
| **SEQ ID N°772** | QPQQPVTDNTLFEVGSLSK | 76-94 pour la protéine de SEQ N°737 |
| **SEQ ID N°773** | QPQQPVTENTLFEVGSLSK | 72-90 pour les protéines de SEQ N°738, 739; 76-94 pour la protéine de séquence SEQ ID N°736 |
| **SEQ ID N°774** | SISHYVPELR | 115-124 pour la protéine de SEQ N°737 |
| **SEQ ID N°775** | SLGVSYEDAIEK | 187-198 pour les protéines de SEQ N°738, 739 |
| **SEQ ID N°776** | SVATPIVPPLPPQENVWINK | 318-337 pour les protéines de SEQ N°738, 739; 322-341 pour la protéine de séquence SEQ ID N°736, 737 |
| **SEQ ID N°777** | TFAAILASYAQASGK | 95-109 pour la protéine de SEQ N°736 |
| **SEQ ID N°778** | TFAATLASYAQVSGK | 91-105 pour les protéines de SEQ N°738, 739; 95-109 pour la protéine de séquence SEQ ID N°737 |
| **SEQ ID N°779** | TGSTNGFGAYIAFVPAK | 338-354 pour les protéines de SEQ N°738, 739; 342-358 pour la protéine de séquence SEQ ID N°736, 737 |
| **SEQ ID N°780** | TLLPK | 203-207 pour la protéine de SEQ N°737 |
| **SEQ ID N°781** | TLLPQLGMHHSYLK | 199-212 pour les protéines de SEQ N°738, 739 |
| **SEQ ID N°782** | TNASDLIR | 252-259 pour la protéine de SEQ N°737 |
| **SEQ ID N°783** | TTSSDLLR | 248-255 pour les protéines de SEQ N°738, 739; 252-259 pour la protéine de séquence SEQ ID N°736 |
| **SEQ ID N°784** | VPADQMENYAWGYNK | 213-227 pour les protéines de SEQ N°738, 739; 217-231 pour la protéine de séquence SEQ ID N°736 |
| **SEQ ID N°785** | VTVAYK | 372-377 pour les protéines de SEQ N°738, 739; 376-381 pour la protéine de séquence SEQ ID N°736 |
| **SEQ ID N°786** | VYSNIGTGLLGMIAAK | 171-186 pour les protéines de SEQ N°738, 739; 175-190 pour la protéine de séquence SEQ ID N°736 |
| **SEQ ID N°787** | YVQANMGQLK | 256-265 pour les protéines de SEQ N°738, 739; 260-269 pour la protéine de séquence SEQ ID N°736, 737 |

La détection d'un mécanisme de résistance aux céphalosporines induit par l'expression de la protéine ACT est caractérisée par la détection d'au moins un peptide appartenant à la protéine ACT et à ses différents variants de séquences **SEQ ID N° 788** à **SEQ ID N°794.**
**SEQ ID N°788:** 
MMMTKSLCCALLLSTSCSVLATPMSEKQLAEVVERTVTPLMKAQAIPGMAVAVIYE
GQPHYFTFGKADVAANKPVTPQTLFELGSISKTFTGVLGGDAIARGEISLGDPVTKY
WPELTGKQWQGIRMLDLATYTAGGLPLQVPDEVKDNASLLRFYQNWQPQWKPGT
TRLYANASIGLFGALAVKPSGMSYEQAITTRVFKPLKLDHTWINVPKAEEAHYAWGY
RDGKAVHVSPGMLDAEAYGVKTNVQDMASWVMVNMKPDSLQDNSLRKGLTLAQS
RYWRVGAMYQGLGWEMLNWPVDAKTVVEGSDNKVALAPLPAREVNPPAPPVNAS
WVHKTGSTGGFGSYVAFIPEKQLGIVMLANKSYPNPARVEAAYRILSAL
**SEQ ID N°789:** 
MMMTKSLCCALLLSTSCSVLATPMSEKQLAEVVERTVTPLMKAQAIPGMAVAVIYE
GQPHYFTFGKADVAANKPVTPQTLFELGSISKTFTGVLGGDAIARGEISLGDPVTKY
WPELTGKQWQGIRMLDLATYTAGGLPLQVPDEVTDNASLLRFYQNWQPQWKPGT
TRLYANASIGLFGALAVKPSGMSYEQAITTRVFKPLKLDHTWINVPKAEEAHYAWGY
RDGKAIHVSPGMLDAEAYGVKTNVQDMASWVMVNMKPDSLQDNSLRKGLTLAQS
RYWRVGAMYQGLGWEMLNWPVDAKTVVEGSDNKVALAPLPAREVNPPAPPVNAS
WVHKTGSTGGFGSYVAFIPEKQLGIVMLANKSYPNPARVEAAYRILSAL
**SEQ ID N°790:** 
MMTKSLCCALLLSTSCSVLAAPMSEKQLAEVVERTVTPLMKAQAIPGMAVAVIYQG
QPHYFTFGKADVAANKPVTPQTLFELGSISKTFTGVLGGDAIARGEISLGDPVTKYW
PELTGKQWQGIRMLDLATYTAGGLPLQVPDEVADNASLLRFYQNWQPQWKPGTT
RLYANTSIGLFGALAVKPSGMSYEQAITTRVFKPLKLDHTWINVPKAEEAHYAWGYR
DGKAVHVSPGMLDAEAYGVKTNVQDMASWVMVNMKPDSLQDNSLRQGIALAQSR
YWRVGAMYQGLGWEMLNWPVDAKTVVEGSDNKVALAPLPAREVNPPAPPVNAS
WVHKTGSTGGFGSYVAFIPEKQLGIVMLANKSYPNPARVEAAYRILSAL
**SEQ ID N°791:** 
MMTKSLCCALLLSTSCSVLAAPMSEKQLAEVVERTVTPLMKAQAIPGMAVAVIYQG
QPHYFTFGKADVAANKPVTPQTLFELGSISKTFTGVLGGDAIARGEISLGDPVTKYW
PELTGKQWQGIRMLDLATYTAGGLPLQVPDEVTDNASLLRFYQNWQPQWKPGTTR
LYANASIGLFGALAVKPSGMSYEQAITTRVFKPLKLDHTWINVPKAEEAHYAWGYRD
GKAVHVSPGMLDAEAYGVKTNVKDMANWVMVNMKPDSLQDSSLKEGITLAQSRY
WRVGAMYQGLGWEMLNWPVDAKTVVEGSDNKVALAPLPAREVNPPAPPVNASW
VHKTGSTGGFGSYVAFIPEKQLGIVMLANKSYPNPARVEAAYRILDALQ
**SEQ ID N°792:** 
MMRKSLCCALLLGISCSALATPVSEKQLAEVVANTVTPLMKAQSVPGMAVAVIYQG
KPHYYTFGKADIAANKPVTPQTLFELGSISKTFTGVLGGDAIARGEISLDDPVTRYWP
QLTGKQWQGIRMLDLATYTAGGLPLQVPDEVTDNASLLRFYQNWQPQWKPGTTRL
YANASIGLFGALAVKPSGMPYEQAMTTRVLKPLKLDHTWINVPKAEEAHYAWGYRD
GKAVRVSPGMLDAQAYGVKTNVQDMANWVMANMAPENVADASLKQGIALAQSRY
WRIGSMYQGLGWEMLNWPVEANTVVEGSDSKVALAPLPVAEVNPPAPPVKASWV
HKTGSTGGFGSYVAFIPEKQIGIVMLANTSYPNPARVEAAYHILEALQ
**SEQ ID N°793:** 
MMKKSLCCALLLGISCSALAAPVSEKQLAEVVANTVTPLMKAQSIPGMAVAVIYQGK
PHYYTFGKADIAASKPVTPQTLFELGSISKTFTGVLGGDAIARGEISLDDPVTRYWPQ
LTGKQWQGIRMLDLATYTAGGLPLQVPDEVTDNAALLRFYQNWQPQWKPGTTRLY
ANASIGLFGALAVKPSGMPFEQAMTTRVLKPLKLDHTWINVPKAEEAHYAWGYRDG
KAVRVSPGMLDAQAYGMKTNVQDMANWVMANMAPENVADASLKQGISLAQSRY
WRIGSMYQGLGWEMLNWPVEANTVIEGSDSKVALAPLPVAEVNPPAPPVKASWVH
KTGSTGGFGSYVAFIPEKQIGIVMLANKSYPNPARVEAAYPILDALQ
**SEQ ID N°794:** 
MMMTKSLCCALLLSTSCSVLATPMSEKQLAEVVERTVTPLMKAQAIPGMAVAVIYE
GQPHYFTFGKADVAANKPVTPQTLFELGSISKTFTGVLGGDAIARGEISLGDPVTKY
WPELTGKQWQGIRMLDLATYTAGGLPLQVPDEVTDNASLLRFYQNWQPQWKPGT
TRLYANASIGLFGALAVKPSGMSYEQAITTRVFKPLKLDHTWINVPKAEEAHYAWGY
RDGKAVHVSPGMLDAEAYGVKTNVQDMASWVMVNMKPDSLQDNSLRKGLTLAQS
RYWRVGAMYQGLGWEMLNWPVDAKTVVEGSDNKVALAPLPAREVNPPAPPVNAS
WVHKTGSTGGFGSYVAFIPEKQLGIVMLANKSYPNPARVEAAYRILSAL
lesdits peptides étant choisis, de préférence, parmi les peptides de séquence **SEQ ID N° 795** à **SEQ ID N° 841** tels que définis ci-après :

| **Peptide SEQ ID N°** | **Séquence d'acides aminés** | **Position du peptide dans la ou les protéines ACT** |
|---|---|---|
| **SEQ ID N°795** | ADIAANK | 66-72 pour la protéine de SEQ N°792 |
| **SEQ ID N°796** | ADIAASK | 66-72 pour la protéine de SEQ N°793 |
| **SEQ ID N°797** | ADVAANK | 67-73 pour les protéines de SEQ N°788, 789, 794; 66-72 pour la protéine de séquence SEQ ID N°790, 791 |
| **SEQ ID N°798** | AEEAHYAWGYR | 215-225 pour les protéines de SEQ N°788, 789, 794; 214-224 pour les protéines de séquence SEQ ID N°790, 791, 792, 793 |
| **SEQ ID N°799** | AIHVSPGMLDAEAYGVK | 229-245 pour la protéine de SEQ N°789 |
| **SEQ ID N°800** | AQSIPGMAVAVIYQGK | 42-57 pour la protéine de SEQ N°793 |
| **SEQ ID N°801** | AQSVPGMAVAVIYQGK | 42-57 pour la protéine de SEQ N°792 |
| **SEQ ID N°802** | AVHVSPGMLDAEAYGVK | 229-245 pour les protéines de SEQ N°788, 794; 228-244 pour les protéines de séquence SEQ ID N°790, 791 |
| **SEQ ID N°803** | DMANWVMVNMK | 249-259 pour la protéine de SEQ N°791 |
| **SEQ ID N°804** | DMANWVMVNMKPDSLQDSSLK | 249-269 pour la protéine de SEQ N°791 |
| **SEQ ID N°805** | DNASLLR | 148-154 pour les protéines de SEQ N°788, 789, 794; 147-153 pour les protéines de séquence SEQ ID N°790, 791,792 |
| **SEQ ID N°806** | EGITLAQSR | 270-278 pour la protéine de SEQ N°791 |
| **SEQ ID N°807** | EVNPPAPPVNASWVHK | 321-336 pour les protéines de SEQ N°788, 789, 794; 320-335 pour les protéines de séquence SEQ ID N°790, 791 |
| **SEQ ID N°808** | FYQNWQPQWK | 155-164 pour les protéines de SEQ N°788, 789, 794; 154-163 pour les protéines de séquence SEQ ID N°790, 791, 792, 793 |
| **SEQ ID N°809** | FYQNWQPQWKPGTTR | 155-169 pour les protéines de SEQ N°788, 789, 794; 154-168 pour les protéines de séquence SEQ ID N°790, 791, 792, 793 |
| **SEQ ID N°810** | GEISLDDPVTR | 101-111 pour les protéines de SEQ N°792, 793 |
| **SEQ ID N°811** | GEISLGDPVTK | 102-112 pour les protéines de SEQ N°788, 789, 794; 101-111 pour les protéines de séquence SEQ ID N°790, 791 |
| **SEQ ID N°812** | GLTLAQSR | 272-279 pour les protéines de SEQ N°788, 789, 794 |
| **SEQ ID N°813** | LDHTWINVPK | 205-214 pour les protéines de SEQ N°788, 789, 794; 204-213 les protéines de séquence SEQ ID N°791, 792, 793 |
| **SEQ ID N°814** | LYANASIGLFGALAVK | 170-185 pour les protéines de SEQ N°788, 789, 794; 169-184 pour les protéines de séquence SEQ ID N°791, 792,793 |
| **SEQ ID N°815** | LYANTSIGLFGALAVK | 169-184 pour la protéine de SEQ N°790 |
| **SEQ ID N°816** | MLDLATYTAGGLPLQVPDEVK | 127-147 pour la protéine de SEQ N°788 |
| **SEQ ID N°817** | QGIALAQSR | 270-278 pour les protéines de SEQ N°790, 792 |
| **SEQ ID N°818** | QGISLAQSR | 270-278 pour la protéine de SEQ N°793 |
| **SEQ ID N°819** | QIGIVMLANK | 353-362 pour la protéine de SEQ N°793 |
| **SEQ ID N°820** | QIGIVMLANTSYPNPAR | 353-369 pour la protéine de SEQ N°792 |
| **SEQ ID N°821** | QLAEVVANTVTPLMK | 27-41 pour les protéines de SEQ N°792, 793 |
| **SEQ ID N°822** | QLAEVVER | 28-35 pour les protéines de SEQ N°788, 789, 794; 27-34 pour les protéines de séquence SEQ ID N°790, 791 |
| **SEQ ID N°823** | QLGIVMLANK | 354-363 pour les protéines de SEQ N°788, 789, 794; 353-362 pour les protéines de séquence SEQ ID N°790, 791 |
| **SEQ ID N°824** | SYPNPAR | 364-370 pour les protéines de SEQ N°788, 789, 794; 363-369 pour les protéines de séquence SEQ ID N°790, 791,792,793 |
| **SEQ ID N°825** | TFTGVLGGDAIAR | 89-101 pour les protéines de SEQ N°788, 789, 794; 88-100 pour les protéines de séquence SEQ ID N°790, 791, 792, 793 |
| **SEQ ID N°826** | TGSTGGFGSYVAFIPEK | 337-353 pour les protéines de SEQ N°788, 789, 794; 336-352 pour les protéines de séquence SEQ ID N°790, 791, 792, 793 |
| **SEQ ID N°827** | TNVQDMASWVMVNMK | 246-260 pour les protéines de SEQ N°788, 789, 794; 245-259 pour la protéine de séquence SEQ ID N°790 |
| **SEQ ID N°828** | TVTPLMK | 36-42 pour les protéines de SEQ N°788, 789, 794; 35-41 pour les protéines de séquence SEQ ID N°790, 791, 792, 793 |
| **SEQ ID N°829** | TWEGSDNK | 303-311 pour les protéines de SEQ N°788, 789, 794; 302-310 pour les protéines de séquence SEQ ID N°790, 791 |
| **SEQ ID N°830** | VALAPLPAR | 312-320 pour les protéines de SEQ N°788, 789, 794; 311-319 pour les protéines de séquence SEQ ID N°790, 791 |
| **SEQ ID N°831** | VALAPLPVAEVNPPAPPVK | 311-329 pour les protéines de SEQ N°792, 793 |
| **SEQ ID N°832** | VEAAYR | 371-376 pour les protéines de SEQ N°788, 789, 794; 370-375 pour les protéines de séquence SEQ ID N°790, 791 |
| **SEQ ID N°833** | VFKPLK | 199-204 pour les protéines de SEQ N°788, 789, 794; 198-203 pour les protéines de séquence SEQ ID N°790, 791 |
| **SEQ ID N°834** | VGAMYQGLGWEMLNWPVDAK | 283-302 pour les protéines de SEQ N°788, 789, 794; 282-301 pour les protéines de séquence SEQ ID N°790, 791 |
| **SEQ ID N°835** | VLKPLK | 198-203 pour les protéines de SEQ N°792, 793 |
| **SEQ ID N°836** | VSPGMLDAQAYGMK | 231-244 pour la protéine de SEQ N°793 |
| **SEQ ID N°837** | VSPGMLDAQAYGVK | 231-244 pour la protéine de SEQ N°792 |
| **SEQ ID N°838** | YWPQLTGK | 112-119 pour les protéines de SEQ N°792, 793 |
| **SEQ ID N°839** | ASWVHK | 331-336 pour les protéines de SEQ N°788, 789, 794; 330-335 pour les protéines de séquence SEQ ID N°790, 791,792,793 |
| **SEQ ID N°840** | QWQGIR | 121-126 pour les protéines de SEQ N°788, 789, 794; 120-125 pour les protéines de séquence SEQ ID N°790, 791, 792, 793 |
| **SEQ ID N°841** | YWPELTGK | 113-120 pour les protéines de SEQ N°788, 789, 794; 112-119 pour les protéines de séquence SEQ ID N°790, 791 |

La détection d'un mécanisme de résistance aux céphalosporines induit par l'expression de la protéine CARB est caractérisée par la détection d'au moins un peptide appartenant à la protéine CARB et à ses différents variants de séquences **SEQ ID N° 842** à **SEQ ID N°852.**
**SEQ ID N°842:** 
MLLYKMCDNQNYGVTYMKFLLAFSLLIPSVVFASSSKFQQVEQDVKAIEVSLSARIG
VSVLDTQNGEYWDYNGNQRFPLTSTFKTIACAKLLYDAEQGKVNPNSTVEIKKADL
VTYSPVIEKQVGQAITLDDACFATMTTSDNTAANIILSAVGGPKGVTDFLRQIGDKET
RLDRIEPDLNEGKLGDLRDTTTPKAIASTLNKFLFGSALSEMNQKKLESWMVNNQVT
GNLLRSVLPAGWNIADRSGAGGFGARSITAVVWSEHQAPIIVSIYLAQTQASMAERN
DAIVKIGHSIFDVYTSQSR
**SEQ ID N°843:** 
MLLYKMCDNQNYGVTYMKFLLAFSLLIPSVVFASSSKFQQVEQDVKAIEVSLSARIG
VSVLDTQNGEYWDYNGNQRFPLTSTFKTIACAKLLYDAEQGKVNPNSTVEIKKADL
VTYSPVIEKQVGQAITLDDACFATMTTSDNTAANIILSAVGGPKGVTDFLRQIGDKET
RLDRIEPDLNEGKLGDLRDTTTPKAIASTLNKLLFGSALSEMNQKKLESWMVNNQVT
GNLLRSVLPAGWNIADRSGAGGFGARSITAVVWSEHQAPIIVSIYLAQTQASMAERN
DAIVKIGHSIFDVYTSQSR
**SEQ ID N°844:** 
MKLLLVFSLLIPSMVFANSSKFQQVEQDAKVIEASLSAHIGISVLDTQTGEYWDYNG
NQRFPLTSTFKTIACAKLLYDAEQGEINPKSTIEIKKADLVTYSPVIEKQVGQAITLDDA
CFATMTTSDNAAANIILNALGGPESVTDFLRQIGDKETRLDRIEPELNEGKLGDLRDT
TTPNAIVNTLNELLFGSTLSQDGQKKLEYWMVNNQVTGNLLRSVLPEGWNIADRSG
AGGFGARSITAVVWSEAQSPIIVSIYLAQTEASIADRNDAIVKIGRSIFEVYSSQSR
**SEQ ID N°845:** 
MKFLLAFSLLIPSVVFASSSKFQQVEQDVKAIEVSLSARIGVSVLDTQNGEYWDYNG
NQRFPLTSTFKTIACAKLLYDAEQGKVNPNSTVEIKKADLVTYSPVIEKQVGQAITLD
DACFATMTTSDNTAANIILSAVGGPKGVTDFLRQIGDKETRLDRIEPDLNEGKLGDLR
 
DTTTPKAIASTLNQLLFGSTLSEASQKKLESWMVNNQVTGNLLRSVLPVKWSIADRS
GAGGFGARSITAIVWSEEKKTIIVSIYLAQTEASMAERNDAIVKIGRSIFEVYTSQSR
**SEQ ID N°846:** 
MNVRKHKASFFSVVITFLCLTLSLNANATDSVLEAVTNAETELGARIGLAVHDLETGK
RWEHKSNERFPLSSTFKTLACANVLQRVDLGKERIDRVVRFSESNLVTYSPVTEKH
VGKKGMSLAELCQATLSTSDNSAANFILQAIGGPKALTKFLRSIGDDTTRLDRWETE
LNEAVPGDKRDTTTPIAMVTTLEKLLIDETLSIKSRQQLESWLKGNEVGDALFRKGV
PSDWIVADRTGAGGYGSRAITAVMWPPNRKPIVAALYITETDASFEERNAVIAKIGE
QIAKTVLMENSRN
**SEQ ID N°847:** 
MKFLLAFSLLIPSVVFASSSKFQQVEQDVKAIEVSLSARIGVSVLDTQNGEYWDYNG
NQRFPLTSTFKTIACAKLLYDAEQGKVNSNSTVEIKKADLVTYSPVIEKQVGQAITLD
DACFATMTTSDNTAANIILSAVGGPKGVTDFLRQIGDKETRLDRIEPDLNEGKLGDLR
DTTTPKAIASTLNKFLFGSALSEMNQKKLESWMVNNQVTGNLLRSVLPAGWNIADR
SGAGGFGARSITAVVWSEHQAPIIVSIYLAQTQASMAERNDAIVKIGHSIFDVYTSQS
R
**SEQ ID N°848:** 
MKFLLAFSLLIPSVVFASSSKFQQVEQDVKAIEVSLSARIGVSVLDTQNGEYWDYNG
NQRFPLTSTFKTIACAKLLYDAEQGKVNPNSTVEIKKADLVTYSPVIEKQVGQAITLD
DACFATMTTSDNTAANIILSAVGGPKGVTDFLRQIGDKETRLDRIEPDLNEGKLGDLR
DTTTPKAIASTLNKFLFGSALSEMNQKKLESWMVNNQVTGNLLRSVLPAGWNIADR
SGAGGFGARSITAVVWSEHQAPIIVSIYLAQTQASMEERNDAIVKIGHSIFDVYTSQS
R
**SEQ ID N°849:** 
MKSLLVFALLMPSVVFASSSKFQSVEQEIKGIESSLSARIGVAILDTQNGESWDYNG
DQRFPLTSTFKTIACAKLLYDAEHGKVNLNSTVEIKKADLVTYSPVLEKQVGKPITLS
DACLATMTTSDNTAANIVINAVGDPKSITDFLRQIGDKETRLDRVEPELNEGKLGDLR
DTTTPNAITSTLNQLLFGSTLSEASQKKLESWMVNNQVTGNLLRSVLPVKWSIADRS
GAGGFGARSITAIVWSEEKKPIIVSIYLAQTEASMAERNDAIVKIGRSIFEVYTSQSR
**SEQ ID N°850:** 
MDVRKHKASFFSVVITFLCLTLSLNANATDSVLEAVTNAETELGARIGLAVHDLETGK
RWEHKSNERFPLSSTFKTLACANVLQRVDLGKERIDRVVRFSESNLVTYSPVTEKH
VGKKGMSLAELCQATLSTSDNSAANFILQAIGGPKALTKFLRSIGDDTTRLDRWETE
LNEAVPGDKRDTTTPIAMVTTLEKLLIDETLSIKSRQQLESWLKGNEVGDALFRKGV
PSDWIVADRTGAGGYGSRAITAVMWPPNRKPIVAALYITETDASFEERNAVIAKIGE
QIAKTILMENSRN
**SEQ ID N°851:** 
MKSLLVFALLMPSVVFASSSKFQSVEQEIKGIESSLSARIGVAILDTQNGESWDYNG
DQRFPLTSTFKTIACAKLLYDAEHGKVNLNSTVEVKKADLVTYSPVLEKQVGKPITLS
DACFATMTTSDNTAANIVINAVGDPKSITDFLRQIGDKETRLDRVEPELNEGKLGDLR
DTTTPNAITSTLNQLLFGSTLSEASQKKLESWMVNNQVTGNLLRSVLPVTWSIADRS
GAGGFGARSITAIVWSEEKKPIIVSIYLAQTEASMAERNDAIVKIGRSIFEVYTSQSR
**SEQ ID N°852:** 
MKFLLVFSLLIPSVVFASSSKFRQVEQDVKAIEVSLSARIGVSVLDTQNGEYWDYNG
NQRFPLTSTFKTIACAKLLYDAEQGKVNPNSTVEIKKADLVTYSPVIEKQVGQAITLD
DACFATMTTSDNTAANIILSAVGGSKGVTDFLRQIGDKETRLDRIEPDLNEGKLGDLR
DTTTPKAIASTLNKFLFGSALSEMNKKKLESWMVNNQVTGNLLRSVLPAGWNIADR
SGAGGFGARSITAVVWSEHQAPIIVSIYLAQTQASMAERNDAIVKIGRSIFDVYTSQS
R
 lesdits peptides étant choisis, de préférence, parmi les peptides de séquence **SEQ ID N° 853** à **SEQ ID N° 921** tels que définis ci-après :

| **Peptide SEQ ID N°** | **Séquence d'acides aminés** | **Position du peptide dans la ou les protéines CARB** |
|---|---|---|
| **SEQ ID N°853** | ADLVTYSPVIEK | 95-106 pour les protéines de SEQ N°844, 845, 847, 848, 852; 111-122 pour la protéine de séquence SEQ ID N°842; 111-122 pour la protéine de séquence SEQ ID N°843 |
| **SEQ ID N°854** | ADLVTYSPVLEK | 95-106 pour les protéines de SEQ N°849, 851 |
| **SEQ ID N°855** | AIASTLNK | 180-187 pour les protéines de SEQ N°847, 848, 852; 196-203 pour la protéine de séquence SEQ ID N°842; 196-203 pour la protéine de séquence SEQ ID N°843 |
| **SEQ ID N°856** | AIASTLNQLLFGSTLSEASQK | 180-200 pour la protéine de SEQ N°845 |
| **SEQ ID N°857** | AIEVSLSAR | 31-39 pour les protéines de SEQ N°845, 847, 848, 852; 47-55 pour la protéine de séquence SEQ ID N°842; 47-55 pour la protéine de séquence SEQ ID N°843 |
| **SEQ ID N°858** | AITAVMWPPNR | 247-257 pour les protéines de SEQ N°846, 850 |
| **SEQ ID N°859** | DTTTPIAMVTTLEK | 182-195 pour les protéines de SEQ N°846, 850 |
| **SEQ ID N°860** | DTTTPK | 174-179 pour les protéines de SEQ N°845, 847, 848, 852; 190-195 pour la protéine de séquence SEQ ID N°842; 190-195 pour la protéine de séquence SEQ ID N°843 |
| **SEQ ID N°861** | FLFGSALSEMNK | 188-199 pour la protéine de SEQ N°852 |
| **SEQ ID N°862** | FLFGSALSEMNQK | 188-200 pour les protéines de SEQ N°847, 848; 204-216 pour la protéine de séquence SEQ ID N°842 |
| **SEQ ID N°863** | FLLAFSLLIPSWFASSSK | 3-21 pour les protéines de SEQ N°845, 847, 848; 19-37 pour la protéine de séquence SEQ ID N°842; 19-37 pour la protéine de séquence SEQ ID N°843 |
| **SEQ ID N°864** | FLLVFSLLIPSVVFASSSK | 3-21 pour la protéine de SEQ N°852 |
| **SEQ ID N°865** | FPLSSTFK | 68-75 pour les protéines de SEQ N°846, 850 |
| **SEQ ID N°866** | FPLTSTFK | 61-68 pour les protéines de SEQ N°844, 845, 847, 848, 849, 851, 852; 77-84 pour la protéine de séquence SEQ ID N°842; 77-84 pour la protéine de séquence SEQ ID N°843 |
| **SEQ ID N°867** | FQQVEQDAK | 22-30 pour la protéine de SEQ N°844 |
| **SEQ ID N°868** | FQQVEQDVK | 22-30 pour les protéines de SEQ N°845, 847, 848; 38-46 pour la protéine de séquence SEQ ID N°842; 38-46 pour la protéine de séquence SEQ ID N°843 |
| **SEQ ID N°869** | FQSVEQEIK | 22-30 pour les protéines de SEQ N°849, 851 |
| **SEQ ID N°870** | FSESNLVTYSPVTEK | 99-113 pour les protéines de SEQ N°846, 850 |
| **SEQ ID N°871** | GIESSLSAR | 31-39 pour les protéines de SEQ N°849, 851 |
| **SEQ ID N°872** | GNEVGDALFR | 216-225 pour les protéines de SEQ N°846, 850 |
| **SEQ ID N°873** | GVPSDWIVADR | 227-237 pour les protéines de SEQ N°846, 850 |
| **SEQ ID N°874** | GVTDFLR | 142-148 pour les protéines de SEQ N°845, 847, 848, 852; 158-164 pour la protéine de séquence SEQ ID N°842; 158-164 pour la protéine de séquence SEQ ID N°843 |
| **SEQ ID N°875** | IEPDLNEGK | 160-168 pour les protéines de SEQ N°845, 847, 848, 852; 176-184 pour la protéine de séquence SEQ ID N°842; 176-184 pour la protéine de séquence SEQ ID N°843 |
| **SEQ ID N°876** | IEPELNEGK | 160-168 pour la protéine de SEQ N°844 |
| **SEQ ID N°877** | IGEQIAK | 283-289 pour les protéines de SEQ N°846, 850 |
| **SEQ ID N°878** | IGLAVHDLETGK | 47-58 pour les protéines de SEQ N°846, 850 |
| **SEQ ID N°879** | IGVAILDTQNGESWDYNGDQR | 40-60 pour les protéines de SEQ N°849, 851 |
| **SEQ ID N°880** | IGVSVLDTQNGEYWDYNGNQR | 40-60 pour les protéines de SEQ N°845, 847, 848, 852; 56-76 pour la protéine de séquence SEQ ID N°842; 56-76 pour la protéine de séquence SEQ ID N°843 |
| **SEQ ID N°881** | KPIIVSIYLAQTEASMAER | 250-268 pour les protéines de SEQ N°849, 851 |
| **SEQ ID N°882** | KPIVAALYITETDASFEER | 258-276 pour les protéines de SEQ N°846, 850 |
| **SEQ ID N°883** | LESWMVNNQVTGNLLR | 202-217 pour les protéines de SEQ N°845, 847, 848, 849, 851, 852; 218-233 pour la protéine de séquence SEQ ID N°842; 218-233 pour la protéine de séquence SEQ ID N°843 |
| **SEQ ID N°884** | LEYWMVNNQVTGNLLR | 202-217 pour la protéine de SEQ N°844 |
| **SEQ ID N°885** | LGDLR | 169-173 pour les protéines de SEQ N°844, 845, 847, 848, 849, 851, 852; 185-189 pour la protéine de séquence SEQ ID N°842; 185-189 pour la protéine de séquence SEQ ID N°843 |
| **SEQ ID N°886** | LLFGSALSEMNQK | 204-216 pour la protéine de SEQ N°843 |
| **SEQ ID N°887** | LLIDETLSIK | 196-205 pour les protéines de SEQ N°846, 850 |
| **SEQ ID N°888** | LLLVFSLLIPSMVFANSSK | 3-21 pour la protéine de SEQ N°844 |
| **SEQ ID N°889** | LLYDAEHGK | 75-83 pour les protéines de SEQ N°849, 851 |
| **SEQ ID N°890** | LLYDAEQGEINPK | 75-87 pour la protéine de SEQ N°844 |
| **SEQ ID N°891** | LLYDAEQGK | 75-83 pour les protéines de SEQ N°845, 847, 848, 852; 91-99 pour la protéine de séquence SEQ ID N°842; 91-99 pour la protéine de séquence SEQ ID N°843 |
| **SEQ ID N°892** | MCDNQNYGVTYMK | 6-18 pour les protéines de SEQ N°842, 843 |
| **SEQ ID N°893** | NAVIAK | 277-282 pour les protéines de SEQ N°846, 850 |
| **SEQ ID N°894** | NDAIVK | 269-274 pour les protéines de SEQ N°844, 845, 847, 848, 849, 851, 852; 285-290 pour la protéine de séquence SEQ ID N°842; 285-290 pour la protéine de séquence SEQ ID N°843 |
| **SEQ ID N°895** | QIGDK | 149-153 pour les protéines de SEQ N°844, 845, 847, 848, 849, 851, 852; 165-169 pour la protéine de séquence SEQ ID N°842; 165-169 pour la protéine de séquence SEQ ID N°843 |
| **SEQ ID N°896** | QQLESWLK | 208-215 pour les protéines de SEQ N°846, 850 |
| **SEQ ID N°897** | QVEQDVK | 24-30 pour les protéines de SEQ N°845, 847, 848, 852; 40-46 pour la protéine de séquence SEQ ID N°842; 40-46 pour la protéine de séquence SEQ ID N°843 |
| **SEQ ID N°898** | SGAGGFGAR | 230-238 pour les protéines de SEQ N°844, 845, 847, 848, 849, 851, 852; 246-254 pour la protéine de séquence SEQ ID N°842; 246-254 pour la protéine de séquence SEQ ID N°843 |
| **SEQ ID N°899** | SIGDDTTR | 157-164 pour les protéines de SEQ N°846, 850 |
| **SEQ ID N°900** | SITAIVWSEEK | 239-249 pour les protéines de SEQ N°845, 849,851 |
| **SEQ ID N°901** | SITDFLR | 142-148 pour les protéines de SEQ N°849, 851 |
| **SEQ ID N°902** | SLLVFALLMPSVVFASSSK | 3-21 pour les protéines de SEQ N°849, 851 |
| **SEQ ID N°903** | STIEIK | 88-93 pour la protéine de SEQ N°844 |
| **SEQ ID N°904** | SVLPAGWNIADR | 218-229 pour les protéines de SEQ N°847, 848, 852; 234-245 pour la protéine de séquence SEQ ID N°842; 234-245 pour la protéine de séquence SEQ ID N°843 |
| **SEQ ID N°905** | SVLPEGWNIADR | 218-229 pour la protéine de SEQ N°844 |
| **SEQ ID N°906** | SVLPVK | 218-223 pour les protéines de SEQ N°845, 849 |
| **SEQ ID N°907** | SVLPVTWSIADR | 218-229 pour la protéine de SEQ N°851 |
| **SEQ ID N°908** | TGAGGYGSR | 238-246 pour les protéines de SEQ N°846, 850 |
| **SEQ ID N°909** | TIACAK | 69-74 pour les protéines de SEQ N°844, 845, 847, 848, 849, 851, 852; 85-90 pour la protéine de séquence SEQ ID N°842; 85-90 pour la protéine de séquence SEQ ID N°843 |
| **SEQ ID N°910** | TIIVSIYLAQTEASMAER | 251-268 pour la protéine de SEQ N°845 |
| **SEQ ID N°911** | TILMENSR | 290-297 pour la protéine de SEQ N°850 |
| **SEQ ID N°912** | TLACANVLQR | 76-85 pour les protéines de SEQ N°846, 850 |
| **SEQ ID N°913** | TVLMENSR | 290-297 pour la protéine de SEQ N°846 |
| **SEQ ID N°914** | VDLGK | 86-90 pour les protéines de SEQ N°846, 850 |
| **SEQ ID N°915** | VEPELNEGK | 160-168 pour les protéines de SEQ N°849, 851 |
| **SEQ ID N°916** | VNLNSTVEIK | 84-93 pour la protéine de SEQ N°849 |
| **SEQ ID N°917** | VNLNSTVEVK | 84-93 pour la protéine de SEQ N°851 |
| **SEQ ID N°918** | VNPNSTVEIK | 84-93 pour les protéines de SEQ N°845, 848, 852; 100-109 pour la protéine de séquence SEQ ID N°842; 100-109 pour la protéine de séquence SEQ ID N°843 |
| **SEQ ID N°919** | VNSNSTVEIK | 84-93 pour la protéine de SEQ N°847 |
| **SEQ ID N°920** | WETELNEAVPGDK | 168-180 pour les protéines de SEQ N°846, 850 |
| **SEQ ID N°921** | WSIADR | 224-229 pour les protéines de SEQ N°845, 849,851 |

La détection d'un mécanisme de résistance aux céphalosporines induit par l'expression de la protéine DHA est caractérisée par la détection d'au moins un peptide appartenant à la protéine DHA et à ses différents variants de séquences **SEQ ID N° 922** à **SEQ ID N°927.**
**SEQ ID N°922:** 
MKKSLSATLISALLAFSAPGFSAADNVAAVVDSTIKPLMAQQDIPGMAVAVSVKGKP
YYFNYGFADVQAKQPVTENTLFELGSVSKTFTGVLGAVSVAKKEMMLNDPAEKYQ
PELALPQWKGITLLDLATYTTGGLPLQVPDAVKNRAELLHFYQQWQPSRKPGDMRL
YANSSIGLFGALTANAAGMPYEQLLTARILAPLGLSHTFITVPESAQSQYAYGYKNKK
PVRVSPGQLDAESYGVKSASKDMLRWAEMNMEPSRAGNADLEMAMYLAQTRYYK
TAAINQGLGWEMYDWPQQKDMIINGVTNEVALQPHPVTDNQVQPYNRASWVHKT
GATTGFGAYVAFIPEKQVAIVILANKNYPNTERVKAAQAILSALE
**SEQ ID N°923:** 
MKKSLSATLISALLAFSAPGFSAADNVAAVVDSTIKPLMAQQDIPGMAVAVSVKGKP
YYFNYGFADIQAKQPVTENTLFELGSVSKTFTGVLGAVSVAKKEMALNDPAAKYQP
ELALPQWKGITLLDLATYTAGGLPLQVPDAVKSRADLLNFYQQWQPSRKPGDMRLY
ANSSIGLFGALTANAAGMPYEQLLTARILAPLGLSHTFITVPDSAQSQYAYGYKNKKP
VRVSPGQLDAESYGVKSASKDMLRWAEMNIEPSRAGNADLEMAMYLAQTRYYKTA
AINQGLGWEMYDWPQQKDMIINGVTNEVALQPHPVTDNQVQPYNRASWVHKTGA
TTGFGAYVAFIPEKQVAIVILANKNYPNTERVKAAQAILSALE
**SEQ ID N°924:** 
MKKSLSATLISALLAFSAPGFSAADNVAAVVDSTIKPLMAQQDIPGMAVAVSVKGKP
YYFNYGFADIQAKQPVTENTLFELGSVSKTFTGVLGAVSVAKKEMALNDPAAKYQP
ELALPQWKGITLLDLATYTAGGLPLQVPDAVKSRADLLNFYQQWQPSRKPGDMRLY
ANSSIGLFGALTANAAGMPYEQLLTARILAPLGLSHTFITVPESAQSQYAYGYKNKKP
VRVSPGQLDAESYGVKSASKDMLRWAEMNMEPSRAGNADLEMAMYLAQTRYYKT
AAINQGLGWEMYDWPQQKDMIINGVTNEVALQPHPVTDNQVQPYNRASWVHKTG
ATTGSGAYVAFIPEKQVAIVILANKNYPNTERVKAAQAILSALE
**SEQ ID N°925:** 
MKKSLSATLISALLAFSAPGFSAADNVAAVVDSTIKPLMAQQDIPGMAVAVSVKGKP
YYFNYGFADIQAKQPVTENTLFELGSVSKTFTGVLGAVSVAKKEMALNDPAAKYQP
ELALPQWKGITLLDLATYTAGGLPLQVPDAVKSRADLLNFYQQWQPSRKPGDMRLY
ANSSIGLFGALTANAAGMPYEQLLTARILAPLGLSHTFITVPESAQSQYAYGYKNKKP
VRVSPGQLDAESYGVKSTSKDMLRWAEMNMEPSRAGNADLEMAMYLAQTRYYKT
AAINQGLGWEMYDWPQQKDMIINGVTNEVALQPHPVTDNQVQPYNRASWVHKTG
ATTGFGAYVAFIPEKQVAIVILANKNYPNTERVKAAQAILSALE
**SEQ ID N°926:** 
MKKSLSATLISALLAFSAPGFSAADNVAAVVDSTIKPLMAQQDIPGMAVAVSVKGRP
YYFNYGFADVQAKQPVTENTLFELGSVSKTFTGVLGAVSVAKKEMALNDPAAKYQP
ELALPQWKGITLLDLATYTAGGLPLQVPDAVKSRADLLHFYQQWQPSRKPGDMRLY
ANSSIGLFGALTANAAGMPYEQLLTARILAPLGLSHTFITVPESAQSQYAYGYKNKKP
VRVSPGQLDAESYGVKSASKDMLRWAEMNMEPSRAGNADLEMAMYLAQTRYYKT
AAINQGLGWEMYDWPQQKDMIINGVTNEVALQPHPVTDNQVQPYNRASWVHKTG
ATTGFGAYVAFIPEKQVAIVILANKNYPNTERVKAAQAILSALE
**SEQ ID N°927:** 
MKKSLSATLISALLAFSAPGFSAADNVAAVVDSTIKPLMAQQDIPGMAVAVSVKGKP
YYFNYGFADIQAKQPVTENTLFELGSVSKTFTGVLGAVSVAKKEMALNDPAAKYQP
ELALPQWKGITLLDLATYTAGGLPLQVPDAVKSRADLLNFYQQWQPSRKPGDMRLY
ANSSIGLFGALTANAAGMPYEQLLTARILAPLGLSHTFITVPESAQSQYAYGYKNKKP
VRVSPGQLDAESYGVKSASKDMLRWAEMNMEPSRAGNADLEMAMYLAQTRYYKT
AAINQGLGWEMYDWPQQKDMIINGVTNEVALQPHPVTDNQVQPYNRASWVHKTG
ATTGFGAYVAFIPEKQVAIVILANKNYPNTERVKAAQAILSALE
 lesdits peptides étant choisis, de préférence, parmi les peptides de séquence **SEQ ID N° 928** à **SEQ ID N° 948** tels que définis ci-après :

| **Peptide SEQ ID N°** | **Séquence d'acides aminés** | **Position du peptide dans la ou les protéines DHA** |
|---|---|---|
| **SEQ ID N°928** | ADLLHFYQQWQPSR | 148-161 pour la protéine de SEQ N°926 |
| **SEQ ID N°929** | ADLLNFYQQWQPSR | 148-161 pour les protéines de SEQ N°923, 924, 925, 927 |
| **SEQ ID N°930** | AELLHFYQQWQPSR | 148-161 pour la protéine de SEQ N°922 |
| **SEQ ID N°931** | AGNADLEMAMYLAQTR | 262-277 pour les protéines de SEQ N°922, 923, 924, 925, 926, 927 |
| **SEQ ID N°932** | EMALNDPAAK | 101-110 pour les protéines de SEQ N°923, 924, 925, 926, 927 |
| **SEQ ID N°933** | EMMLNDPAEK | 101-110 pour la protéine de SEQ N°922 |
| **SEQ ID N°934** | GKPYYFNYGFADIQAK | 55-70 pour les protéines de SEQ N°923, 924, 925, 927 |
| **SEQ ID N°935** | GKPYYFNYGFADVQAK | 55-70 pour la protéine de SEQ N°922 |
| **SEQ ID N°936** | KPGDMR | 162-167 pour les protéines de SEQ N°922, 923, 924, 925, 926, 927 |
| **SEQ ID N°937** | NYPNTER | 361-367 pour les protéines de SEQ N°922, 923, 924, 925, 926, 927 |
| **SEQ ID N°938** | QPVTENTLFELGSVSK | 71-86 pour les protéines de SEQ N°922, 923, 924, 925, 926, 927 |
| **SEQ ID N°939** | QVAIVILANK | 351-360 pour les protéines de SEQ N°922, 923, 924, 925, 926, 927 |
| **SEQ ID N°940** | TAAINQGLGWEMYDWPQQK | 281-299 pour les protéines de SEQ N°922, 923, 924, 925, 926, 927 |
| **SEQ ID N°941** | TFTGVLGAVSVAK | 87-99 pour les protéines de SEQ N°922, 923, 924, 925, 926, 927 |
| **SEQ ID N°942** | TGATTGFGAYVAFIPEK | 334-350 pour les protéines de SEQ N°922, 923, 925, 926, 927 |
| **SEQ ID N°943** | TGATTGSGAYVAFIPEK | 334-350 pour la protéine de SEQ N°924 |
| **SEQ ID N°944** | VSPGQLDAESYGVK | 230-243 pour les protéines de SEQ N°922, 923, 924, 925, 926, 927 |
| **SEQ ID N°945** | WAEMNIEPSR | 252-261 pour la protéine de SEQ N°923 |
| **SEQ ID N°946** | WAEMNMEPSR | 252-261 pour les protéines de SEQ N°922, 924, 925, 926, 927 |
| **SEQ ID N°947** | YQPELALPQWK | 111-121 pour les protéines de SEQ N°922, 923, 924, 925, 926, 927 |
| **SEQ ID N°948** | ASWVHK | 328-333 pour les protéines de SEQ N°922, 923, 924, 925, 926, 927 |

La détection d'un mécanisme de résistance aux céphalosporines induit par l'expression de la protéine MIR est caractérisée par la détection d'au moins un peptide appartenant à la protéine MIR et à ses différents variants de séquences **SEQ ID N° 949** à **SEQ ID N°953.**
**SEQ ID N°949:** 
MMTKSLSCALLLSVASSAFAAPMSEKQLAEVVERTVTPLMNAQAIPGMAVAVIYQG
QPHYFTFGKADVAANKPVTPQTLFELGSISKTFTGVLGGDAIARGEIALGDPVAKYW
PELTGKQWQGIRMLDLATYTAGGLPLQVPDEVTDTASLLRFYQNWQPQWKPGTTR
LYANASIGLFGALAVKPSGMSYEQAMTTRVFKPLKLDHTWINVPKAEEAHFAWGYR
EGKAVHVSPGMLDAEAYGVKTNVKDMASWLIANMKPDSLQAPSLKQGIALAQSRY
WRVGAMYQGLGWEMLNWPVDAKTVVGGSDNKVALAPLPVAEVNPPAPPVKASW
VHKTGSTGGFGSYVAFIPEKQLGIVMLANKSYPNPARVEAAYRILDALQ
**SEQ ID N°950:** 
MMTKSLSCALLLSVASAAFAAPMSETQLAEVVERTVTPLMNAQAIPGMAVAVIYQG
QPHYFTFGKADVAANKPVTPQTLFELGSISKTFTGVLGGDAIARGEIALGDPVAKYW
PELTGKQWQGIRMLDLATYTAGGLPLQVPDEVTDTASLLRFYQNWQPQWKPGTTR
LYANASIGLFGALAVKPSGMSYEQAMTTRVFKPLKLDHTWINVPKAEEAHYAWGYR
EGKAVHVSPGMLDAEAYGVKTNVKDMASWVIANMKPDSLQAPSLKQGIALAQSRY
WRVGAMYQGLGWEMLNWPVDAKTVVGGSDNKVALAPLPVAEVNPPAPPVKASW
VHKTGSTGGFGSYVAFIPEKQLGIVMLANKSYPNPARVEAAYHILDALQ
**SEQ ID N°951:** 
MMTKSLSCALLLSVASAAFAAPMSEKQLAEVVERTVTPLMNAQAIPGMAVAVIYQG
QPHYFTFGKADVAANKPVTPQTLFELGSISKTFTGVLGGDAIARGEIALGDPVAKYW
PELTGKQWQGIRMLDLATYTAGGLPLQVPDEVTDTASLLRFYQNWQPQWKPGTTR
LYANASIGLFGALAVKPSGMSYEQAMTTRVFKPLKLDHTWINVPKAEEAHYAWGYR
EGKAVHVSPGMLDAEAYGVKTNVKDMASWLIANMKPDSLHAPSLKQGIALAQSRY
WRVGAMYQGLGWEMLNWPVDAKTVVGGSDNKVALAPLPVAEVNPPAPPVKASW
VHKTGSTGGFGSYVAFIPEKQLGIVMLANKSYPNPARVEAAYRILDALQ
**SEQ ID N°952:** 
MMTKSLSCALLLSVASAAFAAPMFEKQLAEVVERTVTPLMNAQAIPGMAVAVIYQG
QPHYFTFGKADVAANKPVTPQTLFELGSISKTFTGVLGGDAIARGEIALGDPVAKYW
PELTGKQWQGIRMLDLATYTAGGLPLQVPDEVTDTASLLRFYQNWQPQWKPGTTR
LYANASIGLFGALAVKPSGMSYEQAMTTRVFKPLKLDHTWINVPKAEEAHYAWGYR
EGKAVHVSPGMLDAEAYGVKTNVKDMASWLIANMKPDSLHAPSLKQGIALAQSRY
WRVGAMYQGLGWEMLNWPVDAKTVVGGSDNKVALAPLPVAEVNPPAPPVKASW
VHKTGSTGGFGSYVAFIPEKQLGIVMLANKSYPNPARVEAAYRILDALQ
**SEQ ID N°953:** 
MMTKSLSCALLLSVASSAFAAPMSEKQLAEVVERTVTPLMNAQAIPGMAVAVIYQG
QPHYFTFGKADVAANKPVTPQTLFELGSISKTFTGVLGGDAIARGEIALGDPVAKYW
PELTGKQWQGIRMLDLATYTAGGLPLQVPDEVTDTASLLRFYQNWQPQWKPGTTR
LYANASIGLFGALAVKPSGMSYEQAMTTRVFKPLKLDHTWINVPKAEEAHYAWGYR
EGKAVHVSPGMLDAEAYGVKTNVKDMASWLIANMKPDSLQAPSLKQGIALAQSRY
WRVGAMYQGLGWEMLNWPVDAKTVVGGSDNKVALAPLPVAEVNPPAPPVKASW
VHKTGSTGGFGSYVAFIPEKQLGIVMLANKSYPNPARVEAAYRILDALQ
lesdits peptides étant choisis, de préférence, parmi les peptides de séquence **SEQ ID N° 954** à **SEQ ID N° 981** tels que définis ci-après :

| **Peptide SEQ ID N°** | **Séquence d'acides aminés** | **Position du peptide dans la ou les protéines MIR** |
|---|---|---|
| **SEQ ID N°954** | AEEAHFAWGYR | 214-224 pour la protéine de SEQ N°949 |
| **SEQ ID N°955** | DMASWLIANMK | 249-259 pour les protéines de SEQ N°949, 951, 952, 953 |
| **SEQ ID N°956** | DMASWLIANMKPDSLHAPSLK | 249-269 pour les protéines de SEQ N°951, 952 |
| **SEQ ID N°957** | DMASWLIANMKPDSLQAPSLK | 249-269 pour les protéines de SEQ N°949, 953 |
| **SEQ ID N°958** | DMASWVIANMK | 249-259 pour la protéine de SEQ N°950 |
| **SEQ ID N°959** | DMASWVIANMKPDSLQAPSLK | 249-269 pour la protéine de SEQ N°950 |
| **SEQ ID N°960** | GEIALGDPVAK | 101-111 pour les protéines de SEQ N°949, 950, 951, 952, 953 |
| **SEQ ID N°961** | TVVGGSDNK | 302-310 pour les protéines de SEQ N°949, 950, 951, 952, 953 |
| **SEQ ID N°962** | ADVAANK | 66-72 pour les protéines de SEQ N°949, 950, 951, 952, 953 |
| **SEQ ID N°963** | AEEAHYAWGYR | 214-224 pour les protéines de SEQ N°950, 951, 952, 953 |
| **SEQ ID N°964** | AVHVSPGMLDAEAYGVK | 228-244 pour les protéines de SEQ N°949, 950, 951, 952, 953 |
| **SEQ ID N°965** | FYQNWQPQWK | 154-163 pour les protéines de SEQ N°949, 950, 951, 952, 953 |
| **SEQ ID N°966** | FYQNWQPQWKPGTTR | 154-168 pour les protéines de SEQ N°949, 950, 951, 952, 953 |
| **SEQ ID N°967** | LDHTWINVPK | 204-213 pour les protéines de SEQ N°949, 950, 951, 952, 953 |
| **SEQ ID N°968** | LYANASIGLFGALAVK | 169-184 pour les protéines de SEQ N°949, 950, 951, 952, 953 |
| **SEQ ID N°969** | QGIALAQSR | 270-278 pour les protéines de SEQ N°949, 950, 951, 952, 953 |
| **SEQ ID N°970** | QLAEVVER | 27-34 pour les protéines de SEQ N°949, 950, 951, 952, 953 |
| **SEQ ID N°971** | QLGIVMLANK | 353-362 pour les protéines de SEQ N°949, 950, 951, 952, 953 |
| **SEQ ID N°972** | SYPNPAR | 363-369 pour les protéines de SEQ N°949, 950, 951, 952, 953 |
| **SEQ ID N°973** | TFTGVLGGDAIAR | 88-100 pour les protéines de SEQ N°949, 950, 951, 952, 953 |
| **SEQ ID N°974** | TGSTGGFGSYVAFIPEK | 336-352 pour les protéines de SEQ N°949, 950, 951, 952, 953 |
| **SEQ ID N°975** | VALAPLPVAEVNPPAPPVK | 311-329 pour les protéines de SEQ N°949, 950,951,952,953 |
| **SEQ ID N°976** | VEAAYR | 370-375 pour les protéines de SEQ N°949, 951, 952, 953 |
| **SEQ ID N°977** | VFKPLK | 198-203 pour les protéines de SEQ N°949, 950, 951, 952, 953 |
| **SEQ ID N°978** | VGAMYQGLGWEMLNWPVDAK | 282-301 pour les protéines de SEQ N°949, 950, 951, 952, 953 |
| **SEQ ID N°979** | ASWVHK | 330-335 pour les protéines de SEQ N°949, 950, 951, 952, 953 |
| **SEQ ID N°980** | QWQGIR | 120-125 pour les protéines de SEQ N°949, 950, 951, 952, 953 |
| **SEQ ID N°981** | YWPELTGK | 112-119 pour les protéines de SEQ N°949, 950, 951, 952, 953 |

La détection d'un mécanisme de résistance aux céphalosporines induit par l'expression de la protéine MOX est caractérisée par la détection d'au moins un peptide appartenant à la protéine MOX et à ses différents variants de séquences **SEQ ID N° 982** à **SEQ ID N°988.**
**SEQ ID N°982:** 
MQQRQSILWGAVATLMWAGLAHAGETSPVDPLRPVVDASIQPLLKEHRIPGMAVAV
LKDGKAHYFNYGVADRERAVGVSEQTLFEIGSVSKPLTATLGAYAVVKGAMQLDDK
ASRHAPWLKGSAFDSITMGELATYSAGGLPLQFPEEVDSLEKMQAYYRQWTPAYS
PGSHRQYSNPSIGLFGHLAASSLKQPFAQLMEQTLLPGLGLHHTYVNVPKQAMASY
AYGYSKEDKPIRVSPGMLADEAYGIKTSSADLLRFVKANISGVDDKALQQAISLTHK
GHYSVGGMTQGLGWERYAYPVSEQTLLAGNSAKVILEANPTAAPRESGSQMLFNK
TGSTSGFGAYVAFVPAKGIGIVMLANRNYPIPARVKAAHAILTQLAR
**SEQ ID N°983:** 
MQQRQSILWGALATLMWAGLAHAGDTSAVDPLRPVVDASIRPLLKEHRIPGMAVAV
LKDGKAHYFNYGVADRERAVGVSEQTLFEIGSVSKPLTATLGAYAVVKGAMQLDDK
ASRHAPWLKGSAFDSITMGELATYSAGGLPLQFPEEVDSLEKMQAYYRQWTPAYS
PGSHRQYSNPSIGLFGHLAASSMKQPFAQLMEQTLLPGLGLHHTYVNVPKQAMAS
YAYGYSKEDKPIRVSPGMLADEAYGIKTSSADLLRFVKANISGVDDKALQQAISLTHK
GHYSVGGMTQGLGWESYAYPVSEQTLLAGNSAEVILEANPTAAPRESGNLMLFNK
TGSTSGFGAYVAFVPAKGIGIVMLANRNYPIPARVKAAHAILTQLAR
**SEQ ID N°984:** 
MQQRQSILWGALATLMWAGLAHAGDKAATDPLRPVVDASIRPLLKEHRIPGMAVAV
LKDGKAHYFNYGVADRERAVGVSEQTLFEIGSVSKTLTATLGAYAVVQGGFELDDK
ASLFAPWLKGSAFDNITMGELATYSAGGLPLQFPEEVDSLEKMQAYYRQWTPAYS
RGSHRQYANPSIGLFGYLAASSMKQPFDRLMEQTMLPGLGLYHTYLNVPEQPMGH
YAYGYWKEDKPFRVTPAMLAEEPYGIKTSSADLLRFVKANISGVDNAAMQQAIDLTH
QGQYAVGEMTQGLGWERYPYPVSEQTLLAGNSPAMIYNANPAAPAPAAAGHPVLF
KKTGSTNGFGAYVAFVPAKGIGVVMLANRNYPNEGTLKAGHAILTQLAR
**SEQ ID N°985:** 
MQQRQSILWGVLPTLMWAGLAHAGDRAATDPLRPVVDASIRPLLKEHRIPGMAVAV
LKDGKAHYFNYGVADRERAVGVSEQTLFEIGSVSKTLTATLGAYAVVQGSFELDDK
ASLFAPWLKGSVFDNITMGELATYSAGGLPLQFPEEVDSLEKMQAYYRQWTPAYS
PGSHRQYANPSIGLFGYLAASSMKQPFDRLMEQTILPGLGLYHTYLNVPEQAMGHY
AYGYSKEDKPIRVTPGMLADEAYGIKTSSADLLRFVKANISGVDNAAMQQAIDLTHQ
GQYAVGEMTQGLGWERYAYPVSEQTLLAGNSAAMIYNANPAAPAPAARGHPVLFN
KTGSTNGFGAYVAFVPAKGIGIVMLANRNSPIEGTLKAGHAILTQLAR
**SEQ ID N°986:** 
MQQRQSILWGALATLMWAGLVHAGDKAATDPLRPVVDASIRPLLKEHRIPGMAVAV
LKDGKAHYFNYGVADRERAVGVSEQTLFEIGSVSKTLTATLGAYAVVQGSFELDDK
ASLFAPWLKGSVFDNITMGELATYSAGGLPLQFPEEVDSLEKMQAYYRQWTPAYS
PGSHRQYANPSIGLFGYLAASSMKQPFDRLMEQTMLPGLGLYHTYLTVPEQAMGH
YAYGYSKEDKPIRVTPGMLADEAYGIKTSSADLLRFVKANIGGVDNAAMQQAIDLTH
 
QGQYAVGEMTQGLGWERYAYPVSEQTLLAGNSPAMIYNAIPAVPAPAAAGHPVLF
NKTGSTNGFGAYVAFVPAKGIGIVMLANRNSPIEARIKAAHAILTQLAR
**SEQ ID N°987:** 
MQQRQSILWGAVATLMWAGLAHAGEASPVDPLRPVVDASIQPLLKEHRIPGMAVAV
LKDGKAHYFNYGVANRESGASVSEQTLFEIGSVSKTLTATLGAYAVVKGAMQLDDK
ASRHAPWLKGSVFDSITMGELATYSAGGLPLQFPEEVDSSEKMRAYYRQWAPVYS
PGSHRQYSNPSIGLFGHLAASSLKQPFAQLMEQTLLPGLGMHHTYVNVPKQAMAS
YAYGYSKEDKPIRVNPGMLADEAYGIKTSSADLLAFVKANIGGVDDKALQQAISLTH
KGHYSVGGMTQGLGWESYAYPVTEQTLLAGNSAKVILEANPTAAPRESGSQVLFN
KTGSSNGFGAYVAFVPARGIGIVMLANRNYPIPARVKAAHAILAQLAG
**SEQ ID N°988:** 
MQQRQSILWGALATLMWAGLAHAGETSPVDPLRPVVDASIRPLLKEHRIPGMAVAV
LKDGKAHYFNYGVADRERAVGVSEQTLFEIGSVSKPLTATLGAYAVVKGAMQLDDK
ASRHAPWLKGSAFDSITMGELATYSAGGLPLQFPEEVDSLEKMQAYYRQWTPAYS
PGSHRQYSNPSIGLFGHLAASSMKQPFAQLMEQTLLPGLGLHHTYVNVPKQAMAS
YAYGYSKEDKPIRVSPGMLADEAYGIKTSSADLLRFVKANISGVHDKALQQAISLTHK
GHYSVGGMTQGLGWESYAYPVSEQTLLAGNSAKVILEANPTAAPRESGSQMLFNK
TGSTSGFGAYVAFVPAKGIGIVMLANRNYPIPARVKAAHAILTQLAR
 lesdits peptides étant choisis, de préférence, parmi les peptides de séquence **SEQ ID N°989** à **SEQ ID N°1037** tels que définis ci-après :

| **Peptide SEQ ID N°** | **Séquence d'acides aminés** | **Position du peptide dans la ou les protéines MOX** |
|---|---|---|
| **SEQ ID N°989** | AATDPLR | 27-33 pour les protéines de SEQ N°984, 985, 986 |
| **SEQ ID N°990** | AHYFNYGVADR | 62-72 pour les protéines de SEQ N°982, 983, 984, 985, 986, 988 |
| **SEQ ID N°991** | ANISGVDDK | 261-269 pour les protéines de SEQ N°982, 983 |
| **SEQ ID N°992** | ANISGVHDK | 261-269 pour la protéine de SEQ N°988 |
| **SEQ ID N°993** | ASLFAPWLK | 113-121 pour les protéines de SEQ N°984, 985, 986 |
| **SEQ ID N°994** | AVGVSEQTLFEIGSVSK | 75-91 pour les protéines de SEQ N°982, 983, 984, 985, 986, 988 |
| **SEQ ID N°995** | EDKPFR | 230-235 pour la protéine de SEQ N°984 |
| **SEQ ID N°996** | ESGNLMLFNK | 326-335 pour la protéine de SEQ N°983 |
| **SEQ ID N°997** | ESGSQMLFNK | 326-335 pour les protéines de SEQ N°982, 988 |
| **SEQ ID N°998** | GHPVLFNK | 329-336 pour les protéines de SEQ N°985, 986 |
| **SEQ ID N°999** | GHYSVGGMTQGLGWER | 281-296 pour la protéine de SEQ N°982 |
| **SEQ ID N°1000** | GIGVVMLANR | 354-363 pour la protéine de SEQ N°984 |
| **SEQ ID N°1001** | MQAYYR | 155-160 pour les protéines de SEQ N°982, 983, 984, 985, 986, 988 |
| **SEQ ID N°1002** | NSPIEAR | 364-370 pour la protéine de SEQ N°986 |
| **SEQ ID N°1003** | NSPIEGTLK | 364-372 pour la protéine de SEQ N°985 |
| **SEQ ID N°1004** | NYPNEGTLK | 364-372 pour la protéine de SEQ N°984 |
| **SEQ ID N°1005** | QPFDR | 192-196 pour les protéines de SEQ N°984, 985, 986 |
| **SEQ ID N°1006** | QWTPAYSPGSHR | 161-172 pour les protéines de SEQ N°982, 983, 985, 986, 988 |
| **SEQ ID N°1007** | QWTPAYSR | 161-168 pour la protéine de SEQ N°984 |
| **SEQ ID N°1008** | QYANPSIGLFGYLAASSMK | 173-191 pour les protéines de SEQ N°984, 985, 986 |
| **SEQ ID N°1009** | QYSNPSIGLFGHLAASSMK | 173-191 pour les protéines de SEQ N°983, 988 |
| **SEQ ID N°1010** | TGSSNGFGAYVAFVPAR | 336-352 pour la protéine de SEQ N°987 |
| **SEQ ID N°1011** | TGSTNGFGAYVAFVPAK | 337-353 pour les protéines de SEQ N°984, 985, 986 |
| **SEQ ID N°1012** | TGSTSGFGAYVAFVPAK | 336-352 pour les protéines de SEQ N°982, 983, 988 |
| **SEQ ID N°1013** | TLTATLGAYAVVQGGFELDDK | 92-112 pour la protéine de SEQ N°984 |
| **SEQ ID N°1014** | TLTATLGAYAVVQGSFELDDK | 92-112 pour les protéines de SEQ N°985, 986 |
| **SEQ ID N°1015** | VSPGMLADEAYGIK | 236-249 pour les protéines de SEQ N°982, 983, 988 |
| **SEQ ID N°1016** | VTPAMLAEEPYGIK | 236-249 pour la protéine de SEQ N°984 |
| **SEQ ID N°1017** | VTPGMLADEAYGIK | 236-249 pour les protéines de SEQ N°985, 986 |
| **SEQ ID N°1018** | YAYPVSEQTLLAGNSAK | 297-313 pour les protéines de SEQ N°982, 988 |
| **SEQ ID N°1019** | ALQQAISLTHK | 270-280 pour les protéines de SEQ N°982, 983, 987,988 |
| **SEQ ID N°1020** | ANIGGVDDK | 261-269 pour la protéine de SEQ N°987 |
| **SEQ ID N°1021** | EDKPIR | 230-235 pour les protéines de SEQ N°982, 983, 985, 986, 987, 988 |
| **SEQ ID N°1022** | ESGASVSEQTLFEIGSVSK | 73-91 pour la protéine de SEQ N°987 |
| **SEQ ID N°1023** | ESGSQVLFNK | 326-335 pour la protéine de SEQ N°987 |
| **SEQ ID N°1024** | GAMQLDDK | 105-112 pour les protéines de SEQ N°982, 983, 987,988 |
| **SEQ ID N°1025** | GIGIVMLANR | 353-362 pour les protéines de SEQ N°982, 983, 987, 988; 354-363 pour les protéines de séquence SEQ ID N°985, 986 |
| **SEQ ID N°1026** | HAPWLK | 116-121 pour les protéines de SEQ N°982, 983, 987,988 |
| **SEQ ID N°1027** | NYPIPAR | 363-369 pour les protéines de SEQ N°982, 983, 987,988 |
| **SEQ ID N°1028** | QAMASYAYGYSK | 218-229 pour les protéines de SEQ N°982, 983, 987,988 |
| **SEQ ID N°1029** | QWAPVYSPGSHR | 161-172 pour la protéine de SEQ N°987 |
| **SEQ ID N°1030** | QYSNPSIGLFGHLAASSLK | 173-191 pour les protéines de SEQ N°982, 987 |
| **SEQ ID N°1031** | TLTATLGAYAVVK | 92-104 pour la protéine de SEQ N°987 |
| **SEQ ID N°1032** | TSSADLLAFVK | 250-260 pour la protéine de SEQ N°987 |
| **SEQ ID N°1033** | TSSADLLR | 250-257 pour les protéines de SEQ N°982, 983, 984,985,986,988 |
| **SEQ ID N°1034** | VILEANPTAAPR | 314-325 pour les protéines de SEQ N°982, 983, 987,988 |
| **SEQ ID N°1035** | VNPGMLADEAYGIK | 236-249 pour la protéine de SEQ N°987 |
| **SEQ ID N°1036** | AHYFNYGVANR | 62-72 pour la protéine de SEQ N°987 |
| **SEQ ID N°1037** | IPGMAVAVLK | 49-58 pour les protéines de SEQ N°982, 983, 984, 985, 986, 987, 988 |

La détection d'un mécanisme de résistance aux céphalosporines induit par l'expression de la protéine PER est caractérisée par la détection d'au moins un peptide appartenant à la protéine PER et à ses différents variants de séquences **SEQ ID N°1038 à SEQ ID N°1044.**
**SEQ ID N°1038:** 
MNVIIKAVVTASTLLMVSFSSFETSAQSPLLKEQIESIVIGKKATVGVAVWGPDDLEPL
LINPFEKFPMQSVFKLHLAMLVLHQVDQGKLDLNQTVIVNRAKVLQNTWAPIMKAYQ
GDEFSVPVQQLLQYSVSLSDNVACDLLFELVGGPAALHDYIQSMGIKETAVVANEA
QMHADDQVQYQNWTSMKGAAEILKKFEQKTQLSETSQALLWKWMVETTTGPERL
KGLLPAGTVVAHKTGTSGIKAGKTAATNDLGIILLPDGRPLLVAVFVKDSAESSRTNE
AIIAQVAQTAYQFELKKLSALSPN
**SEQ ID N°1039:** 
MNVIIKAVVTASTLLMVSFSSFETSAQSPLLKEQIESIVIGKKATVGVAVWGPDDLEPL
LINPFEKFPMQSVFKLHLAMLVLHQVDQGKLDLNQTVIVNRAKVLQNTWAPIMKAYQ
GDEFSVPVQQLLQYSVSHTDNVACDLLFELVGGPAALHDYIQSMGIKETAVVANEA
QMHADDQVQYQNWTSMKGAAEILKKFEQKTQLSETSQALLWKWMVETTTGPERL
KGLLPAGTVVAHKTGTSGIKAGKTAATNDLGIILLPDGRPLLVAVFVKDSAESSRTNE
AIIAQVAQTAYQFELKKLSALSPN
**SEQ ID N°1040:** 
MNVIIKAVVTASTLLMVSFSSFETSAQSPLLKGQIESIVIGKKATVGVAVWGPDDLEP
LLINPFEKFPMQSVFKLHLAMLVLHQVDQGKLDLNQTVIVNRAKVLQNTWAPIMKAY
QGDEFSVPVQQLLQYSVSHSDNVACDLLFELVGGPAALHDYIQSMGIKETAVVANE
AQMHADDQVQYQNWTSMKGAAEILKKFEQKTQLSETSQALLWKWMVETTTGPER
LKGLLPAGTVVAHKTGTSGIKAGKTAATNDLGIILLPDGRPLLVAVFVKDSAESSRTN
EAIIAQVAQTAYQFELKKLSALSPN
**SEQ ID N°1041:** 
MNVIAKGVFTTTALLMLSLSSWVVSAQSPLLKEQIETIVTGKKATVGVAVWGPDDLE
PLLVNPFEKFPMQSVFKMHLAMLVLHQVDQGKLDLNKTVAVNRAAVLQNTWSPMM
KDHQGDEFTVTVQQLLQYSVSHSDNVACDLLFELVGGPAALHAYIQSLGIKETEVVA
NEAQMHADDQVQYKNWTSMKAAAQLLRKFEQKKQLSETSQALLWKWMVETTTGP
QRLKGLLPAGTVVAHKTGTSGVRAGKTAATNDIGVIMLPDGRPLLVAVFVKDSAESA
RTNEAIIAQVAQAAYQFELKKLSAVSPD
**SEQ ID N°1042:** 
MNVIIKAVVTASTLLMVSFSSFETSAQSPLLKEQIESIVIGKKATVGVAVWGPDDLEPL
LINPFEKFPMQSVFKLHLAMLVLHQVDQGKLDLNQTVIVNRAKVLQNTWAPIMKAYQ
GDQFSVPVQQLLQYSVSHSDNVACDLLFELVGGPAALHDYIQSMGIKETAVVANEA
QMHADDQVQYQNWTSMKGAAEILKKFEQKTQLSETSQALLWKWMVETTTGPERL
KGLLPAGTVVAHKTGTSGVRAGKTAATNDLGIILLPDGRPLLVAVFVKDSAESSRTN
EAIIAQVAQAAYQFELKKLSALSPN
**SEQ ID N°1043:** 
MNVITKCVFTASALLMLGLSSFVVSAQSPLLKEQIETIVTGKKATVGVAVWGPDDLE
PLLLNPFEKFPMQSVFKLHLAMLVLHQVDQGKLDLNQSVTVNRAAVLQNTWSPMM
KDHQGDEFTVAVQQLLQYSVSHSDNVACDLLFELVGGPQALHAYIQSLGVKEAAVV
 
ANEAQMHADDQVQYQNWTSMKAAAQVLQKFEQKKQLSETSQALLWKWMVETTT
GPQRLKGLLPAGTIVAHKTGTSGVRAGKTAATNDAGVIMLPDGRPLLVAVFVKDSA
ESERTNEAIIAQVAQAAYQFELKKLSAVSPD
**SEQ ID** N°1044: 
MNVIIKAVVTASTLLMVSFSSFETSAQSPLLKEQIESIVIGKKATVGVAVWGPDDLEPL
LINPFEKFPMQSVFKLHLAMLVLHQVDQGKLDLNQTVIVNRAKVLQNTWAPIMKAYQ
GDEFSVPVQQLLQYSVSHSDNVACDLLFELVGGPAALHDYIQSMGIKETAVVANEA
QMHADDQVQYQNWTSMKGAAEILKKFEQKTQLSETSQALLWKWMVETTTGPERL
KGLLPAGTVVAHKTGTSGIKAGKTAATNDLGIILLPDGRPLLVAVFVKDSAESSRTNE
AIIAQVAQTAYQFELKKLSALSPN
 lesdits peptides étant choisis, de préférence, parmi les peptides de séquence **SEQ ID N°1045** à **SEQ ID N°1077** tels que définis ci-après :

| **Peptide SEQ ID N°** | **Séquence d'acides aminés** | **Position du peptide dans la ou les protéines PER** |
|---|---|---|
| **SEQ ID N°1045** | AAAQLLR | 190-196 pour les protéines de SEQ N°1041 |
| **SEQ ID N°1046** | AAAQVLQK | 190-197 pour les protéines de SEQ N°1043 |
| **SEQ ID N°1047** | AAVLQNTWSPMMK | 101-113 pour les protéines de SEQ N°1041, 1043 |
| **SEQ ID N°1048** | DSAESAR | 275-281 pour les protéines de SEQ N°1041 |
| **SEQ ID N°1049** | DSAESER | 275-281 pour les protéines de SEQ N°1043 |
| **SEQ ID N°1050** | DSAESSR | 275-281 pour les protéines de SEQ N°1038, 1039, 1040, 1042, 1044 |
| **SEQ ID N°1051** | EQIESIVIGK | 33-42 pour les protéines de SEQ N°1038, 1039, 1042, 1044 |
| **SEQ ID N°1052** | EQIETIVTGK | 33-42 pour les protéines de SEQ N°1041, 1043 |
| **SEQ ID N°1053** | ETEVVANEAQMHADDQVQYK | 164-183 pour les protéines de SEQ N°1041 |
| **SEQ ID N°1054** | FPMQSVFK | 67-74 pour les protéines de SEQ N°1038, 1039, 1040, 1041, 1042, 1043, 1044 |
| **SEQ ID N°1055** | GAAEILK | 190-196 pour les protéines de SEQ N°1038, 1039, 1040, 1042, 1044 |
| **SEQ ID N°1056** | GLLPAGTIVAHK | 228-239 pour les protéines de SEQ N°1043 |
| **SEQ ID N°1057** | GLLPAGTVVAHK | 228-239 pour les protéines de SEQ N°1038, 1039, 1040, 1041, 1042, 1044 |
| **SEQ ID N°1058** | GQIESIVIGK | 33-42 pour les protéines de SEQ N°1040 |
| **SEQ ID N°1059** | LDLNK | 90-94 pour les protéines de SEQ N°1041 |
| **SEQ ID N°1060** | LDLNQSVTVNR | 90-100 pour les protéines de SEQ N°1043 |
| **SEQ ID N°1061** | LDLNQTVIVNR | 90-100 pour les protéines de SEQ N°1038, 1039, 1040, 1042, 1044 |
| **SEQ ID N°1062** | LHLAMLVLHQVDQGK | 75-89 pour les protéines de SEQ N°1038, 1039, 1040, 1042, 1043, 1044 |
| **SEQ ID N°1063** | MHLAMLVLHQVDQGK | 75-89 pour les protéines de SEQ N°1041 |
| **SEQ ID N°1064** | NWTSMK | 184-189 pour les protéines de SEQ N°1038, 1039, 1040, 1041, 1042, 1043, 1044 |
| **SEQ ID N°1065** | QLSETSQALLWK | 203-214 pour les protéines de SEQ N°1038, 1039, 1040, 1041, 1042, 1043, 1044 |
| **SEQ ID N°1066** | TAATNDAGVIMLPDGR | 250-265 pour les protéines de SEQ N°1043 |
| **SEQ ID N°1067** | TAATNDIGVIMLPDGR | 250-265 pour les protéines de SEQ N°1041 |
| **SEQ ID N°1068** | TAATNDLGIILLPDGR | 250-265 pour les protéines de SEQ N°1038, 1039, 1040, 1042, 1044 |
| **SEQ ID N°1069** | TGTSGIK | 240-246 pour les protéines de SEQ N°1038, 1039, 1040, 1044 |
| **SEQ ID N°1070** | TGTSGVR | 240-246 pour les protéines de SEQ N°1041, 1042, 1043 |
| **SEQ ID N°1071** | TNEAIIAQVAQAAYQFELK | 282-300 pour les protéines de SEQ N°1041, 1042, 1043 |
| **SEQ ID N°1072** | TNEAIIAQVAQTAYQFELK | 282-300 pour les protéines de SEQ N°1038, 1039, 1040, 1044 |
| **SEQ ID N°1073** | TQLSETSQALLWK | 202-214 pour les protéines de SEQ N°1038, 1039, 1040, 1042, 1044 |
| **SEQ ID N°1074** | TVAVNR | 95-100 pour les protéines de SEQ N°1041 |
| **SEQ ID N°1075** | VLQNTWAPIMK | 103-113 pour les protéines de SEQ N°1038, 1039, 1040, 1042, 1044 |
| **SEQ ID N°1076** | WMVETTTGPER | 215-225 pour les protéines de SEQ N°1038, 1039, 1040, 1042, 1044 |
| **SEQ ID N°1077** | WMVETTTGPQR | 215-225 pour les protéines de SEQ N°1041, 1043 |

La détection d'un mécanisme de résistance aux céphalosporines induit par l'expression de la protéine PER indique une résistance de type ESBL. Elle est préférentiellement détectée à l'aide des **SEQ ID N°1045** à **SEQ ID N°1065** et **SEQ ID N°1069** à **SEQ ID N°1077.**

La détection d'un mécanisme de résistance aux céphalosporines induit par l'expression de la protéine VEB est caractérisée par la détection d'au moins un peptide appartenant à la protéine VEB et à ses différents variants de séquences **SEQ ID N°1078** à **SEQ ID N°1084.**
**SEQ ID N°1078:** 
MKIVKRILLVLLSLFFTIVYSNAQTDNLTLKIENVLKAKNARIGVAIFNSNEKDTLKINND
FHFPMQSVMKFPIALAVLSEIDKGNLSFEQKIEITPQDLLPKTWSPIKEEFPNGTTLTI
EQILNYTVSESDNIGCDILLKLIGGTDSVQKFLNANHFTDISIKANEEQMHKDWNTQY
QNWATPTAMNKLLIDTYNNKNQLLSKKSYDFIWKIMRETTTGSNRLKGQLPKNTIVA
HKTGTSGINNGIAAATNDVGVITLPNGQLIFISVFVAESKETSEINEKIISDIAKITWNYY
LNK
**SEQ ID N°1079:** 
MKIVKRILLVLLSLFFTIVYSNAQADNLTLKIENVLKAKNARIGVAIFNSNEKDTLKINND
FHFPMQSVMKFPIALAVLSEIDKGNLSFEQKIEITPQDLLPKTWSPIKEEFPNGTTLTI
EQILNYTVSESDNIGCDILLKLIGGTDSVQKFLNANHFTDISIKANEEQMHKDWNTQY
QNWATPTAMNKLLIDTYNNKNQLLSKKSYDFIWKIMRETTTGSNRLKGQLPKNTIVA
HKTGTSGINNGIAAATNDVGVITLPNGQLIFISVFVAESKETSEINEKIISDIAKITWNYY
LNK
**SEQ ID N°1080:** 
MKIVKRILLVLLSLFFTVVYSNAQTDNLTLKIENVLKAKNARIGVAIFNSNEKDTFKINN
DFHFPMQSVMKFPIALAVLSEIDKGNLSFEQKIEITPQDLLPKTWSPIKEEFPNGTTLT
IEQILNYTVSESDNIGCDILLKLIGGTDSVQKFLNANHFTDISIKANEEQMHKDWNTQY
QNWATPTAMNKLLIDTYNNKNQLLSKKSYDFIWKIMRETTTGSNRLKGQLPKNTIVA
HKTGTSGINNGIAAATNDVGVITLPNGQLIFISVFVAESKETSEINEKIISDIAKITWNYY
LNK
**SEQ ID N°1081:** 
MKIVKRILLVLLSLFFTIVYSNAQADNLTLKIENVLKAKNARIGVAIFNSNEKDTLKINND
FHFPMQSVMKFPIALAVLSEIDKGNLSFEQKIEITPQDLLPKMWSPIKEEFPNGTTLTI
EQILNYTVSESDNIGCDILLKLIGGTDSVQKFLNANHFTDISIKANEEQMHKDWNTQY
QNWATPTAMNKLLIDTYNNKNQLLSKKSYDFIWKIMRETTTGSNRLKGQLPKNTIVA
HKTGTSGINNGIAAATNDVGVITLPNGQLIFISVFVAESKETSEINEKIISDIAKITWNYY
LNK
**SEQ ID** N°1082: 
MKIVKRILLVLLSLFFTVEYSNAQTDNLTLKIENVLKAKNARIGVAIFNSNEKDTLKINN
DFHFPMQSVMKFPIALAVLSEIDKGNLSFEQKIEITPQDLLPKMWSPIKEEFPNGTTL
TIEQILNYTVSESDNIGCDILLKLIGGTDSVQKFLNANHFTDISIKANEEQMHKDWNTQ
YQNWATPTAMNKLLIDTYNNKNQLLSKKSYDFIWKIMRETTTGSNRLKGQLPKNTIV
AHKTGTSGINNGIAAATNDVGVITLPNGQLIFISVFVAESKETSEINEKIISDIAKITWNY
YLNK
**SEQ ID** N°1083: 
MKIVKRILLVLLSLFFTVVYSNAQADNLTLKIENVLKAKNARIGVAIFNSNEKDTLKINN
DFHFPMQSVMKFPIALAVLSEIDKGNLSFEQKIEITPQDLLPKMWSPIKEEFPNGTTL
TIEQILNYTVSESDNIGCDILLKLIGGTDSVQKFLNANHFTDISIKANEEQMHKDWNTQ
YQNWATPTAMNKLLIDTYNNKNQLLSKKSYDFIWKIMRETTTGSNRLKGQLPKNTIV
AHKTGTSGINNGIAAATNDVGVITLPNGQLIFISVFVAESKETSEINEKIISDIAKITWNY
YLNK
**SEQ ID** N°1084: 
MKIVKRILLVLLSLFFTVEYSNAQTDNLTLKIENVLKAKNARIGVAIFNSNEKDTLKINN
DFHFPMQSVMKFPIALAVLSEIDKGNLSFEQKIEITPQDLLPKTWSPIKEEFPNGTTLT
IEQILNYTVSESDNIGCDILLKLIGGTDSVQKFLNANHFTDISIKANEEQMHKDWNTQY
QNWATPTAMNKLLIDTYNNKNQLLSKKSYDFIWKIMRETTTGSNRLKGQLPKNTIVA
HKTGTSGINNGITAATNDVGVITLPNGQLIFISVFVAESKETSEINEKIISDIAKITWNYY
LNK
 lesdits peptides étant choisis, de préférence, parmi les peptides de séquence **SEQ ID N° 1085** à **SEQ ID N° 1104** tels que définis ci-après :

| **Peptide SEQ ID N°** | **Séquence d'acides aminés** | **Position du peptide dans la ou les protéines VEB** |
|---|---|---|
| **SEQ ID N°1085** | ANEEQMHK | 165-172 pour les protéines de SEQ N°1078, 1079, 1080, 1081, 1082, 1083, 1084 |
| **SEQ ID N°1086** | DWNTQYQNWATPTAMNK | 173-189 pour les protéines de SEQ N°1078, 1079, 1080, 1081, 1082, 1083, 1084 |
| **SEQ ID N°1087** | ETSEINEK | 276-283 pour les protéines de SEQ N°1078, 1079, 1080, 1081, 1082, 1083, 1084 |
| **SEQ ID N°1088** | ETTTGSNR | 216-223 pour les protéines de SEQ N°1078, 1079, 1080, 1081, 1082, 1083, 1084 |
| **SEQ ID N°1089** | FLNANHFTDISIK | 152-164 pour les protéines de SEQ N°1078, 1079, 1080, 1081, 1082, 1083, 1084 |
| **SEQ ID N°1090** | FPIALAVLSEIDK | 72-84 pour les protéines de SEQ N°1078, 1079, 1080, 1081, 1082, 1083, 1084 |
| **SEQ ID N°1091** | GNLSFEQK | 85-92 pour les protéines de SEQ N°1078, 1079, 1080, 1081, 1082, 1083, 1084 |
| **SEQ ID N°1092** | GQLPK | 226-230 pour les protéines de SEQ N°1078, 1079, 1080, 1081, 1082, 1083, 1084 |
| **SEQ ID N°1093** | IEITPQDLLPK | 93-103 pour les protéines de SEQ N°1078, 1079, 1080, 1081, 1082, 1083, 1084 |
| **SEQ ID N°1094** | IENVLK | 32-37 pour les protéines de SEQ N°1078, 1079, 1080, 1081, 1082, 1083, 1084 |
| **SEQ ID N°1095** | IGVAIFNSNEK | 43-53 pour les protéines de SEQ N°1078, 1079, 1080, 1081, 1082, 1083, 1084 |
| **SEQ ID N°1096** | IISDIAK | 284-290 pour les protéines de SEQ N°1078, 1079, 1080, 1081, 1082, 1083, 1084 |
| **SEQ ID N°1097** | INNDFHFPMQSVMK | 58-71 pour les protéines de SEQ N°1078, 1079, 1080, 1081, 1082, 1083, 1084 |
| **SEQ ID N°1098** | LIGGTDSVQK | 142-151 pour les protéines de SEQ N°1078, 1079, 1080, 1081, 1082, 1083, 1084 |
| **SEQ ID N°1099** | LLIDTYNNK | 190-198 pour les protéines de SEQ N°1078, 1079, 1080, 1081, 1082, 1083, 1084 |
| **SEQ ID N°1100** | MWSPIK | 104-109 pour les protéines de SEQ N°1081, 1082, 1083 |
| **SEQ ID N°1101** | NQLLSK | 199-204 pour les protéines de SEQ N°1078, 1079, 1080, 1081, 1082, 1083, 1084 |
| **SEQ ID N°1102** | NTIVAHK | 231-237 pour les protéines de SEQ N°1078, 1079, 1080, 1081, 1082, 1083, 1084 |
| **SEQ ID N°1103** | SYDFIWK | 206-212 pour les protéines de SEQ N°1078, 1079, 1080, 1081, 1082, 1083, 1084 |
| **SEQ ID N°1104** | TWSPIK | 104-109 pour les protéines de SEQ N°1078, 1079, 1080,1084 |

La détection d'un mécanisme de résistance aux céphalosporines induit par l'expression de la protéine VEB indique une résistance de type ESBL.

La détection d'un mécanisme de résistance aux céphalosporines ou aux carbapénèmes induit par l'expression de la protéine OXA est caractérisée par la détection d'au moins un peptide appartenant à la protéine OXA et à ses différents variants de séquences **SEQ ID N° 1105** à **SEQ ID N°1266** :
**SEQ ID N°1105:** 
MSRLLLSGLLATGLLCAVPASAASGCFLYADGNGQTLSSEGDCSSQLPPASTFKIPL
ALMGYDSGFLVNEEHPALPYKPSYDGWLPAWRETTTPRRWETYSVVWFSQQITE
WLGMERFQQYVDRFDYGNRDLSGNPGKHDGLTQAWLSSSLAISPEEQARFLGKM
VSGKLPVSAQTLQYTANILKVSEVEGWQIHGKTGMGYPKKLDGSLNRDQQIGWFV
GWASKPGKQLIFVHTVVQKPGKQFASIKAKEEVLAALPAQLKKL
**SEQ ID N°1106:** 
IACLSSTALAGSITENTSWNKEFSAEAVNGVFVLCKSSSKSCATNDLARASKEYLPA
STFKIPNAIIGLETGVIKNEHQVFKWDGKPRAMKQWERDLTLRGAIQVSAVPVFQQI
AREVGEVRMQKYLKKFSYGNQNISGGIDKFWLEDQLRISAVNQVEFLESLYLNKLSA
SKENQLIVKEALVTEAAPEYLVHSKTGFSGVGTESNPGVAWWVGWVEKETEVYFF
AFNMDIDNESKLPLRKSIPTKIMESEGIIGG
**SEQ ID N°1107:** 
MKKILLLHMLVFVSATLPISSVASDEVETLKCTIIADAITGNTLYETGECARRVSPCSS
FKLPLAIMGFDSGILQSPKSPTWELKPEYNPSPRDRTYKQVYPALWQSDSVVWFSQ
QLTSRLGVDRFTEYVKKFEYGNQDVSGDSGKHNGLTQSWLMSSLTISPKEQIQFLL
RFVAHKLPVSEAAYDMAYATIPQYQAAEGWAVHGKSGSGWLRDNNGKINESRPQ
GWFVGWAEKNGRQVVFARLEIGKEKSDIPGGSKAREDILVELPVLMGNK
**SEQ ID N°1108:** 
MAIRIFAILFSIFSLATFAHAQEGTLERSDWRKFFSEFQAKGTIVVADERQADRAMLV
FDPVRSKKRYSPASTFKIPHTLFALDAGAVRDEFQIFRWDGVNRGFAGHNQDQDLR
SAMRNSTVWVYELFAKEIGDDKARRYLKKIDYGNAGPSTSNGDYWIEGSLAISAQE
QIAFLRKLYRNELPFRVEHQRLVKDLMIVEAGRNWILRAKTGWEGRMGWWVGWVE
WPTGSVFFALNIDTPNRMDDLFKREAIVRAILRSIEALPPNPAVNSDAAR
**SEQ ID N°1109:** 
MQRSLSMSGKRHFIFAVSFVISTVCLTFSPANAAQKLSCTLVIDEASGDLLHREGSC
DKAFAPMSTFKLPLAIMGYDADILLDATTPRWDYKPEFNGYKSQQKPTDPTIWLKDS
IVWYSQELTRRLGESRFSDYVQRFDYGNKDVSGDPGKHNGLTHAWLASSLKISPEE
QVRFLRRFLRGELPVSEDALEMTKAVVPHFEAGDWDVQGKTGTGSLSDAKGGKAP
IGWFIGWATRDDRRVVFARLTVGARKGEQPAGPAARDEFLNTLPALSENF
**SEQ ID N°1110:** 
MKTFAAYVIIACLSSTALAGSITENTSWNKEFSAEAVNGVFVLCKSSSKSCATNDLAR
ASKEYLPASTFKIPNAIIGLETGVIKNEHQVFKWDGKPRAMKQWERDLTLRGAIQVS
AVPVFQQIAREVGEVRMQKYLKKFSYGNQNISGGIDKFWLEGQLRISAVNQVEFLE
SLYLNKLSASKENQLIVKEALVTEAAPEYLVHSKTGFSGVGTESNPGVAWWVGWVE
KETEVYFFAFNMDIDNESKLPLRKSIPTKIMESEGIIGG
**SEQ ID N°1111:** 
IACLSSTALAGSITENTSWNKEFSAEAVNGVFVLCKSSSKSCATNDLARASKEYLPA
STFKIPNAIIGLETGVIKNEHQVFKWDGKPRAMKQWERDLTLRGAIQVSAVPVFQQIT
REVGEVRMQKYLKKFSYGNQNISGGIDKFWLEDQLRISAVNQVEFLESLYLNKLSAS
KENQLIVKEALVTEAAPEYLVHSKTGFSGVGTESNPGVAWWVGWVEKETEVYFFA
FNMDIDNESKLPLRKSIPTKIMESEGIIGG
**SEQ ID N°1112:** 
IACLSSTALAGSITENTSWNKEFSAEAVNGVFVLCKSSSKSCATNDLARASKEYLPA
STFKIPSAIIGLETGVIKNEHQVFKWDGKPRAMKQWERDLTLRGAIQVSAVPVFQQIA
REVGEVRMQKYLKKFSYGNQNISGGIDKFWLEGQLRISAVNQVEFLESLYLNKLSAS
KENQLIVKEALVTEAAPEYLVHSKTGFSGVGTESNPGVAWWVGWVEKETEVYFFA
FNMDIDNESKLPLRKSIPTKIMESEGIIGG
**SEQ ID N°1113:** 
MIIRFLALLFSAVVLVSLGHAQEKTHESSNWGKYFSDFNAKGTIVVVDERTNGNSTS
VYNESRAQQRYSPASTFKIPHTLFALDAGAVRDEFHVFRWDGAKRSFAGHNQDQN
LRSAMRNSTVWVYQLFAKEIGENKARSYLEKLNYGNADPSTKSGDYWIDGNLAISA
NEQISILKKLYRNELPFRVEHQRLVKDLMIVEAKRDWILRAKTGWDGQMGWWVGW
VEWPTGPVFFALNIDTPNRMEDLHKREAIARAILQSVNALPPN
**SEQ ID N°1114:** MAIRIFAILFSTFVFGTFAHAQEGMRERSDWRKFFSEFQAKGTIVVADERQTDRVILV 
FDQVRSEKRYSPASTFKIPHTLFALDAGAARDEFQVFRWDGIKRSFAAHNQDQDLR
SAMRNSTVWIYELFAKEIGEDKARRYLKQIDYGNADPSTSNGDYWIDGNLAIAAQEQ
IAFLRKLYHNELPFRVEHQRLVKDLMIVEAGRNWILRAKTGWEGRIGWWVGWVEW
PTGPVFFALNIDTPNRMDDLFKREAIVRAILRSIEALPPNPAVNSDAAR
**SEQ ID N°1115:** 
MKTFAAYVITACLSSTALASSITENTSWNKEFSAEAVNGVFVLCKSSSKSCATNNLA
RASKEYLPASTFKIPSAIIGLETGVIKNEHQVFKWDGKPRAMKQWERDLSLRGAIQV
SAVPVFQQIAREVGEVRMQKYLKKFSYGNQNISGGIDKFWLEGQLRISAVNQVEFL
ESLFLNKLSASKENQLIVKEALVTEAAPEYLVHSKTGFSGVGTESNPGVAWWVGWV
EKGTEVYFFAFNMDIDNENKLPLRKSIPTKIMASEGIIGG
**SEQ ID N°1116:** 
MKTFAAYVITACLSSTALASSITENTSWNKEFSAEAVNGVFVLCKSSSKSCATNNLA
RASKEYLPASTFKIPNAIIGLETGVIKNEHQVFKWDGKPRAMKQWERDLSLRGAIQV
SAVPVFQQIAREVGEVRMQKYLKKFSYGNQNISGGIDKFWLEDQLRISAVNQVEFLE
SLFLNKLSASKENQLIVKEALVTEAAPEYLVHSKTGFSGVGTESNPGVAWWVGWVE
KGTEVYFFAFNMDIDNENKLPLRKSIPTKIMASEGIIGG
**SEQ ID N°1117:** 
MKTFAAYVITACLSSTALASSITENTSWNKEFSAEAVNGVFVLCKSSSKSCATNNLA
RASKEYLPASTFKIPNAIIGLETGVIKNEHQVFKWDGKPRAMKQWERDLSLRGAIQV
SAVPVFQQIAREVGEVRMQKYLKKFSYGNQNISGGIDKFGLEGQLRISAVNQVEFLE
SLFLNKLSASKENQLIVKEALVTEAAPEYLVHSKTGFSGVGTESNPGVAWWVGWVE
KGTEVYFFAFNMDIDNENKLPLRKSIPTKIMASEGIIGG
**SEQ ID N°1118:** 
MKNTIHINFAIFLIIANIIYSSASASTDISTVASPLFEGTEGCFLLYDASTNAEIAQFNKA
KCATQMAPDSTFKIALSLMAFDAEIIDQKTIFKWDKTPKGMEIWNSNHTPKTWMQFS
VVWVSQEITQKIGLNKIKNYLKDFDYGNQDFSGDKERNNGLTEAWLESSLKISPEEQ
IQFLRKIINHNLPVKNSAIENTIENMYLQDLDNSTKLYGKTGAGFTANRTLQNGWFEG
FIISKSGHKYVFVSALTGNLGSNLTSSIKAKKNAITILNTLNL
**SEQ ID N°1119:** 
ANIIYSSASASTDISTVASPLFEGTEGCFLLYDVSTNAEIAQFNKAKCATQMAPDSTF
KIALSLMAFDAEIIDQKTIFKWDKTPKGMEIWNSNHTPKTWMQFSVVWVSQEITQKI
GLNKIKNYLKDFDYGNQDFSGDKERNNGLTEAWLESSLKISPEEQIQFLRKIINHNLP
VKNSAIENTIENMYLQDLENSTKLYGKTGAGFTANRTLQNGWFEGFIISKSGHKYVF
VSALTGNLGSNLTSSIKAKKNAITIL
**SEQ ID N°1120:** 
IFSLATFAHAQEGTLERSDWRKFFSEFQAKGTIVVADERQADRAMLVFDPVRSKKR
YSPASTFKIPHTLFALDAGAVRDEFQIFRWDGVNRGFAGHNQDQDLRSAMRNSTV
WVYELFAKEIGDDKARRYLKKIDYGNAYPSTSNGDYWIEGSLAISAQEQIAFLRKLYR
NELPFRVEHQRLVKDLMIVEAGRNWILRAKTGWEGRMGWWVGWVEWPTGSVFFA
LNIDTPNRMDDLFKREAIVRAIL
**SEQ ID N°1121:** 
MIIRFLALLFSAVVLVSLGHAQDKTHESSNWGKYFSDFNAKGTIVVVDERTNGNSTS
VYNESRAQQRYSPASTFKIPHTLFALDAGAVRDEFHVFRWDGAKRSFAGHNQDQN
LRSAMRNSTVWVYQLFAKEIGENKARSYLEKLNYGNADPSTKSGDYWIDGNLAISA
NEQISILKKLYRNELPFRVEHQRLVKDLMIVEAKRDWILRAKTGWDGQMGWWVGW
VEWPTGPVFFALNIDTPNRMEDLHKREAIARAILQSVNALPPN
**SEQ ID** N°1122: 
MKKFILPIFSISILVSLSACSSIKTKSEDNFHISSQQHEKAIKSYFDEAQTQGVIIIKEGK
NLSTYGNALARANKEYVPASTFKMLNALIGLENHKATTNEIFKWDGKKRTYPMWEK
DMTLGEAMALSAVPVYQELARRTGLELMQKEVKRVNFGNTNIGTQVDNFWLVGPL
 
KITPVQEVNFADDLAHNRLPFKLETQEEVEKMLLIKEVNGSKIYAKSGWGMGVTPQV
GWLTGWVEQANGKKIPFSLNLEMKEGMSGSIRNEITYKLLENLGII
**SEQ ID N°1123:** 
MKKFILPIFSISILVSLSACSSIKTKSEDNFHISSQQHEKAIKSYFDEAQTQGVIIIKEGK
NLSTYGNALARANKEYVPASTFKMLNALIGLENHKATTNEIFKWDGKKRTYPMWEK
DMTLGEAMALSAVPVYQELARRTGLELMQKEVKRVNFGNTNIGTQVDNFWLVGPL
KITPVQEVNFADDLAHNRLPFKLETQEEVKKMLLIKEVNGSKIYAKSGWGMGVTPQV
GWLTGWVEQANGKKIPFSLNLEMKEGMTGSIRNEITYKSLENLGII
**SEQ ID N°1124:** 
MNKYFTCYVVASLFLSGCTVQHNLINETPSQIVQGHNQVIHQYFDEKNTSGVLVIQT
DKKINLYGNALSRANTEYVPASTFKMLNALIGLENQKADINEIFKWKGEKRSFTAWE
KDMTLGEAMKLSAVPVYQELARRIGLDLMQKEVKRIGFGNAEIGQQVDNFWLVGPL
KVTPIQEVEFVSQLAHTQLPFSEKVQANVKNMLLLEESNGYKIFGKTGWAMDIKPQV
GWLTGWVEQPDGKIVAFALKMEMRSEMPASIRNELLMKSLKQLNII
**SEQ ID N°1125:** 
MAIRIFAILFSIFSLATFAHAQEGTLERSDWRKFFSEFQAKGTIVVADERQADRAMLV
FDPVRSKKRYSPASTFKIPHTLFALDAGAVRDEFQIFRWDGVNRGFAGHNQDQDLR
SAMRNSTVWVYELFAKEIGDDKARRYLKKIDYGNADPSTSNGDYCIEGSLAISAQEQ
IAFLRKLYRNELPFRVEHQRLVKDLMIVEAGRNWILRAKTGWEGRMGWWVGWVE
WPTGSVFFALNIDTPNRMDDLFKREAIVRAIL
**SEQ ID N°1126:** 
MKTFAAYVITACLSSTALASSITENTSWNKEFSAEAVNGVFVLCKSSSKSCATNNLA
RASKEYLPASTFKIPNAIIGLETGVIKNEHQVFKWDGKPRAMKQWERDLSLRGAIQV
SAVPVFQQIAREVGEVRMQKYLKKFSYGNQNISGGIDKFWLEGQLRISAVNQVEFL
ESLFLNKLSASKENQLIVKEALVTEAAPEYLVHSKTGFSGVGTESNPGVAWWVGWV
EKGTEVYFFAFNMDIDNENKLPLRKSIPTKIMASEGIIGG
**SEQ ID N°1127:** 
MAIRFLTILLSTFFLTSFVHAQEHVLERSDWKKFFSDLRAEGAIVISDERQAEHALLVF
GQERAAKRYSPASTFKLPHTLFALDADAVRDEFQVFRWDGVKRSFAGHNQDQDLR
SAMRNSAVWVYELFAKEIGKDKARHYLKQIDYGNADPSTIKGDYWIDGNLEISAHEQ
ISFLRKLYRNQLPFQVEHQRLVKDLMITEAGRNWILRAKTGWEGRFGWWVGWVE
WPTGPVFFALNIDTPNRTDDLFKREAIARAILRSIDALPPN
**SEQ ID N°1128:** 
MAIRIFAILFSIFSLATFAHAQEGTLERSDWRKFFSEFQAKGTIVVADERQADRAMLV
FDPVRSKKRYSPASTFKIPHTLFALDAGAVRDEFQIFRWDGVNRGFAGHNQDQDLR
SAMRNSTVWVYELFAKEIGDDKARRYLKKIDYGNADPSTSNGDYWIEGSIAISAQEQ
IAFLRKLYRNELPFRVEHQRLVKDLMIVEAGRNWILRAKTGWEGRMGWWVGWVE
WPTGSVFFALNIDTPNRMDDLFKREAIVRAILRSIEALPPNPAVNSDAAR
**SEQ ID N°1129:** 
MKKFILPIFSISILVSLSACSSIKTKSEDNFHISSQQHEKAIKSYFDEAQTQGVIIIKEGK
NLSTYGNALARANKEYVPASTFKMLNALIGLENHKATTNEIFKWDGKKRTYPMWEK
DMTLGEAMALSAVPVYQELARRTGLELMQKEVKRVNFGNTNIGTQVDNFWLVGPL
KITPVQEVNFADDLAHNRLPFKLETQEEVKKMLLIKEVNGSKIYAKSGWGMGVTPQV
GWLTGWVEQANGKKIPFSLNLEMKEGMSGSIRNEITYKSLENLGII
**SEQ ID N°1130:** 
MAIRFLTILLSTFFLTSFVHAQEHVLERSDWKKFFSDLRAEGAIVISDERQAEHALLVF
GQERAAKRYSPASTFKLPHTLFALDADAVRDEFQVFRWDGVKRSFAGHNQDQDLR
SAMRNSAVWVYELFAKEIGEDKARRYLKQIDYGNADPSTIKGDYWIDGNLEISAHEQ
ISFLRKLYRNQLPFQVEHQRLVKDLMITEAGRNWILRAKTGWEGRFGWWVGWVE
WPTGPVFFALNIDTPNRTDDLFKREAIARAILRSIDALPPN
**SEQ ID N°1131:** 
MAIRFFTILLSTFFLTSFVYAQEHVVIRSDWKKFFSDLQAEGAIVIADERQAKHTLSVF
DQERAAKRYSPASTFKIPHTLFALDADAVRDEFQVFRWDGVNRSFAGHNQDQDLR
SAMRNSTVWVYELFAKDIGEDKARRYLKQIDYGNVDPSTIKGDYWIDGNLKISAHEQ
ILFLRKLYRNQLPFKVEHQRLVKDLMITEAGRSWILRAKTGWEGRFGWWVGWIEW
PTGPVFFALNIDTPNRTDDLFKREAIARAILRSIDALPPN
**SEQ ID N°1132:** 
MNKYFTCYVVASLFLSGCTVQHNLINETPSQIVQGHNQVIHQYFDEKNTSGVLVIQT
DKKINLYGNALSRANTEYVPASTFKMLNALIGLENQKTDINEIFKWKGEKRSFTAWE
KDMTLGEAMKLSAVPVYQELARRIGLDLMQKEVERIGFGNAEIGQQVDNFWLVGPL
KVTPIQEVEFVSQLAHTQLPFSEKVQANVKNMLLLEESNGYKIFGKTGWAAMDIKPQ
VGWLTGWVEQPDGKIVAFALNMEMRSEMPASIRNELLMKSLKQLNII
**SEQ ID N°1133:** 
MAIQIFAILFSTFVLATFAHAQDGTLERSDWGKFFSDFQAKGTIVVADERQADHAILV
FDQARSMKRYSPASTFKIPHTLFALDAGAVRDEFQIFRWDGVKRSFAGHNKDQDLR
SAMRNSTVWVYELFAKEIGDGKARRYLKQIGYGNADPSTSHGDYWIEGSLAISAQE
QIAFLRKLYQNDLPFRVEHQRLVKDLMIVEAGRNWILRAKTGWEGSMGWWVGWV
EWPTGPVFFALNIDTPNRMDDLFKREAIARAILLSIEALPPNPAVHSDAAR
**SEQ ID N°1134:** 
MKNTIHINFAIFLIIANIIYSSASASTDISTVASQLFEGTEGCFLLYDASTNAEIAQFNKA
KCAAQMAPDSTFKIALSLMAFDAEIIDQKTIFKWDKIPKGMEIWNSNHTPKTWMQFS
VVWVSQEITQKIGLNKIKNYLKDFDYGNQDFSGDKERNNGLTEAWLESSLKISPEEQ
IQFLRKIINHNLPVRNSAIENTIDNMYLQDLENSTKLYGKTGAGFTANRTLQNGWFEG
FIISKSGHKYVFVSALTGSLGSNLTSSIKAKKNAITILNTLNL
**SEQ ID N°1135:** 
MLLFMFSIISFGNENQFMKEIFERKGLNGTFVVYDLKNDKIDYYNLDRANERFYPASS
FKIFNTLIGLENGIVKNVDEMFYYYDGSKVFLDSWAKDSNLRYAIKVSQVPAYKKLAR
ELGKERMQEGLNKLNYGNKEIGSEIDKFWLEGPLKISAMEQVKLLNLLSQSKLPFKL
ENQEQVKDITILEKKDDFILHGKTGWATDNIVVPIGWFVGWIETSDNIYSFAINLDISD
SKFLPKREEIVREYFKNINVIK
**SEQ ID N°1136:** 
MRVLALSAVFLVASIIGMPAVAKEWQENKSWNAHFTEHKSQGVVVLWNENKQQGF
TNNLKRANQAFLPASTFKIPNSLIALDLGVVKDEHQVFKWDGQTRDIATWNRDHNLI
TAMKYSVVPVYQEFARQIGEARMSKMLHAFDYGNEDISGNVDSFWLDGGIRISATE
QISFLRKLYHNKLHVSERSQRIVKQAMLTEANGDYIIRAKTGYSTRIEPKIGWWVGW
VELDDNVWFFAMNMDMPTSDGLGLRQAITKEVLKQEKIIP
**SEQ ID N°1137:** 
MLSRYSKTLAFAVVACTLAISTATAHAELVVRNDLKRVFDDAGVSGTFVLMDITADR
TYVVDPARAARSIHPASTFKIPNSLIAFDTGAVRDDQEVLPYGGKPQPYEQWEHDM
ALPEAIRLSAVPIYQEVARRVGFERMQAYVDAFDYGNRQLGSAIDQFWLRGPLEISA
FEEARFTSRMALKQLPVKPRTWDMVQRMLLIEQQGDAALYAKTGVATEYQPEIGW
WAGWVERAGHVYAFALNIDMPREGDMAKRIPLGKQLMRALEVWPAP
**SEQ ID N°1138:** 
MRPLLFSALLLLSGHTQASEWNDSQAVDKLFGAAGVKGTFVLYDVQRQRYVGHDR
ERAETRFVPASTYKVANSLIGLSTGAVRSADEVLPYGGKPQRFKAWEHDMSLRDAI
KASNVPVYQELARRIGLERMRANVSRLGYGNAEIGQVVDNFWLVGPLKISAMEQTR
FLLRLAQGELPFPAPVQSTVRAMTLLESGPGWELHGKTGWCFDCTPELGWWVGW
VKRNERLYGFALNIDMPGGEADIGKRVELGKASLKALGILP
**SEQ ID N°1139:** MNKGLHRKRLSKRLLLPMLLCLLAQQTQAVAAEQTKVSDVCSEVTAEGWQEVRRW 
DKLFESAGVKGSLLLWDQKRSLGLSNNLSRAAEGFIPASTFKLPSSLIALETGAVRD
ETSRFSWDGKVREIAVWNRDQSFRTAMKYSVVPVYQQLAREIGPKVMAAMVRQLE
YGNQDIGGQADSFWLDGQLRITAFQQVDFLRQLHDNKLPVSERSQRIVKQMMLTE
ASTDYIIRAKTGYGVRRTPAIGWWVGWLELDDNTVYFAVNLDLASASQLPLRQQLV
KQVLKQEQLLP
**SEQ ID N°1140:** 
MNTIISRRWRAGLWRRLVGAVVLPATLAATPAAYAADVPKAALGRITERADWGKLF
AAEGVKGTIVVLDARTQTYQAYDAARAEKRMSPASTYKIFNSLLALDSGALDNERAII
PWDGKPRRIKNWNAAMDLRTAFRVSCLPCYQVVSHKIGRRYAQAKLNEVGYGNRT
IGGAPDAYWVDDSLQISAREQVDFVQRLARGTLPFSARSQDIVRQMSIVEATPDYVL
HGKTGWFVDKKPDIGWWVGWIERDGNITSVAINIDMLSEADAPKRARIVKAVLKDLK
LI
**SEQ ID N°1141:** 
MKTFAAYVITACLSSTALASSITENTFWNKEFSAEAVNGVFVLCKSSSKSCATNNLA
RASKEYLPASTFKIPNAIIGLETGVIKNEHQIFKWDGKPRAMKQWERDLSLRGAIQVS
AVPVFQQIAREVGEVRMQKYLKKFSYGNQNISGGIDKFWLEGQLRISAVNQVEFLE
SLFLNKLSASKENQLIVKEALVTEAAPEYLVHSKTGFSGVGTESNPGVAWWVGWVE
KGAEVYFFAFNMDIDNENKLPLRKSIPTKIMASEGIIGG
**SEQ ID N°1142:** 
MRVLALSAVLVVASIVGMPAMANEWQEKPSWNTHFSEHKAQGVIVLWNENKQQGF
TNNLKRANQAFLPASTFKIPNSLIALDLGVVKDEHQVFKWDGQTRDIAAWNRDHDLI
TAMKYSVVPVYQEFARQIGQARMSKMLHAFDYGNEDISGNLDSFWLDGGIRISATE
QVAFLRKLYHNKLHVSERSQRIVKQAMLTEANSDYIIRAKTGYSTRIEPQIGWWVGW
VELDDNVWFFAMNMDMPTADGLGLRQAITKEVLKQEKIIP
**SEQ ID N°1143:** 
MKKITLFLLFLNLVFGQDKILNNWFKEYNTSGTFVFYDGKTWASNDFSRAMETFSPA
STFKIFNALIALDSGVIKTKKEIFYHYRGEKVFLSSWAQDMNLSSAIKYSNVLAFKEVA
RRIGIKTMQEYLNKLHYGNAKISKIDTFWLDNSLKISAKEQAILLFRLSQNSLPFSQEA
MNSVKEMIYLKNMENLELFGKTGFNDGQKIAWIVGFVYLKDENKYKAFALNLDIDKF
EDLYKREKILEKYLDELVKKVKNDG
**SEQ ID N°1144:** 
MSKKNFILIFIFVILISCKNTEKISNETTLIDNIFTNSNAEGTLVIYNLNDDKYIIHNKERAE
QRFYPASTFKIYNSLIGLNEKAVKDVDEVFYKLMAKSFLESWAKDSNLRYAIKNSQV
PAYKELARRIGIKKMKENIEKLDFGNKSIGDSVDTFWLEGPLEISAMEQVKLLTKLAQ
NELQYPIEIQKAISDITITRANLHITLHGKTGLADSKNMTTEPIGWFVGWLEENDNIYV
FALNIDNINSDDLAKRINIVKESLKALNLLK
**SEQ ID N°1145:** 
MNKYFTCYVVASLFLSGCTVQHNLINETPSQIVQGHNQVIHQYFDEKNTSGVLVIQT
DKKINLYGNALSRANTEYVPASTFKMLNALIGLENQKTDINEIFKWKGEKRSFTAWE
KDMTLGEAMKLSAVPVYQELARRIGLDLMQKEVKRIGFGNAEIGQQVDNFWLVGPL
KVTPIQEVEFVSQLAHTQLPFSEKVQANVKNMLLLEESNGYKIFGKTGWAMDIKPQV
GWLTGWVEQPDGKIVAFALKMEMRSEMPASIRNELLMKSLKQLNII
**SEQ ID N°1146:** 
MNIQALLLITSAIFISACSPYIVTANPNYSASKSDEKAEKIKNLFNEAHTTGVLVIQQGQ
TQQSYGNDLARASTEYVPASTFKMLNALIGLEHHKATTTEVFKWDGQKRLFPEWEK
NMTLGDAMKASAIPVYQDLARRIGLELMSNEVKRIGYGNADIGTQVDNFWLVGPLKI
TPQQEAQFAYKLANKTLPFSQKVQDEVQSMLFIEEKNGNKIYAKSGWGWDVNPQV
GWLTGWVVQPQGNIVAFSLNLEMKKGISSSVRKEITYRGLEQLGIL
**SEQ ID N°1147:** MNIKALLLITSAIFISACSPYIVTANPNHSASKSDEKGEKIKNLFNEAHTTGVLVIQQGQ 
TQQSYGNDLARASTEYVPASTFKMLNALIGLEHHKATTTEVFKWDGQKRLFPEWEK
DMTLGDAMKASAIPVYQDLARRIGLELMSKEVKRVGYGNADIGTQVDNFWLVGPLK
ITPQQEAQFAYKLANKTLPFSPKVQDEVQSMLFIEEKNGNKIYAKSGWGWDVDPQV
GWLTGWVVQPQGNIVAFSLNLEMKKGIPSSVRKEITYKSLEQLGIL
**SEQ ID N°1148:** 
MNIKALLLITSAIFISACSPYIVTANPNHSASKSDEKAEKIKNLFNEAHTTGVLVIQQGQ
TQQSYGNDLARASTEYVPASTFKMLNALIGLEHHKATTTEVFKWDGKKRLFPEWEK
DMTLGDAMKASAIPVYQDLARRIGLELMSKEVKRVGYGNADIGTQVDNFWLVGPLK
ITPQQEAQFAYKLANKTLPFSQKVQDEVQSMLFIEEKNGNKIYAKSGWGWDVNPQV
GWLTGWVVQPQGNIVAFSLNLEMKKGIPSSVRKEITYKSLEQLGIL
**SEQ ID N°1149:** 
MNIKALLLITSAIFISACSPYIVTANPNHSASKSDVKAEKIKNLFNEAHTTGVLVIQQGQ
TQQSYGNDLARASTEYVPASTFKMLNALIGLEHHKATTTEVFKWDGKKRLFPEWEK
DMTLGDAMKASAIPVYQDLARRIGLELMSKEVKRVGYGNADIGTQVDNFWLVGPLK
ITPQQEAQFAYKLANKTLPFSQKVQDEVQSMLFIEEKNGNKIYAKSGWGWDVNPQV
GWLTGWVVQPQGNIVAFSLNLEMKKGIPSSVRKEITYKSLEQLGIL
**SEQ ID N°1150:** 
MNIKALLLITSAIFISACSPYIVSANPNHSASKSDEKAEKIKNLFNEAHTTGVLVIQQGQ
TQQSYGNDLARASTEYVPASTFKMLNALIGLEHHKATTTEVFKWDGQKRLFPEWEK
NMTLGDAMKASAIPVYQDLARRIGLELMSNEVKRVGYGNADIGTQVDNFWLVGPLK
ITPQQEAQFAYKLANKTLPFSQEVQDEVQSMLFIEEKNGNKIYAKSGWGWDVNPQV
GWLTGWVVQPQGNIVAFSLNLEMKKGIPSSVRKEITYKSLEQLGIL
**SEQ ID N°1151:** 
MNIKALLLITSAIFISACSPYIVTANPNHSASKSDDKAEKIKNLFNEAHTTGVLVIHQGQ
TQQSYGNDLARASTEYVPASTFKMLNALIGLEHHKATTTEVFKWDGEKRLFPEWEK
NMTLGDAMKASAIPVYQDLARRIGLELMSKEVKRVGYGNADIGTQVDNFWLVGPLK
ITPQQEAQFAYKLANKTLPFSQKVQDEVQSMLFIEEKNGNKIYAKSGWGWDVNPQV
GWLTGWVVQPQGNIVAFSLNLEMKKGIPSSVRKEITYKSLEQLGIL
**SEQ ID N°1152:** 
MNIKALLLITSAIFISACSPYIVTANPNHSASKSDKKAEKIKNLFNEAHTTGVLVIQQGQ
TQQSYGNDLARASTEYVPASTFKMLNALIGLEHHKATTTEVFKWNGQKRLFPEWEK
DMTLGDAMKASAIPVYQDLARRIGLELMSNEVKRVGYGNADIGTQVDNFWLVGPLK
ITPQQEAQFAYKLANKTLPFSQKVQHEVQSMLFIEEKNGNKIYAKSGWGWDVDPQV
GWLTGWVVQPQGNIVAFSLNLEMKKGIPSSVRKEITYKSLEQLGIL
**SEQ ID N°1153:** 
MNIKALLLITSAIFISACSPYIVTANPNHSASKSDEKAEKIKNLFNEAHTTGVLVIQQGQ
TQQSYGNDLARASTEYVPASTFKMLNALIGLEHHKTTTTEVFKWDGQKRLFPEWEK
DMTLGDAMKASAIPVYQDLARRIGLELMSKEVKRVGYGNADIGTQVDNFWLVGPLK
ITPQQEAQFAYKLANKTLPFSPKVQDEVQSMLFIEEKNGNKIYAKSGWGWDVDPQV
GWLTGWVVQPQGNIVAFSLNLEMKKGIPSSVRKEITYKSLEQLGIL
**SEQ ID N°1154:** 
MNIKALLLITSAIFISACSPYIVSANPNHSASKSDEKAEKIKNLFNEAHTTGVLVIQQGQ
TQQSYGNDLARASTEYVPASTFKMLNALIGLEHHKATTTAVFKWDGQKRLFPEWEK
NMTLGDAMKASAIPVYQDLARRIGLELMSNEVKRVGYGNADIGTQVDNFWLVGPLK
ITPQQEAQFAYKLANKTLPFSQKVQDEVQSMLFIEEKNGNKIYAKSGWGWDVNPQV
GWLTGWVVQPQGNIVAFSLNLEMKKGTPSSVRKEITYKSLEQLGIL
**SEQ ID N°1155:** 
MNIKALLLITSAIFISACSPYIVSANPNHSASKSDEKAEKIKNLFNEAHTTGVLVIQQGQ
TQQSYGNDLARASTEYVPASTFKMLNALIGLEHHKATTTEVFKWDGQKRLFPEWEK
NMTLGDAMKASAIPVYQDLPRRIGLELMSNEVKRVGYGNADIGTQVDNFWLVGPLK
 
ITPQQEAQFAYKLANKTLPFSQKVQDEVQSMLFIEEKNGNKIYAKSGWGWDVNPQV
GWLTGWVVQPQGNIVAFSLNLEMKKGIPSSVRKEITYKSLEQLGIL
**SEQ ID N°1156:** 
MNIKALLLITSAIFISACSPYIVTANPNHSASKSDVKAEKIKNLFNEAHTTGVLVIQQGQ
TQQSYGNDLARASTEYVPASTFKMLNALIGLEHHKATTTEVFKWDGKKRLFPEWEK
DMTLGDAMKASAIPVYQDLARRIGLELMSKEVKRVGYGNADIGTQVDNFWLVGPLK
ITPQQEAQFAYKLANKTLPFSQKVQDEVQSMLFIEEKNGNKIYAKSGWGWDVNPQV
GWLTGWVVQPQGNIVAFSLNLEMKKGIPSSVRKEITYKSLEKLGIL
**SEQ ID N°1157:** 
MNIKTLLLITSTIFISACSPYIVTANPNHSTSKSDEKAEKIKNLFNEAHTTGVLVIQQGQ
TQQSYGNDLARASIEYVPASTFKMLNALIGLEHHKATTTEIFKWDGQKRLFPEWEKD
MTLGDAMKASAIPVYQDLARRIGLELMSKEVKRVGYGNADIGTQVDNFWLVGPLKIT
PQQEAQFAYKLANKTLPFSLKVQDEVQSMLFIEEKNGNKIYAKSGWGWDVDPQVG
WLTGWVVQPQGNIVAFSLNLEMKKGIPSSVRKEITYKSLEQLGIL
**SEQ ID N°1158:** 
MNIKALLLITSAIFISACSPYIVTANPNHSASKSDVKAEKIKNLFNEAHTTGVLVIQQGQ
TQQSYGNDLARASTEYVPASTFKMLNALIGLEHHKATTTEVFKWDGKKRLFPEWEK
DMTLGDAMKASAISVYQDLARRIGLELMSKEVKRVGYGNADIGTQVDNFWLAGPLK
ITPQQEAQFAYKLANKTLPFSQKVQDEVQSMLFIEEKNGNKIYAKSGWGWDVNPQV
GWLTGWVVQPQGNIVAFSLNLEMKKGIPSSVRKEITYKSLEQLGIL
**SEQ ID N°1159:** 
MNIKALLLITSAIFISACSPYIVTANPNHSASKSDVKAEKIKNLFNEAHTTGVLVIQQGQ
TQQSYGNDLARASTEYVPASTFKMLNALIGLEHHKATTTEVFKWDGKKRLFPEWEK
DMTLGDAMKASALPVYQDLARRIGLELMSKEVKRVGYGNADIGTQVDNFWLVGPL
KITPQQEAQFAYKLANKTLPFSQKVQDEVQSMLFIEEKNGNKIYAKSGWGWDVNPQ
VGWLTGWVVQPQGNIVAFSLNLEMKKGIPSSVRKEITYKSLEQLGIL
**SEQ ID N°1160:** 
MNIKALLLITSAIFISACSPYIVTANPNHSASKSDDKAEKIKNLFNEAHTTGVLVIHQGQ
TQQSYGNDLARASTEYVPASTFKMLNALIGLEHHKATTTEVFKWDGEKRLFPEWEK
NMTLGDAMKASAIPVYQDLARRIGLELMSKEVKRVGYGNADIGTQVDNFWLVGPLK
ITPQQEAQFAYKLANKTLPFSQKVQDEVQSMLFIEEKNGNKIYAKSGWGWDVNPQV
GWLTGSVVQPQGNIVAFSLNLEMKKGIPSSVRKEITYKSLEQLGIL
**SEQ ID N°1161:** 
MNIKTLLLITSTIFISACSPYIVTANPNHSTSKSDEKAEKIKNLFNEAHTTGVLVIQQGQ
TQQSYGNDLARASIEYVPASTFKMLNALIGLEHHKATTTEIFKWDGQKRLFPEWEKD
MTLGDAMKASAIPVYQDLARRIGLELMSKEVKRVGYGNADIGTQVDNFWLVGPLKIT
PQQEAQFAYKLANKTLPFSLKAQDEVQSMLFIEEKNGNKIYAKSGWGWDVDPQVG
WLTGWVVQPQGNIVAFSLNLEMKKGIPSSVRKEITYKSLEQLGIL
**SEQ ID N°1162:** 
MNIKALLLITSAIFISACSPYIVTANPNHSASKSDKKAEKIKNLFNEAHTTGVLVIQQGQ
TQQSYGNDLARASTEYVPASTFKMLNALIGLEHHKATTTEVFKWDGKKRLFPEWEK
DMTLGDAMKASAIPVYQDLARRIGLELMSKEVKRVGYGNADIGTQVDNFWLVGPLK
ITPQQEAQFAYKLANKTLPFSQKVQDEVQSMLFIEEKNGNKIYAKSGWGWDVNPQV
GWLTGWVVQPQGNIVAFSLNLEMKKGIPSSVRKEITYKSLEQLGIL
**SEQ ID N°1163:** 
MNIKTLLLITSAIFISACSHYIVSANPNHSASKSDEKAEKIKNLFNEAHTTGVLVIQQGQ
TQQSYGNDLARASTEYVPASTFKMLNALIGLEHHKATTTEVFKWDGQKRLFPEWEK
NMTLGDAMKASAIPVYQDLARRIGLELMSNEVKRVGYGNADIGTQVDNFWLVGPLK
ITPQQEAQFTYKLANKTLPFSQKVQDEVQSMLFIEEKNGNKIYAKSGWGWDVNPQV
GWLTGWVVQPQGNIVAFSLNLEMKKGIPSSVRKEITYKSLEQLGIL
**SEQ ID N°1164:** 
MNIKALLLITSAIFISACSPYIVSANPNHSASKSDEKAEKIKNLFNEAHTTGVLVIQQGQ
TQQSYGNDLARASTEYVPASTFKMLNALIGLEHHKATTTEVFKWDGQKRLFPEWEK
NMTLGDAMKASAIPVYQDLARRIGLELMSNEVKRVGYGNADIGTQVDNFWLVGPLK
ITPQQEAQFAYKLANKTLPFSQKVQDEVQSMLFIEEKNGNKIYAKSGWGWDVDPQV
GWLTGWVVQPQGNIVAFSLNLEMKKGIPSSVRKEITYKSLEQLGIL
**SEQ ID N°1165:** 
MNIKALLLITSTIFISACSPYIVTANPNHSASKSDEKAEKIKNLFNEAHTTGVLVIQQGQ
TQQSYGNDLARASTEYVPASTFKMLNALISLEHHKATTTEVFKWDGQKRLFPEWEK
DMTLGDAMKASAIPVYQDLARRIGLELMSNEVKRVGYGNADIGTQVDNFWLVGPLK
ITPQQEAQFAYKLANKTLPFSQKVQDEVQSMLFIEEKNGNKIYAKSGWGWDVNPQV
GWLTGWVVQPQGNIVAFSLNLEMKKGIPSSVRKEITYKSLEQLGIL
**SEQ ID N°1166:** 
MNIQALLLITSAIFISACSPYIVSANPNHSASKSDEKAEKIKNLFNEAHTTGVLVIQQGQ
TQQSYGNDLARASTEYVPASTFKMLNALIGLEHHKATTTEVFKWDGKKRLFPEWEK
DMTLGDAMKASAIPVYQDLARRIGLELMSKEVKRVGYGNADIGTQVDNFWLVGPLK
ITPQQEAQFAYKLANKTLPFSQKVQDEVQSMLFIEEKNGNKIYAKSGWGWDVNPQV
GWLTGWVVQPHGNIVAFSLNLEMKKGIPSSVRKEITYKSLEQLGIL
**SEQ ID N°1167:** 
MNIKALFLITSAIFISACSPYIVTANPNHSASKSDEKAEKIKNLFNEAHTTGVLVIQQGQ
TQQSYGNDLARASTEYVPASTFKMLNALIGLEHHKATTTEVFKWDGKKRLFPEWEK
DMTLGDAMKASAIPVYQDLARRIGLELMSKEVKRVGYGNADIGTQVDNFWLVGPLK
ITPQQEAQFAYKLANKTLPFSQKVQDEVQSMLFIEEKNGNKIYAKSGWGWDVDPQV
GWLTGWVVQPQGNIVAFSLNLEMKKGIPSSVRKEITYKSLEQLGIL
**SEQ ID N°1168:** 
MKLLKILSLVCLSISIGACAEHSMSRAKTSTIPQVNNSIIDQNVQALFNEISADAVFVTY
DGQNIKKYGTHLDRAKTAYIPASTFKIANALIGLENHKATSTEIFKWDGKPRFFKAWD
KDFTLGEAMQASTVPVYQELARRIGPSLMQSELQRIGYGNMQMGTEVDQFWLKGP
LTITPIQEVKFVYDLAQGQLPFKPEVQQQVKEMLYVERRGENRLYAKSGWGMAVD
PQVGWYVGFVEKADGQVVAFALNMQMKAGDDIALRKQLSLDVLDKLGVFHYL
**SEQ ID N°1169:** 
MNIKALLLITSTIFISACSPYIVTANPNHSTSKSDEKAEKIKNLFNEAHTTGVLVIQQGQ
TQQSYGNDLARASTEYVPASTFKMLNALIGLEHHKATTTEIFKWDGQKRLFPEWEK
DMTLGDAMKASAIPVYQDLARRIGLELMSKEVKRVGYGNADIGTQVDNFWLVGPLK
ITPQQEAQFAYKLANKTLPFSLKVQDEVQSMLFIEEKNGNKIYAKSGWGWDVDPQV
GWLTGWVVQPQGNIVAFSLNLEMKKGIPSSVRKEITYKSLEQLGIL
**SEQ ID N°1170:** 
MNIKTLLLITSAIFISACSPYIVTANPNHSASKSDEKAEKIKNLFNEVHTTGVLVIRQGQ
TQQSYGNDLARASTEYVPASTFKMLNALIGLEHHKATTTEVFKWDGQKRLFPEWEK
DMTLGDAMKASAIPVYQDLARRIGLELMSKEVKRVGYGNADIGTQVDNFWLVGPLK
ITPQQEAQFAYKLANKTLPFSPKVQDEVQSMLFIEEMNGNKIYAKSGWGWDVDPQV
GWLTGWVVQPQGNIVAFSLNLEMKKGIPSSVRKEITYKSLEQLGIL
**SEQ ID N°1171:** 
MKLLKILSLVCLSISIGACAEHSMSRAKTSTIPQVNNSIIDQNVQALFNEISGDAVFVTY
DGQNIKKYGTHLDRAKTAYIPASTFKIANALIGLENHKATSTEIFKWDGKPRFFKAWD
KDFTLGEAMQASTVPVYQELARRIGPSLMQSELQRIGYGNMQIGTEVDQFWLKGPL
TITPIQEVKFVYDLAQGQLPFKPEVQQQVKEMLYVERRGENRLYAKSGWGMAVDP
QVGWYVGFVEKADGQVVAFALNMQMKAGDDIALRKQLSLDVLDKLGVFHYL
**SEQ ID N°1172:** MKKFILPIFSISILVSLSACSSIKTKSEDNFHISSQQHEKAIKSYFDEAQTQGVIIIKEGK 
NLSTYGNALARANKEYVPASTFKMLNALIGLENHKATTNEIFKWDGKKRTYPMWEK
DMTLGEAMALSAVPVYQELARRTGLELMQKEVKRVNFGNTNIGTQVDNFWLVGPL
KITPVQEVNFADDLAHNRLPFKLETQEEVKKMLLIKEVNGSKIYAKSGWGMDVTPQV
GWLTGWVEQANGKKIPFSLNLEMKEGMSGSIRNEITYKSLENLGII
**SEQ ID N°1173:** 
MAIRIFAILFSIFSLATFAHAQEGTLERSDWRKFFSEFQAKGTIVVADERQADRAMLV
FDPVRSKKRYSPASTFKIPHTLFALDAGAVRDEFQIFRWDGVNRGFAGHNQDQDLR
SAMRNSTVWVYELFAKEIGDDKARRYLKKIDYGNADPSTSNGDYWIESSLAISAQE
QIAFLRKLYRNELPFRVEHQRLVKDLMIVEAGRNWILRAKTGWEGRMGWWVGWVE
WPTGSVFFALNIDTPNRMDDLFKREAIVRAILRSIEALPPNPAVNSDAAR
**SEQ ID N°1174:** 
MNIKALLLITSAIFISACSPYIVTANPNHSASKSDEKAEKIKNLFNEAHTTGVLVIQQGQ
TQQSYGNDLARASTEYVPASTFKMLNALIGLEHHKATTTEVFKWDGKKRLFPEWEK
NMTLGDAMKASAIPVYQDLARRIGLELMSNEVKRVGYGNADIGTQVDNFWLVGPLK
ITPQQEAQFAYKLANKTLPFSQKVQDEVQSMLFIEEKNGNKIYAKSGWGWDVDPQV
GWLTGWVVQPQGNIVAFSLNLEMKKGIPSSVRKEITYKSLEQLGIL
**SEQ ID N°1175:** 
MNIKALLLITSAIFISACSPYIVTANPNHSASKSDVKAEKIKNLFNEAHTTGVLVIQQGQ
TQQSYGNDLARASTEYVPASTFKMLNALIGLEHHKATTTEVFKWDGKKRLFPEWEK
DMTLGDAMKASAILVYQDLARRIGLELMSKEVKRVGYGNADIGTQVDNFWVVGPLK
ITPQQEAQFAYKLANKTLPFSQKVQDEVQSMLFIEEKNGNKIYAKSGWGWDVNPQV
GWLTGWVVQPQGNIVAFSLNLEMKKGIPSSVRKEITYKSLEQLGIL
**SEQ ID N°1176:** 
MNIKALLLITSAIFISACSPYIVTANPNHSASKSDKKAEKIKNLFNEAHTTGVLVIQQGQ
TQQSYGNDLARASTEYVPASTFKMLNALIGLEHHKATTTEVFKWNGQKRLFPEWEK
DMTLGDAMKASAIPVYQDLARRIGLELMSNEVKRVGYGNADIGTQVDNFWLVGPLK
ITPQQEAQFAYKLANKTLPFSQKVQDEVQSMLFIEEKNGNKIYAKSGWGWDVDPQV
GWLTGWVVQPQGNIVAFSLNLEMKKGIPSSVRKEITYKSLEQLGIL
**SEQ ID N°1177:** 
MNIKALLLITSAIFISACSPYIVTANPNHSASKSDDKAEKIKNLFNEAHTTGVLVIHQGQ
TQQSYGNDLARASTEYVPASTFKMLNALIGLEHHKATTTEVFKWDGEKRLFPEWEK
NMTLGDAMKASAIPVYQDLARRIGLELMSKEVKRVGYGNADIGTQVDNFWVVGPLK
ITPQQEAQFAYKLANKTLPFSQKVQDEVQSMLFIEEKNGNKIYAKSGWGWDVNPQV
GWLTGWVVQPQGNIVAFSLNLEMKKGIPSSVRKEITYKSLEQLGIL
**SEQ ID N°1178:** 
MNIKALLLITSAIFISACSPYIVTTNPNHSASKSDEKAEKIKNLFNEAHTTGVLVIQQGQ
TQQSYGNDLARASTEYVPASTFKMLNALIGLEHHKATTTEVFKWDGKKRLFPEWEK
DMTLGDAMKASAIPVYQDLARRIGLELMSKEVKRVGYGNTDIGTQVDNFWVVGPLK
ITPQQEAQFAYKLANKTLPFSQKVQDEVQSMLFIEEKNGNKIYAKSGWGWDVDPQV
GWLTGWVVQPQGNIVAFSLNLEMKKGIPSSVRKEITYKSLEQLGIL
**SEQ ID N°1179:** 
MNIKALLLITSAIFISACSPYIVTANPNHSASKSDVKAEKIKNLFNEAHTTGVLVIQQGQ
TQQSYGNDLARASTEYVPASTFKMLNALIGLEHHKATTTEVFKWDGKKRLFPEWEK
DMTLGDAMKASAIQVYQDLARRIGLELMSKEVKRVGYGNADIGTQVDNFWLVGPLK
ITPQQEAQFAYKLANKTLPFSQKVQDEVQSMLFIEEKNGNKIYAKSGWGWDVNPQV
GWLTGWVVQPQGNIVAFSLNLEMKKGIPSSVRKEITYKSLEQLGIL
**SEQ ID N°1180:** 
MNIKALLLITSAIFISACSPYIVTANPNHSASKSDDKAEKIKNLFNEAHTTGVLVIHQGQ
TQQSYGNDLARASTEYVPASTFKMLNALIGLEHHKATTTEVFKWDGEKRLFPEWEK
NMTLGDAMKASALPVYQDLARRIGLELMSKEVKRVGYGNADIGTQVDNFWLVGPL
 
KITPQQEAQFAYKLANKTLPFSQKVQDEVQSMLFIEEKNGNKIYAKSGWGWDVNPQ
VGWLTGWVVQPQGNIVAFSLNLEMKKGIPSSVRKEITYKSLEQLGIL
**SEQ ID N°1181:** 
MNIKTLLLITSAIFISACSPYIVSANPNHSASKSDEKAEKIKNLFNEAHTTGVLVIQQGQ
TQQSYGNDLARASTEYVPASTFKMLNALIGLEHHKATTTEVFKWDGQKRLFPEWEK
DMTLGDAMKASAIPVYQDLARRIGLELMSKEVKRVGYGNADIGTQVDNFWLVGPLK
ITPQQEAQFAYKLANKTLPFSPKVQDEVQSMLFIEEMNGNKIYAKSGWGWDVDPQV
GWLTGWVVQPQGNIVAFSLNLEMKKGIPSSVRKEITYKSLEQLGIL
**SEQ ID N°1182:** 
MNIKALLLITSAIFISACSPYIVTANPNHSASKSDDKAEKIKNLFNEAHTTGVLVIHQGQ
TQQSYGNDLARASTEYVPASTFKMLNALIGLEHHKATTTEVFKWDGEKRLFPEWEK
NMTLGDAMKASAIPVYQDLARRIGLELMSKEVKRVGYGNADIGTQVDNFWLVGPLK
ITPQQEAQFAYKLANKTLPFSQKVQDEVQSMLFIEEKNGNKIYAKSGWGWDVNPQV
GWLTGWVVQPQGNIVAFPLNLEMKKGIPSSVRKEITYKSLEQLGIL
**SEQ ID N°1183:** 
MNIKALLLITSAIFISACSPYIVTANPNHSASKSDVKAEKIKNLFNEAHTTGVLVIQQGQ
TQQSYGNDLARASTEYVPASTFKMLNALIGLEHHKATTTEVFKWDGKKRLFPEWEK
DMTLGDAMKASAIPVYQDLARRIGLELMSKEVKRVGYGNADIGTQVDNFWLVGPLK
ITPQQEAQFAYKLANKTLPFSQKVQDEVQSMLFIEEKNGNKIYAKSGWGGDVNPQV
GWLTGWVVQPQGNIVAFSLNLEMKKGIPSSVRKEITYKSLEQLGIL
**SEQ ID N°1184:** 
MNIKALLLITSAIFISACSPYIVTANPNHSASKSDVKAEKIKNLFNEAHTTGVLVIQQGQ
TQQSYGNDLARASTEYVPASTFKMLNALIGLEHHKATTTEVFKWDGKKRLFPEWEK
DMTLGDAMKASAILVYQDLARRIGLELMSKEVKRVGYGNADIGTQVDNFWLVGPLKI
TPQQEAQFAYKLANKTLPFSQKVQDEVQSMLFIEEKNGNKIYAKSGWGWDVNPQV
GWLTGWVVQPQGNIVAFSLNLEMKKGIPSSVRKEITYKSLEQLGIL
**SEQ ID N°1185:** 
MNIKALLLITSAIFISACSPYIVTANPNHSASKSDVKAEKIKNLFNEAHTTGVLVIQQGQ
TQQSYGNDLARASTEYVPASTFKMLNALIGLEHHKATTTEVFKWDGKKRLFPEWEK
DMTLGDAMKASAIPVYQDLARRIGLELMSKEVKRVGYGNADIGTQVDNFWVVGPLK
ITPQQEAQFAYKLANKTLPFSQKVQDEVQSMLFIEEKNGNKIYAKSGWGWDVNPQV
GWLTGWVVQPQGNIVAFSLNLEMKKGIPSSVRKEITYKSLEQLGIL
**SEQ ID N°1186:** 
MNIKALLLITSAIFISACSPYIVTANPNHSASKSDEKAEKIKNLFNEAHTTGVLVIQQGQ
TQQSYGNDLARASTEYVPASTFKMLNALIGLEHHKTTTTEVFKWDGQKRLFPEWEK
DMTLGDAMKASAIPVYQDLARRIGLELMSKEVKRVGYGNADIGTQVDNFWVVGPLK
ITPQQEAQFAYKLANKTLPFSPKVQDEVQSMLFIEEKNGNKIYAKSGWGWDVDPQV
GWLTGWVVQPQGNIVAFSLNLEMKKGIPSSVRKEITYKSLEQLGIL
**SEQ ID N°1187:** 
MSKKNFILIFIFVILISCKNTEKISNETTLIDNIFTNSNAEGTLVIYNLNDDKYIIHNKERAE
QRFYPASTFKIYNSLIGLNEKAVKDVDEVFYKYNGEKVFLESWAKDSNLRYAIKNSQ
VPAYKELARRIGLKKMKENIEKLDFGNKSIGDSVDTFWLEGPLEISAMEQVKLLTKLA
QNELPYPIEIQKAVSDITILEQTYNYTLHGKTGLADSKNMTTEPIGWFVGWLEENDNI
YVFALNIDNINSDDLAKRINIVKESLKALNLLK
**SEQ ID N°1188:** 
MSKKNFILIFIFVILTSCKNTEKISNETTLIDNIFTNSNAEGTLVIYNLNDDKYIIHNKERA
EQRFYPASTFKIYNSLIGLNEKAVKDVDEVFYKYNGEKVFLESWAKDSNLRYAIKNS
QVPAYKELARRIGLKKMKENIEKLDFGNKSIGDSVDTFWLEGPLEISAMEQIKLLTKL
AQNELPYPIEIQKAVSDITILEQTYNYTLHGKTGLADSKNMTTEPIGWFVGWLEENDN
IYVFALNIDNINSDDLAKRINIVKESLKALNLLK
**SEQ ID N°1189:** 
LLITSAIFISACSPYIVSANPNHSASKSDDKAEKIKNLFNEAHTTGVLVIQQGQTQQSY
GNDLARASTEYVPASTFKMLNALIGLEHHKATTTEVFKWDGQKRLFPEWEKNMTLG
DAMKASAIPVYQDLARRIGLELMSNEVKRVGYGNADIGTQVDNFWLVGPLKITPQQ
EAQFAYKLANKTLPFSQKVQDEVQSMLFIEEKNGNKIYAKSGWGWDVDPQVGWLT
GWVVQPQGNIVAFSLNLEMKKGIPSSVRKEITYKSL
**SEQ ID N°1190:** 
LLITSAIFISACSPYIVSANPNHSASKSDEKAEKIKNLFNEAHTTGVLVIQQGQTQQSY
GNDLARASTEYVPASTFKMLNALIGLEHHKATTTEVFKWDGQKRLFPEWEKNMTLG
DAMKASAIPVYQDLARRIGLELMSNEVKRVGYGNADIGTQVDNFWLVGPLKITPQQ
EAQFAYKLANKTLPFSQKVQDEVQSMLFIEEKNGNKIYAKSGWGWDVNPQVGWLT
EWVVQPQGNIVAFSLNLEMKKGIPSSVRKEITYKSL
**SEQ ID N°1191:** 
MTVRRLSCALGAALSLSALGGGPVQAAVLCTVVADAADGRILFQQGTQQACAERYT
PASTFKLAIALMGADAGILQGPHEPVWNYQPAYPDWGGDAWRQPTDPARWIKYSV
VWYSQLTAKALGQDRFQRYTSAFGYGNADVSGEPGKHNGTDGAWIISSLRISPLEQ
LAFLRKLVNRQLPVKAAAYELAENLFEAGQADGWRLYGKTGTGSPGSNGVYTAAN
AYGWFVGWARKDGRQLVYARLLQDERATRPNAGLRARDELVRDWPAMAGAWRP
**SEQ ID N°1192:** 
MNIKALLLITSAIFISACSPYIVSANPNHSASKSDEKAEKIKNLFNEAHTTGVLVIQQGQ
TQQSYGNVLARASTEYVPASTFKMLNALIGLEHHKATTTEVFKWDGQKRLFPEWEK
NMTLGDAMKASAIPVYQDLARRIGLELMSNEVKRVGYGNADIGTQVDNFWLVGPLK
ITPQQEAQFAYKLANKTLPFSQEVQDEVQSMLFIEEKNGNKIYAKSGWGWDVNPQV
GWLTGWVVQPQGNIVAFSLNLEMKKGIPSSVRKEITYKSLEQLGIL
**SEQ ID N°1193:** 
SEQ ID N[?] [?][?][?][?]:
MKTFAAYVIIACLSSTALAGSITENTSWNKEFSAEAVNGVFVLCKSSSKSCATNDLAR
ASKEYLPASTFKIPSAIIGLETGVIKNEHQVFKWDGKPRAMKQWERDLTLRGAIQVS
AVPVFQQIAREVGEVRMQKYLKKFSYGNQNISGGIDKFWLEDQLRISAVNQVEFLES
LYLNKLSASKENQLIVKEALVTEAAPEYLVHSKTGFSGVGTESNPGVAWWVGWVEK
ETEVYFFAFNMDIDNESKLPLRKSIPTKIMESEGIIGG
**SEQ ID N°1194:** 
MNIKTLLLITSAIFISACSPYIVTANPNHSASKSDVKAEKIKNLFNEAHTTGVLVIQQGQ
TQQSYGNDLARASTEYVPASTFKMLNALIGLEHHKATTTEVFKWDGKKRLFPEWEK
DMTLGDAMKASAIPVYQDLARRIGLELMSKEVKRVGYGNADIGTQVDNFWVVGPLK
ITPQQEAQFAYKLANKTLPFSQKVQDEVQSMLFIEEKNGNKIYAKSGWGWDVNPQV
GWLTGWVVQPQGNIVAFSLNLEMKKGIPSSVRKEITYKSLEQLGIL
**SEQ ID N°1195:** 
SEQ ID NO: [?]
MNIKALLLITSAIFISACSPYIVTTNPNHSASKSDEKAEKIKNLFNEAHTTGVLVIQQGQ
TQQSYGNDLARASTEYVPASTFKMLNALIGLEHHKATTTEVFKWDGKKRLFPEWEK
DMTLGDAMKASAIPVYQDLARRIGLELMSKEVKRVGYGNTDIGTQVDNFWLVGPLKI
TPQQEAQFAYKLANKTLPFSQKVQDEVQSMLFIEEKNGNKIYAKSGWGWDVDPQV
GWLTGWVVQPQGNIVAFSLNLEMKKGIPSSVRKEITYKSLEQLGIL
**SEQ ID N°1196:** 
MNIKALLLITSAIFISACSPYIVTANPNHSASKSDVKAEKIKNLFNEAHTTGVLVIQQGQ
TQQSYGNDLARASTEYVPASTFKMLNALIGLEHHKATTTEVFKWDGKKRLFPEWEK
DMTLGDAMKASAVPVYQDLARRIGLELMSKEVKRVGYGNADIGTQVDNFWLVGPL
KITPQQEAQFAYKLANKTLPFSQKVQDEVQSMLFIEEKNGNKIYAKSGWGWDVNQ
QVGWLTGWVVQPQGNIVAFSLNLEMKKGIPSSVRKEITYKSLEQLGIL
**SEQ ID N°1197:** MNIKTLLLITSAIFISACSPYIVTANPNHSASKSDEKAEKIKNLFNEVHTTGVLVIQQGQ 
TQQSYGNDLARASTEYVPASTFKMLNALIGLEHHKATTTEVFKWDGQKRLFPEWEK
DMTLGDAMKASAIPVYQDLARRIGLELMSKEVKRVGYGNADIGTQVDNFWLVGPLK
ITPQQEAQFAYKLANKTLPFSPKVQDEVQSMLFIEEKNGNKIYAKSGWGWDVDPQV
GWLTGWVVQPQGNIVAFSLNLEMKKGIPSSVRKEMTYKSLEQLGIL
**SEQ ID N°1198:** 
MNKYFTCYVVASLFFSGCTVQHNLINETQSQIVQGHNQVIHQYFDEKNTSGVLVIQT
DKKINLYGNALSRANTEYVPASTFKMLNALIGLENQKTDINEIFKWKGEKRSFTTWE
KDMTLGEAMKLSAVPVYQELARRIGLDLMQKEVERIDFGNAEIGQQVDNFWLIGPLK
VTPIQEVEFVSQLAHTQLPFSEKVQANVKNMLLLEENNGYKIFGKTGWAMDIKPQV
GWLTGWVEQPDGKIVAFALNMEMRSEMPASIRNELLMKSLKQLNII
**SEQ ID N°1199:** 
MNKYFTCYVVASLFLSGCTVQHNLINETPSQIVQGHNQVIHQYFDEKNTSGVLVIQT
DKKINLYGNALSRANTEYVPASTFKMLNALIGLENQKTDINEIFKWKGEKRSFTAWE
KDMTLGEAMKLSAVPVYQELARRIGLDLMQKEVKRIGFGNAEIGQQVDNFWLVGPL
KVTPIQEVEFVSQLAHTQLPFSEKVQANVKNMLLLEESNGYKIFGKTGWAAMDIKPQ
VGWLTGWVEQPDGKIVAFALNMEMRSEMPASIRNELLMKSLKQLNII
**SEQ ID N°1200:** 
MKTFAAYVITACLSSTALASSITENTSWNKEFSAEAVNGVFVLCKSSSKSCATNNLA
RASKEYLPASTFKIPNAIIGLETGVIKNEHQVFKWDGKPRAMKQWERDLSLRGAIQV
SAVPVFQQIAREVGEVRMQKYLKKFSYGNQNISGGIDKFLLEGQLRISAVNQVEFLE
SLFLNKLSASKENQLIVKEALVTEAAPEYLVHSKTGFSGVGTESNPGVAWWVGWVE
KGTEVYFFAFNMDIDNENKLPLRKSIPTKIMASEGIIGG
**SEQ ID N°1201:** 
MAIRIFAILFSIFSLATFAHAQEGTLERSDWRKFFSEFQAKGTIVVADERQADRAMLV
FDPVRSKKRYSPASTFKIPHTLFALDAGAVRDEFQIFRWDGVNRGFAGHNQDQDLR
SAMRNSTVWVYELFAKEIGDDKARRYLKKIDYGDADPSTSNGDYWIEGSLAISAQE
QIAFLRKLYRNELPFRVEHQRLVKDLMIVEAGRNWILRAKTGWEGRMGWWVGWVE
WPTGSVFFALNIDTPNRMDDLFKREAIVRAILRSIEALPPNPAVNSDAAR
**SEQ ID N°1202:** 
MNIKALLLITSAIFISACSPYIVTANPNHSASKSDKKAEKIKNLFNEAHTTGVLVIQQGQ
TQQSYGNDLARASTEYVPASTFKMLNALIGLEHHKASTTEVFKWNGQKRLFPEWEK
DMTLGDAMKASAIPVYQDLARRIGLELMSNEVKRVGYGNADIGTQVDNFWLVGPLK
ITPQQEAQFAYKLANKTLPFSQKVQDEVKSMLFIEEKNGNKIYAKSGWGWDVDPQV
GWLTGWVVQPQGNIVAFSLNLEMKKGIPSSVRKEITYKSLEQLGIL
**SEQ ID N°1203:** 
MNIKALLLITSAIFISACSPYIVSANPNHSASKSDEKAEKIKNLFNEAHTTGVLVIQQGQ
TQQSYGNDLARASTEYVPASTFKMLNALIGLEHHKATTTEVFKWDGQKRLFPEWEK
NMTLGDAMKASAIPVYQDLARRIGLELMSNEVKHVGYGNADIGTQVDNFWLVGPLK
ITPQQEAQFAYKLANKTLPFSPKVQDEVQSMLFIEEKNGNKIYAKSGWGWDVNPQV
GWLTGWVVQPQGNIVAFSLNLEMKKGIPSSVRKEITYKSLEQLGIL
**SEQ ID N°1204:** 
MNIKALLLITSAIFISACSPYIVSANPNHSASKSDEKAEKIKNLFNEAHTTGVLVIQQGQ
TQQSYGNDLARASTEYVPASTFKMLNALIGLEHHKATTTEVFKWDGQKRLFPEWEK
NMTLGDAMKASAIPVYQDLARRIGLELMSNEVKHVGYGNADIGTQVDNFWLVGPLK
ITPQQEAQFAYKLANKTLPFSQKVQDEVQSMLFIEEKNGNKIYAKSGWGWDVNPQV
GWLTGWVVQPQGNIVAFSLNLEMKKGIPSSVRKEITYKSLEQLGIL
**SEQ ID N°1205:** 
MKKFILPILSISTLLSVSACSSIQTKFEDTFHTSNQQHEKAIKSYFDEAQTQGVIIIKKG
KNISTYGNNLTRAHTEYVPASTFKMLNALIGLENHKATTTEIFKWDGKKRSYPMWEK
DMTLGDAMALSAVPVYQELARRTGLDLMQKEVKRVGFGNMNIGTQVDNFWLVGPL
 
KITPIQEVNFADDFANNRLPFKLETQEEVKKMLLIKEFNGSKIYAKSGWGMDVTPQV
GWLTGWVEKSNGEKVAFSLNIEMKQGMPGSIRNEITYKSLENLGII
**SEQ ID N°1206:** 
MNIKALLLITSAIFISACSPYIVSANPNHSASKSDEKAEKIKNLFNEAHTTGVLVIQQGQ
TQQSYGNDLARASTEYVPASTFKMLNALIGLEHHKATTTEVFKWDGQKRLFPEWEK
NMTLGDAMKASAIPVYQDLARRIGLELMSNEVKRVGYGNADIGTQVDNFWLVGPLK
ITPQQEAQFAYKLANKTLPFSQEVQDEVQSILFIEEKNGNKIYAKSGWGWDVNPQV
GWLTGWVVQPQGNIVAFSLNLEMKKGIPSSVRKEITYKSLEQLGIL
**SEQ ID N°1207:** 
MKKFILPIFSISILVSLSACSSIKTKSEDNFHISSQQHEKAIKSYFDEAQTQGVIIIKEGK
NLSTYGNALARANKEYVPASTFKMLNALIGLENHKATTNEIFKWDGKKRTYPMWEK
DMTLGEAMALSAVPVYQELARRTGLELMQKEVKRVNFGNTNIGTQVDNFWLVGPL
KITPVQEVNFADDLAHNRLPFKLETQEEVKKMLLIKEVNGSKIYAKSGWGMGVTSQV
GWLTGWVEQANGKKIPFSLNLEMKEGMSGSIRNEITYKSLENLGII
**SEQ ID N°1208:** 
MRVLALSAVFLVASIIGMPAVAKEWQENKSWNAHFTEHKSQGVVVLWNENKQQGF
TNNLKRANQAFLPASTFKIPNSLIALDLGVVKDEHQVFKWDGQTRDIATWNRDHNLI
TAMKYSVVPVYQEFARQIGEARMSKMLHAFDYGNEDISGNVDSFWLDGGIRISATE
QISFLRKLYHNKLHVSERSQRIVKQAMLTEANGDYIIRAKTGYSARIEPKIGWWVGW
VELDDNVWFFAMNMDMPTSDGLGLRQAITKEVLKQEKIIP
**SEQ ID N°1209:** 
MNIKALLLITSAIFISACSPYIVTANPNHSASKSDVKAEKIKNLFNEAHTTGVLVIQQGQ
TQQSYGNDLARASTEYVPASTFKMLNALIGLEHHKATTTEVFKWDGKKRLFPEWEK
DMTLGDAMKASAVPVYQDLARRIGLELMSKEVKRVGYGNADIGTQVDNFWLVGPL
KITPQQEAQFAYKLANKTLPFSQKVQDEVQSMLFIEEKNGNKIYAKSGWGLDVNPQ
VGWLTGWVVQPQGNIVAFSLNLEMKKGIPSSVRKEITYKSLEQLGIL
**SEQ ID N°1210:** 
MNIKALLLITSAIFISACSPYIVTANPNHSASKSDVKAEKIKNLFNEAHTTGVLVIQQGQ
TQQSYGNDLARASTEYVPASTFKMLNALIGLEHHKATTTEVFKWDGKKRLFPEWEK
DMTLGDAMKASAVPVYQDLARRIGLELMSKEVKRVGYGNADIGTQVDNFWLVGPL
KITPQQEAQFAYKLANKTLPFSQKVQDEVQSMLFIEEKNGNKIYAKSGWGLDVNLQ
VGWLTGWVVQPQGNIVAFSLNLEMKKGIPSSVRKEITYKSLEQLGIL
**SEQ ID N°1211:** 
MNIKALLLITSAIFISACSPYIVTANPNHSASKSDVKAEKIKNLFNEAHTTGVLVIQQGQ
TQQSYGNDLARASTEYVPASTFKMLNALIGLEHHKATTTEVFKWDGKKRLFPEWEK
DMTLGDAMKASAIPVYQDLARRIGLELMSKEVKRVGYGNADIGTQVDNFWVVGPLK
ITPQQEAQFAYKLANKTLPFSQKVQDEVQSMLFIEEKNGNKIYAKSGWGWDVNPQV
GWLTGWVVQPQGNIVAFSLNLEMKKGIPSSVRKEITYKSLEKLGIL
**SEQ ID N°1212:** 
MNIKALLLITSAIFISACSPYIVTANPNHSASKSDVKAEKIRNLFNEAHTTGVLVIQQGQ
TQQSYGNDLARASTEYVPASTFKMLNALIGLEHHKATTTEVFKWDGKKRLFPEWEK
DMTLGDAMKASAIPVYQDLARRIGLELMSKEVKRVGYGNADIGTQVDNFWVVGPLK
ITPQQEAQFAYKLANKTLPFSQKVQDEVQSMLFIEEKNGNKIYAKSGWGWDVNPQV
GWLTGWVVQPQGNIVAFSLNLEMKKGIPSSVRKEITYKSLEQLGIL
**SEQ ID N°1213:** 
MNIKALLLITSAIFISACSPYIVTANPNHSASKSDVKAEKIKNLFNEAHTTGVLVIQQGQ
TQQSYGNDLARASTEYVPASTFKMLNALIGLEHHKATTTEVFKWDSKKRLFPEWEK
DMTLGDAMKASAIPVYQDLARRIGLELMSKEVKRVGYGNADIGTQVDNFWVVGPLK
ITPQQEAQFAYKLANKTLPFSQKVQDEVQSMLFIEEKNGNKIYAKSGWGWDVNPQV
GWLTGWVVQPQGNIVAFSLNLEMKKGIPSSVRKEITYKSLEQLGIL
**SEQ ID N°1214:** 
MNIKALLLITSAIFISACSPYIVTANPNHSASKSDVKAEKIKNLFNEAHTTGVLVIQQGQ
TQQSYGNDLARASTEYVPASTFKMLNALIGLEHHKATTTEVFKWDGKKRLFPEWEK
DMTLGDAMKASAIPVYQDLARRIGLELMSKEVKRVGYGNADIGTQVDNFWVVGPLK
ITPQQEAQFAYKLANKTLPFSQKVQDGVQSMLFIEEKNGNKIYAKSGWGWDVNPQ
VGWLTGWVVQPQGNIVAFSLNLEMKKGIPSSVRKEITYKSLEQLGIL
**SEQ ID N°1215:** 
MNIKALLLITSAIFISACSPYIVTANPNHSASKSDVKAEKIKNLFNEAHTTGVLVIQQGQ
TQQSYGNDLARASTEYVPASTFKMLNALIGLEHHKATTTEVFKWDSKKRLFPEWEK
DMTLGDAMKASAILVYQDLARRIGLELMSKEVKRVGYGNADIGTQVDNFWLVGPLKI
TPQQEAQFAYKLANKTLPFSQKVQDEVQSMLFIEEKNGNKIYAKSGWGWDVNPQV
GWLTGWVVQPQGNIVAFSLNLEMKKGIPSSVRKEITYKSLEQLGIL
**SEQ ID N°1216:** 
MKLLKILSLVCLSISIGACAEHSMSRAKTSTIPQVNNSIIDQNVQALFNEISADAVFVTY
DGQNIKKYGTHLDRAKTAYIPASTFKIANALIGLENHKATSTEIFKWDGKPRFLKAWD
KDFTLGEAMQASTVPVYQELARRIGPSLMQSELQRIGYGNMQIGTEVDQFWLKGPL
TITPIQEVKFVYDLAQGQLPFKPEVQQQVKEMLYVERRGENRLYAKSGWGMAVDP
QVGWYVGFVEKADGQVVAFALNMQMKAGDDIALRKQLSLDVLDKLGVFHYL
**SEQ ID N°1217:** 
MNKYFTCYVVASLFLSGCTVQHNLINETPSQIVQGHNQVIHQYFDEKNTSGVLVIQT
DKKINLYGNALSRANTEYVPASTFKMLNALIGLENQKTDINEIFKWKGEKRSFTAWE
KDMTLGEAMKLSAVPVYQELARRIGLDLMQKEVKRIGFGNAEIGQQVDNFWLVGPL
KVTPIQEVEFVSQLAHTQLPFSEKVQANVKNMLLLEESNGYKIFGKTGWAMDVKPQ
VGWLTGWVEQPDGKIVAFALNMEMRSEMPASIRNELLMKSLKQLNII
**SEQ ID N°1218:** 
MNKYFTCYVVASLFLSGCTVQHNLINETPSQIVQGHNQVIHQYFDEKNTSGALVIQT
DKKINLYGNALSRANTEYVPASTFKMLNALIGLENQKTDINEIFKWKGEKRSFTAWE
KDMTLGEAMKLSAVPVYQELARRIGLDLMQKEVKRIGFGNAEIGQQVDNFWLVGPL
KVTPIQEVEFVSQLAHTQLPFSEKVQANVKNMLLLEESNGYKIFGKTGWAMDIKPQV
GWLTGWVEQPDGKIVAFALNMEMRSEMPASIRNELLMKSLKQLNII
**SEQ ID N°1219:** 
MNKYFTCYVVASLFLSGCTVQHNLINETPSQIVQGHNQVIHQYFDEKNTSGVLVIQT
DKKINLYGNALSRANTEYVPASTFKMLNALIGLENQKTDINEIFKWKGEKRSFTAWE
KDMTLGEAMKLSAVPVYQELARRIGLDLMQKEVKRIGFGNAEIGQQVDNFWLVGPL
KVTPIQEVEFVSQLAHTQLPFSEKVQANVKNMLLLEESNGYKIFGKTGWAMDIKPQV
GWLTGWVEQPDGKIVAFALNMEMRSEMPASIRNELMMKSLKQLNII
**SEQ ID N°1220:** 
MNKYFTCYVVASLFLSGCTVQHNLINETPSQIVQGHNQVIHQYFDEKNTSGVLVIQT
DKKINLYGNALSRANTEYVPASTFKMLNALIGLENQKTDINEIFKWKGEKRSFTAWE
KDMTLGEAMKLSAVPVYQELARRIGLDLMQKEVKRIGFGNAEIGQQVDNFWLVGPL
KVTPIQEVEFVSQLAHTQLPFSEKVQANVKNMLLLEESNGYKIFGKTGWAMDIKPQV
GWLAGWVEQPDGKIVAFALNMEMRSEMPASIRNELLMKSLKQLNII
**SEQ ID N°1221:** 
MNKYFTCYVVASLFLSGCTVQHNLINETPSQIVQGHNQVIHQYFDERNTSGVLVIQT
DKKINLYGNALSRANTEYVPASTFKMLNALIGLENQKTDINEIFKWKGEKRSFTAWE
KDMTLGEAMKLSAVPVYQELARRIGLDLMQKEVKRIGFGNAEIGQQVDNFWLVGPL
KVTPIQEVEFVSQLAHTQLPFSEKVQANVKNMLLLEESNGYKIFGKTGWAMDIKPQV
GWLTGWVEQPDGKIVAFALNMEMRSEMPASIRNELLMKSLKQLNII
**SEQ ID N°1222:** MNKYFTCYVVASLFLSGCTVQHNLINETPSQIVQGHNQVIHQYFDEKNTSGVLVIQT 
DKKINLYGNALSRANTEYVPASTFKMLNALIGLENQKTDINEIFKWKGEKRSFTAWE
KDMTLGEAMKLSAVPVYQELARRIGLDLMQKEVKRIGFGNAEIGQQVDNFWLVGPL
KVTPIQEVEFVSQLAHTQLPFSEKVQANVKNMLLLEKSNGYKIFGKTGWAMDIKPQV
GWLTGWVEQPDGKIVAFALNMEMRSEMPASIRNELLMKSLKQLNII
**SEQ ID N°1223:** 
MNKYFTCYVVASLFLSGCTVQHNLINETPSQIVQGHNQVIHQYFDEKNTSGVLVIQT
DKKINLYGNALSRANTEYVPASTFKMLNALIGLENQKTDINEIFKWKGEKRSFTAWE
KDMTLGEAMKLSAVPVYQELARRIGLDLMQKEVKRIGFGNAEIGQQVDNFWLVGPL
KVTPIQEVEFVSQLAHTQLPFSEKVQANVKNMLLLEESNGYKIFGKTGWAMDIKPQV
GWLTGWVEQPDGKIVAFALNMEMRSEMPASTRNELLMKSLKQLNII
**SEQ ID N°1224:** 
MKKFILPIFSISILLSLSACSSIQTKFEDTFHISNQKHEKAIKSYFDEAQTQGVIIIKEGKN
ISSYGNNLVRAHTEYVPASTFKMLNALIGLENHKATTNEIFKWDGKKRSYPMWEKD
MTLGEAMALSAVPVYQDLARRIGLNLMQKEVKRVGFGNMNIGTQVDNFWLIGPLKI
TPIQEVNFADDLANNRLPFKLETQEEVKKMLLIKEVNGSKIYAKSGWGMDVSPQVG
WLTGWVEKSNGEKVSFSLNIEMKQGMSGSIRNEITYKSLENLGII
**SEQ ID N°1225:** 
MNIKALLLITSAIFISACSPYIVTANPNHSASKSDEKAEKIKNLFNEAHTTGVLVIQQGQ
TQQSYGNDLARASTEYVPASTFKMLNALIGLEHHKATTTEVFKWDGQKRLFPEWEK
DMTLGDAMKASAIAVYQDLARRIGLELMSKEVKRVGYGNADIGTQVDNFWLVGPLK
ITPQQEAQFAYKLANKTLPFSPKVQDEVQSMLFIEEKNGNKIYAKSGWGWDVDPQV
GWLTGWVVQPQGNIVAFSLNLEMKKGIPSSVRKEITYKSLEQLGIL
**SEQ ID N°1226:** 
MNIKALLLITSAIFISACSPYIVTANPNHSASKSDEKAEKIKNLFNEAHTTGVLVIQQGQ
TQQSYGNDLARASTEYVPASTFKMLNALIGLEHHKATTTEVFKWDGQKRLFPEWEK
DMTLGDAIKASAIPVYQDLARRIGLELMSKEVKRVGYGNADIGTQVDNFWLVGPLKI
TPQQEAQFAYKLANKTLPFSQKVQDEVQSMLFIEEKNGNKIYAKSGWGWDVDPQV
GWLTGWVVQPQGNIVAFSLNLEMKKGIPSSVRKEITYKSLEQLGIL
**SEQ ID N°1227:** 
MKILIFLPLLSCLGLTACSLPVSSLPSQSISTQAIASLFDQAQSSGVLVIQRDQQVQVY
GNDLNRANTEYVPASTFKMLNALIGLQHGKATTNEIFKWDGKKRSFTAWEKDMTLG
QAMQASAVPVYQELARRIGLELMQQEVQRIQFGNQQIGQQVDNFWLVGPLKVTPK
QEVQFVSALAREQLAFDPQVQQQVKAMLFLQERKAYRLYVKSGWGMDVEPQVGW
LTGWVETPQAEIVAFSLNMQMQNGIDPAIRLEILQQALAELGLYPKAEG
**SEQ ID N°1228:** 
MHKHMSKLFIAFLAFLLSVPAAAEDQTLAELFAQQGIDGTIVISSLHNGKTFIHNDPRA
KQRFSTASTFKILNTLISLEEKAISGKDDVLKWDGHIYDFPDWNRDQTLESAFKVSCV
WCYQALARQVGAEKYRNYLRKSVYGELREPFEETTFWLDGSLQISAIEQVNFLKKV
HLRTLPFSASSYETLRQIMLIEQTPAFTLRAKTGWATRVKPQVGWYVGHVETPTDV
WFFATNIEVRDEKDLPLRQKLTRKALQAKGIIE
**SEQ ID N°1229:** 
MKTFAAYVITACLSSTALASSITENTSWNKEFSAEAVNGVFVLCKSSSKSCATNNLA
RASKEYLPASTFKIPNAIIGLETGVIKNEHQVFKWDGKPRAMKQWERDLSLRGAIQV
SAVPVFQQIAREVGEVRMQKYLKKFSYGNQNISGGTDKFWLEDQLRISAVNQVEFL
ESLFLNKLSASKENQLIVKEALVTEAAPEYLVHSKTGFSGVGTESNPGVAWWVGWV
EKGTEVYFFAFNMDIDNENKLPLRKSIPTKIMASEGIIGG
**SEQ ID N°1230:** 
MNIKALLLITSAIFISACSPYIVTANPNHSASKSDEKAEKIKNLFNEAHTTGVLVIQQGQ
TQQSYGNDLARASTEYVPASTFKMLNALIGLEHHKATTTEVFKWDGKKRLFPEWEK
DMTLGDAMKASAILVYQDLARRIGLELMSKEVKRVGYGNADIGTQVDNFWLVGPLKI
 
TPQQEAQFAYKLANKTLPFSQKVQDEVQSMLFIEEKNGNKIYAKSGWGLDVDPQV
GWLTGWVVQPQGNIVAFSLNLEMKKGIPSSVRKEITYKSLEQLGIL
**SEQ ID N°1231:** 
MNIKALLLITSAIFISACSPYIVTANPNHSASKSDVKAEKIKNLFNEAHTTGVLVIQQGQ
TQQSYGNDLARASTEYVPASTFKMLNALIGLEHHKATTTEVFKWDGKKRLFPEWEK
DMTLGDAMKASAIQVYQDLARRIGLELMSKEVKRVGYGNADIGTQVDNFWVVGPLK
ITPQQEAQFAYKLANKTLPFSQKVQDEVQSMLFIEEKNGNKIYAKSGWGWDVNPQV
GWLTGWVVQPQGNIVAFSLNLEMKKGIPSSVRKEITYKSLEQLGIL
**SEQ ID N°1232:** 
MNIKALLLITSAIFISACSPYIVTANPNHSASKSDVKAEKIKNLFNEAHTTGVLVIQQGQ
TQQSYGNDLARASTEYVPASTFKMLNALIGLEHHKATTTEVFKWDGKKRLFPEWEK
DMTLGDAMKASAMPVYQDLARRIGLELMSKEVKRVGYGNADIGTQVDNFWLVGPL
KITPQQEAQFAYKLANKTLPFSQKVQDEVQSMLFIEEKNGNKIYAKSGWGWDVNPQ
VGWLTGWVVQPQGNIVAFSLNLEMKKGIPSSVRKEITYKSLEQLGIL
**SEQ ID N°1233:** 
MNIKALLLITSAIFISACSPYIVTANPNHSASKSDEKAEKIKNLFNEVHTTGVLVIQQGQ
TQQSYGNDLARASTEYVPASTFKMLNALIGLEHHKATTTEVFKWDGKKRLFPEWEK
NMTLGDAMKASAIPVYQDLARRIGLELMSNEVKRVGYGNADIGTQVDNFWLVGPLK
ITPQQEAQFAYKLANKTLPFSQKVQDEVQSMLFIEEKNGNKIYAKSGWGWDVDPQV
GWLTGWVVQPQGNIVAFSLNLEMKKGIPSSVRKEITYKSLEQLGIL
**SEQ ID N°1234:** 
MRVLALSAVFLVASIIGMPAVAKEWQENKSWNAHFTEHKSQGVVVLWNENKQQGF
TNNLKRANQAFLPASTFKIPNSLIALDLGVVKDEHQVFKWDGQTRDIATWNRDHNLI
TAMKYSVVPVYQEFARQIGEARMSKMLHAFDYGNEDISGNVDSFWLDGGIRISATE
QISFLRKLYHNKLHVSERSQRIVKQAMLTEANGDYIIRAKTGYDTKIGWWVGWVELD
DNVWFFAMNMDMPTSDGLGLRQAITKEVLKQEKIIP
**SEQ ID N°1235:** 
MSKKNFILIFIFVILISCKNTEKTSNETTLIDNIFTNSNAEGTLVIYNLNDDKYIIHNKERA
EQRFYPASTFKIYNSLIGLNEKAVKDVDEVFYKYNGEKVFLESWAKDSNLRYAIKNS
QVPAYKELARRIGLEKMKENIEKLDFGNKNIGDSVDTFWLEGPLEISAMEQVKLLTKL
AQNELPYPIEIQKAVSDITILEQTDNYTLHGKTGLADSENMTTEPIGWLVGWLEENNN
IYVFALNIDNINSDDLAKRINIVKESLKALNLLK
**SEQ ID N°1236:** 
MNIKALFLITSAIFISACSPYIVTANPNHSASKSDVKAEKIKNLFNEAHTTGVLVIQQGQ
TQQSYGNDLARASTEYVPASTFKMLNALIGLEHHKATTTEVFKWDGKKRLFPEWEK
DMTLGDAMKASAIPVYQDLARRIGLELMSKEVKRVGYGNADIGTQVDNFWVVGPLK
ITPQQEAQFAYKLANKTLPFSQKVQDEVQSMLFIEEKNGNKIYAKSGWGWDVNPQV
GWLTGWVVQPQGNIVAFSLNLEMKKGIPSSVRKEITYKSLEQLGIL
**SEQ ID N°1237:** 
MNIKALFLITSAIFISACSPYIVTANPNHSASKSDVKAEKIKNLFNEAHTTGVLVIQQGQ
TQQSYGNDLARASTEYVPASTFKMLNALIGLEHHKATTTEVFKWDGKKRLFPEWEK
DMTLGDAMKASAIPVYQDLARRIGLELMSKEVKRVGYGNADIGTQVDNFWVVGPLK
ITPQQEAQFAYKLANKTLPSSQKVQDEVQSMLFIEEKNGNKMYAKSGWGWDVNPQ
VGWLTGWVVQPQGNIVAFSLNLEMKKGIPSSVRKEITYKSLEQLGIL
**SEQ ID N°1238:** 
MNIKALFLITSAIFISACSPYIVTANPNHSASKSDVKAEKIKNLFNEAHTTGVLVIQQGQ
TQQSYGNDLARASTEYVPASTFKMLNALIGLEHHKATTTEVFKWDGKKRLFPEWEK
DMTLGDAMKASAIPVYQDLARRIGLELMSKEVKRVGYGNADIGTQVDNFWVVGPLK
ITPQQEAQFAYKLANKTLPFSQKVQDEVQSMLFIEEKNGNKIYAKSGWGWDVNPQV
GWLTGWVVQPQGNIVAFSLNLEMKKGIPSSVRKEIAYKSLEQLGIL
**SEQ ID N°1239:** 
MAIRIFAILFSIFSLATFAHAQEGTLERSDWRKFFSEFQAKGTIVVADERQADRAMLV
FDPVRSKKRYSPASTFKIPHTLFALDAGAVRDEFQIFRWDGVNRGFAGHNQDQDLR
SAMRNSTVWVYELFAKEIGDDKARRYLKKIDYGNADPSTSNGDCWIEGSLAISAQE
QIAFLRKLYRNELPFRVEHQRLVKDLMIVEAGRNWILRAKTGWEGRMGWWVGWVE
WPTGSVFFALNIDTPNRMDDLFKREAIVRAILRSIEALPPNPAVNSDAAR
**SEQ ID N°1240:** 
MNIKTLLLITSAIFISACSPYIVTANPNHSASKSDEKAEKIKNLFNEVHTTGVLVIQQGQ
TQQSYGNDLARASTEYVPASTFKMLNALIGLEHHKATTTEVFKWDGQKRLFPEWEK
DMTLGDAMKASAIPVYQDLARRIGLELMSKEVKRVGYGNADIGTQVDNFWVVGPLK
ITPQQEAQFAYKLANKTLPFSPKVQDEVQSMLFIEEKNGNKIYAKSGWGWDVDPQV
GWLTGWVVQPQGNIVAFSLNLEMKKGIPSSVRKEITYKSLEQLGIL
**SEQ ID N°1241:** 
MNIKTLLLITSAIFISACSPYIVTANPNHSASKSDEKAEKIKNLFNEVHTTGVLVIQQGQ
TQQSYGNDLARASTEYVPASTFKMLNALIGLEYHKATTTEVFKWDGQKRLFPEWEK
DMTLGDAMKASAIPVYQDLARRIGLELMSKEVKRVGYGNADIGTQVDNFWLVGPLK
ITPQQEAQFAYKLANKTLPFSPKVQDEVQSMLFIEEKNGNKIYAKSGWGWDVNPQV
GWLTGWVVQPQGNIVAFSLNLEMKKGIPSSVRKEITYKSLEQLGIL
**SEQ ID N°1242:** 
MAIRFLTILLSTFFLTSFVHAQEHVVVRSDWKKFFSDLQAEGAIVIADERQAEHALLV
FGQERAAKRYSPASTFKLPHTLFALDAGAVRDEFQVFRWDGVKRSFAGHNQDQDL
RSAMRNSAVWVYELFAKEIGEDNARRYLKQIDYGNADPSTIKGNYWIDGNLEISAHE
QISFLRKLYRNQLPFQVEHQRLVKYLMITEAGRNWILRAKTGWEGRFGWWIGWVE
WPTGPVFFALNIDTPNRTDDLFKREAIARAILRSIDALPPN
**SEQ ID N°1243:** 
MRVLALSAVFLVASIIGMPAVAKEWQENKSWNAHFTEHKSQGVVVLWNENKQQGF
TNNLKRANQAFLPASTFKIPNSLIALDLGVVKDEHQVFKWDGQTRDIAAWNRDHDLI
TAMKYSVVPVYQEFARQIGEARMSKMLHAFDYGNEDISGNVDSFWLDGGIRISATQ
QIAFLRKLYHNKLHVSERSQRIVKQAMLTEANGDYIIRAKTGYSTRIEPKIGWWVGW
VELDDNVWFFAMNMDMPTSDGLGLRQAITKEVLKQEKIIP
**SEQ ID N°1244:** 
MNKYFTCYVVASLFLSGCTVQHNLINETPSQIVQGHNQVIHQYFDEKNTSGVLVIQT
DKKINLYGNALSRANTEYVPASTFKMLNALIGLENQKTDINEIFKWKGEKRSFTAWE
KDMTLGEAMKLSAVPVYQELARRIGLDLMQKEVKRIGFGNAEIGQQVDNFWLVGPL
KVTPIQEVEFVSQLAHTQLPFSEKVQANVKNMLLLEESNGYKIFGKTGWAMDIKSQV
GWLTGWVEQPDGKIVAFALNMEMRSEMPASIRNELLMKSLKQLNII
**SEQ ID N°1245:** 
MNIKALLLITSAIFISACSPYIVTANPNHSASKSDVKAEKIKNLFNEAHTTGVLVIQQGQ
TQQSYGNDLARASTEYVPASTFKMLNALIGLEHHKATTTEVFKWDGKKRLFPEWEK
DMTLGDAMKASAISVYQDLARRIGLELMSKEVKRVGYGNADIGTQVDNFWLVGPLK
ITPQQEAQFAYKLANKTLPFSQKVQDEVQSMLFIEEKNGNKIYAKSGWGWDVNPQV
GWLTGWVVQPQGNIVAFSLNLEMKKGIPSSVRKEITYKSLEQLGIL
**SEQ ID N°1246:** 
MAIRIFAILFSIFSLATFAHAQEGTLERSDWRKFFSEFQAKGTIVVADERQADRAMLV
FDPVRSKKRYSPASTFKIPHTLFALDAGAVRDEFQIFRWDGVNRGFAGHNQDQDLR
SAMRNSTVWVYELFAKEIGDDKARRYLKKIDYGNADPSTSNGDYWIEGSLAISAQE
QIAFLRKLYRNELPFRVEHQRLVKDLMIVEAGRNWILRAKTGWEGRMGWWVGWVE
WPTGSVFFALNIDTPNRMDDLFKREAIVRAILRSIEALPPNPAVNSDAAR
**SEQ ID N°1247:** MKTIAAYLVLVFYASTALSESISENLAWNKEFSSESVHGVFVLCKSSSNSCTTNNAA 
RASTAYIPASTFKIPNALIGLETGAIKDERQVFKWDGKPRAMKQWEKDLKLRGAIQV
SAVPVFQQIAREVGEIRMQKYLNLFSYGNANIGGGIDKFWLEGQLRISAFNQVKFLE
SLYLNNLPASKANQLIVKEAIVTEATPEYIVHSKTGYSGVGTESSPGVAWWVGWVE
KGTEVYFFAFNMDIDNESKLPSRKSISTKIMASEGIIIGG
**SEQ ID N°1248:** 
MKTFAAYVITACLSSTALASSITENTFWNKEFSAEAVNGVFVLCKSSSKLACATNNLA
RASKEYLPASTFKIPNAIIGLETGVIKNEHQIFKWDGKPRAMKQWERDLSLRGAIQVS
AVPVFQQIAREVGEVRMQKYLKKFSYGNQNISGGIDKFWLEGQLRISAVNQVEFLE
SLFLNKLSASKENQLIVKEALVTEAPEYLVHSKTGFSGVGTESNPGVAWWVGWVEK
GAEVYFFAFNMDIDNENKLPLRKSIPTKIMASEGIIGG
**SEQ ID N°1249:** 
MAIRIFAILFSTFVFGTFAHAQEGMRERSDWRKFFSEFQAKGTIVVADERQTDRVILV
FDQVRSEKRYSPASTFKIPHTLFALDAGAARDEFQVFRWDGIKRSFAAHNQDQDLR
SAMRNSTVWIYELFAKEIGEDKARRYLKQIDYGNADPSTSNGDYWIDGNLAIAAQEQ
IAFLRKLYHNELPFRVEHQRLVKDLMIVEAGRNWILRAKTGWEGRMGWWVGWVE
WPTGPVFFALNIDTPNRMDDLFKREAIVRAILRSIEALPPNPAVNSDAAR
**SEQ ID N°1250:** 
MKTFAAYVIIACLSSTALAGSITENTSWNKEFSAEAVNGVFVLCKSSSKSCATNDLAR
ASKEYLPASTFKIPNAIIGLETGVIKNEHQVFKWDGKPRAMKQWERDLTLRGAIQVS
AVPVFQQIAREVGEVRMQKYLKKFSYGSQNISGGIDKFWLEDQLRISAVNQVEFLES
LYLNKLSASKENQLIVKEALVTEAAPEYLVHSKTGFSGVGTESNPGVAWWVGWVEK
ETEVYFFAFNMDIDNESKLPLRKSIPTKIMESEGIIGG
**SEQ ID N°1251:** 
MNKYFTCYVVASLFLSGCTVQHNLINETPSQIVQGHNQVIHQYFDEKNTSGVLVIQT
DKKINLYGNALSRANTEYVPASTFKMLNALIGLENQKTDINEIFKWKGEKRSFTAWE
KDMTLGEAMKLSAVPVYQELARRIGLDLMQKEVKRIGFGNAEIGQQVDNFWLVGPL
KVTPIQEVEFVSQLAHTQLPFSEKVQANVKNMLLLEESNGYKIFGKTGWAMDIKPQV
GWLTGWVEQPDGKIVAFALNMEMRSEMPASIRNELLMKSLKQLNII
**SEQ ID N°1252:** 
MKKLSVLLWLTLFYCGTIWAQSTCFLVQENQTVLKHEGKDCNKRFAPESTFKIALSL
MGFDSGILKDTLNPEWPYKKEYELYLNVWKYPHNPRTWIRDSCVWYSQVLTQQLG
MTRFKNYVDAFHYGNQDISGDKGQNNGLTHSWLSSSLAISPSEQIQFLQKIVNKKLS
VNPKAFTMTKDILYIQELAGGWKLYGKTGNGRQLTKDKSQKLSLQHGWFIGWIEKD
GRVITFTKHIADSKKHVTFASFRAKNETLNQLFYLINELEK
**SEQ ID N°1253:** 
MNIKTLLLITSAIFISACSPYIVTANPNHSASKSDEKAEKIKNLFNEVHTTGVLVIQQGQ
TQQSYGNDLARASTEYVPASTFKMLNALIGLEHHKATTTEVFKWDGQKRLFPEWEK
DMTLGDAMKASAIPVYQDLARRIGLELMSKEVKRVGYGNADIGTQVDNFWLVGPLK
ITPQQEAQFAYKLANKTLPFSPKVQDEVQSMLFIEEKNGNKIYAKSGWGWDVDPQV
GWLTGWVVQPQGNIVAFSLNLEMKKGIPSSVRKEITYKSLEQLGIL
**SEQ ID N°1254:** 
MKFRHALSSAFVLLGCIAASAHAKTICTAIADAGTGKLLVQDGDCGRRASPASTFKIA
ISLMGYDAGFLRNEHDPVLPYRDSYIAWGGEAWKQPTDPTRWLKYSVVWYSQQV
AHHLGAQRFAQYAKAFGYGNADVSGDPGQNNGLDRAWIGSSLQISPLEQLEFLGK
MLNRKLPVSPTAVDMTERIVESTTLADGTVVHGKTGVSYPLLADGTRDWARGSGW
FVGWIVRGNQTLVFARLTQDERKQPVSAGIRTREAFLRDLPRLLAAR
**SEQ ID N°1255:** 
MKFRHALSSAFVLLGCIAASAHAKTICTAIADAGTGKLLVQDGDCGRRASPASTFKIA
ISLMGYDAGFLRNEHDPVLPYRDSYIAWGGEAWKQPTDPTRWLKYPVVWYSQQV
AHHLGAQRFAQYAKAFGYGNADVSGDPGQNNGLDRAWIGSSLQISPLEQLEFLGK
 
MLNRKLPVSPTAVDMTERIVESTTLADGTVVHGKTGVSYPLLADGTRDWARGSGW
FVGWIVRGKQTLVFARLTQDERKQPVSAGIRTREAFLRDLPRLLAAR
**SEQ ID N°1256:** 
MRGKHTVILGAALSALFAGAAGAQMLECTLVADAASGQELYRKGACDKAFAPMSTF
KVPLAVMGYDAGILVDAHNPRWDYKPEFNGYKFQQKTTDPTIWEKDSIVWYSQQLT
RKMGQKRFAAYVAGFGYGNGDISGEPGKSNGLTHSWLGSSLKISPEGQVRFVRDL
LSAKLPASKDAQQMTVSILPHFAAGDWAVQGKTGTGSFIDARGAKAPLGWFIGWAT
HEERRVVFARMTAGGKKGEQPAGPAARDAFLKALPDLAKRF
**SEQ ID N°1257:** 
MKFRHALSSAFVLLGCIAASAHAKTICTAIADAGTGKLLVQDGDCGRRASPASTFKIA
ISLMGYDAGFLRNEHDPVLPYRDSYIAWGGEAWKQPTDPTRWLKYSVVWYSQQV
AHHLGAQRFAQYAKAFGYGNADVSGDPGQNNGLDRAWIGSSLQISPLEQLEFLGK
MLDRKLPVSPTAVDMTERIVESTTLADGTVVHGKTGVSYPLLADGTRDWARGSGW
FVGWIVRGKQTLVFARLTQDERKQPVSAGIRTREAFLRDLPRLLAAR
**SEQ ID N°1258:** 
MKFRHALSSAFVLLGCIAASAHAKTICTAIADAGTGKLLVQDGDCGRRASPASTFKIA
ISLMGYDAGFLRNEHDPVLPYRDSYIAWGGEAWKQPTDPTRWLKYSVVWYSQQV
AHHLGAQRFAQYAKAFGYGNADVSGDPGQNNGLDRAWIGSSLQISPLEQLEFLGK
MLNRKLPVSPTAVDMTERIVESTTLADGTVVHGKTGVSYPLLADGTRDWARGSGW
FVGWIVRGKQTLVFARLTQDERKQPVSAGIRTREAFLRDLPRLLAAR
**SEQ ID N°1259:** 
MKTFAAYVIIACLSSTALAGSITENTSWNKEFSAEAVNGVFVLCKSSSKSCATNDLAR
ASKEYLPVSTFKIPSAIIGLETGVIKNEHQVFKWDGKPRAMKQWERDLTLRGAIQVS
AVPVFQQIAREVGEVRMQKYLKKFSYGNQNISGGIDKFWLEGQLRISAVNQVEFLE
SLYLNKLSASKENQLIVKEALVTEAAPEYLVHSKTGFSGVGTESNPGVAWWVGWVE
KETEVYFFAFNMDIDNESKLPLRKSIPTKIMESEGIIGG
**SEQ ID N°1260:** 
MNIKALLLITSAIFISACSPYIVTANPNHSASKSDEKAEKIKNLFNEAHTTGVLVIQQGQ
TQQSYGNDLARASTEYVPASTFKMLNALIGLEHHKATTTEVFKWDGKKRLFPEWEK
DMTLGDAMKASAIPVYQDLARRIGLELMSKEVKRVGYGNADIGTQVDNFWLVGPLK
ITPQQEAQFAYKLANKTLPFSQKVQDEVQSMLFIEEKNGNKIYAKSGWGWDVDPQV
GWLTGWVVQPQGNIVAFSLNLEMKKGIPSSVRKEITYKSLEQLGIL
**SEQ ID N°1261:** 
MNIKALLLITSAIFISACSPYIVSANPNHSASKSDEKAEKIKNLFNEAHTTGVLVIQQGQ
TQQSYGNDLARASTEYVPASTFKMLNALIGLEHHKATTTEVFKWDGQKRLFPEWEK
NMTLGDAMKASAIPVYQDLARRIGLELMSNEVKRVGYGNADIGTQVDNFWLVGPLK
ITPQQEAQFAYKLANKTLPFSQKVQDEVQSMLFIEEKNGNKIYAKSGWGWDVNPQV
GWLTGWVVQPQGNIVAFSLNLEMKKGIPSSVRKEITYKSLEQLGIL
**SEQ ID N°1262:** 
MKTFAAYVITACLSSTALASSITENTFWNKEFSAEAVNGVFVLCKSSSKSCATNNLA
RASKEYLPASTFKIPNAIIGLETGVIKNEHQVFKWDGKPRAMKQWERDLSLRGAIQV
SAVPVFQQIAREVGEVRMQKYLKKFSYGNQNISGGIDKFWLEGQLRISAVNQVEFL
ESLFLNKLSASKENQLIVKEALVTEAAPEYLVHSKTGFSGVGTESNPGVAWWVGWV
EKGTEVYFFAFNMDIDNENKLPLRKSIPTKIMASEGIIGG
**SEQ ID N°1263:** 
MKTIAAYLVLVFFAGTALSESISENLAWNKEFSSESVHGVFVLCKSSSNSCTTNNAT
RASTAYIPASTFKIPNALIGLETGAIKDARQVFKWDGKPRAMKQWEKDLTLRGAIQV
SAVPVFQQIARDIGKKRMQKYLNLFSYGNANIGGGIDKFWLEGQLRISAVNQVKFLE
SLYLNNLPASKANQLIVKEAIVTEATPEYIVHSKTGYSGVGTESNPGVAWWVGWVE
KGTEVYFFAFNMDIDNESKLPSRKSIPTKIMASEGIIIGG
**SEQ ID N°1264:** 
MKKFILPIFSISILVSLSACSSIKTKSEDNFHISSQQHEKAIKSYFDEAQTQGVIIIKEGK
NLSTYGNALARANKEYVPASTFKMLIALIGLENHKATTNEIFKWDGKKRTYPMWEKD
MTLGEAMALSAVPVYQELARRTGLELMQKEVKRVNFGNTNIGTQVDNFWLVGPLKI
TPVQEVNFADDLAHNRLPFKLETQEEVKKMLLIKEVNGSKIYAKSGWGMGVTPQVG
WLTGWVEQANGKKIPFSLNLEMKEGMSGSIRNEITYKSLENLGII
**SEQ ID N°1265:** 
MNIKALLLITSAIFISACSPYIVTANPNHSASKSDDKAEKIKNLFNEAHTTGVLVIHQGQ
TQQSYGNDLARASTEYVPASTFKMLNALIGLEHHKATTTEVFKWNGQKRLFPEWEK
DMTLGDAMKASAIPVYQDLARRIGLELMSNEVKRVGYGNADIGTQVDNFWLVGPLK
ITPQQEAQFAYKLANKTLPFSQKVQDEVQSMLFIEEKNGNKIYAKSGWGWDVDPQV
GWLTGWVVQPQGNIVAFSLNLEMKKGIPSSVRKEITYKSLEQLGIL
**SEQ ID N°1266:** 
MNIKALLLITSAISISACSPYIVTANPNHSASKSDEKAEKIKNLFNEAHTTGVLVIQQGQ
TQQSYGNDLARASTEYVPASTFKMLNALIGLEHHKATTTEVFKWDGQKRLFPEWEK
DMTLGDAIKASAIPVYQDLARRIGLELMSKEVKRVGYGNADIGTQVDNFWLVGPLKI
TPQQEAQFAYKLANKTLPFSQKVQDEVQSMLFIEEKNGNKIYAKSGWGWDVDPQV
 lesdits peptides étant choisis, de préférence, parmi les peptides de séquence **SEQ ID N° 1267** à **SEQ ID N° 1835 et SEQ ID N° 2160** à **SEQ ID N° 2171** tels que définis ci-après :

| **Peptide SEQ ID N°** | **Séquence d'acides aminés** | **Position du peptide dans la ou les protéines OXA** | **Intérêt clinique** |
|---|---|---|---|
| **SEQ ID N°1267** | AAAYELAENLFEAGQADG WR | 183-202 pour les protéines de SEQ N°1191 | 2d |
| **SEQ ID N°1268** | AAEGFIPASTFK | 86-97 pour les protéines de SEQ N°1139 | 2df |
| **SEQ ID N°1269** | AALGR | 41-45 pour les protéines de SEQ N°1140 | 2df |
| **SEQ ID N°1270** | ADGQWAFALNMQMK | 241-255 pour les protéines de SEQ N°1168, 1171, 1216 | 2df |
| **SEQ ID N°1271** | ADINEIFK | 95-102 pour les protéines de SEQ N°1124 | 2df |
| **SEQ ID N°1272** | ADWGK | 50-54 pour les protéines de SEQ N°1140 | 2df |
| **SEQ ID N°1273** | AEGAIVISDER | 40-50 pour les protéines de SEQ N°1127, 1130 | OXA |
| **SEQ ID N°1274** | AFALNLDIDK | 222-231 pour les protéines de SEQ N°1143 | 2d |
| **SEQ ID N°1275** | AFAPMSTFK | 49-57 pour les protéines de SEQ N°1256; 60-68 pour la protéine de séquence SEQ ID N°1109 | OXA |
| **SEQ ID N°1276** | AFGYGNADVSGDPGQNNG LDR | 127-147 pour les protéines de SEQ N°1254, 1255, 1257, 1258 | 2d |
| **SEQ ID N°1277** | AFTMTK | 174-179 pour les protéines de SEQ N°1252 | 2de |
| **SEQ ID N°1278** | AGDDIALR | 256-263 pour les protéines de SEQ N°1168, 1171, 1216 | 2df |
| **SEQ ID N°1279** | AGHVYAFALNIDMPR | 233-247 pour les protéines de SEQ N°1137 | 2df |
| **SEQ ID N°1280** | AGLWR | 11-15 pour les protéines de SEQ N°1140 | 2df |
| **SEQ ID N°1281** | AHTEYVPASTFK | 73-84 pour les protéines de SEQ N°1205, 1224 | 2df |
| **SEQ ID N°1282** | AIIPWDGK | 112-119 pour les protéines de SEQ N°1140 | 2df |
| **SEQ ID N°1283** | AIIPWDGKPR | 112-121 pour les protéines de SEQ N°1140 | 2df |
| **N°1284** | AISDITITR | 190-198 pour les protéines de SEQ N°1144 | 2d |
| **SEQ ID N°1285** | AISGK | 82-86 pour les protéines de SEQ N°1228 | 2df |
| **SEQ ID N°1286** | ALGQDR | 121-126 pour les protéines de SEQ N°1191 | 2d |
| **SEQ ID N°1287** | ALPDLAK | 256-262 pour les protéines de SEQ N°1256 | 2d |
| **SEQ ID N°1288** | ALQAK | 254-258 pour les protéines de SEQ N°1228 | 2df |
| **SEQ ID N°1289** | AMETFSPASTFK | 50-61 pour les protéines de SEQ N°1143 | 2d |
| **SEQ ID N°1290** | AMLFLQER | 196-203 pour les protéines de SEQ N°1227 | 2df |
| **SEQ ID N°1291** | AMLVFDPVR | 55-63 pour les protéines de SEQ N°1108, 1125, 1128, 1173, 1201, 1239, 1246; 44-52 pour la protéine de séquence SEQ ID N°1120 | OXA |
| **SEQ ID N°1292** | AMTLLESGPGWELHGK | 189-204 pour les protéines de SEQ N°1138 | 2d |
| **SEQ ID N°1293** | ANLHITLHGK | 199-208 pour les protéines de SEQ N°1144 | 2d |
| **SEQ ID N°1294** | ANQLIVK | 183-189 pour les protéines de SEQ N°1247, 1263 | OXA |
| **SEQ ID N°1295** | ANTEYVPASTFK | 71-82 pour les protéines de SEQ N°1124, 1132, 1145, 1198, 1199, 1217, 1218, 1219, 1220, 1221, 1222, 1223, 1244, 1251; 66-77 pour la protéine de séquence SEQ ID N°1227 | 2df |
| **SEQ ID N°1296** | ANVSR | 133-137 pour les protéines de SEQ N°1138 | 2d |
| **SEQ ID N°1297** | APIGWFIGWATR | 224-235 pour les protéines de SEQ N°1109 | 2de |
| **SEQ ID N°1298** | APLGWFIGWATHEER | 213-227 pour les protéines de SEQ N°1256 | 2d |
| **SEQ ID N°1299** | AQDEVQSMLFIEEK | 196-209 pour les protéines de SEQ N°1161 | 2df |
| **SEQ ID N°1300** | AQGVIVLWNENK | 40-51 pour les protéines de SEQ N°1142 | 2df |
| **SEQ ID N°1301** | ASAIAVYQDLAR | 126-137 pour les protéines de SEQ N°1225 | 2df |
| **SEQ ID N°1302** | ASAILVYQDLAR | 126-137 pour les protéines de SEQ N°1175, 1184, 1215, 1230 | 2df |
| **SEQ ID N°1303** | ASAIPVYQDLAR | 126-137 pour les protéines de SEQ N°1146, 1147,1148,1149,1150,1151,1152,1153, 1154, 1156, 1157, 1160, 1161, 1162, 1163, 1164, 1165, 1166, 1167, 1169, 1170, 1174, 1176,1177,1178,1181,1182,1183,1185, 1186, 1192, 1194, 1195, 1197, 1202, 1203, 1204, 1206, 1211, 1212, 1213, 1214, 1226, 1233, 1236, 1237, 1238, 1240, 1241, 1253, 1260, 1261, 1265, 1266; 120-131 pour la protéine de séquence SEQ IDN°1189; 120-131 pour la protéine de séquence SEQ ID N°1190 | 2df |
| **SEQ ID N°1304** | ASAIPVYQDLPR | 126-137 pour les protéines de SEQ N°1155 | 2df |
| **SEQ ID N°1305** | ASAIQVYQDLAR | 126-137 pour les protéines de SEQ N°1179, 1231 | 2df |
| **SEQ ID N°1306** | ASAISVYQDLAR | 126-137 pour les protéines de SEQ N°1158, 1245 | 2df |
| **SEQ ID N°1307** | ASALPVYQDLAR | 126-137 pour les protéines de SEQ N°1159, 1180 | 2df |
| **SEQ ID N°1308** | ASAMPVYQDLAR | 126-137 pour les protéines de SEQ N°1232 | 2df |
| **SEQ ID N°1309** | ASAVPVYQDLAR | 126-137 pour les protéines de SEQ N°1196, 1209, 1210 | 2df |
| **SEQ ID N°1310** | ASIEYVPASTFK | 72-83 pour les protéines de SEQ N°1157, 1161 | 2df |
| **SEQ ID N°1311** | ASNVPVYQELAR | 113-124 pour les protéines de SEQ N°1138 | 2d |
| **SEQ ID N°1312** | ASPASTFK | 49-56 pour les protéines de SEQ N°1254, 1255, 1257, 1258 | 2d |
| **SEQ ID N°1313** | ASTAYIPASTFK | 59-70 pour les protéines de SEQ N°1247, 1263 | OXA |
| **SEQ ID N°1314** | ASTEYVPASTFK | 72-83 pour les protéines de SEQ N°1146, 1147, 1148, 1149, 1150, 1151, 1152, 1153, 1154, 1155, 1156, 1158, 1159, 1160, 1162, 1163, 1164, 1165, 1166, 1167, 1169, 1170, 1174, 1175, 1176, 1177, 1178, 1179, 1180, 1181, 1182, 1183, 1184, 1185, 1186, 1192, 1194, 1195, 1196, 1197, 1202, 1203, 1204, 1206, 1209, 1210, 1211, 1212, 1213, 1214, 1215, 1225, 1226, 1230, 1231, 1232, 1233, 1236, 1237, 1238, 1240, 1241, 1245, 1253, 1260, 1261, 1265, 1266; 66-77 pour la protéine de séquence SEQ ID N°1189; 66-77 pour la protéine de séquence SEQ ID N°1190 | 2df |
| **SEQ ID N°1315** | ASTTEVFK | 96-103 pour les protéines de SEQ N°1202 | 2df |
| **SEQ ID N°1316** | ATSTEIFK | 99-106 pour les protéines de SEQ N°1168, 1171, 1216 | 2df |
| **SEQ ID N°1317** | ATTNEIFK | 97-104 pour les protéines de SEQ N°1122, 1123, 1129, 1172, 1207, 1224, 1264; 90-97 pour la protéine de séquence SEQ ID N°1227 | 2df |
| **SEQ ID N°1318** | ATTTAVFK | 96-103 pour les protéines de SEQ N°1154 | 2df |
| **SEQ ID N°1319** | ATTTEIFK | 97-104 pour les protéines de SEQ N°1205; 96-103 pour la protéine de séquence SEQ ID N°1157; 96-103 pour la protéine de séquence SEQ ID N°1161; 96-103 pour la protéine de séquence SEQ ID N°1169 | 2df |
| **SEQ ID N°1320** | ATTTEVFK | 96-103 pour les protéines de SEQ N°1146, 1147, 1148, 1149, 1150, 1151, 1152, 1155, 1156, 1158, 1159, 1160, 1162, 1163, 1164, 1165, 1166, 1167, 1170, 1174, 1175, 1176, 1177, 1178, 1179, 1180, 1181, 1182, 1183, 1184, 1185, 1192, 1194, 1195, 1196, 1197, 1203, 1204, 1206, 1209, 1210, 1211, 1212, 1213, 1214, 1215, 1225, 1226, 1230, 1231, 1232, 1233, 1236, 1237, 1238, 1240, 1241, 1245, 1253, 1260, 1261, 1265, 1266; 90-97 pour la protéine de séquence SEQ ID N°1189; 90-97 pour la protéine de séquence SEQ ID N°1190 | 2df |
| **SEQ ID N°1321** | AVSDITILEQTDNYTLHGK | 191-209 pour les protéines de SEQ N°1235 | OXA |
| **SEQ ID N°1322** | AVSDITILEQTYNYTLHGK | 191-209 pour les protéines de SEQ N°1187, 1188 | 2d |
| **SEQ ID N°1323** | AVVPHFEAGDWDVQGK | 195-210 pour les protéines de SEQ N°1109 | 2de |
| **SEQ ID N°1324** | AWEHDMSLR | 100-108 pour les protéines de SEQ N°1138 | 2d |
| **SEQ ID N°1325** | AWIGSSLQISPLEQLEFLGK | 148-167 pour les protéines de SEQ N°1254, 1255, 1257, 1258 | 2d |
| **SEQ ID N°1326** | CAAQMAPDSTFK | 63-74 pour les protéines de SEQ N°1134 | 2d |
| **SEQ ID N°1327** | CATQMAPDSTFK | 48-59 pour les protéines de SEQ N°1119; 63-74 pour la protéine de séquence SEQ ID N°1118 | 2d |
| **SEQ ID N°1328** | CTIIADAITGNTLYETGECA R | 32-52 pour les protéines de SEQ N°1107 | 2d |
| **SEQ ID N°1329** | DAFLK | 251-255 pour les protéines de SEQ N°1256 | 2d |
| **SEQ ID N°1330** | DDFILHGK | 189-196 pour les protéines de SEQ N°1135 | 2d |
| **SEQ ID N°1331** | DDQEVLPYGGK | 92-102 pour les protéines de SEQ N°1137 | 2df |
| **SEQ ID N°1332** | DDVLK | 87-91 pour les protéines de SEQ N°1228 | 2df |
| **SEQ ID N°1333** | DEFHVFR | 90-96 pour les protéines de SEQ N°1113, 1121 | 2d |
| **SEQ ID N°1334** | DEFQIFR | 90-96 pour les protéines de SEQ N°1108, 1125, 1128, 1133, 1173, 1201, 1239, 1246; 79-85 pour la protéine de séquence SEQ ID N°1120 | OXA |
| **SEQ ID N°1335** | DEFQVFR | 90-96 pour les protéines de SEQ N°1114, 1127, 1130, 1131, 1242, 1249 | 2d |
| **SEQ ID N°1336** | DELVR | 260-264 pour les protéines de SEQ N°1191 | 2d |
| **SEQ ID N°1337** | DETSR | 112-116 pour les protéines de SEQ N°1139 | 2df |
| **SEQ ID N°1338** | DFDYGNQDFSGDK | 141-153 pour les protéines de SEQ N°1118, 1134; 126-138 pour la protéine de séquence SEQ ID N°1119 | 2d |
| **SEQ ID N°1339** | DFTLGEAMQASTVPVYQEL AR | 120-140 pour les protéines de SEQ N°1168, 1171, 1216 | 2df |
| **SEQ ID N°1340** | DGNITSVAINIDMLSEADAP K | 250-270 pour les protéines de SEQ N°1140 | 2df |
| **SEQ** ID **N°1341** | DHDLITAMK | 108-116 pour les protéines de SEQ N°1142, 1243 | 2df |
| **SEQ ID N°1342** | DIAAWNR | 101-107 pour les protéines de SEQ N°1142, 1243 | 2df |
| **SEQ ID N°1343** | DIGEDK | 131-136 pour les protéines de SEQ N°1131 | 2d |
| **SEQ ID N°1344** | DILYIQELAGGWK | 180-192 pour les protéines de SEQ N°1252 | 2de |
| **SEQ ID N°1345** | DITILEK | 181-187 pour les protéines de SEQ N°1135 | 2d |
| **N°1346** | DLLSAK | 166-171 pour les protéines de SEQ N°1256 | 2d |
| **SEQ ID N°1347** | DLMITEAGR | 195-203 pour les protéines de SEQ N°1127, 1130, 1131 | 2d |
| **SEQ ID N°1348** | DLMIVEAGR | 195-203 pour les protéines de SEQ N°1108, 1114, 1125, 1128, 1133, 1173, 1201, 1239, 1246, 1249; 184-192 pour la protéine de séquence SEQ ID N° 1120 | OXA |
| **SEQ ID N°1349** | DLMIVEAK | 195-202 pour les protéines de SEQ N°1113, 1121 | 2d |
| **SEQ ID N°1350** | DLPLR | 243-247 pour les protéines de SEQ N°1228 | 2df |
| **SEQ ID N°1351** | DLSGNPGK | 131-138 pour les protéines de SEQ N°1105 | 2d |
| **SEQ ID N°1352** | DLSLR | 105-109 pour les protéines de SEQ N°1115, 1116, 1117, 1126, 1141, 1200, 1229, 1262; 106-110 pour la protéine de séquence SEQ ID N°1248 | OXA |
| **SEQ ID N°1353** | DLTLR | 105-109 pour les protéines de SEQ N°1110, 1193, 1250, 1259, 1263; 96- 100 pour la protéine de séquence SEQ ID N°1106; 96-100 pour la protéine de séquence SEQ ID N°1111; 96-100 pour la protéine de séquence SEQ ID N°1112 | OXA |
| **SEQ ID N°1354** | DMTLGDAIK | 117-125 pour les protéines de SEQ N°1226, 1266 | 2df |
| **SEQ ID N°1355** | DMTLGDAMALSAVPVYQE LAR | 118-138 pour les protéines de SEQ N°1205 | 2df |
| **SEQ ID N°1356** | DMTLGDAMK | 117-125 pour les protéines de SEQ N°1147, 1148, 1149, 1152, 1153, 1156, 1157, 1158, 1159, 1161, 1162, 1165, 1166, 1167, 1169, 1170, 1175, 1176, 1178, 1179, 1181, 1183, 1184, 1185, 1186, 1194, 1195, 1196, 1197, 1202, 1209, 1210, 1211, 1212, 1213, 1214, 1215, 1225, 1230, 1231, 1232, 1236, 1237, 1238, 1240, 1241, 1245, 1253, 1260, 1265 | 2df |
| **SEQ** ID **N°1357** | DMTLGEAMALSAVPVYQD LAR | 118-138 pour les protéines de SEQ N°1224 | 2df |
| **SEQ ID N°1358** | DMTLGEAMALSAVPVYQE LAR | 118-138 pour les protéines de SEQ N°1122, 1123, 1129, 1172, 1207, 1264 | 2df |
| **SEQ ID N°1359** | DMTLGEAMK | 116-124 pour les protéines de SEQ N°1124, 1132, 1145, 1198, 1199, 1217, 1218, 1219, 1220, 1221, 1222, 1223, 1244, 1251 | 2df |
| **SEQ ID N°1360** | DMTLGQAMQASAVPVYQE LAR | 111-131 pour les protéines de SEQ N°1227 | 2df |
| **SEQ ID N°1361** | DNNGK | 214-218 pour les protéines de SEQ N°1107 | 2d |
| **SEQ ID N°1362** | DQDLR | 110-114 pour les protéines de SEQ N°1108, 1114, 1125, 1127, 1128, 1130, 1131, 1133, 1173, 1201, 1239, 1242, 1246, 1249; 99-103 pour la protéine de séquence SEQ ID N°1120 | 2d |
| **SEQ ID N°1363** | DQQIGWFVGWASK | 213-225 pour les protéines de SEQ N°1105 | 2d |
| **SEQ ID N°1364** | DQQIGWFVGWASKPGK | 213-228 pour les protéines de SEQ N°1105 | 2d |
| **SEQ ID N°1365** | DQQVQVYGNDLNR | 53-65 pour les protéines de SEQ N°1227 | 2df |
| **SEQ ID N°1366** | DQSFR | 132-136 pour les protéines de SEQ N°1139 | 2df |
| **SEQ ID N°1367** | DQTLESAFK | 105-113 pour les protéines de SEQ N°1228 | 2df |
| **SEQ ID N°1368** | DSCVWYSQVLTQQLGMTR | 98-115 pour les protéines de SEQ N°1252 | 2de |
| **SEQ ID N°1369** | DSIVWYSQELTR | 113-124 pour les protéines de SEQ N°1109 | 2de |
| **SEQ ID N°1370** | DSIVWYSQQLTR | 102-113 pour les protéines de SEQ N°1256 | 2d |
| **SEQ ID N°1371** | DSNLR | 109-113 pour les protéines de SEQ N°1187, 1188, 1235; 96-100 pour la protéine de séquence SEQ ID N°1135; 108-112 pour la protéine de séquence SEQ ID N°1144 | 2d |
| **SEQ ID N°1372** | DSYIAWGGEAWK | 81-92 pour les protéines de SEQ N°1254, 1255, 1257, 1258 | 2d |
| **SEQ ID N°1373** | DTLNPEWPYK | 67-76 pour les protéines de SEQ N°1252 | 2de |
| **SEQ ID N°1374** | DVDEVFYK | 88-95 pour les protéines de SEQ N°1144, 1187, 1188, 1235 | 2d |
| **SEQ ID N°1375** | DVSGDPGK | 144-151 pour les protéines de SEQ N°1109 | 2de |
| **SEQ ID N°1376** | DWILR | 204-208 pour les protéines de SEQ N°1113, 1121 | 2d |
| **SEQ ID N°1377** | DWPAMAGAWR | 265-274 pour les protéines de SEQ N°1191 | 2d |
| **SEQ ID N°1378** | EAFLR | 256-260 pour les protéines de SEQ N°1254, 1255, 1257, 1258 | 2d |
| **SEQ ID N°1379** | EAIAR | 250-254 pour les protéines de SEQ N°ll13, 1121, 1127, 1130, 1131, 1133, 1242 | 2d |
| **SEQ ID N°1380** | EAIVR | 250-254 pour les protéines de SEQ N°1108, 1114, 1125, 1128, 1173, 1201, 1239, 1246, 1249; 239-243 pour la protéine de séquence SEQ ID N°1120 | OXA |
| **SEQ ID N°1381** | EAIVTEATPEYIVHSK | 190-205 pour les protéines de SEQ N°1247, 1263 | OXA |
| **SEQ ID N°1382** | EALVTEAAPEYLVHSK | 190-205 pour les protéines de SEQ N°1110, 1115, 1116, 1117, 1126, 1141, 1193, 1200, 1229, 1250, 1259, 1262; 181-196 pour la protéine de séquence SEQ ID N°1106; 181-196 pour la protéine de séquence SEQ ID N°1111; 181-196 pour la protéine de séquence SEQ ID N°1112 | OXA |
| **SEQ ID N°1383** | EALVTEAPEYLVHSK | 191-205 pour les protéines de SEQ N°1248 | 2d |
| **SEQ ID N°1384** | EEIVR | 240-244 pour les protéines de SEQ N°1135 | 2d |
| **SEQ ID N°1385** | EEVLAALPAQLK | 251-262 pour les protéines de SEQ N°1105 | 2d |
| **SEQ ID N°1386** | EFNGSK | 209-214 pour les protéines de SEQ N°1205 | 2df |
| **SEQ ID N°1387** | EFSAEAVNGVFVLCK | 31-45 pour les protéines de SEQ N°1110, 1115, 1116, 1117, 1126, 1141, 1193, 1200, 1229, 1248, 1250, 1259, 1262; 22-36 pour la protéine de séquence SEQ ID N°1106; 22-36 pour la protéine de séquence SEQ ID N°1111; 22-36 pour la protéine de séquence SEQ ID N°1112 | OXA |
| **SEQ ID N°1388** | EFSSESVHGVFVLCK | 31-45 pour les protéines de SEQ N°1247, 1263 | OXA |
| **SEQ ID N°1389** | EGDMAK | 248-253 pour les protéines de SEQ N°1137 | 2df |
| **SEQ ID N°1390** | EGMSGSIR | 254-261 pour les protéines de SEQ N°1122, 1129, 1172, 1207, 1264 | 2df |
| **SEQ ID N°1391** | EGMTGSIR | 254-261 pour les protéines de SEQ N°1123 | 2df |
| **SEQ ID N°1392** | EGSCDK | 54-59 pour les protéines de SEQ N°1109 | 2de |
| **SEQ ID N°1393** | EIAVWNR | 125-131 pour les protéines de SEQ N°1139 | 2df |
| **SEQ ID N°1394** | EIAYK | 262-266 pour les protéines de SEQ N°1238 | 2df |
| **SEQ ID N°1395** | EIFER | 20-24 pour les protéines de SEQ N°1135 | 2d |
| **SEQ ID N°1396** | EIFYHYR | 79-85 pour les protéines de SEQ N°1143 | 2d |
| **SEQ ID N°1397** | EIGDDK | 131-136 pour les protéines de SEQ N°1108, 1125, 1128, 1173, 1201, 1239, 1246; 120-125 pour la protéine de séquence SEQ ID N°1120 | OXA |
| **SEQ ID N°1398** | EIGDGK | 131-136 pour les protéines de SEQ N°1133 | 2d |
| **SEQ ID N°1399** | EIGEDK | 131-136 pour les protéines de SEQ N°1114, 1130, 1249 | 2d |
| **SEQ ID N°1400** | EIGEDNAR | 131-138 pour les protéines de SEQ N°1242 | OXA |
| **SEQ ID N°1401** | EIGENK | 131-136 pour les protéines de SEQ N°1113, 1121 | 2d |
| **SEQ ID N°1402** | EIGPK | 153-157 pour les protéines de SEQ N°1139 | 2df |
| **SEQ ID N°1403** | EIGSEIDK | 136-143 pour les protéines de SEQ N°1135 | 2d |
| **SEQ ID N°1404** | EITYK | 262-266 pour les protéines de SEQ N°1147, 1148, 1149, 1150, 1151, 1152, 1153, 1154, 1155, 1156, 1157, 1158, 1159, 1160, 1161, 1162, 1163, 1164, 1165, 1166, 1167, 1169, 1170, 1174, 1175, 1176, 1177, 1178, 1179, 1180, 1181, 1182, 1183, 1184, 1185, 1186, 1192, 1194, 1195, 1196, 1202, 1203, 1204, 1206, 1209, 1210, 1211, 1212, 1213, 1214, 1215, 1225, 1226, 1230, 1231, 1232, 1233, 1236, 1237, 1240, 1241, 1245, 1253, 1260, 1261, 1265, 1266; 263-267 pour la protéine de séquence SEQ ID N°1122; 263-267 pour la protéine de séquence SEQ ID N°1123; 263-267 pour la protéine de séquence SEQ ID N°1129; 263-267 pour la protéine de séquence SEQ ID N°1172; 256-260 pour la protéine de séquence SEQ ID N°1189; 256-260 pour la protéine de séquence SEQ ID N°1190; 263-267 pour la protéine de séquence SEQ ID N°1205; 263-267 pour la protéine de séquence SEQ ID N°1207; 263-267 pour la protéine de séquence SEQ ID N°1224; 263-267 pour la protéine de séquence SEQ ID N°1264 | 2df |
| **SEQ ID N°1405** | EITYR | 262-266 pour les protéines de SEQ N°1146 | 2df |
| **SEQ ID N°1406** | EMIYLK | 181-186 pour les protéines de SEQ N°1143 | 2d |
| **SEQ ID N°1407** | EMLYVER | 205-211 pour les protéines de SEQ N°1168, 1171, 1216 | 2df |
| **SEQ ID N°1408** | EMTYK | 262-266 pour les protéines de SEQ N°1197 | 2df |
| **SEQ ID N°1409** | ENIEK | 138-142 pour les protéines de SEQ N°1187, 1188, 1235; 137-141 pour la protéine de séquence SEQ ID N°1144 | 2d |
| **SEQ ID N°1410** | ENQLIVK | 183-189 pour les protéines de SEQ N°1110, 1115, 1116, 1117, 1126, 1141, 1193, 1200, 1229, 1250, 1259, 1262; 174-180 pour la protéine de séquence SEQ ID N°1106; 174-180 pour la protéine de séquence SEQ ID N°1111; 174-180 pour la protéine de séquence SEQ ID N°1112; 184-190 pour la protéine de séquence SEQ ID N°1248 | OXA |
| **SEQ ID N°1411** | EQAILLFR | 156-163 pour les protéines de SEQ N°1143 | 2d |
| **SEQ ID N°1412** | EQIQFLLR | 165-172 pour les protéines de SEQ N°1107 | 2d |
| **SEQ ID N°1413** | EQLAFDPQVQQQVK | 182-195 pour les protéines de SEQ N°1227 | 2df |
| **SEQ ID N°1414** | EQVDFVQR | 189-196 pour les protéines de SEQ N°1140 | 2df |
| **SEQ ID N°1415** | ETEVYFFAFNMDIDNESK | 229-246 pour les protéines de SEQ N°1110, 1193, 1250, 1259; 220-237 pour la protéine de séquence SEQ ID N°1106; 220-237 pour la protéine de séquence SEQ ID N°1111; 220-237 pour la protéine de séquence SEQ ID N°1112 | OXA |
| **SEQ ID N°1416** | ETTTPR | 90-95 pour les protéines de SEQ N°1105 | 2d |
| **SEQ ID N°1417** | EVGEIR | 126-131 pour les protéines de SEQ N°1247 | 2d |
| **SEQ ID N°1418** | EVGEVR | 126-131 pour les protéines de SEQ N°1110, 1115, 1116, 1117, 1126, 1141, 1193, 1200, 1229, 1250, 1259, 1262; 117-122 pour la protéine de séquence SEQ ID N°1106; 117-122 pour la protéine de séquence SEQ ID N°1111; 117-122 pour la protéine de séquence SEQ ID N°1112; 127-132 pour la protéine de séquence SEQ ID N°1248 | OXA |
| **SEQ ID N°1419** | EVNGSK | 209-214 pour les protéines de SEQ N°ll22, 1123, 1129, 1172, 1207, 1224, 1264 | 2df |
| **SEQ ID N°1420** | EWQENK | 24-29 pour les protéines de SEQ N°1136, 1208, 1234, 1243 | 2df |
| **SEQ ID N°1421** | EYELYLNVWK | 78-87 pour les protéines de SEQ N°1252 | 2de |
| **SEQ ID N°1422** | EYLPASTFK | 62-70 pour les protéines de SEQ N°1110, 1115, 1116, 1117, 1126, 1141, 1193, 1200, 1229, 1250, 1262; 53-61 pour la protéine de séquence SEQ ID N°1106; 53-61 pour la protéine de séquence SEQ ID N°1111; 53-61 pour la protéine de séquence SEQ ID N°1112; 63-71 pour la protéine de séquence SEQ ID N°1248 | OXA |
| **SEQ ID N°1423** | EYLPVSTFK | 62-70 pour les protéines de SEQ N°1259 | 2de |
| **SEQ ID N°1424** | EYNTSGTFVFYDGK | 27-40 pour les protéines de SEQ N°1143 | 2d |
| **SEQ ID N°1425** | EYVPASTFK | 75-83 pour les protéines de SEQ N°1146, 1147, 1148, 1149, 1150, 1151, 1152, 1153, 1154, 1155, 1156, 1157, 1158, 1159, 1160, 1161, 1162, 1163, 1164, 1165, 1166, 1167, 1169, 1170, 1174, 1175, 1176, 1177, 1178, 1179, 1180, 1181, 1182, 1183, 1184, 1185, 1186, 1192, 1194, 1195, 1196, 1197, 1202, 1203, 1204, 1206, 1209, 1210, 1211, 1212, 1213, 1214, 1215, 1225, 1226, 1230, 1231, 1232, 1233, 1236, 1237, 1238, 1240, 1241, 1245, 1253, 1260, 1261, 1265, 1266; 76-84 pour la protéine de séquence SEQ ID N°1122; 76-84 pour la protéine de séquence SEQ ID N°1123; 74-82 pour la protéine de séquence SEQ ID N°1124; 76-84 pour la protéine de séquence SEQ ID N°1129; 74-82 pour la protéine de séquence SEQ ID N°1132; 74-82 pour la protéine de séquence SEQ ID N°1145; 76-84 pour la protéine de séquence SEQ ID N°1172; 69-77 pour la protéine de séquence SEQ ID N°1189; 69-77 pour la protéine de séquence SEQ ID N°1190; 74-82 pour la protéine de séquence SEQ ID N°1198; 74-82 pour la protéine de séquence SEQ ID N°1199; 76-84 pour la protéine de séquence SEQ ID N°1205; 76-84 pour la protéine de séquence SEQ ID N°1207; 74-82 pour la protéine de séquence SEQ ID N°1217; 74-82 pour la protéine de séquence SEQ ID N°1218; 74-82 pour la protéine de séquence SEQ ID N°1219; 74-82 pour la protéine de séquence SEQ ID N°1220; 74-82 pour la protéine de séquence SEQ ID N°1221; 74-82 pour la protéine de séquence SEQ ID N°1222; 74-82 pour la protéine de séquence SEQ ID N°1223; 76-84 pour la protéine de séquence SEQ ID N°1224; 69-77 pour la protéine de séquence SEQ ID N°1227; 74-82 pour la protéine de séquence SEQ ID N°1244; 74-82 pour la protéine de séquence SEQ ID N°1251; 76-84 pour la protéine de séquence SEQ ID N°1264 | 2df |
| **SEQ ID N°1426** | FAAYVAGFGYGNGDISGEP GK | 120-140 pour les protéines de SEQ N°1256 | 2d |
| **SEQ ID N°1427** | FAPESTFK | 45-52 pour les protéines de SEQ N°1252 | 2de |
| **SEQ ID N°1428** | FAQYAK | 121-126 pour les protéines de SEQ N°1254, 1255, 1257, 1258 | 2d |
| **SEQ ID N°1429** | FDYGNK | 138-143 pour les protéines de SEQ N°1109 | 2de |
| **SEQ ID N°1430** | FDYGNR | 146-151 pour les protéines de SEQ N°1137; 125-130 pour la protéine de séquence SEQ ID N°1105 | 2d |
| **SEQ ID N°1431** | FEDLYK | 232-237 pour les protéines de SEQ N°1143 | 2d |
| **SEQ ID N°1432** | FEDTFHISNQK | 27-37 pour les protéines de SEQ N°1224 | 2df |
| **SEQ ID N°1433** | FEDTFHTSNQQHEK | 27-40 pour les protéines de SEQ N°1205 | 2df |
| **SEQ ID N°1434** | FEYGNQDVSGDSGK | 133-146 pour les protéines de SEQ N°1107 | 2d |
| **SEQ ID N°1435** | FFSDFQAK | 34-41 pour les protéines de SEQ N°1133 | 2d |
| **SEQ ID N°1436** | FFSDLQAEGAIVIADER | 34-50 pour les protéines de SEQ N°1131, 1242 | 2d |
| **SEQ ID N°1437** | FFSDLR | 34-39 pour les protéines de SEQ N°1127, 1130 | OXA |
| **SEQ ID N°1438** | FFSEFQAK | 34-41 pour les protéines de SEQ N°1108, 1114, 1125, 1128, 1173, 1201, 1239, 1246, 1249; 23-30 pour la protéine de séquence SEQ ID N°1120 | OXA |
| **SEQ ID N°1439** | FGLEGQLR | 153-160 pour les protéines de SEQ N°1117 | 2de |
| **SEQ ID N°1440** | FILPIFSISILVSLSACSSIK | 4-24 pour les protéines de SEQ N°1122, 1123, 1129, 1172, 1207, 1264 | 2df |
| **SEQ ID N°1441** | FLALLFSAVVLVSLGHAQD K | 5-24 pour les protéines de SEQ N°1121 | 2d |
| **SEQ ID N°1442** | FLALLFSAVVLVSLGHAQE K | 5-24 pour les protéines de SEQ N°1113 | 2d |
| **SEQ ID N°1443** | FLESLYLNNLPASK | 169-182 pour les protéines de SEQ N°1247, 1263 | OXA |
| **SEQ ID N°1444** | FLLEGQLR | 153-160 pour les protéines de SEQ N°1200 | 2de |
| **SEQ ID N°1445** | FQQYVDR | 118-124 pour les protéines de SEQ N°1105 | 2d |
| **SEQ ID N°1446** | FSDYVQR | 131-137 pour les protéines de SEQ N°1109 | 2de |
| **SEQ ID N°1447** | FSTASTFK | 63-70 pour les protéines de SEQ N°1228 | 2df |
| **SEQ ID N°1448** | FSWDGK | 117-122 pour les protéines de SEQ N°1139 | 2df |
| **SEQ ID N°1449** | FSYGNQNISGGIDK | 139-152 pour les protéines de SEQ N°1110, 1115, 1116, 1117, 1126, 1141, 1193, 1200, 1259, 1262; 130-143 pour la protéine de séquence SEQ ID N°1106; 130-143 pour la protéine de séquence SEQ ID N°1111; 130-143 pour la protéine de séquence SEQ ID N°1112; 140-153 pour la protéine de séquence SEQ ID N°1248 | OXA |
| **SEQ ID N°1450** | FSYGNQNISGGTDK | 139-152 pour les protéines de SEQ N°1229 | 2de |
| **SEQ ID N°1451** | FSYGSQNISGGIDK | 139-152 pour les protéines de SEQ N°1250 | 2de |
| **SEQ ID N°1452** | FTEYVK | 126-131 pour les protéines de SEQ N°1107 | 2d |
| **SEQ ID N°1453** | FVAHK | 173-177 pour les protéines de SEQ N°1107 | 2d |
| **SEQ ID N°1454** | FVPASTYK | 62-69 pour les protéines de SEQ N°1138 | 2d |
| **SEQ ID N°1455** | FVYDLAQGQLPFK | 184-196 pour les protéines de SEQ N°1168, 1171, 1216 | 2df |
| **SEQ ID N°1456** | FVYDLAQGQLPFKPEVQQQ VK | 184-204 pour les protéines de SEQ N°1168, 1171, 1216 | 2df |
| **SEQ ID N°1457** | FWLEDQLR | 153-160 pour les protéines de SEQ N°1116, 1193, 1229, 1250; 144-151 pour la protéine de séquence SEQ ID N°1106; 144-151 pour la protéine de séquence SEQ ID N°1111 | 2de |
| **SEQ ID N°1458** | FWLEGPLK | 144-151 pour les protéines de SEQ N°1135 | 2d |
| **SEQ ID N°1459** | FWLEGQLR | 153-160 pour les protéines de SEQ N°1110, 1115, 1126, 1141, 1247, 1259, 1262, 1263; 144-151 pour la protéine de séquence SEQ ID N°1112; 154-161 pour la protéine de séquence SEQ ID N°1248 | OXA |
| **SEQ ID N°1460** | FYPASSFK | 53-60 pour les protéines de SEQ N°1135 | 2d |
| **SEQ ID N°1461** | FYPASTFK | 66-73 pour les protéines de SEQ N°1144, 1187, 1188, 1235 | 2d |
| **SEQ ID N°1462** | GACDK | 44-48 pour les protéines de SEQ N°1256 | 2d |
| **SEQ ID N°1463** | GAEVYFFAFNMDIDNENK | 229-246 pour les protéines de SEQ N°1141, 1248 | 2d |
| **SEQ ID N°1464** | GAIQVSAVPVFQQIAR | 110-125 pour les protéines de SEQ N°1110, 1115, 1116, 1117, 1126, 1141, 1193, 1200, 1229, 1247, 1250, 1259, 1262, 1263; 101-116 pour la protéine de séquence SEQ ID N°1106; 101-116 pour la protéine de séquence SEQ ID N°1112; 111-126 pour la protéine de séquence SEQ ID N°1248 | OXA |
| **SEQ ID N°1465** | GAIQVSAVPVFQQITR | 101-116 pour les protéines de SEQ N°1111 | 2de |
| **SEQ ID N°1466** | GDYWIDGNLEISAHEQISFL R | 156-176 pour les protéines de SEQ N°1127, 1130 | OXA |
| **SEQ ID N°1467** | GDYWIDGNLK | 156-165 pour les protéines de SEQ N°1131 | 2d |
| **SEQ ID N°1468** | GELPVSEDALEMTK | 181-194 pour les protéines de SEQ N°1109 | 2de |
| **SEQ ID N°1469** | GEQPAGPAAR | 241-250 pour les protéines de SEQ N°1256; 252-261 pour la protéine de séquence SEQ ID N°1109 | OXA |
| **SEQ ID N°1470** | GFAGHNQDQDLR | 103-114 pour les protéines de SEQ N°1108, 1125, 1128, 1173, 1201, 1239, 1246; 92-103 pour la protéine de séquence SEQ ID N°1120 | OXA |
| **SEQ ID N°1471** | GIPSSVR | 254-260 pour les protéines de SEQ N°1147, 1148, 1149, 1150, 1151, 1152, 1153, 1155, 1156, 1157, 1158, 1159, 1160, 1161, 1162, 1163, 1164, 1165, 1166, 1167, 1169, 1170, 1174, 1175, 1176, 1177, 1178, 1179, 1180, 1181, 1182, 1183, 1184, 1185, 1186, 1192, 1194, 1195, 1196, 1197, 1202, 1203, 1204, 1206, 1209, 1210, 1211, 1212, 1213, 1214, 1215, 1225, 1226, 1230, 1231, 1232, 1233, 1236, 1237, 1238, 1240, 1241, 1245, 1253, 1260, 1261, 1265, 1266; 248-254 pour la protéine de séquence SEQ ID N°1189; 248-254 pour la protéine de séquence SEQ ID N°1190 | 2df |
| **SEQ ID N°1472** | GISSSVR | 254-260 pour les protéines de SEQ N°1146 | 2df |
| **SEQ ID N°1473** | GLNGTFVVYDLK | 26-37 pour les protéines de SEQ N°1135 | 2d |
| **SEQ ID N°1474** | GMEIWNSNHTPK | 101-112 pour les protéines de SEQ N°1118, 1134; 86-97 pour la protéine de séquence SEQ ID N°1119 | 2d |
| **SEQ ID N°1475** | GNQTLVFAR | 230-238 pour les protéines de SEQ N°1254 | 2d |
| **SEQ ID N°1476** | GNYWIDGNLEISAHEQISFL R | 156-176 pour les protéines de SEQ N°1242 | OXA |
| **SEQ ID N°1477** | GPLEISAFEEAR | 164-175 pour les protéines de SEQ N°1137 | 2df |
| **SEQ ID N°1478** | GPLTITPIQEVK | 172-183 pour les protéines de SEQ N°1168, 1171, 1216 | 2df |
| **SEQ ID N°1479** | GSGWFVGWIVR | 219-229 pour les protéines de SEQ N°1254, 1255, 1257, 1258 | 2d |
| **SEQ ID N°1480** | GSLLLWDQK | 66-74 pour les protéines de SEQ N°1139 | 2df |
| **SEQ ID N°1481** | GTEVYFFAFNMDIDNENK | 229-246 pour les protéines de SEQ N°1115, 1116, 1117, 1126, 1200, 1229, 1262 | OXA |
| **SEQ ID N°1482** | GTEVYFFAFNMDIDNESK | 229-246 pour les protéines de SEQ N°1247, 1263 | OXA |
| **SEQ ID N°1483** | GTFVLYDVQR | 38-47 pour les protéines de SEQ N°1138 | 2d |
| **SEQ ID N°1484** | GTIWADER | 42-50 pour les protéines de SEQ N°1108, 1114, 1125, 1128, 1133, 1173, 1201, 1239, 1246, 1249; 31-39 pour la protéine de séquence SEQ ID N°1120 | OXA |
| **SEQ ID N°1485** | GTIVVLDAR | 63-71 pour les protéines de SEQ N°1140 | 2df |
| **SEQ ID N°1486** | GTIVVDER | 42-50 pour les protéines de SEQ N°1113, 1121 | 2d |
| **SEQ ID N°1487** | GTLPFSAR | 200-207 pour les protéines de SEQ N°1140 | 2df |
| **SEQ ID N°1488** | GTPSSVR | 254-260 pour les protéines de SEQ N°1154 | 2df |
| **SEQ ID N°1489** | HALSSAFVLLGCIAASAHA K | 5-24 pour les protéines de SEQ N°1254, 1255, 1257, 1258 | 2d |
| **SEQ ID N°1490** | HIADSK | 234-239 pour les protéines de SEQ N°1252 | 2de |
| **SEQ ID N°1491** | HNGLTHAWLASSLK | 152-165 pour les protéines de SEQ N°1109 | 2de |
| **SEQ ID N°1492** | HNGLTQSWLMSSLTISPK | 147-164 pour les protéines de SEQ N°1107 | 2d |
| **SEQ ID N°1493** | HNGTDGAWIISSLR | 148-161 pour les protéines de SEQ N°1191 | 2d |
| **SEQ ID N°1494** | HTLSVFDQER | 54-63 pour les protéines de SEQ N°1131 | 2d |
| **SEQ ID N°1495** | HVTFASFR | 241-248 pour les protéines de SEQ N°1252 | 2de |
| **SEQ ID N°1496** | IAISLMGYDAGFLR | 57-70 pour les protéines de SEQ N°1254, 1255, 1257, 1258 | 2d |
| **SEQ ID N°1497** | IALSLMAFDAEIIDQK | 75-90 pour les protéines de SEQ N°1118, 1134; 60-75 pour la protéine de séquence SEQ ID N°1119 | 2d |
| **SEQ ID N°1498** | IALSLMGFDSGILK | 53-66 pour les protéines de SEQ N°1252 | 2de |
| **SEQ ID N°1499** | IANALIGLENHK | 87-98 pour les protéines de SEQ N°1168, 1171, 1216 | 2df |
| **SEQ ID N°1500** | IAWIVGFVYLK | 205-215 pour les protéines de SEQ N°1143 | 2d |
| **SEQ ID N°1501** | IDTFWLDNSLK | 141-151 pour les protéines de SEQ N°1143 | 2d |
| **SEQ ID N°1502** | IDYYNLDR | 41-48 pour les protéines de SEQ N°1135 | 2d |
| **SEQ ID N°1503** | IFNALIALDSGVIK | 62-75 pour les protéines de SEQ N°1143 | 2d |
| **SEQ ID N°1504** | IFNSLLALDSGALDNER | 95-111 pour les protéines de SEQ N°1140 | 2df |
| **SEQ ID N°1505** | IFNTLIGLENGIVK | 61-74 pour les protéines de SEQ N°1135 | 2d |
| **SEQ ID N°1506** | IGLDLMQK | 138-145 pour les protéines de SEQ N°1124, 1132, 1145, 1198, 1199, 1217, 1218, 1219, 1220, 1221, 1222, 1223, 1244, 1251 | 2df |
| **SEQ ID N°1507** | IGLEK | 131-135 pour les protéines de SEQ N°1235 | OXA |
| **SEQ ID N°1508** | IGLELMQQEVQR | 133-144 pour les protéines de SEQ N°1227 | 2df |
| **SEQ ID N°1509** | IGLELMSK | 139-146 pour les protéines de SEQ N°1147, 1148, 1149, 1151, 1153, 1156, 1157, 1158, 1159, 1160, 1161, 1162, 1166, 1167, 1169, 1170, 1175, 1177, 1178, 1179, 1180, 1181, 1182, 1183, 1184, 1185, 1186, 1194, 1195, 1196, 1197, 1209, 1210, 1211, 1212, 1213, 1214, 1215, 1225, 1226, 1230, 1231, 1232, 1236, 1237, 1238, 1240, 1241, 1245, 1253, 1260, 1266 | 2df |
| **SEQ ID N°1510** | IGLELMSNEVK | 139-149 pour les protéines de SEQ N°1146, 1150, 1152, 1154, 1155, 1163, 1164, 1165, 1174, 1176, 1192, 1202, 1203, 1204, 1206, 1233, 1261, 1265; 133-143 pour la protéine de séquence SEQ ID N°1189; 133-143 pour la protéine de séquence SEQ ID N°1190 | 2df |
| **SEQ ID N°1511** | IGLER | 126-130 pour les protéines de SEQ N°1138 | 2d |
| **SEQ ID N°1512** | IGLNK | 130-134 pour les protéines de SEQ N°1118, 1134; 115-119 pour la protéine de séquence SEQ ID N°1119 | 2d |
| **SEQ ID N°1513** | IGLNLMQK | 140-147 pour les protéines de SEQ N01224 | 2df |
| **SEQ ID N°1514** | IGPSLMQSELQR | 142-153 pour les protéines de SEQ N°1168, 1171, 1216 | 2df |
| **SEQ ID N°1515** | IGYGNMQIGTEVDQFWLK | 154-171 pour les protéines de SEQ N°1171, 1216 | 2df |
| **SEQ ID N°1516** | IGYGNMQMGTEVDQFWLK | 154-171 pour les protéines de SEQ N°1168 | 2df |
| **SEQ ID N°1517** | IINHNLPVK | 167-175 pour les protéines de SEQ N°1119; 182-190 pour la protéine de séquence SEQ ID N°1118 | 2d |
| **SEQ ID N°1518** | IINHNLPVR | 182-190 pour les protéines de SEQ N°1134 | 2d |
| **SEQ ID N°1519** | ILFQQGTQQACAER | 41-54 pour les protéines de SEQ N°1191 | 2d |
| **SEQ ID N°1520** | ILNNWFK | 20-26 pour les protéines de SEQ N°1143 | 2d |
| **SEQ ID N°1521** | ILNTLISLEEK | 71-81 pour les protéines de SEQ N°1228 | 2df |
| **SEQ ID N°1522** | ILSLVCLSISIGACAEHSMSR | 6-26 pour les protéines de SEQ N°1168, 1171, 1216 | 2df |
| **SEQ ID N°1523** | INESR | 219-223 pour les protéines de SEQ N°1107 | 2d |
| **SEQ ID N°1524** | INIVK | 255-259 pour les protéines de SEQ N°1187, 1188, 1235; 254-258 pour la protéine de séquence SEQ ID N°1144 | 2d |
| **SEQ ID N°1525** | INLYGNALSR | 61-70 pour les protéines de SEQ N°1124, 1132, 1145, 1198, 1199, 1217, 1218, 1219, 1220, 1221, 1222, 1223, 1244, 1251 | 2df |
| **SEQ ID N°1526** | IPFSLNLEMK | 244-253 pour les protéines de SEQ N°1122, 1123, 1129, 1172, 1207, 1264 | 2df |
| **SEQ ID N°1527** | IPHTLFALDADAVR | 76-89 pour les protéines de SEQ N°1131 | 2d |
| **SEQ ID N°1528** | IPHTLFALDAGAAR | 76-89 pour les protéines de SEQ N°1114, 1249 | 2d |
| **SEQ ID N°1529** | IPHTLFALDAGAVR | 76-89 pour les protéines de SEQ N°1108, 1113, 1121, 1125, 1128, 1133, 1173, 1201, 1239, 1246; 65-78 pour la protéine de séquence SEQ ID N°1120 | OXA |
| **SEQ ID N°1530** | IPLGK | 255-259 pour les protéines de SEQ N°1137 | 2df |
| **SEQ ID N°1531** | IPNAIIGLETGVIK | 71-84 pour les protéines de SEQ N°1110, 1116, 1117, 1126, 1141, 1200, 1229, 1250, 1262; 62-75 pour la protéine de séquence SEQ ID N°1106; 62-75 pour la protéine de séquence SEQ ID N°1111; 72-85 pour la protéine de séquence SEQ ID N°1248 | OXA |
| **SEQ ID N°1532** | IPNALIGLETGAIK | 71-84 pour les protéines de SEQ N°1247, 1263 | OXA |
| **SEQ ID N°1533** | IPNSLIAFDTGAVR | 78-91 pour les protéines de SEQ N°1137 | 2df |
| **SEQ ID N°1534** | IPSAIIGLETGVIK | 71-84 pour les protéines de SEQ N°1115, 1193,1259; 62-75 pour la protéine de séquence SEQ ID N°1112 | 2de |
| **SEQ ID N°1535** | ISAFNQVK | 161-168 pour les protéines de SEQ N°1247 | 2d |
| **SEQ ID N°1536** | ISAHEQILFLR | 166-176 pour les protéines de SEQ N°1131 | 2d |
| **SEQ ID N°1537** | ISAMEQTR | 160-167 pour les protéines de SEQ N°1138 | 2d |
| **SEQ ID N°1538** | ISAMEQVK | 165-172 pour les protéines de SEQ N°1187, 1235; 152-159 pour la protéine de séquence SEQ ID N°1135; 164-171 pour la protéine de séquence SEQ ID N°1144 | 2d |
| **SEQ ID N°1539** | ISATEQVAFLR | 164-174 pour les protéines de SEQ N°1142 | 2df |
| **SEQ ID N°1540** | ISATQQIAFLR | 164-174 pour les protéines de SEQ N°1243 | 2df |
| **SEQ ID N°1541** | ISAVNQVEFLESLFLNK | 161-177 pour les protéines de SEQ N°1115, 1116, 1117, 1126, 1141, 1200, 1229, 1262; 162-178 pour la protéine de séquence SEQ ID N°1248 | OXA |
| **SEQ ID N°1542** | ISAVNQVEFLESLYLNK | 161-177 pour les protéines de SEQ N°1110, 1193, 1250, 1259; 152-168 pour la protéine de séquence SEQ ID N°1106; 152-168 pour la protéine de séquence SEQ ID N°1111; 152-168 pour la protéine de séquence SEQ ID N°1112 | OXA |
| **SEQ ID N°1543** | ISAVNQVK | 161-168 pour les protéines de SEQ N°1263 | 2de |
| **SEQ ID N°1544** | ISPEEQIQFLR | 170-180 pour les protéines de SEQ N°1118, 1134; 155-165 pour la protéine de séquence SEQ ID N°1119 | 2d |
| **SEQ ID N°1545** | ISPEEQVR | 166-173 pour les protéines de SEQ N°1109 | 2de |
| **SEQ ID N°1546** | ISPEGQVR | 155-162 pour les protéines de SEQ N°1256 | 2d |
| **SEQ ID N°1547** | ISPLEQLAFLR | 162-172 pour les protéines de SEQ N°1191 | 2d |
| **SEQ ID N°1548** | ITAFQQVDFLR | 188-198 pour les protéines de SEQ N°1139 | 2df |
| **SEQ ID N°1549** | ITLFLLFLNLVFGQDK | 4-19 pour les protéines de SEQ N°1143 | 2d |
| **SEQ ID N°1550** | ITPIQEVNFADDFANNR | 174-190 pour les protéines de SEQ N°1205 | 2df |
| **SEQ ID N°1551** | ITPIQEVNFADDLANNR | 174-190 pour les protéines de SEQ N°1224 | 2df |
| **SEQ ID N°1552** | ITPQQEAQFAYK | 173-184 pour les protéines de SEQ N°1146, 1147, 1148, 1149, 1150, 1151, 1152, 1153, 1154, 1155, 1156, 1157, 1158, 1159, 1160, 1161, 1162, 1164, 1165, 1166, 1167, 1169, 1170, 1174, 1175, 1176, 1177, 1178, 1179, 1180, 1181, 1182, 1183, 1184, 1185, 1186, 1192, 1194, 1195, 1196, 1197, 1202, 1203, 1204, 1206, 1209, 1210, 1211, 1212, 1213, 1214, 1215, 1225, 1226, 1230, 1231, 1232, 1233, 1236, 1237, 1238, 1240, 1241, 1245, 1253, 1260, 1261, 1265, 1266; 167-178 pour la protéine de séquence SEQ ID N°1189; 167-178 pour la protéine de séquence SEQ ID N°1190 | 2df |
| **SEQ ID N°1553** | ITPQQEAQFTYK | 173-184 pour les protéines de SEQ N°1163 | 2df |
| **SEQ ID N°1554** | ITPVQEVNFADDLAHNR | 174-190 pour les protéines de SEQ N°1122, 1123, 1129, 1172, 1207, 1264 | 2df |
| **SEQ ID N°1555** | IVAFALK | 241-247 pour les protéines de SEQ N°1124, 1145 | 2df |
| **SEQ ID N°1556** | IVAFALNMEMR | 241-251 pour les protéines de SEQ N°1198, 1217, 1218, 1219, 1220, 1221, 1222, 1223, 1244, 1251; 242-252 pour la protéine de séquence SEQ ID N°1132; 242-252 pour la protéine de séquence SEQ ID N°1199 | 2df |
| **SEQ ID N°1557** | IVESTTLADGTVVHGK | 186-201 pour les protéines de SEQ N°1254, 1255, 1257, 1258 | 2d |
| **SEQ ID N°1558** | IYNSLIGLNEK | 74-84 pour les protéines de SEQ N°1144, 1187, 1188, 1235 | 2d |
| **SEQ ID N°1559** | KPDIGWWVGWIER | 237-249 pour les protéines de SEQ N°1140 | 2df |
| **SEQ ID N°1560** | LACATNNLAR | 50-59 pour les protéines de SEQ N°1248 | 2d |
| **SEQ ID N°1561** | LAQGELPFPAPVQSTVR | 172-188 pour les protéines de SEQ N°1138 | 2d |
| **SEQ ID N°1562** | LAQNELPYPIEIQK | 177-190 pour les protéines de SEQ N°1187, 1188, 1235 | 2d |
| **SEQ ID N°1563** | LAQNELQYPIEIQK | 176-189 pour les protéines de SEQ N°1144 | 2d |
| **SEQ ID N°1564** | LDFGNK | 143-148 pour les protéines de SEQ N°1187, 1188, 1235; 142-147 pour la protéine de séquence SEQ ID N°1144 | 2d |
| **SEQ ID N°1565** | LDGSLNR | 206-212 pour les protéines de SEQ N°1105 | 2d |
| **SEQ ID N°1566** | LEIGK | 244-248 pour les protéines de SEQ N°1107 | 2d |
| **SEQ ID N°1567** | LEILQQALAELGLYPK | 255-270 pour les protéines de SEQ N°1227 | 2df |
| **SEQ ID N°1568** | LENQEQVK | 173-180 pour les protéines de SEQ N°1135 | 2d |
| **SEQ ID N°1569** | LETQEEVEK | 195-203 pour les protéines de SEQ N°1122 | 2df |
| **SEQ ID N°1570** | LETQEEVK | 195-202 pour les protéines de SEQ N°1123, 1129, 1172, 1205, 1207, 1224, 1264 | 2df |
| **SEQ ID N°1571** | LFAAEGVK | 55-62 pour les protéines de SEQ N°1140 | 2df |
| **SEQ ID N°1572** | LFESAGVK | 58-65 pour les protéines de SEQ N°1139 | 2df |
| **SEQ ID N°1573** | LFGAAGVK | 30-37 pour les protéines de SEQ N°1138 | 2d |
| **SEQ ID N°1574** | LFPEWEK | 110-116 pour les protéines de SEQ N°1146, 1147, 1148, 1149, 1150, 1151, 1152, 1153, 1154, 1155, 1156, 1157, 1158, 1159, 1160, 1161, 1162, 1163, 1164, 1165, 1166, 1167, 1169, 1170, 1174, 1175, 1176, 1177, 1178, 1179, 1180, 1181, 1182, 1183, 1184, 1185, 1186, 1192, 1194, 1195, 1196, 1197, 1202, 1203, 1204, 1206, 1209, 1210, 1211, 1212, 1213, 1214, 1215, 1225, 1226, 1230, 1231, 1232, 1233, 1236, 1237, 1238, 1240, 1241, 1245, 1253, 1260, 1261, 1265, 1266; 104-110 pour la protéine de séquence SEQ ID N°1189; 104-110 pour la protéine de séquence SEQ ID N°1190 | 2df |
| **SEQ ID N°1575** | LGESR | 126-130 pour les protéines de SEQ N°1109 | 2de |
| **SEQ ID N°1576** | LGVDR | 121-125 pour les protéines de SEQ N°1107 | 2d |
| **SEQ ID N°1577** | LHVSER | 181-186 pour les protéines de SEQ N°1136, 1142, 1208, 1234, 1243 | 2df |
| **SEQ ID N°1578** | LHYGNAK | 131-137 pour les protéines de SEQ N°1143 | 2d |
| **SEQ ID N°1579** | LLNLLSQSK | 160-168 pour les protéines de SEQ N°1135 | 2d |
| **SEQ ID N°1580** | LLQDER | 243-248 pour les protéines de SEQ N°1191 | 2d |
| **SEQ ID N°1581** | LLVQDGDCGR | 38-47 pour les protéines de SEQ N°1254, 1255, 1257, 1258 | 2d |
| **SEQ ID N°1582** | LNEVGYGNR | 160-168 pour les protéines de SEQ N°1140 | 2df |
| **SEQ ID N°1583** | LNYGNADPSTK | 144-154 pour les protéines de SEQ N°1113, 1121 | 2d |
| **SEQ ID N°1584** | LNYGNK | 130-135 pour les protéines de SEQ N°1135 | 2d |
| **SEQ ID N°1585** | LPASK | 178-182 pour les protéines de SEQ N° 1247, 1263; 172-176 pour la protéine de séquence SEQ ID N°1256 | 2d |
| **SEQ ID N°1586** | LPHTLFALDADAVR | 76-89 pour les protéines de SEQ N°1127, 1130 | OXA |
| **SEQ ID N°1587** | LPHTLFALDAGAVR | 76-89 pour les protéines de SEQ N°1242 | OXA |
| **SEQ ID N°1588** | LPLAIMGFDSGILQSPK | 62-78 pour les protéines de SEQ N°1107 | 2d |
| **SEQ ID N°1589** | LPLAIMGYDADILLDATTP R | 69-88 pour les protéines de SEQ N°1109 | 2de |
| **SEQ ID N°1590** | LPSSLIALETGAVR | 98-111 pour les protéines de SEQ N°1139 | 2df |
| **SEQ ID N°1591** | LPVSAQTLQYTANILK | 170-185 pour les protéines de SEQ N°1105 | 2d |
| **SEQ ID N°1592** | LPVSER | 205-210 pour les protéines de SEQ N°1139 | 2df |
| **SEQ ID N°1593** | LPVSPTAVDMTER | 173-185 pour les protéines de SEQ N°1254, 1255, 1257, 1258 | 2d |
| **SEQ ID N°1594** | LSASK | 178-182 pour les protéines de SEQ N°1110, 1115, 1116, 1117, 1126, 1141, 1193, 1200, 1229, 1250, 1259, 1262; 169-173 pour la protéine de séquence SEQ ID N°1106; 169-173 pour la protéine de séquence SEQ ID N°1111; 169-173 pour la protéine de séquence SEQ ID N°1112; 179-183 pour la protéine de séquence SEQ ID N°1248 | OXA |
| **SEQ ID N°1595** | LSAVPIYQEVAR | 121-132 pour les protéines de SEQ N°1137 | 2df |
| **SEQ ID N°1596** | LSAVPVYQELAR | 127-138 pour les protéines de SEQ N°1122, 1123, 1129, 1172, 1205, 1207, 1264; 125-136 pour la protéine de séquence SEQ ID N°1124; 125-136 pour la protéine de séquence SEQ ID N°1132; 125-136 pour la protéine de séquence SEQ ID N°1145; 125-136 pour la protéine de séquence SEQ ID N°1198; 125-136 pour la protéine de séquence SEQ ID N°1199; 125-136 pour la protéine de séquence SEQ ID N°1217; 125-136 pour la protéine de séquence SEQ ID N°1218; 125-136 pour la protéine de séquence SEQ ID N°1219; 125-136 pour la protéine de séquence SEQ ID N°1220; 125-136 pour la protéine de séquence SEQ ID N°1221; 125-136 pour la protéine de séquence SEQ ID N°1222; 125-136 pour la protéine de séquence SEQ ID N°1223; 125-136 pour la protéine de séquence SEQ ID N°1244; 125-136 pour la protéine de séquence SEQ ID N°1251 | 2df |
| **SEQ ID N°1597** | LSCTLVIDEASGDLLHR | 37-53 pour les protéines de SEQ N°1109 | 2de |
| **SEQ ID N°1598** | LSLQHGWFIGWIEK | 211-224 pour les protéines de SEQ N°1252 | 2de |
| **SEQ ID N°1599** | LSQNSLPFSQEAMNSVK | 164-180 pour les protéines de SEQ N°1143 | 2d |
| **SEQ ID N°1600** | LSVNPK | 168-173 pour les protéines de SEQ N°1252 | 2de |
| **SEQ ID N°1601** | LTQDER | 239-244 pour les protéines de SEQ N°1254, 1255, 1257, 1258 | 2d |
| **SEQ ID N°1602** | LTVGAR | 245-250 pour les protéines de SEQ N°1109 | 2de |
| **SEQ ID N°1603** | LYGFALNIDMPGGEADIGK | 228-246 pour les protéines de SEQ N°1138 | 2d |
| **SEQ ID N°1604** | LYHNELPFR | 178-186 pour les protéines de SEQ N°1114, 1249 | 2d |
| **SEQ ID N°1605** | LYHNK | 176-180 pour les protéines de SEQ N°1136, 1142, 1208, 1234, 1243 | 2df |
| **SEQ ID N°1606** | LYQNDLPFR | 178-186 pour les protéines de SEQ N°1133 | 2d |
| **SEQ ID N°1607** | MDDLFK | 243-248 pour les protéines de SEQ N°1108, 1114, 1125, 1128, 1133, 1173, 1201, 1239, 1246, 1249; 232-237 pour la protéine de séquence SEQ ID N°1120 | OXA |
| **SEQ ID N°1608** | MEDLHK | 243-248 pour les protéines de SEQ N°1113, 1121 | 2d |
| **SEQ ID N°1609** | MLIALIGLENHK | 85-96 pour les protéines de SEQ N°1264 | 2df |
| **SEQ ID N°1610** | MLLIEQQGDAALYAK | 198-212 pour les protéines de SEQ N°1137 | 2df |
| **SEQ ID N°1611** | MLLIK | 204-208 pour les protéines de SEQ N°1122, 1123, 1129, 1172, 1205, 1207, 1224, 1264 | 2df |
| **SEQ ID N°1612** | MLNALIGLEHHK | 84-95 pour les protéines de SEQ N°1146, 1147, 1148, 1149, 1150, 1151, 1152, 1153, 1154, 1155, 1156, 1157, 1158, 1159, 1160, 1161, 1162, 1163, 1164, 1166, 1167, 1169, 1170, 1174, 1175, 1176, 1177, 1178, 1179, 1180, 1181, 1182, 1183, 1184, 1185, 1186, 1192, 1194, 1195, 1196, 1197, 1202, 1203, 1204, 1206, 1209, 1210, 1211, 1212, 1213, 1214, 1215, 1225, 1226, 1230, 1231, 1232, 1233, 1236, 1237, 1238, 1240, 1245, 1253, 1260, 1261, 1265, 1266; 78-89 pour la protéine de séquence SEQ ID N°1189; 78-89 pour la protéine de séquence SEQ ID N°1190 | 2df |
| **SEQ ID N°1613** | MLNALIGLENHK | 85-96 pour les protéines de SEQ N°1122, 1123, 1129, 1172, 1205, 1207, 1224 | 2df |
| **SEQ ID N°1614** | MLNALIGLENQK | 83-94 pour les protéines de SEQ N°1124, 1132, 1145, 1198, 1199, 1217, 1218, 1219, 1220, 1221, 1222, 1223, 1244, 1251 | 2df |
| **SEQ ID N°1615** | MLNALIGLEYHK | 84-95 pour les protéines de SEQ N°1241 | 2df |
| **SEQ ID N°1616** | MLNALIGLQHGK | 78-89 pour les protéines de SEQ N°1227 | 2df |
| **SEQ ID N°1617** | MLNALISLEHHK | 84-95 pour les protéines de SEQ N°1165 | 2df |
| **SEQ ID N°1618** | MQAYVDAFDYGNR | 139-151 pour les protéines de SEQ N°1137 | 2df |
| **SEQ ID** | MQEGLNK | 123-129 pour les protéines de SEQ N°1135 | 2d |
| **SEQ ID N°1620** | MSPASTYK | 87-94 pour les protéines de SEQ N°1140 | 2df |
| **SEQ ID N°1621** | MTAGGK | 234-239 pour les protéines de SEQ N°1256 | 2d |
| **SEQ ID N°1622** | MVSGK | 165-169 pour les protéines de SEQ N°1105 | 2d |
| **SEQ ID N°1623** | NEHDPVLPYR | 71-80 pour les protéines de SEQ N°1254, 1255, 1257, 1258 | 2d |
| **SEQ ID N°1624** | NEHQIFK | 86-92 pour les protéines de SEQ N°1248; 85-91 pour la protéine de séquence SEQ ID N°1141 | 2d |
| **SEQ ID N°1625** | NEHQVFK | 85-91 pour les protéines de SEQ N°1110, 1115, 1116, 1117, 1126, 1193, 1200, 1229, 1250, 1259, 1262; 76-82 pour la protéine de séquence SEQ ID N°1106; 76-82 pour la protéine de séquence SEQ ID N°1111; 76-82 pour la protéine de séquence SEQ ID N°1112 | OXA |
| **SEQ ID N°1626** | NEITYK | 262-267 pour les protéines de SEQ N°1122, 1123, 1129, 1172, 1205, 1207, 1224, 1264 | 2df |
| **SEQ ID N°1627** | NELLMK | 260-265 pour les protéines de SEQ N°1124, 1145, 1198, 1217, 1218, 1220, 1221, 1222, 1223, 1244, 1251; 261-266 pour la protéine de séquence SEQ ID N°1132; 261-266 pour la protéine de séquence SEQ ID N°1199 | 2df |
| **SEQ ID N°1628** | NELMMK | 260-265 pour les protéines de SEQ N°1219 | 2df |
| **SEQ ID N°1629** | NELPFR | 181-186 pour les protéines de SEQ N°1108, 1113, 1114, 1121, 1125, 1128, 1173, 1201, 1239, 1246, 1249; 170-175 pour la protéine de séquence SEQ ID N°1120 | OXA |
| **SEQ ID N°1630** | NFILIFIFVILISCK | 5-19 pour les protéines de SEQ N°1144, 1187, 1235 | 2d |
| **SEQ ID N°1631** | NFILIFIFVILTSCK | 5-19 pour les protéines de SEQ N°1188 | 2d |
| **SEQ ID N°1632** | NISSYGNNLVR | 62-72 pour les protéines de SEQ N°1224 | 2df |
| **SEQ ID N°1633** | NISTYGNNLTR | 62-72 pour les protéines de SEQ N°1205 | 2df |
| **SEQ ID N°1634** | NLFNEVHTTGVLVIR | 43-57 pour les protéines de SEQ N°1170 | 2df |
| **SEQ ID N°1635** | NLSTYGNALAR | 62-72 pour les protéines de SEQ N°1122, 1123, 1129, 1172, 1207, 1264 | 2df |
| **SEQ ID N°1636** | NMENLELFGK | 187-196 pour les protéines de SEQ N°1143 | 2d |
| **SEQ ID N°1637** | NMLLLEENNGYK | 201-212 pour les protéines de SEQ N°1198 | 2df |
| **SEQ ID N°1638** | NMLLLEESNGYK | 201-212 pour les protéines de SEQ N°1124, 1132, 1145, 1199, 1217, 1218, 1219, 1220, 1221, 1223, 1244, 1251 | 2df |
| **SEQ ID N°1639** | NMLLLEK | 201-207 pour les protéines de SEQ N°1222 | 2df |
| **SEQ ID N°1640** | NMTLGDAMK | 117-125 pour les protéines de SEQ N°1146, 1150,1151,1154,1155,1160,1163,1164, 1174, 1177, 1180, 1182, 1192, 1203, 1204, 1206, 1233, 1261; 111-119 pour la protéine de séquence SEQ ID N°1189; 111-119 pour la protéine de séquence SEQ ID N°1190 | 2df |
| **SEQ ID N°1641** | NNGLTEAWLES SLK | 156-169 pour les protéines de SEQ N°1118, 1134; 141-154 pour la protéine de séquence SEQ ID N°1119 | 2d |
| **SEQ ID N°1642** | NQLPFK | 181-186 pour les protéines de SEQ N°1131 | 2d |
| **SEQ ID N°1643** | NQLPFQVEHQR | 181-191 pour les protéines de SEQ N°1127, 1130,1242 | OXA |
| **SEQ ID N°1644** | NSAIENTIDNMYLQDLENS TK | 191-211 pour les protéines de SEQ N°1134 | 2d |
| **SEQ ID N°1645** | NSAIENTIENMYLQDLDNS TK | 191-211 pour les protéines de SEQ N°1118 | 2d |
| **SEQ ID N°1646** | NSAIENTIENMYLQDLENST K | 176-196 pour les protéines de SEQ N°1119 | 2d |
| **SEQ ID N°1647** | NSAVWVYELFAK | 119-130 pour les protéines de SEQ N°1127, 1130, 1242 | OXA |
| **SEQ ID N°1648** | NSQVPAYK | 118-125 pour les protéines de SEQ N°1187, 1188, 1235; 117-124 pour la protéine de séquence SEQ ID N°1144 | 2d |
| **SEQ ID N°1649** | NSTVWIYELFAK | 119-130 pour les protéines de SEQ N°1114, 1249 | 2d |
| **SEQ ID N°1650** | NSTVWVYELFAK | 119-130 pour les protéines de SEQ N°1108, 1125, 1128, 1131, 1133, 1173, 1201, 1239, 1246; 108-119 pour la protéine de séquence SEQ ID N°1120 | OXA |
| **SEQ ID N°1651** | NSTVWVYQLFAK | 119-130 pour les protéines de SEQ N°1113, 1121 | 2d |
| **SEQ ID N°1652** | NTSGALVIQTDK | 48-59 pour les protéines de SEQ N°1218 | 2df |
| **SEQ ID N°1653** | NTSGVLVIQTDK | 48-59 pour les protéines de SEQ N°1124, 1132, 1145, 1198, 1199, 1217, 1219, 1220, 1221, 1222, 1223, 1244, 1251 | 2df |
| **SEQ ID N°1654** | NVDEMFYYYDGSK | 75-87 pour les protéines de SEQ N°1135 | 2d |
| **SEQ ID N°1655** | NWILR | 204-208 pour les protéines de SEQ N°1108, 1114, 1125, 1127, 1128, 1130, 1133, 1173, 1201, 1239, 1242, 1246, 1249; 193-197 pour la protéine de séquence SEQ ID N°1120 | OXA |
| **SEQ ID N°1656** | NWNAAMDLR | 125-133 pour les protéines de SEQ N°1140 | 2df |
| **SEQ ID N°1657** | NYVDAFHYGNQDISGDK | 118-134 pour les protéines de SEQ N°1252 | 2de |
| **SEQ ID N°1658** | QADHAILVFDQAR | 51-63 pour les protéines de SEQ N°1133 | 2d |
| **SEQ ID N°1659** | QAEHALLVFGQER | 51-63 pour les protéines de SEQ N°1127, 1130, 1242 | OXA |
| **SEQ ID N°1660** | QAITK | 251-255 pour les protéines de SEQ N°1136, 1142, 1208, 1243; 247-251 pour la protéine de séquence SEQ ID N°1234 | 2df |
| **SEQ ID N°1661** | QAMLTEANSDYIIR | 193-206 pour les protéines de SEQ N°1142 | 2df |
| **SEQ ID N°1662** | QEVQFVSALAR | 171-181 pour les protéines de SEQ N°1227 | 2df |
| **SEQ ID N°1663** | QFASIK | 243-248 pour les protéines de SEQ N°1105 | 2d |
| **SEQ ID N°1664** | QGMPGSIR | 254-261 pour les protéines de SEQ N°1205 | 2df |
| **SEQ ID N°1665** | QGMSGSIR | 254-261 pour les protéines de SEQ N°1224 | 2df |
| **SEQ ID N°1666** | QGQTQQSYGNDLAR | 58-71 pour les protéines de SEQ N°1146, 1147, 1148, 1149, 1150, 1151, 1152, 1153, 1154, 1155, 1156, 1157, 1158, 1159, 1160, 1161, 1162, 1163, 1164, 1165, 1166, 1167, 1169, 1170, 1174, 1175, 1176, 1177, 1178, 1179, 1180, 1181, 1182, 1183, 1184, 1185, 1186, 1194, 1195, 1196, 1197, 1202, 1203, 1204, 1206, 1209, 1210, 1211, 1212, 1213, 1214, 1215, 1225, 1226, 1230, 1231, 1232, 1233, 1236, 1237, 1238, 1240, 1241, 1245, 1253, 1260, 1261, 1265, 1266; 52-65 pour la protéine de séquence SEQ ID N°1189; 52-65 pour la protéine de séquence SEQ ID N°1190 | 2df |
| **SEQ ID N°1667** | QIDYGNADPSTIK | 143-155 pour les protéines de SEQ N°1127, 1130, 1242 | OXA |
| **SEQ ID N°1668** | QIDYGNVDPSTIK | 143-155 pour les protéines de SEQ N°1l31 | 2d |
| **SEQ ID N°1669** | QIGEAR | 129-134 pour les protéines de SEQ N°1136, 1208, 1234, 1243 | 2df |
| **SEQ ID N°1670** | QIGQAR | 129-134 pour les protéines de SEQ N°1142 | 2df |
| **SEQ ID N°1671** | QIMLIEQTPAFTLR | 190-203 pour les protéines de SEQ N°1228 | 2df |
| **SEQ ID N°1672** | QLGSAIDQFWLR | 152-163 pour les protéines de SEQ N°1137 | 2df |
| **SEQ ID N°1673** | QLHDNK | 199-204 pour les protéines de SEQ N°1139 | 2df |
| **SEQ ID N°1674** | QLIFVHTVVQK | 229-239 pour les protéines de SEQ N°1105 | 2d |
| **SEQ ID N°1675** | QLIFVHTVVQKPGK | 229-242 pour les protéines de SEQ N°1105 | 2d |
| **SEQ ID N°1676** | QLPVK | 178-182 pour les protéines de SEQ N°1191; 184-188 pour la protéine de séquence SEQ ID N°1137 | OXA |
| **SEQ ID N°1677** | QLPVKPR | 184-190 pour les protéines de SEQ N°1137 | 2df |
| **SEQ ID N°1678** | QLSLDVLDK | 265-273 pour les protéines de SEQ N°1168, 1171, 1216 | 2df |
| **SEQ ID N°1679** | QLVYAR | 237-242 pour les protéines de SEQ N°1191 | 2d |
| **SEQ ID N°1680** | QMMLTEASTDYIIR | 217-230 pour les protéines de SEQ N°1139 | 2df |
| **SEQ ID N°1681** | QMSIVEATPDYVLHGK | 214-229 pour les protéines de SEQ N°1140 | 2df |
| **SEQ ID N°1682** | QPTDPAR | 99-105 pour les protéines de SEQ N°1191 | 2d |
| **SEQ ID N°1683** | QPTDPTR | 93-99 pour les protéines de SEQ N°1254, 1255, 1257, 1258 | 2d |
| **SEQ ID N°1684** | QPVSAGIR | 246-253 pour les protéines de SEQ N°1254, 1255, 1257, 1258 | 2d |
| **SEQ ID N°1685** | QQLVK | 275-279 pour les protéines de SEQ N°1139 | 2df |
| **SEQ ID N°1686** | QTLVFAR | 232-238 pour les protéines de SEQ N°1254, 1255, 1257, 1258 | 2d |
| **SEQ ID N°1687** | QVGAEK | 126-131 pour les protéines de SEQ N°1228 | 2df |
| **SEQ ID N°1688** | QVVFAR | 238-243 pour les protéines de SEQ N°1107 | 2d |
| **SEQ ID N°1689** | SADEVLPYGGK | 84-94 pour les protéines de SEQ N°1138 | 2d |
| **SEQ ID N°1690** | SADEVLPYGGKPQR | 84-97 pour les protéines de SEQ N°1138 | 2d |
| **SEQ ID N°1691** | SCATNDLAR | 50-58 pour les protéines de SEQ N°1110, 1193, 1250, 1259; 41-49 pour la protéine de séquence SEQ ID N°1106; 41-49 pour la protéine de séquence SEQ ID N°1111; 41-49 pour la protéine de séquence SEQ ID N°1112 | OXA |
| **SEQ ID N°1692** | SCATNNLAR | 50-58 pour les protéines de SEQ N°1115, 1116, 1117, 1126, 1141, 1200, 1229, 1262 | OXA |
| **SEQ ID N°1693** | SDIPGGSK | 251-258 pour les protéines de SEQ N°1107 | 2d |
| **SEQ ID** | SDWGK | 29-33 pour les protéines de SEQ N°1133 | 2d |
| **SEQ ID N°1695** | SEDNFHISSQQHEK | 27-40 pour les protéines de SEQ N°1122, 1123, 1129, 1172, 1207, 1264 | 2df |
| **SEQ ID N°1696** | SEMPASIR | 252-259 pour les protéines de SEQ N°1124, 1145, 1198, 1217, 1218, 1219, 1220, 1221, 1222, 1244, 1251; 253-260 pour la protéine de séquence SEQ ID N°1132; 253-260 pour la protéine de séquence SEQ ID N°1199 | 2df |
| **SEQ ID N°1697** | SEMPASTR | 252-259 pour les protéines de SEQ N°1223 | 2df |
| **SEQ ID N°1698** | SFAAHNQDQDLR | 103-114 pour les protéines de SEQ N°1114, 1249 | 2d |
| **SEQ ID N°1699** | SFAGHNK | 103-109 pour les protéines de SEQ N°1133 | 2d |
| **SEQ ID N°1700** | SFAGHNQDQDLR | 103-114 pour les protéines de SEQ N°1127, 1130, 1131, 1242 | 2d |
| **SEQ ID N°1701** | SFAGHNQDQNLR | 103-114 pour les protéines de SEQ N°1113, 1121 | 2d |
| **SEQ ID N°1702** | SFLESWAK | 100-107 pour les protéines de SEQ N°1144 | 2d |
| **SEQ ID N°1703** | SFTAWEK | 109-115 pour les protéines de SEQ N°1124, 1132, 1145, 1199, 1217, 1218, 1219, 1220, 1221, 1222, 1223, 1244, 1251; 104-110 pour la protéine de séquence SEQ ID N°1227 | 2df |
| **SEQ ID N°1704** | SFTTWEK | 109-115 pour les protéines de SEQ N°1198 | 2df |
| **SEQ ID N°1705** | SGSGWLR | 207-213 pour les protéines de SEQ N°ll07 | 2d |
| **SEQ ID N°1706** | SGWGMAVDPQVGWYVGF VEK | 221-240 pour les protéines de SEQ N°1168, 1171, 1216 | 2df |
| **SEQ ID N°1707** | SGWGMDVSPQVGWLTGW VEK | 219-238 pour les protéines de SEQ N°1224 | 2df |
| **SEQ ID N°1708** | SGWGMDVTPQVGWLTGW VEK | 219-238 pour les protéines de SEQ N°1205 | 2df |
| **SEQ ID N°1709** | SIHPASTFK | 69-77 pour les protéines de SEQ N°1137 | 2df |
| **SEQ ID N°1710** | SIPTK | 252-256 pour les protéines de SEQ N°1110, 1115, 1116, 1117, 1126, 1141, 1193, 1200, 1229, 1248, 1250, 1259, 1262, 1263; 243-247 pour la protéine de séquence SEQ ID N°1106; 243-247 pour la protéine de séquence SEQ ID N°1111; 243-247 pour la protéine de séquence SEQ ID N°1112 | OXA |
| **SEQ ID N°1711** | SISTK | 252-256 pour les protéines de SEQ N°1247 | 2d |
| **SEQ ID N°1712** | SLGLSNNLSR | 76-85 pour les protéines de SEQ N°1139 | 2df |
| **SEQ ID N°1713** | SLSMSGK | 4-10 pour les protéines de SEQ N°1109 | 2de |
| **SEQ ID N°1714** | SMLFIEEK | 202-209 pour les protéines de SEQ N°1146, 1147, 1148, 1149, 1150, 1151, 1152, 1153, 1154, 1155, 1156, 1157, 1158, 1159, 1160, 1161, 1162, 1163, 1164, 1165, 1166, 1167, 1169, 1174, 1175, 1176, 1177, 1178, 1179, 1180, 1182, 1183, 1184, 1185, 1186, 1192, 1194, 1195, 1196, 1197, 1202, 1203, 1204, 1209, 1210, 1211, 1212, 1213, 1214, 1215, 1225, 1226, 1230, 1231, 1232, 1233, 1236, 1237, 1238, 1240, 1241, 1245, 1253, 1260, 1261, 1265, 1266; 196-203 pour la protéine de séquence SEQ ID N°1189; 196-203 pour la protéine de séquence SEQ ID N°1190 | 2df |
| **SEQ ID N°1715** | SNGEK | 239-243 pour les protéines de SEQ N°1205, 1224 | 2df |
| **SEQ ID N°1716** | SNGLTHSWLGSSLK | 141-154 pour les protéines de SEQ N°1256 | 2d |
| **SEQ ID N°1717** | SNGYK | 208-212 pour les protéines de SEQ N°1124, 1132, 1145, 1199, 1217, 1218, 1219, 1220, 1221, 1222, 1223, 1244, 1251 | 2df |
| **SEQ ID N°1718** | SPTWELK | 79-85 pour les protéines de SEQ N°1107 | 2d |
| **SEQ ID N°1719** | SPTWELKPEYNPSPR | 79-93 pour les protéines de SEQ N°1107 | 2d |
| **SEQ ID N°1720** | SQDIVR | 208-213 pour les protéines de SEQ N°1140 | 2df |
| **SEQ ID N°1721** | SQQKPTDPTIWLK | 100-112 pour les protéines de SEQ N°1109 | 2de |
| **SEQ ID N°1722** | SQVGWLTGWVEQPDGK | 225-240 pour les protéines de SEQ N°1244 | 2df |
| **SEQ ID N°1723** | SSSNSCTTNNAAR | 46-58 pour les protéines de SEQ N°1247 | 2d |
| **SEQ ID N°1724** | SSSNSCTTNNATR | 46-58 pour les protéines de SEQ N°1263 | 2de |
| **SEQ ID N°1725** | SVYGELR | 139-145 pour les protéines de SEQ N°1228 | 2df |
| **SEQ ID N°1726** | SWILR | 204-208 pour les protéines de SEQ N°1131 | 2d |
| **SEQ ID N°1727** | SYFDEAQTQGVIIIK | 44-58 pour les protéines de SEQ N°1122, 1123, 1129, 1172, 1205, 1207, 1224, 1264 | 2df |
| **SEQ ID N°1728** | SYLEK | 139-143 pour les protéines de SEQ N°1113, 1121 | 2d |
| **SEQ ID N°1729** | SYPMWEK | 111-117 pour les protéines de SEQ N°1205, 1224 | 2df |
| **SEQ ID N°1730** | TAYIPASTFK | 61-70 pour les protéines de SEQ N°1247, 1263; 77-86 pour la protéine de séquence SEQ ID N°1168; 77-86 pour la protéine de séquence SEQ ID N°1171; 77-86 pour la protéine de séquence SEQ ID N°1216 | 2df |
| **SEQ ID N°1731** | TDDLFK | 243-248 pour les protéines de SEQ N°1127, 1130, 1131, 1242 | 2d |
| **SEQ ID N°1732** | TDINEIFK | 95-102 pour les protéines de SEQ N°1132, 1145, 1198, 1199, 1217, 1218, 1219, 1220, 1221, 1222, 1223, 1244, 1251 | 2df |
| **SEQ ID N°1733** | TFIHNDPR | 51-58 pour les protéines de SEQ N°1228 | 2df |
| **SEQ ID N°1734** | TGAGFTANR | 216-224 pour les protéines de SEQ N°1118, 1134; 201-209 pour la protéine de séquence SEQ ID N°1119 | 2d |
| **SEQ ID N°1735** | TGFNDGQK | 197-204 pour les protéines de SEQ N°1143 | 2d |
| **SEQ ID N°1736** | TGLADSK | 210-216 pour les protéines de SEQ N°1187, 1188; 209-215 pour la protéine de séquence SEQ ID N°1144 | 2d |
| **SEQ ID N°1737** | TGLDLMQK | 140-147 pour les protéines de SEQ N°1205 | 2df |
| **SEQ ID N°1738** | TGLELMQK | 140-147 pour les protéines de SEQ N°1122, 1123, 1129, 1172, 1207, 1264 | 2df |
| **SEQ ID N°1739** | TGMGYPK | 198-204 pour les protéines de SEQ N°1105 | 2d |
| **SEQ ID N°1740** | TGNGR | 197-201 pour les protéines de SEQ N°1252 | 2de |
| **SEQ ID N°1741** | TGTGSFIDAR | 200-209 pour les protéines de SEQ N°1256 | 2d |
| **SEQ ID N°1742** | TGTGSLSDAK | 211-220 pour les protéines de SEQ N°1109 | 2de |
| **SEQ ID N°1743** | TGVATEYQPEIGWWAGWV ER | 213-232 pour les protéines de SEQ N°1137 | 2df |
| **SEQ ID N°1744** | TGVSYPLLADGTR | 202-214 pour les protéines de SEQ N°1254, 1255, 1257, 1258 | 2d |
| **SEQ ID N°1745** | TGWAAMDIK | 217-225 pour les protéines de SEQ N°1132, 1199 | 2df |
| **SEQ ID N°1746** | TGWAMDIK | 217-224 pour les protéines de SEQ N°1124, 1145, 1198, 1218, 1219, 1220, 1221, 1222, 1223, 1244, 1251 | 2df |
| **SEQ ID N°1747** | TGWAMDVK | 217-224 pour les protéines de SEQ N°1217 | 2df |
| **SEQ ID N°1748** | TGWATR | 206-211 pour les protéines de SEQ N°1228 | 2df |
| **SEQ ID N°1749** | TGWCFDCTPELGWWVGW VK | 205-223 pour les protéines de SEQ N°1138 | 2d |
| **SEQ ID N°1750** | TGWEGR | 211-216 pour les protéines de SEQ N°1108, 1114, 1125, 1127, 1128, 1130, 1131, 1173, 1201, 1239, 1242, 1246, 1249; 200-205 pour la protéine de séquence SEQ ID N°1120 | OXA |
| **SEQ ID N°1751** | TGWFVDK | 230-236 pour les protéines de SEQ N°1140 | 2df |
| **SEQ ID N°1752** | TGYDTK | 209-214 pour les protéines de SEQ N°1234 | 2df |
| **SEQ ID N°1753** | TGYGVR | 233-238 pour les protéines de SEQ N°1139 | 2df |
| **SEQ ID N°1754** | TGYSAR | 209-214 pour les protéines de SEQ N°1208 | 2df |
| **SEQ ID N°1755** | TGYSTR | 209-214 pour les protéines de SEQ N°1136, 1142, 1243 | 2df |
| **SEQ ID N°1756** | THESSNWGK | 25-33 pour les protéines de SEQ N°1113, 1121 | 2d |
| **SEQ ID N°1757** | TICTAIADAGTGK | 25-37 pour les protéines de SEQ N°1254, 1255, 1257, 1258 | 2d |
| **SEQ ID N°1758** | TIGGAPDAYWVDDSLQISA R | 169-188 pour les protéines de SEQ N°1140 | 2df |
| **SEQ ID N°1759** | TLPFSASSYETLR | 177-189 pour les protéines de SEQ N°1228 | 2df |
| **SEQ ID N°1760** | TLPFSLK | 189-195 pour les protéines de SEQ N°1157, 1161, 1169 | 2df |
| **SEQ ID N°1761** | TLPFSPK | 189-195 pour les protéines de SEQ N°1147, 1153, 1170, 1181, 1186, 1197, 1203, 1225, 1240, 1241, 1253 | 2df |
| **SEQ ID N°1762** | TLPFSQEVQDEVQSILFIEE K | 189-209 pour les protéines de SEQ N°1206 | 2df |
| **SEQ ID N°1763** | TLPFSQEVQDEVQSMLFIEE K | 189-209 pour les protéines de SEQ N°1150, 1192 | 2df |
| **SEQ ID N°1764** | TLPFSQK | 189-195 pour les protéines de SEQ N°1146, 1148, 1149, 1151, 1152, 1154, 1155, 1156, 1158, 1159, 1160, 1162, 1163, 1164, 1165, 1166, 1167, 1174, 1175, 1176, 1177, 1178, 1179, 1180, 1182, 1183, 1184, 1185, 1194, 1195, 1196, 1202, 1204, 1209, 1210, 1211, 1212, 1213, 1214, 1215, 1226, 1230, 1231, 1232, 1233, 1236, 1238, 1245, 1260, 1261, 1265, 1266; 183-189 pour la protéine de séquence SEQ ID N°1189; 183-189 pour la protéine de séquence SEQ ID N°1190 | 2df |
| **SEQ ID N°1765** | TLPSSQK | 189-195 pour les protéines de SEQ N°1237 | 2df |
| **SEQ ID N°1766** | TLQNGWFEGFIISK | 225-238 pour les protéines de SEQ N°1118, 1134; 210-223 pour la protéine de séquence SEQ ID N°1119 | 2d |
| **SEQ ID N°1767** | TMQEYLNK | 123-130 pour les protéines de SEQ N°1143 | 2d |
| **SEQ ID N°1768** | TNGNSTSVYNESR | 51-63 pour les protéines de SEQ N°1113, 1121 | 2d |
| **SEQ ID N°1769** | TQTYQAYDAAR | 72-82 pour les protéines de SEQ N°1140 | 2df |
| **SEQ ID N°1770** | TTDPTIWEK | 93-101 pour les protéines de SEQ N°1256 | 2d |
| **SEQ ID N°1771** | TTTTEVFK | 96-103 pour les protéines de SEQ N°1153, 1186 | 2df |
| **SEQ ID N°1772** | TWASNDFSR | 41-49 pour les protéines de SEQ N°1143 | 2d |
| **SEQ ID N°1773** | TWDMVQR | 191-197 pour les protéines de SEQ N°1137 | 2df |
| **SEQ ID N°1774** | TWMQFSVVWVSQEITQK | 113-129 pour les protéines de SEQ N°1118, 1134; 98-114 pour la protéine de séquence SEQ ID N°1119 | 2d |
| **SEQ ID N°1775** | TYPMWEK | 111-117 pour les protéines de SEQ N°1122, 1123, 1129, 1172, 1207, 1264 | 2df |
| **SEQ ID N°1776** | TYVVDPAR | 58-65 pour les protéines de SEQ N°1137 | 2df |
| **SEQ ID N°1777** | VAFSLNIEMK | 244-253 pour les protéines de SEQ N°1205 | 2df |
| **SEQ ID N°1778** | VANSLIGLSTGAVR | 70-83 pour les protéines de SEQ N°1138 | 2d |
| **SEQ ID N°1779** | VEHQR | 187-191 pour les protéines de SEQ N°1108, 1113, 1114, 1121, 1125, 1127, 1128, 1130, 1131, 1133, 1173, 1201, 1239, 1242, 1246, 1249; 176-180 pour la protéine de séquence SEQ ID N°1120 | OXA |
| **SEQ ID N°1780** | VELGK | 248-252 pour les protéines de SEQ N°1138 | 2d |
| **SEQ ID N°1781** | VFDDAGVSGTFVLMDITAD R | 38-57 pour les protéines de SEQ N°1137 | 2df |
| **SEQ ID N°1782** | VFLDSWAK | 88-95 pour les protéines de SEQ N°1135 | 2d |
| **SEQ ID N°1783** | VFLESWAK | 101-108 pour les protéines de SEQ N°1187, 1188, 1235 | 2d |
| **SEQ ID N°1784** | VFLSSWAQDMNLSSAIK | 89-105 pour les protéines de SEQ N°1143 | 2d |
| **SEQ ID N°1785** | VGFER | 134-138 pour les protéines de SEQ N°1137 | 2df |
| **SEQ ID N°1786** | VILVFDQVR | 55-63 pour les protéines de SEQ N°1114, 1249 | 2d |
| **SEQ ID N°1787** | VITFTK | 228-233 pour les protéines de SEQ N°1252 | 2de |
| **SEQ ID N°1788** | VMAAMVR | 158-164 pour les protéines de SEQ N°1139 | 2df |
| **SEQ ID N°1789** | VPLAVMGYDAGILVDAHN PR | 58-77 pour les protéines de SEQ N°1256 | 2d |
| **SEQ ID N°1790** | VQANVK | 195-200 pour les protéines de SEQ N°1124, 1132, 1145, 1198, 1199, 1217, 1218, 1219, 1220, 1221, 1222, 1223, 1244, 1251 | 2df |
| **SEQ ID N°1791** | VQDEVK | 196-201 pour les protéines de SEQ N°1202 | 2df |
| **SEQ ID N°1792** | VQDEVQSMLFIEEK | 196-209 pour les protéines de SEQ N°1146, 1147, 1148, 1149, 1150, 1151, 1153, 1154, 1155, 1156, 1157, 1158, 1159, 1160, 1162, 1163, 1164, 1165, 1166, 1167, 1169, 1174, 1175, 1176, 1177, 1178, 1179, 1180, 1182, 1183, 1184, 1185, 1186, 1192, 1194, 1195, 1196, 1197, 1203, 1204, 1209, 1210, 1211, 1212, 1213, 1215, 1225, 1226, 1230, 1231, 1232, 1233, 1236, 1237, 1238, 1240, 1241, 1245, 1253, 1260, 1261, 1265, 1266; 190-203 pour la protéine de séquence SEQ ID N°1189; 190-203 pour la protéine de séquence SEQ ID N°1190 | 2df |
| **SEQ ID N°1793** | VQDEVQSMLFIEEMNGNK | 196-213 pour les protéines de SEQ N°1170, 1181 | 2df |
| **SEQ ID N°1794** | VQDGVQSMLFIEEK | 196-209 pour les protéines de SEQ N°1214 | 2df |
| **SEQ ID N°1795** | VQHEVQSMLFIEEK | 196-209 pour les protéines de SEQ N°1152 | 2df |
| **SEQ ID N°1796** | VSCLPCYQVVSHK | 138-150 pour les protéines de SEQ N°1140 | 2df |
| **SEQ ID N°1797** | VSCVWCYQALAR | 114-125 pour les protéines de SEQ N°1228 | 2df |
| **SEQ ID N°1798** | VSDVCSEVTAEGWQEVR | 37-53 pour les protéines de SEQ N°1139 | 2df |
| **SEQ ID N°1799** | VSEVEGWQIHGK | 186-197 pour les protéines de SEQ N°1105 | 2d |
| **SEQ ID N°1800** | VSFSLNIEMK | 244-253 pour les protéines de SEQ N°1224 | 2df |
| **SEQ ID N°1801** | VSPCSSFK | 54-61 pour les protéines de SEQ N°1107 | 2d |
| **SEQ ID N°1802** | VSQVPAYK | 105-112 pour les protéines de SEQ N°1135 | 2d |
| **SEQ ID N°1803** | VVFAR | 229-233 pour les protéines de SEQ N°1256; 239-243 pour la protéine de séquence SEQ ID N°1107; 240-244 pour la protéine de séquence SEQ ID N°1109 | OXA |
| **SEQ ID N°1804** | WDGAK | 97-101 pour les protéines de SEQ N°1113, 1121 | 2d |
| **SEQ ID N°1805** | WDGEK | 104-108 pour les protéines de SEQ N°1151, 1160,1177,1180,1182 | 2df |
| **SEQ ID N°1806** | WDGHIYDFPDWNR | 92-104 pour les protéines de SEQ N°1228 | 2df |
| **SEQ ID N°1807** | WDGIK | 97-101 pour les protéines de SEQ N°1114, 1249 | 2d |
| **SEQ ID N°1808** | WDGKPR | 92-97 pour les protéines de SEQ N°1110, 1115, 1116, 1117, 1126, 1141, 1193, 1200, 1229, 1247, 1250, 1259, 1262, 1263; 83-88 pour la protéine de séquence SEQ ID N°1106; 83-88 pour la protéine de séquence SEQ ID N°1111; 83-88 pour la protéine de séquence SEQ ID N°1112; 116-121 pour la protéine de séquence SEQ ID N°1140; 107-112 pour la protéine de séquence SEQ ID N°1168; 107-112 pour la protéine de séquence SEQ ID N°1171; 107-112 pour la protéine de séquence SEQ ID N°1216; 93-98 pour la protéine de séquence SEQ ID N°1248 | OXA |
| **SEQ ID N°1809** | WDGQK | 104-108 pour les protéines de SEQ N°1146, 1147, 1150, 1153, 1154, 1155, 1157, 1161, 1163,1164,1165,1169,1170,1181,1186, 1192, 1197, 1203, 1204, 1206, 1225, 1226, 1240, 1241, 1253, 1261, 1266; 98-102 pour la protéine de séquence SEQ ID N°1189; 98-102 pour la protéine de séquence SEQ ID N°1190 | 2df |
| **SEQ ID N°1810** | WDGQTR | 95-100 pour les protéines de SEQ N°1136, 1142, 1208, 1234, 1243 | 2df |
| **SEQ ID N°1811** | WDGVK | 97-101 pour les protéines de SEQ N°1127, 1130, 1133, 1242 | 2d |
| **SEQ ID N°1812** | WDGVNR | 97-102 pour les protéines de SEQ N°1108, 1125, 1128, 1131, 1173, 1201, 1239, 1246; 86-91 pour la protéine de séquence SEQ ID N°1120 | OXA |
| **SEQ ID N°1813** | WDYKPEFNGYK | 78-88 pour les protéines de SEQ N°1256; 89-99 pour la protéine de séquence SEQ ID N°1109 | OXA |
| **SEQ ID N°1814** | WETYSVVWFSQQITEWLG MER | 97-117 pour les protéines de SEQ N°1105 | 2d |
| **SEQ ID N°1815** | WNGQK | 104-108 pour les protéines de SEQ N°1152, 1176, 1202, 1265 | 2df |
| **SEQ ID N°1816** | YAQAK | 155-159 pour les protéines de SEQ N°1140 | 2df |
| **SEQ ID N°1817** | YFSDFNAK | 34-41 pour les protéines de SEQ N°1113, 1121 | 2d |
| **SEQ ID N°1818** | YGTHLDR | 68-74 pour les protéines de SEQ N°1168, 1171, 1216 | 2df |
| **SEQ ID N°1819** | YIIHNK | 54-59 pour les protéines de SEQ N°1144, 1187, 1188, 1235 | 2d |
| **SEQ ID N°1820** | YLDELVK | 245-251 pour les protéines de SEQ N°1143 | 2d |
| **SEQ ID N°1821** | YLMITEAGR | 195-203 pour les protéines de SEQ N°1242 | OXA |
| **SEQ ID N°1822** | YLNLFSYGNANIGGGIDK | 135-152 pour les protéines de SEQ N°1247, 1263 | OXA |
| **SEQ ID N°1823** | YNGEK | 96-100 pour les protéines de SEQ N°1187, 1188, 1235 | 2d |
| **SEQ ID N°1824** | YPHNPR | 88-93 pour les protéines de SEQ N°1252 | 2de |
| **SEQ ID N°1825** | YPVVWYSQQVAHHLGAQR | 103-120 pour les protéines de SEQ N°1255 | 2d |
| **SEQ ID N°1826** | YSNVLAFK | 106-113 pour les protéines de SEQ N°1143 | 2d |
| **SEQ ID N°1827** | YSPASTFK | 68-75 pour les protéines de SEQ N°1108, 1113, 1114, 1121, 1125, 1127, 1128, 1130, 1131, 1133, 1173, 1201, 1239, 1242, 1246, 1249; 57-64 pour la protéine de séquence SEQ ID N°1120 | OXA |
| **SEQ ID N°1828** | YSVVPVYQQLAR | 141-152 pour les protéines de SEQ N°1139 | 2df |
| **SEQ ID N°1829** | YSVVWYSQLTAK | 109-120 pour les protéines de SEQ N°1191 | 2d |
| **SEQ ID N°1830** | YSVVWYSQQVAHHLGAQR | 103-120 pour les protéines de SEQ N°1254, 1257, 1258 | 2d |
| **SEQ ID N°1831** | YTPASTFK | 55-62 pour les protéines de SEQ N°1191 | 2d |
| **SEQ ID N°1832** | YTSAFGYGNADVSGEPGK | 130-147 pour les protéines de SEQ N°1191 | 2d |
| **SEQ ID N°1833** | YVFVSALTGNLGSNLTSSIK | 228-247 pour les protéines de SEQ N°1119; 243-262 pour la protéine de séquence SEQ ID N°1118 | 2d |
| **SEQ ID N°1834** | YVFVSALTGSLGSNLTSSIK | 243-262 pour les protéines de SEQ N°1134 | 2d |
| **SEQ ID N°1835** | YVGHDR | 50-55 pour les protéines de SEQ N°1138 | 2d |
| **SEQ ID N°2160** | ANQAFLPASTFK | 62-73 pour les protéines de SEQ N°1136, 1142, 1208, 1234, 1243 | 2df |
| **SEQ ID N°2161** | DEHQVFK | 88-94 pour les protéines de SEQ N°1136, 1142, 1208, 1234, 1243 | 2df |
| **SEQ ID N°2162** | DHNLITAMK | 108-116 pour les protéines de SEQ N°1136, 1208, 1234 | 2df |
| **SEQ ID N°2163** | DIATWNR | 101-107 pour les protéines de SEQ N°1136, 1208, 1234 | 2df |
| **SEQ ID N°2164** | IPNSLIALDLGVVK | 74-87 pour les protéines de SEQ N°1136, 1142, 1208, 1234, 1243 | 2df |
| **SEQ ID N°2165** | ISATEQISFLR | 164-174 pour les protéines de SEQ N°1136, 1208, 1234 | 2df |
| **SEQ ID N°2166** | QAMLTEANGDYIIR | 193-206 pour les protéines de SEQ N°1136, 1208, 1234, 1243 | 2df |
| **SEQ ID N°2167** | QQGFTNNLK | 52-60 pour les protéines de SEQ N°1136, 1142, 1208, 1234, 1243 | 2df |
| **SEQ ID N°2168** | SQGVVVLWNENK | 40-51 pour les protéines de SEQ N°1136, 1208, 1234, 1243 | 2df |
| **SEQ ID N°2169** | SWNAHFTEHK | 30-39 pour les protéines de SEQ N°1136, 1208, 1234, 1243 | 2df |
| **SEQ ID N°2170** | VLALSAVFLVASIIGMPAVA K | 3-23 pour les protéines de SEQ N°1136, 1208, 1234,1243 | 2df |
| **SEQ ID N°2171** | YSVVPVYQEFAR | 117-128 pour les protéines de SEQ N°1136, 1142, 1208, 1234, 1243 | 2df |

Dans la colonne intérêt clinique, les mentions 2d, 2de, 2df correspondent aux sous-groupes fonctionnels des béta-lactamases OXA que permet de détecter le peptide correspondant. Ainsi la détection d'un peptide 2df signera la présence d'une beta-lactamase de type carbapénèmase capable d'hydrolyser les carbapénèmes.

La mention 2de signera la présence d'une beta-lactamase avec un spectre étendu (BLSE) capable d'hydrolyser les pénicillines, les céphalosporines de première génération telles que la céphaloridine et la céphalotine ainsi qu'au moins un antibiotique de la classe des oxyimino-beta-lactames tels que le cefotaxime, le ceftazidime ou les monobactames tels que l'aztreoname.

La mention OXA indique un peptide commun entre au moins deux des sous-groupes 2d, 2de et 2df. Le peptide correspondant signe la présence d'une béta-lactamase de type OXA et la présence d'un mécanisme de résistance au moins aux pénicillines et aux céphalosporines de première génération.

La détection d'un mécanisme de résistance aux céphalosporines de type BLSE (beta-lactamase à spectre étendu) induit par la protéine OXA, est caractérisée par la détection d'au moins un peptide marqueur de résistance, de type 2de, choisi parmi les séquences **SEQ ID N° 1277, 1297, 1323, 1344, 1368, 1369, 1373, 1375, 1392, 1421, 1423, 1427, 1429, 1439, 1444, 1446, 1450, 1451, 1457, 1465, 1468, 1490, 1491, 1495, 1498, 1534, 1543, 1545, 1575, 1589, 1597, 1598, 1600, 1602, 1657, 1713, 1721, 1724, 1740, 1742, 1787, 1824, 1268, 1269, 1270, 1271, 1272, 1278, 1279, 1280, 1281, 1283, 1285, 1288, 1290, 1295, 1299, 1300, 1301, 1302, 1303, 1304, 1305, 1306, 1307, 1308, 1309, 1310, 1314, 1315, 1316, 1317, 1318, 1319, 1320, 1332, 1337, 1339, 1340, 1341, 1342, 1350, 1354, 1355, 1356, 1357, 1358, 1359, 1360, 1365, 1366, 1367, 1386, 1389, 1390, 1391, 1393, 1394, 1402, 1404, 1405, 1407, 1408, 1413, 1414, 1419, 1420, 1425, 1432, 1433, 1440, 1447, 1448, 1456, 1471, 1472, 1477, 1478, 1480, 1485, 1487, 1488, 1499, 1504, 1506, 1508, 1509, 1510, 1513, 1514, 1515, 1516, 1521, 1522, 1525, 1526, 1530, 1533, 1539, 1540, 1548, 1550, 1551, 1552, 1553, 1554, 1555, 1556, 1559, 1567, 1569, 1570, 1571, 1572, 1574, 1577, 1582, 1590, 1592, 1595, 1596, 1605, 1609, 1610, 1611, 1612, 1613, 1614, 1615, 1616, 1617, 1618, 1620, 1626, 1627, 1628, 1632, 1633, 1634, 1635, 1637, 1638, 1639, 1640, 1652, 1653, 1656, 1660, 1661, 1662, 1664, 1665, 1666, 1669, 1670, 1671, 1672, 1673, 1677, 1678, 1680, 1681, 1685, 1687, 1695, 1696, 1697, 1703, 1704, 1706, 1707, 1708, 1709, 1712, 1714, 1715, 1717, 1720, 1722, 1725, 1727, 1729, 1730, 1732, 1733, 1737, 1738, 1743, 1746, 1748, 1751, 1752, 1753, 1754, 1755, 1758, 1759, 1760, 1761, 1762, 1763, 1764, 1765, 1769, 1771, 1773, 1775, 1776, 1777, 1781, 1785, 1788, 1790, 1791, 1792, 1793, 1794, 1795, 1796, 1797, 1798, 1800, 1805, 1806, 1809, 1810, 1815, 1816, 1818, 1828, 2160, 2161, 2162, 2163, 2164, 2165, 2166, 2167, 2168, 2169, 2170, 2171.**

La détection d'un mécanisme de résistance aux carbapénèmes ou aux céphalosporines, induit par l'expression de la protéine GES, est caractérisée par la détection d'au moins un peptide appartenant à la protéine GES et à ses différents variants de séquences **SEQ ID N° 2114 à SEQ ID N°2130.**
**SEQ ID N°2114:** 
MRFIHALLLAGIAHSAYASEKLTFKTDLEKLEREKAAQIGVAIVDPQGEIVAGHRMAQ
RFAMCSTFKFPLAALVFERIDSGTERGDRKLSYGPDMIVEWSPATERFLASGHMTV
LEAAQAAVQLSDNGATNLLLREIGGPAAMTQYFRKIGDSVSRLDRKEPEMGDNTPG
DLRDTTTPIAMARTVAKVLYGGALTSTSTHTIERWLIGNQTGDATLRAGFPKDWVVG
EKTGTCANGGRNDIGFFKAQERDYAVAVYTTAPKLSAVERDELVASVGQVITQLILS
TDK
**SEQ ID N°2115:** 
MRFIHALLLAGIAHSAYASEKLTFKTDLEKLEREKAAQIGVAIVDPQGEIVAGHRMAQ
RFAMCSTFKFPLAALVFERIDSGTERGDRKLSYGPDMIVKWSPATERFLASGHMTV
LEAAQAAVQLSDNGATNLLLREIGGPAAMTQYFRKIGDSVSRLDRKEPEMGDNTPG
 
DLRDTTTPIAMARTVAKVLYGGALTSTSTHTIERWLIGNQTGDATLRAGFPKDWVVG
EKTGTCANGGRNDIGFFKAQERDYAVAVYTTAPKLSAVERDELVASVGQVITQLILS
TDK
**SEQ ID N°2116:** 
MRFIHALLLAGIAHSAYASEKLTFKTDLEKLEREKAAQIGVAIVDPQGEIVAGHRMAQ
RFAMCSTFKFPLAALVFERIDSGTERGDRKLSYGPDMIVEWSPATERFLASGHMTV
LEAAQLAVQLSDNGATNLLLREIGGPAAMTQYFRKIGDSVSRLDRKEPEMGDNTPG
DLRDTTTPIAMARTVAKVLYGGALTSTSTHTIERWLIGNQTGDATLRAGFPKDWVVG
EKTGTCANGGRNDIGFFKAQERDYAVAVYTTAPKLSAVERDELVASVGQVITQLILS
TDK
**SEQ ID N°2117:** 
MRFIHALLLAGIAHSAYASEKLTFKTDLEKLEREKAAQIGVAIVDPQGEIVAGHRMAQ
RFAMCSTFKFPLAALVFERIDSGTERGDRKLSYGPDMIVEWSPATERFLASGHMTV
LEAAQAAVQLSDNGATNLLLREIGGPAAMTQYFRKIGDSVSRLDRKEPEMNDNTPG
DLRDTTTPIAMARTVAKVLYGGALTSTSTHTIERWLIGNQTGDATLRAGFPKDWVVG
EKTGTCANGGRNDIGFFKAQERDYAVAVYTTAPKLSAVERDELVASVGQVITQLILS
TDK
**SEQ ID N°2118:** 
MRFIHALLLAGIAHSAYASEKLTFKTDLEKLEREKAAQIGVAIVDPQGEIVAGHRMAQ
RFAMCSTFKFPLAALVFERIDSGTERGDRKLSYGPDMIVEWSPATERFLASGHMTV
LEAAQAAVQLSDNGATNLLLREIGGPAAMTQYFRKIGDSVSRLDRKEPEMSDNTPG
DLRDTTTPIAMARTVAKVLYGGALTSTSTHTIERWLIGNQTGDATLRAGFPKDWVVG
EKTGTCANGGRNDIGFFKAQERDYAVAVYTTAPKLSAVERDELVASVGQVITQLILS
TDK
**SEQ ID N°2119:** 
MRFIHALLLAGIAHSAYASEKLTFKTDLEKLEREKAAQIGVAIVDPQGEIVAGHRMAQ
RFAMCSTFKFPLAALVFERIDSGTERGDRKLSYGPDMIVKWSPATERFLASGHMTV
LEAAQAAVQLSDNGATNLLLREIGGPAAMTQYFRKIGDSVSRLDRKEPEMSDNTPG
DLRDTTTPIAMARTVAKVLYGGALTSTSTHTIERWLIGNQTGDATLRAGFPKDWVVG
EKTGTCANGGRNDIGFFKAQERDYAVAVYTTAPKLSAVERDELVASVGQVITQLILS
TDK
**SEQ ID N°2120:** 
MRFIHALLLAGIAHSAYASEKLTFKTDLEKLEREKAAQIGVAIVDPQGEIVAGHRMAQ
RFAMCSTFKFPLAALVFERIDSGTERGDRKLSYGPDMIVEWSPATERFLASGHMTV
LEAAQAAVQLSDNGATNLLLREIGGPAAMTQYFRKIGDSVSRLDRKEPEMGDNTPG
DLRDTTTPIAMARTVAKVLYGGALTSTSTHTIERWLIGNQTGDATLRAGFPKDWVVG
EKTGTCANGSRNDIGFFKAQERDYAVAVYTTAPKLSAVERDELVASVGQVITQLILS
TDK
**SEQ ID N°2121:** 
MRFIHALLLAGIAHSAYASEKLTFKTDLEKLEREKAAQIGVAIVDPQGEIVAGHRMAQ
RFAMCSTFKFPLAALVFERIDSGTERGDRKLSYGPDMIVEWSPATERFLASGHMTV
LEAAQAAVQLSDNGATNLLLREIGGPAAMTQYFRKIGDSVSRLDRKEPEMGDNTPG
DLRDTTTPIAMARTVAKVLYGGALTSTSTHTIERWLIGNQTGDATLRAGFPKDWVVG
EKTGTCANGARNDIGFFKAQERDYAVAVYTTAPKLSAVERDELVASVGQVITQLILS
TDK
**SEQ ID N°2122:** 
MRFIHALLLAGTAHSAYASEKLTFKTDLEKLEREKAAQIGVAIVDPQGEIVAGHRTAQ
RFAMCSTFKFPLAALVFERIDSGTERGDRKLSYGPDMIVEWSPATERFLASGHMTV
LEAAQAAVQLCDNGATNLLLREIGGPAAMTQYFRKIGDSVSRLDRKEPEMGDNTPG
DLRDTTTPIAMARTVAKVLYGGALTSTSTHTIERWLIGNQTGDATLRAGFPKDWVVG
 
EKTGTCANGGRNDIGFFKAQERDYAVAVYTTAPKLSAVERDELVASVGQVITQLILS
TDK
**SEQ ID N°2123:** 
MRFIHALLLAGIAHSAYASEKLTFKTDLEKLEREKAAQIGVAIVDPQGEIVAGHRMAQ
RFAMCSTFKFPLAALVFERIDSGTERGDRKLSYGPDMIVKWSPATERFLASGHMTV
LEAAQAAVQLSDNGATNLLLREIGGPAAMTQYFRKIGDSVSRLDRKEPEMNDNTPG
DLRDTTTPIAMARTVAKVLYGGALTSTSTHTIERWLIGNQTGDATLRAGFPKDWVVG
EKTGTCANGGRNDIGFFKAQERDYAVAVYTTAPKLSAVERDELVASVGQVITQLILS
TDK
**SEQ ID N°2124:** 
MRFIHALLLAGIAHSAYASEKLTFKTDLEKLEREKAAQIGVAIVDPQGEIVAGHRMAQ
RFAMCSTFKFPLAALVFERIDSGTERGDRKLSYGPDMIVEWSPATERFLASGHMTV
LEAAQAAVQLSDNGATNLLLREIGGPAAMTQYFRKIGDSVSRLDRKESEMSDNTPG
DLRDTTTPIAMARTVAKVLYGGALTSTSTHTIERWLIGNQTGDATLRAGFPKDWVVG
EKTGTCANGGRNDIGFFKAQERDYAVAVYTTAPKLSAVERDELVASVGQVITQLILS
TDK
**SEQ ID N°2125:** 
MRFIHALLLAGIAHSAYASEKLTFKTDLEKLEREKAAQIGVAIVDPQGEIVAGHRMAQ
RFAMCSTFKFPLAALVFERIDSGTERGDRKLSYGPDMIVEWSPATERFLASGHMTV
LEAAQAAVQLSDNGATNLLLREIGGPAAMTQYFRKIGDSVSRLDRKEPEMSDNTPG
DLRDTTTPIAMARTVAKVLYGGALTSTSTHTIERWLIGNQTGDATLRAGFPKDWVVG
EKTGTCANGARNDIGFFKAQERDYAVAVYTTAPKLSAVERDELVASVGQVITQLILS
TDK
**SEQ ID N°2126:** 
MRFIHALLLAGIAHSAYASEKLTFKTDLEKLEREKAAEIGVAIVDPQGEIVAGHRMAQ
RFAMCSTFKFPLAALVFERIDSGTERGDRKLSYGPDMIVEWSPATERFLASGHMTV
LEAAQAAVQLSDNGATNLLLREIGGPAAMTQYFRKIGDSVSRLDRKEPEMSDNTPG
DLRDTTTPIAMARTVAKVLYGGALTSTSTHTIERWLIGNQTGDATLRAGFPKDWVVG
EKTGTCANGGRNDIGFFKAQERDYAVAVYTTAPKLSAVERDELVASVGQVITQLILS
TDK
**SEQ ID N°2127:** 
MRFIHALLLAGIAHSAYASEKLTFKTDLEKLEREKAAQIGVAIVDPQGEIVAGHRMAQ
RFAMCSTFKFPLAALVFERIDSGTERGDRKLSYGPDMIVKWSPATERFLASGHMTV
LEAAQAAVQLSDNGATNLLLREIGGPAAMTQYFRKIGDSVSRLDRKEPEMGDNTPG
DLRDTTTPIAMARTVAKVLYGGALTSTSTHTIERWLIGNQTGDATLRAGFPKDWVVG
EKTGTCANGARNDIGFFKAQERDYAVAVYTTAPKLSAVERDELVASVGQVITQLILS
TDK
**SEQ ID N°2128:** 
MRFIHALLLAGIAHSAYASEKLTFKTDLEKLEREKAAQIGVAIVDPQGEIVAGHRTAQ
RFAMCSTFKFPLAALVFERIDSGTERGDRKLSYGPDMIVKWSPATERFLASGHMTV
LEAAQAAVQLSDNGATNLLLREIGGPAAMTQYFRKIGDSVSRLDRKEPEMGDNTPG
DLRDTTTPIAMARTVAKVLYGGALTSTSTHTIERWLIGNQTGDATLRAGFPKDWVVG
EKTGTCANGGRNDIGFFKAQERDYAVAVYTTAPKLSAVERDELVASVGQVITQLILS
TDK
**SEQ ID N°2129:** 
MRFIHALLLAGIAHSAYASEKLTFKTDLEKLEREKAAQIGVAIVDPQGEIVAGHRTAQ
RFAMCSTFKFPLAALVFERIDSGTERGDRKLSYGPDMIVKWSPATERFLASGHMTV
LEAAQAAVQLSDNGATNLLLREIGGPAAMTQYFRKIGDSVSRLDRKEPEMSDNTPG
DLRDTTTPIAMARTVAKVLYGGALTSTSTHTIERWLIGNQTGDATLRAGFPKDWVVG
 
EKTGTCANGGRNDIGFFKAQERDYAVAVYTTAPKLSAVERDELVASVGQVITQLILS
TDK
**SEQ ID N°2130:** 
MRFIHALLLAGIAHSAYASEKLTFKTDLEKLEREKAAQIGVAIVDPQGEIVAGHRMAQ
RFAMCSTFKFPLAALVFERIDSGTERGDRKLSYGPDMIVEWSPATERFLASGHMTV
LEAAQAAVQLSDNGATNLLLREIGGPAAMTQYFRKIGDSVSRLDRKEPEMGDNTPG
DLRDTTTPIAMARTVAKVLYGGALTSTSTHTIERWLIGNQTGDATLRAGFPKDWVVG
EKTGACANGARNDIGFFKAQERDYAVAVYTTAPKLSAVERDELVASVGQVITQLILS
TDK
 lesdits peptides étant choisis, de préférence, parmi les peptides de séquence **SEQ ID N° 2131** à **SEQ ID N° 2159** tels que définis ci-après :

| **Peptide SEQ ID N°** | **Séquence d'acides aminés** | **Position du peptide dans la ou les protéines GES** | **Intérêt clinique** |
|---|---|---|---|
| **SEQ ID N°2131** | AAEIGVAIVDPQGEIVAGHR | 36-55 pour les protéines de SEQ N°2126 | **carba** |
| **SEQ ID N°2132** | AAQIGVAIVDPQGEIVAGHR | 36-55 pour les protéines de SEQ N°2114, 2115, 2116,2117,2118,2119,2120,2121,2122, 2123,2124,2125,2127,2128,2129,2130 | **BLSE** |
| **SEQ ID N°2133** | AGFPK | 218-222 pour les protéines de SEQ N°2114, 2115,2116,2117,2118,2119,2120,2121, 2122,2123,2124,2125,2126,2127,2128, 2129,2130 | **BLSE** |
| **SEQ ID N°2134** | DTTTPIAMAR | 174-183 pour les protéines de SEQ N°2114, 2115,2116,2117,2118,2119,2120,2121, 2122,2123,2124,2125,2126,2127,2128, 2129,2130 | **BLSE** |
| **SEQ ID N°2135** | DWVVGEK | 223-229 pour les protéines de SEQ N°2114, 2115,2116,2117,2118,2119,2120,2121, 2122,2123,2124,2125,2126,2127,2128, 2129,2130 | **BLSE** |
| **SEQ ID N°2136** | DYAVAVYTTAPK | 250-261 pour les protéines de SEQ N°2114, 2115,2116,2117,2118,2119,2120,2121, 2122,2123,2124,2125,2126,2127,2128, 2129,2130 | **BLSE** |
| **SEQ ID N°2137** | EIGGPAAMTQYFR | 136-148 pour les protéines de SEQ N°2114, 2115,2116,2117,2118,2119,2120,2121, 2122,2123,2124,2125,2126,2127,2128, 2129,2130 | **BLSE** |
| **SEQ ID N°2138** | EPEMGDNTPGDLR | 161-173 pour les protéines de SEQ N°2114, 2115,2116,2120,2121,2122,2127,2128,2130 | **BLSE** |
| **SEQ ID N°2139** | EPEMNDNTPGDLR | 161-173 pour les protéines de SEQ N°2117, 2123 | **carba** |
| **SEQ ID N°2140** | EPEMSDNTPGDLR | 161-173 pour les protéines de SEQ N°2118, 2119,2125,2126,2129 | **carba** |
| **SEQ ID N°2141** | ESEMSDNTPGDLR | 161-173 pour les protéines de SEQ N°2124 | **carba** |
| **SEQ ID N°2142** | FAMCSTFK | 60-67 pour les protéines de SEQ N°2114, 2115, 2116,2117,2118,2119,2120,2121,2122, 2123,2124,2125,2126,2127,2128,2129,2130 | **BLSE** |
| **SEQ ID N°2143** | FIHALLLAGIAHSAYASEK | 3-21 pour les protéines de SEQ N°2114, 2115, 2116,2117,2118,2119,2120,2121,2123, 2124,2125,2126,2127,2128,2129,2130 | **BLSE** |
| **SEQ ID N°2144** | FIHALLLAGTAHSAYASEK | 3-21 pour les protéines de SEQ N°2122 | **carba** |
| **SEQ ID N°2145** | FPLAALVFER | 68-77 pour les protéines de SEQ N°2114, 2115, 2116, 2117, 2118, 2119, 2120, 2121, 2122, 2123, 2124, 2125, 2126, 2127, 2128, 2129, 2130 | **BLSE** |
| **SEQ ID N°2146** | IDSGTER | 78-84 pour les protéines de SEQ N°2114, 2115, 2116, 2117, 2118, 2119, 2120, 2121, 2122, 2123, 2124, 2125, 2126, 2127, 2128, 2129, 2130 | **BLSE** |
| **SEQ ID N°2147** | IGDSVSR | 150-156 pour les protéines de SEQ N°2114, 2115, 2116, 2117, 2118, 2119, 2120, 2121, 2122, 2123, 2124, 2125, 2126, 2127, 2128, 2129, 2130 | **BLSE** |
| **SEQ ID N°2148** | LSAVER | 262-267 pour les protéines de SEQ N°2114, 2115, 2116, 2117, 2118, 2119, 2120, 2121, 2122, 2123, 2124, 2125, 2126, 2127, 2128, 2129,2130 | **BLSE** |
| **SEQ ID N°2149** | LSYGPDMIVEWSPATER | 89-105 pour les protéines de SEQ N°2114, 2116,2117,2118,2120,2121,2122,2124, 2125, 2126, 2130 | **BLSE** |
| **SEQ ID N°2150** | LSYGPDMIVK | 89-98 pour les protéines de SEQ N°2115, 2119, 2123, 2127, 2128, 2129 | **carba** |
| **SEQ ID N°2151** | NDIGFFK | 239-245 pour les protéines de SEQ N°2114, 2115,2116,2117,2118,2119,2120,2121, 2122,2123,2124,2125,2126,2127,2128, 2129,2130 | **BLSE** |
| **SEQ ID N°2152** | TDLEK | 26-30 pour les protéines de SEQ N°2114, 2115, 2116,2117,2118,2119,2120,2121,2122, 2123,2124,2125,2126,2127,2128,2129,2130 | **BLSE** |
| **SEQ ID N°2153** | TGACANGAR | 230-238 pour les protéines de SEQ N°2130 | **carba** |
| **SEQ ID N°2154** | TGTCANGAR | 230-238 pour les protéines de SEQ N°2121, 2125,2127 | **carba** |
| **SEQ ID N°2155** | TGTCANGGR | 230-238 pour les protéines de SEQ N°2114, 2115,2116,2117,2118,2119,2122,2123, 2124,2126,2128,2129 | **BLSE** |
| **SEQ ID N°2156** | TGTCANGSR | 230-238 pour les protéines de SEQ N°2120 | **carba** |
| **SEQ ID N°2157** | VLYGGALTSTSTHTIER | 188-204 pour les protéines de SEQ N°2114, 2115,2116,2117,2118,2119,2120,2121, 2122,2123,2124,2125,2126,2127,2128, 2129,2130 | **BLSE** |
| **SEQ ID N°2158** | WLIGNQTGDATLR | 205-217 pour les protéines de SEQ N°2114, 2115,2116,2117,2118,2119,2120,2121, 2122,2123,2124,2125,2126,2127,2128, 2129,2130 | **BLSE** |
| **SEQ ID N°2159** | WSPATER | 99-105 pour les protéines de SEQ N°2114, 2115, 2116, 2117, 2118, 2119, 2120, 2121, 2122,2123,2124,2125,2126,2127,2128, 2129,2130 | **carba** |

Dans la colonne intérêt clinique, les mentions BLSE et carba correspondent aux activités béta-lactamases GES que permet de détecter le peptide correspondant. Ainsi la détection d'un peptide carba signera la présence d'une béta-lactamase de type carbapénèmase capable d'hydrolyser les carbapénèmes, les pénicillines, les céphalosporines de première génération telles que la céphaloridine et la céphalotine ainsi qu'au moins un antibiotique de la classe des oxyimino-beta-lactames tels que le cefotaxime, le ceftazidime ou les monobactames tels que l'aztreoname.

Si aucun peptide noté carba n'est détecté, la détection d'un peptide noté BLSE signera la présence d'une béta-lactamase avec un spectre étendu (BLSE) capable d'hydrolyser les pénicillines, les céphalosporines de première génération telles que la céphaloridine et la céphalotine ainsi qu'au moins un antibiotique de la classe des oxyimino-beta-lactames tels que le cefotaxime, le ceftazidime ou les monobactames tels que l'aztreoname.

La détection d'un mécanisme de résistance aux céphalosporinases avec un spectre étendu (BLSE) induit par la protéine GES, est ainsi caractérisée par la détection d'au moins un peptide marqueur de résistance, de type BLSE, choisi parmi les séquences **SEQ ID N° 2131** à **SEQ ID N°2159**

Certaines séquences peptidiques peuvent être communes à plusieurs mécanismes de résistance. Ainsi les séquences suivantes sont identiques :
SEQ ID N°834 et SEQ ID N°978
SEQ ID N°833 et SEQ ID N°977
SEQ ID N°832 et SEQ ID N°976
SEQ ID N°831 et SEQ ID N°975
SEQ ID N°826 et SEQ ID N°974
SEQ ID N°825 et SEQ ID N°973
SEQ ID N°824 et SEQ ID N°972
SEQ ID N°823 et SEQ ID N°971
SEQ ID N°822 et SEQ ID N°970
SEQ ID N°817 et SEQ ID N°969
SEQ ID N°813 et SEQ ID N°967
SEQ ID N°802 et SEQ ID N°964
SEQ ID N°798 et SEQ ID N°963
SEQ ID N°666 et SEQ ID N°895
SEQ ID N°350 et SEQ ID N°1035
SEQ ID N°349 et SEQ ID N°1031
SEQ ID N°347 et SEQ ID N°1030
SEQ ID N°346 et SEQ ID N°1029
SEQ ID N°345 et SEQ ID N°1028
SEQ ID N°343 et SEQ ID N°1037 et SEQ ID N°725
SEQ ID N°342 et SEQ ID N°1026
SEQ ID N°341 et SEQ ID N°1025
SEQ ID N°340 et SEQ ID N°1024
SEQ ID N°339 et SEQ ID N°1023
SEQ ID N°338 et SEQ ID N°1020
SEQ ID N°337 et SEQ ID N°1036 et SEQ ID N°719
SEQ ID N°337 et SEQ ID N°1036
SEQ ID N°336 et SEQ ID N°841 et SEQ ID N°981
SEQ ID N°323 et SEQ ID N°840 et SEQ ID N°980
SEQ ID N°315 et SEQ ID N°839 et SEQ ID N°948 et SEQ ID N°979
SEQ ID N°240 et SEQ ID N°679
SEQ ID N°184 et SEQ ID N°626
SEQ ID N°182 et SEQ ID N°625
SEQ ID N°1034 et SEQ ID N°1999
SEQ ID N°1032 et SEQ ID N°1988
SEQ ID N°1027 et SEQ ID N°1970
SEQ ID N°1022 et SEQ ID N°1952
SEQ ID N°1021 et SEQ ID N°1937
SEQ ID N°1019 et SEQ ID N°1933
SEQ ID N°809 et SEQ ID N°966
SEQ ID N°1033 et SEQ ID N°1989

Dans tous les cas, les séquences ci-dessus signent l'expression d'un mécanisme de résistance aux céphalosporines.

Le procédé de l'invention et ses avantages ressortiront de la suite de la présente description qui présente plusieurs exemples, non limitatifs, de mise en oeuvre dudit procédé.

### Exemple 1 : Identification de microorganismes à partir d'un échantillon par profil biochimique

### 1. Mise-en en culture de l'échantillon sur milieu de culture

Les milieux de culture optimaux et les conditions de culture optimales sont différents selon les espèces de microorganisme. Par défaut l'échantillon est ensemencé sur différents milieux :
o gélose Columbia au sang de mouton (réf bioMérieux 43041) pendant 18 à 24 h à 35°C, en atmosphère aérobie ou anaérobie ;
o gélose TSA (référence bioMérieux 43011) pendant 18 à 24 h à 37°C.

### 2. Identification des microorganisme

L'identification est mise en oeuvre comme suit :
1. Sélection de colonies isolées
2. En respectant les conditions d'asepsie, transfert de 3,0 mL de solution saline stérile aqueuse (à 0,45-0,50 % de NaCl, de pH 4,5 à 7,0) dans un tube à essai en plastique transparent (polystyrène)
3. A l'aide d'un bâtonnet ou d'un écouvillon stérile, transfert d'un nombre suffisant de colonies identiques dans le tube de solution saline préparé à l'étape 2 et ajustement de la suspension bactérienne entre 0,50 et 0,63 McFarland avec un DENSICHEK étalonné du VITEK®
4. Positionnement du tube de suspension bactérienne et d'une carte d'identification VITEK® sur une cassette VITEK®
5. Chargement de la cassette dans l'instrument VITEK®
6. Les opérations de remplissage, scellage, incubation et lecture sont automatiques
7. Acquisition d'un profil biochimique
8. Identification avec le système VITEK® réalisée par comparaison à des profils biochimiques de souches connues

### Exemple 2 : Préparation d'un échantillon primaire urinaire par enrichissement en microorganismes:

Le protocole suivant est mis en oeuvre en 16 étapes (les étapes 5 à 12 sont optionnelles et pourraient être omises si l'échantillon enrichi est ultérieurement traité selon les exemples 4 et suivants) :
1. Centrifugation de 5 mL d'urine contaminée, à 2000g pendant 30 secondes
2. Récupération du surnageant
3. Centrifugation à 15000g pendant 5 minutes
4. Elimination du surnageant
5. Lavage du culot avec 3 mL d'eau distillée par remise en suspension
6. Centrifugation à 15000g pendant 5 minutes
7. Elimination du surnageant
8. Mettre le culot en présence de solvant (8 volumes acétone pour 1 volume de méthanol) en dilution au 1/10
9. Laisser 1 heure à -20°C
10. Centrifugation à 15000g pendant 5 minutes
11. Elimination du surnageant
12. Mettre le culot en présence de solvant (8 volumes acétone pour 1 volume de méthanol) en dilution au 1/10
13. Laisser 1 heure à -20°C
14. Centrifugation à 15000g pendant 5 minutes
15. Elimination du surnageant
16. Le culot constitue l'échantillon enrichi en microorganisme

### Exemple 3 : Identification de microorganismes à partir d'un échantillon par MALDI-TOF

L'identification est mise en oeuvre comme suit :
1. Transfert à l'aide d'une oese 1µl, d'une portion de colonie de microorganisme obtenue selon l'exemple 1, ou d'un échantillon enrichi selon l'exemple 2, et dépôt uniforme sur une plaque pour spectrométrie de masse par MALDI-TOF
2. Recouvrement du dépôt avec 1 µl de matrice. La matrice utilisée est une solution saturée d'HCCA (acide alpha-cyano-4-hydroxycinnamique) en solvant organique (50% d'acétonitrile et 2,5% d'acide trifluoroacétique)
3. Séchage à température ambiante
4. Introduction de la plaque dans le spectromètre de masse
5. Acquisition d'un spectre de masse
6. Comparaison du spectre obtenu avec les spectres contenus dans une base de connaissance
7. Identification du microorganisme par comparaison des pics obtenus à ceux de la base de connaissance

### Exemple 4 : Identification de microorganismes à partir d'un échantillon par ESI-MS

L'identification est mise en oeuvre comme suit :
1. Prélèvement d'une colonie de microorganisme, obtenue selon l'exemple 1, ou d'un échantillon enrichi selon l'exemple 2, et mise en suspension dans 100µl d'eau déminéralisée.
2. Centrifugation à 3000g pendant 5 minutes.
3. Elimination du surnageant.
4. Remise en suspension dans 100µl d'eau déminéralisée.
5. Centrifugation à 3000g pendant 5 minutes.
6. Elimination du surnageant.
7. Remise en suspension dans 100µl d'un mélange Acétonitrile, eau déminéralisée, acide formique (50/50/0.1 %).
8. Filtration avec un filtre de porosité 0.45 µm.
9. Injection dans un spectromètre de masse en mode MS simple.
10. Acquisition d'un spectre de masse.
11. Comparaison du spectre obtenu avec les spectres contenus dans une base de connaissance.
12. Identification du microorganisme par référence à des spectres de référence.

### Exemple 5 : Obtention de protéines digérées à partir de microorganismes

Classiquement le protocole suivant est mis en oeuvre en 17 étapes :
1. Prélèvement d'une colonie de microorganisme, obtenue selon l'exemple 1, ou d'un échantillon enrichi selon l'exemple 2, et mise en suspension dans 10 à 100µl d'une solution d'hydrochlorure de guanidine 6M, 50 mM Tris-HCl, pH=8,0.
2. Ajout de dithiothréitol (DTT) pour obtenir une concentration finale de 5mM.
3. Réduction pendant 20 minutes à 95°C dans un bain-marie.
4. Refroidissement des tubes à température ambiante.
5. Ajout d'iodoacétamide pour obtenir une concentration finale de 12.5 mM.
6. Alkylation pendant 40 minutes à température ambiante et à l'obscurité.
7. Dilution d'un facteur 6 avec une solution de NH₄HCO₃ 50 mM, pH=8.0 pour obtenir une concentration finale en hydrochlorure de guanidine de 1M.
8. Ajout de 1 µg de trypsine.
9. Digestion à 37°C pendant 6 heures jusqu'à une nuit.
10. Ajout d'acide formique jusqu'à un pH inférieur à 4 pour stopper la réaction.
11. Le volume d'échantillon est complété à 1mL avec eau/acide formique 0,5% (v/v)
12. Equilibrage des colonnes HLB Oasis Waters avec 1ml de méthanol puis 1 ml H₂O/acide formique 0,1 % (v/v)
13. Dépôt de l'échantillon qui s'écoule par gravité
14. Lavage avec 1 ml H₂O/acide formique 0,1% (v/v)
15. Elution avec 1ml d'un mélange 80% de méthanol et 20% d'eau/acide formique 0,1% (v/v)
16. L'éluat est évaporé avec un évaporateur de type SpeedVac® SPD2010 (Thermo Electron Corporation, Waltham, Massachusetts, Etats-Unis d'Amérique), durant 2 heures, afin d'obtenir un volume d'environ 100µl.
17. L'éluat est ensuite repris dans une solution eau/acide formique 0,5% (v/v) quantité suffisante pour (QSP) 250µl

### Exemple 6 : Identification d'une résistance aux béta-lactamines de type TEM:

Les échantillons Ech10 à Ech61 sont identifiés selon l'une des méthodes décrites dans les exemples 1, 3 ou 4. L'identification des espèces est reportée dans le **TABLEAU 1.**

**TABLEAU 1 :**

| Noms | Espèces |
|---|---|
| Ech10 | *P. mirabilis* |
| Ech11 | *E. coli* |
| Ech12 | *E. aerogenes* |
| Ech13 | *E. coli* |
| Ech14 | *E. coli* |
| Ech15 | *E. coli* |
| Ech16 | *S*. *marcescens* |
| Ech17 | *E. coli* |
| Ech18 | *E. coli* |
| Ech19 | *P. mirabilis* |
| Ech20 | C. *freundii* |
| Ech21 | *E. coli* |
| Ech22 | *P. mirabilis* |
| Ech23 | *E. coli* |
| Ech24 | *K. pneumoniae* |
| Ech25 | *E. coli* |
| Ech26 | *K. oxytoca* |
| Ech27 | *P. mirabilis* |
| Ech28 | *E. coli* |
| Ech29 | *P. mirabilis* |
| Ech30 | *P. rettgeri* |
| Ech31 | *P. stuartii* |
| Ech32 | S. *Derby* |
| Ech33 | *E. coli* |
| Ech34 | *E. coli* |
| Ech35 | *E. coli* |
| Ech36 | *E. coli* |
| Ech37 | *E. coli* |
| Ech38 | *E. coli* |
| Ech39 | *E. coli* |
| Ech40 | *E. coli* |
| Ech41 | *E. coli* |
| Ech42 | *E. coli* |
| Ech43 | *E. coli* |
| Ech44 | *E. coli* |
| Ech45 | *K. pneumoniae* |
| Ech46 | *E. coli* |
| Ech47 | *K. pneumoniae* |
| Ech48 | *P. mirabilis* |
| Ech49 | *P. mirabilis* |
| Ech50 | *P. mirabilis* |
| Ech51 | *P. mirabilis* |
| Ech52 | *P. mirabilis* |
| Ech53 | *K. pneumoniae* |
| Ech54 | *P. mirabilis* |
| Ech55 | *E. coli* |
| Ech56 | *E. coli* |
| Ech57 | *P. mirabilis* |
| Ech58 | *E. aerogenes* |
| Ech59 | *E. aerogenes* |
| Ech60 | *E. coli* |
| Ech61 | *E. coli* |

Les échantillons Ech10 à Ech61 correspondent à une espèce pouvant comporter un mécanisme de résistance de type TEM (Enterobacteriaceae...). La méthode suivante est alors mise en oeuvre pour rechercher un tel mécanisme.

Chaque échantillon est traité selon l'exemple 5, puis un volume de 50µl de protéines digérées est injecté et analysé selon les conditions suivantes :
- Chaîne chromatographique Dionex Ultimate 3000 de la société Dionex Corporation (Sunnyvale, Etats Unis d'Amerique).
- Colonne Waters BEH130 C18, 2,1mm de diamètre interne, 100mm de long, taille de particule de 3,5µm (Waters, Saint-Quentin En Yvelines, France).
- Solvant A : H₂O + 0,1% acide formique.
- Solvant B : ACN + 0,1 % acide formique.

Gradient HPLC défini dans le **TABLEAU 2** ci-après.

**TABLEAU 2 :**

| Temps (min) | Débit (µl) | Solvant A (%) | Solvant B (%) |
|---|---|---|---|
| 0 | 300 | 98 | 2 |
| 3 | 300 | 98 | 2 |
| 34 | 300 | 54.6 | 45.4 |
| 35 | 300 | 0 | 100 |
| 55 | 300 | 0 | 100 |
| 55.1 | 300 | 98 | 2 |
| 74 | 300 | 98 | 2 |

- L'éluat en sortie de colonne chromatographique est directement injecté dans la source d'ionisation du spectromètre de masse de type QTRAP® 5500 de la société Applied Biosystems (Foster City, Etats Unis d'Amérique).
- Les peptides, issus de la digestion des protéines du microorganisme, sont analysés par le spectromètre de masse en mode MRM. Seuls les peptides indiqués dans le **TABLEAU 3** sont détectés. Pour cela, le ou les fragments des peptides indiqués dans le **TABLEAU 3** sont détectés. L'état de charge du précuseur, ainsi que l'existence éventuelle d'une méthionine oxydée sont également indiqués dans le TABLEAU 3.

**TABLEAU 3 :**

| Transition numéro | Peptide | Oxydation méthionine | Ion fragment | Etat de charge du précurseur | Intérêt clinique |
|---|---|---|---|---|---|
| 1 | DAENQLGAR | non | y4 monochargé | 2 | TEM |
| 2 | DAENQLGAR | non | y6 monochargé | 2 | TEM |
| 3 | DAENQLGAR | non | y7 monochargé | 2 | TEM |
| 4 | ELTAFLHNIGDHVTR | non | y4 monochargé | 2 | TEM |
| 5 | ELTAFLHNIGDHVTR | non | y8 monochargé | 2 | TEM |
| 6 | ELTAFLHNIGDHVTR | non | y9 monochargé | 2 | TEM |
| 7 | ELTAFLHNMGDNVTR | non | y7 monochargé | 2 | 2b |
| 8 | ELTAFLHNMGDNVTR | non | y8 monochargé | 2 | 2b |
| 9 | ELTAFLHNMGDNVTR | non | y9 monochargé | 2 | 2b |
| 10 | FPMISTFK | oui | y5 monochargé | 2 | 2br |
| 11 | FPMISTFK | oui | y6 monochargé | 2 | 2br |
| 12 | FPMISTFK | oui | y7 monochargé | 2 | 2br |
| 13 | FPMISTFK | non | y5 monochargé | 2 | 2br |
| 14 | FPMISTFK | non | y6 monochargé | 2 | 2br |
| 15 | FPMISTFK | non | y7 monochargé | 2 | 2br |
| 16 | GEPELNEAIPNDER | non | y12 dichargé | 2 | 2be |
| 17 | GEPELNEAIPNDER | non | y5 monochargé | 2 | 2be |
| 18 | GEPELNEAIPNDER | non | y7 monochargé | 2 | 2be |
| 19 | GSLGIIAALGPDGKPSR | non | y10 monochargé | 2 | 2br |
| 20 | GSLGIIAALGPDGKPSR | non | y8 monochargé | 2 | 2br |
| 21 | GSLGIIAALGPDGKPSR | non | y9 monochargé | 2 | 2br |
| 22 | IHYSQNDLVEYSPVTEK | non | y6 monochargé | 3 | TEM |
| 23 | IHYSQNDLVEYSPVTEK | non | y7 monochargé | 3 | TEM |
| 24 | IHYSQNDLVEYSPVTEK | non | y8 monochargé | 3 | TEM |
| 25 | ILESFRPEER | non | b6 monochargé | 2 | TEM |
| 26 | ILESFRPEER | non | b8 monochargé | 2 | TEM |
| 27 | ILESFRPEER | non | y7 dichargé | 2 | TEM |
| 28 | IVVIYTTGGQATMDER | oui | y7 monochargé | 2 | 2be |
| 29 | IVVIYTTGGQATMDER | oui | y8 monochargé | 2 | 2be |
| 30 | IVVIYTTGGQATMDER | oui | y9 monochargé | 2 | 2be |
| 31 | IVVIYTTGGQATMDER | non | y6 monochargé | 2 | 2be |
| 32 | IVVIYTTGGQATMDER | non | y8 monochargé | 2 | 2be |
| 33 | IVVIYTTGGQATMDER | non | y9 monochargé | 2 | 2be |
| 34 | LDSWEPELNEAIPNDER | non | y5 monochargé | 3 | 2be |
| 35 | LDSWEPELNEAIPNDER | non | y6 monochargé | 3 | 2be |
| 36 | LDSWEPELNEAIPNDER | non | y7 monochargé | 3 | 2be |
| 37 | QIAEICASLIK | non | y6 monochargé | 2 | TEM |
| 38 | QIAEICASLIK | non | y7 monochargé | 2 | TEM |
| 39 | QIAEICASLIK | non | y8 monochargé | 2 | TEM |
| 40 | SGANER | non | y3 monochargé | 2 | TEM |
| 41 | SGANER | non | y4 monochargé | 2 | TEM |
| 42 | SGANER | non | y5 monochargé | 2 | TEM |
| 43 | SGGSER | non | y3 monochargé | 2 | 2be |
| 44 | SGGSER | non | y4 monochargé | 2 | 2be |
| 45 | SGGSER | non | y5 monochargé | 2 | 2be |
| 46 | VDAGQEQLDR | non | y7 monochargé | 2 | TEM |
| 47 | VDAGQEQLDR | non | y8 monochargé | 2 | TEM |
| 48 | VDAGQEQLDR | non | y9 monochargé | 2 | TEM |
| 49 | VKPAEDK | non | y4 monochargé | 2 | 2be |
| 50 | VKPAEDK | non | y5 monochargé | 2 | 2be |
| 51 | VKPAEDK | non | y6 monochargé | 2 | 2be |
| 52 | WEPELNEAIPIDER | non | y12 dichargé | 2 | 2be |
| 53 | WEPELNEAIPIDER | non | y5 monochargé | 2 | 2be |
| 54 | WEPELNEAIPIDER | non | y7 monochargé | 2 | 2be |
| 55 | DTTMPAAMATK | oui | y7 monochargé | 2 | TEM |
| 56 | DTTMPAAMATK | oui | y8 monochargé | 2 | TEM |
| 57 | DTTMPAAMATK | oui | y9 monochargé | 2 | TEM |
| 58 | DTTMPAAMATK | non | y7 monochargé | 2 | TEM |
| 59 | DTTMPAAMATK | non | y8 monochargé | 2 | TEM |
| 60 | DTTMPAAMATK | non | y9 monochargé | 2 | TEM |
| 61 | ELTAFLHNMGDHVTR | non | y13 dichargé | 2 | TEM |
| 62 | ELTAFLHNMGDHVTR | non | y4 monochargé | 2 | TEM |
| 63 | ELTAFLHNMGDHVTR | non | y8 monochargé | 2 | TEM |
| 64 | EPELNEAIPNDER | non | y5 monochargé | 2 | TEM |
| 65 | EPELNEAIPNDER | non | y7 monochargé | 2 | TEM |
| 66 | EPELNEAIPNDER | non | y8 monochargé | 2 | TEM |
| 67 | FPMMSTFK | oui | y6 monochargé | 2 | TEM |
| 68 | FPMMSTFK | oui | y7 monochargé | 2 | TEM |
| 69 | FPMMSTFK | oui | y7 dichargé | 2 | TEM |
| 70 | FPMMSTFK | non | y6 monochargé | 2 | TEM |
| 71 | FPMMSTFK | non | y7 monochargé | 2 | TEM |
| 72 | FPMMSTFK | non | y7 dichargé | 2 | TEM |
| 73 | GIIAALGPDGKPSR | non | y7 monochargé | 2 | TEM |
| 74 | GIIAALGPDGKPSR | non | y8 monochargé | 2 | TEM |
| 75 | GIIAALGPDGKPSR | non | y9 monochargé | 2 | TEM |
| 76 | IDAGQEQLGR | non | y7 monochargé | 2 | TEM |
| 77 | IDAGQEQLGR | non | y8 monochargé | 2 | TEM |
| 78 | IDAGQEQLGR | non | y9 monochargé | 2 | TEM |
| 79 | IHYSQNDLVK | non | y7 monochargé | 2 | 2be |
| 80 | IHYSQNDLVK | non | y8 monochargé | 2 | 2be |
| 81 | IHYSQNDLVK | non | y9 dichargé | 2 | 2be |
| 82 | IHYSQSDVVEYSPVTEK | non | y16 dichargé | 2 | TEM |
| 83 | IHYSQSDVVEYSPVTEK | non | y5 monochargé | 2 | TEM |
| 84 | IHYSQSDVVEYSPVTEK | non | y6 monochargé | 2 | TEM |
| 85 | IVVIYMTGGQATMDER | non | y6 monochargé | 2 | 2be |
| 86 | IVVIYMTGGQATMDER | non | y8 monochargé | 2 | 2be |
| 87 | IVVIYMTGGQATMDER | non | y9 monochargé | 2 | 2be |
| 88 | LDCWEPELNEAIPNDER | non | y5 monochargé | 3 | 2be |
| 89 | LDCWEPELNEAIPNDER | non | y6 monochargé | 3 | 2be |
| 90 | LDCWEPELNEAIPNDER | non | y7 monochargé | 3 | 2be |
| 91 | MSIQHFR | oui | y4 monochargé | 2 | TEM |
| 92 | MSIQHFR | oui | y5 monochargé | 2 | TEM |
| 93 | MSIQHFR | oui | y6 monochargé | 2 | TEM |
| 94 | MSIQHFR | non | y4 monochargé | 2 | TEM |
| 95 | MSIQHFR | non | y5 monochargé | 2 | TEM |
| 96 | MSIQHFR | non | y6 monochargé | 2 | TEM |
| 97 | QQLIDWMEADK | non | y5 monochargé | 2 | TEM |
| 98 | QQLIDWMEADK | non | y6 monochargé | 2 | TEM |
| 99 | QQLIDWMEADK | non | y7 monochargé | 2 | TEM |
| 100 | SGASER | non | y3 monochargé | 2 | 2be |
| 101 | SGASER | non | y4 monochargé | 2 | 2be |
| 102 | SGASER | non | y5 monochargé | 2 | 2be |
| 103 | VALIPFLAAFCLPVFAHPETLVK | non | y11 dichargé | 3 | 2ber |
| 104 | VALIPFLAAFCLPVFAHPETLVK | non | y6 monochargé | 3 | 2ber |
| 105 | VALIPFLAAFCLPVFAHPETLVK | non | y8 monochargé | 3 | 2ber |
| 106 | VGYIELDLNSGK | non | y7 monochargé | 2 | TEM |
| 107 | VGYIELDLNSGK | non | y8 monochargé | 2 | TEM |
| 108 | VGYIELDLNSGK | non | y9 monochargé | 2 | TEM |
| 109 | VLLCGAELSR | non | y6 monochargé | 2 | TEM |
| 110 | VLLCGAELSR | non | y7 monochargé | 2 | TEM |
| 111 | VLLCGAELSR | non | y8 monochargé | 2 | TEM |
| 112 | DAEDQLGAR | non | y5 monochargé | 2 | TEM |
| 113 | DAEDQLGAR | non | y6 monochargé | 2 | TEM |
| 114 | DAEDQLGAR | non | y7 monochargé | 2 | TEM |
| 115 | ETTTPAAMATTLR | oui | y7 monochargé | 2 | 2be |
| 116 | ETTTPAAMATTLR | oui | y9 monochargé | 2 | 2be |
| 117 | ETTTPAAMATTLR | oui | y9 dichargé | 2 | 2be |
| 118 | ETTTPAAMATTLR | non | y7 monochargé | 2 | 2be |
| 119 | ETTTPAAMATTLR | non | y9 monochargé | 2 | 2be |
| 120 | ETTTPAAMATTLR | non | y9 dichargé | 2 | 2be |
| 121 | FPMVSTFK | oui | y6 monochargé | 2 | TEM |
| 122 | FPMVSTFK | oui | y7 monochargé | 2 | TEM |
| 123 | FPMVSTFK | oui | y7 dichargé | 2 | TEM |
| 124 | FPMVSTFK | non | y6 monochargé | 2 | TEM |
| 125 | FPMVSTFK | non | y7 monochargé | 2 | TEM |
| 126 | FPMVSTFK | non | y7 dichargé | 2 | TEM |
| 127 | GSGGIIAALGPDGKPSR | non | y7 monochargé | 2 | 2br |
| 128 | GSGGIIAALGPDGKPSR | non | y8 monochargé | 2 | 2br |
| 129 | GSGGIIAALGPDGKPSR | non | y9 monochargé | 2 | 2br |
| 130 | HLTDGMTVR | oui | y5 monochargé | 2 | TEM |
| 131 | HLTDGMTVR | oui | y7 monochargé | 2 | TEM |
| 132 | HLTDGMTVR | oui | y8 monochargé | 2 | TEM |
| 133 | HLTDGMTVR | non | y4 monochargé | 2 | TEM |
| 134 | HLTDGMTVR | non | y7 monochargé | 2 | TEM |
| 135 | HLTDGMTVR | non | y8 monochargé | 2 | TEM |
| 136 | IVIIYTTGSQATMDER | non | b4 monochargé | 2 | 2br |
| 137 | IVIIYTTGSQATMDER | non | y2 monochargé | 2 | 2br |
| 138 | IVIIYTTGSQATMDER | non | y9 monochargé | 2 | 2br |
| 139 | IWIYTTGSQATMDEQNR | oui | y5 monochargé | 3 | 2br |
| 140 | IVVIYTTGSQATMDEQNR | oui | y6 monochargé | 3 | 2br |
| 141 | IVVIYTTGSQATMDEQNR | oui | y7 monochargé | 3 | 2br |
| 142 | IVVIYTTGSQATMDEQNR | non | y5 monochargé | 3 | 2br |
| 143 | IVVIYTTGSQATMDEQNR | non | y6 monochargé | 3 | 2br |
| 144 | IVVIYTTGSQATMDEQNR | non | y7 monochargé | 3 | 2br |
| 145 | LDHWEPELNEAVPNDER | non | y5 monochargé | 3 | 2be |
| 146 | LDHWEPELNEAVPNDER | non | y6 monochargé | 3 | 2be |
| 147 | LDHWEPELNEAVPNDER | non | y7 monochargé | 3 | 2be |
| 148 | LLTGELLTLASQQQLIDWMEADK | oui | b8 monochargé | 3 | TEM |
| 149 | LLTGELLTLASQQQLIDWMEADK | oui | y6 monochargé | 3 | TEM |
| 150 | LLTGELLTLASQQQLIDWMEADK | oui | y7 monochargé | 3 | TEM |
| 151 | LLTGELLTLASQQQLIDWMEADK | non | b4 monochargé | 3 | TEM |
| 152 | LLTGELLTLASQQQLIDWMEADK | non | y6 monochargé | 3 | TEM |
| 153 | LLTGELLTLASQQQLIDWMEADK | non | y7 monochargé | 3 | TEM |
| 154 | QIAEIGASLIK | non | y7 monochargé | 2 | TEM |
| 155 | QIAEIGASLIK | non | y8 monochargé | 2 | TEM |
| 156 | QIAEIGASLIK | non | y9 monochargé | 2 | TEM |
| 157 | QTAEIGASLIK | non | y7 monochargé | 2 | 2be |
| 158 | QTAEIGASLIK | non | y8 monochargé | 2 | 2be |
| 159 | QTAEIGASLIK | non | y9 monochargé | 2 | 2be |
| 160 | SGADER | non | y3 monochargé | 2 | TEM |
| 161 | SGADER | non | y4 monochargé | 2 | TEM |
| 162 | SGADER | non | y5 monochargé | 2 | TEM |
| 163 | SGASK | non | y2 monochargé | 2 | 2be |
| 164 | SGASK | non | y3 monochargé | 2 | 2be |
| 165 | SGASK | non | y4 monochargé | 2 | 2be |
| 166 | VAGPLLR | non | y4 monochargé | 2 | TEM |
| 167 | VAGPLLR | non | y5 monochargé | 2 | TEM |
| 168 | VAGPLLR | non | y6 monochargé | 2 | TEM |
| 169 | VGYIEMDLNSGK | oui | y10 dichargé | 2 | 2be |
| 170 | VGYIEMDLNSGK | oui | y7 monochargé | 2 | 2be |
| 171 | VGYIEMDLNSGK | oui | y8 monochargé | 2 | 2be |
| 172 | VGYIEMDLNSGK | non | y7 monochargé | 2 | 2be |
| 173 | VGYIEMDLNSGK | non | y8 monochargé | 2 | 2be |
| 174 | VGYIEMDLNSGK | non | y9 monochargé | 2 | 2be |
| 175 | WEPELNEAIPNDER | non | y12 dichargé | 2 | TEM |
| 176 | WEPELNEAIPNDER | non | y5 monochargé | 2 | TEM |
| 177 | WEPELNEAIPNDER | non | y7 monochargé | 2 | TEM |
| 178 | ELTAFLHNMGEHVTR | non | y7 monochargé | 2 | 2b |
| 179 | ELTAFLHNMGEHVTR | non | y8 monochargé | 2 | 2b |
| 180 | ELTAFLHNMGEHVTR | non | y9 monochargé | 2 | 2b |
| 181 | FPMLSTFK | oui | y6 monochargé | 2 | TEM |
| 182 | FPMLSTFK | oui | y7 monochargé | 2 | TEM |
| 183 | FPMLSTFK | oui | y7 dichargé | 2 | TEM |
| 184 | FPMLSTFK | non | y6 monochargé | 2 | TEM |
| 185 | FPMLSTFK | non | y7 monochargé | 2 | TEM |
| 186 | FPMLSTFK | non | y7 dichargé | 2 | TEM |
| 187 | GSCGIIAALGPDGKPSR | non | y7 monochargé | 2 | 2br |
| 188 | GSCGIIAALGPDGKPSR | non | y8 monochargé | 2 | 2br |
| 189 | GSCGIIAALGPDGKPSR | non | y9 monochargé | 2 | 2br |
| 190 | GSSGIIAALGPDGKPSR | non | y7 monochargé | 2 | TEM |
| 191 | GSSGIIAALGPDGKPSR | non | y8 monochargé | 2 | TEM |
| 192 | GSSGIIAALGPDGKPSR | non | y9 monochargé | 2 | TEM |
| 193 | ILESFRPEK | non | y5 monochargé | 2 | TEM |
| 194 | ILESFRPEK | non | y6 monochargé | 2 | TEM |
| 195 | ILESFRPEK | non | y7 monochargé | 2 | TEM |
| 196 | IVVIYTTGSQATMDELNR | oui | y5 monochargé | 3 | 2br |
| 197 | IVVIYTTGSQATMDELNR | oui | y6 monochargé | 3 | 2br |
| 198 | IVVIYTTGSQATMDELNR | oui | y7 monochargé | 3 | 2br |
| 199 | IVVIYTTGSQATMDELNR | non | y5 monochargé | 3 | 2br |
| 200 | IVVIYTTGSQATMDELNR | non | y6 monochargé | 3 | 2br |
| 201 | IVVIYTTGSQATMDELNR | non | y7 monochargé | 3 | 2br |
| 202 | IVVIYTTGSQATMDER | non | y6 monochargé | 2 | TEM |
| 203 | IVVIYTTGSQATMDER | non | y8 monochargé | 2 | TEM |
| 204 | IVVIYTTGSQATMDER | non | y9 monochargé | 2 | TEM |
| 205 | LHCWEPELNEAIPNDER | non | y5 monochargé | 3 | 2be |
| 206 | LHCWEPELNEAIPNDER | non | y6 monochargé | 3 | 2be |
| 207 | LHCWEPELNEAIPNDER | non | y7 monochargé | 3 | 2be |
| 208 | LLTGELLTLASR | non | y6 monochargé | 2 | TEM |
| 209 | LLTGELLTLASR | non | y7 monochargé | 2 | TEM |
| 210 | LLTGELLTLASR | non | y9 monochargé | 2 | TEM |
| 211 | QQLIDWMADK | oui | y6 monochargé | 2 | 2ber |
| 212 | QQLIDWMADK | oui | y7 monochargé | 2 | 2ber |
| 213 | QQLIDWMADK | oui | y8 monochargé | 2 | 2ber |
| 214 | QQLIDWMADK | non | y6 monochargé | 2 | 2ber |
| 215 | QQLIDWMADK | non | y7 monochargé | 2 | 2ber |
| 216 | QQLIDWMADK | non | y8 monochargé | 2 | 2ber |
| 217 | SALPAGWFIADK | non | y7 monochargé | 2 | TEM |
| 218 | SALPAGWFIADK | non | y9 monochargé | 2 | TEM |
| 219 | SALPAGWFIADK | non | y9 dichargé | 2 | TEM |
| 220 | SGAGVR | non | y3 monochargé | 2 | 2be |
| 221 | SGAGVR | non | y4 monochargé | 2 | 2be |
| 222 | SGAGVR | non | y5 monochargé | 2 | 2be |
| 223 | SGTGER | non | y3 monochargé | 2 | 2be |
| 224 | SGTGER | non | y4 monochargé | 2 | 2be |
| 225 | SGTGER | non | y5 monochargé | 2 | 2be |
| 226 | VALIPFFAAFCIPVFAHPETLVK | non | y11 dichargé | 3 | 2br |
| 227 | VALIPFFAAFCIPVFAHPETLVK | non | y6 monochargé | 3 | 2br |
| 228 | VALIPFFAAFCIPVFAHPETLVK | non | y7 monochargé | 3 | 2br |
| 229 | VLLCGAVLSR | non | y6 monochargé | 2 | TEM |
| 230 | VLLCGAVLSR | non | y7 monochargé | 2 | TEM |
| 231 | VLLCGAVLSR | non | y8 monochargé | 2 | TEM |
| 232 | YSPVTEK | non | y4 monochargé | 2 | 2be |
| 233 | YSPVTEK | non | y5 monochargé | 2 | 2be |
| 234 | YSPVTEK | non | y6 monochargé | 2 | 2be |
| 235 | CEPELNEAIPNDER | non | y12 dichargé | 2 | 2br |
| 236 | CEPELNEAIPNDER | non | y5 monochargé | 2 | 2br |
| 237 | CEPELNEAIPNDER | non | y8 monochargé | 2 | 2br |
| 238 | DAEDQVGAR | non | y5 monochargé | 2 | 2be |
| 239 | DAEDQVGAR | non | y6 monochargé | 2 | 2be |
| 240 | DAEDQVGAR | non | y7 monochargé | 2 | 2be |
| 241 | DTTMPVAMATTLR | non | y7 monochargé | 2 | TEM |
| 242 | DTTMPVAMATTLR | non | y9 monochargé | 2 | TEM |
| 243 | DTTMPVAMATTLR | non | y9 dichargé | 2 | TEM |
| 244 | ELTAFLR | non | y4 monochargé | 2 | 2be |
| 245 | ELTAFLR | non | y5 monochargé | 2 | 2be |
| 246 | ELTAFLR | non | y6 monochargé | 2 | 2be |
| 247 | GSTGIIAALGPDGKPSR | non | y10 monochargé | 2 | TEM |
| 248 | GSTGIIAALGPDGKPSR | non | y7 monochargé | 2 | TEM |
| 249 | GSTGIIAALGPDGKPSR | non | y9 monochargé | 2 | TEM |
| 250 | HLTGGMTVR | oui | y5 monochargé | 2 | 2b |
| 251 | HLTGGMTVR | oui | y6 monochargé | 2 | 2b |
| 252 | HLTGGMTVR | oui | y7 monochargé | 2 | 2b |
| 253 | HLTGGMTVR | non | y5 monochargé | 2 | 2b |
| 254 | HLTGGMTVR | non | y6 monochargé | 2 | 2b |
| 255 | HLTGGMTVR | non | y7 monochargé | 2 | 2b |
| 256 | IVVIYMTGSQATMDELNR | oui | y6 monochargé | 3 | 2ber |
| 257 | IVVIYMTGSQATMDELNR | oui | y7 monochargé | 3 | 2ber |
| 258 | IVVIYMTGSQATMDELNR | oui | y8 monochargé | 3 | 2ber |
| 259 | IVVIYMTGSQATMDELNR | non | y5 monochargé | 3 | 2ber |
| 260 | IVVIYMTGSQATMDELNR | non | y6 monochargé | 3 | 2ber |
| 261 | IVVIYMTGSQATMDELNR | non | y7 monochargé | 3 | 2ber |
| 262 | LDHWEPELNEAIPNDER | non | y5 monochargé | 3 | 2be |
| 263 | LDHWEPELNEAIPNDER | non | y6 monochargé | 3 | 2be |
| 264 | LDHWEPELNEAIPNDER | non | y7 monochargé | 3 | 2be |
| 265 | LLTSELLTLASR | non | y10 monochargé | 2 | TEM |
| 266 | LLTSELLTLASR | non | y7 monochargé | 2 | TEM |
| 267 | LLTSELLTLASR | non | y9 monochargé | 2 | TEM |
| 268 | QIAEIGGSLIK | non | y7 monochargé | 2 | 2ber |
| 269 | QIAEIGGSLIK | non | y8 monochargé | 2 | 2ber |
| 270 | QIAEIGGSLIK | non | y9 monochargé | 2 | 2ber |
| 271 | QQLIDWMEVDK | oui | y6 monochargé | 2 | TEM |
| 272 | QQLIDWMEVDK | oui | y7 monochargé | 2 | TEM |
| 273 | QQLIDWMEVDK | oui | y8 monochargé | 2 | TEM |
| 274 | QQLIDWMEVDK | non | y5 monochargé | 2 | TEM |
| 275 | QQLIDWMEVDK | non | y6 monochargé | 2 | TEM |
| 276 | QQLIDWMEVDK | non | y7 monochargé | 2 | TEM |
| 277 | SVLPAGWFIADK | non | y10 dichargé | 2 | 2be |
| 278 | SVLPAGWFIADK | non | y9 monochargé | 2 | 2be |
| 279 | SVLPAGWFIADK | non | y9 dichargé | 2 | 2be |
| 280 | VAGPLMR | oui | y4 monochargé | 2 | 2br |
| 281 | VAGPLMR | oui | y5 monochargé | 2 | 2br |
| 282 | VAGPLMR | oui | y6 monochargé | 2 | 2br |
| 283 | VAGPLMR | non | y4 monochargé | 2 | 2br |
| 284 | VAGPLMR | non | y5 monochargé | 2 | 2br |
| 285 | VAGPLMR | non | y6 monochargé | 2 | 2br |
| 286 | VALIPFFAAFCLPVFAHPDTLVK | non | y11 dichargé | 3 | TEM |
| 287 | VALIPFFAAFCLPVFAHPDTLVK | non | y17 dichargé | 3 | TEM |
| 288 | VALIPFFAAFCLPVFAHPDTLVK | non | y6 monochargé | 3 | TEM |
| 289 | VALIPFFAAFCLPVFAHPK | non | y15 dichargé | 3 | TEM |
| 290 | VALIPFFAAFCLPVFAHPK | non | y7 monochargé | 3 | TEM |
| 291 | VALIPFFAAFCLPVFAHPK | non | y9 dichargé | 3 | TEM |
| 292 | VEDAEDQLGAR | non | y7 monochargé | 2 | 2b |
| 293 | VEDAEDQLGAR | non | y8 monochargé | 2 | 2b |
| 294 | VEDAEDQLGAR | non | y9 monochargé | 2 | 2b |
| 295 | DAEDQLGSTSGYIELDLNSGK | non | y7 monochargé | 3 | 2ber |
| 296 | DAEDQLGSTSGYIELDLNSGK | non | y8 monochargé | 3 | 2ber |
| 297 | DAEDQLGSTSGYIELDLNSGK | non | y9 monochargé | 3 | 2ber |
| 298 | DTTMPAAMATTLR | non | y7 monochargé | 2 | TEM |
| 299 | DTTMPAAMATTLR | non | y8 monochargé | 2 | TEM |
| 300 | DTTMPAAMATTLR | non | y9 monochargé | 2 | TEM |
| 301 | DTTTPAAMATTLR | oui | y7 monochargé | 2 | TEM |
| 302 | DTTTPAAMATTLR | oui | y8 monochargé | 2 | TEM |
| 303 | DTTTPAAMATTLR | oui | y9 dichargé | 2 | TEM |
| 304 | DTTTPAAMATTLR | non | y7 monochargé | 2 | TEM |
| 305 | DTTTPAAMATTLR | non | y9 monochargé | 2 | TEM |
| 306 | DTTTPAAMATTLR | non | y9 dichargé | 2 | TEM |
| 307 | GSHGIIAALGPDGKPSR | non | b6 monochargé | 2 | 2br |
| 308 | GSHGIIAALGPDGKPSR | non | y15 dichargé | 2 | 2br |
| 309 | GSHGIIAALGPDGKPSR | non | y8 monochargé | 2 | 2br |
| 310 | HLPDGMTVR | non | y5 monochargé | 2 | TEM |
| 311 | HLPDGMTVR | non | y7 monochargé | 2 | TEM |
| 312 | HLPDGMTVR | non | y8 monochargé | 2 | TEM |
| 313 | IHYSQSDLVEYSPVTEK | non | y16 dichargé | 2 | 2be |
| 314 | IHYSQSDLVEYSPVTEK | non | y7 monochargé | 2 | 2be |
| 315 | IHYSQSDLVEYSPVTEK | non | y8 monochargé | 2 | 2be |
| 316 | IVVIYMTGSQATMDER | non | y6 monochargé | 2 | TEM |
| 317 | IVVIYMTGSQATMDER | non | y8 monochargé | 2 | TEM |
| 318 | IVVIYMTGSQATMDER | non | y9 monochargé | 2 | TEM |
| 319 | LLTDELLTLASR | non | y5 monochargé | 2 | 2be |
| 320 | LLTDELLTLASR | non | y6 monochargé | 2 | 2be |
| 321 | LLTDELLTLASR | non | y7 monochargé | 2 | 2be |
| 322 | NMGDHVTR | oui | y4 monochargé | 2 | 2be |
| 323 | NMGDHVTR | oui | y5 monochargé | 2 | 2be |
| 324 | NMGDHVTR | oui | y6 monochargé | 2 | 2be |
| 325 | NMGDHVTR | non | y4 monochargé | 2 | 2be |
| 326 | NMGDHVTR | non | y5 monochargé | 2 | 2be |
| 327 | NMGDHVTR | non | y6 monochargé | 2 | 2be |
| 328 | QIVEIGASLIK | non | y7 monochargé | 2 | 2be |
| 329 | QIVEIGASLIK | non | y8 monochargé | 2 | 2be |
| 330 | QIVEIGASLIK | non | y9 monochargé | 2 | 2be |
| 331 | SGAGER | non | y3 monochargé | 2 | TEM |
| 332 | SGAGER | non | y4 monochargé | 2 | TEM |
| 333 | SGAGER | non | y5 monochargé | 2 | TEM |
| 334 | VAEPLLR | non | y4 monochargé | 2 | 2b |
| 335 | VAEPLLR | non | y5 monochargé | 2 | 2b |
| 336 | VAEPLLR | non | y6 monochargé | 2 | 2b |
| 337 | VALIPFFAAFCFPVFAHPETLVK | non | b11 monochargé | 3 | TEM |
| 338 | VALIPFFAAFCFPVFAHPETLVK | non | y11 dichargé | 3 | TEM |
| 339 | VALIPFFAAFCFPVFAHPETLVK | non | y6 monochargé | 3 | TEM |
| 340 | VALIPFFAAFCLPVFAHPETLVK | non | b7 monochargé | 3 | TEM |
| 341 | VALIPFFAAFCLPVFAHPETLVK | non | y11 dichargé | 3 | TEM |
| 342 | VALIPFFAAFCLPVFAHPETLVK | non | y6 monochargé | 3 | TEM |
| 343 | VDAGQEQLGR | non | y5 monochargé | 2 | TEM |
| 344 | VDAGQEQLGR | non | y7 monochargé | 2 | TEM |
| 345 | VDAGQEQLGR | non | y8 monochargé | 2 | TEM |
| 346 | VGYIELDPNSGK | non | y5 monochargé | 2 | 2be |
| 347 | VGYIELDPNSGK | non | y7 monochargé | 2 | 2be |
| 348 | VGYIELDPNSGK | non | y8 monochargé | 2 | 2be |

Les transitions mentionnées dans le **TABLEAU 3** sont détectées en utilisant les paramètres figurant dans le **TABLEAU 4.** Le temps de rétention du peptide précurseur et les transitions, c'est-à-dire les rapports (m/z)1 en Q1 et (m/z)2 en Q3 ainsi que l'énergie de collision utilisée pour fragmenter l'ion précurseur sont indiqués dans le TABLEAU 4. Le seuil au dela duquel la transition est considérée comme détectée est également indiquée dans le TABLEAU 4.

**TABLEAU 4:**

| Transition numéro | Temps de rétention (minute) | (m/z) filtré en Q1 | (m/z) filtré Q3 | Energie de collision (eV) | Seuil de positivité |
|---|---|---|---|---|---|
| 1 | 9,9 | 487,24 | 416,26 | 26 | 2500 |
| 2 | 9,9 | 487,24 | 658,36 | 26 | 2500 |
| 3 | 9,9 | 487,24 | 787,41 | 26 | 2500 |
| 4 | 19,3 | 861,95 | 512,29 | 43 | 2500 |
| 5 | 19,3 | 861,95 | 911,47 | 43 | 2500 |
| 6 | 19,3 | 861,95 | 1048,53 | 43 | 2500 |
| 7 | 19,3 | 859,42 | 792,37 | 43 | 2500 |
| 8 | 19,3 | 859,42 | 906,41 | 43 | 2500 |
| 9 | 19,3 | 859,42 | 1043,47 | 43 | 2500 |
| 10 | 18,1 | 493,751 | 595,34 | 27 | 2500 |
| 11 | 18,1 | 493,751 | 742,38 | 27 | 2500 |
| 12 | 18,1 | 493,751 | 839,43 | 27 | 2500 |
| 13 | 19,8 | 485,757 | 595,34 | 26 | 2500 |
| 14 | 19,8 | 485,757 | 726,39 | 26 | 2500 |
| 15 | 19,8 | 485,757 | 823,44 | 26 | 2500 |
| 16 | 15 | 791,87 | 698,84 | 40 | 2500 |
| 17 | 15 | 791,87 | 630,28 | 40 | 2500 |
| 18 | 15 | 791,87 | 814,41 | 40 | 2500 |
| 19 | 19,1 | 804,96 | 997,54 | 40 | 5000 |
| 20 | 19,1 | 804,96 | 813,42 | 40 | 5000 |
| 21 | 19,1 | 804,96 | 926,51 | 40 | 5000 |
| 22 | 16,6 | 674,67 | 660,36 | 38 | 2500 |
| 23 | 16,6 | 674,67 | 823,42 | 38 | 2500 |
| 24 | 16,6 | 674,67 | 952,46 | 38 | 2500 |
| 25 | 14,6 | 638,34 | 746,42 | 33 | 2500 |
| 26 | 14,6 | 638,34 | 972,51 | 33 | 2500 |
| 27 | 14,6 | 638,34 | 460,73 | 33 | 2500 |
| 28 | 15,9 | 885,44 | 866,37 | 44 | 2500 |
| 29 | 15,9 | 885,44 | 923,39 | 44 | 2500 |
| 30 | 15,9 | 885,44 | 980,41 | 44 | 2500 |
| 31 | 17 | 877,44 | 722,31 | 44 | 2500 |
| 32 | 17 | 877,44 | 907,39 | 44 | 2500 |
| 33 | 17 | 877,44 | 964,42 | 44 | 2500 |
| 34 | 19,5 | 676,32 | 630,28 | 38 | 2500 |
| 35 | 19,5 | 676,32 | 743,37 | 38 | 2500 |
| 36 | 19,5 | 676,32 | 814,41 | 38 | 2500 |
| 37 | 18,6 | 623,35 | 691,38 | 32 | 2500 |
| 38 | 18,6 | 623,35 | 804,46 | 32 | 2500 |
| 39 | 18,6 | 623,35 | 933,51 | 32 | 2500 |
| 40 | 1,3 | 317,15 | 418,2 | 19 | 6000 |
| 41 | 1,3 | 317,15 | 489,24 | 19 | 6000 |
| 42 | 1,3 | 317,15 | 546,26 | 19 | 6000 |
| 43 | 1 | 296,64 | 391,19 | 18 | 2500 |
| 44 | 1 | 296,64 | 448,22 | 18 | 2500 |
| 45 | 1 | 296,64 | 505,24 | 18 | 2500 |
| 46 | 10,5 | 565,78 | 845,41 | 30 | 2500 |
| 47 | 10,6 | 565,78 | 916,45 | 30 | 2500 |
| 48 | 10,6 | 565,78 | 1031,48 | 30 | 2500 |
| 49 | 4,1 | 393,72 | 462,22 | 22 | 2500 |
| 50 | 4,1 | 393,72 | 559,27 | 22 | 2500 |
| 51 | 4,1 | 393,72 | 687,37 | 22 | 2500 |
| 52 | 20,1 | 855,92 | 698,36 | 43 | 2500 |
| 53 | 20,1 | 855,92 | 629,33 | 43 | 2500 |
| 54 | 20,1 | 855,92 | 813,44 | 43 | 2500 |
| 55 | 17 | 585,26 | 705,36 | 31 | 4000 |
| 56 | 17 | 585,26 | 852,4 | 31 | 4000 |
| 57 | 16,9 | 585,26 | 953,44 | 31 | 4000 |
| 58 | 13,2 | 569,27 | 689,37 | 30 | 2500 |
| 59 | 13,2 | 569,27 | 820,41 | 30 | 2500 |
| 60 | 13,2 | 569,27 | 921,45 | 30 | 2500 |
| 61 | 18,3 | 870,93 | 749,86 | 43 | 2500 |
| 62 | 18,3 | 870,93 | 512,29 | 43 | 2500 |
| 63 | 18,3 | 870,93 | 929,43 | 43 | 2500 |
| 64 | 14,9 | 763,36 | 630,28 | 39 | 2500 |
| 65 | 14,9 | 763,36 | 814,41 | 39 | 2500 |
| 66 | 14,9 | 763,36 | 943,45 | 39 | 2500 |
| 67 | 14,9 | 510,73 | 776,33 | 27 | 2500 |
| 68 | 14,9 | 510,73 | 873,38 | 27 | 2500 |
| 69 | 14,9 | 510,73 | 437,2 | 27 | 2500 |
| 70 | 18,9 | 494,74 | 744,34 | 27 | 2500 |
| 71 | 18,9 | 494,74 | 841,39 | 27 | 2500 |
| 72 | 18,9 | 494,74 | 421,2 | 27 | 2500 |
| 73 | 15,5 | 676,39 | 756,4 | 35 | 2500 |
| 74 | 15,5 | 676,39 | 813,42 | 35 | 2500 |
| 75 | 15,5 | 676,39 | 926,51 | 35 | 2500 |
| 76 | 11,5 | 543,78 | 787,41 | 29 | 2500 |
| 77 | 11,5 | 543,78 | 858,44 | 29 | 2500 |
| 78 | 11,5 | 543,78 | 973,47 | 29 | 2500 |
| 79 | 12,5 | 608,82 | 803,43 | 32 | 2500 |
| 80 | 12,5 | 608,82 | 966,49 | 32 | 2500 |
| 81 | 12,5 | 608,82 | 552,28 | 32 | 2500 |
| 82 | 15,5 | 990,98 | 934,44 | 49 | 2500 |
| 83 | 15,5 | 990,98 | 573,32 | 49 | 2500 |
| 84 | 15,5 | 990,98 | 660,36 | 49 | 2500 |
| 85 | 19 | 892,44 | 722,31 | 44 | 2500 |
| 86 | 19 | 892,44 | 907,39 | 44 | 2500 |
| 87 | 19 | 892,44 | 964,42 | 44 | 2500 |
| 88 | 19,4 | 700,65 | 630,28 | 39 | 2500 |
| 89 | 19,4 | 700,65 | 743,37 | 39 | 2500 |
| 90 | 19,4 | 700,65 | 814,41 | 39 | 2500 |
| 91 | 10,4 | 467,73 | 587,3 | 26 | 2500 |
| 92 | 10,4 | 467,73 | 700,39 | 26 | 2500 |
| 93 | 10,4 | 467,73 | 787,42 | 26 | 2500 |
| 94 | 12,8 | 459,73 | 587,3 | 25 | 2500 |
| 95 | 12,8 | 459,73 | 700,39 | 25 | 2500 |
| 96 | 12,8 | 459,73 | 787,42 | 25 | 2500 |
| 97 | 20,1 | 688,83 | 593,26 | 35 | 2500 |
| 98 | 20,1 | 688,83 | 779,34 | 35 | 2500 |
| 99 | 20,1 | 688,83 | 894,37 | 35 | 2500 |
| 100 | 1,4 | 303,646 | 391,19 | 18 | 13000 |
| 101 | 1,4 | 303,646 | 462,23 | 18 | 13000 |
| 102 | 1,4 | 303,646 | 519,25 | 18 | 13000 |
| 103 | 31,6 | 851,81 | 619,35 | 47 | 2500 |
| 104 | 31,6 | 851,81 | 686,41 | 47 | 2500 |
| 105 | 31,6 | 851,81 | 894,5 | 47 | 2500 |
| 106 | 18,8 | 654,35 | 746,4 | 34 | 2500 |
| 107 | 18,8 | 654,35 | 875,45 | 34 | 2500 |
| 108 | 18,8 | 654,35 | 988,53 | 34 | 2500 |
| 109 | 16,3 | 559,31 | 632,34 | 30 | 2500 |
| 110 | 16,3 | 559,31 | 792,37 | 30 | 2500 |
| 111 | 16,3 | 559,31 | 905,45 | 30 | 2500 |
| 112 | 10,5 | 487,73 | 544,32 | 26 | 2500 |
| 113 | 10,5 | 487,73 | 659,35 | 26 | 2500 |
| 114 | 10,5 | 487,73 | 788,39 | 26 | 2500 |
| 115 | 12,6 | 690,35 | 779,41 | 35 | 2500 |
| 116 | 12,6 | 690,35 | 947,5 | 35 | 2500 |
| 117 | 12,6 | 690,35 | 474,26 | 35 | 2500 |
| 118 | 15,9 | 682,35 | 763,41 | 35 | 2500 |
| 119 | 15,9 | 682,35 | 931,5 | 35 | 2500 |
| 120 | 15,9 | 682,35 | 466,26 | 35 | 2500 |
| 121 | 19,8 | 486,75 | 728,36 | 26 | 2500 |
| 122 | 19,8 | 486,75 | 825,42 | 26 | 2500 |
| 123 | 19,8 | 486,75 | 413,21 | 26 | 2500 |
| 124 | 18,4 | 478,75 | 712,37 | 26 | 2500 |
| 125 | 18,4 | 478,75 | 809,42 | 26 | 2500 |
| 126 | 18,4 | 478,75 | 405,21 | 26 | 2500 |
| 127 | 16,3 | 776,93 | 756,4 | 39 | 2500 |
| 128 | 16,3 | 776,93 | 813,42 | 39 | 2500 |
| 129 | 16,3 | 776,93 | 926,51 | 39 | 2500 |
| 130 | 12,2 | 523,26 | 579,3 | 28 | 2500 |
| 131 | 12,2 | 523,26 | 795,37 | 28 | 2500 |
| 132 | 12,2 | 523,26 | 908,45 | 28 | 2500 |
| 133 | 12 | 515,26 | 506,28 | 28 | 2500 |
| 134 | 12 | 515,26 | 779,37 | 28 | 2500 |
| 135 | 12 | 515,26 | 892,46 | 28 | 2500 |
| 136 | 18 | 899,46 | 439,33 | 36 | 2500 |
| 137 | 18 | 899,46 | 304,16 | 57 | 2500 |
| 138 | 18 | 899,46 | 994,43 | 36 | 2500 |
| 139 | 15,6 | 681,33 | 661,29 | 38 | 2500 |
| 140 | 15,6 | 681,33 | 808,33 | 38 | 2500 |
| 141 | 15,6 | 681,33 | 909,37 | 38 | 2500 |
| 142 | 16,6 | 676 | 661,29 | 38 | 3100 |
| 143 | 16,6 | 676 | 792,33 | 38 | 3100 |
| 144 | 16,6 | 676 | 893,38 | 38 | 3100 |
| 145 | 17,2 | 688,32 | 630,28 | 38 | 2500 |
| 146 | 17,2 | 688,32 | 729,35 | 38 | 2500 |
| 147 | 17,2 | 688,32 | 800,39 | 38 | 2500 |
| 148 | 27 | 878,12 | 841,5 | 48 | 2500 |
| 149 | 27 | 878,12 | 795,34 | 48 | 2500 |
| 150 | 27 | 878,12 | 910,37 | 48 | 2500 |
| 151 | 27,9 | 872,79 | 462,22 | 48 | 2500 |
| 152 | 27,9 | 872,79 | 779,34 | 48 | 2500 |
| 153 | 27,9 | 872,79 | 894,37 | 48 | 2500 |
| 154 | 18,8 | 571,84 | 701,46 | 30 | 2500 |
| 155 | 18,8 | 571,84 | 830,5 | 30 | 2500 |
| 156 | 18,8 | 571,84 | 901,54 | 30 | 2500 |
| 157 | 16,6 | 565,82 | 701,46 | 30 | 2500 |
| 158 | 16,6 | 565,82 | 830,5 | 30 | 2500 |
| 159 | 16,6 | 565,82 | 901,54 | 30 | 2500 |
| 160 | 1,5 | 317,64 | 419,19 | 19 | 9000 |
| 161 | 1,5 | 317,64 | 490,23 | 19 | 9000 |
| 162 | 1,5 | 317,64 | 547,25 | 19 | 9000 |
| 163 | 0,9 | 225,12 | 234,14 | 18 | 2500 |
| 164 | 0,9 | 225,12 | 305,18 | 13 | 2500 |
| 165 | 0,9 | 225,12 | 362,2 | 13 | 2500 |
| 166 | 14,1 | 363,24 | 498,34 | 21 | 2500 |
| 167 | 14,1 | 363,24 | 555,36 | 21 | 2500 |
| 168 | 14,1 | 363,24 | 626,4 | 21 | 2500 |
| 169 | 14,7 | 671,32 | 593,28 | 35 | 4500 |
| 170 | 14,7 | 671,32 | 780,36 | 35 | 4500 |
| 171 | 14,7 | 671,32 | 909,4 | 35 | 4500 |
| 172 | 17,5 | 663,32 | 764,36 | 34 | 2500 |
| 173 | 17,5 | 663,32 | 893,4 | 34 | 2500 |
| 174 | 17,5 | 663,32 | 1006,49 | 34 | 2500 |
| 175 | 18 | 856,4 | 698,84 | 43 | 2500 |
| 176 | 18 | 856,4 | 630,28 | 43 | 2500 |
| 177 | 18 | 856,4 | 814,41 | 43 | 2500 |
| 178 | 18,6 | 877,94 | 829,4 | 44 | 2500 |
| 179 | 18,6 | 877,94 | 943,44 | 44 | 2500 |
| 180 | 18,6 | 877,94 | 1080,5 | 44 | 2500 |
| 181 | 18,1 | 493,75 | 742,38 | 27 | 2500 |
| 182 | 18,1 | 493,75 | 839,43 | 27 | 2500 |
| 183 | 18,1 | 493,75 | 420,22 | 27 | 2500 |
| 184 | 19,8 | 485,76 | 726,39 | 26 | 2500 |
| 185 | 19,8 | 485,76 | 823,44 | 26 | 2500 |
| 186 | 19,8 | 485,76 | 412,22 | 26 | 2500 |
| 187 | 16,5 | 828,43 | 756,4 | 41 | 2500 |
| 188 | 16,5 | 828,43 | 813,42 | 41 | 2500 |
| 189 | 16,5 | 828,43 | 926,51 | 41 | 2500 |
| 190 | 16,1 | 791,93 | 756,4 | 40 | 2500 |
| 191 | 16,1 | 791,93 | 813,42 | 40 | 2500 |
| 192 | 16,1 | 791,93 | 926,51 | 40 | 2500 |
| 193 | 15,7 | 559,81 | 676,38 | 30 | 2500 |
| 194 | 14,1 | 559,81 | 763,41 | 30 | 2500 |
| 195 | 14,1 | 559,81 | 892,45 | 30 | 2500 |
| 196 | 16,6 | 676,34 | 646,31 | 38 | 2500 |
| 197 | 16,6 | 676,34 | 793,96 | 38 | 2500 |
| 198 | 16,6 | 676,34 | 894,4 | 38 | 2500 |
| 199 | 18,9 | 671,01 | 646,31 | 38 | 4000 |
| 200 | 18,9 | 671,01 | 777,96 | 38 | 4000 |
| 201 | 18,9 | 671,01 | 878,4 | 38 | 4000 |
| 202 | 16,9 | 892,45 | 722,31 | 44 | 2500 |
| 203 | 16,9 | 892,45 | 937,4 | 44 | 2500 |
| 204 | 16,9 | 892,45 | 994,43 | 44 | 2500 |
| 205 | 17,9 | 707,99 | 630,28 | 39 | 6000 |
| 206 | 17,9 | 707,99 | 743,37 | 39 | 6000 |
| 207 | 17,9 | 707,99 | 814,41 | 39 | 6000 |
| 208 | 22,5 | 643,89 | 660,4 | 33 | 2500 |
| 209 | 22,5 | 643,89 | 773,49 | 33 | 2500 |
| 210 | 22,5 | 643,89 | 959,55 | 33 | 2500 |
| 211 | 17,5 | 632,31 | 781,32 | 33 | 3000 |
| 212 | 17,5 | 632,31 | 894,4 | 33 | 3000 |
| 213 | 17,5 | 632,31 | 1007,49 | 33 | 3000 |
| 214 | 19,8 | 624,31 | 765,32 | 32 | 2500 |
| 215 | 19,8 | 624,31 | 878,41 | 32 | 2500 |
| 216 | 19,8 | 624,31 | 991,49 | 32 | 2500 |
| 217 | 21,4 | 638,34 | 836,43 | 33 | 2500 |
| 218 | 21,4 | 638,34 | 1004,52 | 33 | 2500 |
| 219 | 21,5 | 638,34 | 502,76 | 33 | 2500 |
| 220 | 3,2 | 273,65 | 331,21 | 17 | 2500 |
| 221 | 3,2 | 273,65 | 402,25 | 17 | 2500 |
| 222 | 3,2 | 273,65 | 459,27 | 17 | 2500 |
| 223 | 1,3 | 303,65 | 361,18 | 18 | 21000 |
| 224 | 1,3 | 303,65 | 462,23 | 18 | 21000 |
| 225 | 1,3 | 303,65 | 519,25 | 18 | 21000 |
| 226 | 31,3 | 863,138 | 619,35 | 47 | 2500 |
| 227 | 31,3 | 863,138 | 686,41 | 47 | 2500 |
| 228 | 31,3 | 863,138 | 823,47 | 47 | 2500 |
| 229 | 17,7 | 544,32 | 602,36 | 29 | 2500 |
| 230 | 17,7 | 544,32 | 762,39 | 29 | 2500 |
| 231 | 17,7 | 544,32 | 875,48 | 29 | 2500 |
| 232 | 9,8 | 412,21 | 476,27 | 23 | 2500 |
| 233 | 9,8 | 412,21 | 573,32 | 23 | 2500 |
| 234 | 9,8 | 412,21 | 660,36 | 23 | 2500 |
| 235 | 15,2 | 843,38 | 698,84 | 42 | 2500 |
| 236 | 15,2 | 843,38 | 630,28 | 42 | 2500 |
| 237 | 15,2 | 843,38 | 943,45 | 42 | 2500 |
| 238 | 8,7 | 480,72 | 530,3 | 26 | 2500 |
| 239 | 8,7 | 480,72 | 645,33 | 26 | 2500 |
| 240 | 8,7 | 480,72 | 774,37 | 26 | 2500 |
| 241 | 18,8 | 704,35 | 763,41 | 36 | 2500 |
| 242 | 18,8 | 704,35 | 959,53 | 36 | 2500 |
| 243 | 18,8 | 704,35 | 480,27 | 36 | 2500 |
| 244 | 18,3 | 425,25 | 506,31 | 24 | 2500 |
| 245 | 18,3 | 425,25 | 607,36 | 24 | 2500 |
| 246 | 18,3 | 425,25 | 720,44 | 24 | 2500 |
| 247 | 16,4 | 798,94 | 997,54 | 40 | 2500 |
| 248 | 16,3 | 798,94 | 756,4 | 40 | 2500 |
| 249 | 16,3 | 798,94 | 926,51 | 40 | 2500 |
| 250 | 8,3 | 494,26 | 850,45 | 27 | 2500 |
| 251 | 8,3 | 494,26 | 636,31 | 27 | 2500 |
| 252 | 8,3 | 494,26 | 737,36 | 27 | 2500 |
| 253 | 10,9 | 486,26 | 834,45 | 26 | 2500 |
| 254 | 10,9 | 486,26 | 620,32 | 26 | 2500 |
| 255 | 10,9 | 486,26 | 721,37 | 26 | 2500 |
| 256 | 19,3 | 691,67 | 793,36 | 39 | 2500 |
| 257 | 19,3 | 691,67 | 894,4 | 39 | 2500 |
| 258 | 19,3 | 691,67 | 965,44 | 39 | 2500 |
| 259 | 20,6 | 681,01 | 646,31 | 38 | 2500 |
| 260 | 20,6 | 681,01 | 777,36 | 38 | 2500 |
| 261 | 20,6 | 681,01 | 878,4 | 38 | 2500 |
| 262 | 18 | 692,99 | 630,28 | 39 | 2500 |
| 263 | 18 | 692,99 | 743,37 | 39 | 2500 |
| 264 | 18 | 692,99 | 814,41 | 39 | 2500 |
| 265 | 23 | 658,89 | 1090,61 | 34 | 2500 |
| 266 | 23 | 658,89 | 773,49 | 34 | 2500 |
| 267 | 23 | 658,89 | 989,56 | 34 | 2500 |
| 268 | 17,9 | 564,83 | 687,44 | 30 | 2500 |
| 269 | 17,9 | 564,83 | 816,48 | 30 | 2500 |
| 270 | 17,9 | 564,83 | 887,52 | 30 | 2500 |
| 271 | 18,9 | 710,84 | 823,37 | 36 | 2500 |
| 272 | 18,9 | 710,84 | 938,39 | 36 | 2500 |
| 273 | 18,9 | 710,84 | 1051,48 | 36 | 2500 |
| 274 | 20,9 | 702,84 | 621,29 | 36 | 2500 |
| 275 | 20,9 | 702,84 | 807,37 | 36 | 2500 |
| 276 | 20,9 | 702,84 | 922,4 | 36 | 2500 |
| 277 | 22,6 | 652,36 | 559,31 | 34 | 2500 |
| 278 | 22,6 | 652,36 | 1004,52 | 34 | 2500 |
| 279 | 22,6 | 652,36 | 502,76 | 34 | 2500 |
| 280 | 11,1 | 380,21 | 532,29 | 22 | 2500 |
| 281 | 11,1 | 380,21 | 589,31 | 22 | 2500 |
| 282 | 11,1 | 380,21 | 660,35 | 22 | 2500 |
| 283 | 13,1 | 372,22 | 516,3 | 21 | 2500 |
| 284 | 13,1 | 372,22 | 573,32 | 21 | 2500 |
| 285 | 13,1 | 372,22 | 644,35 | 21 | 2500 |
| 286 | 31,3 | 858,47 | 612,34 | 47 | 2500 |
| 287 | 31,3 | 858,47 | 967,1 | 47 | 2500 |
| 288 | 31,3 | 858,47 | 672,39 | 47 | 2500 |
| 289 | 30,5 | 715,73 | 874,95 | 40 | 2500 |
| 290 | 30,5 | 715,73 | 795,45 | 40 | 2500 |
| 291 | 30,6 | 715,73 | 534,79 | 40 | 2500 |
| 292 | 11,7 | 601,79 | 788,39 | 31 | 2500 |
| 293 | 11,7 | 601,79 | 859,43 | 31 | 2500 |
| 294 | 11,7 | 601,79 | 974,45 | 31 | 2500 |
| 295 | 20 | 738,01 | 746,4 | 41 | 2500 |
| 296 | 20 | 738,01 | 875,45 | 41 | 2500 |
| 297 | 20 | 738,01 | 988,53 | 41 | 2500 |
| 298 | 17,6 | 690,34 | 763,61 | 35 | 2500 |
| 299 | 17,6 | 690,34 | 834,45 | 35 | 2500 |
| 300 | 17,6 | 690,34 | 931,5 | 35 | 2500 |
| 301 | 19,5 | 683,34 | 779,41 | 35 | 6000 |
| 302 | 19,5 | 683,34 | 850,45 | 35 | 6000 |
| 303 | 19,5 | 683,34 | 474,25 | 35 | 6000 |
| 304 | 16,1 | 675,34 | 763,41 | 35 | 2500 |
| 305 | 16,1 | 675,34 | 931,5 | 35 | 2500 |
| 306 | 16,1 | 675,34 | 466,25 | 35 | 2500 |
| 307 | 14,4 | 816,94 | 565,31 | 41 | 2500 |
| 308 | 14,4 | 816,94 | 744,92 | 41 | 2500 |
| 309 | 14,4 | 816,94 | 813,42 | 41 | 2500 |
| 310 | 13 | 513,26 | 563,3 | 28 | 2500 |
| 311 | 13 | 513,26 | 775,38 | 28 | 2500 |
| 312 | 13 | 513,26 | 888,46 | 28 | 2500 |
| 313 | 16,6 | 997,99 | 941,45 | 49 | 2500 |
| 314 | 16,6 | 997,99 | 573,32 | 49 | 2500 |
| 315 | 16,6 | 997,99 | 952,46 | 49 | 2500 |
| 316 | 18,9 | 907,44 | 722,32 | 45 | 2500 |
| 317 | 18,9 | 907,44 | 937,4 | 45 | 2500 |
| 318 | 18,9 | 907,44 | 994,43 | 45 | 2500 |
| 319 | 23,7 | 672,89 | 547,32 | 35 | 2500 |
| 320 | 23,7 | 672,89 | 660,4 | 35 | 2500 |
| 321 | 23,7 | 672,89 | 773,49 | 35 | 2500 |
| 322 | 17,1 | 473,21 | 528,29 | 26 | 2500 |
| 323 | 17,9 | 473,21 | 627,32 | 26 | 2500 |
| 324 | 17,8 | 473,21 | 684,34 | 26 | 2500 |
| 325 | 8,4 | 465,22 | 512,29 | 25 | 9000 |
| 326 | 8,4 | 465,22 | 627,32 | 25 | 9000 |
| 327 | 8,4 | 465,22 | 684,34 | 25 | 9000 |
| 328 | 19,8 | 585,86 | 701,46 | 31 | 2500 |
| 329 | 19,8 | 585,86 | 830,5 | 31 | 2500 |
| 330 | 19,8 | 585,86 | 929,57 | 31 | 2500 |
| 331 | 1,3 | 288,64 | 361,18 | 18 | 8000 |
| 332 | 1,3 | 288,64 | 432,22 | 18 | 8000 |
| 333 | 1,3 | 288,64 | 489,24 | 18 | 8000 |
| 334 | 14,5 | 399,25 | 498,34 | 23 | 6000 |
| 335 | 14,5 | 399,25 | 627,38 | 23 | 6000 |
| 336 | 14,5 | 399,25 | 698,42 | 23 | 6000 |
| 337 | 31,3 | 874,47 | 823,47 | 48 | 2500 |
| 338 | 31,3 | 874,47 | 619,41 | 48 | 2500 |
| 339 | 31,3 | 874,47 | 686,41 | 48 | 2500 |
| 340 | 31,3 | 863,14 | 823,47 | 47 | 2500 |
| 341 | 31,3 | 863,14 | 619,34 | 47 | 2500 |
| 342 | 31,3 | 863,14 | 686,41 | 47 | 2500 |
| 343 | 10,6 | 536,77 | 602,38 | 29 | 2500 |
| 344 | 10,6 | 536,77 | 787,41 | 29 | 2500 |
| 345 | 10,6 | 536,77 | 858,44 | 29 | 2500 |
| 346 | 16,5 | 646,33 | 502,26 | 33 | 7000 |
| 347 | 16,5 | 646,33 | 730,37 | 33 | 7000 |
| 348 | 16,5 | 646,33 | 859,42 | 33 | 7000 |

Les autres paramètres machine utilisés sont les suivants :
- Type de balayage: MRM
- MRM planifié: oui
- Polarité: Positive
- Source d'ionisation:Turbo V™ (Applied BioSystems)
- Réglage Q1: Filtrage avec résolution unitaire
- Réglage Q3: Filtrage avec résolution unitaire
- Pause inter-scan: 5.00 msec
- Vitesse de balayage: 10 Da/s
- Gaz rideau: 50,00 psi
- Tension de cône: 5500,00 V
- Température de source: 500,00 °C
- Gaz de nébulisation: 50,00 psi
- Gaz chauffant: 40,00 psi
- Gaz de collision induisant dissociation : 9,00 psi
- Remplissage dynamique: activé
- Potentiel d'orifice : 80, 00 V
- (en anglais declustering potential (DP))
- Potentiel d'entrée avant Q0 (EP): 10,00 V
- Potentiel en sortie de cellule de collision : 35 V
- (en anglais cell exit potential (CXP))
- Temps de cycle total: 1.2 sec
- Fenêtre de détection : 90 sec

Les aires obtenues pour chacune des transitions et pour chacun des microorganismes étudiés ont été mesurées. Lorsque les aires des 3 transitions d'un même peptide sont supérieures ou égales au seuil de positivité décrit dans le **TABLEAU 4,** la détection du peptide est considérée comme positive et est notée « 1 » dans le **TABLEAU 5.** Lorsque au moins une transition comporte une aire inférieure au seuil de positivité décrit dans le **TABLEAU 4,** le peptide correspondant est considéré comme non détecté et est noté 0 dans le **TABLEAU 5.**

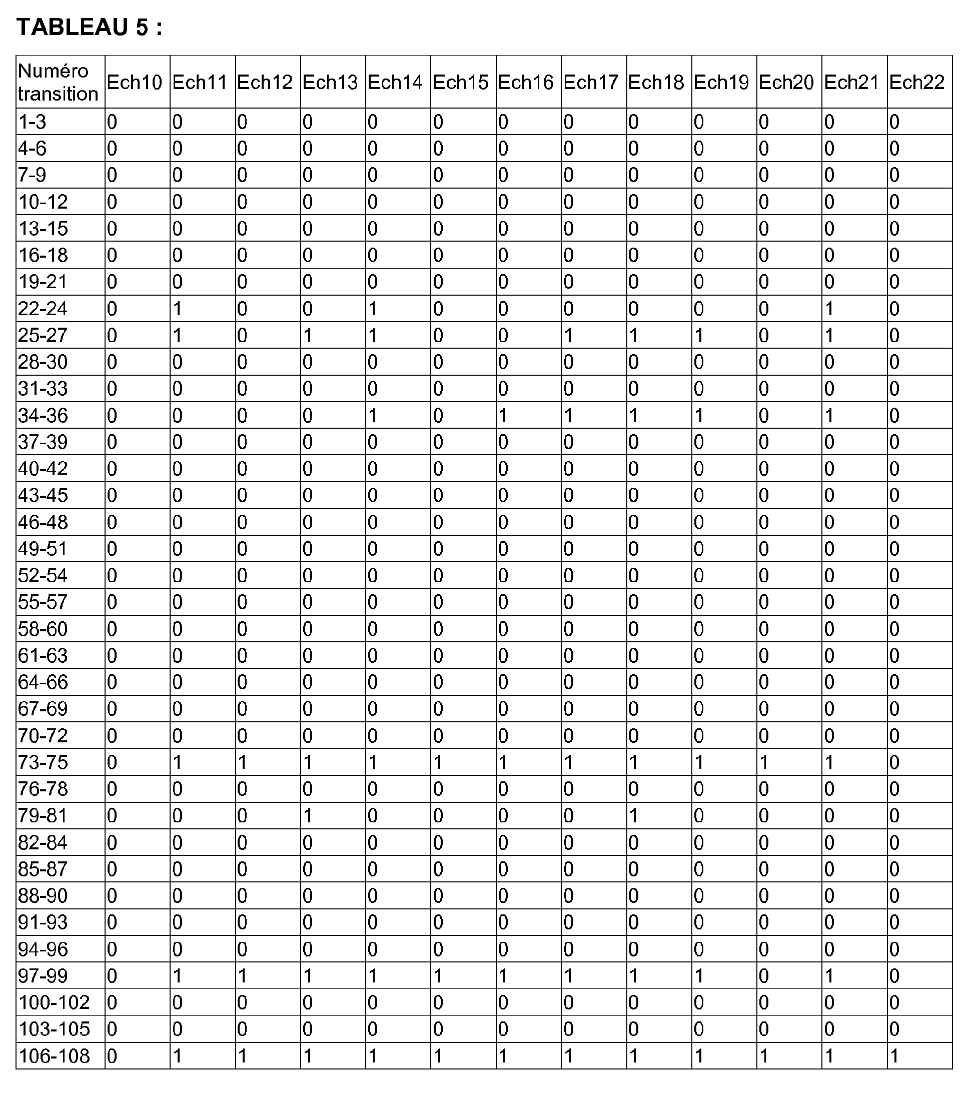

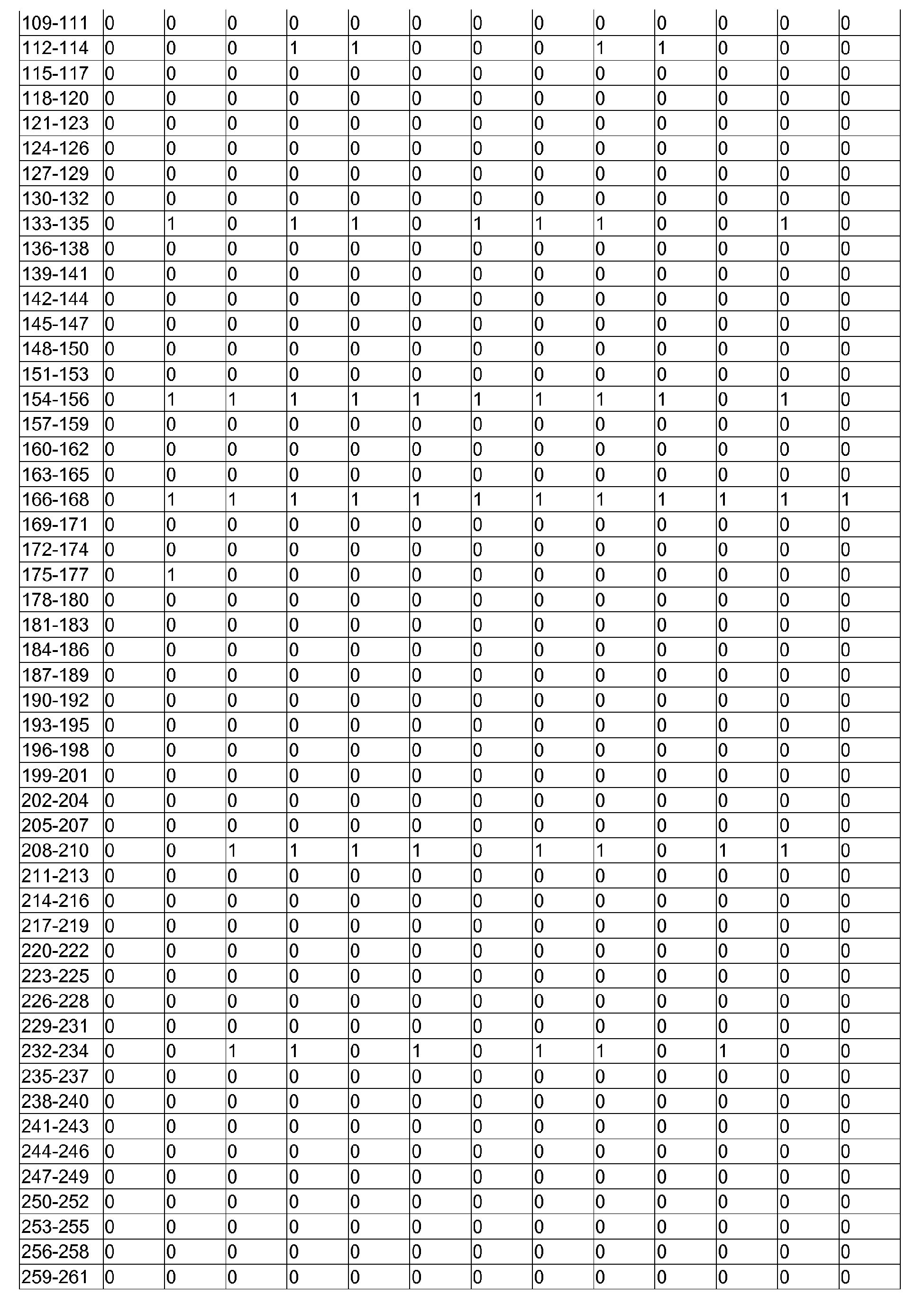

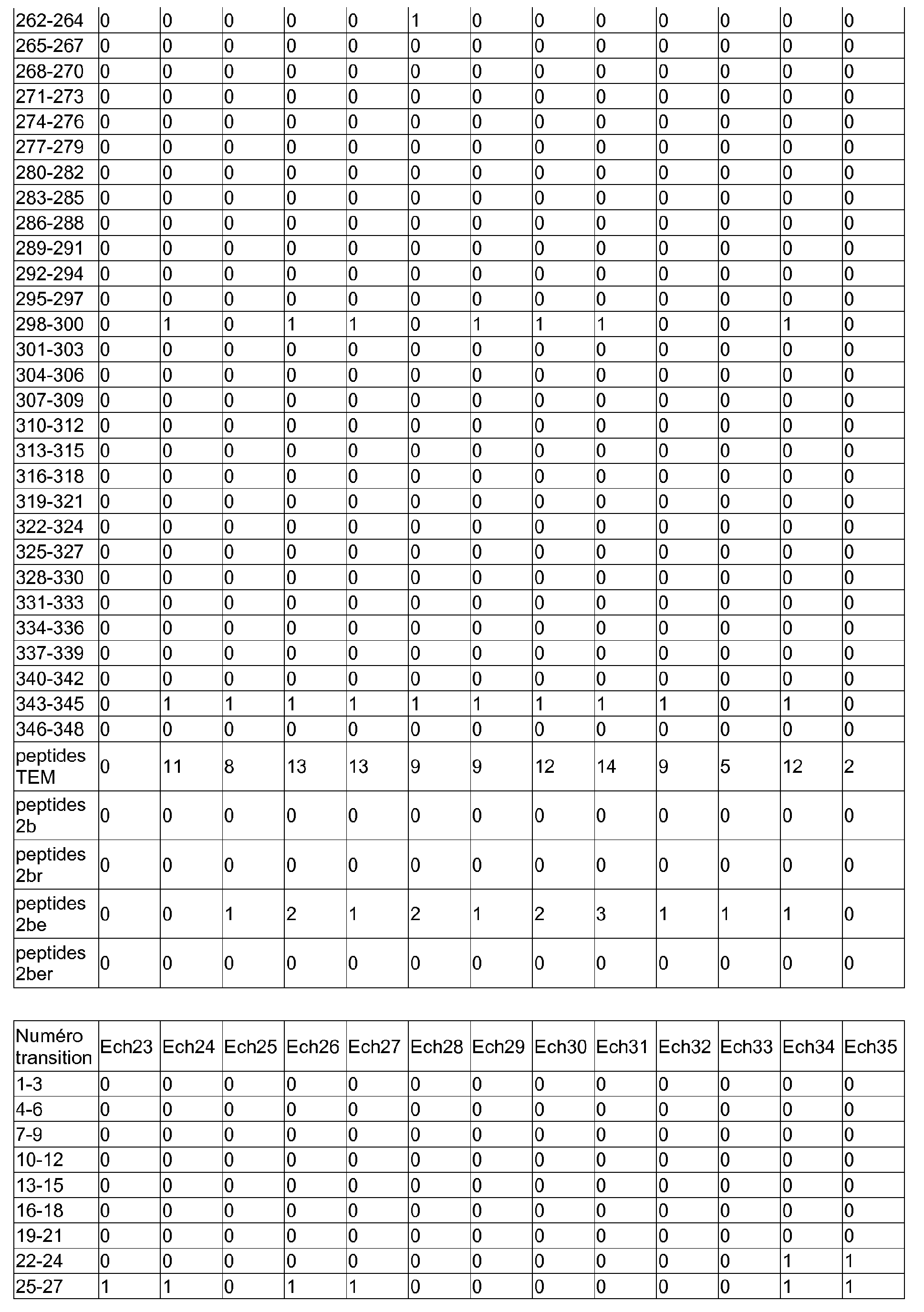

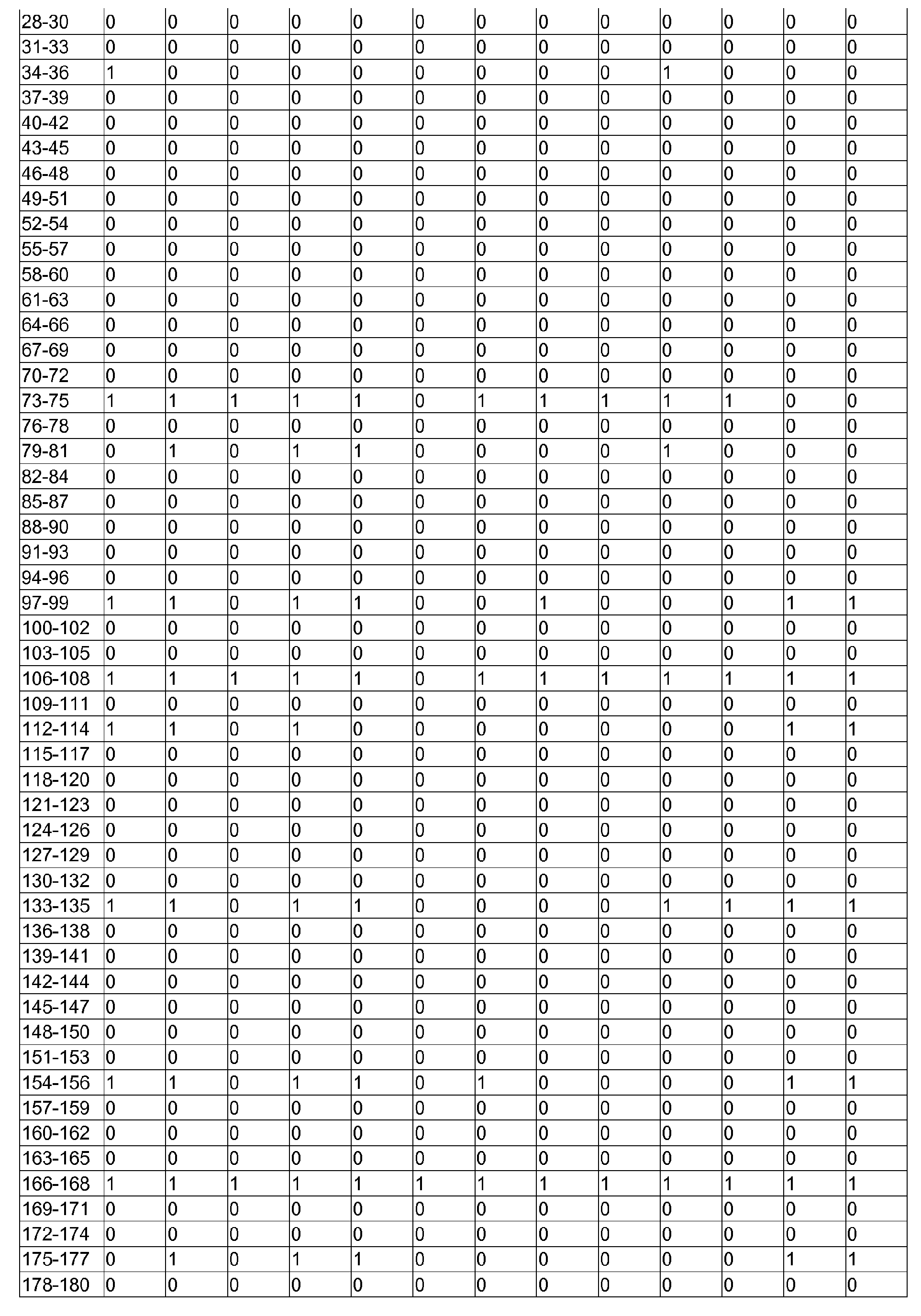

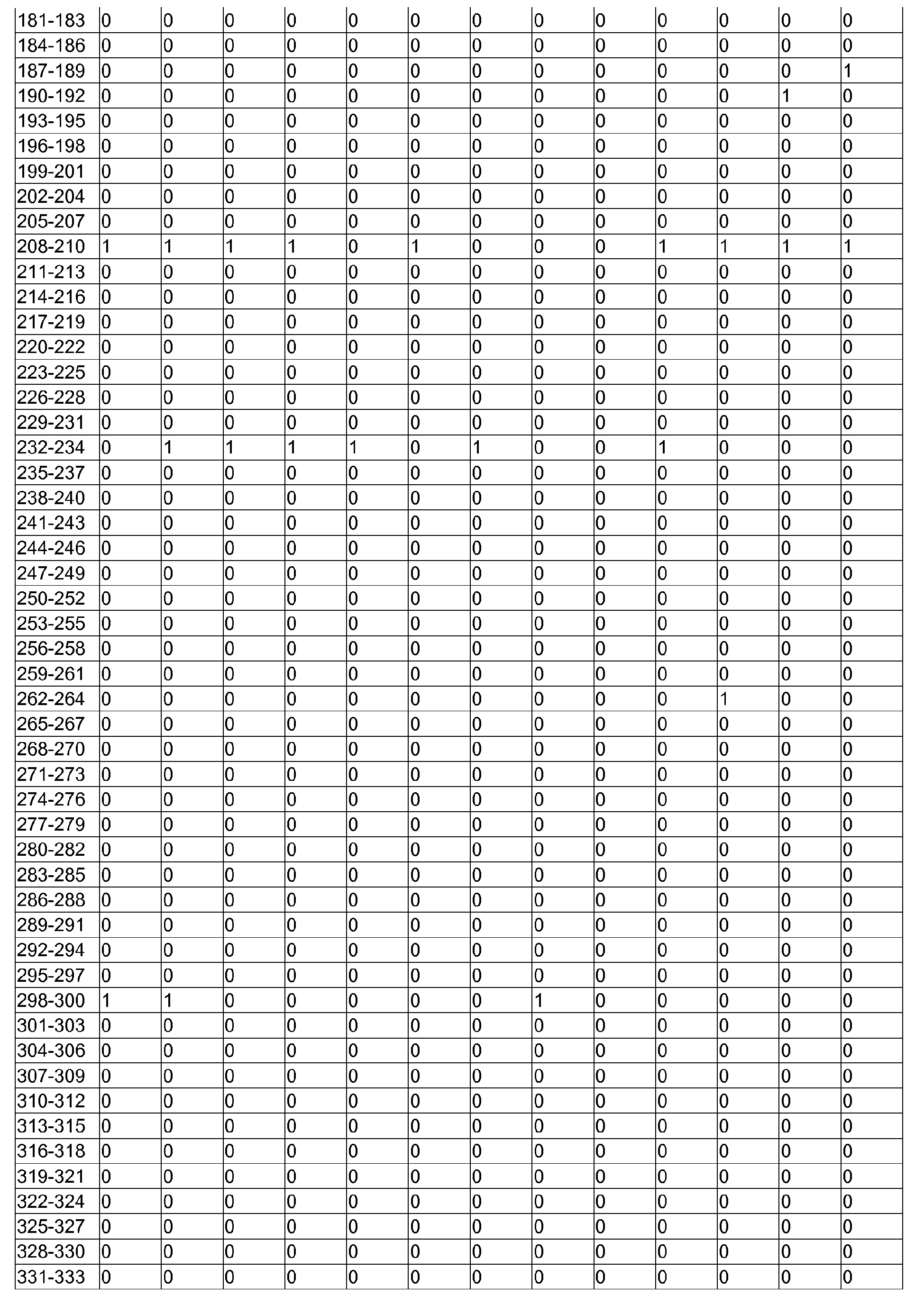

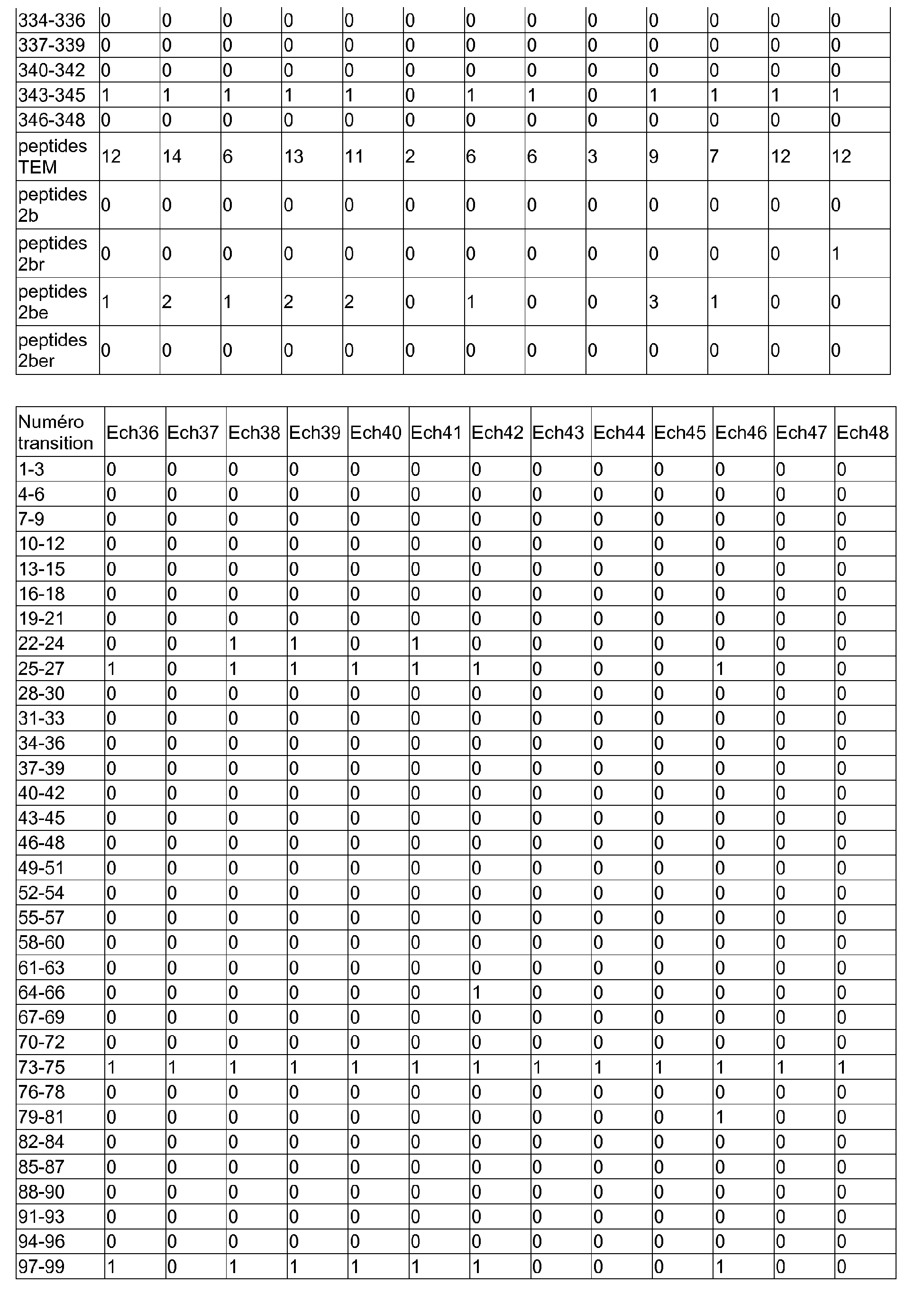

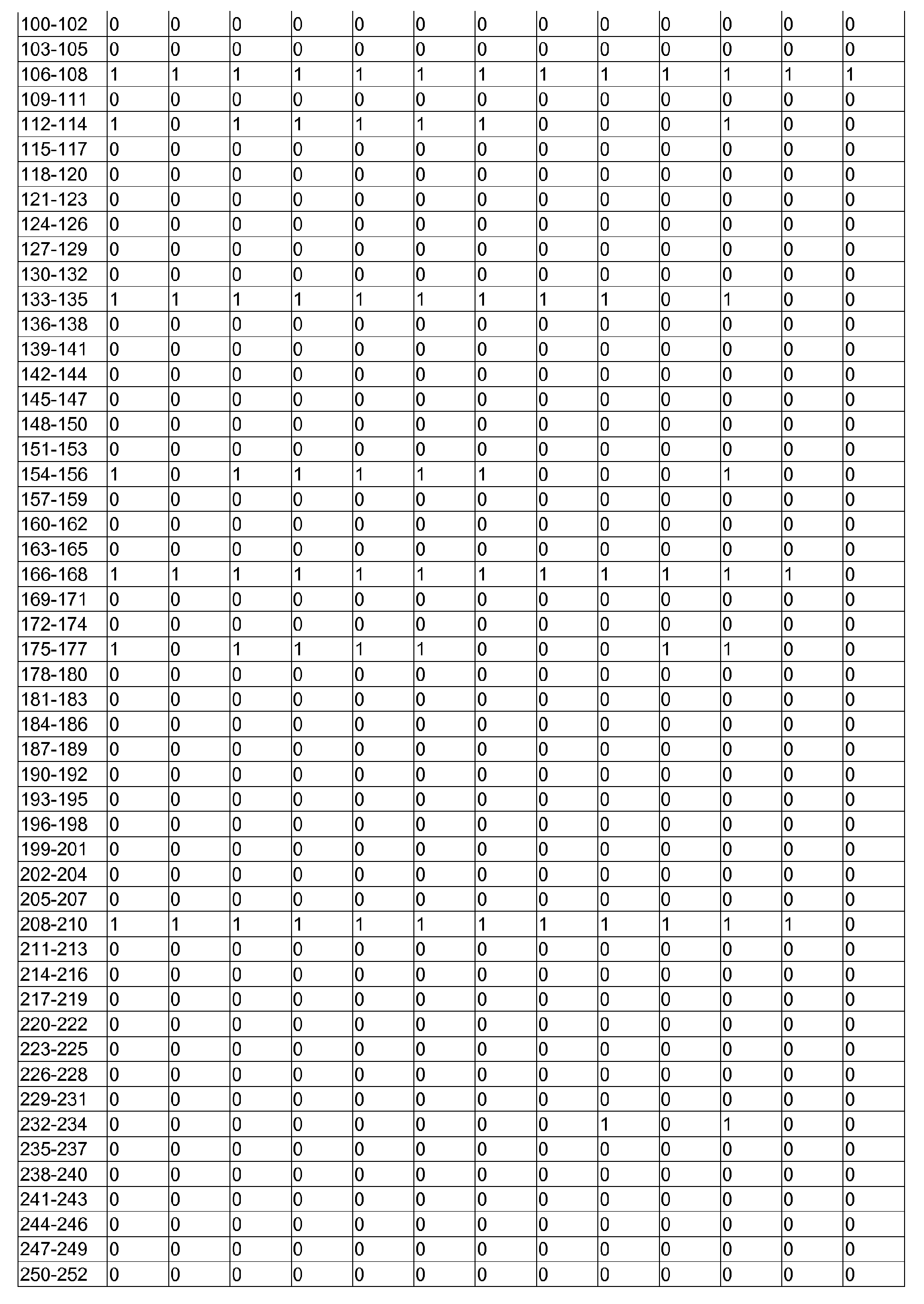

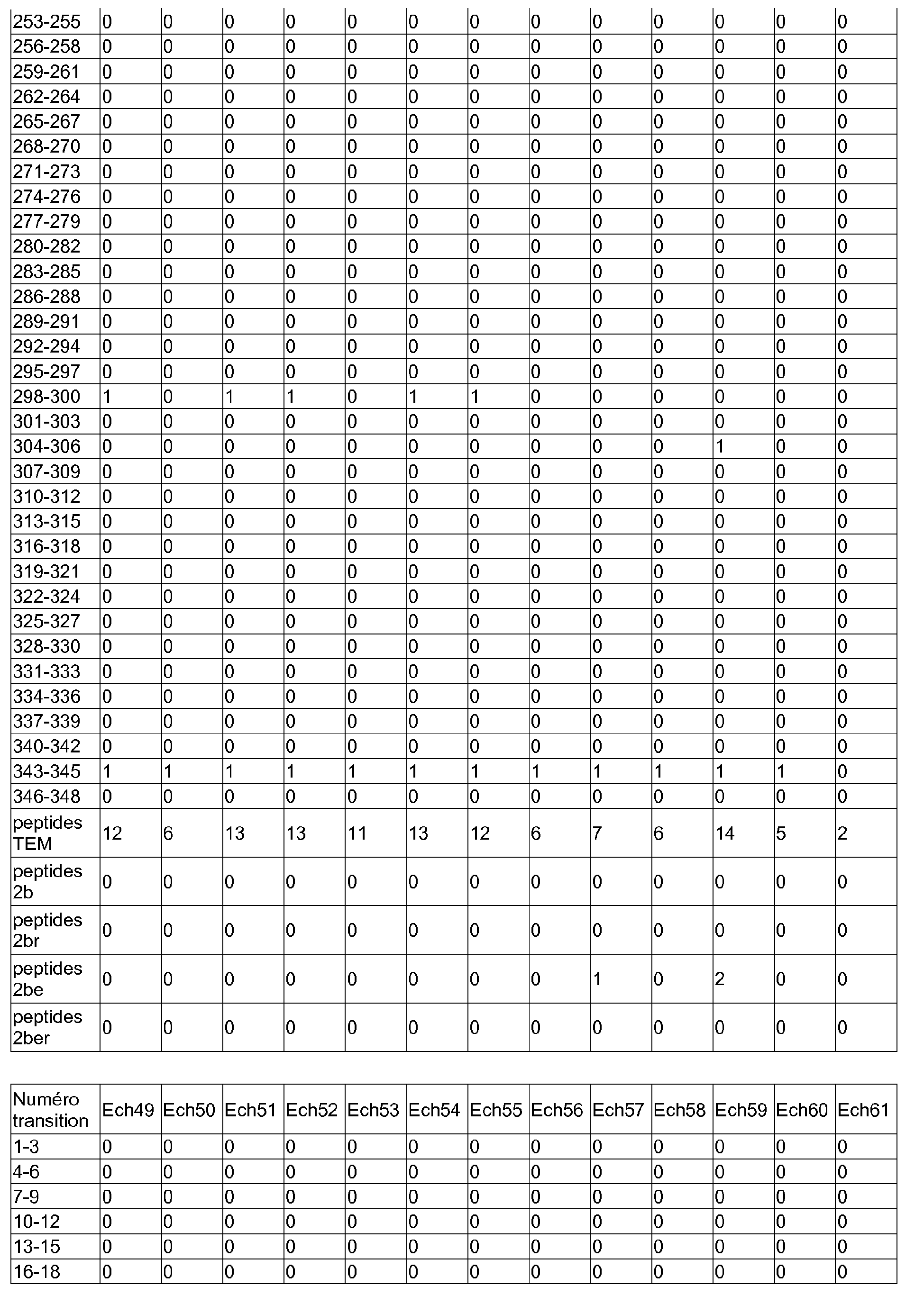

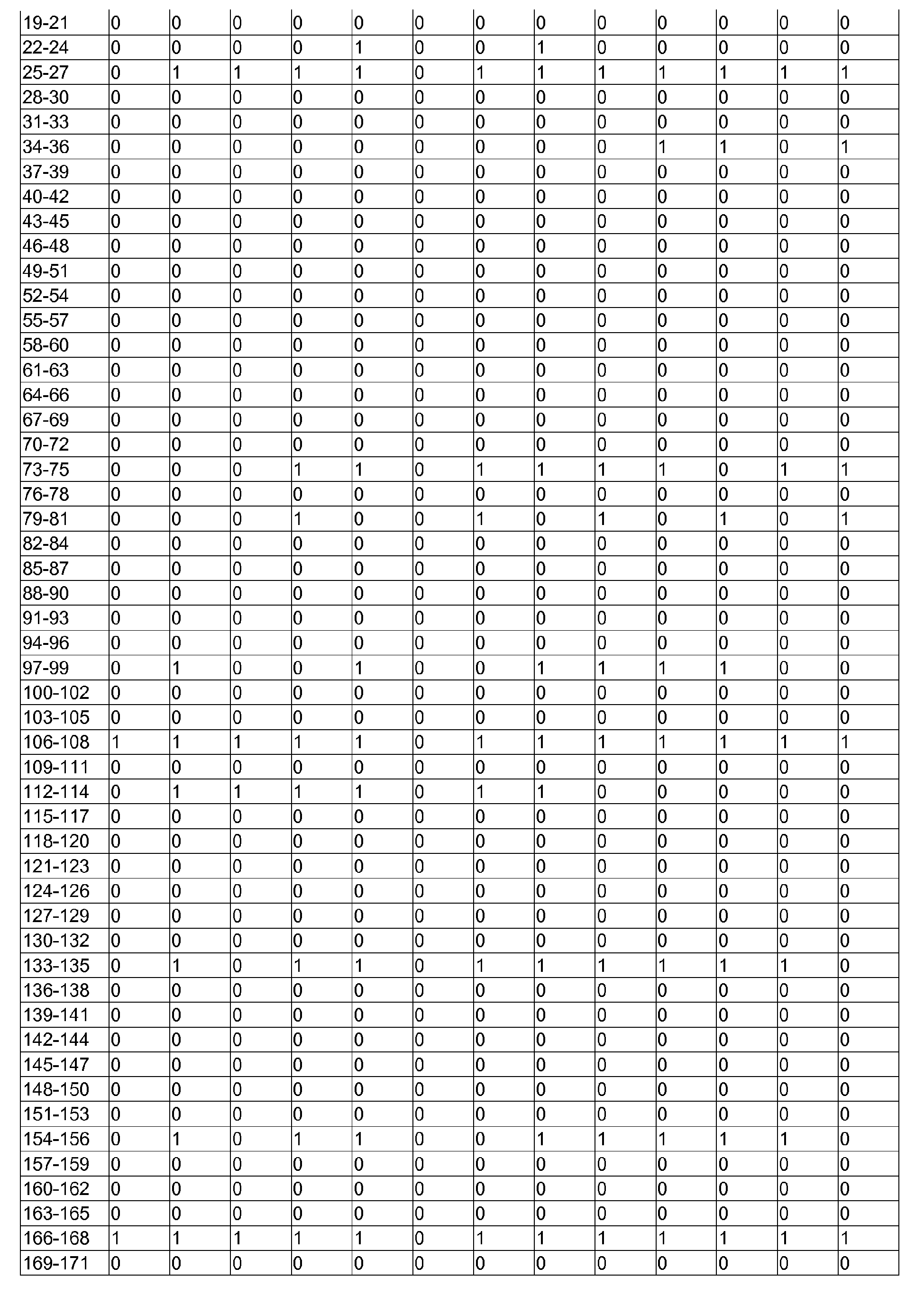

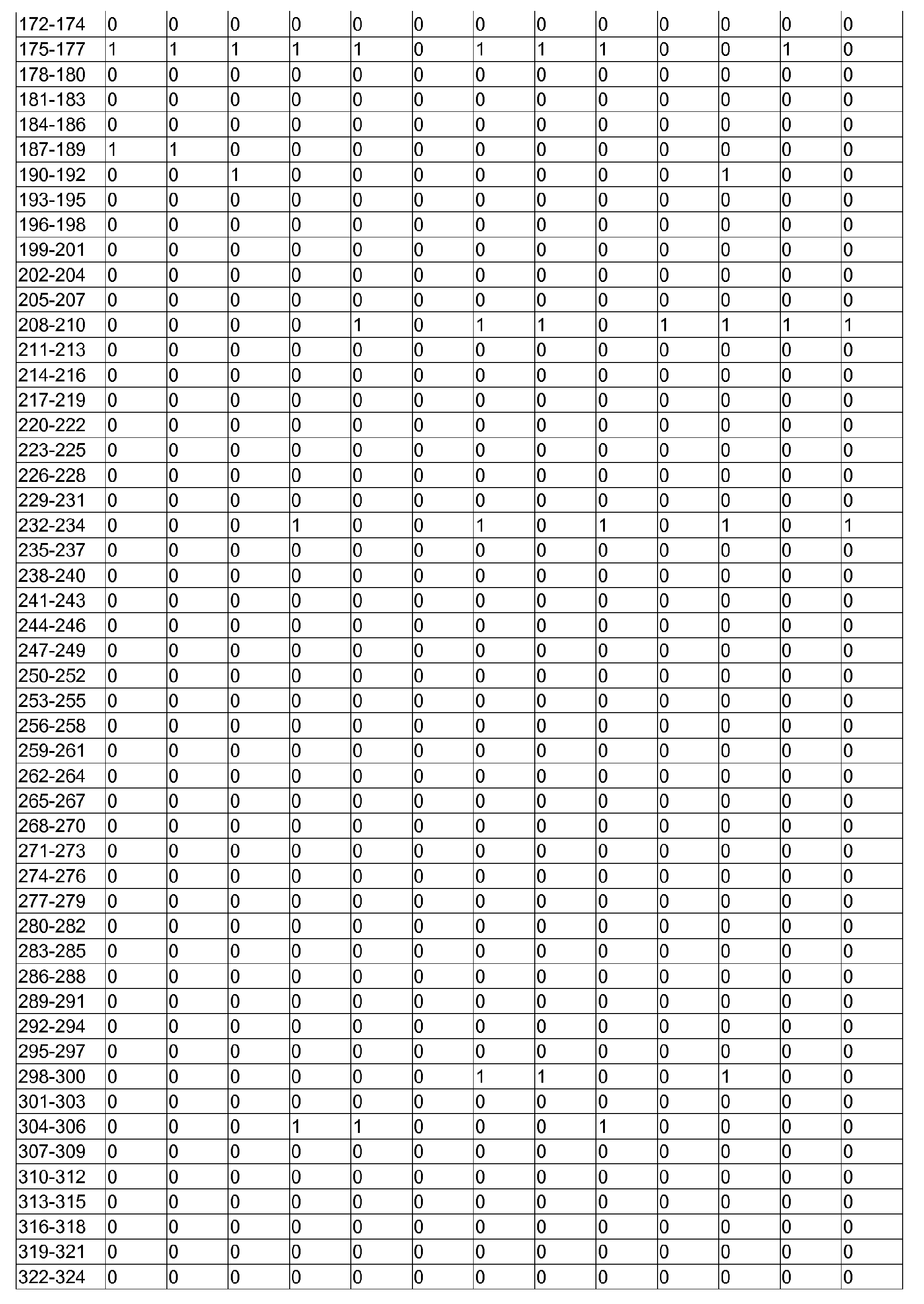

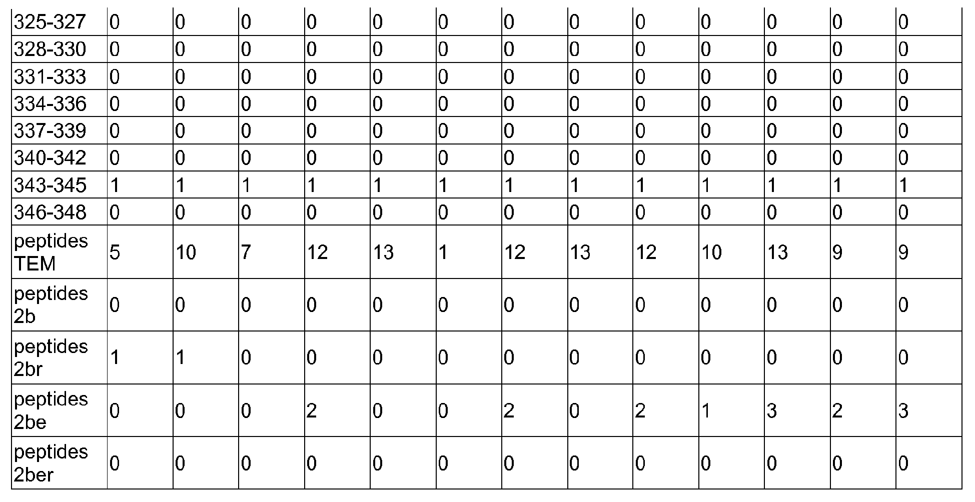

L'échantillon Ech10 ne présente aucun peptide caractéristique des TEM. La bactérie présente dans l'échantillon Ech10 peut être sensible aux céphalosporines et aux pénicillines.

Les échantillons Ech11 à Ech61 comportent au moins un peptide caractéristique des TEM. Les bactéries présentes dans les échantillons Ech11 à Ech61 expriment donc une béta-lactamase qui leur confère une résistance aux pénicillines.

Aucun peptide spécifique du phénotype 2b n'est observé, aucun échantillon testé n'est identifié comme résistant seulement aux pénicillines.

Les échantillons Ech35, Ech49 et Ech50 comportent au moins un peptide spécifique du phénotype 2br. Les échantillons Ech35, Ech49 et Ech50 sont donc résistants aux pénicillines associé à un inhibiteur de type acide clavulanique et tazobactame.

Aucun peptide spécifique du phénotype 2ber n'est observé, aucun échantillon testé n'est identifié comme appartenant uniquement à ce phénotype.

Les échantillons Ech12 à Ech21, Ech23 à Ech27, Ech29, Ech32, Ech33, Ech44, Ech46, Ech52, Ech55, Ech57 à Ech61 comportent au moins un peptide spécifique du phénotype 2be ou du phénotype 2ber. Les échantillons Ech12 à Ech21, Ech23 à Ech27, Ech29, Ech32, Ech33, Ech44, Ech46, Ech52, Ech55, Ech57 à Ech61 sont donc résistants aux pénicillines, aux céphalosporines et aux monobactames.

### Exemple 7 : Identification d'une résistance aux béta-lactamines de type CMY:

Les échantillons correspondant à une espèce pouvant comporter un mécanisme de résistance de type CMY peuvent être détectés en mettant en oeuvre la méthode suivante.

Chaque échantillon est traité selon l'exemple 5, puis analysé selon l'exemple 6 en détectant les peptides du **TABLEAU 6** en lieu et place des peptides du **TABLEAU 3.**

**TABLEAU 6 :**

| Transition numéro | Peptide | Ion fragment | Etat de charge du précurseur | Temps de rétention | (m/z) filtré en Q1 | (m/z) filtré en Q3 | Energie de collision |
|---|---|---|---|---|---|---|---|
| 1 | LSDPVTK | y6 | 2 | 10 | 380.22 | 646.34 | 22 |
| 2 | LSDPVTK | y5 | 2 | 10 | 380.22 | 559.31 | 22 |
| 3 | LSDPVTK | y4 | 2 | 10 | 380.22 | 444.28 | 22 |
| 4 | ADSIINGSDSK | y9 | 2 | 10.2 | 553.77 | 920.47 | 29 |
| 5 | ADSIINGSDSK | y8 | 2 | 10.2 | 553.77 | 833.44 | 29 |
| 6 | ADSIINGSDSK | y7 | 2 | 10.2 | 553.77 | 720.35 | 29 |
| 7 | ASWVHK | y5 | 2 | 11.6 | 364.2 | 656.35 | 21 |
| 8 | ASWVHK | y4 | 2 | 11.6 | 364.2 | 569.32 | 21 |
| 9 | ASWVHK | y3 | 2 | 11.6 | 364.2 | 383.24 | 21 |
| 10 | SYPNPVR | y6 | 2 | 11.6 | 416.72 | 745.4 | 23 |
| 11 | SYPNPVR | y5 | 2 | 11.6 | 416.72 | 582.34 | 23 |
| 12 | SYPNPVR | y4 | 2 | 11.6 | 416.72 | 485.28 | 23 |
| 13 | VEAAWR | y5 | 2 | 12.3 | 366.2 | 632.32 | 21 |
| 14 | VEAAWR | y4 | 2 | 12.3 | 366.2 | 503.27 | 21 |
| 15 | VEAAWR | y3 | 2 | 12.3 | 366.2 | 432.24 | 21 |
| 16 | QWQGIR | y5 | 2 | 13.8 | 394.21 | 659.36 | 22 |
| 17 | QWQGIR | y4 | 2 | 13.8 | 394.21 | 473.28 | 22 |
| 18 | QWQGIR | y3 | 2 | 13.8 | 394.21 | 345.22 | 22 |
| 19 | VLQPLK | y5 | 2 | 14 | 349.23 | 598.39 | 20 |
| 20 | VLQPLK | y4 | 2 | 14 | 349.23 | 485.31 | 20 |
| 21 | VLQPLK | y3 | 2 | 14 | 349.23 | 357.25 | 20 |
| 22 | SSVIDMAR | y7 | 2 | 14.2 | 439.72 | 791.41 | 24 |
| 23 | SSVIDMAR | y6 | 2 | 14.2 | 439.72 | 704.38 | 24 |
| 24 | SSVIDMAR | y5 | 2 | 14.2 | 439.72 | 605.31 | 24 |
| 25 | WVQANMDASHVQEK | y9 | 2 | 14.4 | 821.89 | 1044.48 | 41 |
| 26 | WVQANMDASHVQEK | y8 | 2 | 14.4 | 821.89 | 913.44 | 41 |
| 27 | WVQANMDASHVQEK | y7 | 2 | 14.4 | 821.89 | 798.41 | 41 |
| 28 | TLQQGIALAQSR | y9 | 2 | 15.2 | 643.36 | 943.53 | 33 |
| 29 | TLQQGIALAQSR | y8 | 2 | 15.2 | 643.36 | 815.47 | 33 |
| 30 | TLQQGIALAQSR | y7 | 2 | 15.2 | 643.36 | 758.45 | 33 |
| 31 | TEQQIADIVNR | y9 | 2 | 15.6 | 643.84 | 1056.58 | 33 |
| 32 | TEQQIADIVNR | y8 | 2 | 15.6 | 643.84 | 928.52 | 33 |
| 33 | TEQQIADIVNR | y7 | 2 | 15.6 | 643.84 | 800.46 | 33 |
| 34 | LAHTWITVPQNEQK | y9 | 2 | 16 | 832.94 | 1056.57 | 42 |
| 35 | LAHTWITVPQNEQK | y8 | 2 | 16 | 832.94 | 943.48 | 42 |
| 36 | LAHTWITVPQNEQK | y7 | 2 | 16 | 832.94 | 842.44 | 42 |
| 37 | DYAWGYR | y6 | 2 | 16.4 | 465.71 | 815.38 | 25 |
| 38 | DYAWGYR | y5 | 2 | 16.4 | 465.71 | 652.32 | 25 |
| 39 | DYAWGYR | y4 | 2 | 16.4 | 465.71 | 581.28 | 25 |
| 40 | YWPELTGK | y7 | 2 | 17.4 | 497.26 | 830.44 | 27 |
| 41 | YWPELTGK | y6 | 2 | 17.4 | 497.26 | 644.36 | 27 |
| 42 | YWPELTGK | y5 | 2 | 17.4 | 497.26 | 547.31 | 27 |
| 43 | TFNGVLGGDAIAR | y9 | 2 | 17.6 | 645.84 | 871.5 | 33 |
| 44 | TFNGVLGGDAIAR | y8 | 2 | 17.6 | 645.84 | 772.43 | 33 |
| 45 | TFNGVLGGDAIAR | y7 | 2 | 17.6 | 645.84 | 659.35 | 33 |
| 46 | NLGIVMLANK | y9 | 2 | 19.1 | 536.81 | 958.58 | 29 |
| 47 | NLGIVMLANK | y8 | 2 | 19.1 | 536.81 | 845.49 | 29 |
| 48 | NLGIVMLANK | y7 | 2 | 19.1 | 536.81 | 788.47 | 29 |
| 49 | TGSTGGFGSYVAFVPEK | y9 | 2 | 19.5 | 852.42 | 1039.55 | 43 |
| 50 | TGSTGGFGSYVAFVPEK | y8 | 2 | 19.5 | 852.42 | 952.51 | 43 |
| 51 | TGSTGGFGSYVAFVPEK | y7 | 2 | 19.5 | 852.42 | 789.45 | 43 |
| 52 | ADIANNHPVTQQTLFELGSVSK | y9 | 3 | 20 | 790.41 | 979.55 | 44 |
| 53 | ADIANNHPVTQQTLFELGSVSK | y8 | 3 | 20 | 790.41 | 866.46 | 44 |
| 54 | ADIANNHPVTQQTLFELGSVSK | y7 | 3 | 20 | 790.41 | 719.39 | 44 |
| 55 | VALAALPAVEVNPPAPAVK | y9 | 2 | 20.4 | 914.04 | 892.53 | 45 |
| 56 | VALAALPAVEVNPPAPAVK | y8 | 2 | 20.4 | 914.04 | 793.46 | 45 |
| 57 | VALAALPAVEVNPPAPAVK | y7 | 2 | 20.4 | 914.04 | 679.41 | 45 |
| 58 | LLHLATYTAGGLPLQIPDDVR | y9 | 3 | 22.4 | 755.09 | 1052.57 | 42 |
| 59 | LLHLATYTAGGLPLQIPDDVR | y8 | 3 | 22.4 | 755.09 | 955.52 | 42 |
| 60 | LLHLATYTAGGLPLQIPDDVR | y7 | 3 | 22.4 | 755.09 | 842.44 | 42 |
| 61 | TITPLMQEQAIPGMAVAVIYQGK | y9 | 3 | 22.5 | 820.44 | 948.55 | 45 |
| 62 | TITPLMQEQAIPGMAVAVIYQGK | y8 | 3 | 22.5 | 820.44 | 877.51 | 45 |
| 63 | TITPLMQEQAIPGMAVAVIYQGK | y7 | 3 | 22.5 | 820.44 | 778.45 | 45 |
| 64 | AALLHFYQNWQPQWTPGAK | y9 | 3 | 23.2 | 752.72 | 1012.52 | 42 |
| 65 | AALLHFYQNWQPQWTPGAK | y8 | 3 | 23.2 | 752.72 | 884.46 | 42 |
| 66 | AALLHFYQNWQPQWTPGAK | y7 | 3 | 23.2 | 752.72 | 787.41 | 42 |
| 67 | LYANSSIGLFGALAVK | y9 | 2 | 25.1 | 812.46 | 875.53 | 41 |
| 68 | LYANSSIGLFGALAVK | y8 | 2 | 25.1 | 812.46 | 818.51 | 41 |
| 69 | LYANSSIGLFGALAVK | y7 | 2 | 25.1 | 812.46 | 705.43 | 41 |
| 70 | SLCCALLLTASFSTFAAAK | y9 | 3 | 25.4 | 678.01 | 929.47 | 38 |
| 71 | SLCCALLLTASFSTFAAAK | y8 | 3 | 25.4 | 678.01 | 842.44 | 38 |
| 72 | SLCCALLLTASFSTFAAAK | y7 | 3 | 25.4 | 678.01 | 695.37 | 38 |
| 73 | IGDMYQGLGWEMLNWPLK | y9 | 3 | 28.5 | 717.68 | 1216.62 | 40 |
| 74 | IGDMYQGLGWEMLNWPLK | y8 | 3 | 28.5 | 717.68 | 1030.54 | 40 |
| 75 | IGDMYQGLGWEMLNWPLK | y7 | 3 | 28.5 | 717.68 | 901.5 | 40 |
| 76 | AHYFNYGVANR | y7 | 2 | 15.5 | 656.32 | 793.4 | 34 |
| 77 | AHYFNYGVANR | y8 | 2 | 15.5 | 656.32 | 940.46 | 34 |
| 78 | AHYFNYGVANR | y9 | 2 | 15.5 | 656.32 | 1103.53 | 34 |
| 79 | ANIGGVDDK | y5 | 2 | 9.6 | 444.72 | 533.26 | 25 |
| 80 | ANIGGVDDK | y6 | 2 | 9.6 | 444.72 | 590.28 | 25 |
| 81 | ANIGGVDDK | y7 | 2 | 9.6 | 444.72 | 703.36 | 25 |
| 82 | ESGSQVLFNK | y6 | 2 | 15.1 | 554.79 | 748.44 | 29 |
| 83 | ESGSQVLFNK | y7 | 2 | 15.1 | 554.79 | 835.47 | 29 |
| 84 | ESGSQVLFNK | y8 | 2 | 15.1 | 554.79 | 892.49 | 29 |
| 85 | GAMQLDDK | y5 | 2 | 11.5 | 439.21 | 618.31 | 24 |
| 86 | GAMQLDDK | y6 | 2 | 11.5 | 439.21 | 749.35 | 24 |
| 87 | GAMQLDDK | y7 | 2 | 11.5 | 439.21 | 820.39 | 24 |
| 88 | GIGIVMLANR | y6 | 2 | 18.7 | 522.31 | 703.39 | 28 |
| 89 | GIGIVMLANR | y7 | 2 | 18.7 | 522.31 | 816.48 | 28 |
| 90 | GIGIVMLANR | y8 | 2 | 18.7 | 522.31 | 873.5 | 28 |
| 91 | HAPWLK | y4 | 2 | 15 | 376.22 | 543.33 | 22 |
| 92 | HAPWLK | y5 | 2 | 15 | 376.22 | 614.37 | 22 |
| 93 | HAPWLK | b5 | 2 | 15 | 376.22 | 605.32 | 22 |
| 94 | IPGMAVAVLK | y6 | 2 | 18.1 | 499.81 | 600.41 | 27 |
| 95 | IPGMAVAVLK | y7 | 2 | 18.1 | 499.81 | 731.45 | 27 |
| 96 | IPGMAVAVLK | y8 | 2 | 18.1 | 499.81 | 788.47 | 27 |
| 97 | PVVDASIQPLLK | y7 | 2 | 19.3 | 640.38 | 798.51 | 33 |
| 98 | PVVDASIQPLLK | y8 | 2 | 19.3 | 640.38 | 869.55 | 33 |
| 99 | PVVDASIQPLLK | y9 | 2 | 19.3 | 640.38 | 984.57 | 33 |
| 100 | QAMASYAYGYSK | y7 | 2 | 15.5 | 670.3 | 851.39 | 34 |
| 101 | QAMASYAYGYSK | y8 | 2 | 15.5 | 670.3 | 938.43 | 34 |
| 102 | QAMASYAYGYSK | y9 | 2 | 15.5 | 670.3 | 1009.46 | 34 |
| 103 | QWAPVYSPGSHR | y8 | 2 | 14.8 | 692.84 | 902.45 | 35 |
| 104 | QWAPVYSPGSHR | y9 | 2 | 14.8 | 692.84 | 999.5 | 35 |
| 105 | QWAPVYSPGSHR | y10 | 2 | 14.8 | 692.84 | 1070.54 | 35 |
| 106 | QYSNPSIGLFGHLAASSLK | y11 | 2 | 22 | 995.52 | 1143.65 | 49 |
| 107 | QYSNPSIGLFGHLAASSLK | y12 | 2 | 22 | 995.52 | 1200.67 | 49 |
| 108 | QYSNPSIGLFGHLAASSLK | y11 | 2 | 22 | 664.02 | 1143.65 | 37 |
| 109 | TGSTNGFGAYVAFVPAR | y9 | 2 | 19.4 | 857.93 | 993.55 | 43 |
| 110 | TGSTNGFGAYVAFVPAR | y10 | 2 | 19.4 | 857.93 | 1050.57 | 43 |
| 111 | TGSTNGFGAYVAFVPAR | y11 | 2 | 19.4 | 857.93 | 1197.64 | 43 |
| 112 | TLTATLGAYAVVK | y8 | 2 | 18.3 | 654.38 | 820.49 | 34 |
| 113 | TLTATLGAYAVVK | y9 | 2 | 18.3 | 654.38 | 921.54 | 34 |
| 114 | TLTATLGAYAVVK | y10 | 2 | 18.3 | 654.38 | 992.58 | 34 |
| 115 | VNPGMLADEAYGIK | y8 | 2 | 18.8 | 739.37 | 866.43 | 38 |
| 116 | VNPGMLADEAYGIK | y9 | 2 | 18.8 | 739.37 | 979.51 | 38 |
| 117 | VNPGMLADEAYGIK | y10 | 2 | 18.8 | 739.37 | 1110.55 | 38 |
| 118 | PSGMSYEEAMTR | y10 | 2 | 15.5 | 679.79 | 1174.49 | 35 |
| 119 | PSGMSYEEAMTR | y9 | 2 | 15.5 | 679.79 | 1117.47 | 35 |
| 120 | PSGMSYEEAMTR | y8 | 2 | 15.5 | 679.79 | 986.42 | 35 |
| 121 | PYYFTWGK | y7 | 2 | 20.3 | 531.26 | 964.46 | 29 |
| 122 | PYYFTWGK | y6 | 2 | 20.3 | 531.26 | 801.39 | 29 |
| 123 | PYYFTWGK | y5 | 2 | 20.3 | 531.26 | 638.33 | 29 |

Les aires obtenues pour chacune des transitions et pour chacun des microorganismes étudiés ont été mesurées. Lorsque les aires des 3 transitions d'un même peptide sont supérieures ou égales à 2500 la détection du peptide est considérée comme positive et est noté « 1 ». Lorsque au moins une transition comporte une aire inférieure à 2500, le peptide correspondant est considéré comme non détecté et est noté « 0 ».

### Exemple 8 : Identification d'une résistance aux béta-lactamines de type CTX-M:

Les échantillons correspondant à une espèce pouvant comporter un mécanisme de résistance de type CTX-M peuvent être détectés en mettant en oeuvre la méthode suivante.

Chaque échantillon est traité selon l'exemple 5, puis analysé selon l'exemple 6 en détectant les peptides du **TABLEAU 7** en lieu et place des peptides du **TABLEAU 3.**

**TABLEAU 7:**

| Transition numéro | Peptide | Etat de charge du précurseur | ion fragment de première génération | Temps de rétention (minutes) | (m/z) filtré en Q1 | (m/z) filtré en Q3 | Energie de collision (eV) |
|---|---|---|---|---|---|---|---|
| 1 | AGLPK | 2 | y4 | 9.5 | 243.16 | 414.27 | 16 |
| 2 | AGLPK | 2 | y3 | 9.5 | 243.16 | 357.25 | 16 |
| 3 | AGLPK | 2 | y2 | 9.5 | 243.16 | 244.17 | 16 |
| 4 | AGLPTSWTVGDK | 2 | y9 | 16.6 | 616.32 | 990.49 | 32 |
| 5 | AGLPTSWTVGDK | 2 | y8 | 16.6 | 616.32 | 893.44 | 32 |
| 6 | AGLPTSWTVGDK | 2 | y7 | 16.6 | 616.32 | 792.39 | 32 |
| 7 | AIGDETFR | 2 | y7 | 14 | 454.73 | 837.41 | 25 |
| 8 | AIGDETFR | 2 | y6 | 14 | 454.73 | 724.33 | 25 |
| 9 | AIGDETFR | 2 | y5 | 14 | 454.73 | 667.3 | 25 |
| 10 | ALAETQR | 2 | y6 | 7.4 | 394.72 | 717.39 | 22 |
| 11 | ALAETQR | 2 | y5 | 7.4 | 394.72 | 604.3 | 22 |
| 12 | ALAETQR | 2 | y4 | 7.4 | 394.72 | 533.27 | 22 |
| 13 | ALGDSQR | 2 | y6 | 7 | 373.69 | 675.34 | 21 |
| 14 | ALGDSQR | 2 | y5 | 7 | 373.69 | 562.26 | 21 |
| 15 | ALGDSQR | 2 | y4 | 7 | 373.69 | 505.24 | 21 |
| 16 | AMAQTLR | 2 | y6 | 12.1 | 395.72 | 719.39 | 22 |
| 17 | AMAQTLR | 2 | y5 | 12.1 | 395.72 | 588.35 | 22 |
| 18 | AMAQTLR | 2 | y4 | 12.1 | 395.72 | 517.31 | 22 |
| 19 | APLILVTYFTQPQPK | 2 | y9 | 22.4 | 858.49 | 1109.6 | 43 |
| 20 | APLILVTYFTQPQPK | 2 | y8 | 22.4 | 858.49 | 1008.5 | 43 |
| 21 | APLILVTYFTQPQPK | 2 | y7 | 22.4 | 858.49 | 845.45 | 43 |
| 22 | APLVLVTYFTQPQQNAESR | 3 | y9 | 21.4 | 721.38 | 1057.5 | 40 |
| 23 | APLVLVTYFTQPQQNAESR | 3 | y8 | 21.4 | 721.38 | 929.44 | 40 |
| 24 | APLVLVTYFTQPQQNAESR | 3 | y7 | 21.4 | 721.38 | 832.39 | 40 |
| 25 | AQLVTWLK | 2 | y7 | 21 | 479.79 | 887.53 | 26 |
| 26 | AQLVTWLK | 2 | y6 | 21 | 479.79 | 759.48 | 26 |
| 27 | AQLVTWLK | 2 | y5 | 21 | 479.79 | 646.39 | 26 |
| 28 | AQLVTWMK | 2 | y7 | 19 | 488.77 | 905.49 | 27 |
| 29 | AQLVTWMK | 2 | y6 | 19 | 488.77 | 777.43 | 27 |
| 30 | AQLVTWMK | 2 | y5 | 19 | 488.77 | 664.35 | 27 |
| 31 | DILAAAAK | 2 | y7 | 12.1 | 386.73 | 657.43 | 22 |
| 32 | DILAAAAK | 2 | y6 | 12.1 | 386.73 | 544.35 | 22 |
| 33 | DILAAAAK | 2 | y5 | 12.1 | 386.73 | 431.26 | 22 |
| 34 | DTTSPR | 2 | y5 | 4.6 | 338.67 | 561.3 | 20 |
| 35 | DTTSPR | 2 | y4 | 4.6 | 338.67 | 460.25 | 20 |
| 36 | DTTSPR | 2 | y3 | 4.6 | 338.67 | 359.2 | 20 |
| 37 | DTTTPLAMAQTLK | 2 | y9 | 17 | 695.87 | 972.55 | 36 |
| 38 | DTTTPLAMAQTLK | 2 | y8 | 17 | 695.87 | 875.5 | 36 |
| 39 | DTTTPLAMAQTLK | 2 | y7 | 17 | 695.87 | 762.42 | 36 |
| 40 | DTTTPR | 2 | y5 | 4.8 | 345.67 | 575.31 | 20 |
| 41 | DTTTPR | 2 | y4 | 4.8 | 345.67 | 474.27 | 20 |
| 42 | DTTTPR | 2 | y3 | 4.8 | 345.67 | 373.22 | 20 |
| 43 | DVLAAAAK | 2 | y7 | 10.5 | 379.72 | 643.41 | 22 |
| 44 | DVLAAAAK | 2 | y6 | 10.5 | 379.72 | 544.35 | 22 |
| 45 | DVLAAAAK | 2 | y5 | 10.5 | 379.72 | 431.26 | 22 |
| 46 | DVLASAAK | 2 | y7 | 10.8 | 387.72 | 659.41 | 22 |
| 47 | DVLASAAK | 2 | y6 | 10.8 | 387.72 | 560.34 | 22 |
| 48 | DVLASAAK | 2 | y5 | 10.8 | 387.72 | 447.26 | 22 |
| 49 | DVLASAAR | 2 | y7 | 11.4 | 401.72 | 687.41 | 23 |
| 50 | DVLASAAR | 2 | y6 | 11.4 | 401.72 | 588.35 | 23 |
| 51 | DVLASAAR | 2 | y5 | 11.4 | 401.72 | 475.26 | 23 |
| 52 | FAMCSTSK | 2 | y7 | 11.1 | 466.2 | 784.33 | 26 |
| 53 | FAMCSTSK | 2 | y6 | 11.1 | 466.2 | 713.3 | 26 |
| 54 | FAMCSTSK | 2 | y5 | 11.1 | 466.2 | 582.26 | 26 |
| 55 | FPMCSTSK | 2 | y7 | 12.1 | 479.21 | 810.35 | 26 |
| 56 | FPMCSTSK | 2 | y6 | 12.1 | 479.21 | 713.3 | 26 |
| 57 | FPMCSTSK | 2 | y5 | 12.1 | 479.21 | 582.26 | 26 |
| 58 | GNTTGAASIQAGLPASWVVGDK | 3 | y9 | 19.5 | 700.7 | 958.5 | 39 |
| 59 | GNTTGAASIQAGLPASWVVGDK | 3 | y8 | 19.5 | 700.7 | 861.45 | 39 |
| 60 | GNTTGAASIQAGLPASWVVGDK | 3 | y7 | 19.5 | 700.7 | 790.41 | 39 |
| 61 | GNTTGAASIQAGLPTSWVVGDK | 3 | y9 | 19.3 | 710.7 | 988.51 | 40 |
| 62 | GNTTGAASIQAGLPTSWVVGDK | 3 | y8 | 19.3 | 710.7 | 891.46 | 40 |
| 63 | GNTTGAASIQAGLPTSWVVGDK | 3 | y7 | 19.3 | 710.7 | 790.41 | 40 |
| 64 | GNTTGAASIR | 2 | y9 | 9 | 474.25 | 890.47 | 26 |
| 65 | GNTTGAASIR | 2 | y8 | 9 | 474.25 | 776.43 | 26 |
| 66 | GNTTGAASIR | 2 | y7 | 9 | 474.25 | 675.38 | 26 |
| 67 | GNTTGSASIR | 2 | y9 | 8.5 | 482.25 | 906.46 | 26 |
| 68 | GNTTGSASIR | 2 | y8 | 8.5 | 482.25 | 792.42 | 26 |
| 69 | GNTTGSASIR | 2 | y7 | 8.5 | 482.25 | 691.37 | 26 |
| 70 | HLLNQR | 2 | y5 | 10.2 | 390.73 | 643.39 | 22 |
| 71 | HLLNQR | 2 | y4 | 10.2 | 390.73 | 530.3 | 22 |
| 72 | HLLNQR | 2 | y3 | 10.2 | 390.73 | 417.22 | 22 |
| 73 | LAALEK | 2 | y5 | 11 | 322.7 | 531.31 | 19 |
| 74 | LAALEK | 2 | y4 | 11 | 322.7 | 460.28 | 19 |
| 75 | LAALEK | 2 | y3 | 11 | 322.7 | 389.24 | 19 |
| 76 | LAELER | 2 | y5 | 12.1 | 365.71 | 617.33 | 21 |
| 77 | LAELER | 2 | y4 | 12.1 | 365.71 | 546.29 | 21 |
| 78 | LAELER | 2 | y3 | 12.1 | 365.71 | 417.25 | 21 |
| 79 | LGVALIDTADNTQVLYR | 2 | y9 | 21.4 | 931.5 | 1079.6 | 46 |
| 80 | LGVALIDTADNTQVLYR | 2 | y8 | 21.4 | 931.5 | 1008.5 | 46 |
| 81 | LGVALIDTADNTQVLYR | 2 | y7 | 21.4 | 931.5 | 893.48 | 46 |
| 82 | LGVALINTADNSQILYR | 2 | y9 | 21.1 | 931.01 | 1079.6 | 46 |
| 83 | LGVALINTADNSQILYR | 2 | y8 | 21.1 | 931.01 | 1008.5 | 46 |
| 84 | LGVALINTADNSQILYR | 2 | y7 | 21.1 | 931.01 | 893.48 | 46 |
| 85 | LIAHLGGPDK | 2 | y9 | 12.7 | 510.8 | 907.5 | 27 |
| 86 | LIAHLGGPDK | 2 | y8 | 12.7 | 510.8 | 794.42 | 27 |
| 87 | LIAHLGGPDK | 2 | y7 | 12.7 | 510.8 | 723.38 | 27 |
| 88 | LIAHVGGPASVTAFAR | 2 | y9 | 17.5 | 783.94 | 919.5 | 39 |
| 89 | LIAHVGGPASVTAFAR | 2 | y8 | 17.5 | 783.94 | 822.45 | 39 |
| 90 | LIAHVGGPASVTAFAR | 2 | y7 | 17.5 | 783.94 | 751.41 | 39 |
| 91 | LIAQLGGPGGVTAFAR | 2 | y9 | 19.3 | 764.44 | 875.47 | 39 |
| 92 | LIAQLGGPGGVTAFAR | 2 | y8 | 19.3 | 764.44 | 778.42 | 39 |
| 93 | LIAQLGGPGGVTAFAR | 2 | y7 | 19.3 | 764.44 | 721.4 | 39 |
| 94 | NLTLGK | 2 | y5 | 12.2 | 323.2 | 531.35 | 19 |
| 95 | NLTLGK | 2 | y4 | 12.2 | 323.2 | 418.27 | 19 |
| 96 | NLTLGK | 2 | y3 | 12.2 | 323.2 | 317.22 | 19 |
| 97 | QLGDETFR | 2 | y7 | 14.4 | 483.24 | 837.41 | 26 |
| 98 | QLGDETFR | 2 | y6 | 14.4 | 483.24 | 724.33 | 26 |
| 99 | QLGDETFR | 2 | y5 | 14.4 | 483.24 | 667.3 | 26 |
| 100 | QLLNQPVEIK | 2 | y9 | 16.2 | 591.35 | 1053.6 | 31 |
| 101 | QLLNQPVEIK | 2 | y8 | 16.2 | 591.35 | 940.55 | 31 |
| 102 | QLLNQPVEIK | 2 | y7 | 16.2 | 591.35 | 827.46 | 31 |
| 103 | QLTLGHALGETQR | 2 | y9 | 14.9 | 712.39 | 968.49 | 36 |
| 104 | QLTLGHALGETQR | 2 | y8 | 14.9 | 712.39 | 911.47 | 36 |
| 105 | QLTLGHALGETQR | 2 | y7 | 14.9 | 712.39 | 774.41 | 36 |
| 106 | QSESDK | 2 | y5 | 1.7 | 347.16 | 565.25 | 20 |
| 107 | QSESDK | 2 | y4 | 1.7 | 347.16 | 478.21 | 20 |
| 108 | QSESDK | 2 | y3 | 1.7 | 347.16 | 349.17 | 20 |
| 109 | QSETQK | 2 | y5 | 3.7 | 360.68 | 592.29 | 21 |
| 110 | QSETQK | 2 | y4 | 3.7 | 360.68 | 505.26 | 21 |
| 111 | QSETQK | 2 | y3 | 3.7 | 360.68 | 376.22 | 21 |
| 112 | QSGGR | 2 | y4 | 5.5 | 252.63 | 376.19 | 16 |
| 113 | QSGGR | 2 | y3 | 5.5 | 252.63 | 289.16 | 16 |
| 114 | QSGGR | 2 | b4 | 5.5 | 252.63 | 330.14 | 16 |
| 115 | SDLVNYNPIAEK | 2 | y9 | 17.1 | 681.85 | 1047.6 | 35 |
| 116 | SDLVNYNPIAEK | 2 | y8 | 17.1 | 681.85 | 948.48 | 35 |
| 117 | SDLVNYNPIAEK | 2 | y7 | 17.1 | 681.85 | 834.44 | 35 |
| 118 | SESEPNLLNQR | 2 | y9 | 13.3 | 643.82 | 1070.6 | 33 |
| 119 | SESEPNLLNQR | 2 | y8 | 13.3 | 643.82 | 983.53 | 33 |
| 120 | SESEPNLLNQR | 2 | y7 | 13.3 | 643.82 | 854.48 | 33 |
| 121 | SLGDETFR | 2 | y7 | 14.6 | 462.72 | 837.41 | 25 |
| 122 | SLGDETFR | 2 | y6 | 14.6 | 462.72 | 724.33 | 25 |
| 123 | SLGDETFR | 2 | y5 | 14.6 | 462.72 | 667.3 | 25 |
| 124 | SSGGR | 2 | y4 | 5.9 | 232.12 | 376.19 | 15 |
| 125 | SSGGR | 2 | y3 | 5.9 | 232.12 | 289.16 | 15 |
| 126 | SWVVGDK | 2 | y6 | 14.2 | 395.71 | 703.38 | 22 |
| 127 | SWVVGDK | 2 | y5 | 14.2 | 395.71 | 517.3 | 22 |
| 128 | SWVVGDK | 2 | y4 | 14.2 | 395.71 | 418.23 | 22 |
| 129 | TEPTLNTAIPGDPR | 2 | y9 | 15.6 | 741.38 | 940.48 | 38 |
| 130 | TEPTLNTAIPGDPR | 2 | y8 | 15.6 | 741.38 | 826.44 | 38 |
| 131 | TEPTLNTAIPGDPR | 2 | y7 | 15.6 | 741.38 | 725.39 | 38 |
| 132 | TGSGDYGTTNDIAVIWPK | 2 | y8 | 19.9 | 947.96 | 941.55 | 47 |
| 133 | TGSGDYGTTNDIAVIWPK | 2 | y7 | 19.9 | 947.96 | 826.52 | 47 |
| 134 | TGSGDYGTTNDIAVIWPK | 2 | y6 | 19.9 | 947.96 | 713.43 | 47 |
| 135 | TGSGDYGTTNDIAVIWPQGR | 3 | y9 | 19.7 | 703.34 | 1039.6 | 39 |
| 136 | TGSGDYGTTNDIAVIWPQGR | 3 | y8 | 19.7 | 703.34 | 926.52 | 39 |
| 137 | TGSGDYGTTNDIAVIWPQGR | 3 | y7 | 19.7 | 703.34 | 855.48 | 39 |
| 138 | TGSGGYGTTNDIAVIWPK | 2 | y9 | 19.5 | 918.96 | 1055.6 | 45 |
| 139 | TGSGGYGTTNDIAVIWPK | 2 | y8 | 19.5 | 918.96 | 941.55 | 45 |
| 140 | TGSGGYGTTNDIAVIWPK | 2 | y7 | 19.5 | 918.96 | 826.52 | 45 |
| 141 | VMAAAAVLK | 2 | y8 | 15.3 | 437.26 | 774.45 | 24 |
| 142 | VMAAAAVLK | 2 | y7 | 15.3 | 437.26 | 643.41 | 24 |
| 143 | VMAAAAVLK | 2 | y6 | 15.3 | 437.26 | 572.38 | 24 |
| 144 | VMAVAAVLK | 2 | y8 | 16.3 | 451.28 | 802.49 | 25 |
| 145 | VMAVAAVLK | 2 | y7 | 16.3 | 451.28 | 671.45 | 25 |
| 146 | VMAVAAVLK | 2 | y6 | 16.3 | 451.28 | 600.41 | 25 |
| 147 | VTAFAR | 2 | y5 | 11.6 | 332.69 | 565.31 | 20 |
| 148 | VTAFAR | 2 | y4 | 11.6 | 332.69 | 464.26 | 20 |
| 149 | VTAFAR | 2 | y3 | 11.6 | 332.69 | 393.22 | 20 |

Les aires obtenues pour chacune des transitions et pour chacun des microorganismes étudiés ont été mesurées. Lorsque les aires des 3 transitions d'un même peptide sont supérieures ou égales à 2500, la détection du peptide est considérée comme positive et est noté « 1 ». Lorsque au moins une transition comporte une aire inférieure à 2500, le peptide correspondant est considéré comme non détecté et est noté « 0 ».

### Exemple 9 : Identification d'une résistance aux béta-lactamines de type SHV:

Les échantillons correspondant à une espèce pouvant comporter un mécanisme de résistance de type SHV peuvent être détectés en mettant en oeuvre la méthode suivante.

Chaque échantillon est traité selon l'exemple 5, puis analysé selon l'exemple 6 en détectant les peptides du **TABLEAU 8** en lieu et place des peptides du **TABLEAU 3.**

**TABLEAU 8 :**

| Transition numéro | Peptide | Etat de charge du précurseur | ion fragment de première génération | Temps de rétention (minutes) | (m/z) filtré en Q1 | (m/z) filtré en Q3 | Energie de collision (eV) |
|---|---|---|---|---|---|---|---|
| 1 | AGAGER | 2 | y5 | 5 | 280.64 | 489.24 | 17 |
| 2 | AGAGER | 2 | y4 | 5 | 280.64 | 432.22 | 17 |
| 3 | AGAGER | 2 | y3 | 5 | 280.64 | 361.18 | 17 |
| 4 | ATTTPASMAATLR | 2 | y9 | 15.1 | 646.34 | 917.49 | 33 |
| 5 | ATTTPASMAATLR | 2 | y8 | 15.1 | 646.34 | 820.43 | 33 |
| 6 | ATTTPASMAATLR | 2 | y7 | 15.1 | 646.34 | 749.4 | 33 |
| 7 | CIISLLATLPLAVHASPQPLEQIK | 3 | y9 | 27.3 | 871.5 | 1039.6 | 48 |
| 8 | CIISLLATLPLAVHASPQPLEQIK | 3 | y8 | 27.3 | 871.5 | 952.55 | 48 |
| 9 | CIISLLATLPLAVHASPQPLEQIK | 3 | y7 | 27.3 | 871.5 | 855.49 | 48 |
| 10 | DMPASMAER | 2 | y8 | 12 | 504.22 | 892.4 | 27 |
| 11 | DMPASMAER | 2 | y7 | 12 | 504.22 | 761.36 | 27 |
| 12 | DMPASMAER | 2 | y6 | 12 | 504.22 | 664.31 | 27 |
| 13 | DSPASMAER | 2 | y8 | 8.8 | 482.21 | 848.39 | 26 |
| 14 | DSPASMAER | 2 | y7 | 8.8 | 482.21 | 761.36 | 26 |
| 15 | DSPASMAER | 2 | y6 | 8.8 | 482.21 | 664.31 | 26 |
| 16 | DTLASMAER | 2 | y8 | 13.3 | 497.24 | 878.44 | 27 |
| 17 | DTLASMAER | 2 | y7 | 13.3 | 497.24 | 777.39 | 27 |
| 18 | DTLASMAER | 2 | y6 | 13.3 | 497.24 | 664.31 | 27 |
| 19 | DTPASMAER | 2 | y8 | 9.2 | 489.22 | 862.41 | 27 |
| 20 | DTPASMAER | 2 | y7 | 9.2 | 489.22 | 761.36 | 27 |
| 21 | DTPASMAER | 2 | y6 | 9.2 | 489.22 | 664.31 | 27 |
| 22 | DTPASMAK | 2 | y7 | 8.5 | 410.7 | 705.36 | 23 |
| 23 | DTPASMAK | 2 | y6 | 8.5 | 410.7 | 604.31 | 23 |
| 24 | DTPASMAK | 2 | y5 | 8.5 | 410.7 | 507.26 | 23 |
| 25 | DTTTPASMAATLR | 2 | y9 | 15.2 | 668.33 | 917.49 | 34 |
| 26 | DTTTPASMAATLR | 2 | y8 | 15.2 | 668.33 | 820.43 | 34 |
| 27 | DTTTPASMAATLR | 2 | y7 | 15.2 | 668.33 | 749.4 | 34 |
| 28 | DTTTPASMAGTLR | 2 | y9 | 15.1 | 661.32 | 903.47 | 34 |
| 29 | DTTTPASMAGTLR | 2 | y8 | 15.1 | 661.32 | 806.42 | 34 |
| 30 | DTTTPASMAGTLR | 2 | y7 | 15.1 | 661.32 | 735.38 | 34 |
| 31 | DTTTPASMTATLR | 2 | y9 | 14.9 | 683.34 | 947.5 | 35 |
| 32 | DTTTPASMTATLR | 2 | y8 | 14.9 | 683.34 | 850.45 | 35 |
| 33 | DTTTPASMTATLR | 2 | y7 | 14.9 | 683.34 | 779.41 | 35 |
| 34 | FPMISTFK | 2 | y7 | 19.7 | 485.76 | 823.44 | 26 |
| 35 | FPMISTFK | 2 | y6 | 19.7 | 485.76 | 726.39 | 26 |
| 36 | FPMISTFK | 2 | y5 | 19.7 | 485.76 | 595.34 | 26 |
| 37 | FPMMSTFK | 2 | y7 | 19.3 | 494.74 | 841.39 | 27 |
| 38 | FPMMSTFK | 2 | y6 | 19.3 | 494.74 | 744.34 | 27 |
| 39 | FPMMSTFK | 2 | y5 | 19.3 | 494.74 | 613.3 | 27 |
| 40 | GIVALLGGNIK | 2 | y9 | 18.4 | 527.83 | 884.56 | 28 |
| 41 | GIVALLGGNIK | 2 | y8 | 18.4 | 527.83 | 785.49 | 28 |
| 42 | GIVALLGGNIK | 2 | y7 | 18.4 | 527.83 | 714.45 | 28 |
| 43 | GIVALLGPDNK | 2 | y8 | 16.9 | 548.82 | 827.46 | 29 |
| 44 | GIVALLGPDNK | 2 | y7 | 16.9 | 548.82 | 756.43 | 29 |
| 45 | GIVALLGPDNK | 2 | y6 | 16.9 | 548.82 | 643.34 | 29 |
| 46 | GIVALLGPNHK | 2 | y9 | 16.3 | 559.84 | 948.56 | 30 |
| 47 | GIVALLGPNHK | 2 | y8 | 16.3 | 559.84 | 849.49 | 30 |
| 48 | GIVALLGPNHK | 2 | y7 | 16.3 | 559.84 | 778.46 | 30 |
| 49 | GIVALLGPNNK | 2 | y9 | 16.5 | 548.33 | 925.55 | 29 |
| 50 | GIVALLGPNNK | 2 | y8 | 16.5 | 548.33 | 826.48 | 29 |
| 51 | GIVALLGPNNK | 2 | y7 | 16.5 | 548.33 | 755.44 | 29 |
| 52 | GIVALLGPNNNAER | 2 | y9 | 16.4 | 719.39 | 984.49 | 37 |
| 53 | GIVALLGPNNNAER | 2 | y8 | 16.4 | 719.39 | 871.4 | 37 |
| 54 | GIVALLGPNNNAER | 2 | y7 | 16.4 | 719.39 | 814.38 | 37 |
| 55 | GIVALR | 2 | y5 | 14.2 | 314.71 | 571.39 | 19 |
| 56 | GIVALR | 2 | y4 | 14.2 | 314.71 | 458.31 | 19 |
| 57 | GIVALR | 2 | y3 | 14.2 | 314.71 | 359.24 | 19 |
| 58 | GPNNK | 2 | y4 | 5.3 | 265.14 | 472.25 | 17 |
| 59 | GPNNK | 2 | y3 | 5.3 | 265.14 | 375.2 | 17 |
| 60 | GPNNK | 2 | b4 | 5.3 | 265.14 | 383.17 | 17 |
| 61 | GTTTPASMAATLR | 2 | y9 | 15.3 | 639.33 | 917.49 | 33 |
| 62 | GTTTPASMAATLR | 2 | y8 | 15.3 | 639.33 | 820.43 | 33 |
| 63 | GTTTPASMAATLR | 2 | y7 | 15.3 | 639.33 | 749.4 | 33 |
| 64 | HLADGMTVGELCAAAITMSDNSAAK | 3 | y9 | 20 | 845.39 | 924.41 | 46 |
| 65 | HLADGMTVGELCAAAITMSDNSAAK | 3 | y8 | 20 | 845.39 | 823.36 | 46 |
| 66 | HLADGMTVGELCAAAITMSDNSAAK | 3 | y7 | 20 | 845.39 | 692.32 | 46 |
| 67 | HLLQWMVDDR | 2 | y9 | 21.2 | 656.83 | 1175.6 | 34 |
| 68 | HLLQWMVDDR | 2 | y8 | 21.2 | 656.83 | 1062.5 | 34 |
| 69 | HLLQWMVDDR | 2 | y7 | 21.2 | 656.83 | 949.42 | 34 |
| 70 | IHYLQQDLVDYSPVSEK | 3 | y9 | 19.9 | 678.68 | 1023.5 | 38 |
| 71 | IHYLQQDLVDYSPVSEK | 3 | y8 | 19.9 | 678.68 | 924.43 | 38 |
| 72 | IHYLQQDLVDYSPVSEK | 3 | y7 | 19.9 | 678.68 | 809.4 | 38 |
| 73 | IVVIYLR | 2 | y6 | 19.1 | 438.29 | 762.49 | 24 |
| 74 | IVVIYLR | 2 | y5 | 19.1 | 438.29 | 663.42 | 24 |
| 75 | IVVIYLR | 2 | y4 | 19.1 | 438.29 | 564.35 | 24 |
| 76 | LCIISLLAALPLAVHASPQPLEQIK | 3 | y9 | 29.8 | 899.19 | 1039.6 | 49 |
| 77 | LCIISLLAALPLAVHASPQPLEQIK | 3 | y8 | 29.8 | 899.19 | 952.55 | 49 |
| 78 | LCIISLLAALPLAVHASPQPLEQIK | 3 | y7 | 29.8 | 899.19 | 855.49 | 49 |
| 79 | LCIISLLATLPLAVHASPQPLDQIK | 3 | y9 | 29.5 | 904.52 | 1025.6 | 49 |
| 80 | LCIISLLATLPLAVHASPQPLDQIK | 3 | y8 | 29.5 | 904.52 | 938.53 | 49 |
| 81 | LCIISLLATLPLAVHASPQPLDQIK | 3 | y7 | 29.5 | 904.52 | 841.48 | 49 |
| 82 | LCIISLLATLPLAVHASPQPLEQIK | 3 | y9 | 29.6 | 909.19 | 1039.6 | 49 |
| 83 | LCIISLLATLPLAVHASPQPLEQIK | 3 | y8 | 29.6 | 909.19 | 952.55 | 49 |
| 84 | LCIISLLATLPLAVHASPQPLEQIK | 3 | y7 | 29.6 | 909.19 | 855.49 | 49 |
| 85 | LCIISLLATLPLAVHSSPQPLEQIK | 3 | y9 | 29.3 | 914.53 | 1039.6 | 50 |
| 86 | LCIISLLATLPLAVHSSPQPLEQIK | 3 | y8 | 29.3 | 914.53 | 952.55 | 50 |
| 87 | LCIISLLATLPLAVHSSPQPLEQIK | 3 | y7 | 29.3 | 914.53 | 855.49 | 50 |
| 88 | LCIISLLATLPLAVHTSPQPLEQIK | 3 | y9 | 29.5 | 919.2 | 1039.6 | 50 |
| 89 | LCIISLLATLPLAVHTSPQPLEQIK | 3 | y8 | 29.5 | 919.2 | 952.55 | 50 |
| 90 | LCIISLLATLPLAVHTSPQPLEQIK | 3 | y7 | 29.5 | 919.2 | 855.49 | 50 |
| 91 | LCIISLLATLPLTVHASPQPLEQIK | 3 | y9 | 29.3 | 919.2 | 1039.6 | 50 |
| 92 | LCIISLLATLPLTVHASPQPLEQIK | 3 | y8 | 29.3 | 919.2 | 952.55 | 50 |
| 93 | LCIISLLATLPLTVHASPQPLEQIK | 3 | y7 | 29.3 | 919.2 | 855.49 | 50 |
| 94 | LCIISLLATLPLVVHASPQPLEQIK | 3 | y9 | 30.3 | 918.54 | 1039.6 | 50 |
| 95 | LCIISLLATLPLVVHASPQPLEQIK | 3 | y8 | 30.3 | 918.54 | 952.55 | 50 |
| 96 | LCIISLLATLPLVVHASPQPLEQIK | 3 | y7 | 30.3 | 918.54 | 855.49 | 50 |
| 97 | LCIISLLATLSLAVHASPQPLEQIK | 3 | y9 | 29.5 | 905.85 | 1039.6 | 49 |
| 98 | LCIISLLATLSLAVHASPQPLEQIK | 3 | y8 | 29.5 | 905.85 | 952.55 | 49 |
| 99 | LCIISLLATLSLAVHASPQPLEQIK | 3 | y7 | 29.5 | 905.85 | 855.49 | 49 |
| 100 | LCIISLLATMPLAVHASPQPLEQIK | 3 | y9 | 27.4 | 915.18 | 1039.6 | 50 |
| 101 | LCIISLLATMPLAVHASPQPLEQIK | 3 | y8 | 27.4 | 915.18 | 952.55 | 50 |
| 102 | LCIISLLATMPLAVHASPQPLEQIK | 3 | y7 | 27.4 | 915.18 | 855.49 | 50 |
| 103 | LCIISLLAVLPLAVHASPQPLEQIK | 3 | y9 | 30.5 | 908.53 | 1039.6 | 49 |
| 104 | LCIISLLAVLPLAVHASPQPLEQIK | 3 | y8 | 30.5 | 908.53 | 952.55 | 49 |
| 105 | LCIISLLAVLPLAVHASPQPLEQIK | 3 | y7 | 30.5 | 908.53 | 855.49 | 49 |
| 106 | LLISQR | 2 | y5 | 12.9 | 365.23 | 616.38 | 21 |
| 107 | LLISQR | 2 | y4 | 12.9 | 365.23 | 503.29 | 21 |
| 108 | LLISQR | 2 | y3 | 12.9 | 365.23 | 390.21 | 21 |
| 109 | LLLATVGGPAGLTAFLR | 2 | y9 | 24 | 835.5 | 945.55 | 42 |
| 110 | LLLATVGGPAGLTAFLR | 2 | y8 | 24 | 835.5 | 848.5 | 42 |
| 111 | LLLATVGGPAGLTAFLR | 2 | y7 | 24 | 835.5 | 777.46 | 42 |
| 112 | LLNSQR | 2 | y5 | 9.4 | 365.71 | 617.34 | 21 |
| 113 | LLNSQR | 2 | y4 | 9.4 | 365.71 | 504.25 | 21 |
| 114 | LLNSQR | 2 | y3 | 9.4 | 365.71 | 390.21 | 21 |
| 115 | LLTNQR | 2 | y5 | 9.8 | 372.72 | 631.35 | 21 |
| 116 | LLTNQR | 2 | y4 | 9.8 | 372.72 | 518.27 | 21 |
| 117 | LLTNQR | 2 | y3 | 9.8 | 372.72 | 417.22 | 21 |
| 118 | LLTSQR | 2 | y5 | 9.8 | 359.22 | 604.34 | 21 |
| 119 | LLTSQR | 2 | y4 | 9.8 | 359.22 | 491.26 | 21 |
| 120 | LLTSQR | 2 | y3 | 9.8 | 359.22 | 390.21 | 21 |
| 121 | LNIISLLATLPLAVHASPQPLEQIK | 3 | y9 | 29.6 | 893.87 | 1039.6 | 49 |
| 122 | LNIISLLATLPLAVHASPQPLEQIK | 3 | y8 | 29.6 | 893.87 | 952.55 | 49 |
| 123 | LNIISLLATLPLAVHASPQPLEQIK | 3 | y7 | 29.6 | 893.87 | 855.49 | 49 |
| 124 | LSASSQR | 2 | y6 | 6.2 | 374.7 | 635.31 | 21 |
| 125 | LSASSQR | 2 | y5 | 6.2 | 374.7 | 548.28 | 21 |
| 126 | LSASSQR | 2 | y4 | 6.2 | 374.7 | 477.24 | 21 |
| 127 | LSESQLSGR | 2 | y8 | 10.5 | 488.76 | 863.42 | 27 |
| 128 | LSESQLSGR | 2 | y7 | 10.5 | 488.76 | 776.39 | 27 |
| 129 | LSESQLSGR | 2 | y6 | 10.5 | 488.76 | 647.35 | 27 |
| 130 | LSESQLSGSVGMIEMDLASGR | 3 | y9 | 21.1 | 723.02 | 991.49 | 40 |
| 131 | LSESQLSGSVGMIEMDLASGR | 3 | y8 | 21.1 | 723.02 | 878.4 | 40 |
| 132 | LSESQLSGSVGMIEMDLASGR | 3 | y7 | 21.1 | 723.02 | 749.36 | 40 |
| 133 | MVVIYLR | 2 | y6 | 18.9 | 447.27 | 762.49 | 25 |
| 134 | MVVIYLR | 2 | y5 | 18.9 | 447.27 | 663.42 | 25 |
| 135 | MVVIYLR | 2 | y4 | 18.9 | 447.27 | 564.35 | 25 |
| 136 | NEALPGDAR | 2 | y8 | 10.6 | 471.74 | 828.42 | 26 |
| 137 | NEALPGDAR | 2 | y7 | 10.6 | 471.74 | 699.38 | 26 |
| 138 | NEALPGDAR | 2 | y6 | 10.6 | 471.74 | 628.34 | 26 |
| 139 | NQHIAGIGAALIEHWQR | 2 | y9 | 21.2 | 957.51 | 1123.6 | 47 |
| 140 | NQHIAGIGAALIEHWQR | 2 | y8 | 21.2 | 957.51 | 1052.6 | 47 |
| 141 | NQHIAGIGAALIEHWQR | 2 | y7 | 21.2 | 957.51 | 981.53 | 47 |
| 142 | NQQIAGIGAALIEHWQR | 2 | y9 | 22.1 | 953.01 | 1123.6 | 47 |
| 143 | NQQIAGIGAALIEHWQR | 2 | y8 | 22.1 | 953.01 | 1052.6 | 47 |
| 144 | NQQIAGIGAALIEHWQR | 2 | y7 | 22.1 | 953.01 | 981.53 | 47 |
| 145 | NQQIAGLGAALIEHWQR | 2 | y9 | 22.3 | 953.01 | 1123.6 | 47 |
| 146 | NQQIAGLGAALIEHWQR | 2 | y8 | 22.3 | 953.01 | 1052.6 | 47 |
| 147 | NQQIAGLGAALIEHWQR | 2 | y7 | 22.3 | 953.01 | 981.53 | 47 |
| 148 | NTTTPASMAATLR | 2 | y9 | 15.3 | 667.84 | 917.49 | 34 |
| 149 | NTTTPASMAATLR | 2 | y8 | 15.3 | 667.84 | 820.43 | 34 |
| 150 | NTTTPASMAATLR | 2 | y7 | 15.3 | 667.84 | 749.4 | 34 |
| 151 | NVLTSQR | 2 | y6 | 9.6 | 409.23 | 703.41 | 23 |
| 152 | NVLTSQR | 2 | y5 | 9.6 | 409.23 | 604.34 | 23 |
| 153 | NVLTSQR | 2 | y4 | 9.6 | 409.23 | 491.26 | 23 |
| 154 | QIDDNVTR | 2 | y7 | 9.4 | 480.74 | 832.42 | 26 |
| 155 | QIDDNVTR | 2 | y6 | 9.4 | 480.74 | 719.33 | 26 |
| 156 | QIDDNVTR | 2 | y5 | 9.4 | 480.74 | 604.3 | 26 |
| 157 | QIGDK | 2 | y4 | 5.8 | 280.66 | 432.25 | 17 |
| 158 | QIGDK | 2 | y3 | 5.8 | 280.66 | 319.16 | 17 |
| 159 | QIGDK | 2 | b4 | 5.8 | 280.66 | 414.2 | 17 |
| 160 | QIGDNVTR | 2 | y7 | 9.5 | 451.74 | 774.41 | 25 |
| 161 | QIGDNVTR | 2 | y6 | 9.5 | 451.74 | 661.33 | 25 |
| 162 | QIGDNVTR | 2 | y5 | 9.5 | 451.74 | 604.3 | 25 |
| 163 | QIGENVTR | 2 | y7 | 9.5 | 458.75 | 788.43 | 25 |
| 164 | QIGENVTR | 2 | y6 | 9.5 | 458.75 | 675.34 | 25 |
| 165 | QIGENVTR | 2 | y5 | 9.5 | 458.75 | 618.32 | 25 |
| 166 | QLLQWMVDAR | 2 | y9 | 21.5 | 630.33 | 1131.6 | 33 |
| 167 | QLLQWMVDAR | 2 | y8 | 21.5 | 630.33 | 1018.5 | 33 |
| 168 | QLLQWMVDAR | 2 | y7 | 21.5 | 630.33 | 905.43 | 33 |
| 169 | QLLQWMVDDGVAGPLIR | 2 | y9 | 25 | 956.01 | 897.52 | 47 |
| 170 | QLLQWMVDDGVAGPLIR | 2 | y8 | 25 | 956.01 | 782.49 | 47 |
| 171 | QLLQWMVDDGVAGPLIR | 2 | y7 | 25 | 956.01 | 725.47 | 47 |
| 172 | QLLQWMVDDR | 2 | y9 | 21.7 | 652.33 | 1175.6 | 34 |
| 173 | QLLQWMVDDR | 2 | y8 | 21.7 | 652.33 | 1062.5 | 34 |
| 174 | QLLQWMVDDR | 2 | y7 | 21.7 | 652.33 | 949.42 | 34 |
| 175 | QLLQWMVDGR | 2 | y8 | 21.3 | 623.32 | 1004.5 | 32 |
| 176 | QLLQWMVDGR | 2 | y7 | 21.3 | 623.32 | 891.41 | 32 |
| 177 | QLLQWMVDGR | 2 | y6 | 21.3 | 623.32 | 763.36 | 32 |
| 178 | QLLQWMVEDR | 2 | y9 | 21.9 | 659.33 | 1189.6 | 34 |
| 179 | QLLQWMVEDR | 2 | y8 | 21.9 | 659.33 | 1076.5 | 34 |
| 180 | QLLQWMVEDR | 2 | y7 | 21.9 | 659.33 | 963.44 | 34 |
| 181 | QQDLVDYSPVSEK | 2 | y9 | 15.6 | 754.37 | 1023.5 | 38 |
| 182 | QQDLVDYSPVSEK | 2 | y8 | 15.6 | 754.37 | 924.43 | 38 |
| 183 | QQDLVDYSPVSEK | 2 | y7 | 15.6 | 754.37 | 809.4 | 38 |
| 184 | QQHLVDYSPVSEK | 2 | y9 | 13.9 | 765.38 | 1023.5 | 39 |
| 185 | QQHLVDYSPVSEK | 2 | y8 | 13.9 | 765.38 | 924.43 | 39 |
| 186 | QQHLVDYSPVSEK | 2 | y7 | 13.9 | 765.38 | 809.4 | 39 |
| 187 | QSESQLSGR | 2 | y8 | 7.6 | 496.24 | 863.42 | 27 |
| 188 | QSESQLSGR | 2 | y7 | 7.6 | 496.24 | 776.39 | 27 |
| 189 | QSESQLSGR | 2 | y6 | 7.6 | 496.24 | 647.35 | 27 |
| 190 | QSESQLSGSVGMIEMDLASGR | 3 | y9 | 19.7 | 728.01 | 991.49 | 40 |
| 191 | QSESQLSGSVGMIEMDLASGR | 3 | y8 | 19.7 | 728.01 | 878.4 | 40 |
| 192 | QSESQLSGSVGMIEMDLASGR | 3 | y7 | 19.7 | 728.01 | 749.36 | 40 |
| 193 | SQLQLLQWMVDDR | 2 | y9 | 26.3 | 816.41 | 1175.6 | 41 |
| 194 | SQLQLLQWMVDDR | 2 | y8 | 26.3 | 816.41 | 1062.5 | 41 |
| 195 | SQLQLLQWMVDDR | 2 | y7 | 26.3 | 816.41 | 949.42 | 41 |
| 196 | SVLPAGWFIADK | 2 | y9 | 22.9 | 652.36 | 1004.5 | 34 |
| 197 | SVLPAGWFIADK | 2 | y8 | 22.9 | 652.36 | 907.47 | 34 |
| 198 | SVLPAGWFIADK | 2 | y7 | 22.9 | 652.36 | 836.43 | 34 |
| 199 | SVLPAGWFIADR | 2 | y9 | 23 | 666.36 | 1032.5 | 34 |
| 200 | SVLPAGWFIADR | 2 | y8 | 23 | 666.36 | 935.47 | 34 |
| 201 | SVLPAGWFIADR | 2 | y7 | 23 | 666.36 | 864.44 | 34 |
| 202 | SVLSAGWFIADK | 2 | y9 | 22.7 | 647.35 | 994.5 | 33 |
| 203 | SVLSAGWFIADK | 2 | y8 | 22.7 | 647.35 | 907.47 | 33 |
| 204 | SVLSAGWFIADK | 2 | y7 | 22.7 | 647.35 | 836.43 | 33 |
| 205 | TGAAER | 2 | y5 | 4.8 | 302.66 | 503.26 | 18 |
| 206 | TGAAER | 2 | y4 | 4.8 | 302.66 | 446.24 | 18 |
| 207 | TGAAER | 2 | y3 | 4.8 | 302.66 | 375.2 | 18 |
| 208 | TGAAK | 2 | y4 | 6 | 224.13 | 346.21 | 15 |
| 209 | TGAAK | 2 | y3 | 6 | 224.13 | 289.19 | 15 |
| 210 | TGAAK | 2 | b4 | 6 | 224.13 | 301.15 | 15 |
| 211 | TGAGER | 2 | y5 | 4.9 | 295.65 | 489.24 | 18 |
| 212 | TGAGER | 2 | y4 | 4.9 | 295.65 | 432.22 | 18 |
| 213 | TGAGER | 2 | y3 | 4.9 | 295.65 | 361.18 | 18 |
| 214 | TGAGK | 2 | y4 | 6.1 | 217.12 | 332.19 | 15 |
| 215 | TGAGK | 2 | y3 | 6.1 | 217.12 | 275.17 | 15 |
| 216 | TGAGK | 2 | b4 | 6.1 | 217.12 | 287.13 | 15 |
| 217 | TGASER | 2 | y5 | 4.5 | 310.65 | 519.25 | 19 |
| 218 | TGASER | 2 | y4 | 4.5 | 310.65 | 462.23 | 19 |
| 219 | TGASER | 2 | y3 | 4.5 | 310.65 | 391.19 | 19 |
| 220 | TGASK | 2 | y4 | 5.8 | 232.13 | 362.2 | 15 |
| 221 | TGASK | 2 | y3 | 5.8 | 232.13 | 305.18 | 15 |
| 222 | TGASK | 2 | b4 | 5.8 | 232.13 | 317.15 | 15 |
| 223 | TGASR | 2 | y4 | 5.8 | 246.13 | 390.21 | 16 |
| 224 | TGASR | 2 | y3 | 5.8 | 246.13 | 333.19 | 16 |
| 225 | TGASR | 2 | y2 | 5.8 | 246.13 | 262.15 | 16 |
| 226 | TLTAWCADER | 2 | y9 | 15.9 | 611.78 | 1121.5 | 32 |
| 227 | TLTAWCADER | 2 | y8 | 15.9 | 611.78 | 1008.4 | 32 |
| 228 | TLTAWCADER | 2 | y7 | 15.9 | 611.78 | 907.37 | 32 |
| 229 | TLTAWHADER | 2 | y9 | 14.4 | 600.29 | 1098.5 | 31 |
| 230 | TLTAWHADER | 2 | y8 | 14.4 | 600.29 | 985.45 | 31 |
| 231 | TLTAWHADER | 2 | y7 | 14.4 | 600.29 | 884.4 | 31 |
| 232 | TLTAWR | 2 | y5 | 15 | 374.21 | 646.37 | 21 |
| 233 | TLTAWR | 2 | y4 | 15 | 374.21 | 533.28 | 21 |
| 234 | TLTAWR | 2 | y3 | 15 | 374.21 | 432.24 | 21 |
| 235 | TVGGPAGLTAFLR | 2 | y9 | 19.7 | 630.36 | 945.55 | 33 |
| 236 | TVGGPAGLTAFLR | 2 | y8 | 19.7 | 630.36 | 848.5 | 33 |
| 237 | TVGGPAGLTAFLR | 2 | y7 | 19.7 | 630.36 | 777.46 | 33 |
| 238 | TVVIYLR | 2 | y6 | 17.4 | 432.27 | 762.49 | 24 |
| 239 | TVVIYLR | 2 | y5 | 17.4 | 432.27 | 663.42 | 24 |
| 240 | TVVIYLR | 2 | y4 | 17.4 | 432.27 | 564.35 | 24 |
| 241 | VAGPLIR | 2 | y6 | 14.1 | 363.24 | 626.4 | 21 |
| 242 | VAGPLIR | 2 | y5 | 14.1 | 363.24 | 555.36 | 21 |
| 243 | VAGPLIR | 2 | y4 | 14.1 | 363.24 | 498.34 | 21 |
| 244 | VALCGAVLAR | 2 | y9 | 17.2 | 515.3 | 930.52 | 28 |
| 245 | VALCGAVLAR | 2 | y8 | 17.2 | 515.3 | 859.48 | 28 |
| 246 | VALCGAVLAR | 2 | y7 | 17.2 | 515.3 | 746.4 | 28 |
| 247 | VDAGDEQLER | 2 | y9 | 10.9 | 566.27 | 1032.5 | 30 |
| 248 | VDAGDEQLER | 2 | y8 | 10.9 | 566.27 | 917.43 | 30 |
| 249 | VDAGDEQLER | 2 | y7 | 10.9 | 566.27 | 846.4 | 30 |
| 250 | VDAGDK | 2 | y5 | 4.1 | 302.65 | 505.23 | 18 |
| 251 | VDAGDK | 2 | y4 | 4.1 | 302.65 | 390.2 | 18 |
| 252 | VDAGDK | 2 | y3 | 4.1 | 302.65 | 319.16 | 18 |
| 253 | VGMIEMDLASGR | 2 | y9 | 18.7 | 639.81 | 991.49 | 33 |
| 254 | VGMIEMDLASGR | 2 | y8 | 18.7 | 639.81 | 878.4 | 33 |
| 255 | VGMIEMDLASGR | 2 | y7 | 18.7 | 639.81 | 749.36 | 33 |
| 256 | VGMIEMDLASR | 2 | y9 | 18.7 | 611.3 | 1065.5 | 32 |
| 257 | VGMIEMDLASR | 2 | y8 | 18.7 | 611.3 | 934.47 | 32 |
| 258 | VGMIEMDLASR | 2 | y7 | 18.7 | 611.3 | 821.38 | 32 |
| 259 | VGMIEMDLASSR | 2 | y9 | 18.4 | 654.82 | 1021.5 | 34 |
| 260 | VGMIEMDLASSR | 2 | y8 | 18.4 | 654.82 | 908.41 | 34 |
| 261 | VGMIEMDLASSR | 2 | y7 | 18.4 | 654.82 | 779.37 | 34 |
| 262 | VLLCGAVLAR | 2 | y9 | 19.3 | 536.32 | 972.57 | 29 |
| 263 | VLLCGAVLAR | 2 | y8 | 19.3 | 536.32 | 859.48 | 29 |
| 264 | VLLCGAVLAR | 2 | y7 | 19.3 | 536.32 | 746.4 | 29 |
| 265 | VVLCGAMLAR | 2 | y8 | 18.1 | 545.3 | 891.45 | 29 |
| 266 | VVLCGAMLAR | 2 | y7 | 18.1 | 545.3 | 778.37 | 29 |
| 267 | VVLCGAMLAR | 2 | y6 | 18.1 | 545.3 | 618.34 | 29 |
| 268 | VVLCGAVLAR | 2 | y9 | 18 | 529.31 | 958.55 | 28 |
| 269 | VVLCGAVLAR | 2 | y8 | 18 | 529.31 | 859.48 | 28 |
| 270 | VVLCGAVLAR | 2 | y7 | 18 | 529.31 | 746.4 | 28 |
| 271 | VVLCGTVLAR | 2 | y9 | 16.6 | 544.32 | 988.56 | 29 |
| 272 | VVLCGTVLAR | 2 | y8 | 16.6 | 544.32 | 889.49 | 29 |
| 273 | VVLCGTVLAR | 2 | y7 | 16.6 | 544.32 | 776.41 | 29 |
| 274 | WETDR | 2 | y4 | 9.8 | 353.66 | 520.24 | 21 |
| 275 | WETDR | 2 | y3 | 9.8 | 353.66 | 391.19 | 21 |
| 276 | WETDR | 2 | b4 | 9.8 | 353.66 | 532.2 | 21 |
| 277 | WETELNEAFPGDAR | 2 | y9 | 19 | 817.88 | 976.45 | 41 |
| 278 | WETELNEAFPGDAR | 2 | y8 | 19 | 817.88 | 862.41 | 41 |
| 279 | WETELNEAFPGDAR | 2 | y7 | 19 | 817.88 | 733.36 | 41 |
| 280 | WETELNEALPADAR | 2 | y9 | 18.9 | 807.89 | 956.48 | 41 |
| 281 | WETELNEALPADAR | 2 | y8 | 18.9 | 807.89 | 842.44 | 41 |
| 282 | WETELNEALPADAR | 2 | y7 | 18.9 | 807.89 | 713.39 | 41 |
| 283 | WETELNEALPGDAR | 2 | y9 | 18.5 | 800.88 | 942.46 | 40 |
| 284 | WETELNEALPGDAR | 2 | y8 | 18.5 | 800.88 | 828.42 | 40 |
| 285 | WETELNEALPGDAR | 2 | y7 | 18.5 | 800.88 | 699.38 | 40 |
| 286 | WETELNEALSGDAR | 2 | y9 | 18 | 795.87 | 932.44 | 40 |
| 287 | WETELNEALSGDAR | 2 | y8 | 18 | 795.87 | 818.4 | 40 |
| 288 | WETELNEALSGDAR | 2 | y7 | 18 | 795.87 | 689.36 | 40 |
| 289 | WETELNEVLPGDAR | 2 | y9 | 19.4 | 814.9 | 970.5 | 41 |
| 290 | WETELNEVLPGDAR | 2 | y8 | 19.4 | 814.9 | 856.45 | 41 |
| 291 | WETELNEVLPGDAR | 2 | y7 | 19.4 | 814.9 | 727.41 | 41 |
| 292 | WETER | 2 | y4 | 10.4 | 360.67 | 534.25 | 21 |
| 293 | WETER | 2 | y3 | 10.4 | 360.67 | 405.21 | 21 |
| 294 | WETER | 2 | b4 | 10.4 | 360.67 | 546.22 | 21 |

Les aires obtenues pour chacune des transitions et pour chacun des microorganismes étudiés ont été mesurées. Lorsque les aires des 3 transitions d'un même peptide sont supérieures ou égales à 2500 la détection du peptide est considérée comme positive et est noté « 1 ». Lorsque au moins une transition comporte une aire inférieure à 2500, le peptide correspondant est considéré comme non détecté et est noté « 0 ».

### Exemple 10 : Identification d'une résistance aux béta-lactamines de type FOX:

Les échantillons correspondant à une espèce pouvant comporter un mécanisme de résistance de type FOX peuvent être détectés en mettant en oeuvre la méthode suivante.

Chaque échantillon est traité selon l'exemple 5, puis analysé selon l'exemple 6 en détectant les peptides du **TABLEAU 9** en lieu et place des peptides du **TABLEAU 3.**

**TABLEAU 9 :**

| Transition numéro | Peptide | Etat de charge du précurseur | ion fragment de première génération | Temps de rétention (minutes) | (m/z) filtré en Q1 | (m/z) filtré en Q3 | Energie de collision (eV) |
|---|---|---|---|---|---|---|---|
| 1 | AHYFNYGVANR | 2 | y9 | 15.5 | 656.32 | 1103.53 | 34 |
| 2 | AHYFNYGVANR | 2 | y8 | 15.5 | 656.32 | 940.46 | 34 |
| 3 | AHYFNYGVANR | 2 | y7 | 15.5 | 656.32 | 793.4 | 34 |
| 4 | AMGEQR | 2 | y5 | 5.3 | 346.16 | 620.28 | 20 |
| 5 | AMGEQR | 2 | y4 | 5.3 | 346.16 | 489.24 | 20 |
| 6 | AMGEQR | 2 | y3 | 5.3 | 346.16 | 432.22 | 20 |
| 7 | ESGQR | 2 | y4 | 4.3 | 288.64 | 447.23 | 18 |
| 8 | ESGQR | 2 | y3 | 4.3 | 288.64 | 360.2 | 18 |
| 9 | ESGQR | 2 | b4 | 4.3 | 288.64 | 402.16 | 18 |
| 10 | FAVPK | 2 | y4 | 11.3 | 281.17 | 414.27 | 17 |
| 11 | FAVPK | 2 | y3 | 11.3 | 281.17 | 343.23 | 17 |
| 12 | FAVPK | 2 | b4 | 11.3 | 281.17 | 415.23 | 17 |
| 13 | GGFELDDK | 2 | y7 | 14.5 | 440.71 | 823.38 | 24 |
| 14 | GGFELDDK | 2 | y6 | 14.5 | 440.71 | 766.36 | 24 |
| 15 | GGFELDDK | 2 | y5 | 14.5 | 440.71 | 619.29 | 24 |
| 16 | GIAIVMLANR | 2 | y9 | 19.3 | 529.31 | 1000.6 | 28 |
| 17 | GIAIVMLANR | 2 | y8 | 19.3 | 529.31 | 887.51 | 28 |
| 18 | GIAIVMLANR | 2 | y7 | 19.3 | 529.31 | 816.48 | 28 |
| 19 | IPGMAVAVLK | 2 | y9 | 18.1 | 499.81 | 885.52 | 27 |
| 20 | IPGMAVAVLK | 2 | y8 | 18.1 | 499.81 | 788.47 | 27 |
| 21 | IPGMAVAVLK | 2 | y7 | 18.1 | 499.81 | 731.45 | 27 |
| 22 | NYPIEAR | 2 | y6 | 12.2 | 431.72 | 748.4 | 24 |
| 23 | NYPIEAR | 2 | y5 | 12.2 | 431.72 | 585.34 | 24 |
| 24 | NYPIEAR | 2 | y4 | 12.2 | 431.72 | 488.28 | 24 |
| 25 | SWSPVYPAGTHR | 2 | y9 | 15.5 | 679.34 | 997.52 | 35 |
| 26 | SWSPVYPAGTHR | 2 | y8 | 15.5 | 679.34 | 900.47 | 35 |
| 27 | SWSPVYPAGTHR | 2 | y7 | 15.5 | 679.34 | 801.4 | 35 |
| 28 | TGSADLLK | 2 | y7 | 14 | 402.73 | 703.4 | 23 |
| 29 | TGSADLLK | 2 | y6 | 14 | 402.73 | 646.38 | 23 |
| 30 | TGSADLLK | 2 | y5 | 14 | 402.73 | 559.34 | 23 |
| 31 | TGSTGGFGAYVAFVPAR | 2 | y9 | 19.5 | 829.42 | 993.55 | 41 |
| 32 | TGSTGGFGAYVAFVPAR | 2 | y8 | 19.5 | 829.42 | 922.51 | 41 |
| 33 | TGSTGGFGAYVAFVPAR | 2 | y7 | 19.5 | 829.42 | 759.45 | 41 |
| 34 | TLTATLGAYAAVK | 2 | y9 | 17.2 | 640.37 | 893.51 | 33 |
| 35 | TLTATLGAYAAVK | 2 | y8 | 17.2 | 640.37 | 792.46 | 33 |
| 36 | TLTATLGAYAAVK | 2 | y7 | 17.2 | 640.37 | 679.38 | 33 |
| 37 | VSEQTLFEIGSVSK | 2 | y9 | 19.6 | 762.4 | 979.55 | 39 |
| 38 | VSEQTLFEIGSVSK | 2 | y8 | 19.6 | 762.4 | 866.46 | 39 |
| 39 | VSEQTLFEIGSVSK | 2 | y7 | 19.6 | 762.4 | 719.39 | 39 |
| 40 | VSQHAPWLK | 2 | y8 | 15.3 | 533.3 | 966.52 | 28 |
| 41 | VSQHAPWLK | 2 | y7 | 15.3 | 533.3 | 879.48 | 28 |
| 42 | VSQHAPWLK | 2 | y6 | 15.3 | 533.3 | 751.42 | 28 |
| 43 | VTPGVLAAEAYGIK | 2 | y9 | 18.4 | 694.89 | 935.52 | 36 |
| 44 | VTPGVLAAEAYGIK | 2 | y8 | 18.4 | 694.89 | 822.44 | 36 |
| 45 | VTPGVLAAEAYGIK | 2 | y7 | 18.4 | 694.89 | 751.4 | 36 |

Les aires obtenues pour chacune des transitions et pour chacun des microorganismes étudiés ont été mesurées. Lorsque les aires des trois transitions d'un même peptide sont supérieures ou égales à 2500 la détection du peptide est considérée comme positive et est noté « 1 ». Lorsque au moins une transition comporte une aire inférieure à 2500, le peptide correspondant est considéré comme non détecté et est noté « 0 ».

### Exemple 11 : Identification d'une résistance aux béta-lactamines de type TEM ou CTX-M:

Les échantillons correspondant à une espèce pouvant comporter un mécanisme de résistance de type TEM ou CTX-M peuvent être détectés en mettant en oeuvre la méthode suivante.

Chaque échantillon est traité selon l'exemple 5, puis analysé selon l'exemple 6 en détectant les peptides du **TABLEAU 10** en lieu et place des peptides du **TABLEAU 3.**

**TABLEAU 10 :**

| Transition numéro | Protéine | Peptide | Ion fragment de première génération | Etat de charge du précurseur | Intérêt clinique |
|---|---|---|---|---|---|
| 1 | CTX-M | AMAQTLR | y4 monochargé | 2 | 2be |
| 2 | CTX-M | AMAQTLR | y5 monochargé | 2 | 2be |
| 3 | CTX-M | AMAQTLR | y6 monochargé | 2 | 2be |
| 4 | CTX-M | AQLVTWLK | y5 monochargé | 2 | 2be |
| 5 | CTX-M | AQLVTWLK | y6 monochargé | 2 | 2be |
| 6 | CTX-M | AQLVTWLK | y7 monochargé | 2 | 2be |
| 7 | CTX-M | FAMCSTSK | y5 monochargé | 2 | 2be |
| 8 | CTX-M | FAMCSTSK | y6 monochargé | 2 | 2be |
| 9 | CTX-M | FAMCSTSK | y7 monochargé | 2 | 2be |
| 10 | CTX-M | LAALEK | y3 monochargé | 2 | 2be |
| 11 | CTX-M | LAALEK | y4 monochargé | 2 | 2be |
| 12 | CTX-M | LAALEK | y5 monochargé | 2 | 2be |
| 13 | CTX-M | LGVALINTADNSQILYR | y7 monochargé | 2 | 2be |
| 14 | CTX-M | LGVALINTADNSQILYR | y8 monochargé | 2 | 2be |
| 15 | CTX-M | LGVALINTADNSQILYR | y9 monochargé | 2 | 2be |
| 16 | CTX-M | NLTLGK | y3 monochargé | 2 | 2be |
| 17 | CTX-M | NLTLGK | y4 monochargé | 2 | 2be |
| 18 | CTX-M | NLTLGK | y5 monochargé | 2 | 2be |
| 19 | CTX-M | QSETQK | y3 monochargé | 2 | 2be |
| 20 | CTX-M | QSETQK | y4 monochargé | 2 | 2be |
| 21 | CTX-M | QSETQK | y5 monochargé | 2 | 2be |
| 22 | CTX-M | SDLVNYNPIAEK | y7 monochargé | 2 | 2be |
| 23 | CTX-M | SDLVNYNPIAEK | y8 monochargé | 2 | 2be |
| 24 | CTX-M | SDLVNYNPIAEK | y9 monochargé | 2 | 2be |
| 25 | CTX-M | TEPTLNTAIPGDPR | y7 monochargé | 2 | 2be |
| 26 | CTX-M | TEPTLNTAIPGDPR | y8 monochargé | 2 | 2be |
| 27 | CTX-M | TEPTLNTAIPGDPR | y9 monochargé | 2 | 2be |
| 28 | CTX-M | VMAAAAVLK | y6 monochargé | 2 | 2be |
| 29 | CTX-M | VMAAAAVLK | y7 monochargé | 2 | 2be |
| 30 | CTX-M | VMAAAAVLK | y8 monochargé | 2 | 2be |
| 31 | TEM | DAEDQLGAR | y5 monochargé | 2 | TEM |
| 32 | TEM | DAEDQLGAR | y6 monochargé | 2 | TEM |
| 33 | TEM | DAEDQLGAR | y7 monochargé | 2 | TEM |
| 34 | TEM | DTTMPAAMATTLR | y7 monochargé | 2 | TEM |
| 35 | TEM | DTTMPAAMATTLR | y8 monochargé | 2 | TEM |
| 36 | TEM | DTTMPAAMATTLR | y9 monochargé | 2 | TEM |
| 37 | TEM | DTTTPAAMATTLR | y7 monochargé | 2 | TEM |
| 38 | TEM | DTTTPAAMATTLR | y9 dichargé | 2 | TEM |
| 39 | TEM | DTTTPAAMATTLR | y9 monochargé | 2 | TEM |
| 40 | TEM | EPELNEAIPNDER | y5 monochargé | 2 | TEM |
| 41 | TEM | EPELNEAIPNDER | y7 monochargé | 2 | TEM |
| 42 | TEM | EPELNEAIPNDER | y8 monochargé | 2 | TEM |
| 43 | TEM | GIIAALGPDGKPSR | y7 monochargé | 2 | TEM |
| 44 | TEM | GIIAALGPDGKPSR | y8 monochargé | 2 | TEM |
| 45 | TEM | GIIAALGPDGKPSR | y9 monochargé | 2 | TEM |
| 46 | TEM | GSCGIIAALGPDGKPSR | y7 monochargé | 2 | 2br |
| 47 | TEM | GSCGIIAALGPDGKPSR | y8 monochargé | 2 | 2br |
| 48 | TEM | GSCGIIAALGPDGKPSR | y9 monochargé | 2 | 2br |
| 49 | TEM | GSSGIIAALGPDGKPSR | y7 monochargé | 2 | TEM |
| 50 | TEM | GSSGIIAALGPDGKPSR | y8 monochargé | 2 | TEM |
| 51 | TEM | GSSGIIAALGPDGKPSR | y9 monochargé | 2 | TEM |
| 52 | TEM | HLTDGMTVR | y4 monochargé | 2 | TEM |
| 53 | TEM | HLTDGMTVR | y7 monochargé | 2 | TEM |
| 54 | TEM | HLTDGMTVR | y8 monochargé | 2 | TEM |
| 55 | TEM | IHYSQNDLVEYSPVTEK | y6 monochargé | 3 | TEM |
| 56 | TEM | IHYSQNDLVEYSPVTEK | y7 monochargé | 3 | TEM |
| 57 | TEM | IHYSQNDLVEYSPVTEK | y8 monochargé | 3 | TEM |
| 58 | TEM | IHYSQNDLVK | y7 monochargé | 2 | 2be |
| 59 | TEM | IHYSQNDLVK | y8 monochargé | 2 | 2be |
| 60 | TEM | IHYSQNDLVK | y9 dichargé | 2 | 2be |
| 61 | TEM | ILESFRPEER | b6 monochargé | 2 | TEM |
| 62 | TEM | ILESFRPEER | b8 monochargé | 2 | TEM |
| 63 | TEM | ILESFRPEER | y7 dichargé | 2 | TEM |
| 64 | TEM | LDHWEPELNEAIPNDER | y5 monochargé | 3 | 2be |
| 65 | TEM | LDHWEPELNEAIPNDER | y6 monochargé | 3 | 2be |
| 66 | TEM | LDHWEPELNEAIPNDER | y7 monochargé | 3 | 2be |
| 67 | TEM | LDSWEPELNEAIPNDER | y5 monochargé | 3 | 2be |
| 68 | TEM | LDSWEPELNEAIPNDER | y6 monochargé | 3 | 2be |
| 69 | TEM | LDSWEPELNEAIPNDER | y7 monochargé | 3 | 2be |
| 70 | TEM | LLTGELLTLASR | y6 monochargé | 2 | TEM |
| 71 | TEM | LLTGELLTLASR | y7 monochargé | 2 | TEM |
| 72 | TEM | LLTGELLTLASR | y9 monochargé | 2 | TEM |
| 73 | TEM | QIAEIGASLIK | y7 monochargé | 2 | TEM |
| 74 | TEM | QIAEIGASLIK | y8 monochargé | 2 | TEM |
| 75 | TEM | QIAEIGASLIK | y9 monochargé | 2 | TEM |
| 76 | TEM | QQLIDWMEADK | y5 monochargé | 2 | TEM |
| 77 | TEM | QQLIDWMEADK | y6 monochargé | 2 | TEM |
| 78 | TEM | QQLIDWMEADK | y7 monochargé | 2 | TEM |
| 79 | TEM | VAGPLLR | y4 monochargé | 2 | TEM |
| 80 | TEM | VAGPLLR | y5 monochargé | 2 | TEM |
| 81 | TEM | VAGPLLR | y6 monochargé | 2 | TEM |
| 82 | TEM | VDAGQEQLGR | y5 monochargé | 2 | TEM |
| 83 | TEM | VDAGQEQLGR | y7 monochargé | 2 | TEM |
| 84 | TEM | VDAGQEQLGR | y8 monochargé | 2 | TEM |
| 85 | TEM | VGYIELDLNSGK | y7 monochargé | 2 | TEM |
| 86 | TEM | VGYIELDLNSGK | y8 monochargé | 2 | TEM |
| 87 | TEM | VGYIELDLNSGK | y9 monochargé | 2 | TEM |
| 88 | TEM | WEPELNEAIPNDER | y12 dichargé | 2 | TEM |
| 89 | TEM | WEPELNEAIPNDER | y5 monochargé | 2 | TEM |
| 90 | TEM | WEPELNEAIPNDER | y7 monochargé | 2 | TEM |
| 91 | TEM | YSPVTEK | y4 monochargé | 2 | 2be |
| 92 | TEM | YSPVTEK | y5 monochargé | 2 | 2be |
| 93 | TEM | YSPVTEK | y6 monochargé | 2 | 2be |

Dans la colonne intérêt clinique, les mentions TEM, 2b, 2br, 2be et 2ber correspondent aux mêmes indications que dans le TABLEAU 4.

Les transitions mentionnées dans le **TABLEAU 10** sont détectées en utilisant les paramètres figurant dans le **TABLEAU 11.**

**TABLEAU 11 :**

| Transition numéro | Temps de rétention (minute) | (m/z) filtré en Q1 | (m/z) filtré en Q3 | Energie de collision (eV) | Seuil de positivité |
|---|---|---|---|---|---|
| 1 | 12.1 | 395.72 | 517.31 | 22 | 2500 |
| 2 | 12.1 | 395.72 | 588.35 | 22 | 2500 |
| 3 | 12.1 | 395.72 | 719.39 | 22 | 2500 |
| 4 | 21 | 479.79 | 646.39 | 26 | 2500 |
| 5 | 21 | 479.79 | 759.48 | 26 | 2500 |
| 6 | 21 | 479.79 | 887.53 | 26 | 2500 |
| 7 | 11.1 | 466.2 | 582.26 | 26 | 2500 |
| 8 | 11.1 | 466.2 | 713.3 | 26 | 2500 |
| 9 | 11.1 | 466.2 | 784.33 | 26 | 2500 |
| 10 | 11 | 322.7 | 389.24 | 19 | 2500 |
| 11 | 11 | 322.7 | 460.28 | 19 | 2500 |
| 12 | 11 | 322.7 | 531.31 | 19 | 2500 |
| 13 | 21.1 | 931.01 | 893.48 | 46 | 2500 |
| 14 | 21.1 | 931.01 | 1008.51 | 46 | 2500 |
| 15 | 21.1 | 931.01 | 1079.55 | 46 | 2500 |
| 16 | 12.2 | 323.2 | 317.22 | 19 | 2500 |
| 17 | 12.2 | 323.2 | 418.27 | 19 | 2500 |
| 18 | 12.2 | 323.2 | 531.35 | 19 | 2500 |
| 19 | 3.7 | 360.68 | 376.22 | 21 | 2500 |
| 20 | 3.7 | 360.68 | 505.26 | 21 | 2500 |
| 21 | 3.7 | 360.68 | 592.29 | 21 | 2500 |
| 22 | 17.1 | 681.85 | 834.44 | 35 | 2500 |
| 23 | 17.1 | 681.85 | 948.48 | 35 | 2500 |
| 24 | 17.1 | 681.85 | 1047.55 | 35 | 2500 |
| 25 | 15.6 | 741.38 | 725.39 | 38 | 2500 |
| 26 | 15.6 | 741.38 | 826.44 | 38 | 2500 |
| 27 | 15.6 | 741.38 | 940.48 | 38 | 2500 |
| 28 | 15.3 | 437.26 | 572.38 | 24 | 2500 |
| 29 | 15.3 | 437.26 | 643.41 | 24 | 2500 |
| 30 | 15.3 | 437.26 | 774.45 | 24 | 2500 |
| 31 | 10.5 | 487.73 | 544.32 | 26 | 2500 |
| 32 | 10.5 | 487.73 | 659.35 | 26 | 2500 |
| 33 | 10.5 | 487.73 | 788.39 | 26 | 2500 |
| 34 | 17.6 | 690.34 | 763.61 | 35 | 2500 |
| 35 | 17.6 | 690.34 | 834.45 | 35 | 2500 |
| 36 | 17.6 | 690.34 | 931.5 | 35 | 2500 |
| 37 | 16.1 | 675.34 | 763.41 | 35 | 2500 |
| 38 | 16.1 | 675.34 | 466.25 | 35 | 2500 |
| 39 | 16.1 | 675.34 | 931.5 | 35 | 2500 |
| 40 | 14.9 | 763.36 | 630.28 | 39 | 2500 |
| 41 | 14.9 | 763.36 | 814.41 | 39 | 2500 |
| 42 | 14.9 | 763.36 | 943.45 | 39 | 2500 |
| 43 | 15.5 | 676.39 | 756.4 | 35 | 2500 |
| 44 | 15.5 | 676.39 | 813.42 | 35 | 2500 |
| 45 | 15.5 | 676.39 | 926.51 | 35 | 2500 |
| 46 | 16.5 | 828.43 | 756.4 | 41 | 2500 |
| 47 | 16.5 | 828.43 | 813.42 | 41 | 2500 |
| 48 | 16.5 | 828.43 | 926.51 | 41 | 2500 |
| 49 | 16.1 | 791.93 | 756.4 | 40 | 2500 |
| 50 | 16.1 | 791.93 | 813.42 | 40 | 2500 |
| 51 | 16.1 | 791.93 | 926.51 | 40 | 2500 |
| 52 | 12 | 515.26 | 506.28 | 28 | 2500 |
| 53 | 12 | 515.26 | 779.37 | 28 | 2500 |
| 54 | 12 | 515.26 | 892.46 | 28 | 2500 |
| 55 | 16.6 | 674.67 | 660.36 | 38 | 2500 |
| 56 | 16.6 | 674.67 | 823.42 | 38 | 2500 |
| 57 | 16.6 | 674.67 | 952.46 | 38 | 2500 |
| 58 | 12.5 | 608.82 | 803.43 | 32 | 2500 |
| 59 | 12.5 | 608.82 | 966.49 | 32 | 2500 |
| 60 | 12.5 | 608.82 | 552.28 | 32 | 2500 |
| 61 | 14.6 | 638.34 | 746.42 | 33 | 2500 |
| 62 | 14.6 | 638.34 | 972.51 | 33 | 2500 |
| 63 | 14.6 | 638.34 | 460.73 | 33 | 2500 |
| 64 | 18 | 692.99 | 630.28 | 39 | 2500 |
| 65 | 18 | 692.99 | 743.37 | 39 | 2500 |
| 66 | 18 | 692.99 | 814.41 | 39 | 2500 |
| 67 | 19.5 | 676.32 | 630.28 | 38 | 2500 |
| 68 | 19.5 | 676.32 | 743.37 | 38 | 2500 |
| 69 | 19.5 | 676.32 | 814.41 | 38 | 2500 |
| 70 | 22.5 | 643.89 | 660.4 | 33 | 2500 |
| 71 | 22.5 | 643.89 | 773.49 | 33 | 2500 |
| 72 | 22.5 | 643.89 | 959.55 | 33 | 2500 |
| 73 | 18.8 | 571.84 | 701.46 | 30 | 2500 |
| 74 | 18.8 | 571.84 | 830.5 | 30 | 2500 |
| 75 | 18.8 | 571.84 | 901.54 | 30 | 2500 |
| 76 | 20.1 | 688.83 | 593.26 | 35 | 2500 |
| 77 | 20.1 | 688.83 | 779.34 | 35 | 2500 |
| 78 | 20.1 | 688.83 | 894.37 | 35 | 2500 |
| 79 | 14.1 | 363.24 | 498.34 | 21 | 2500 |
| 80 | 14.1 | 363.24 | 555.36 | 21 | 2500 |
| 81 | 14.1 | 363.24 | 626.4 | 21 | 2500 |
| 82 | 10.6 | 536.77 | 602.38 | 29 | 2500 |
| 83 | 10.6 | 536.77 | 787.41 | 29 | 2500 |
| 84 | 10.6 | 536.77 | 858.44 | 29 | 2500 |
| 85 | 18.8 | 654.35 | 746.4 | 34 | 2500 |
| 86 | 18.8 | 654.35 | 875.45 | 34 | 2500 |
| 87 | 18.8 | 654.35 | 988.53 | 34 | 2500 |
| 88 | 18 | 856.4 | 698.84 | 43 | 2500 |
| 89 | 18 | 856.4 | 630.28 | 43 | 2500 |
| 90 | 18 | 856.4 | 814.41 | 43 | 2500 |
| 91 | 9.8 | 412.21 | 476.27 | 23 | 2500 |
| 92 | 9.8 | 412.21 | 573.32 | 23 | 2500 |
| 93 | 9.8 | 412.21 | 660.36 | 23 | 2500 |

Lorsque les aires d'au moins deux transitions d'un même peptide sont supérieures ou égales au seuil de positivité décrit dans le **TABLEAU 11**, la détection du peptide est considérée comme positive. Lorsque plus de deux transitions d'un même peptide comportent une aire inférieure au seuil de positivité décrit dans le **TABLEAU 11**, le peptide correspondant est considéré comme non détecté.

Un échantillon contient des bactéries qui expriment la protéine TEM, lorsque qu'au moins un peptide, correspondant au mécanisme de résistance TEM, est détecté. Ces bactéries sont résistantes aux pénicillines.

Un échantillon contient des bactéries qui expriment la protéine TEM, phénotype 2b, lorsque qu'au moins un peptide, correspondant au mécanisme de résistance TEM intérêt clinique 2b, est détecté. Ces bactéries sont seulement résistantes aux pénicillines.

Un échantillon contient des bactéries qui expriment la protéine TEM, phénotype 2br, lorsque qu'au moins un peptide, correspondant au mécanisme de résistance TEM intérêt clinique 2br, est détecté. Ces bactéries sont résistantes aux pénicillines associé à un inhibiteur de type acide clavulanique et tazobactame.

Un échantillon contient des bactéries qui expriment la protéine CTX-M ou la protéine TEM, phénotype 2be, lorsque qu'au moins un peptide, correspondant au mécanisme de résistance CTX-M ou TEM intérêt clinique 2be, est détecté. Ces bactéries sont résistantes aux pénicillines, aux céphalosporines et aux monobactames.

Un échantillon contient des bactéries qui expriment la protéine TEM, phénotype 2ber, lorsque qu'au moins un peptide, correspondant au mécanisme de résistance TEM intérêt clinique 2ber, est détecté. Ces bactéries sont résistantes aux pénicillines, aux céphalosporines et aux monobactames et sont insensibles à l'inhibition par l'acide calvulanique, le sulfobactame ou le tazobactame.

### Exemple 12 : Identification d'une résistance aux béta-lactamines de type PER:

Les échantillons Ech74 à Ech78 sont identifiés selon l'une des méthodes décrites dans les exemples 1, 3 ou 4. L'identification des espèces est reportée dans le **TABLEAU 12.**

**TABLEAU 12 :**

| Noms | Espèces |
|---|---|
| Ech74 | *A. baumannii* |
| Ech75 | *A. baumannii* |
| Ech76 | *P. aeruginosa* |
| Ech77 | *P. aeruginosa* |
| Ech78 | *P. aeruginosa* |

Les échantillons Ech74 à Ech78 correspondent à une espèce pouvant comporter un mécanisme de résistance de type PER. Chaque échantillon est traité selon l'exemple 5, puis analysé selon l'exemple 6 en détectant les peptides du **TABLEAU 13** en lieu et place des peptides du **TABLEAU 3.**

**TABLEAU 13 :**

| Transition numero | Peptide | Temps de rétention (minutes) | (m/z) filtré en Q1 | (m/z) filtré en Q3 | Energie de collision (eV) | seuil de positivité |
|---|---|---|---|---|---|---|
| 1 | AAAQLLR | 12,91 | 371,73 | 401,29 | 16,9 | 2000 |
| 2 | AAAQLLR | 12,89 | 371,73 | 529,35 | 16,9 | 2000 |
| 3 | AAAQLLR | 12,91 | 371,73 | 600,38 | 16,9 | 2000 |
| 4 | AAAQVLQK | 10,24 | 414,75 | 487,32 | 19,4 | 2000 |
| 5 | AAAQVLQK | 10,24 | 414,75 | 615,38 | 19,4 | 2000 |
| 6 | AAAQVLQK | 10,18 | 414,75 | 686,42 | 19,4 | 2000 |
| 7 | AAVLQNTWSPMMK | 19,35 | 738,87 | 506,25 | 37,9 | 2000 |
| 8 | AAVLQNTWSPMMK | 19,35 | 738,87 | 593,28 | 37,9 | 2000 |
| 9 | AAVLQNTWSPMMK | 19,35 | 738,87 | 994,45 | 37,9 | 2000 |
| 10 | EQIESIVIGK | 17,24 | 558,32 | 317,22 | 27,6 | 2000 |
| 11 | EQIESIVIGK | 17,26 | 558,32 | 616,40 | 27,6 | 2000 |
| 12 | EQIESIVIGK | 17,24 | 558,32 | 745,45 | 27,6 | 2000 |
| 13 | EQIETIVTGK | 15,48 | 559,31 | 305,18 | 27,6 | 2000 |
| 14 | EQIETIVTGK | 15,48 | 559,31 | 618,38 | 27,6 | 2000 |
| 15 | EQIETIVTGK | 15,48 | 559,31 | 747,43 | 27,6 | 2000 |
| 16 | ETEVVANEAQMHADDQVQYK | 14,17 | 769,02 | 838,87 | 30,9 | 5000 |
| 17 | ETEVVANEAQMHADDQVQYK | 14,17 | 769,02 | 874,39 | 30,9 | 5000 |
| 18 | ETEVVANEAQMHADDQVQYK | 14,17 | 769,02 | 923,92 | 30,9 | 5000 |
| 19 | FPMQSVFK | 19,09 | 492,26 | 418,72 | 23,8 | 2000 |
| 20 | FPMQSVFK | 19,09 | 492,26 | 739,38 | 23,8 | 2000 |
| 21 | FPMQSVFK | 19,07 | 492,26 | 836,43 | 23,8 | 2000 |
| 22 | GAAEILK | 12,66 | 351,21 | 322,70 | 15,8 | 2000 |
| 23 | GAAEILK | 12,65 | 351,21 | 502,32 | 15,8 | 2000 |
| 24 | GAAEILK | 12,65 | 351,21 | 573,36 | 15,8 | 2000 |
| 25 | GLLPAGTIVAHK | 16,8 | 588,86 | 447,26 | 29,3 | 2000 |
| 26 | GLLPAGTIVAHK | 16,8 | 588,86 | 503,81 | 29,3 | 2000 |
| 27 | GLLPAGTIVAHK | 16,82 | 588,86 | 893,52 | 29,3 | 2000 |
| 28 | GLLPAGTVVAHK | 15,73 | 581,85 | 440,26 | 28,9 | 2000 |
| 29 | GLLPAGTVVAHK | 15,73 | 581,85 | 496,80 | 28,9 | 2000 |
| 30 | GLLPAGTVVAHK | 15,75 | 581,85 | 879,51 | 28,9 | 2000 |
| 31 | GQIESIVIGK | 16,8 | 522,31 | 616,40 | 25,5 | 5000 |
| 32 | GQIESIVIGK | 16,8 | 522,31 | 745,45 | 25,5 | 5000 |
| 33 | GQIESIVIGK | 16,8 | 522,31 | 858,53 | 25,5 | 5000 |
| 34 | LDLNK | 10,5 | 301,68 | 342,20 | 12,9 | 2000 |
| 35 | LDLNK | 10,5 | 301,68 | 374,24 | 12,9 | 2000 |
| 36 | LDLNK | 10,52 | 301,68 | 489,27 | 12,9 | 2000 |
| 37 | LDLNQSVTVNR | 15,11 | 629,84 | 338,19 | 31,6 | 2000 |
| 38 | LDLNQSVTVNR | 15,11 | 629,84 | 489,28 | 31,6 | 2000 |
| 39 | LDLNQSVTVNR | 15,11 | 629,84 | 675,38 | 31,6 | 2000 |
| 40 | LDLNQTVIVNR | 17,29 | 642,87 | 501,31 | 32,4 | 2000 |
| 41 | LDLNQTVIVNR | 17,29 | 642,87 | 600,38 | 32,4 | 2000 |
| 42 | LDLNQTVIVNR | 17,28 | 642,87 | 701,43 | 32,4 | 2000 |
| 43 | LHLAMLVLHQVDQGK | 19,84 | 567,99 | 568,83 | 24,7 | 2000 |
| 44 | LHLAMLVLHQVDQGK | 19,84 | 567,99 | 669,86 | 24,7 | 2000 |
| 45 | LHLAMLVLHQVDQGK | 19,84 | 567,99 | 726,41 | 24,7 | 2000 |
| 46 | MHLAMLVLHQVDQGK | 19,35 | 573,97 | 332,19 | 24,9 | 2000 |
| 47 | MHLAMLVLHQVDQGK | 19,35 | 573,97 | 669,86 | 24,9 | 2000 |
| 48 | MHLAMLVLHQVDQGK | 19,35 | 573,97 | 726,41 | 24,9 | 2000 |
| 49 | NWTSMK | 12,59 | 383,68 | 365,19 | 17,6 | 2000 |
| 50 | NWTSMK | 12,59 | 383,68 | 466,23 | 17,6 | 2000 |
| 51 | NWTSMK | 12,61 | 383,68 | 652,31 | 17,6 | 2000 |
| 52 | QLSETSQALLWK | 18,84 | 702,38 | 1162,61 | 35,8 | 2000 |
| 53 | QLSETSQALLWK | 18,84 | 702,38 | 446,28 | 35,8 | 2000 |
| 54 | QLSETSQALLWK | 18,84 | 702,38 | 845,49 | 35,8 | 2000 |
| 55 | TGTSGIK | 3,31 | 332,19 | 404,25 | 14,7 | 2000 |
| 56 | TGTSGIK | 3,29 | 332,19 | 505,30 | 14,7 | 2000 |
| 57 | TGTSGIK | 3,23 | 332,19 | 562,32 | 14,7 | 2000 |
| 58 | TGTSGVR | 1,56 | 339,18 | 418,24 | 15,1 | 2000 |
| 59 | TGTSGVR | 1,56 | 339,18 | 519,29 | 15,1 | 2000 |
| 60 | TGTSGVR | 1,56 | 339,18 | 576,31 | 15,1 | 2000 |
| 61 | TNEAIIAQVAQAAYQFELK | 26,27 | 703,37 | 1168,60 | 28,9 | 2000 |
| 62 | TNEAIIAQVAQAAYQFELK | 26,21 | 703,37 | 827,43 | 28,9 | 2000 |
| 63 | TNEAIIAQVAQAAYQFELK | 26,25 | 703,37 | 898,47 | 28,9 | 2000 |
| 64 | TNEAIIAQVAQTAYQFELK | 25,39 | 713,38 | 1198,61 | 29,2 | 2000 |
| 65 | TNEAIIAQVAQTAYQFELK | 25,37 | 713,38 | 827,43 | 29,2 | 2000 |
| 66 | TNEAIIAQVAQTAYQFELK | 25,37 | 713,38 | 898,47 | 29,2 | 2000 |
| 67 | TQLSETSQALLWK | 19,41 | 502,27 | 559,36 | 38,7 | 2000 |
| 68 | TQLSETSQALLWK | 19,41 | 502,27 | 630,40 | 22,7 | 2000 |
| 69 | TQLSETSQALLWK | 19,43 | 752,90 | 1162,61 | 22,7 | 2000 |
| 70 | TVAVNR | 7,08 | 330,20 | 388,23 | 14,6 | 2000 |
| 71 | TVAVNR | 7,08 | 330,20 | 459,27 | 14,6 | 2000 |
| 72 | TVAVNR | 7,05 | 330,20 | 558,34 | 14,6 | 2000 |
| 73 | VLQNTWAPIMK | 19,32 | 650,86 | 488,29 | 32,8 | 2000 |
| 74 | VLQNTWAPIMK | 19,3 | 650,86 | 559,33 | 32,8 | 2000 |
| 75 | VLQNTWAPIMK | 19,3 | 650,86 | 745,41 | 32,8 | 2000 |
| 76 | WMVETTTGPER | 15,5 | 653,81 | 761,38 | 33 | 2000 |
| 77 | WMVETTTGPER | 15,48 | 653,81 | 890,42 | 33 | 2000 |
| 78 | WMVETTTGPER | 15,48 | 653,81 | 989,49 | 33 | 2000 |
| 79 | WMVETTTGPQR | 15,19 | 653,32 | 457,25 | 33 | 2000 |
| 80 | WMVETTTGPQR | 15,19 | 653,32 | 889,44 | 33 | 2000 |
| 81 | WMVETTTGPQR | 15,23 | 653,32 | 988,51 | 33 | 2000 |

Les autres paramètres machine utilisés sont les suivants :
- Type de balayage: MRM
- MRM planifié : oui
- Polarité: Positive
- Source d'ionisation:Turbo V™ (Applied BioSystems)
- Réglage Q1: Filtrage avec résolution unitaire
- Réglage Q3: Filtrage avec résolution unitaire
- Pause inter-scan: 5.00 msec
- Vitesse de balayage: 10 Da/s
- Gaz rideau: 50,00 psi
- Tension de cône: 5500,00 V
- Température de source: 500,00 °C
- Gaz de nébulisation: 40,00 psi
- Gaz chauffant: 40,00 psi
- Gaz de collision induisant dissociation : 9,00 psi
- Remplissage dynamique: activé
- Potentiel d'orifice : 100, 00 V
- (en anglais declustering potential (DP))
- Potentiel d'entrée avant Q0 (EP): 10,00 V
- Potentiel en sortie de cellule de collision : 15 V
- (en anglais cell exit potential (CXP))
- Temps de cycle total: 1 sec
- Fenêtre de détection : 240 sec

Les aires obtenues pour chacune des transitions et pour chacun des microorganismes étudiés ont été mesurées. Lorsque les aires des transitions sont supérieures ou égales au seuil de positivité décrit dans le **TABLEAU 13,** la détection de la transition est considérée comme positive et est notée « 1 » dans le **TABLEAU 14.** Lorsqu'une transition a une aire inférieure au seuil de positivité décrit dans le **TABLEAU 13,** la transition est considérée comme non détectée et est notée « 0 » dans le **TABLEAU 14.**

Pour un peptide donné, lorsque au moins 3 transitions sont notées « 1 », le peptide est considéré comme détecté.

**TABLEAU 14 :**

| Numéro transition | Ech74 | Ech75 | Ech76 | Ech77 | Ech78 |
|---|---|---|---|---|---|
| 1 | 0 | 0 | 0 | 0 | 0 |
| 2 | 0 | 0 | 0 | 0 | 0 |
| 3 | 0 | 0 | 0 | 0 | 0 |
| 4 | 0 | 0 | 0 | 0 | 0 |
| 5 | 0 | 0 | 0 | 0 | 0 |
| 6 | 0 | 0 | 0 | 0 | 0 |
| 7 | 0 | 1 | 0 | 1 | 0 |
| 8 | 0 | 0 | 0 | 0 | 1 |
| 9 | 1 | 1 | 1 | 1 | 1 |
| 10 | 1 | 1 | 1 | 1 | 1 |
| 11 | 1 | 1 | 1 | 1 | 1 |
| 12 | 1 | 1 | 1 | 1 | 1 |
| 13 | 0 | 0 | 0 | 0 | 0 |
| 14 | 0 | 0 | 0 | 0 | 0 |
| 15 | 0 | 0 | 0 | 0 | 0 |
| 16 | 0 | 0 | 0 | 0 | 0 |
| 17 | 1 | 1 | 0 | 0 | 0 |
| 18 | 0 | 0 | 0 | 0 | 0 |
| 19 | 1 | 1 | 0 | 0 | 0 |
| 20 | 1 | 1 | 0 | 0 | 0 |
| 21 | 1 | 1 | 0 | 0 | 0 |
| 22 | 1 | 1 | 1 | 1 | 1 |
| 23 | 1 | 1 | 1 | 1 | 1 |
| 24 | 1 | 1 | 1 | 1 | 1 |
| 25 | 0 | 0 | 0 | 0 | 0 |
| 26 | 0 | 0 | 0 | 0 | 0 |
| 27 | 0 | 0 | 0 | 0 | 0 |
| 28 | 1 | 1 | 1 | 1 | 1 |
| 29 | 1 | 1 | 1 | 1 | 1 |
| 30 | 1 | 1 | 1 | 1 | 1 |
| 31 | 0 | 0 | 0 | 0 | 0 |
| 32 | 0 | 0 | 0 | 0 | 0 |
| 33 | 1 | 1 | 0 | 0 | 0 |
| 34 | 0 | 0 | 0 | 0 | 0 |
| 35 | 0 | 0 | 0 | 0 | 0 |
| 36 | 0 | 0 | 0 | 0 | 0 |
| 37 | 0 | 0 | 1 | 1 | 1 |
| 38 | 0 | 0 | 0 | 0 | 0 |
| 39 | 0 | 0 | 0 | 0 | 0 |
| 40 | 1 | 1 | 1 | 1 | 1 |
| 41 | 1 | 1 | 1 | 1 | 1 |
| 42 | 1 | 1 | 1 | 1 | 1 |
| 43 | 0 | 0 | 0 | 0 | 0 |
| 44 | 0 | 0 | 0 | 0 | 0 |
| 45 | 0 | 0 | 0 | 0 | 0 |
| 46 | 0 | 0 | 0 | 0 | 0 |
| 47 | 0 | 0 | 0 | 0 | 0 |
| 48 | 0 | 0 | 0 | 0 | 0 |
| 49 | 0 | 0 | 0 | 0 | 0 |
| 50 | 0 | 0 | 0 | 0 | 0 |
| 51 | 0 | 0 | 0 | 0 | 0 |
| 52 | 0 | 0 | 0 | 0 | 0 |
| 53 | 0 | 0 | 0 | 0 | 0 |
| 54 | 0 | 0 | 0 | 0 | 0 |
| 55 | 1 | 1 | 0 | 1 | 1 |
| 56 | 1 | 1 | 0 | 0 | 0 |
| 57 | 1 | 1 | 0 | 0 | 0 |
| 58 | 0 | 0 | 0 | 0 | 0 |
| 59 | 0 | 0 | 0 | 0 | 0 |
| 60 | 0 | 0 | 0 | 0 | 0 |
| 61 | 0 | 0 | 0 | 0 | 0 |
| 62 | 0 | 0 | 0 | 0 | 0 |
| 63 | 0 | 0 | 0 | 0 | 0 |
| 64 | 0 | 0 | 0 | 0 | 0 |
| 65 | 0 | 0 | 0 | 0 | 0 |
| 66 | 0 | 0 | 0 | 0 | 0 |
| 67 | 1 | 1 | 1 | 1 | 1 |
| 68 | 1 | 1 | 1 | 1 | 1 |
| 69 | 1 | 1 | 1 | 1 | 0 |
| 70 | 0 | 0 | 0 | 0 | 0 |
| 71 | 0 | 0 | 0 | 0 | 0 |
| 72 | 0 | 0 | 0 | 0 | 0 |
| 73 | 1 | 1 | 1 | 1 | 1 |
| 74 | 1 | 1 | 1 | 1 | 1 |
| 75 | 1 | 1 | 1 | 1 | 1 |
| 76 | 1 | 1 | 1 | 1 | 1 |
| 77 | 1 | 1 | 1 | 1 | 1 |
| 78 | 1 | 1 | 1 | 1 | 1 |
| 79 | 0 | 0 | 0 | 0 | 0 |
| 80 | 0 | 0 | 0 | 0 | 0 |
| 81 | 0 | 0 | 0 | 0 | 0 |

Les échantillons Ech74 à Ech78 comportent au moins un peptide caractéristique des PER. Les bactéries présentes dans les échantillons Ech74 à Ech78 expriment donc une béta-lactamase qui leur confère une résistance aux pénicillines, aux céphalosporines et aux monobactames

### Exemple 13 : Identification d'une résistance aux béta-lactamines de type VEB:

Les échantillons Ech79 à Ech82 sont identifiés selon l'une des méthodes décrites dans les exemples 1, 3 ou 4. L'identification des espèces est reportée dans le **TABLEAU 15.**

**TABLEAU 15 :**

| Noms | Espèces |
|---|---|
| Ech79 | *A. baumannii* |
| Ech80 | *A. baumannii* |
| Ech81 | *A. baumannii* |
| Ech82 | *E. coli* |

Les échantillons Ech79 à Ech82 correspondent à une espèce pouvant comporter un mécanisme de résistance de type VEB. Chaque échantillon est traité selon l'exemple 5, puis analysé selon l'exemple 6 en détectant les peptides du **TABLEAU 16** en lieu et place des peptides du **TABLEAU 3.**

**TABLEAU 16 :**

| Transition numéro | Peptide | Temps de rétention (minutes) | (m/z) filtré en Q1 | (m/z) filtré en Q3 | Energie de collision (eV) | Seuil de positivité |
|---|---|---|---|---|---|---|
| 1 | ANEEQMHK | 1,44 | 493,72 | 458,20 | 23,9 | 2000 |
| 2 | ANEEQMHK | 1,44 | 493,72 | 672,31 | 23,9 | 2000 |
| 3 | ANEEQMHK | 1,44 | 493,72 | 801,36 | 23,9 | 2000 |
| 4 | DWNTQYQNWATPTAMNK | 19,05 | 1034,96 | 661,33 | 54,7 | 2000 |
| 5 | DWNTQYQNWATPTAMNK | 19 | 690,31 | 661,33 | 28,5 | 2000 |
| 6 | DWNTQYQNWATPTAMNK | 19,02 | 690,31 | 762,38 | 28,5 | 2000 |
| 7 | ETSEINEK | 6,84 | 475,23 | 390,20 | 22,8 | 2000 |
| 8 | ETSEINEK | 6,84 | 475,23 | 503,28 | 22,8 | 2000 |
| 9 | ETSEINEK | 6,83 | 475,23 | 719,36 | 22,8 | 2000 |
| 10 | ETTTGSNR | 1,38 | 433,20 | 433,22 | 20,4 | 1500 |
| 11 | ETTTGSNR | 1,38 | 433,20 | 534,26 | 20,4 | 1500 |
| 12 | ETTTGSNR | 1,38 | 433,20 | 635,31 | 20,4 | 1500 |
| 13 | FLNANHFTDISIK | 18,72 | 507,27 | 573,30 | 22,8 | 2000 |
| 14 | FLNANHFTDISIK | 18,72 | 507,27 | 630,32 | 22,8 | 2000 |
| 15 | FLNANHFTDISIK | 18,74 | 507,27 | 686,87 | 22,8 | 2000 |
| 16 | FPIALAVLSEIDK | 27,01 | 708,41 | 634,88 | 36,1 | 2000 |
| 17 | FPIALAVLSEIDK | 27,04 | 708,41 | 704,38 | 36,1 | 2000 |
| 18 | FPIALAVLSEIDK | 27,01 | 708,41 | 874,49 | 36,1 | 2000 |
| 19 | GNLSFEQK | 12,65 | 461,74 | 551,28 | 22,1 | 2000 |
| 20 | GNLSFEQK | 12,65 | 461,74 | 638,31 | 22,1 | 2000 |
| 21 | GNLSFEQK | 12,65 | 461,74 | 751,40 | 22,1 | 2000 |
| 22 | GQLPK | 7,19 | 271,67 | 243,16 | 11,2 | 2000 |
| 23 | GQLPK | 7,19 | 271,67 | 357,25 | 11,2 | 2000 |
| 24 | GQLPK | 7,19 | 271,67 | 485,31 | 11,2 | 2000 |
| 25 | IEITPQDLLPK | 21,67 | 633,87 | 405,74 | 31,9 | 2000 |
| 26 | IEITPQDLLPK | 21,67 | 633,87 | 810,47 | 31,9 | 2000 |
| 27 | IEITPQDLLPK | 21,63 | 633,87 | 911,52 | 31,9 | 2000 |
| 28 | IENVLK | 12,89 | 358,22 | 359,27 | 16,2 | 2000 |
| 29 | IENVLK | 12,91 | 358,22 | 473,31 | 16,2 | 2000 |
| 30 | IENVLK | 12,89 | 358,22 | 602,35 | 16,2 | 2000 |
| 31 | IGVAIFNSNEK | 18,09 | 596,32 | 738,34 | 29,7 | 2000 |
| 32 | IGVAIFNSNEK | 18,09 | 596,32 | 851,43 | 29,7 | 2000 |
| 33 | IGVAIFNSNEK | 18,09 | 596,32 | 922,46 | 29,7 | 2000 |
| 34 | IISDIAK | 12,6 | 380,23 | 331,23 | 17,4 | 2000 |
| 35 | IISDIAK | 12,58 | 380,23 | 533,29 | 17,4 | 2000 |
| 36 | IISDIAK | 12,56 | 380,23 | 646,38 | 17,4 | 2000 |
| 37 | INNDFHFPMQSVMK | 20,04 | 569,94 | 740,84 | 24,8 | 2000 |
| 38 | INNDFHFPMQSVMK | 20,04 | 569,94 | 797,86 | 24,8 | 2000 |
| 39 | INNDFHFPMQSVMK | 20,05 | 569,94 | 820,41 | 24,8 | 2000 |
| 40 | LIGGTDSVQK | 11,48 | 509,28 | 734,37 | 24,8 | 2000 |
| 41 | LIGGTDSVQK | 11,46 | 509,28 | 791,39 | 24,8 | 2000 |
| 42 | LIGGTDSVQK | 11,46 | 509,28 | 904,47 | 24,8 | 2000 |
| 43 | LLIDTYNNK | 15,7 | 547,30 | 639,31 | 26,9 | 2000 |
| 44 | LLIDTYNNK | 15,7 | 547,30 | 754,34 | 26,9 | 2000 |
| 45 | LLIDTYNNK | 15,7 | 547,30 | 867,42 | 26,9 | 2000 |
| 46 | MWSPIK | 16,91 | 381,20 | 357,25 | 17,5 | 2000 |
| 47 | MWSPIK | 16,91 | 381,20 | 444,28 | 17,5 | 2000 |
| 48 | MWSPIK | 16,87 | 381,20 | 630,36 | 17,5 | 2000 |
| 49 | NQLLSK | 10,59 | 351,71 | 347,23 | 15,8 | 2000 |
| 50 | NQLLSK | 10,59 | 351,71 | 460,31 | 15,8 | 2000 |
| 51 | NQLLSK | 10,59 | 351,71 | 588,37 | 15,8 | 2000 |
| 52 | NTIVAHK | 23,75 | 391,73 | 454,28 | 18,1 | 2000 |
| 53 | NTIVAHK | 23,73 | 391,73 | 567,36 | 18,1 | 2000 |
| 54 | NTIVAHK | 23,75 | 391,73 | 668,41 | 18,1 | 2000 |
| 55 | SYDFIWK | 19,92 | 479,74 | 446,28 | 23,1 | 2000 |
| 56 | SYDFIWK | 19,9 | 479,74 | 593,35 | 23,1 | 2000 |
| 57 | SYDFIWK | 19,9 | 479,74 | 708,37 | 23,1 | 2000 |
| 58 | TWSPIK | 14,95 | 366,21 | 357,25 | 16,6 | 2000 |
| 59 | TWSPIK | 14,93 | 366,21 | 444,28 | 16,6 | 2000 |
| 60 | TWSPIK | 14,95 | 366,21 | 630,36 | 16,6 | 2000 |

Les autres paramètres machine utilisés sont les suivants :
- Type de balayage: MRM
- MRM planifié : oui
- Polarité: Positive
- Source d'ionisation:Turbo V™ (Applied BioSystems)
- Réglage Q1: Filtrage avec résolution unitaire
- Réglage Q3: Filtrage avec résolution unitaire
- Pause inter-scan: 5.00 msec
- Vitesse de balayage: 10 Da/s
- Gaz rideau: 50,00 psi
- Tension de cône: 5500,00 V
- Température de source: 500,00 °C
- Gaz de nébulisation: 40,00 psi
- Gaz chauffant: 40,00 psi
- Gaz de collision induisant dissociation : 9,00 psi
- Remplissage dynamique: activé
- Potentiel d'orifice : 100, 00 V
- (en anglais declustering potential (DP))
- Potentiel d'entrée avant Q0 (EP): 10,00 V
- Potentiel en sortie de cellule de collision : 15 V
- (en anglais cell exit potential (CXP))
- Temps de cycle total: 1.2 sec
- Fenêtre de détection : 240 sec

Les aires obtenues pour chacune des transitions et pour chacun des microorganismes étudiés ont été mesurées. Lorsque les aires des transitions sont supérieures ou égales au seuil de positivité décrit dans le **TABLEAU 16,** la détection de la transition est considérée comme positive et est notée « 1 » dans le **TABLEAU 17.** Lorsqu'une transition a une aire inférieure au seuil de positivité décrit dans le **TABLEAU 16,** la transition est considérée comme non détectée et est notée « 0 » dans le **TABLEAU 17.**

Pour un peptide donné, lorsque au moins 3 transitions sont notées « 1 », le peptide est considéré comme détecté.

**TABLEAU 17 :**

| Transition numéro | Ech79 | Ech80 | Ech81 | Ech82 |
|---|---|---|---|---|
| 1 | 0 | 0 | 0 | 0 |
| 2 | 0 | 0 | 0 | 0 |
| 3 | 0 | 0 | 0 | 0 |
| 4 | 0 | 0 | 0 | 0 |
| 5 | 0 | 0 | 0 | 0 |
| 6 | 0 | 0 | 0 | 0 |
| 7 | 0 | 0 | 0 | 0 |
| 8 | 0 | 0 | 0 | 0 |
| 9 | 0 | 0 | 0 | 0 |
| 10 | 1 | 1 | 1 | 1 |
| 11 | 1 | 1 | 1 | 1 |
| 12 | 1 | 1 | 1 | 0 |
| 13 | 0 | 1 | 1 | 0 |
| 14 | 0 | 1 | 1 | 0 |
| 15 | 0 | 1 | 1 | 0 |
| 16 | 0 | 0 | 0 | 0 |
| 17 | 0 | 0 | 0 | 0 |
| 18 | 0 | 0 | 0 | 0 |
| 19 | 0 | 0 | 0 | 0 |
| 20 | 0 | 0 | 0 | 0 |
| 21 | 0 | 0 | 0 | 0 |
| 22 | 0 | 0 | 0 | 0 |
| 23 | 0 | 0 | 0 | 0 |
| 24 | 0 | 0 | 0 | 0 |
| 25 | 1 | 1 | 1 | 1 |
| 26 | 1 | 1 | 1 | 1 |
| 27 | 1 | 1 | 1 | 1 |
| 28 | 0 | 0 | 0 | 0 |
| 29 | 0 | 0 | 0 | 0 |
| 30 | 0 | 0 | 0 | 0 |
| 31 | 0 | 0 | 0 | 0 |
| 32 | 0 | 0 | 0 | 0 |
| 33 | 0 | 0 | 0 | 0 |
| 34 | 0 | 0 | 0 | 0 |
| 35 | 0 | 0 | 0 | 0 |
| 36 | 0 | 0 | 0 | 0 |
| 37 | 0 | 0 | 0 | 0 |
| 38 | 0 | 0 | 0 | 0 |
| 39 | 0 | 0 | 0 | 0 |
| 40 | 1 | 1 | 1 | 0 |
| 41 | 0 | 1 | 0 | 0 |
| 42 | 1 | 0 | 1 | 0 |
| 43 | 0 | 0 | 0 | 0 |
| 44 | 0 | 0 | 0 | 0 |
| 45 | 0 | 0 | 0 | 0 |
| 46 | 0 | 0 | 0 | 0 |
| 47 | 0 | 0 | 0 | 0 |
| 48 | 0 | 0 | 0 | 0 |
| 49 | 0 | 0 | 0 | 0 |
| 50 | 0 | 0 | 0 | 0 |
| 51 | 0 | 0 | 0 | 0 |
| 52 | 0 | 0 | 0 | 0 |
| 53 | 0 | 0 | 0 | 0 |
| 54 | 0 | 0 | 0 | 0 |
| 55 | 1 | 1 | 1 | 0 |
| 56 | 1 | 1 | 1 | 0 |
| 57 | 1 | 1 | 1 | 0 |
| 58 | 0 | 0 | 0 | 0 |
| 59 | 0 | 0 | 0 | 0 |
| 60 | 0 | 0 | 0 | 0 |

Les échantillons Ech79 à Ech82 comportent au moins un peptide caractéristique des VEB. Les bactéries présentes dans les échantillons Ech79 à Ech82 expriment donc une béta-lactamase qui leur confère une résistance aux pénicillines, aux céphalosporines, et aux monobactames

### Exemple 14 : Identification d'une résistance aux béta-lactamines de type MOX:

L'échantillon Ech83 est identifié selon l'une des méthodes décrites dans les exemples 1, 3 ou 4. L'identification de l'espèce est reportée dans le **TABLEAU 18.**

**TABLEAU 18 :**

| Noms | Espèces |
|---|---|
| Ech83 | E. coli |

L'échantillon Ech83 correspond à une espèce pouvant comporter un mécanisme de résistance de type MOX. Chaque échantillon est traité selon l'exemple 5, puis analysé selon l'exemple 6 en détectant les peptides du **TABLEAU 19** en lieu et place des peptides du **TABLEAU 3.**

**TABLEAU 19 :**

| Transition numéro | Peptide | Temps de retention (minutes) | (m/z) filtré en Q1 | (m/z) filtré en Q3 | Energie de Collision (eV) | Seuil de positivité |
|---|---|---|---|---|---|---|
| 1 | AHYFNYGVADR | 14,36 | 656,807 | 621,289 | 33,2 | 2000 |
| 2 | AHYFNYGVADR | 14,36 | 656,807 | 941,448 | 33,2 | 2000 |
| 3 | AHYFNYGVADR | 14,34 | 656,807 | 1104,511 | 33,2 | 2000 |
| 4 | ANISGVDDK | 9,88 | 459,73 | 533,257 | 21,9 | 2000 |
| 5 | ANISGVDDK | 9,86 | 459,73 | 620,289 | 21,9 | 2000 |
| 6 | ANISGVDDK | 9,86 | 459,73 | 733,373 | 21,9 | 2000 |
| 7 | ANISGVHDK | 7,75 | 470,746 | 555,289 | 22,6 | 2000 |
| 8 | ANISGVHDK | 7,67 | 470,746 | 642,321 | 22,6 | 2000 |
| 9 | ANISGVHDK | 7,69 | 470,746 | 755,405 | 22,6 | 2000 |
| 10 | ASLFAPWLK | 23,52 | 516,797 | 543,329 | 25,2 | 2000 |
| 11 | ASLFAPWLK | 23,56 | 516,797 | 614,366 | 25,2 | 2000 |
| 12 | ASLFAPWLK | 23,56 | 516,797 | 761,434 | 25,2 | 2000 |
| 13 | EDKPFR | 8,09 | 396,206 | 419,24 | 18,3 | 2000 |
| 14 | EDKPFR | 8,07 | 396,206 | 547,335 | 18,3 | 2000 |
| 15 | EDKPFR | 8,09 | 396,206 | 662,362 | 18,3 | 2000 |
| 16 | ESGNLMLFNK | 18,39 | 576,79 | 408,224 | 28,6 | 2000 |
| 17 | ESGNLMLFNK | 18,39 | 576,79 | 521,308 | 28,6 | 2000 |
| 18 | ESGNLMLFNK | 18,39 | 576,79 | 652,349 | 28,6 | 2000 |
| 19 | ESGSQMLFNK | 15,08 | 570,771 | 408,224 | 28,3 | 2000 |
| 20 | ESGSQMLFNK | 15,14 | 570,771 | 521,308 | 28,3 | 2000 |
| 21 | ESGSQMLFNK | 15,12 | 570,771 | 652,349 | 28,3 | 2000 |
| 22 | GHPVLFNK | 12,74 | 456,259 | 408,224 | 21,7 | 2000 |
| 23 | GHPVLFNK | 12,78 | 456,259 | 521,308 | 21,7 | 2000 |
| 24 | GHPVLFNK | 12,78 | 456,259 | 717,429 | 21,7 | 2000 |
| 25 | GIGVVMLANR | 18,19 | 515,297 | 604,324 | 25,1 | 2000 |
| 26 | GIGVVMLANR | 18,17 | 515,297 | 703,392 | 25,1 | 2000 |
| 27 | GIGVVMLANR | 18,17 | 515,297 | 859,482 | 25,1 | 2000 |
| 28 | MQAYYR | 11,12 | 416,195 | 501,246 | 19,5 | 2000 |
| 29 | MQAYYR | 11,14 | 416,195 | 572,283 | 19,5 | 2000 |
| 30 | MQAYYR | 11,12 | 416,195 | 700,341 | 19,5 | 2000 |
| 31 | NSPIEAR | 9,4 | 393,709 | 375,199 | 18,2 | 2000 |
| 32 | NSPIEAR | 9,4 | 393,709 | 488,283 | 18,2 | 2000 |
| 33 | NSPIEAR | 9,4 | 393,709 | 585,335 | 18,2 | 2000 |
| 34 | NSPIEGTLK | 13,36 | 479,764 | 379,226 | 23,1 | 2000 |
| 35 | NSPIEGTLK | 13,36 | 479,764 | 547,309 | 23,1 | 2000 |
| 36 | NSPIEGTLK | 13,34 | 479,764 | 757,445 | 23,1 | 2000 |
| 37 | NYPNEGTLK | 11,6 | 518,259 | 379,706 | 25,3 | 2000 |
| 38 | NYPNEGTLK | 11,62 | 518,259 | 661,352 | 25,3 | 2000 |
| 39 | NYPNEGTLK | 11,58 | 518,259 | 758,404 | 25,3 | 2000 |
| 40 | QPFDR | 9,48 | 331,666 | 437,214 | 14,6 | 2000 |
| 41 | QPFDR | 9,5 | 331,666 | 488,214 | 14,6 | 2000 |
| 42 | QPFDR | 9,48 | 331,666 | 534,267 | 14,6 | 2000 |
| 43 | QWTPAYSPGSHR | 13,34 | 693,831 | 486,238 | 35,3 | 2000 |
| 44 | QWTPAYSPGSHR | 13,34 | 693,831 | 640,316 | 35,3 | 2000 |
| 45 | QWTPAYSPGSHR | 13,34 | 693,831 | 971,469 | 35,3 | 2000 |
| 46 | QWTPAYSR | 13,46 | 504,749 | 496,251 | 24,5 | 2000 |
| 47 | QWTPAYSR | 13,48 | 504,749 | 593,304 | 24,5 | 2000 |
| 48 | QWTPAYSR | 13,46 | 504,749 | 694,352 | 24,5 | 2000 |
| 49 | QYANPSIGLFGYLAASSMK | 25,46 | 673,34 | 594,292 | 28 | 2000 |
| 50 | QYANPSIGLFGYLAASSMK | 25,5 | 673,34 | 594,31 | 28 | 2000 |
| 51 | QYANPSIGLFGYLAASSMK | 25,5 | 673,34 | 927,46 | 28 | 2000 |
| 52 | QYSNPSIGLFGHLAASSMK | 21,21 | 670,003 | 609,819 | 27,9 | 2000 |
| 53 | QYSNPSIGLFGHLAASSMK | 21,17 | 670,003 | 758,403 | 27,9 | 2000 |
| 54 | QYSNPSIGLFGHLAASSMK | 21,21 | 670,003 | 858,941 | 27,9 | 2000 |
| 55 | TGSSNGFGAYVAFVPAR | 20,79 | 850,923 | 343,209 | 100 | 2000 |
| 56 | TGSSNGFGAYVAFVPAR | 20,78 | 850,923 | 380,229 | 100 | 2000 |
| 57 | TGSSNGFGAYVAFVPAR | 20,78 | 850,923 | 442,277 | 100 | 2000 |
| 58 | TGSTNGFGAYVAFVPAK | 20,45 | 843,928 | 315,203 | 100 | 2000 |
| 59 | TGSTNGFGAYVAFVPAK | 20,46 | 843,928 | 366,226 | 100 | 2000 |
| 60 | TGSTNGFGAYVAFVPAK | 20,46 | 843,928 | 414,271 | 100 | 2000 |
| 61 | TGSTSGFGAYVAFVPAK | 20,64 | 553,951 | 632,377 | 24,3 | 2000 |
| 62 | TGSTSGFGAYVAFVPAK | 20,66 | 830,422 | 315,203 | 43,1 | 2000 |
| 63 | TGSTSGFGAYVAFVPAK | 20,66 | 830,422 | 414,271 | 43,1 | 2000 |
| 64 | TLTATLGAYAVVQGSFELDDK | 18,71 | 733,711 | 569,275 | 29,8 | 2000 |
| 65 | TLTATLGAYAVVQGSFELDDK | 18,71 | 733,711 | 654,328 | 29,8 | 2000 |
| 66 | TLTATLGAYAVVQGSFELDDK | 18,71 | 733,711 | 1137,542 | 29,8 | 2000 |
| 67 | VSPGMLADEAYGIK | 18,63 | 725,866 | 632,816 | 37,1 | 2000 |
| 68 | VSPGMLADEAYGIK | 18,63 | 725,866 | 866,425 | 37,1 | 2000 |
| 69 | VSPGMLADEAYGIK | 18,65 | 725,866 | 1167,571 | 37,1 | 2000 |
| 70 | VTPAMLAEEPYGIK | 19,05 | 506,934 | 577,334 | 22,8 | 2000 |
| 71 | VTPAMLAEEPYGIK | 19,05 | 759,897 | 659,839 | 39 | 2000 |
| 72 | VTPAMLAEEPYGIK | 19,07 | 759,897 | 906,457 | 39 | 2000 |
| 73 | YAYPVSEQTLLAGNSAK | 18,09 | 604,644 | 547,283 | 25,8 | 2000 |
| 74 | YAYPVSEQTLLAGNSAK | 18,07 | 604,644 | 660,368 | 25,8 | 2000 |
| 75 | YAYPVSEQTLLAGNSAK | 18,09 | 906,462 | 707,88 | 47,4 | 2000 |

Les autres paramètres machine utilisés sont les suivants :
- Type de balayage: MRM
- MRM planifié: oui
- Polarité: Positive
- Source d'ionisation:Turbo V™ (Applied BioSystems)
- Réglage Q1: Filtrage avec résolution unitaire
- Réglage Q3: Filtrage avec résolution unitaire
- Pause inter-scan: 5.00 msec
- Vitesse de balayage: 10 Da/s
- Gaz rideau: 40,00 psi
- Tension de cône: 5500,00 V
- Température de source: 500,00 °C
- Gaz de nébulisation: 40,00 psi
- Gaz chauffant: 40,00 psi
- Gaz de collision induisant dissociation : 9,00 psi
- Remplissage dynamique: activé
- Potentiel d'orifice : 100, 00 V
- (en anglais declustering potential (DP))
- Potentiel d'entrée avant Q0 (EP): 10,00 V
- Potentiel en sortie de cellule de collision : 15 V
- (en anglais cell exit potential (CXP))
- Temps de cycle total: 1 sec
- Fenêtre de détection : 240 sec

Les aires obtenues pour chacune des transitions et pour chacun des microorganismes étudiés ont été mesurées. Lorsque les aires des transitions sont supérieures ou égales au seuil de positivité décrit dans le **TABLEAU 19,** la détection de la transition est considérée comme positive et est notée « 1 » dans le **TABLEAU 20.** Lorsqu'une transition a une aire inférieure au seuil de positivité décrit dans le **TABLEAU 19,** la transition est considérée comme non détectée et est notée « 0 » dans le **TABLEAU 20.**

Pour un peptide donné, lorsque au moins 3 transitions sont notées « 1 », le peptide est considéré comme détecté.

**TABLEAU 20 :**

| Transition numéro | Ech83 |
|---|---|
| 1 | 0 |
| 2 | 0 |
| 3 | 0 |
| 4 | 0 |
| 5 | 0 |
| 6 | 0 |
| 7 | 1 |
| 8 | 1 |
| 9 | 1 |
| 10 | 0 |
| 11 | 0 |
| 12 | 0 |
| 13 | 0 |
| 14 | 0 |
| 15 | 0 |
| 16 | 0 |
| 17 | 0 |
| 18 | 0 |
| 19 | 0 |
| 20 | 0 |
| 21 | 0 |
| 22 | 0 |
| 23 | 0 |
| 24 | 0 |
| 25 | 0 |
| 26 | 0 |
| 27 | 0 |
| 28 | 0 |
| 29 | 0 |
| 30 | 0 |
| 31 | 0 |
| 32 | 0 |
| 33 | 0 |
| 34 | 0 |
| 35 | 0 |
| 36 | 0 |
| 37 | 0 |
| 38 | 0 |
| 39 | 0 |
| 40 | 0 |
| 41 | 0 |
| 42 | 0 |
| 43 | 1 |
| 44 | 1 |
| 45 | 1 |
| 46 | 0 |
| 47 | 0 |
| 48 | 0 |
| 49 | 0 |
| 50 | 0 |
| 51 | 0 |
| 52 | 0 |
| 53 | 0 |
| 54 | 0 |
| 55 | 0 |
| 56 | 0 |
| 57 | 0 |
| 58 | 0 |
| 59 | 0 |
| 60 | 0 |
| 61 | 0 |
| 62 | 0 |
| 63 | 0 |
| 64 | 0 |
| 65 | 0 |
| 66 | 0 |
| 67 | 1 |
| 68 | 1 |
| 69 | 1 |
| 70 | 0 |
| 71 | 0 |
| 72 | 0 |
| 73 | 0 |
| 74 | 0 |
| 75 | 0 |

L'échantillon, Ech83, comporte au moins un peptide caractéristique des MOX. Les bactéries présentes dans l'échantillon Ech83 expriment donc une béta-lactamase qui leur confère une résistance aux pénicillines et aux céphalosporines excepté les céphalosporines de quatrième génération.

### Exemple 15 : Identification d'une résistance aux béta-lactamines de type ACC:

Les échantillons correspondant à une espèce pouvant comporter un mécanisme de résistance de type ACC peuvent être détectés en mettant en oeuvre la méthode suivante.

Chaque échantillon est traité selon l'exemple 5, puis analysé selon l'exemple 6 en détectant les peptides du **TABLEAU 21** en lieu et place des peptides du **TABLEAU 3.**

**TABLEAU 21 :**

| Transition numéro | Peptide | Temps de rétention (minutes) | (m/z) filtré en Q1 | (m/z) filtré en Q3 | Energie de collision (eV) |
|---|---|---|---|---|---|
| 1 | ALTDTHIGYFK | 15,83 | 422,56 | 457,24 | 25 |
| 2 | ALTDTHIGYFK | 15,83 | 422,56 | 514,27 | 25 |
| 3 | ALTDTHIGYFK | 15,83 | 422,56 | 627,35 | 25 |
| 4 | AWKPADPAGTHR | 10,49 | 436,23 | 470,25 | 26 |
| 5 | AWKPADPAGTHR | 10,49 | 436,23 | 638,34 | 26 |
| 6 | AWKPADPAGTHR | 10,49 | 436,23 | 669,34 | 26 |
| 7 | DEPVHGNMEILGNEAYGIK | 18,07 | 696 | 851,43 | 39 |
| 8 | DEPVHGNMEILGNEAYGIK | 18,07 | 696 | 1009,4 | 39 |
| 9 | DEPVHGNMEILGNEAYGIK | 18,07 | 696 | 1122,49 | 39 |
| 10 | DTVDGLIQPLMQK | 20,72 | 729,39 | 857,49 | 37 |
| 11 | DTVDGLIQPLMQK | 20,72 | 729,39 | 970,58 | 37 |
| 12 | DTVDGLIQPLMQK | 20,72 | 729,39 | 1027,6 | 37 |
| 13 | DTVDSLIQPLMQK | 21,62 | 744,39 | 857,49 | 38 |
| 14 | DTVDSLIQPLMQK | 21,62 | 744,39 | 1057,61 | 38 |
| 15 | DTVDSLIQPLMQK | 21,62 | 744,39 | 1172,63 | 38 |
| 16 | IQHALTATHTGYFK | 12,76 | 397,71 | 514,27 | 23 |
| 17 | IQHALTATHTGYFK | 12,76 | 397,71 | 615,31 | 23 |
| 18 | IQHALTATHTGYFK | 12,76 | 397,71 | 752,37 | 23 |
| 19 | LDGNSTLQK | 9,44 | 488,26 | 576,34 | 26 |
| 20 | LDGNSTLQK | 9,44 | 488,26 | 747,4 | 26 |
| 21 | LDGNSTLQK | 9,44 | 488,26 | 862,43 | 26 |
| 22 | LSLDK | 10,73 | 288,17 | 375,22 | 18 |
| 23 | LSLDK | 10,73 | 288,17 | 429,23 | 18 |
| 24 | LSLDK | 10,73 | 288,17 | 462,26 | 18 |
| 25 | LSLEQSVSHYVPELR | 20,3 | 586,31 | 776,43 | 33 |
| 26 | LSLEQSVSHYVPELR | 20,3 | 586,31 | 913,49 | 33 |
| 27 | LSLEQSVSHYVPELR | 20,3 | 586,31 | 1000,52 | 33 |
| 28 | NEPIHVNMEVLGNEAYGIR | 19,55 | 719,02 | 879,43 | 40 |
| 29 | NEPIHVNMEVLGNEAYGIR | 19,55 | 719,02 | 992,52 | 40 |
| 30 | NEPIHVNMEVLGNEAYGIR | 19,55 | 719,02 | 1163,55 | 40 |
| 31 | NNIPGMSVAVTIR | 18,3 | 457,92 | 559,36 | 27 |
| 32 | NNIPGMSVAVTIR | 18,3 | 686,37 | 933,52 | 35 |
| 33 | NNIPGMSVAVTIR | 18,3 | 686,37 | 1030,57 | 35 |
| 34 | NTDQLMAYLK | 18,78 | 598,8 | 625,34 | 31 |
| 35 | NTDQLMAYLK | 18,78 | 598,8 | 738,42 | 31 |
| 36 | NTDQLMAYLK | 18,78 | 598,8 | 981,51 | 31 |
| 37 | NTTQLMTYLK | 17,75 | 606,82 | 768,43 | 32 |
| 38 | NTTQLMTYLK | 17,75 | 606,82 | 896,49 | 32 |
| 39 | NTTQLMTYLK | 17,75 | 606,82 | 997,54 | 32 |
| 40 | NYIYNYGLASK | 15,9 | 653,33 | 752,39 | 34 |
| 41 | NYIYNYGLASK | 15,9 | 653,33 | 915,46 | 34 |
| 42 | NYIYNYGLASK | 15,9 | 653,33 | 1028,54 | 34 |
| 43 | SISHYVPELR | 15 | 400,88 | 514,3 | 24 |
| 44 | SISHYVPELR | 15 | 400,88 | 613,37 | 24 |
| 45 | SISHYVPELR | 15 | 400,88 | 776,43 | 24 |
| 46 | TFAAILASYAQASGK | 21,64 | 749,9 | 811,39 | 38 |
| 47 | TFAAILASYAQASGK | 21,64 | 749,9 | 882,43 | 38 |
| 48 | TFAAILASYAQASGK | 21,64 | 749,9 | 995,52 | 38 |
| 49 | TLLPK | 12,05 | 286,19 | 357,25 | 18 |
| 50 | TLLPK | 12,05 | 286,19 | 425,28 | 18 |
| 51 | TLLPK | 12,05 | 286,19 | 470,33 | 18 |
| 52 | TNASDLIR | 12,95 | 445,24 | 516,31 | 25 |
| 53 | TNASDLIR | 12,95 | 445,24 | 603,35 | 25 |
| 54 | TNASDLIR | 12,95 | 445,24 | 674,38 | 25 |
| 55 | VTVAYK | 10,34 | 340,7 | 381,21 | 20 |
| 56 | VTVAYK | 10,34 | 340,7 | 480,28 | 20 |
| 57 | VTVAYK | 10,34 | 340,7 | 581,33 | 20 |

Les aires obtenues pour chacune des transitions et pour chacun des microorganismes étudiés ont été mesurées. Lorsque les aires des trois transitions d'un même peptide sont supérieures ou égales à 2500 la détection du peptide est considérée comme positive et est noté « 1 ». Lorsque au moins une transition comporte une aire inférieure à 2500, le peptide correspondant est considéré comme non détecté et est noté « 0 ».

### Exemple 16 : Identification d'une résistance aux béta-lactamines de type ACT:

Les échantillons correspondant à une espèce pouvant comporter un mécanisme de résistance de type ACT peuvent être détectés en mettant en oeuvre la méthode suivante.

Chaque échantillon est traité selon l'exemple 5, puis analysé selon l'exemple 6 en détectant les peptides du **TABLEAU 22** en lieu et place des peptides du **TABLEAU 3.**

**TABLEAU 22 :**

| Transition numéro | Peptide | Temps de rétention (minutes) | (m/z) filtré en Q1 | (m/z) filtré en Q3 | Energie de collision (eV) |
|---|---|---|---|---|---|
| 1 | AEEAHYAWGYR | 14,21 | 676,81 | 815,38 | 34,8 |
| 2 | AEEAHYAWGYR | 14,21 | 676,81 | 952,44 | 34,8 |
| 3 | AEEAHYAWGYR | 14,21 | 676,81 | 1152,52 | 34,8 |
| 4 | AIHVSPGMLDAEAYGVK | 18,71 | 586,64 | 666,35 | 33,3 |
| 5 | AIHVSPGMLDAEAYGVK | 18,71 | 586,64 | 737,38 | 33,3 |
| 6 | AIHVSPGMLDAEAYGVK | 18,74 | 586,64 | 852,41 | 33,3 |
| 7 | AVHVSPGMLDAEAYGVK | 17,93 | 581,96 | 666,35 | 33,1 |
| 8 | AVHVSPGMLDAEAYGVK | 17,93 | 581,96 | 737,38 | 33,1 |
| 9 | AVHVSPGMLDAEAYGVK | 17,93 | 581,96 | 852,41 | 33,1 |
| 10 | DMANWVMVNMKPDSLQDSSLK | 22,23 | 803,71 | 896,45 | 44,2 |
| 11 | DMANWVMVNMKPDSLQDSSLK | 22,21 | 803,71 | 989,49 | 44,2 |
| 12 | DMANWVMVNMKPDSLQDSSLK | 22,23 | 803,71 | 1082,03 | 44,2 |
| 13 | DNASLLR | 12,76 | 394,72 | 401,29 | 22,4 |
| 14 | DNASLLR | 12,76 | 394,72 | 488,32 | 22,4 |
| 15 | DNASLLR | 12,76 | 394,72 | 559,36 | 22,4 |
| 16 | EGITLAQSR | 13,23 | 487,77 | 574,33 | 26,5 |
| 17 | EGITLAQSR | 13,25 | 487,77 | 675,38 | 26,5 |
| 18 | EGITLAQSR | 13,23 | 487,77 | 788,46 | 26,5 |
| 19 | EVNPPAPPVNASWVHK | 16,59 | 581,31 | 700,38 | 33,1 |
| 20 | EVNPPAPPVNASWVHK | 16,61 | 581,31 | 757,4 | 33,1 |
| 21 | EVNPPAPPVNASWVHK | 16,61 | 581,31 | 1134,61 | 33,1 |
| 22 | FYQNWQPQWKPGTTR | 17,43 | 968,98 | 531,29 | 47,6 |
| 23 | FYQNWQPQWKPGTTR | 17,43 | 968,98 | 1070,57 | 47,6 |
| 24 | FYQNWQPQWKPGTTR | 17,43 | 968,98 | 1198,63 | 47,6 |
| 25 | GEISLDDPVTR | 16,1 | 601,31 | 702,34 | 31,5 |
| 26 | GEISLDDPVTR | 16,1 | 601,31 | 815,43 | 31,5 |
| 27 | GEISLDDPVTR | 16,12 | 601,31 | 902,46 | 31,5 |
| 28 | GEISLGDPVTK | 15,7 | 558,3 | 559,31 | 29,6 |
| 29 | GEISLGDPVTK | 15,68 | 558,3 | 616,33 | 29,6 |
| 30 | GEISLGDPVTK | 15,68 | 558,3 | 816,45 | 29,6 |
| 31 | GLTLAQSR | 12,67 | 423,25 | 461,25 | 23,6 |
| 32 | GLTLAQSR | 12,68 | 423,25 | 574,33 | 23,6 |
| 33 | GLTLAQSR | 12,65 | 423,25 | 675,38 | 23,6 |
| 34 | LDHTWINVPK | 16,79 | 611,83 | 497,78 | 31,9 |
| 35 | LDHTWINVPK | 16,79 | 611,83 | 756,44 | 31,9 |
| 36 | LDHTWINVPK | 16,79 | 611,83 | 857,49 | 31,9 |
| 37 | MLDLATYTAGGLPLQVPDEVK | 23,87 | 744,39 | 512,79 | 41,2 |
| 38 | MLDLATYTAGGLPLQVPDEVK | 23,89 | 744,39 | 587,3 | 41,2 |
| 39 | MLDLATYTAGGLPLQVPDEVK | 23,89 | 1116,09 | 587,3 | 54,1 |
| 40 | QGIALAQSR | 12,72 | 472,27 | 574,33 | 25,8 |
| 41 | QGIALAQSR | 12,7 | 472,27 | 645,37 | 25,8 |
| 42 | QGIALAQSR | 12,72 | 472,27 | 758,45 | 25,8 |
| 43 | QGISLAQSR | 12,83 | 480,27 | 661,36 | 26,1 |
| 44 | QGISLAQSR | 12,83 | 480,27 | 774,45 | 26,1 |
| 45 | QGISLAQSR | 12,83 | 480,27 | 831,47 | 26,1 |
| 46 | QIGIVMLANK | 18,95 | 543,82 | 576,32 | 28,9 |
| 47 | QIGIVMLANK | 18,95 | 543,82 | 675,39 | 28,9 |
| 48 | QIGIVMLANK | 18,95 | 543,82 | 845,49 | 28,9 |
| 49 | QIGIVMLANTSYPNPAR | 21,86 | 615,66 | 554,31 | 34,8 |
| 50 | QIGIVMLANTSYPNPAR | 21,86 | 615,66 | 717,37 | 34,8 |
| 51 | QIGIVMLANTSYPNPAR | 21,86 | 922,99 | 554,31 | 45,6 |
| 52 | QLAEVVANTVTPLMK | 21,94 | 807,45 | 488,29 | 40,5 |
| 53 | QLAEVVANTVTPLMK | 21,94 | 807,45 | 974,53 | 40,5 |
| 54 | QLAEVVANTVTPLMK | 21,94 | 807,45 | 1073,6 | 40,5 |
| 55 | QLAEVVER | 13,05 | 472,26 | 502,3 | 25,8 |
| 56 | QLAEVVER | 13,03 | 472,26 | 631, 34 | 25,8 |
| 57 | QLAEVVER | 13,03 | 472,26 | 702,38 | 25,8 |
| 58 | QLGIVMLANK | 19,27 | 543,82 | 576,32 | 28,9 |
| 59 | QLGIVMLANK | 19,27 | 543,82 | 675,39 | 28,9 |
| 60 | QLGIVMLANK | 19,27 | 543,82 | 845,49 | 28,9 |
| 61 | SYPNPAR | 9,83 | 402,7 | 343,21 | 22,7 |
| 62 | SYPNPAR | 9,83 | 402,7 | 457,25 | 22,7 |
| 63 | SYPNPAR | 9,83 | 402,7 | 554,31 | 22,7 |
| 64 | TFTGVLGGDAIAR | 18,42 | 639,35 | 659,35 | 33,1 |
| 65 | TFTGVLGGDAIAR | 18,39 | 639,35 | 772,43 | 33,1 |
| 66 | TFTGVLGGDAIAR | 18,42 | 639,35 | 1029,57 | 33,1 |
| 67 | TGSTGGFGSYVAFIPEK | 21,14 | 573,29 | 373,21 | 32,7 |
| 68 | TGSTGGFGSYVAFIPEK | 21,14 | 573,29 | 633,36 | 32,7 |
| 69 | TGSTGGFGSYVAFIPEK | 21,14 | 573,29 | 704,4 | 32,7 |
| 70 | TVTPLMK | 13,65 | 395,23 | 488,29 | 22,4 |
| 71 | TVTPLMK | 13,65 | 395,23 | 589,34 | 22,4 |
| 72 | TVTPLMK | 13,62 | 395,23 | 688,41 | 22,4 |
| 73 | TWEGSDNK | 4,64 | 474,74 | 520,24 | 25,9 |
| 74 | TWEGSDNK | 4,64 | 474,74 | 649,28 | 25,9 |
| 75 | TWEGSDNK | 4,64 | 474,74 | 748,35 | 25,9 |
| 76 | VALAPLPAR | 15,96 | 454,29 | 553,35 | 25 |
| 77 | VALAPLPAR | 15,99 | 454,29 | 624,38 | 25 |
| 78 | VALAPLPAR | 15,96 | 454,29 | 737,47 | 25 |
| 79 | VALAPLPVAEVNPPAPPVK | 21,12 | 627,04 | 705,43 | 35,4 |
| 80 | VALAPLPVAEVNPPAPPVK | 21,14 | 627,04 | 762,94 | 35,4 |
| 81 | VALAPLPVAEVNPPAPPVK | 21,14 | 627,04 | 819,47 | 35,4 |
| 82 | VEAAYR | 8,39 | 354,69 | 409,22 | 20,6 |
| 83 | VEAAYR | 8,39 | 354,69 | 480,26 | 20,6 |
| 84 | VEAAYR | 8,39 | 354,69 | 609,3 | 20,6 |
| 85 | VFKPLK | 11,66 | 366,24 | 357,25 | 21,1 |
| 86 | VFKPLK | 11,66 | 366,24 | 485,35 | 21,1 |
| 87 | VFKPLK | 11,66 | 366,24 | 632,41 | 21,1 |
| 88 | VGAMYQGLGWEMLNWPVDAK | 26,23 | 755,7 | 529,3 | 41,8 |
| 89 | VGAMYQGLGWEMLNWPVDAK | 26,23 | 755,7 | 829,42 | 41,8 |
| 90 | VGAMYQGLGWEMLNWPVDAK | 26,26 | 755,7 | 1073,55 | 41,8 |
| 91 | VLKPLK | 10,38 | 349,25 | 357,25 | 20,4 |
| 92 | VLKPLK | 10,36 | 349,25 | 485,35 | 20,4 |
| 93 | VLKPLK | 10,36 | 349,25 | 598,43 | 20,4 |
| 94 | VSPGMLDAQAYGMK | 17,62 | 734,35 | 641,3 | 37,3 |
| 95 | VSPGMLDAQAYGMK | 17,62 | 734,35 | 883,4 | 37,3 |
| 96 | VSPGMLDAQAYGMK | 17,62 | 734,35 | 996,48 | 37,3 |
| 97 | VSPGMLDAQAYGVK | 17,36 | 718,37 | 625,32 | 36,6 |
| 98 | VSPGMLDAQAYGVK | 17,36 | 718,37 | 851,43 | 36,6 |
| 99 | VSPGMLDAQAYGVK | 17,36 | 718,37 | 964,51 | 36,6 |
| 100 | YWPQLTGK | 17,33 | 496,76 | 322,19 | 26,9 |
| 101 | YWPQLTGK | 17,33 | 496,76 | 643,38 | 26,9 |
| 102 | YWPQLTGK | 17,31 | 496,76 | 829,46 | 26,9 |

Les aires obtenues pour chacune des transitions et pour chacun des microorganismes étudiés ont été mesurées. Lorsque les aires des trois transitions d'un même peptide sont supérieures ou égales à 2500 la détection du peptide est considérée comme positive et est noté « 1 ». Lorsque au moins une transition comporte une aire inférieure à 2500, le peptide correspondant est considéré comme non détecté et est noté « 0 ».

### Exemple 17 : Identification d'une résistance aux béta-lactamines de type CMY:

Les échantillons correspondant à une espèce pouvant comporter un mécanisme de résistance de type CMY peuvent être détectés en mettant en oeuvre la méthode suivante.

Chaque échantillon est traité selon l'exemple 5, puis analysé selon l'exemple 6 en détectant les peptides du **TABLEAU 23** en lieu et place des peptides du **TABLEAU 3.**

**TABLEAU 23 :**

| Transition numéro | Peptide | Temps de rétention (minutes) | (m/z) filtré en Q1 | (m/z) filtré en Q3 | Energie de collision (eV) |
|---|---|---|---|---|---|
| 1 | AALLR | 10,85 | 272,18 | 288,2 | 17 |
| 2 | AALLR | 10,85 | 272,18 | 401,29 | 17 |
| 3 | AALLR | 10,85 | 272,18 | 472,32 | 17 |
| 4 | ADSIINGSDNK | 10,62 | 567,28 | 747,36 | 30 |
| 5 | ADSIINGSDNK | 10,62 | 567,28 | 860,45 | 30 |
| 6 | ADSIINGSDNK | 10,62 | 567,28 | 947,48 | 30 |
| 7 | AELLR | 11,13 | 301,19 | 401,29 | 18 |
| 8 | AELLR | 11,13 | 301,19 | 427,26 | 18 |
| 9 | AELLR | 11,13 | 301,19 | 530,33 | 18 |
| 10 | ALQQAISLTHK | 13,79 | 605,35 | 698,42 | 32 |
| 11 | ALQQAISLTHK | 13,79 | 605,35 | 769,46 | 32 |
| 12 | ALQQAISLTHK | 13,79 | 605,35 | 897,52 | 32 |
| 13 | AVHVSPGQLDAEAYGVK | 15,59 | 580,97 | 737,38 | 33 |
| 14 | AVHVSPGQLDAEAYGVK | 15,59 | 580,97 | 776,4 | 33 |
| 15 | AVHVSPGQLDAEAYGVK | 15,59 | 580,97 | 852,41 | 33 |
| 16 | DYAC[CAM]GYR | 9,86 | 452,68 | 555,23 | 25 |
| 17 | DYAC[CAM]GYR | 9,86 | 452,68 | 626,27 | 25 |
| 18 | DYAC[CAM]GYR | 9,86 | 452,68 | 789,33 | 25 |
| 19 | DYALGYR | 13,44 | 429,21 | 508,29 | 24 |
| 20 | DYALGYR | 13,44 | 429,21 | 579,32 | 24 |
| 21 | DYALGYR | 13,44 | 429,21 | 742,39 | 24 |
| 22 | EGKPVHASPGQLDAEAYGVK | 13,47 | 685,02 | 737,38 | 38 |
| 23 | EGKPVHASPGQLDAEAYGVK | 13,47 | 685,02 | 852,41 | 38 |
| 24 | EGKPVHASPGQLDAEAYGVK | 13,47 | 685,02 | 965,49 | 38 |
| 25 | EGKPVHGSPGQLDAEAYGVK | 13,08 | 510,51 | 537,3 | 29 |
| 26 | EGKPVHGSPGQLDAEAYGVK | 13,08 | 510,51 | 666,35 | 29 |
| 27 | EGKPVHGSPGQLDAEAYGVK | 13,08 | 510,51 | 737,38 | 29 |
| 28 | EGKPVHVSPEQLDAEAYGVK | 15,07 | 539,03 | 737,38 | 30 |
| 29 | EGKPVHVSPEQLDAEAYGVK | 15,07 | 539,03 | 852,41 | 30 |
| 30 | EGKPVHVSPEQLDAEAYGVK | 15,07 | 718,37 | 852,41 | 40 |
| 31 | EGKPVHVSPGQFDAEAYGVK | 15,01 | 529,52 | 537,3 | 29 |
| 32 | EGKPVHVSPGQFDAEAYGVK | 15,01 | 529,52 | 632,33 | 29 |
| 33 | EGKPVHVSPGQFDAEAYGVK | 15,01 | 705,69 | 999,48 | 39 |
| 34 | EGKPVHVSPGQLDAEAYC[CAM]VK | 14,63 | 546,77 | 569,28 | 30 |
| 35 | EGKPVHVSPGQLDAEAYC[CAM]VK | 14,63 | 546,77 | 640,31 | 30 |
| 36 | EGKPVHVSPGQLDAEAYC[CAM]VK | 14,63 | 546,77 | 769,35 | 30 |
| 37 | EGKPVHVSPGQLDAEAYGVK | 14,65 | 521,02 | 537,3 | 29 |
| 38 | EGKPVHVSPGQLDAEAYGVK | 14,65 | 521,02 | 737,38 | 29 |
| 39 | EGKPVHVSPGQLDAEAYGVK | 14,65 | 521,02 | 852,41 | 29 |
| 40 | EGKPVHVSPGQLDAGAYGVK | 14,19 | 503,02 | 594,32 | 28 |
| 41 | EGKPVHVSPGQLDAGAYGVK | 14,19 | 503,02 | 665,36 | 28 |
| 42 | EGKPVHVSPGQLDAGAYGVK | 14,19 | 503,02 | 780,39 | 28 |
| 43 | EGKPVHVSPGQLNAEAYGVK | 14,25 | 520,78 | 537,3 | 29 |
| 44 | EGKPVHVSPGQLNAEAYGVK | 14,25 | 520,78 | 737,38 | 29 |
| 45 | EGKPVHVSPGQLNAEAYGVK | 14,25 | 520,78 | 834,45 | 29 |
| 46 | EGKPVHVTPGQLDAEAYGVK | 14,72 | 524,53 | 848,46 | 29 |
| 47 | EGKPVHVTPGQLDAEAYGVK | 14,72 | 699,03 | 852,41 | 39 |
| 48 | EGKPVHVTPGQLDAEAYGVK | 14,72 | 699,03 | 1247,63 | 39 |
| 49 | EGKPVYVSPGQLDAEAYGVK | 16,73 | 703,03 | 852,41 | 39 |
| 50 | EGKPVYVSPGQLDAEAYGVK | 16,73 | 703,03 | 860,45 | 39 |
| 51 | EGKPVYVSPGQLDAEAYGVK | 16,73 | 703,03 | 1247,63 | 39 |
| 52 | ESGASVSEQTLFDIGSVSK | 20,37 | 970,98 | 976,42 | 48 |
| 53 | ESGASVSEQTLFDIGSVSK | 20,37 | 970,98 | 1066,58 | 48 |
| 54 | ESGASVSEQTLFDIGSVSK | 20,37 | 970,98 | 1194,64 | 48 |
| 55 | FSDPVTK | 11,61 | 397,21 | 559,31 | 22 |
| 56 | FSDPVTK | 11,61 | 397,21 | 646,34 | 22 |
| 57 | FSDPVTK | 11,61 | 397,21 | 647,3 | 22 |
| 58 | FYQNWQPQWAPGAK | 18,61 | 860,92 | 867,38 | 43 |
| 59 | FYQNWQPQWAPGAK | 18,61 | 860,92 | 982,51 | 43 |
| 60 | FYQNWQPQWAPGAK | 18,61 | 860,92 | 1168,59 | 43 |
| 61 | FYQNWQPQWTPGAK | 18,53 | 875,92 | 884,46 | 44 |
| 62 | FYQNWQPQWTPGAK | 18,53 | 875,92 | 1012,52 | 44 |
| 63 | FYQNWQPQWTPGAK | 18,53 | 875,92 | 1198,6 | 44 |
| 64 | HAPWLK | 11,85 | 376,22 | 543,33 | 22 |
| 65 | HAPWLK | 11,85 | 376,22 | 605,32 | 22 |
| 66 | HAPWLK | 11,85 | 376,22 | 614,37 | 22 |
| 67 | IPDDVR | 9,9 | 357,69 | 504,24 | 21 |
| 68 | IPDDVR | 9,9 | 357,69 | 540,27 | 21 |
| 69 | IPDDVR | 9,9 | 357,69 | 601,29 | 21 |
| 70 | LAHTWIK | 12,56 | 434,76 | 547,32 | 24 |
| 71 | LAHTWIK | 12,56 | 434,76 | 684,38 | 24 |
| 72 | LAHTWIK | 12,56 | 434,76 | 755,42 | 24 |
| 73 | LAHTWITVPENEQK | 15,87 | 555,96 | 609,31 | 32 |
| 74 | LAHTWITVPENEQK | 15,87 | 555,96 | 744,35 | 32 |
| 75 | LAHTWITVPENEQK | 15,87 | 555,96 | 823,45 | 32 |
| 76 | LAHTWITVPQSEQK | 15,75 | 546,63 | 1029,56 | 31 |
| 77 | LAHTWITVPQSEQK | 15,75 | 819,44 | 1029,56 | 41 |
| 78 | LAHTWITVPQSEQK | 15,75 | 819,44 | 1215,64 | 41 |
| 79 | LDAEAYGVK | 12,86 | 483,25 | 537,3 | 26 |
| 80 | LDAEAYGVK | 12,86 | 483,25 | 737,38 | 26 |
| 81 | LDAEAYGVK | 12,86 | 483,25 | 852,41 | 26 |
| 82 | LLHLATYTAGGLPLK | 19,26 | 523,31 | 584,38 | 30 |
| 83 | LLHLATYTAGGLPLK | 19,26 | 523,31 | 655,41 | 30 |
| 84 | LLHLATYTAGGLPLK | 19,26 | 523,31 | 756,46 | 30 |
| 85 | LLHLATYTAGGLPLQFPDDVR | 22,76 | 575,06 | 601,29 | 32 |
| 86 | LLHLATYTAGGLPLQFPDDVR | 22,76 | 766,41 | 1086,56 | 42 |
| 87 | LLHLATYTAGGLPLQFPDDVR | 22,76 | 766,41 | 1098,59 | 42 |
| 88 | NYAWGYR | 14,18 | 465,22 | 581,28 | 25 |
| 89 | NYAWGYR | 14,18 | 465,22 | 652,32 | 25 |
| 90 | NYAWGYR | 14,18 | 465,22 | 815,38 | 25 |
| 91 | NYPIPAR | 12,32 | 415,73 | 456,29 | 23 |
| 92 | NYPIPAR | 12,32 | 415,73 | 553,35 | 23 |
| 93 | NYPIPAR | 12,32 | 415,73 | 716,41 | 23 |
| 94 | NYPNEAR | 6,92 | 432,2 | 489,21 | 24 |
| 95 | NYPNEAR | 6,92 | 432,2 | 586,29 | 24 |
| 96 | NYPNEAR | 6,92 | 432,2 | 749,36 | 24 |
| 97 | SLC[CAM]C[CAM]ALLLTAPLSTFAAAK | 26,05 | 670,02 | 905,51 | 38 |
| 98 | SLC[CAM]C[CAM]ALLLTAPLSTFAAAK | 26,05 | 1004,53 | 1077,59 | 49 |
| 99 | SLC[CAM]C[CAM]ALLLTAPLSTFAAAK | 26,05 | 1004,53 | 1190,68 | 49 |
| 100 | SNVTDMAR | 10,56 | 447,21 | 492,22 | 25 |
| 101 | SNVTDMAR | 10,56 | 447,21 | 593,27 | 25 |
| 102 | SNVTDMAR | 10,56 | 447,21 | 692,34 | 25 |
| 103 | TALLHFYQNWQPQWAPGAK | 21,7 | 752,72 | 854,45 | 42 |
| 104 | TALLHFYQNWQPQWAPGAK | 21,7 | 752,72 | 974,51 | 42 |
| 105 | TALLHFYQNWQPQWAPGAK | 21,7 | 752,72 | 1088,55 | 42 |
| 106 | TDSIINGSDSK | 10,86 | 568,78 | 720,35 | 30 |
| 107 | TDSIINGSDSK | 10,86 | 568,78 | 833,44 | 30 |
| 108 | TDSIINGSDSK | 10,86 | 568,78 | 920,47 | 30 |
| 109 | TFIGVLGGDAIAR | 20,29 | 645,36 | 659,35 | 33 |
| 110 | TFIGVLGGDAIAR | 20,29 | 645,36 | 772,43 | 33 |
| 111 | TFIGVLGGDAIAR | 20,29 | 645,36 | 928,52 | 33 |
| 112 | TFNGVLGGDC[CAM]IAR | 16,85 | 690,34 | 748,34 | 35 |
| 113 | TFNGVLGGDC[CAM]IAR | 16,85 | 690,34 | 861,42 | 35 |
| 114 | TFNGVLGGDC[CAM]IAR | 16,85 | 690,34 | 1131,56 | 35 |
| 115 | TFNGVLGGEAIAR | 17,2 | 435,57 | 673,36 | 26 |
| 116 | TFNGVLGGEAIAR | 17,2 | 652,85 | 673,36 | 34 |
| 117 | TFNGVLGGEAIAR | 17,2 | 652,85 | 786,45 | 34 |
| 118 | TGSTVGFGSYVAFVPEK | 20,65 | 873,44 | 1039,55 | 43 |
| 119 | TGSTVGFGSYVAFVPEK | 20,65 | 873,44 | 1096,57 | 43 |
| 120 | TGSTVGFGSYVAFVPEK | 20,65 | 873,44 | 1243,64 | 43 |
| 121 | TGYTGGFGSYVAFVPEK | 20,58 | 890,43 | 1039,55 | 44 |
| 122 | TGYTGGFGSYVAFVPEK | 20,58 | 890,43 | 1096,57 | 44 |
| 123 | TGYTGGFGSYVAFVPEK | 20,58 | 890,43 | 1243,64 | 44 |
| 124 | TLQQGIELAQSR | 15,04 | 448,58 | 461,25 | 26 |
| 125 | TLQQGIELAQSR | 15,04 | 672,37 | 703,37 | 35 |
| 126 | TLQQGIELAQSR | 15,04 | 672,37 | 873,48 | 35 |
| 127 | TSSADLLAFVK | 20,76 | 576,32 | 805,48 | 30 |
| 128 | TSSADLLAFVK | 20,76 | 576,32 | 876,52 | 30 |
| 129 | TSSADLLAFVK | 20,76 | 576,32 | 963,55 | 30 |
| 130 | TSSADLLR | 12,72 | 431,73 | 587,35 | 24 |
| 131 | TSSADLLR | 12,72 | 431,73 | 674,38 | 24 |
| 132 | TSSADLLR | 12,72 | 431,73 | 761,42 | 24 |
| 133 | TYYFTWGK | 18,46 | 533,26 | 801,39 | 28 |
| 134 | TYYFTWGK | 18,46 | 533,26 | 919,4 | 28 |
| 135 | TYYFTWGK | 18,46 | 533,26 | 964,46 | 28 |
| 136 | VAALPAVEVNPPAPAVK | 18,04 | 821,98 | 964,55 | 41 |
| 137 | VAALPAVEVNPPAPAVK | 18,04 | 821,98 | 1021,57 | 41 |
| 138 | VAALPAVEVNPPAPAVK | 18,04 | 821,98 | 1120,64 | 41 |
| 139 | VAFAALPAVEVNPPAPAVK | 21,03 | 621,02 | 679,41 | 35 |
| 140 | VAFAALPAVEVNPPAPAVK | 21,03 | 621,02 | 793,46 | 35 |
| 141 | VAFAALPAVEVNPPAPAVK | 21,03 | 931,03 | 1021,57 | 46 |
| 142 | VALAAIPAVEVNPPAPAVK | 20,17 | 457,52 | 679,41 | 26 |
| 143 | VALAAIPAVEVNPPAPAVK | 20,17 | 609,7 | 679,41 | 34 |
| 144 | VALAAIPAVEVNPPAPAVK | 20,17 | 609,7 | 793,46 | 34 |
| 145 | VALAALHTVEVNPPAPAVK | 18 | 475,03 | 582,36 | 27 |
| 146 | VALAALHTVEVNPPAPAVK | 18 | 475,03 | 679,41 | 27 |
| 147 | VALAALHTVEVNPPAPAVK | 18 | 633,03 | 679,41 | 36 |
| 148 | VALAALPAVEINPPAPAVK | 21,5 | 614,37 | 679,41 | 35 |
| 149 | VALAALPAVEINPPAPAVK | 21,5 | 614,37 | 793,46 | 35 |
| 150 | VALAALPAVEINPPAPAVK | 21,5 | 614,37 | 935,56 | 35 |
| 151 | VALAALPTVEVNPPAPAVK | 20,41 | 619,7 | 679,41 | 35 |
| 152 | VALAALPTVEVNPPAPAVK | 20,41 | 619,7 | 793,46 | 35 |
| 153 | VALAALPTVEVNPPAPAVK | 20,41 | 619,7 | 1021,57 | 35 |
| 154 | VAPAVEVNPPAPAVK | 15,16 | 729,92 | 793,46 | 37 |
| 155 | VAPAVEVNPPAPAVK | 15,16 | 729,92 | 892,53 | 37 |
| 156 | VAPAVEVNPPAPAVK | 15,16 | 729,92 | 1021,57 | 37 |
| 157 | VEAYWR | 13,37 | 412,21 | 524,26 | 23 |
| 158 | VEAYWR | 13,37 | 412,21 | 595,3 | 23 |
| 159 | VEAYWR | 13,37 | 412,21 | 724,34 | 23 |
| 160 | VILEANPTAAPR | 14,25 | 417,91 | 612,35 | 25 |
| 161 | VILEANPTAAPR | 14,25 | 626,36 | 797,43 | 33 |
| 162 | VILEANPTAAPR | 14,25 | 626,36 | 1039,55 | 33 |
| 163 | VSLEANPTAAPR | 13,15 | 613,33 | 726,39 | 32 |
| 164 | VSLEANPTAAPR | 13,15 | 613,33 | 797,43 | 32 |
| 165 | VSLEANPTAAPR | 13,15 | 613,33 | 926,47 | 32 |
| 166 | WIQVNMDASR | 16,68 | 610,3 | 693,3 | 32 |
| 167 | WIQVNMDASR | 16,68 | 610,3 | 792,37 | 32 |
| 168 | WIQVNMDASR | 16,68 | 610,3 | 920,43 | 32 |
| 169 | WVQANMDASR | 12,82 | 589,27 | 764,34 | 31 |
| 170 | WVQANMDASR | 12,82 | 589,27 | 892,39 | 31 |
| 171 | WVQANMDASR | 12,82 | 589,27 | 991,46 | 31 |
| 172 | WVQVNMDASR | 15,27 | 603,29 | 792,37 | 32 |
| 173 | WVQVNMDASR | 15,27 | 603,29 | 920,43 | 32 |
| 174 | WVQVNMDASR | 15,27 | 603,29 | 1019,49 | 32 |
| 175 | YWSELTGK | 14,9 | 492,25 | 547,31 | 27 |
| 176 | YWSELTGK | 14,9 | 492,25 | 634,34 | 27 |
| 177 | YWSELTGK | 14,9 | 492,25 | 820,42 | 27 |

Les aires obtenues pour chacune des transitions et pour chacun des microorganismes étudiés ont été mesurées. Lorsque les aires des trois transitions d'un même peptide sont supérieures ou égales à 2500 la détection du peptide est considérée comme positive et est noté « 1 ». Lorsque au moins une transition comporte une aire inférieure à 2500, le peptide correspondant est considéré comme non détecté et est noté « 0 ».

### Exemple 18 : Identification d'une résistance aux béta-lactamines de type CTX-M:

Les échantillons correspondant à une espèce pouvant comporter un mécanisme de résistance de type CTX-M peuvent être détectés en mettant en oeuvre la méthode suivante.

Chaque échantillon est traité selon l'exemple 5, puis analysé selon l'exemple 6 en détectant les peptides du **TABLEAU 24** en lieu et place des peptides du **TABLEAU 3.**

**TABLEAU 24 :**

| Transition numéro | Peptide | Temps de rétention (minutes) | (m/z) filtré en Q1 | (m/z) filtré en Q3 | Energie de collision (eV) |
|---|---|---|---|---|---|
| 1 | AGADVASLR | 11,83 | 430,24 | 446,27 | 24 |
| 2 | AGADVASLR | 11,83 | 430,24 | 485,24 | 24 |
| 3 | AGADVASLR | 11,83 | 430,24 | 660,37 | 24 |
| 4 | AGLPASWVVGDK | 18,32 | 600,32 | 790,41 | 31 |
| 5 | AGLPASWVVGDK | 18,32 | 600,32 | 861,45 | 31 |
| 6 | AGLPASWVVGDK | 18,32 | 600,32 | 958,5 | 31 |
| 7 | AGLPTSWTAGDK | 15,73 | 602,3 | 764,36 | 32 |
| 8 | AGLPTSWTAGDK | 15,73 | 602,3 | 865,41 | 32 |
| 9 | AGLPTSWTAGDK | 15,73 | 602,3 | 962,46 | 32 |
| 10 | AGLPTSWTVGDR | 17,31 | 630,32 | 820,39 | 33 |
| 11 | AGLPTSWTVGDR | 17,31 | 630,32 | 921,44 | 33 |
| 12 | AGLPTSWTVGDR | 17,31 | 630,32 | 1018,5 | 33 |
| 13 | AGLPTSWWGDK | 18,62 | 615,33 | 790,41 | 32 |
| 14 | AGLPTSWWGDK | 18,62 | 615,33 | 891,46 | 32 |
| 15 | AGLPTSWWGDK | 18,62 | 615,33 | 988,51 | 32 |
| 16 | AIGDDTFR | 12,79 | 447,72 | 653,29 | 25 |
| 17 | AIGDDTFR | 12,79 | 447,72 | 710,31 | 25 |
| 18 | AIGDDTFR | 12,79 | 447,72 | 823,39 | 25 |
| 19 | AMAVAAVLK | 16,36 | 437,26 | 501, 34 | 24 |
| 20 | AMAVAAVLK | 16,36 | 437,26 | 600,41 | 24 |
| 21 | AMAVAAVLK | 16,36 | 437,26 | 671,45 | 24 |
| 22 | APLILVTYFTQPEQK | 23,95 | 583,33 | 730,37 | 33 |
| 23 | APLILVTYFTQPEQK | 23,95 | 874,49 | 1040,5 | 43 |
| 24 | APLILVTYFTQPEQK | 23,95 | 874,49 | 1141,55 | 43 |
| 25 | APLVLVTYFTQPEPK | 23,37 | 568,32 | 699,37 | 32 |
| 26 | APLVLVTYFTQPEPK | 23,37 | 568,32 | 846,44 | 32 |
| 27 | APLVLVTYFTQPEPK | 23,37 | 851,97 | 1110,55 | 42 |
| 28 | APLVLVTYFTQPQQNAENR | 22,93 | 730,38 | 956,45 | 41 |
| 29 | APLVLVTYFTQPQQNAENR | 22,93 | 1095,07 | 1185,56 | 53 |
| 30 | APLVLVTYFTQPQQNAENR | 22,93 | 1095,07 | 1233,69 | 53 |
| 31 | APLVLVTYFTQPQQNAER | 23,02 | 692,37 | 745,36 | 39 |
| 32 | APLVLVTYFTQPQQNAER | 23,02 | 692,37 | 842,41 | 39 |
| 33 | APLVLVTYFTQPQQNAER | 23,02 | 692,37 | 857,51 | 39 |
| 34 | APLVLVTYFTQSEPK | 23,53 | 564,98 | 689,35 | 32 |
| 35 | APLVLVTYFTQSEPK | 23,53 | 846,96 | 1100,53 | 42 |
| 36 | APLVLVTYFTQSEPK | 23,53 | 846,96 | 1199,59 | 42 |
| 37 | AQLVAWLK | 19,52 | 464,78 | 517,31 | 25 |
| 38 | AQLVAWLK | 19,52 | 464,78 | 616,38 | 25 |
| 39 | AQLVAWLK | 19,52 | 464,78 | 729,47 | 25 |
| 40 | AQLVMWLK | 20,53 | 494,79 | 577,32 | 27 |
| 41 | AQLVMWLK | 20,53 | 494,79 | 676,39 | 27 |
| 42 | AQLVMWLK | 20,53 | 494,79 | 789,47 | 27 |
| 43 | ASDLVNYNPIAEK | 16,72 | 717,37 | 834,44 | 37 |
| 44 | ASDLVNYNPIAEK | 16,72 | 717,37 | 948,48 | 37 |
| 45 | ASDLVNYNPIAEK | 16,72 | 717,37 | 1047,55 | 37 |
| 46 | DFLAAAAK | 14,5 | 403,72 | 431,26 | 23 |
| 47 | DFLAAAAK | 14,5 | 403,72 | 518,26 | 23 |
| 48 | DFLAAAAK | 14,5 | 403,72 | 544,35 | 23 |
| 49 | DILASAAK | 12,62 | 394,73 | 447,26 | 22 |
| 50 | DILASAAK | 12,62 | 394,73 | 560,34 | 22 |
| 51 | DILASAAK | 12,62 | 394,73 | 571,31 | 22 |
| 52 | DNTQVLYR | 12,19 | 504,76 | 550,33 | 27 |
| 53 | DNTQVLYR | 12,19 | 504,76 | 678,39 | 27 |
| 54 | DNTQVLYR | 12,19 | 504,76 | 779,44 | 27 |
| 55 | DTTTPLAMAQALR | 19,92 | 694,86 | 760,41 | 36 |
| 56 | DTTTPLAMAQALR | 19,92 | 694,86 | 873,5 | 36 |
| 57 | DTTTPLAMAQALR | 19,92 | 694,86 | 970,55 | 36 |
| 58 | DTTTPLAMAQSLR | 19,22 | 702,86 | 776,41 | 36 |
| 59 | DTTTPLAMAQSLR | 19,22 | 702,86 | 889,49 | 36 |
| 60 | DTTTPLAMAQSLR | 19,22 | 702,86 | 986,55 | 36 |
| 61 | DTTTPLAMAQTLR | 18,99 | 709,87 | 719,39 | 36 |
| 62 | DTTTPLAMAQTLR | 18,99 | 709,87 | 790,42 | 36 |
| 63 | DTTTPLAMAQTLR | 18,99 | 709,87 | 1000,56 | 36 |
| 64 | DVLAAAAR | 12,11 | 393,73 | 459,27 | 22 |
| 65 | DVLAAAAR | 12,11 | 393,73 | 572,35 | 22 |
| 66 | DVLAAAAR | 12,11 | 393,73 | 671,42 | 22 |
| 67 | EIGDETFR | 12,99 | 483,73 | 552,28 | 26 |
| 68 | EIGDETFR | 12,99 | 483,73 | 667,3 | 26 |
| 69 | EIGDETFR | 12,99 | 483,73 | 724,33 | 26 |
| 70 | EQLVTWLK | 19,13 | 508,79 | 547,32 | 27 |
| 71 | EQLVTWLK | 19,13 | 508,79 | 646,39 | 27 |
| 72 | EQLVTWLK | 19,13 | 508,79 | 759,48 | 27 |
| 73 | GNTTGSASIQAGLPK | 13,81 | 701,37 | 813,48 | 36 |
| 74 | GNTTGSASIQAGLPK | 13,81 | 701,37 | 1028,57 | 36 |
| 75 | GNTTGSASIQAGLPK | 13,81 | 701,37 | 1129,62 | 36 |
| 76 | HDVLASAAK | 9,32 | 456,25 | 659,41 | 25 |
| 77 | HDVLASAAK | 9,32 | 456,25 | 765,39 | 25 |
| 78 | HDVLASAAK | 9,32 | 456,25 | 774,44 | 25 |
| 79 | HDVLASAAR | 9,88 | 470,25 | 588,35 | 26 |
| 80 | HDVLASAAR | 9,88 | 470,25 | 687,41 | 26 |
| 81 | HDVLASAAR | 9,88 | 470,25 | 802,44 | 26 |
| 82 | HLTLGSALGETQR | 15,07 | 691,87 | 918,46 | 35 |
| 83 | HLTLGSALGETQR | 15,07 | 691,87 | 1031,55 | 35 |
| 84 | HLTLGSALGETQR | 15,07 | 691,87 | 1132,6 | 35 |
| 85 | LAELEQQSGGR | 11,17 | 594,3 | 761,35 | 31 |
| 86 | LAELEQQSGGR | 11,17 | 594,3 | 874,44 | 31 |
| 87 | LAELEQQSGGR | 11,17 | 594,3 | 1003,48 | 31 |
| 88 | LAGLER | 11,22 | 329,7 | 474,27 | 20 |
| 89 | LAGLER | 11,22 | 329,7 | 484,28 | 20 |
| 90 | LAGLER | 11,22 | 329,7 | 545,3 | 20 |
| 91 | LDGTEPTLNTAIPGDPR | 16,6 | 589,64 | 1053,57 | 33 |
| 92 | LDGTEPTLNTAIPGDPR | 16,6 | 883,95 | 1053,57 | 44 |
| 93 | LDGTEPTLNTAIPGDPR | 16,6 | 883,95 | 1154,62 | 44 |
| 94 | LGVALIDTADNAQTLYR | 20,27 | 917,49 | 980,48 | 45 |
| 95 | LGVALIDTADNAQTLYR | 20,27 | 917,49 | 1051,52 | 45 |
| 96 | LGVALIDTADNAQTLYR | 20,27 | 917,49 | 1152,56 | 45 |
| 97 | LGVALIDTADNTHVLYR | 19,46 | 624,34 | 801,42 | 35 |
| 98 | LGVALIDTADNTHVLYR | 19,46 | 624,34 | 902,48 | 35 |
| 99 | LGVALIDTADNTHVLYR | 19,46 | 624,34 | 1189,6 | 35 |
| 100 | LGVALIDTK | 16,93 | 465,29 | 589,36 | 25 |
| 101 | LGVALIDTK | 16,93 | 465,29 | 660,39 | 25 |
| 102 | LGVALIDTK | 16,93 | 465,29 | 816,48 | 25 |
| 103 | LGVALINTADNSR | 16,92 | 672,37 | 777,35 | 35 |
| 104 | LGVALINTADNSR | 16,92 | 672,37 | 890,43 | 35 |
| 105 | LGVALINTADNSR | 16,92 | 672,37 | 1003,52 | 35 |
| 106 | LGVALINTADNTQTLYR | 19,63 | 621,67 | 1081,53 | 35 |
| 107 | LGVALINTADNTQTLYR | 19,63 | 932 | 1081,53 | 46 |
| 108 | LGVALINTADNTQTLYR | 19,63 | 932 | 1182,57 | 46 |
| 109 | LGVPLIDTADNTQVLYR | 21,54 | 944,51 | 1008,51 | 47 |
| 110 | LGVPLIDTADNTQVLYR | 21,54 | 944,51 | 1079,55 | 47 |
| 111 | LGVPLIDTADNTQVLYR | 21,54 | 944,51 | 1180,6 | 47 |
| 112 | LIAHLGGPGK | 12,27 | 321,53 | 358,21 | 20 |
| 113 | LIAHLGGPGK | 12,27 | 321,53 | 415,23 | 20 |
| 114 | LIAHLGGPGK | 12,27 | 321,53 | 528,31 | 20 |
| 115 | LIAQLGGQGGVTAFAR | 18,73 | 779,94 | 963,5 | 39 |
| 116 | LIAQLGGQGGVTAFAR | 18,73 | 779,94 | 1020,52 | 39 |
| 117 | LIAQLGGQGGVTAFAR | 18,73 | 779,94 | 1133,61 | 39 |
| 118 | LISHVGGPASVTAFAR | 16,27 | 528,29 | 565,31 | 30 |
| 119 | LISHVGGPASVTAFAR | 16,27 | 528,29 | 664,38 | 30 |
| 120 | LISHVGGPASVTAFAR | 16,27 | 791,94 | 1033,54 | 40 |
| 121 | LLLNQR | 12,81 | 378,74 | 417,22 | 22 |
| 122 | LLLNQR | 12,81 | 378,74 | 530,3 | 22 |
| 123 | LLLNQR | 12,81 | 378,74 | 643,39 | 22 |
| 124 | NLTLGNALGDTQR | 16,67 | 686,86 | 931,46 | 35 |
| 125 | NLTLGNALGDTQR | 16,67 | 686,86 | 1044,54 | 35 |
| 126 | NLTLGNALGDTQR | 16,67 | 686,86 | 1145,59 | 35 |
| 127 | NLTLGSALGETQR | 17,15 | 453,91 | 590,29 | 27 |
| 128 | NLTLGSALGETQR | 17,15 | 680,36 | 918,46 | 35 |
| 129 | NLTLGSALGETQR | 17,15 | 680,36 | 1031,55 | 35 |
| 130 | QLGDDTFR | 13,1 | 476,23 | 653,29 | 26 |
| 131 | QLGDDTFR | 13,1 | 476,23 | 710,31 | 26 |
| 132 | QLGDDTFR | 13,1 | 476,23 | 823,39 | 26 |
| 133 | SDLVNYSPIAEK | 16,24 | 668,34 | 807,42 | 34 |
| 134 | SDLVNYSPIAEK | 16,24 | 668,34 | 921,47 | 34 |
| 135 | SDLVNYSPIAEK | 16,24 | 668,34 | 1020,54 | 34 |
| 136 | SESEPSLLNQR | 13,8 | 630,31 | 730,42 | 33 |
| 137 | SESEPSLLNQR | 13,8 | 630,31 | 827,47 | 33 |
| 138 | SESEPSLLNQR | 13,8 | 630,31 | 1043,55 | 33 |
| 139 | SLGDESFR | 12,8 | 455,72 | 653,29 | 25 |
| 140 | SLGDESFR | 12,8 | 455,72 | 710,31 | 25 |
| 141 | SLGDESFR | 12,8 | 455,72 | 823,39 | 25 |
| 142 | SSDLINYNPIAEK | 17,68 | 732,37 | 834,44 | 37 |
| 143 | SSDLINYNPIAEK | 17,68 | 732,37 | 948,48 | 37 |
| 144 | SSDLINYNPIAEK | 17,68 | 732,37 | 1061,56 | 37 |
| 145 | SSDLINYNPITEK | 17,72 | 747,38 | 864,45 | 38 |
| 146 | SSDLINYNPITEK | 17,72 | 747,38 | 978,49 | 38 |
| 147 | SSDLINYNPITEK | 17,72 | 747,38 | 1091,57 | 38 |
| 148 | SWGVGDK | 11,6 | 374,68 | 475,25 | 21 |
| 149 | SWGVGDK | 11,6 | 374,68 | 602,26 | 21 |
| 150 | SWGVGDK | 11,6 | 374,68 | 661,33 | 21 |
| 151 | TELTLNTAIPGDPR | 17,63 | 749,4 | 826,44 | 38 |
| 152 | TELTLNTAIPGDPR | 17,63 | 749,4 | 940,48 | 38 |
| 153 | TELTLNTAIPGDPR | 17,63 | 749,4 | 957,53 | 38 |
| 154 | TEPTLNSAIPGDPR | 15,33 | 734,38 | 812,43 | 37 |
| 155 | TEPTLNSAIPGDPR | 15,33 | 734,38 | 926,47 | 37 |
| 156 | TEPTLNSAIPGDPR | 15,33 | 734,38 | 1237,65 | 37 |
| 157 | TEQTLNTAIPGDPR | 14,47 | 756,89 | 940,48 | 38 |
| 158 | TEQTLNTAIPGDPR | 14,47 | 756,89 | 972,5 | 38 |
| 159 | TEQTLNTAIPGDPR | 14,47 | 756,89 | 1154,62 | 38 |
| 160 | TESTLNTAIPGDPR | 14,52 | 736,37 | 940,48 | 37 |
| 161 | TESTLNTAIPGDPR | 14,52 | 736,37 | 1053,57 | 37 |
| 162 | TESTLNTAIPGDPR | 14,52 | 736,37 | 1154,62 | 37 |
| 163 | TETTLNTAIPGDPR | 14,88 | 743,38 | 826,44 | 38 |
| 164 | TETTLNTAIPGDPR | 14,88 | 743,38 | 940,48 | 38 |
| 165 | TETTLNTAIPGDPR | 14,88 | 743,38 | 945,49 | 38 |
| 166 | TGSC[CAM]DYGTTNDIAVIWPK | 20,38 | 999,47 | 1055,59 | 49 |
| 167 | TGSC[CAM]DYGTTNDIAVIWPK | 20,38 | 999,47 | 1156,64 | 49 |
| 168 | TGSC[CAM]DYGTTNDIAVIWPK | 20,38 | 999,47 | 1172,42 | 49 |
| 169 | TGSC[CAM]GYGTTNDIAVIWPK | 20,21 | 970,46 | 1055,59 | 48 |
| 170 | TGSC[CAM]GYGTTNDIAVIWPK | 20,21 | 970,46 | 1114,41 | 48 |
| 171 | TGSC[CAM]GYGTTNDIAVIWPK | 20,21 | 970,46 | 1156,64 | 48 |
| 172 | TGSGDYGTTNDIAVIWPEGR | 20,27 | 1055 | 1069,41 | 51 |
| 173 | TGSGDYGTTNDIAVIWPEGR | 20,27 | 1055 | 1155,62 | 51 |
| 174 | TGSGDYGTTNDIAVIWPEGR | 20,27 | 1055 | 1182,49 | 51 |
| 175 | TGSGGYGTTNDIAVIWPEGR | 20,16 | 684,33 | 757,4 | 38 |
| 176 | TGSGGYGTTNDIAVIWPEGR | 20,16 | 684,33 | 927,5 | 38 |
| 177 | TGSGGYGTTNDIAVIWPEGR | 20,16 | 684,33 | 1011,4 | 38 |
| 178 | TGSGGYGTTNDIAVIWPQGR | 19,81 | 684 | 756,42 | 38 |
| 179 | TGSGGYGTTNDIAVIWPQGR | 19,81 | 684 | 926,52 | 38 |
| 180 | TGSGGYGTTNDIAVIWPQGR | 19,81 | 684 | 1011,4 | 38 |
| 181 | TIGDDTFR | 12,86 | 462,72 | 538,26 | 25 |
| 182 | TIGDDTFR | 12,86 | 462,72 | 653,29 | 25 |
| 183 | TIGDDTFR | 12,86 | 462,72 | 710,31 | 25 |
| 184 | TQLVTWLK | 19,07 | 494,79 | 646,39 | 27 |
| 185 | TQLVTWLK | 19,07 | 494,79 | 759,48 | 27 |
| 186 | TQLVTWLK | 19,07 | 494,79 | 887,53 | 27 |
| 187 | VEIKPSDLINYNPIAEK | 20,21 | 648,35 | 769,41 | 36 |
| 188 | VEIKPSDLINYNPIAEK | 20,21 | 648,35 | 882,49 | 36 |
| 189 | VEIKPSDLINYNPIAEK | 20,21 | 648,35 | 948,48 | 36 |
| 190 | VEIKPSDLVNYNPIAEK | 19,33 | 643,68 | 769,41 | 36 |
| 191 | VEIKPSDLVNYNPIAEK | 19,33 | 643,68 | 882,49 | 36 |
| 192 | VEIKPSDLVNYNPIAEK | 19,33 | 643,68 | 948,48 | 36 |
| 193 | VIGDDTFR | 13,3 | 461,73 | 538,26 | 25 |
| 194 | VIGDDTFR | 13,3 | 461,73 | 710,31 | 25 |
| 195 | VIGDDTFR | 13,3 | 461,73 | 823,39 | 25 |
| 196 | VMAAAALLK | 17,06 | 444,27 | 586,39 | 25 |
| 197 | VMAAAALLK | 17,06 | 444,27 | 657,43 | 25 |
| 198 | VMAAAALLK | 17,06 | 444,27 | 788,47 | 25 |
| 199 | VMAAAAVLEQSETQK | 16,62 | 788,41 | 962,48 | 40 |
| 200 | VMAAAAVLEQSETQK | 16,62 | 788,41 | 1061,55 | 40 |
| 201 | VMAAAAVLEQSETQK | 16,62 | 788,41 | 1132,58 | 40 |
| 202 | WAKPSGAVGDVAQR | 12,73 | 481,26 | 628,34 | 28 |
| 203 | WAKPSGAVGDVAQR | 12,73 | 481,26 | 645,33 | 28 |
| 204 | WAKPSGAVGDVAQR | 12,73 | 481,26 | 698,36 | 28 |

Les aires obtenues pour chacune des transitions et pour chacun des microorganismes étudiés ont été mesurées. Lorsque les aires des trois transitions d'un même peptide sont supérieures ou égales à 2500 la détection du peptide est considérée comme positive et est noté « 1 ». Lorsque au moins une transition comporte une aire inférieure à 2500, le peptide correspondant est considéré comme non détecté et est noté « 0 ».

### Exemple 19 : Identification d'une résistance aux béta-lactamines de type DHA:

Les échantillons correspondant à une espèce pouvant comporter un mécanisme de résistance de type DHA peuvent être détectés en mettant en oeuvre la méthode suivante.

Chaque échantillon est traité selon l'exemple 5, puis analysé selon l'exemple 6 en détectant les peptides du **TABLEAU 25** en lieu et place des peptides du **TABLEAU 3.**

**TABLEAU 25 :**

| Transition numéro | Peptide | Temps de rétention (minutes) | (m/z) filtré en Q1 | (m/z) filtré en Q3 | Energie de collision (eV) |
|---|---|---|---|---|---|
| 1 | ADLLHFYQQWQPSR | 20,9 | 596,97 | 673,34 | 34 |
| 2 | ADLLHFYQQWQPSR | 20,9 | 596,97 | 988,49 | 34 |
| 3 | ADLLHFYQQWQPSR | 20,9 | 596,97 | 1116,55 | 34 |
| 4 | AELLHFYQQWQPSR | 19,23 | 601,64 | 673,34 | 34 |
| 5 | AELLHFYQQWQPSR | 19,23 | 601,64 | 801,4 | 34 |
| 6 | AELLHFYQQWQPSR | 19,23 | 601,64 | 1130,56 | 34 |
| 7 | EMMLNDPAEK | 13,78 | 589,26 | 786,4 | 31 |
| 8 | EMMLNDPAEK | 13,78 | 589,26 | 917,44 | 31 |
| 9 | EMMLNDPAEK | 13,78 | 589,26 | 1048,48 | 31 |
| 10 | GKPYYFNYGFADVQAK | 18,12 | 623,31 | 631, 34 | 35 |
| 11 | GKPYYFNYGFADVQAK | 18,12 | 623,31 | 778,41 | 35 |
| 12 | GKPYYFNYGFADVQAK | 18,12 | 623,31 | 835,43 | 35 |
| 13 | TAAINQGLGWEMYDWPQQK | 21,57 | 746,02 | 964,45 | 41 |
| 14 | TAAINQGLGWEMYDWPQQK | 21,57 | 746,02 | 1095,49 | 41 |
| 15 | TAAINQGLGWEMYDWPQQK | 21,57 | 1118,53 | 1224,54 | 54 |
| 16 | WAEMNIEPSR | 15,85 | 616,79 | 715,37 | 32 |
| 17 | WAEMNIEPSR | 15,85 | 616,79 | 975,46 | 32 |
| 18 | WAEMNIEPSR | 15,85 | 616,79 | 1046,49 | 32 |

Les aires obtenues pour chacune des transitions et pour chacun des microorganismes étudiés ont été mesurées. Lorsque les aires des trois transitions d'un même peptide sont supérieures ou égales à 2500 la détection du peptide est considérée comme positive et est noté « 1 ». Lorsque au moins une transition comporte une aire inférieure à 2500, le peptide correspondant est considéré comme non détecté et est noté « 0 ».

### Exemple 20 : Identification d'une résistance aux béta-lactamines de type FOX:

Les échantillons correspondant à une espèce pouvant comporter un mécanisme de résistance de type FOX peuvent être détectés en mettant en oeuvre la méthode suivante.

Chaque échantillon est traité selon l'exemple 5, puis analysé selon l'exemple 6 en détectant les peptides du **TABLEAU 26** en lieu et place des peptides du **TABLEAU 3.**

**TABLEAU 26 :**

| Transition numéro | Peptide | Temps de rétention (minutes) | (m/z) filtré en Q1 | (m/z) filtré en Q3 | Energie de collision (eV) |
|---|---|---|---|---|---|
| 1 | ATPGVLAAEAYGIK | 17,72 | 680,88 | 751,4 | 35 |
| 2 | ATPGVLAAEAYGIK | 17,72 | 680,88 | 822,44 | 35 |
| 3 | ATPGVLAAEAYGIK | 17,72 | 680,88 | 935,52 | 35 |
| 4 | FAEANMGYQGDAAVK | 14,08 | 786,36 | 908,45 | 40 |
| 5 | FAEANMGYQGDAAVK | 14,08 | 786,36 | 1039,49 | 40 |
| 6 | FAEANMGYQGDAAVK | 14,08 | 786,36 | 1224,57 | 40 |
| 7 | FAEANMGYQGDALVK | 16,35 | 538,59 | 602,35 | 31 |
| 8 | FAEANMGYQGDALVK | 16,35 | 538,59 | 730,41 | 31 |
| 9 | FAEANMGYQGDALVK | 16,35 | 807,38 | 950,49 | 41 |
| 10 | GEAPLTAAVDGIIQPMLK | 25,85 | 912,5 | 1014,57 | 45 |
| 11 | GEAPLTAAVDGIIQPMLK | 25,85 | 912,5 | 1113,63 | 45 |
| 12 | GEAPLTAAVDGIIQPMLK | 25,85 | 912,5 | 1184,67 | 45 |
| 13 | HWSPVYPAGTHR | 12,55 | 469,9 | 541,28 | 27 |
| 14 | HWSPVYPAGTHR | 12,55 | 469,9 | 607,3 | 27 |
| 15 | HWSPVYPAGTHR | 12,55 | 469,9 | 638,34 | 27 |
| 16 | IPGIAVAVLK | 19,45 | 490,83 | 600,41 | 27 |
| 17 | IPGIAVAVLK | 19,45 | 490,83 | 770,51 | 27 |
| 18 | IPGIAVAVLK | 19,45 | 490,83 | 867,57 | 27 |
| 19 | LMSQTLLPK | 16,18 | 515,8 | 699,44 | 28 |
| 20 | LMSQTLLPK | 16,18 | 515,8 | 786,47 | 28 |
| 21 | LMSQTLLPK | 16,18 | 515,8 | 917,51 | 28 |
| 22 | MQTYYR | 10,72 | 431,2 | 602,29 | 24 |
| 23 | MQTYYR | 10,72 | 431,2 | 687,28 | 24 |
| 24 | MQTYYR | 10,72 | 431,2 | 730,35 | 24 |
| 25 | VSHHAPWLK | 11,49 | 537,8 | 614,37 | 29 |
| 26 | VSHHAPWLK | 11,49 | 537,8 | 751,42 | 29 |
| 27 | VSHHAPWLK | 11,49 | 537,8 | 888,48 | 29 |
| 28 | VTPGMLAAEAYGIK | 19 | 710,88 | 822,44 | 36 |
| 29 | VTPGMLAAEAYGIK | 19 | 710,88 | 1123,58 | 36 |
| 30 | VTPGMLAAEAYGIK | 19 | 710,88 | 1220,63 | 36 |

Les aires obtenues pour chacune des transitions et pour chacun des microorganismes étudiés ont été mesurées. Lorsque les aires des trois transitions d'un même peptide sont supérieures ou égales à 2500 la détection du peptide est considérée comme positive et est noté « 1 ». Lorsque au moins une transition comporte une aire inférieure à 2500, le peptide correspondant est considéré comme non détecté et est noté « 0 ».

### Exemple 21 : Identification d'une résistance aux béta-lactamines de type MIR:

Les échantillons correspondant à une espèce pouvant comporter un mécanisme de résistance de type MIR peuvent être détectés en mettant en oeuvre la méthode suivante.

Chaque échantillon est traité selon l'exemple 5, puis analysé selon l'exemple 6 en détectant les peptides du **TABLEAU 27** en lieu et place des peptides du **TABLEAU 3.**

**TABLEAU 27 :**

| Transition numéro | Peptide | Temps de rétention (minutes) | (m/z) filtré en Q1 | (m/z) filtré en Q3 | Energie de collision (eV) |
|---|---|---|---|---|---|
| 1 | AEEAHFAWGYR | 16,38 | 446,21 | 568,77 | 20,9 |
| 2 | AEEAHFAWGYR | 16,36 | 446,21 | 633,29 | 20,9 |
| 3 | AEEAHFAWGYR | 16,36 | 446,21 | 652,32 | 20,9 |
| 4 | DMASWLIANMKPDSLHAPSLK | 24,18 | 775,73 | 811,44 | 31,1 |
| 5 | DMASWLIANMKPDSLHAPSLK | 24,18 | 775,73 | 867,98 | 31,1 |
| 6 | DMASWLIANMKPDSLHAPSLK | 24,18 | 775,73 | 1004,54 | 31,1 |
| 7 | DMASWLIANMKPDSLQAPSLK | 25,04 | 772,73 | 528,29 | 31 |
| 8 | DMASWLIANMKPDSLQAPSLK | 25,04 | 772,73 | 806,94 | 31 |
| 9 | DMASWLIANMKPDSLQAPSLK | 25,04 | 772,73 | 863,48 | 31 |
| 10 | DMASWVIANMKPDSLQAPSLK | 24 | 768,06 | 806,94 | 30,9 |
| 11 | DMASWVIANMKPDSLQAPSLK | 24 | 768,06 | 856,47 | 30,9 |
| 12 | DMASWVIANMKPDSLQAPSLK | 24 | 768,06 | 949,51 | 30,9 |
| 13 | GEIALGDPVAK | 15,79 | 535,3 | 586,32 | 26,2 |
| 14 | GEIALGDPVAK | 15,77 | 535,3 | 699,4 | 26,2 |
| 15 | GEIALGDPVAK | 15,77 | 535,3 | 770,44 | 26,2 |
| 16 | TVVGGSDNK | 3,35 | 438,73 | 520,24 | 20,7 |
| 17 | TVVGGSDNK | 3,35 | 438,73 | 577,26 | 20,7 |
| 18 | TVVGGSDNK | 3,35 | 438,73 | 676,33 | 20,7 |

Les aires obtenues pour chacune des transitions et pour chacun des microorganismes étudiés ont été mesurées. Lorsque les aires des trois transitions d'un même peptide sont supérieures ou égales à 2500 la détection du peptide est considérée comme positive et est noté « 1 ». Lorsque au moins une transition comporte une aire inférieure à 2500, le peptide correspondant est considéré comme non détecté et est noté « 0 ».

### Exemple 22 : Identification d'une résistance aux béta-lactamines de type SHV:

Les échantillons correspondant à une espèce pouvant comporter un mécanisme de résistance de type SHV peuvent être détectés en mettant en oeuvre la méthode suivante.

Chaque échantillon est traité selon l'exemple 5, puis analysé selon l'exemple 6 en détectant les peptides du **TABLEAU 28** en lieu et place des peptides du **TABLEAU 3.**

**TABLEAU 28 :**

| Transition numéro | Peptide | Temps de rétention (minutes) | (m/z) filtré en Q1 | (m/z) filtré en Q3 | Energie de collision (eV) |
|---|---|---|---|---|---|
| 1 | EIGDNVTR | 10,07 | 452,23 | 489,28 | 25 |
| 2 | EIGDNVTR | 10,07 | 452,23 | 529,23 | 25 |
| 3 | EIGDNVTR | 10,07 | 452,23 | 661,33 | 25 |
| 4 | GIVALLGPHNK | 16,52 | 373,56 | 398,21 | 23 |
| 5 | GIVALLGPHNK | 16,52 | 373,56 | 552,29 | 23 |
| 6 | GIVALLGPHNK | 16,52 | 373,56 | 665,37 | 23 |
| 7 | HLADGMTVGELR | 14,93 | 433,56 | 474,27 | 26 |
| 8 | HLADGMTVGELR | 14,93 | 433,56 | 494,24 | 26 |
| 9 | HLADGMTVGELR | 14,93 | 433,56 | 573,34 | 26 |
| 10 | IHYSQQDLVDYSPVSEK | 16,22 | 669,99 | 872,39 | 37 |
| 11 | IHYSQQDLVDYSPVSEK | 16,22 | 669,99 | 924,43 | 37 |
| 12 | IHYSQQDLVDYSPVSEK | 16,22 | 669,99 | 985,47 | 37 |

Les aires obtenues pour chacune des transitions et pour chacun des microorganismes étudiés ont été mesurées. Lorsque les aires des trois transitions d'un même peptide sont supérieures ou égales à 2500 la détection du peptide est considérée comme positive et est noté « 1 ». Lorsque au moins une transition comporte une aire inférieure à 2500, le peptide correspondant est considéré comme non détecté et est noté « 0 ».

### Exemple 23: Identification d'une résistance aux béta-lactamines de type CARB:

Les échantillons correspondant à une espèce pouvant comporter un mécanisme de résistance de type CARB peuvent être détectés en mettant en oeuvre la méthode suivante.

Chaque échantillon est traité selon l'exemple 5, puis analysé selon l'exemple 6 en détectant les peptides du **TABLEAU 29** en lieu et place des peptides du **TABLEAU 3.**

**TABLEAU 29 :**

| Transition numéro | Peptide | Temps de rétention (minutes) | (m/z) filtré en Q1 | (m/z) filtré en Q3 | Energie de collision (eV) |
|---|---|---|---|---|---|
| 1 | ADLVTYSPVIEK | 17,48 | 667,86 | 672,39 | 34,4 |
| 2 | ADLVTYSPVIEK | 17,48 | 667,86 | 835,46 | 34,4 |
| 3 | ADLVTYSPVIEK | 17,48 | 667,86 | 936,5 | 34,4 |
| 4 | ADLVTYSPVLEK | 18,01 | 667,86 | 672,39 | 34,4 |
| 5 | ADLVTYSPVLEK | 18,01 | 667,86 | 835,46 | 34,4 |
| 6 | ADLVTYSPVLEK | 18,01 | 667,86 | 936,5 | 34,4 |
| 7 | AIASTLNK | 10,86 | 409,24 | 475,29 | 23 |
| 8 | AIASTLNK | 10,86 | 409,24 | 562,32 | 23 |
| 9 | AIASTLNK | 10,86 | 409,24 | 633,36 | 23 |
| 10 | AIASTLNQLLFGSTLSEASQK | 25,39 | 727,06 | 362,2 | 40,4 |
| 11 | AIASTLNQLLFGSTLSEASQK | 25,39 | 727,06 | 649,32 | 40,4 |
| 12 | AIASTLNQLLFGSTLSEASQK | 25,39 | 727,06 | 998,02 | 40,4 |
| 13 | AIEVSLSAR | 15,46 | 473,27 | 533,3 | 25,8 |
| 14 | AIEVSLSAR | 15,46 | 473,27 | 632,37 | 25,8 |
| 15 | AIEVSLSAR | 15,46 | 473,27 | 761,42 | 25,8 |
| 16 | DTTTPIAMVTTLEK | 21,41 | 507,6 | 591,34 | 29,4 |
| 17 | DTTTPIAMVTTLEK | 21,41 | 507,6 | 690,4 | 29,4 |
| 18 | DTTTPIAMVTTLEK | 21,41 | 507,6 | 892,48 | 29,4 |
| 19 | DTTTPK | 1,54 | 331,67 | 345,21 | 19,6 |
| 20 | DTTTPK | 1,54 | 331,67 | 446,26 | 19,6 |
| 21 | DTTTPK | 1,54 | 331,67 | 547,31 | 19,6 |
| 22 | FLFGSALSEMNK | 21,32 | 672,34 | 879,42 | 34,6 |
| 23 | FLFGSALSEMNK | 21,32 | 672,34 | 936,45 | 34,6 |
| 24 | FLFGSALSEMNK | 21,32 | 672,34 | 1083,51 | 34,6 |
| 25 | FPLSSTFK | 17,51 | 463,75 | 390,22 | 25,4 |
| 26 | FPLSSTFK | 17,51 | 463,75 | 682,38 | 25,4 |
| 27 | FPLSSTFK | 17,51 | 463,75 | 779,43 | 25,4 |
| 28 | FPLTSTFK | 17,85 | 470,76 | 397,23 | 25,7 |
| 29 | FPLTSTFK | 17,85 | 470,76 | 696,39 | 25,7 |
| 30 | FPLTSTFK | 17,85 | 470,76 | 793,45 | 25,7 |
| 31 | FQQVEQDAK | 10,02 | 546,77 | 590,28 | 29,1 |
| 32 | FQQVEQDAK | 10,02 | 546,77 | 689,35 | 29,1 |
| 33 | FQQVEQDAK | 10,02 | 546,77 | 817,41 | 29,1 |
| 34 | FQQVEQDVK | 11,76 | 560,79 | 618,31 | 29,7 |
| 35 | FQQVEQDVK | 11,76 | 560,79 | 717,38 | 29,7 |
| 36 | FQQVEQDVK | 11,76 | 560,79 | 845,44 | 29,7 |
| 37 | FQSVEQEIK | 14,17 | 554,29 | 745,41 | 29,4 |
| 38 | FQSVEQEIK | 14,17 | 554,29 | 832,44 | 29,4 |
| 39 | FQSVEQEIK | 14,17 | 554,29 | 960,5 | 29,4 |
| 40 | FSESNLVTYSPVTEK | 17,84 | 567,62 | 462,74 | 32,4 |
| 41 | FSESNLVTYSPVTEK | 17,84 | 567,62 | 777,39 | 32,4 |
| 42 | FSESNLVTYSPVTEK | 17,85 | 567,62 | 924,47 | 32,4 |
| 43 | GIESSLSAR | 12,88 | 460,25 | 533,3 | 25,3 |
| 44 | GIESSLSAR | 12,88 | 460,25 | 620,34 | 25,3 |
| 45 | GIESSLSAR | 12,88 | 460,25 | 749,38 | 25,3 |
| 46 | GNEVGDALFR | 17,15 | 539,27 | 678,36 | 28,7 |
| 47 | GNEVGDALFR | 17,15 | 539,27 | 777,43 | 28,7 |
| 48 | GNEVGDALFR | 17,15 | 539,27 | 906,47 | 28,7 |
| 49 | GVPSDWIVADR | 18,32 | 607,81 | 529,77 | 31,7 |
| 50 | GVPSDWIVADR | 18,32 | 607,81 | 759,42 | 31,7 |
| 51 | GVPSDWIVADR | 18,32 | 607,81 | 1058,53 | 31,7 |
| 52 | GVTDFLR | 17,69 | 404,22 | 326,18 | 22,8 |
| 53 | GVTDFLR | 17,69 | 404,22 | 550,3 | 22,8 |
| 54 | GVTDFLR | 17,69 | 404,22 | 651,35 | 22,8 |
| 55 | IEPDLNEGK | 12,13 | 507,76 | 386,7 | 27,3 |
| 56 | IEPDLNEGK | 12,13 | 507,76 | 772,38 | 27,3 |
| 57 | IEPDLNEGK | 12,13 | 507,76 | 901,43 | 27,3 |
| 58 | IEPELNEGK | 12,2 | 514,77 | 393,7 | 27,6 |
| 59 | IEPELNEGK | 12,2 | 514,77 | 786,4 | 27,6 |
| 60 | IEPELNEGK | 12,2 | 514,77 | 915,44 | 27,6 |
| 61 | IGEQIAK | 10,58 | 379,72 | 459,29 | 21,7 |
| 62 | IGEQIAK | 10,58 | 379,72 | 588,34 | 21,7 |
| 63 | IGEQIAK | 10,58 | 379,72 | 645,36 | 21,7 |
| 64 | IGLAVHDLETGK | 16,05 | 626,85 | 799,39 | 32,6 |
| 65 | IGLAVHDLETGK | 16,05 | 626,85 | 898,46 | 32,6 |
| 66 | IGLAVHDLETGK | 16,05 | 626,85 | 969,5 | 32,6 |
| 67 | KPIVAALYITETDASFEER | 21,09 | 718,38 | 667,31 | 39,9 |
| 68 | KPIVAALYITETDASFEER | 21,09 | 718,38 | 787,37 | 39,9 |
| 69 | KPIVAALYITETDASFEER | 21,09 | 718,38 | 853,37 | 39,9 |
| 70 | LESWMVNNQVTGNLLR | 22,01 | 625,32 | 564,81 | 32,5 |
| 71 | LESWMVNNQVTGNLLR | 22,01 | 625,32 | 673,4 | 32,5 |
| 72 | LESWMVNNQVTGNLLR | 22,01 | 625,32 | 772,47 | 32,5 |
| 73 | LEYWMVNNQVTGNLLR | 22,96 | 650,67 | 564,81 | 33,6 |
| 74 | LEYWMVNNQVTGNLLR | 22,96 | 650,67 | 673,4 | 33,6 |
| 75 | LEYWMVNNQVTGNLLR | 22,96 | 650,67 | 772,47 | 33,6 |
| 76 | LLFGSALSEMNQK | 21,03 | 719,37 | 389,21 | 36,7 |
| 77 | LLFGSALSEMNQK | 21,03 | 719,37 | 736,33 | 36,7 |
| 78 | LLFGSALSEMNQK | 21,03 | 719,37 | 1211,57 | 36,7 |
| 79 | LLIDETLSIK | 20,7 | 572,85 | 805,43 | 30,2 |
| 80 | LLIDETLSIK | 20,7 | 572,85 | 918,51 | 30,2 |
| 81 | LLIDETLSIK | 20,7 | 572,85 | 1031,6 | 30,2 |
| 82 | LLYDAEHGK | 11,75 | 523,27 | 656,3 | 28 |
| 83 | LLYDAEHGK | 11,75 | 523,27 | 819,36 | 28 |
| 84 | LLYDAEHGK | 11,75 | 523,27 | 932,45 | 28 |
| 85 | LLYDAEQGEINPK | 15,72 | 745,38 | 358,21 | 37,8 |
| 86 | LLYDAEQGEINPK | 15,72 | 745,38 | 632,3 | 37,8 |
| 87 | LLYDAEQGEINPK | 15,72 | 745,38 | 785,42 | 37,8 |
| 88 | LLYDAEQGK | 13,05 | 518,77 | 647,3 | 27,8 |
| 89 | LLYDAEQGK | 13,05 | 518,77 | 810,36 | 27,8 |
| 90 | LLYDAEQGK | 13,05 | 518,77 | 923,45 | 27,8 |
| 91 | MCDNQNYGVTYMK | 14,67 | 541,89 | 641,33 | 31,1 |
| 92 | MCDNQNYGVTYMK | 14,67 | 541,89 | 698,35 | 31,1 |
| 93 | MCDNQNYGVTYMK | 14,67 | 541,89 | 861,42 | 31,1 |
| 94 | NAVIAK | 7,68 | 308,2 | 331,23 | 18,6 |
| 95 | NAVIAK | 7,68 | 308,2 | 430,3 | 18,6 |
| 96 | NAVIAK | 7,68 | 308,2 | 501, 34 | 18,6 |
| 97 | NDAIVK | 8,38 | 330,19 | 359,27 | 19,5 |
| 98 | NDAIVK | 8,38 | 330,19 | 430,3 | 19,5 |
| 99 | NDAIVK | 8,38 | 330,19 | 545,33 | 19,5 |
| 100 | QQLESWLK | 17,65 | 516,28 | 533,31 | 27,7 |
| 101 | QQLESWLK | 17,65 | 516,28 | 662,35 | 27,7 |
| 102 | QQLESWLK | 17,65 | 516,28 | 775,44 | 27,7 |
| 103 | QVEQDVK | 6,63 | 423,22 | 361,21 | 23,6 |
| 104 | QVEQDVK | 6,63 | 423,22 | 489,27 | 23,6 |
| 105 | QVEQDVK | 6,63 | 423,22 | 618,31 | 23,6 |
| 106 | SGAGGFGAR | 9,32 | 390,19 | 507,27 | 22,2 |
| 107 | SGAGGFGAR | 9,32 | 390,19 | 564,29 | 22,2 |
| 108 | SGAGGFGAR | 9,32 | 390,19 | 635,33 | 22,2 |
| 109 | SIGDDTTR | 7,76 | 432,71 | 492,24 | 24 |
| 110 | SIGDDTTR | 7,76 | 432,71 | 607,27 | 24 |
| 111 | SIGDDTTR | 7,76 | 432,71 | 664,29 | 24 |
| 112 | SITAIVWSEEK | 18,92 | 631,84 | 405,2 | 32,8 |
| 113 | SITAIVWSEEK | 18,92 | 631,84 | 777,38 | 32,8 |
| 114 | SITAIVWSEEK | 18,92 | 631,84 | 1062,55 | 32,8 |
| 115 | SITDFLR | 19,34 | 426,24 | 435,27 | 23,8 |
| 116 | SITDFLR | 19,34 | 426,24 | 550,3 | 23,8 |
| 117 | SITDFLR | 19,34 | 426,24 | 651,35 | 23,8 |
| 118 | STIEIK | 11,83 | 345,71 | 389,24 | 20,2 |
| 119 | STIEIK | 11,83 | 345,71 | 502,32 | 20,2 |
| 120 | STIEIK | 11,83 | 345,71 | 603,37 | 20,2 |
| 121 | SVLPAGWNIADR | 19,61 | 649,85 | 500,25 | 33,6 |
| 122 | SVLPAGWNIADR | 19,61 | 649,85 | 556,8 | 33,6 |
| 123 | SVLPAGWNIADR | 19,61 | 649,85 | 999,5 | 33,6 |
| 124 | SVLPEGWNIADR | 19,62 | 678,85 | 529,26 | 34,9 |
| 125 | SVLPEGWNIADR | 19,62 | 678,85 | 585,8 | 34,9 |
| 126 | SVLPEGWNIADR | 19,62 | 678,85 | 1057,51 | 34,9 |
| 127 | SVLPVK | 13,24 | 321,71 | 343,23 | 19,2 |
| 128 | SVLPVK | 13,24 | 321,71 | 456,32 | 19,2 |
| 129 | SVLPVK | 13,24 | 321,71 | 555,39 | 19,2 |
| 130 | SVLPVTWSIADR | 21,34 | 672,37 | 522,78 | 34,6 |
| 131 | SVLPVTWSIADR | 21,34 | 672,37 | 1044,55 | 34,6 |
| 132 | SVLPVTWSIADR | 21,34 | 672,37 | 1157,63 | 34,6 |
| 133 | TGAGGYGSR | 3,77 | 413,2 | 596,28 | 23,2 |
| 134 | TGAGGYGSR | 3,77 | 413,2 | 667,32 | 23,2 |
| 135 | TGAGGYGSR | 3,77 | 413,2 | 724,34 | 23,2 |
| 136 | TIACAK | 3,86 | 332,18 | 378,18 | 19,6 |
| 137 | TIACAK | 3,86 | 332,18 | 449,22 | 19,6 |
| 138 | TIACAK | 3,86 | 332,18 | 562,3 | 19,6 |
| 139 | TILMENSR | 13,19 | 482,25 | 505,24 | 26,2 |
| 140 | TILMENSR | 13,19 | 482,25 | 636,28 | 26,2 |
| 141 | TILMENSR | 13,19 | 482,25 | 749,36 | 26,2 |
| 142 | TLACANVLQR | 14,83 | 573,31 | 466,24 | 30,2 |
| 143 | TLACANVLQR | 14,83 | 573,31 | 860,44 | 30,2 |
| 144 | TLACANVLQR | 14,83 | 573,31 | 931,48 | 30,2 |
| 145 | TVLMENSR | 11,76 | 475,24 | 505,24 | 25,9 |
| 146 | TVLMENSR | 11,76 | 475,24 | 636,28 | 25,9 |
| 147 | TVLMENSR | 11,76 | 475,24 | 749,36 | 25,9 |
| 148 | VEPELNEGK | 11,18 | 507,76 | 393,7 | 27,3 |
| 149 | VEPELNEGK | 11,18 | 507,76 | 447,22 | 27,3 |
| 150 | VEPELNEGK | 11,18 | 507,76 | 560,3 | 27,3 |
| 151 | VNLNSTVEIK | 15,48 | 558,82 | 790,43 | 29,6 |
| 152 | VNLNSTVEIK | 15,48 | 558,82 | 903,52 | 29,6 |
| 153 | VNLNSTVEIK | 15,48 | 558,82 | 1017,56 | 29,6 |
| 154 | VNLNSTVEVK | 14,23 | 551,81 | 776,42 | 29,3 |
| 155 | VNLNSTVEVK | 14,23 | 551,81 | 889,5 | 29,3 |
| 156 | VNLNSTVEVK | 14,23 | 551,81 | 1003,54 | 29,3 |
| 157 | VNPNSTVEIK | 12,41 | 550,8 | 444,25 | 29,2 |
| 158 | VNPNSTVEIK | 12,41 | 550,8 | 887,48 | 29,2 |
| 159 | VNPNSTVEIK | 12,41 | 550,8 | 1001,53 | 29,2 |
| 160 | VNSNSTVEIK | 11,24 | 545,79 | 790,43 | 29 |
| 161 | VNSNSTVEIK | 11,24 | 545,79 | 877,46 | 29 |
| 162 | VNSNSTVEIK | 11,24 | 545,79 | 991,51 | 29 |
| 163 | WETELNEAVPGDK | 16,5 | 744,35 | 358,19 | 37,8 |
| 164 | WETELNEAVPGDK | 16,5 | 744,35 | 415,21 | 37,8 |
| 165 | WETELNEAVPGDK | 16,5 | 744,35 | 471,75 | 37,8 |
| 166 | WSIADR | 14,14 | 374,19 | 361,18 | 21,5 |
| 167 | WSIADR | 14,14 | 374,19 | 474,27 | 21,5 |
| 168 | WSIADR | 14,14 | 374,19 | 561,3 | 21,5 |

Les aires obtenues pour chacune des transitions et pour chacun des microorganismes étudiés ont été mesurées. Lorsque les aires des trois transitions d'un même peptide sont supérieures ou égales à 2500 la détection du peptide est considérée comme positive et est noté « 1 ». Lorsque au moins une transition comporte une aire inférieure à 2500, le peptide correspondant est considéré comme non détecté et est noté « 0 ».

### Exemple 24: Identification d'une résistance aux béta-lactamines de type OXA:

Les échantillons correspondant à une espèce pouvant comporter un mécanisme de résistance de type OXA peuvent être détectés en mettant en oeuvre la méthode suivante.

Chaque échantillon est traité selon l'exemple 5, puis analysé selon l'exemple 6 en détectant les peptides du **TABLEAU 30** en lieu et place des peptides du **TABLEAU 3.**

**TABLEAU 30 :**

| Transition numéro | Peptide | Temps de rétention (minutes) | (m/z) filtré en Q1 | (m/z) filtré en Q3 | Energie de collision (eV) |
|---|---|---|---|---|---|
| 1 | AAAYELAENLFEAGQADGWR | 24,48 | 728,01 | 1249,6 | 40 |
| 2 | AAAYELAENLFEAGQADGWR | 24,48 | 1091,51 | 1193,58 | 53 |
| 3 | AAAYELAENLFEAGQADGWR | 24,48 | 1091,51 | 1249,6 | 53 |
| 4 | AAEGFIPASTFK | 17,74 | 619,82 | 763,43 | 32 |
| 5 | AAEGFIPASTFK | 17,74 | 619,82 | 910,5 | 32 |
| 6 | AAEGFIPASTFK | 17,74 | 619,82 | 967,52 | 32 |
| 7 | ADGQVVAFALNMQMK | 21,27 | 811,91 | 982,48 | 41 |
| 8 | ADGQVVAFALNMQMK | 21,29 | 811,91 | 1053,52 | 41 |
| 9 | ADGQVVAFALNMQMK | 21,27 | 811,91 | 1152,59 | 41 |
| 10 | ADINEIFK | 17,3 | 475,25 | 650,35 | 26 |
| 11 | ADINEIFK | 17,3 | 475,25 | 763,43 | 26 |
| 12 | ADINEIFK | 17,3 | 475,25 | 878,46 | 26 |
| 13 | ADWGK | 6,9 | 288,64 | 390,21 | 18 |
| 14 | ADWGK | 6,91 | 288,64 | 430,17 | 18 |
| 15 | ADWGK | 6,89 | 288,64 | 505,24 | 18 |
| 16 | AEGAIVISDER | 13,52 | 387,2 | 419,19 | 23 |
| 17 | AEGAIVISDER | 13,53 | 387,2 | 506,22 | 23 |
| 18 | AEGAIVISDER | 13,52 | 387,2 | 619,3 | 23 |
| 19 | AFALNLDIDK | 20,16 | 560,31 | 717,38 | 30 |
| 20 | AFALNLDIDK | 20,16 | 560,31 | 830,46 | 30 |
| 21 | AFALNLDIDK | 20,16 | 560,31 | 901,5 | 30 |
| 22 | AFAPMSTFK | 16,96 | 500,25 | 710,35 | 27 |
| 23 | AFAPMSTFK | 16,96 | 500,25 | 781,39 | 27 |
| 24 | AFAPMSTFK | 16,96 | 500,25 | 928,46 | 27 |
| 25 | AFGYGNADVSGDPGQNNGLDR | 15,12 | 708,65 | 873,42 | 39 |
| 26 | AFGYGNADVSGDPGQNNGLDR | 15,12 | 708,65 | 970,47 | 39 |
| 27 | AFGYGNADVSGDPGQNNGLDR | 15,12 | 708,65 | 1154,47 | 39 |
| 28 | AFTMTK | 11,32 | 349,68 | 480,25 | 20 |
| 29 | AFTMTK | 11,33 | 349,68 | 552,25 | 20 |
| 30 | AFTMTK | 11,33 | 349,68 | 627,32 | 20 |
| 31 | AGDDIALR | 12,23 | 415,72 | 587,35 | 23 |
| 32 | AGDDIALR | 12,23 | 415,72 | 702,38 | 23 |
| 33 | AGDDIALR | 12,23 | 415,72 | 759,4 | 23 |
| 34 | AGHVYAFALNIDMPR | 20,63 | 558,95 | 631,32 | 32 |
| 35 | AGHVYAFALNIDMPR | 20,63 | 558,95 | 745,37 | 32 |
| 36 | AGHVYAFALNIDMPR | 20,63 | 558,95 | 817,4 | 32 |
| 37 | AGLWR | 13,44 | 301,67 | 361,2 | 18 |
| 38 | AGLWR | 13,44 | 301,67 | 474,28 | 18 |
| 39 | AGLWR | 13,44 | 301,67 | 531,3 | 18 |
| 40 | AHTEYVPASTFK | 13,18 | 450,89 | 553,3 | 27 |
| 41 | AHTEYVPASTFK | 13,18 | 450,89 | 602,26 | 27 |
| 42 | AHTEYVPASTFK | 13,18 | 450,89 | 650,35 | 27 |
| 43 | AIIPWDGKPR | 15,84 | 384,89 | 428,23 | 23 |
| 44 | AIIPWDGKPR | 15,84 | 384,89 | 457,29 | 23 |
| 45 | AIIPWDGKPR | 15,84 | 384,89 | 572,32 | 23 |
| 46 | AISDITITR | 14,8 | 495,28 | 603,38 | 27 |
| 47 | AISDITITR | 14,8 | 495,28 | 718,41 | 27 |
| 48 | AISDITITR | 14,8 | 495,28 | 805,44 | 27 |
| 49 | ALGQDR | 11,25 | 330,18 | 475,23 | 20 |
| 50 | ALGQDR | 11,25 | 330,18 | 485,24 | 20 |
| 51 | ALGQDR | 11,25 | 330,18 | 588,31 | 20 |
| 52 | ALQAK | 1,86 | 265,67 | 346,21 | 17 |
| 53 | ALQAK | 1,87 | 265,67 | 384,22 | 17 |
| 54 | ALQAK | 1,87 | 265,67 | 459,29 | 17 |
| 55 | AMETFSPASTFK | 17,06 | 658,81 | 737,38 | 34 |
| 56 | AMETFSPASTFK | 17,05 | 658,81 | 985,5 | 34 |
| 57 | AMETFSPASTFK | 17,06 | 658,81 | 1114,54 | 34 |
| 58 | AMLFLQER | 18,48 | 504,27 | 545,3 | 27 |
| 59 | AMLFLQER | 18,48 | 504,27 | 692,37 | 27 |
| 60 | AMLFLQER | 18,48 | 504,27 | 805,46 | 27 |
| 61 | AMLVFDPVR | 19,87 | 524,29 | 732,4 | 28 |
| 62 | AMLVFDPVR | 19,87 | 524,29 | 845,49 | 28 |
| 63 | AMLVFDPVR | 19,87 | 524,29 | 976,53 | 28 |
| 64 | AMTLLESGPGWELHGK | 19,32 | 575,96 | 923,47 | 33 |
| 65 | AMTLLESGPGWELHGK | 19,32 | 575,96 | 980,49 | 33 |
| 66 | AMTLLESGPGWELHGK | 19,32 | 575,96 | 1067,53 | 33 |
| 67 | ANLHITLHGK | 12,18 | 368,55 | 403,24 | 22 |
| 68 | ANLHITLHGK | 12,18 | 368,55 | 555,32 | 22 |
| 69 | ANLHITLHGK | 12,18 | 368,55 | 668,41 | 22 |
| 70 | ANQLIVK | 11,87 | 393,25 | 600,41 | 22 |
| 71 | ANQLIVK | 11,86 | 393,25 | 639,38 | 22 |
| 72 | ANQLIVK | 11,86 | 393,25 | 714,45 | 22 |
| 73 | ANTEYVPASTFK | 14,54 | 664,33 | 912,48 | 34 |
| 74 | ANTEYVPASTFK | 14,54 | 664,33 | 1041,53 | 34 |
| 75 | ANTEYVPASTFK | 14,54 | 664,33 | 1142,57 | 34 |
| 76 | ANVSR | 9,57 | 273,65 | 361,22 | 17 |
| 77 | ANVSR | 9,57 | 273,65 | 372,19 | 17 |
| 78 | ANVSR | 9,57 | 273,65 | 475,26 | 17 |
| 79 | APIGWFIGWATR | 25,58 | 687,87 | 850,46 | 35 |
| 80 | APIGWFIGWATR | 25,58 | 687,87 | 1093,56 | 35 |
| 81 | APIGWFIGWATR | 25,58 | 687,87 | 1206,64 | 35 |
| 82 | APLGWFIGWATHEER | 24,69 | 590,63 | 742,35 | 34 |
| 83 | APLGWFIGWATHEER | 24,69 | 590,63 | 985,45 | 34 |
| 84 | APLGWFIGWATHEER | 24,69 | 590,63 | 1098,53 | 34 |
| 85 | AQDEVQSMLFIEEK | 20,15 | 833,9 | 996,51 | 42 |
| 86 | AQDEVQSMLFIEEK | 20,14 | 833,9 | 1124,57 | 42 |
| 87 | AQDEVQSMLFIEEK | 20,15 | 833,9 | 1223,63 | 42 |
| 88 | AQGVIVLWNENK | 18,95 | 685,87 | 902,47 | 35 |
| 89 | AQGVIVLWNENK | 18,95 | 685,87 | 1015,56 | 35 |
| 90 | AQGVIVLWNENK | 18,95 | 685,87 | 1171,65 | 35 |
| 91 | ASAIAVYQDLAR | 18,05 | 639,35 | 765,39 | 33 |
| 92 | ASAIAVYQDLAR | 18,05 | 639,35 | 864,46 | 33 |
| 93 | ASAIAVYQDLAR | 18,05 | 639,35 | 935,49 | 33 |
| 94 | ASAILVYQDLAR | 19,08 | 660,37 | 765,39 | 34 |
| 95 | ASAILVYQDLAR | 19,08 | 660,37 | 864,46 | 34 |
| 96 | ASAILVYQDLAR | 19,08 | 660,37 | 977,54 | 34 |
| 97 | ASAIPVYQDLAR | 17,45 | 652,35 | 765,39 | 34 |
| 98 | ASAIPVYQDLAR | 17,45 | 652,35 | 864,46 | 34 |
| 99 | ASAIPVYQDLAR | 17,45 | 652,35 | 961,51 | 34 |
| 100 | ASAIPVYQDLPR | 17,59 | 665,36 | 791,4 | 34 |
| 101 | ASAIPVYQDLPR | 17,59 | 665,36 | 890,47 | 34 |
| 102 | ASAIPVYQDLPR | 17,6 | 665,36 | 987,53 | 34 |
| 103 | ASAIQVYQDLAR | 18,37 | 667,86 | 765,39 | 34 |
| 104 | ASAIQVYQDLAR | 18,37 | 667,86 | 864,46 | 34 |
| 105 | ASAIQVYQDLAR | 18,37 | 667,86 | 992,52 | 34 |
| 106 | ASAISVYQDLAR | 17,93 | 647,34 | 765,39 | 33 |
| 107 | ASAISVYQDLAR | 17,93 | 647,34 | 864,46 | 33 |
| 108 | ASAISVYQDLAR | 17,93 | 647,34 | 951,49 | 33 |
| 109 | ASALPVYQDLAR | 17,77 | 652,35 | 864,46 | 34 |
| 110 | ASALPVYQDLAR | 17,77 | 652,35 | 961,51 | 34 |
| 111 | ASALPVYQDLAR | 17,77 | 652,35 | 1074,59 | 34 |
| 112 | ASAMPVYQDLAR | 16,64 | 661,33 | 765,39 | 34 |
| 113 | ASAMPVYQDLAR | 16,64 | 661,33 | 864,46 | 34 |
| 114 | ASAMPVYQDLAR | 16,64 | 661,33 | 961,51 | 34 |
| 115 | ASAVPVYQDLAR | 16,29 | 645,35 | 765,39 | 33 |
| 116 | ASAVPVYQDLAR | 16,29 | 645,35 | 864,46 | 33 |
| 117 | ASAVPVYQDLAR | 16,29 | 645,35 | 961,51 | 33 |
| 118 | ASIEYVPASTFK | 16,7 | 656,84 | 749,42 | 34 |
| 119 | ASIEYVPASTFK | 16,7 | 656,84 | 912,48 | 34 |
| 120 | ASIEYVPASTFK | 16,7 | 656,84 | 1041,53 | 34 |
| 121 | ASNVPVYQELAR | 18,48 | 673,86 | 779,4 | 35 |
| 122 | ASNVPVYQELAR | 18,48 | 673,86 | 878,47 | 35 |
| 123 | ASNVPVYQELAR | 18,48 | 673,86 | 975,53 | 35 |
| 124 | ASPASTFK | 10,29 | 404,71 | 553,3 | 23 |
| 125 | ASPASTFK | 10,29 | 404,71 | 650,35 | 23 |
| 126 | ASPASTFK | 10,28 | 404,71 | 737,38 | 23 |
| 127 | ASTAYIPASTFK | 15,69 | 628,83 | 763,43 | 33 |
| 128 | ASTAYIPASTFK | 15,69 | 628,83 | 926,5 | 33 |
| 129 | ASTAYIPASTFK | 15,69 | 628,83 | 997,54 | 33 |
| 130 | ASTEYVPASTFK | 14,59 | 650,82 | 749,42 | 34 |
| 131 | ASTEYVPASTFK | 14,59 | 650,82 | 912,48 | 34 |
| 132 | ASTEYVPASTFK | 14,6 | 650,82 | 1041,53 | 34 |
| 133 | ASTTEVFK | 11,78 | 441,73 | 623,34 | 24 |
| 134 | ASTTEVFK | 11,78 | 441,73 | 724,39 | 24 |
| 135 | ASTTEVFK | 11,78 | 441,73 | 811,42 | 24 |
| 136 | ATSTEIFK | 13,15 | 448,74 | 637,36 | 25 |
| 137 | ATSTEIFK | 13,15 | 448,74 | 724,39 | 25 |
| 138 | ATSTEIFK | 13,15 | 448,74 | 825,44 | 25 |
| 139 | ATTNEIFK | 13,21 | 462,25 | 650,35 | 25 |
| 140 | ATTNEIFK | 13,21 | 462,25 | 751,4 | 25 |
| 141 | ATTNEIFK | 13,21 | 462,25 | 852,45 | 25 |
| 142 | ATTTAVFK | 11,9 | 419,74 | 464,29 | 23 |
| 143 | ATTTAVFK | 11,9 | 419,74 | 565,33 | 23 |
| 144 | ATTTAVFK | 11,9 | 419,74 | 666,38 | 23 |
| 145 | ATTTEIFK | 13,64 | 455,75 | 637,36 | 25 |
| 146 | ATTTEIFK | 13,65 | 455,75 | 738,4 | 25 |
| 147 | ATTTEIFK | 13,65 | 455,75 | 839,45 | 25 |
| 148 | ATTTEVFK | 11,98 | 448,74 | 623,34 | 25 |
| 149 | ATTTEVFK | 11,98 | 448,74 | 724,39 | 25 |
| 150 | ATTTEVFK | 11,98 | 448,74 | 825,44 | 25 |
| 151 | AVSDITILEQTDNYTLHGK | 19,19 | 706,7 | 974,49 | 39 |
| 152 | AVSDITILEQTDNYTLHGK | 19,19 | 706,7 | 1048,51 | 39 |
| 153 | AVSDITILEQTDNYTLHGK | 19,18 | 706,7 | 1176,56 | 39 |
| 154 | AVSDITILEQTYNYTLHGK | 22,29 | 722,71 | 995,49 | 40 |
| 155 | AVSDITILEQTYNYTLHGK | 22,29 | 722,71 | 998,5 | 40 |
| 156 | AVSDITILEQTYNYTLHGK | 22,28 | 722,71 | 1224,6 | 40 |
| 157 | AVVPHFEAGDWDVQGK | 17,81 | 585,62 | 743,34 | 33 |
| 158 | AVVPHFEAGDWDVQGK | 17,81 | 585,62 | 792,88 | 33 |
| 159 | AVVPHFEAGDWDVQGK | 17,81 | 585,62 | 904,42 | 33 |
| 160 | AWEHDMSLR | 13,99 | 572,76 | 758,36 | 30 |
| 161 | AWEHDMSLR | 13,99 | 572,76 | 887,4 | 30 |
| 162 | AWEHDMSLR | 13,99 | 572,76 | 1073,48 | 30 |
| 163 | AWIGSSLQISPLEQLEFLGK | 26,98 | 739,4 | 963,51 | 41 |
| 164 | AWIGSSLQISPLEQLEFLGK | 26,99 | 739,4 | 1173,65 | 41 |
| 165 | AWIGSSLQISPLEQLEFLGK | 26,98 | 1108,6 | 1173,65 | 54 |
| 166 | DAFLK | 12,42 | 297,17 | 407,27 | 18 |
| 167 | DAFLK | 12,43 | 297,17 | 447,22 | 18 |
| 168 | DAFLK | 12,42 | 297,17 | 478,3 | 18 |
| 169 | DDFILHGK | 13,99 | 472,75 | 714,43 | 26 |
| 170 | DDFILHGK | 13,99 | 472,75 | 798,38 | 26 |
| 171 | DDFILHGK | 13,99 | 472,75 | 829,46 | 26 |
| 172 | DDVLK | 8,62 | 295,16 | 359,27 | 18 |
| 173 | DDVLK | 8,63 | 295,16 | 443,21 | 18 |
| 174 | DDVLK | 8,62 | 295,16 | 474,29 | 18 |
| 175 | DEFHVFR | 15,39 | 475,23 | 705,38 | 26 |
| 176 | DEFHVFR | 15,39 | 475,23 | 775,34 | 26 |
| 177 | DEFHVFR | 15,39 | 475,23 | 834,43 | 26 |
| 178 | DEFQIFR | 19,02 | 477,74 | 520,2 | 26 |
| 179 | DEFQIFR | 19,02 | 477,74 | 563,33 | 26 |
| 180 | DEFQIFR | 19,02 | 477,74 | 710,4 | 26 |
| 181 | DEFQVFR | 17,29 | 470,73 | 549,31 | 26 |
| 182 | DEFQVFR | 17,28 | 470,73 | 619,27 | 26 |
| 183 | DEFQVFR | 17,29 | 470,73 | 696,38 | 26 |
| 184 | DELVR | 9,33 | 316,17 | 387,27 | 19 |
| 185 | DELVR | 9,35 | 316,17 | 457,23 | 19 |
| 186 | DELVR | 9,33 | 316,17 | 516,31 | 19 |
| 187 | DFDYGNQDFSGDK | 14,72 | 754,3 | 967,41 | 38 |
| 188 | DFDYGNQDFSGDK | 14,72 | 754,3 | 1130,47 | 38 |
| 189 | DFDYGNQDFSGDK | 14,72 | 754,3 | 1245,5 | 38 |
| 190 | DFTLGEAMQASTVPVYQELAR | 24,19 | 776,05 | 975,53 | 43 |
| 191 | DFTLGEAMQASTVPVYQELAR | 24,19 | 776,05 | 1074,59 | 43 |
| 192 | DFTLGEAMQASTVPVYQELAR | 24,19 | 1163,57 | 1175,64 | 56 |
| 193 | DHDLITAMK | 14,23 | 522,26 | 563,32 | 28 |
| 194 | DHDLITAMK | 14,23 | 522,26 | 695,34 | 28 |
| 195 | DHDLITAMK | 14,23 | 522,26 | 791,43 | 28 |
| 196 | DIAAWNR | 13,63 | 423,22 | 546,28 | 24 |
| 197 | DIAAWNR | 13,63 | 423,22 | 617,32 | 24 |
| 198 | DIAAWNR | 13,62 | 423,22 | 730,4 | 24 |
| 199 | DILYIQELAGGWK | 24,49 | 753,4 | 888,46 | 38 |
| 200 | DILYIQELAGGWK | 24,48 | 753,4 | 1001,54 | 38 |
| 201 | DILYIQELAGGWK | 24,49 | 753,4 | 1164,6 | 38 |
| 202 | DITILEK | 15,9 | 416,24 | 603,37 | 23 |
| 203 | DITILEK | 15,91 | 416,24 | 685,38 | 23 |
| 204 | DITILEK | 15,91 | 416,24 | 716,46 | 23 |
| 205 | DLLSAK | 12,45 | 323,69 | 429,23 | 19 |
| 206 | DLLSAK | 12,44 | 323,69 | 500,27 | 19 |
| 207 | DLLSAK | 12,45 | 323,69 | 531,35 | 19 |
| 208 | DLMITEAGR | 15,07 | 503,26 | 533,27 | 27 |
| 209 | DLMITEAGR | 15,07 | 503,26 | 646,35 | 27 |
| 210 | DLMITEAGR | 15,07 | 503,26 | 777,39 | 27 |
| 211 | DLMIVEAGR | 16,68 | 502,27 | 531,29 | 27 |
| 212 | DLMIVEAGR | 16,68 | 502,27 | 644,37 | 27 |
| 213 | DLMIVEAGR | 16,68 | 502,27 | 775,41 | 27 |
| 214 | DLMIVEAK | 16,23 | 459,75 | 473,24 | 25 |
| 215 | DLMIVEAK | 16,23 | 459,75 | 559,34 | 25 |
| 216 | DLMIVEAK | 16,23 | 459,75 | 690,39 | 25 |
| 217 | DLSGNPGK | 6,69 | 394,2 | 472,25 | 22 |
| 218 | DLSGNPGK | 6,69 | 394,2 | 559,28 | 22 |
| 219 | DLSGNPGK | 6,7 | 394,2 | 672,37 | 22 |
| 220 | DLSLR | 12,37 | 302,18 | 375,24 | 18 |
| 221 | DLSLR | 12,35 | 302,18 | 429,23 | 18 |
| 222 | DLSLR | 12,36 | 302,18 | 488,32 | 18 |
| 223 | DLTLR | 12,48 | 309,18 | 389,25 | 19 |
| 224 | DLTLR | 12,47 | 309,18 | 443,25 | 19 |
| 225 | DLTLR | 12,47 | 309,18 | 502,33 | 19 |
| 226 | DMTLGDAIK | 15,97 | 482,24 | 503,28 | 26 |
| 227 | DMTLGDAIK | 15,97 | 482,24 | 616,37 | 26 |
| 228 | DMTLGDAIK | 15,97 | 482,24 | 717,41 | 26 |
| 229 | DMTLGDAMALSAVPVYQELAR | 25,76 | 751,04 | 975,53 | 42 |
| 230 | DMTLGDAMALSAVPVYQELAR | 25,76 | 1126,06 | 1145,63 | 55 |
| 231 | DMTLGDAMALSAVPVYQELAR | 25,75 | 1126,06 | 1232,66 | 55 |
| 232 | DMTLGDAMK | 14,46 | 491,22 | 634,32 | 27 |
| 233 | DMTLGDAMK | 14,46 | 491,22 | 735,37 | 27 |
| 234 | DMTLGDAMK | 14,46 | 491,22 | 866,41 | 27 |
| 235 | DMTLGEAMALSAVPVYQDLAR | 25,92 | 751,04 | 961,51 | 42 |
| 236 | DMTLGEAMALSAVPVYQDLAR | 25,92 | 1126,06 | 1131,62 | 55 |
| 237 | DMTLGEAMALSAVPVYQDLAR | 25,92 | 1126,06 | 1218,65 | 55 |
| 238 | DMTLGEAMALSAVPVYQELAR | 26,48 | 755,71 | 779,4 | 42 |
| 239 | DMTLGEAMALSAVPVYQELAR | 26,48 | 755,71 | 975,53 | 42 |
| 240 | DMTLGEAMALSAVPVYQELAR | 26,47 | 1133,07 | 1232,66 | 55 |
| 241 | DMTLGEAMK | 15,09 | 498,23 | 535,25 | 27 |
| 242 | DMTLGEAMK | 15,09 | 498,23 | 648,34 | 27 |
| 243 | DMTLGEAMK | 15,09 | 498,23 | 749,39 | 27 |
| 244 | DMTLGQAMQASAVPVYQELAR | 23,29 | 760,38 | 779,4 | 42 |
| 245 | DMTLGQAMQASAVPVYQELAR | 23,29 | 760,38 | 975,53 | 42 |
| 246 | DMTLGQAMQASAVPVYQELAR | 23,29 | 760,38 | 976,42 | 42 |
| 247 | DQDLR | 2,54 | 323,66 | 403,23 | 19 |
| 248 | DQDLR | 2,55 | 323,66 | 472,2 | 19 |
| 249 | DQDLR | 2,55 | 323,66 | 531,29 | 19 |
| 250 | DQQIGWFVGWASKPGK | 21,64 | 601,98 | 830,45 | 34 |
| 251 | DQQIGWFVGWASKPGK | 21,64 | 902,46 | 929,52 | 45 |
| 252 | DQQIGWFVGWASKPGK | 21,64 | 902,46 | 1076,59 | 45 |
| 253 | DQQVQVYGNDLNR | 13,59 | 774,87 | 851,4 | 39 |
| 254 | DQQVQVYGNDLNR | 13,58 | 774,87 | 950,47 | 39 |
| 255 | DQQVQVYGNDLNR | 13,59 | 774,87 | 1078,53 | 39 |
| 256 | DQTLESAFK | 15,21 | 519,76 | 581,29 | 28 |
| 257 | DQTLESAFK | 15,21 | 519,76 | 694,38 | 28 |
| 258 | DQTLESAFK | 15,21 | 519,76 | 795,42 | 28 |
| 259 | DSIVWYSQELTR | 19,61 | 748,87 | 896,45 | 38 |
| 260 | DSIVWYSQELTR | 19,61 | 748,87 | 1082,53 | 38 |
| 261 | DSIVWYSQELTR | 19,61 | 748,87 | 1181,59 | 38 |
| 262 | DSIVWYSQQLTR | 19,1 | 748,38 | 895,46 | 38 |
| 263 | DSIVWYSQQLTR | 19,11 | 748,38 | 1081,54 | 38 |
| 264 | DSIVWYSQQLTR | 19,1 | 748,38 | 1180,61 | 38 |
| 265 | DSNLR | 1,77 | 302,66 | 402,25 | 18 |
| 266 | DSNLR | 1,77 | 302,66 | 430,19 | 18 |
| 267 | DSNLR | 1,77 | 302,66 | 489,28 | 18 |
| 268 | DSYIAWGGEAWK | 19,67 | 691,82 | 833,39 | 35 |
| 269 | DSYIAWGGEAWK | 19,67 | 691,82 | 904,43 | 35 |
| 270 | DSYIAWGGEAWK | 19,66 | 691,82 | 1017,52 | 35 |
| 271 | DTLNPEWPYK | 17,3 | 631,81 | 819,4 | 33 |
| 272 | DTLNPEWPYK | 17,3 | 631,81 | 933,45 | 33 |
| 273 | DTLNPEWPYK | 17,3 | 631,81 | 1046,53 | 33 |
| 274 | DVDEVFYK | 15,62 | 507,74 | 685,36 | 27 |
| 275 | DVDEVFYK | 15,62 | 507,74 | 800,38 | 27 |
| 276 | DVDEVFYK | 15,62 | 507,74 | 899,45 | 27 |
| 277 | DWILR | 17,44 | 351,7 | 415,2 | 20 |
| 278 | DWILR | 17,44 | 351,7 | 528,28 | 20 |
| 279 | DWILR | 17,44 | 351,7 | 587,37 | 20 |
| 280 | EAFLR | 12,51 | 318,18 | 435,27 | 19 |
| 281 | EAFLR | 12,51 | 318,18 | 461,24 | 19 |
| 282 | EAFLR | 12,51 | 318,18 | 506,31 | 19 |
| 283 | EAIVR | 7,84 | 294,18 | 387,27 | 18 |
| 284 | EAIVR | 7,84 | 294,18 | 413,24 | 18 |
| 285 | EAIVR | 7,84 | 294,18 | 458,31 | 18 |
| 286 | EAIVTEATPEYIVHSK | 16,43 | 596,31 | 746,42 | 34 |
| 287 | EAIVTEATPEYIVHSK | 16,43 | 596,31 | 972,51 | 34 |
| 288 | EAIVTEATPEYIVHSK | 16,42 | 596,31 | 1073,56 | 34 |
| 289 | EALVTEAAPEYLVHSK | 17,3 | 586,31 | 875,46 | 33 |
| 290 | EALVTEAAPEYLVHSK | 17,3 | 586,31 | 972,51 | 33 |
| 291 | EALVTEAAPEYLVHSK | 17,3 | 586,31 | 1114,59 | 33 |
| 292 | EALVTEAPEYLVHSK | 17,58 | 562,63 | 637,32 | 32 |
| 293 | EALVTEAPEYLVHSK | 17,58 | 562,63 | 972,51 | 32 |
| 294 | EALVTEAPEYLVHSK | 17,58 | 562,63 | 1043,55 | 32 |
| 295 | EEIVR | 8,41 | 323,18 | 387,27 | 19 |
| 296 | EEIVR | 8,4 | 323,18 | 471,24 | 19 |
| 297 | EEIVR | 8,4 | 323,18 | 516,31 | 19 |
| 298 | EEVLAALPAQLK | 19,48 | 641,37 | 740,47 | 33 |
| 299 | EEVLAALPAQLK | 19,47 | 641,37 | 811,5 | 33 |
| 300 | EEVLAALPAQLK | 19,47 | 641,37 | 924,59 | 33 |
| 301 | EFSAEAVNGVFVLC[CAM]K | 21,1 | 835,42 | 936,5 | 42 |
| 302 | EFSAEAVNGVFVLC[CAM]K | 21,1 | 835,42 | 1106,6 | 42 |
| 303 | EFSAEAVNGVFVLC[CAM]K | 21,1 | 835,42 | 1235,65 | 42 |
| 304 | EFSSESVHGVFVLC[CAM]K | 18,26 | 575,62 | 666,36 | 33 |
| 305 | EFSSESVHGVFVLC[CAM]K | 18,26 | 575,62 | 822,45 | 33 |
| 306 | EFSSESVHGVFVLC[CAM]K | 18,26 | 575,62 | 959,51 | 33 |
| 307 | EGMSGSIR | 9,88 | 418,7 | 432,26 | 23 |
| 308 | EGMSGSIR | 9,88 | 418,7 | 519,29 | 23 |
| 309 | EGMSGSIR | 9,88 | 418,7 | 707,35 | 23 |
| 310 | EGMTGSIR | 10,63 | 425,71 | 432,26 | 24 |
| 311 | EGMTGSIR | 10,63 | 425,71 | 533,3 | 24 |
| 312 | EGMTGSIR | 10,63 | 425,71 | 664,34 | 24 |
| 313 | EIAVWNR | 14,78 | 444,24 | 475,24 | 25 |
| 314 | EIAVWNR | 14,77 | 444,24 | 574,31 | 25 |
| 315 | EIAVWNR | 14,77 | 444,24 | 645,35 | 25 |
| 316 | EIAYK | 8,46 | 312,17 | 381,21 | 19 |
| 317 | EIAYK | 8,46 | 312,17 | 477,23 | 19 |
| 318 | EIAYK | 8,46 | 312,17 | 494,3 | 19 |
| 319 | EIFER | 11,7 | 347,18 | 451,23 | 20 |
| 320 | EIFER | 11,7 | 347,18 | 519,24 | 20 |
| 321 | EIFER | 11,7 | 347,18 | 564,31 | 20 |
| 322 | EIFYHYR | 13,31 | 514,25 | 785,37 | 28 |
| 323 | EIFYHYR | 13,31 | 514,25 | 853,39 | 28 |
| 324 | EIFYHYR | 13,32 | 514,25 | 898,46 | 28 |
| 325 | EIGDDK | 1,99 | 338,66 | 434,19 | 20 |
| 326 | EIGDDK | 1,99 | 338,66 | 530,21 | 20 |
| 327 | EIGDDK | 1,99 | 338,66 | 547,27 | 20 |
| 328 | EIGDGK | 1,76 | 309,66 | 376,18 | 19 |
| 329 | EIGDGK | 1,75 | 309,66 | 472,2 | 19 |
| 330 | EIGDGK | 1,75 | 309,66 | 489,27 | 19 |
| 331 | EIGEDK | 2,32 | 345,67 | 448,2 | 20 |
| 332 | EIGEDK | 2,33 | 345,67 | 544,22 | 20 |
| 333 | EIGEDK | 2,33 | 345,67 | 561,29 | 20 |
| 334 | EIGEDNAR | 10,05 | 452,21 | 604,27 | 25 |
| 335 | EIGEDNAR | 10,05 | 452,21 | 661,29 | 25 |
| 336 | EIGEDNAR | 10,06 | 452,21 | 774,37 | 25 |
| 337 | EIGENK | 1,86 | 345,18 | 447,22 | 20 |
| 338 | EIGENK | 1,86 | 345,18 | 543,24 | 20 |
| 339 | EIGENK | 1,86 | 345,18 | 560,3 | 20 |
| 340 | EIGSEIDK | 11,04 | 445,73 | 591,3 | 25 |
| 341 | EIGSEIDK | 11,04 | 445,73 | 648,32 | 25 |
| 342 | EIGSEIDK | 11,04 | 445,73 | 761,4 | 25 |
| 343 | EMIYLK | 15,11 | 398,72 | 536,34 | 23 |
| 344 | EMIYLK | 15,11 | 398,72 | 650,32 | 23 |
| 345 | EMIYLK | 15,11 | 398,72 | 667,38 | 23 |
| 346 | EMLYVER | 14,12 | 470,23 | 566,29 | 26 |
| 347 | EMLYVER | 14,12 | 470,23 | 679,38 | 26 |
| 348 | EMLYVER | 14,12 | 470,23 | 810,42 | 26 |
| 349 | ENIEK | 11,07 | 316,67 | 389,24 | 19 |
| 350 | ENIEK | 11,07 | 316,67 | 486,22 | 19 |
| 351 | ENIEK | 11,07 | 316,67 | 503,28 | 19 |
| 352 | ENQLIVK | 12,15 | 422,25 | 472,35 | 24 |
| 353 | ENQLIVK | 12,15 | 422,25 | 600,41 | 24 |
| 354 | ENQLIVK | 12,15 | 422,25 | 714,45 | 24 |
| 355 | EQAILLFR | 19,88 | 495,29 | 548,36 | 27 |
| 356 | EQAILLFR | 19,88 | 495,29 | 661,44 | 27 |
| 357 | EQAILLFR | 19,88 | 495,29 | 732,48 | 27 |
| 358 | EQIQFLLR | 19,45 | 523,8 | 548,36 | 28 |
| 359 | EQIQFLLR | 19,45 | 523,8 | 676,41 | 28 |
| 360 | EQIQFLLR | 19,45 | 523,8 | 789,5 | 28 |
| 361 | EQLAFDPQVQQQVK | 16,43 | 829,43 | 954,54 | 41 |
| 362 | EQLAFDPQVQQQVK | 16,42 | 829,43 | 1069,56 | 41 |
| 363 | EQLAFDPQVQQQVK | 16,42 | 829,43 | 1216,63 | 41 |
| 364 | EQVDFVQR | 13,09 | 510,76 | 549,31 | 27 |
| 365 | EQVDFVQR | 13,09 | 510,76 | 664,34 | 27 |
| 366 | EQVDFVQR | 13,09 | 510,76 | 763,41 | 27 |
| 367 | EVGEIR | 9,35 | 351,69 | 474,27 | 20 |
| 368 | EVGEIR | 9,35 | 351,69 | 528,27 | 20 |
| 369 | EVGEIR | 9,35 | 351,69 | 573,34 | 20 |
| 370 | EVGEVR | 6,91 | 344,68 | 460,25 | 20 |
| 371 | EVGEVR | 6,91 | 344,68 | 514,25 | 20 |
| 372 | EVGEVR | 6,91 | 344,68 | 559,32 | 20 |
| 373 | EYLPASTFK | 15,41 | 528,27 | 553,3 | 28 |
| 374 | EYLPASTFK | 15,41 | 528,27 | 650,35 | 28 |
| 375 | EYLPASTFK | 15,41 | 528,27 | 763,43 | 28 |
| 376 | EYLPVSTFK | 17,16 | 542,29 | 581,33 | 29 |
| 377 | EYLPVSTFK | 17,16 | 542,29 | 678,38 | 29 |
| 378 | EYLPVSTFK | 17,16 | 542,29 | 791,47 | 29 |
| 379 | EYNTSGTFVFYDGK | 18,2 | 814,37 | 1033,5 | 41 |
| 380 | EYNTSGTFVFYDGK | 18,2 | 814,37 | 1120,53 | 41 |
| 381 | EYNTSGTFVFYDGK | 18,2 | 814,37 | 1221,58 | 41 |
| 382 | EYVPASTFK | 13,89 | 521,27 | 553,3 | 28 |
| 383 | EYVPASTFK | 13,89 | 521,27 | 650,35 | 28 |
| 384 | EYVPASTFK | 13,89 | 521,27 | 749,42 | 28 |
| 385 | FAPESTFK | 13,67 | 463,73 | 482,26 | 25 |
| 386 | FAPESTFK | 13,67 | 463,73 | 611,3 | 25 |
| 387 | FAPESTFK | 13,67 | 463,73 | 708,36 | 25 |
| 388 | FAQYAK | 9,39 | 364,19 | 509,27 | 21 |
| 389 | FAQYAK | 9,39 | 364,19 | 580,31 | 21 |
| 390 | FAQYAK | 9,39 | 364,19 | 581,27 | 21 |
| 391 | FDYGNR | 10,1 | 386,17 | 509,25 | 22 |
| 392 | FDYGNR | 10,1 | 386,17 | 597,23 | 22 |
| 393 | FDYGNR | 10,09 | 386,17 | 624,27 | 22 |
| 394 | FEDLYK | 13,52 | 407,7 | 423,26 | 23 |
| 395 | FEDLYK | 13,52 | 407,7 | 538,29 | 23 |
| 396 | FEDLYK | 13,52 | 407,7 | 667,33 | 23 |
| 397 | FEDTFHISNQK | 14,33 | 455,89 | 476,25 | 27 |
| 398 | FEDTFHISNQK | 14,33 | 455,89 | 589,33 | 27 |
| 399 | FEDTFHISNQK | 14,33 | 455,89 | 726,39 | 27 |
| 400 | FEDTFHTSNQQHEK | 10,66 | 583,26 | 870,41 | 33 |
| 401 | FEDTFHTSNQQHEK | 10,66 | 583,26 | 971,45 | 33 |
| 402 | FEDTFHTSNQQHEK | 10,66 | 583,26 | 1108,51 | 33 |
| 403 | FEYGNQDVSGDSGK | 11,95 | 751,82 | 764,34 | 38 |
| 404 | FEYGNQDVSGDSGK | 11,95 | 751,82 | 1063,47 | 38 |
| 405 | FEYGNQDVSGDSGK | 11,95 | 751,82 | 1226,53 | 38 |
| 406 | FFSDFQAK | 16 | 495,24 | 608,3 | 27 |
| 407 | FFSDFQAK | 16 | 495,24 | 695,34 | 27 |
| 408 | FFSDFQAK | 16 | 495,24 | 842,4 | 27 |
| 409 | FFSDLQAEGAIVIADER | 20,44 | 627,65 | 1143,6 | 35 |
| 410 | FFSDLQAEGAIVIADER | 20,43 | 940,97 | 1143,6 | 46 |
| 411 | FFSDLQAEGAIVIADER | 20,44 | 940,97 | 1179,57 | 46 |
| 412 | FFSDLR | 15,38 | 392,7 | 490,26 | 22 |
| 413 | FFSDLR | 15,38 | 392,7 | 610,29 | 22 |
| 414 | FFSDLR | 15,38 | 392,7 | 637,33 | 22 |
| 415 | FFSEFQAK | 16,13 | 502,25 | 622,32 | 27 |
| 416 | FFSEFQAK | 16,13 | 502,25 | 709,35 | 27 |
| 417 | FFSEFQAK | 16,13 | 502,25 | 856,42 | 27 |
| 418 | FGLEGQLR | 15,8 | 460,25 | 473,28 | 25 |
| 419 | FGLEGQLR | 15,8 | 460,25 | 602,33 | 25 |
| 420 | FGLEGQLR | 15,8 | 460,25 | 772,43 | 25 |
| 421 | FLESLYLNNLPASK | 20,75 | 804,94 | 856,49 | 40 |
| 422 | FLESLYLNNLPASK | 20,75 | 804,94 | 1019,55 | 40 |
| 423 | FLESLYLNNLPASK | 20,75 | 804,94 | 1219,67 | 40 |
| 424 | FLLEGQLR | 18,06 | 488,28 | 602,33 | 26 |
| 425 | FLLEGQLR | 18,06 | 488,28 | 715,41 | 26 |
| 426 | FLLEGQLR | 18,06 | 488,28 | 828,49 | 26 |
| 427 | FQQYVDR | 11,19 | 478,24 | 552,28 | 26 |
| 428 | FQQYVDR | 11,19 | 478,24 | 680,34 | 26 |
| 429 | FQQYVDR | 11,19 | 478,24 | 808,39 | 26 |
| 430 | FSDYVQR | 11,83 | 457,72 | 565,31 | 25 |
| 431 | FSDYVQR | 11,83 | 457,72 | 680,34 | 25 |
| 432 | FSDYVQR | 11,83 | 457,72 | 767,37 | 25 |
| 433 | FSTASTFK | 12,71 | 444,73 | 553,3 | 25 |
| 434 | FSTASTFK | 12,7 | 444,73 | 654,35 | 25 |
| 435 | FSTASTFK | 12,7 | 444,73 | 741,38 | 25 |
| 436 | FSWDGK | 14,32 | 370,17 | 505,24 | 21 |
| 437 | FSWDGK | 14,32 | 370,17 | 592,27 | 21 |
| 438 | FSWDGK | 14,32 | 370,17 | 593,24 | 21 |
| 439 | FSYGNQNISGGIDK | 14,61 | 750,36 | 803,43 | 38 |
| 440 | FSYGNQNISGGIDK | 14,61 | 750,36 | 1045,53 | 38 |
| 441 | FSYGNQNISGGIDK | 14,61 | 750,36 | 1102,55 | 38 |
| 442 | FSYGNQNISGGTDK | 12,74 | 744,34 | 791,39 | 38 |
| 443 | FSYGNQNISGGTDK | 12,74 | 744,34 | 1033,49 | 38 |
| 444 | FSYGNQNISGGTDK | 12,74 | 744,34 | 1090,51 | 38 |
| 445 | FSYGSQNISGGIDK | 14,74 | 736,85 | 803,43 | 37 |
| 446 | FSYGSQNISGGIDK | 14,74 | 736,85 | 1075,54 | 37 |
| 447 | FSYGSQNISGGIDK | 14,75 | 736,85 | 1238,6 | 37 |
| 448 | FTEYVK | 11,81 | 393,71 | 538,29 | 22 |
| 449 | FTEYVK | 11,81 | 393,71 | 639,33 | 22 |
| 450 | FTEYVK | 11,81 | 393,71 | 640,3 | 22 |
| 451 | FVPASTYK | 11,76 | 456,74 | 498,26 | 25 |
| 452 | FVPASTYK | 11,76 | 456,74 | 569,29 | 25 |
| 453 | FVPASTYK | 11,77 | 456,74 | 666,35 | 25 |
| 454 | FVYDLAQGQLPFKPEVQQQVK | 20,48 | 821,44 | 955,52 | 45 |
| 455 | FVYDLAQGQLPFKPEVQQQVK | 20,48 | 821,44 | 1108,59 | 45 |
| 456 | FVYDLAQGQLPFKPEVQQQVK | 20,49 | 821,44 | 1109,07 | 45 |
| 457 | FWLEDQLR | 20,39 | 553,79 | 660,33 | 29 |
| 458 | FWLEDQLR | 20,39 | 553,79 | 773,42 | 29 |
| 459 | FWLEDQLR | 20,38 | 553,79 | 959,49 | 29 |
| 460 | FWLEGPLK | 20,63 | 495,28 | 543,31 | 27 |
| 461 | FWLEGPLK | 20,63 | 495,28 | 656,4 | 27 |
| 462 | FWLEGPLK | 20,63 | 495,28 | 842,48 | 27 |
| 463 | FWLEGQLR | 19,49 | 524,78 | 602,33 | 28 |
| 464 | FWLEGQLR | 19,49 | 524,78 | 715,41 | 28 |
| 465 | FWLEGQLR | 19,48 | 524,78 | 901,49 | 28 |
| 466 | FYPASSFK | 14,74 | 473,74 | 636,34 | 26 |
| 467 | FYPASSFK | 14,74 | 473,74 | 799,4 | 26 |
| 468 | FYPASSFK | 14,74 | 473,74 | 800,36 | 26 |
| 469 | FYPASTFK | 14,98 | 480,74 | 553,3 | 26 |
| 470 | FYPASTFK | 14,99 | 480,74 | 650,35 | 26 |
| 471 | FYPASTFK | 14,98 | 480,74 | 813,41 | 26 |
| 472 | GAIQVSAVPVFQQIAR | 21,6 | 842,48 | 958,55 | 42 |
| 473 | GAIQVSAVPVFQQIAR | 21,6 | 842,48 | 1057,62 | 42 |
| 474 | GAIQVSAVPVFQQIAR | 21,59 | 842,48 | 1128,65 | 42 |
| 475 | GAIQVSAVPVFQQITR | 21,52 | 857,49 | 988,56 | 43 |
| 476 | GAIQVSAVPVFQQITR | 21,51 | 857,49 | 1087,63 | 43 |
| 477 | GAIQVSAVPVFQQITR | 21,52 | 857,49 | 1158,66 | 43 |
| 478 | GELPVSEDALEMTK | 18,1 | 759,87 | 936,43 | 38 |
| 479 | GELPVSEDALEMTK | 18,11 | 759,87 | 1023,47 | 38 |
| 480 | GELPVSEDALEMTK | 18,11 | 759,87 | 1122,53 | 38 |
| 481 | GISSSVR | 8,65 | 353,2 | 448,25 | 21 |
| 482 | GISSSVR | 8,65 | 353,2 | 535,28 | 21 |
| 483 | GISSSVR | 8,67 | 353,2 | 648,37 | 21 |
| 484 | GNQTLVFAR | 14,83 | 503,28 | 605,38 | 27 |
| 485 | GNQTLVFAR | 14,83 | 503,28 | 706,42 | 27 |
| 486 | GNQTLVFAR | 14,83 | 503,28 | 834,48 | 27 |
| 487 | GPLEISAFEEAR | 18,95 | 659,84 | 809,38 | 34 |
| 488 | GPLEISAFEEAR | 18,94 | 659,84 | 922,46 | 34 |
| 489 | GPLEISAFEEAR | 18,94 | 659,84 | 1051,51 | 34 |
| 490 | GPLTITPIQEVK | 18,14 | 648,38 | 814,47 | 34 |
| 491 | GPLTITPIQEVK | 18,15 | 648,38 | 927,55 | 34 |
| 492 | GPLTITPIQEVK | 18,14 | 648,38 | 1028,6 | 34 |
| 493 | GSLLLWDQK | 19,61 | 530,3 | 576,28 | 28 |
| 494 | GSLLLWDQK | 19,61 | 530,3 | 689,36 | 28 |
| 495 | GSLLLWDQK | 19,61 | 530,3 | 802,45 | 28 |
| 496 | GTFVLYDVQR | 17,93 | 599,32 | 680,34 | 31 |
| 497 | GTFVLYDVQR | 17,93 | 599,32 | 793,42 | 31 |
| 498 | GTFVLYDVQR | 17,93 | 599,32 | 892,49 | 31 |
| 499 | GTIVVADER | 11,82 | 480,26 | 490,23 | 26 |
| 500 | GTIVVADER | 11,82 | 480,26 | 589,29 | 26 |
| 501 | GTIVVADER | 11,82 | 480,26 | 688,36 | 26 |
| 502 | GTIVVLDAR | 15,77 | 472,28 | 573,34 | 26 |
| 503 | GTIVVLDAR | 15,77 | 472,28 | 672,4 | 26 |
| 504 | GTIVVLDAR | 15,77 | 472,28 | 785,49 | 26 |
| 505 | GTIVVVDER | 13,6 | 494,28 | 518,26 | 27 |
| 506 | GTIVVVDER | 13,6 | 494,28 | 617,33 | 27 |
| 507 | GTIVVVDER | 13,6 | 494,28 | 716,39 | 27 |
| 508 | GTLPFSAR | 14,96 | 424,73 | 577,31 | 24 |
| 509 | GTLPFSAR | 14,96 | 424,73 | 690,39 | 24 |
| 510 | GTLPFSAR | 14,97 | 424,73 | 791,44 | 24 |
| 511 | HIADSK | 11,91 | 335,68 | 420,21 | 20 |
| 512 | HIADSK | 11,9 | 335,68 | 524,25 | 20 |
| 513 | HIADSK | 11,91 | 335,68 | 533,29 | 20 |
| 514 | HNGTDGAWIISSLR | 19,36 | 509,6 | 575,35 | 29 |
| 515 | HNGTDGAWIISSLR | 19,35 | 509,6 | 653,26 | 29 |
| 516 | HNGTDGAWIISSLR | 19,36 | 509,6 | 688,44 | 29 |
| 517 | HTLSVFDQER | 14,25 | 411,21 | 432,22 | 25 |
| 518 | HTLSVFDQER | 14,25 | 411,21 | 547,25 | 25 |
| 519 | HTLSVFDQER | 14,25 | 411,21 | 694,32 | 25 |
| 520 | HVTFASFR | 14,36 | 322,17 | 338,18 | 20 |
| 521 | HVTFASFR | 14,36 | 322,17 | 409,22 | 20 |
| 522 | HVTFASFR | 14,36 | 322,17 | 485,25 | 20 |
| 523 | IAISLMGYDAGFLR | 23,93 | 763,91 | 898,44 | 39 |
| 524 | IAISLMGYDAGFLR | 23,93 | 763,91 | 1029,48 | 39 |
| 525 | IAISLMGYDAGFLR | 23,94 | 763,91 | 1229,6 | 39 |
| 526 | IALSLMGFDSGILK | 24,91 | 732,91 | 836,45 | 37 |
| 527 | IALSLMGFDSGILK | 24,91 | 732,91 | 967,49 | 37 |
| 528 | IALSLMGFDSGILK | 24,91 | 732,91 | 1167,61 | 37 |
| 529 | IANALIGLENHK | 15,95 | 431,58 | 697,36 | 26 |
| 530 | IANALIGLENHK | 15,95 | 646,87 | 697,36 | 33 |
| 531 | IANALIGLENHK | 15,95 | 646,87 | 810,45 | 33 |
| 532 | IDTFWLDNSLK | 21,79 | 676,35 | 689,38 | 35 |
| 533 | IDTFWLDNSLK | 21,79 | 676,35 | 875,46 | 35 |
| 534 | IDTFWLDNSLK | 21,79 | 676,35 | 1123,58 | 35 |
| 535 | IDYYNLDR | 14,85 | 536,26 | 680,34 | 29 |
| 536 | IDYYNLDR | 14,85 | 536,26 | 843,4 | 29 |
| 537 | IDYYNLDR | 14,85 | 536,26 | 958,43 | 29 |
| 538 | IFNALIALDSGVIK | 24,74 | 737,44 | 802,47 | 37 |
| 539 | IFNALIALDSGVIK | 24,74 | 737,44 | 915,55 | 37 |
| 540 | IFNALIALDSGVIK | 24,74 | 737,44 | 1028,64 | 37 |
| 541 | IFNSLLALDSGALDNER | 22,76 | 924,48 | 976,43 | 46 |
| 542 | IFNSLLALDSGALDNER | 22,77 | 924,48 | 1089,52 | 46 |
| 543 | IFNSLLALDSGALDNER | 22,76 | 924,48 | 1160,55 | 46 |
| 544 | IFNTLIGLENGIVK | 23,29 | 765,95 | 829,48 | 39 |
| 545 | IFNTLIGLENGIVK | 23,3 | 765,95 | 942,56 | 39 |
| 546 | IFNTLIGLENGIVK | 23,3 | 765,95 | 1055,65 | 39 |
| 547 | IGLDLMQK | 17,7 | 459,26 | 634,32 | 25 |
| 548 | IGLDLMQK | 17,7 | 459,26 | 747,41 | 25 |
| 549 | IGLDLMQK | 17,7 | 459,26 | 804,43 | 25 |
| 550 | IGLEK | 8,54 | 280,18 | 389,24 | 17 |
| 551 | IGLEK | 8,55 | 280,18 | 413,24 | 17 |
| 552 | IGLEK | 8,54 | 280,18 | 446,26 | 17 |
| 553 | IGLELMQQEVQR | 18,73 | 722,38 | 787,41 | 37 |
| 554 | IGLELMQQEVQR | 18,73 | 722,38 | 918,45 | 37 |
| 555 | IGLELMQQEVQR | 18,73 | 722,38 | 1031,53 | 37 |
| 556 | IGLELMSK | 17,52 | 445,75 | 478,27 | 25 |
| 557 | IGLELMSK | 17,52 | 445,75 | 720,4 | 25 |
| 558 | IGLELMSK | 17,52 | 445,75 | 777,42 | 25 |
| 559 | IGLELMSNEVK | 18,73 | 616,83 | 707,34 | 32 |
| 560 | IGLELMSNEVK | 18,73 | 616,83 | 820,42 | 32 |
| 561 | IGLELMSNEVK | 18,73 | 616,83 | 949,47 | 32 |
| 562 | IGLER | 10,96 | 294,18 | 304,16 | 18 |
| 563 | IGLER | 10,96 | 294,18 | 417,25 | 18 |
| 564 | IGLER | 10,96 | 294,18 | 474,27 | 18 |
| 565 | IGLNK | 9,59 | 272,68 | 374,24 | 17 |
| 566 | IGLNK | 9,59 | 272,68 | 398,24 | 17 |
| 567 | IGLNK | 9,59 | 272,68 | 431,26 | 17 |
| 568 | IGLNLMQK | 17,1 | 458,77 | 633,34 | 25 |
| 569 | IGLNLMQK | 17,09 | 458,77 | 746,42 | 25 |
| 570 | IGLNLMQK | 17,11 | 458,77 | 803,44 | 25 |
| 571 | IGPSLMQSELQR | 17,02 | 679,86 | 760,39 | 35 |
| 572 | IGPSLMQSELQR | 17,02 | 679,86 | 891,44 | 35 |
| 573 | IGPSLMQSELQR | 17,02 | 679,86 | 1188,6 | 35 |
| 574 | IGYGNMQIGTEVDQFWLK | 24,31 | 700,35 | 935,5 | 39 |
| 575 | IGYGNMQIGTEVDQFWLK | 24,32 | 1050,02 | 1164,54 | 51 |
| 576 | IGYGNMQIGTEVDQFWLK | 24,3 | 1050,02 | 1222,61 | 51 |
| 577 | IINHNLPVK | 11,88 | 349,88 | 456,32 | 21 |
| 578 | IINHNLPVK | 11,88 | 349,88 | 570,36 | 21 |
| 579 | IINHNLPVK | 11,88 | 349,88 | 592,32 | 21 |
| 580 | IINHNLPVR | 12,04 | 359,22 | 598,37 | 22 |
| 581 | IINHNLPVR | 12,04 | 538,32 | 598,37 | 29 |
| 582 | IINHNLPVR | 12,04 | 538,32 | 849,47 | 29 |
| 583 | ILFQQGTQQAC[CAM]AER | 14,51 | 550,61 | 606,27 | 32 |
| 584 | ILFQQGTQQAC[CAM]AER | 14,51 | 825,41 | 1020,45 | 41 |
| 585 | ILFQQGTQQAC[CAM]AER | 14,51 | 825,41 | 1148,51 | 41 |
| 586 | ILNNWFK | 18,98 | 467,76 | 594,3 | 26 |
| 587 | ILNNWFK | 18,98 | 467,76 | 708,35 | 26 |
| 588 | ILNNWFK | 18,97 | 467,76 | 821,43 | 26 |
| 589 | ILNTLISLEEK | 19,98 | 636,87 | 718,4 | 33 |
| 590 | ILNTLISLEEK | 19,98 | 636,87 | 1046,57 | 33 |
| 591 | ILNTLISLEEK | 19,98 | 636,87 | 1159,66 | 33 |
| 592 | INIVK | 11,43 | 293,7 | 359,27 | 18 |
| 593 | INIVK | 11,43 | 293,7 | 440,29 | 18 |
| 594 | INIVK | 11,43 | 293,7 | 473,31 | 18 |
| 595 | INLYGNALSR | 16,05 | 560,81 | 617,34 | 30 |
| 596 | INLYGNALSR | 16,05 | 560,81 | 780,4 | 30 |
| 597 | INLYGNALSR | 16,05 | 560,81 | 893,48 | 30 |
| 598 | IPFSLNLEMK | 21,68 | 596,33 | 834,44 | 31 |
| 599 | IPFSLNLEMK | 21,67 | 596,33 | 981,51 | 31 |
| 600 | IPFSLNLEMK | 21,67 | 596,33 | 1078,56 | 31 |
| 601 | IPHTLFALDADAVR | 20 | 513,62 | 531,29 | 30 |
| 602 | IPHTLFALDADAVR | 20 | 513,62 | 646,32 | 30 |
| 603 | IPHTLFALDADAVR | 20 | 769,92 | 1191,64 | 39 |
| 604 | IPHTLFALDAGAAR | 18,58 | 726,9 | 744,4 | 37 |
| 605 | IPHTLFALDAGAAR | 18,58 | 726,9 | 891,47 | 37 |
| 606 | IPHTLFALDAGAAR | 18,58 | 726,9 | 1004,55 | 37 |
| 607 | IPHTLFALDAGAVR | 19,72 | 494,28 | 588,31 | 29 |
| 608 | IPHTLFALDAGAVR | 19,71 | 494,28 | 780,44 | 29 |
| 609 | IPHTLFALDAGAVR | 19,72 | 740,92 | 1133,63 | 38 |
| 610 | IPNAIIGLETGVIK | 21,75 | 719,44 | 816,48 | 37 |
| 611 | IPNAIIGLETGVIK | 21,75 | 719,44 | 929,57 | 37 |
| 612 | IPNAIIGLETGVIK | 21,75 | 719,44 | 1227,73 | 37 |
| 613 | IPNALIGLETGAIK | 20,96 | 705,42 | 788,45 | 36 |
| 614 | IPNALIGLETGAIK | 20,96 | 705,42 | 901,54 | 36 |
| 615 | IPNALIGLETGAIK | 20,96 | 705,42 | 1014,62 | 36 |
| 616 | IPNSLIAFDTGAVR | 20,24 | 737,41 | 765,39 | 37 |
| 617 | IPNSLIAFDTGAVR | 20,24 | 737,41 | 836,43 | 37 |
| 618 | IPNSLIAFDTGAVR | 20,24 | 737,41 | 949,51 | 37 |
| 619 | IPSAIIGLETGVIK | 21,66 | 705,93 | 816,48 | 36 |
| 620 | IPSAIIGLETGVIK | 21,67 | 705,93 | 929,57 | 36 |
| 621 | IPSAIIGLETGVIK | 21,66 | 705,93 | 1200,72 | 36 |
| 622 | ISAFNQVK | 13,02 | 453,76 | 488,28 | 25 |
| 623 | ISAFNQVK | 13,02 | 453,76 | 706,39 | 25 |
| 624 | ISAFNQVK | 13,02 | 453,76 | 793,42 | 25 |
| 625 | ISAHEQILFLR | 18,28 | 442,92 | 548,36 | 26 |
| 626 | ISAHEQILFLR | 18,28 | 442,92 | 789,5 | 26 |
| 627 | ISAHEQILFLR | 18,28 | 663,88 | 918,54 | 34 |
| 628 | ISAMEQTR | 9,84 | 468,23 | 664,31 | 26 |
| 629 | ISAMEQTR | 9,84 | 468,23 | 735,35 | 26 |
| 630 | ISAMEQTR | 9,84 | 468,23 | 822,38 | 26 |
| 631 | ISAMEQVK | 11,65 | 453,24 | 634,32 | 25 |
| 632 | ISAMEQVK | 11,65 | 453,24 | 705,36 | 25 |
| 633 | ISAMEQVK | 11,65 | 453,24 | 792,39 | 25 |
| 634 | ISATEQVAFLR | 17,7 | 412,23 | 435,27 | 25 |
| 635 | ISATEQVAFLR | 17,71 | 412,23 | 506,31 | 25 |
| 636 | ISATEQVAFLR | 17,7 | 412,23 | 605,38 | 25 |
| 637 | ISATQQIAFLR | 18,58 | 624,36 | 747,45 | 32 |
| 638 | ISATQQIAFLR | 18,58 | 624,36 | 1047,59 | 32 |
| 639 | ISATQQIAFLR | 18,58 | 624,36 | 1134,63 | 32 |
| 640 | ISAVNQVEFLESLFLNK | 28,77 | 976,03 | 988,51 | 48 |
| 641 | ISAVNQVEFLESLFLNK | 28,77 | 976,03 | 1110,62 | 48 |
| 642 | ISAVNQVEFLESLFLNK | 28,77 | 976,03 | 1239,66 | 48 |
| 643 | ISAVNQVK | 10,32 | 429,76 | 488,28 | 24 |
| 644 | ISAVNQVK | 10,32 | 429,76 | 658,39 | 24 |
| 645 | ISAVNQVK | 10,32 | 429,76 | 745,42 | 24 |
| 646 | ISPEEQIQFLR | 18,87 | 680,37 | 933,52 | 35 |
| 647 | ISPEEQIQFLR | 18,87 | 680,37 | 1062,56 | 35 |
| 648 | ISPEEQIQFLR | 18,87 | 680,37 | 1159,61 | 35 |
| 649 | ISPEEQVR | 10,49 | 479,25 | 531,29 | 26 |
| 650 | ISPEEQVR | 10,49 | 479,25 | 660,33 | 26 |
| 651 | ISPEEQVR | 10,49 | 479,25 | 757,38 | 26 |
| 652 | ISPEGQVR | 9,86 | 443,24 | 459,27 | 25 |
| 653 | ISPEGQVR | 9,86 | 443,24 | 588,31 | 25 |
| 654 | ISPEGQVR | 9,86 | 443,24 | 685,36 | 25 |
| 655 | ISPLEQLAFLR | 24,02 | 643,88 | 876,49 | 33 |
| 656 | ISPLEQLAFLR | 24,01 | 643,88 | 989,58 | 33 |
| 657 | ISPLEQLAFLR | 24,02 | 643,88 | 1086,63 | 33 |
| 658 | ITAFQQVDFLR | 21,11 | 669,36 | 777,43 | 34 |
| 659 | ITAFQQVDFLR | 21,12 | 669,36 | 905,48 | 34 |
| 660 | ITAFQQVDFLR | 21,12 | 669,36 | 1123,59 | 34 |
| 661 | ITPIQEVNFADDFANNR | 21,25 | 655,32 | 736,34 | 37 |
| 662 | ITPIQEVNFADDFANNR | 21,25 | 655,32 | 851,36 | 37 |
| 663 | ITPIQEVNFADDFANNR | 21,25 | 655,32 | 922,4 | 37 |
| 664 | ITPIQEVNFADDLANNR | 20,95 | 643,99 | 817,38 | 36 |
| 665 | ITPIQEVNFADDLANNR | 20,95 | 965,49 | 1149,53 | 47 |
| 666 | ITPIQEVNFADDLANNR | 20,96 | 965,49 | 1248,6 | 47 |
| 667 | ITPQQEAQFAYK | 14,52 | 712,36 | 856,42 | 36 |
| 668 | ITPQQEAQFAYK | 14,52 | 712,36 | 984,48 | 36 |
| 669 | ITPQQEAQFAYK | 14,52 | 712,36 | 1209,59 | 36 |
| 670 | ITPQQEAQFTYK | 14,33 | 485,25 | 558,29 | 28 |
| 671 | ITPQQEAQFTYK | 14,33 | 727,37 | 1014,49 | 37 |
| 672 | ITPQQEAQFTYK | 14,33 | 727,37 | 1239,6 | 37 |
| 673 | ITPVQEVNFADDLAHNR | 18,98 | 646,99 | 840,4 | 36 |
| 674 | ITPVQEVNFADDLAHNR | 18,98 | 646,99 | 862,92 | 36 |
| 675 | ITPVQEVNFADDLAHNR | 18,98 | 646,99 | 911,43 | 36 |
| 676 | IVAFALK | 17,21 | 381,25 | 478,3 | 22 |
| 677 | IVAFALK | 17,22 | 381,25 | 549,34 | 22 |
| 678 | IVAFALK | 17,21 | 381,25 | 648,41 | 22 |
| 679 | IVAFALNMEMR | 17,95 | 647,84 | 864,41 | 34 |
| 680 | IVAFALNMEMR | 17,95 | 647,84 | 1011,48 | 34 |
| 681 | IVAFALNMEMR | 17,97 | 647,84 | 1082,51 | 34 |
| 682 | IVESTTLADGTVVHGK | 13,69 | 542,96 | 697,4 | 31 |
| 683 | IVESTTLADGTVVHGK | 13,69 | 542,96 | 812,43 | 31 |
| 684 | IVESTTLADGTVVHGK | 13,68 | 542,96 | 883,46 | 31 |
| 685 | IYNSLIGLNEK | 17,37 | 632,35 | 673,39 | 33 |
| 686 | IYNSLIGLNEK | 17,37 | 632,35 | 786,47 | 33 |
| 687 | IYNSLIGLNEK | 17,37 | 632,35 | 987,55 | 33 |
| 688 | KPDIGWWVGWIER | 24,47 | 547,96 | 660,35 | 31 |
| 689 | KPDIGWWVGWIER | 24,47 | 547,96 | 883,45 | 31 |
| 690 | KPDIGWWVGWIER | 24,46 | 821,43 | 1188,59 | 41 |
| 691 | LAC[CAM]ATNNLAR | 11,22 | 552,28 | 688,37 | 29 |
| 692 | LAC[CAM]ATNNLAR | 11,22 | 552,28 | 759,41 | 29 |
| 693 | LAC[CAM]ATNNLAR | 11,22 | 552,28 | 919,44 | 29 |
| 694 | LAQGELPFPAPVQSTVR | 19,84 | 905,5 | 954,54 | 45 |
| 695 | LAQGELPFPAPVQSTVR | 19,84 | 905,5 | 1101,61 | 45 |
| 696 | LAQGELPFPAPVQSTVR | 19,84 | 905,5 | 1198,66 | 45 |
| 697 | LAQNELPYPIEIQK | 19,09 | 828,45 | 929,47 | 41 |
| 698 | LAQNELPYPIEIQK | 19,09 | 828,45 | 987,55 | 41 |
| 699 | LAQNELPYPIEIQK | 19,08 | 828,45 | 1100,64 | 41 |
| 700 | LAQNELQYPIEIQK | 17,98 | 843,96 | 890,5 | 42 |
| 701 | LAQNELQYPIEIQK | 17,98 | 843,96 | 1018,56 | 42 |
| 702 | LAQNELQYPIEIQK | 17,98 | 843,96 | 1131,64 | 42 |
| 703 | LDFGNK | 11,75 | 347,18 | 465,25 | 20 |
| 704 | LDFGNK | 11,74 | 347,18 | 547,25 | 20 |
| 705 | LDFGNK | 11,75 | 347,18 | 580,27 | 20 |
| 706 | LDGSLNR | 9,48 | 387,71 | 402,25 | 22 |
| 707 | LDGSLNR | 9,48 | 387,71 | 546,3 | 22 |
| 708 | LDGSLNR | 9,48 | 387,71 | 661,33 | 22 |
| 709 | LEILQQALAELGLYPK | 29,81 | 900,02 | 1003,58 | 45 |
| 710 | LEILQQALAELGLYPK | 29,81 | 900,02 | 1074,62 | 45 |
| 711 | LEILQQALAELGLYPK | 29,81 | 900,02 | 1202,68 | 45 |
| 712 | LENQEQVK | 7,6 | 494,26 | 631,34 | 27 |
| 713 | LENQEQVK | 7,59 | 494,26 | 745,38 | 27 |
| 714 | LENQEQVK | 7,59 | 494,26 | 874,43 | 27 |
| 715 | LETQEEVEK | 9,88 | 552,77 | 633,31 | 29 |
| 716 | LETQEEVEK | 9,88 | 552,77 | 862,42 | 29 |
| 717 | LETQEEVEK | 9,88 | 552,77 | 991,46 | 29 |
| 718 | LETQEEVK | 9,5 | 488,25 | 504,27 | 26 |
| 719 | LETQEEVK | 9,49 | 488,25 | 733,37 | 26 |
| 720 | LETQEEVK | 9,49 | 488,25 | 862,42 | 26 |
| 721 | LFAAEGVK | 13,53 | 417,74 | 503,28 | 23 |
| 722 | LFAAEGVK | 13,53 | 417,74 | 574,32 | 23 |
| 723 | LFAAEGVK | 13,53 | 417,74 | 721,39 | 23 |
| 724 | LFESAGVK | 12,99 | 425,74 | 461,27 | 24 |
| 725 | LFESAGVK | 12,99 | 425,74 | 590,31 | 24 |
| 726 | LFESAGVK | 12,99 | 425,74 | 737,38 | 24 |
| 727 | LFGAAGVK | 13,94 | 381,73 | 445,28 | 22 |
| 728 | LFGAAGVK | 13,94 | 381,73 | 502,3 | 22 |
| 729 | LFGAAGVK | 13,94 | 381,73 | 649,37 | 22 |
| 730 | LGVDR | 8,51 | 280,16 | 290,15 | 17 |
| 731 | LGVDR | 8,51 | 280,16 | 389,21 | 17 |
| 732 | LGVDR | 8,5 | 280,16 | 446,24 | 17 |
| 733 | LLNLLSQSK | 17,97 | 508,31 | 562,32 | 27 |
| 734 | LLNLLSQSK | 17,97 | 508,31 | 789,45 | 27 |
| 735 | LLNLLSQSK | 17,97 | 508,31 | 902,53 | 27 |
| 736 | LLQDER | 9,34 | 387,21 | 547,25 | 22 |
| 737 | LLQDER | 9,31 | 387,21 | 599,3 | 22 |
| 738 | LLQDER | 9,34 | 387,21 | 660,33 | 22 |
| 739 | LLVQDGDC[CAM]GR | 11,92 | 566,77 | 679,25 | 30 |
| 740 | LLVQDGDC[CAM]GR | 11,92 | 566,77 | 807,3 | 30 |
| 741 | LLVQDGDC[CAM]GR | 11,92 | 566,77 | 906,37 | 30 |
| 742 | LNEVGYGNR | 10,74 | 511,26 | 566,27 | 27 |
| 743 | LNEVGYGNR | 10,74 | 511,26 | 665,34 | 27 |
| 744 | LNEVGYGNR | 10,73 | 511,26 | 794,38 | 27 |
| 745 | LNYGNADPSTK | 10,76 | 590,29 | 732,35 | 31 |
| 746 | LNYGNADPSTK | 10,76 | 590,29 | 789,37 | 31 |
| 747 | LNYGNADPSTK | 10,76 | 590,29 | 952,44 | 31 |
| 748 | LNYGNK | 7,21 | 354,69 | 481,24 | 21 |
| 749 | LNYGNK | 7,24 | 354,69 | 562,26 | 21 |
| 750 | LNYGNK | 7,22 | 354,69 | 595,28 | 21 |
| 751 | LPASK | 1,93 | 258,16 | 305,18 | 16 |
| 752 | LPASK | 1,93 | 258,16 | 369,21 | 16 |
| 753 | LPASK | 1,93 | 258,16 | 402,23 | 16 |
| 754 | LPHTLFALDADAVR | 19,98 | 769,92 | 977,51 | 39 |
| 755 | LPHTLFALDADAVR | 19,98 | 769,92 | 1090,59 | 39 |
| 756 | LPHTLFALDADAVR | 19,98 | 769,92 | 1191,64 | 39 |
| 757 | LPHTLFALDAGAVR | 19,7 | 740,92 | 919,5 | 38 |
| 758 | LPHTLFALDAGAVR | 19,67 | 740,92 | 1032,58 | 38 |
| 759 | LPHTLFALDAGAVR | 19,7 | 740,92 | 1133,63 | 38 |
| 760 | LPLAIMGFDSGILQSPK | 25,08 | 893,99 | 944,5 | 44 |
| 761 | LPLAIMGFDSGILQSPK | 25,08 | 893,99 | 1091,57 | 44 |
| 762 | LPLAIMGFDSGILQSPK | 25,08 | 893,99 | 1148,59 | 44 |
| 763 | LPLAIMGYDADILLDATTPR | 27,86 | 720,39 | 773,42 | 40 |
| 764 | LPLAIMGYDADILLDATTPR | 27,87 | 720,39 | 886,5 | 40 |
| 765 | LPLAIMGYDADILLDATTPR | 27,87 | 720,39 | 1160,57 | 40 |
| 766 | LPSSLIALETGAVR | 20,6 | 713,92 | 816,46 | 36 |
| 767 | LPSSLIALETGAVR | 20,6 | 713,92 | 929,54 | 36 |
| 768 | LPSSLIALETGAVR | 20,6 | 713,92 | 1216,69 | 36 |
| 769 | LPVSAQTLQYTANILK | 21,84 | 880,5 | 950,53 | 44 |
| 770 | LPVSAQTLQYTANILK | 21,84 | 880,5 | 1063,61 | 44 |
| 771 | LPVSAQTLQYTANILK | 21,85 | 880,5 | 1164,66 | 44 |
| 772 | LPVSER | 9,57 | 350,7 | 490,26 | 20 |
| 773 | LPVSER | 9,57 | 350,7 | 526,29 | 20 |
| 774 | LPVSER | 9,57 | 350,7 | 587,31 | 20 |
| 775 | LPVSPTAVDMTER | 16,21 | 708,36 | 1019,48 | 36 |
| 776 | LPVSPTAVDMTER | 16,21 | 708,36 | 1106,51 | 36 |
| 777 | LPVSPTAVDMTER | 16,21 | 708,36 | 1205,58 | 36 |
| 778 | LSASK | 10,72 | 253,15 | 305,18 | 16 |
| 779 | LSASK | 10,71 | 253,15 | 359,19 | 16 |
| 780 | LSASK | 10,71 | 253,15 | 392,21 | 16 |
| 781 | LSAVPIYQEVAR | 17,96 | 673,38 | 765,39 | 35 |
| 782 | LSAVPIYQEVAR | 17,96 | 673,38 | 975,53 | 35 |
| 783 | LSAVPIYQEVAR | 17,95 | 673,38 | 1074,59 | 35 |
| 784 | LSAVPVYQELAR | 18,45 | 449,25 | 616,34 | 26 |
| 785 | LSAVPVYQELAR | 18,44 | 673,38 | 779,4 | 35 |
| 786 | LSAVPVYQELAR | 18,44 | 673,38 | 975,53 | 35 |
| 787 | LSC[CAM]TLVIDEASGDLLHR | 20,38 | 633,66 | 797,43 | 36 |
| 788 | LSC[CAM]TLVIDEASGDLLHR | 20,38 | 633,66 | 868,46 | 36 |
| 789 | LSC[CAM]TLVIDEASGDLLHR | 20,38 | 633,66 | 1112,53 | 36 |
| 790 | LSLQHGWFIGWIEK | 23,95 | 571,98 | 632,34 | 33 |
| 791 | LSLQHGWFIGWIEK | 23,95 | 571,98 | 892,49 | 33 |
| 792 | LSLQHGWFIGWIEK | 23,95 | 571,98 | 969,49 | 33 |
| 793 | LSQNSLPFSQEAMNSVK | 18,64 | 627,31 | 1140,54 | 35 |
| 794 | LSQNSLPFSQEAMNSVK | 18,63 | 940,46 | 1140,54 | 46 |
| 795 | LSQNSLPFSQEAMNSVK | 18,64 | 940,46 | 1237,59 | 46 |
| 796 | LSVNPK | 9,8 | 329,2 | 457,28 | 19 |
| 797 | LSVNPK | 9,79 | 329,2 | 511,29 | 19 |
| 798 | LSVNPK | 9,8 | 329,2 | 544,31 | 19 |
| 799 | LTVGAR | 9,51 | 308,69 | 402,25 | 19 |
| 800 | LTVGAR | 9,51 | 308,69 | 442,27 | 19 |
| 801 | LTVGAR | 9,51 | 308,69 | 503,29 | 19 |
| 802 | LYGFALNIDMPGGEADIGK | 23,35 | 661 | 843,42 | 37 |
| 803 | LYGFALNIDMPGGEADIGK | 23,35 | 990,99 | 1089,49 | 49 |
| 804 | LYGFALNIDMPGGEADIGK | 23,35 | 990,99 | 1202,57 | 49 |
| 805 | LYHNELPFR | 15,29 | 396,88 | 414,21 | 24 |
| 806 | LYHNELPFR | 15,29 | 396,88 | 419,24 | 24 |
| 807 | LYHNELPFR | 15,29 | 396,88 | 657,3 | 24 |
| 808 | LYHNK | 8,54 | 337,68 | 414,21 | 20 |
| 809 | LYHNK | 8,53 | 337,68 | 528,26 | 20 |
| 810 | LYHNK | 8,53 | 337,68 | 561,28 | 20 |
| 811 | LYQNDLPFR | 17,2 | 583,3 | 761,39 | 31 |
| 812 | LYQNDLPFR | 17,2 | 583,3 | 889,45 | 31 |
| 813 | LYQNDLPFR | 17,2 | 583,3 | 1052,52 | 31 |
| 814 | MDDLFK | 15,5 | 384,68 | 522,29 | 22 |
| 815 | MDDLFK | 15,5 | 384,68 | 622,25 | 22 |
| 816 | MDDLFK | 15,5 | 384,68 | 637,32 | 22 |
| 817 | MEDLHK | 6,66 | 386,69 | 512,28 | 22 |
| 818 | MEDLHK | 6,65 | 386,69 | 626,26 | 22 |
| 819 | MEDLHK | 6,66 | 386,69 | 641,33 | 22 |
| 820 | MLIALIGLENHK | 21,33 | 451,26 | 527,26 | 27 |
| 821 | MLIALIGLENHK | 21,33 | 451,26 | 697,36 | 27 |
| 822 | MLIALIGLENHK | 21,33 | 451,26 | 810,45 | 27 |
| 823 | MLLIK | 15,81 | 309,21 | 373,28 | 19 |
| 824 | MLLIK | 15,81 | 309,21 | 471,3 | 19 |
| 825 | MLLIK | 15,81 | 309,21 | 486,36 | 19 |
| 826 | MLNALIGLEHHK | 16,89 | 459,26 | 550,27 | 27 |
| 827 | MLNALIGLEHHK | 16,89 | 459,26 | 720,38 | 27 |
| 828 | MLNALIGLEHHK | 16,89 | 459,26 | 833,46 | 27 |
| 829 | MLNALIGLENHK | 18,39 | 451,58 | 697,36 | 27 |
| 830 | MLNALIGLENHK | 18,38 | 676,87 | 697,36 | 35 |
| 831 | MLNALIGLENHK | 18,39 | 676,87 | 810,45 | 35 |
| 832 | MLNALIGLENQK | 19,71 | 672,37 | 688,36 | 35 |
| 833 | MLNALIGLENQK | 19,71 | 672,37 | 801,45 | 35 |
| 834 | MLNALIGLENQK | 19,71 | 672,37 | 914,53 | 35 |
| 835 | MLNALIGLEYHK | 19,6 | 701,38 | 746,38 | 36 |
| 836 | MLNALIGLEYHK | 19,6 | 701,38 | 859,47 | 36 |
| 837 | MLNALIGLEYHK | 19,6 | 701,38 | 1157,63 | 36 |
| 838 | MLNALIGLQHGK | 17,5 | 432,25 | 582,34 | 26 |
| 839 | MLNALIGLQHGK | 17,5 | 432,25 | 639,36 | 26 |
| 840 | MLNALIGLQHGK | 17,5 | 432,25 | 752,44 | 26 |
| 841 | MLNALISLEHHK | 17,2 | 352,2 | 359,17 | 21 |
| 842 | MLNALISLEHHK | 17,21 | 469,26 | 750,39 | 27 |
| 843 | MLNALISLEHHK | 17,2 | 469,26 | 863,47 | 27 |
| 844 | MQAYVDAFDYGNR | 17,56 | 775,34 | 957,41 | 39 |
| 845 | MQAYVDAFDYGNR | 17,56 | 775,34 | 1056,47 | 39 |
| 846 | MQAYVDAFDYGNR | 17,56 | 775,34 | 1219,54 | 39 |
| 847 | MQEGLNK | 8,68 | 410,21 | 560,3 | 23 |
| 848 | MQEGLNK | 8,66 | 410,21 | 673,3 | 23 |
| 849 | MQEGLNK | 8,68 | 410,21 | 688,36 | 23 |
| 850 | MSPASTYK | 9,49 | 442,71 | 569,29 | 24 |
| 851 | MSPASTYK | 9,49 | 442,71 | 666,35 | 24 |
| 852 | MSPASTYK | 9,49 | 442,71 | 753,38 | 24 |
| 853 | NEHDPVLPYR | 13,09 | 413,88 | 435,24 | 25 |
| 854 | NEHDPVLPYR | 13,09 | 620,31 | 744,44 | 32 |
| 855 | NEHDPVLPYR | 13,09 | 620,31 | 859,47 | 32 |
| 856 | NEHQIFK | 9,91 | 458,24 | 509,21 | 25 |
| 857 | NEHQIFK | 9,91 | 458,24 | 622,29 | 25 |
| 858 | NEHQIFK | 9,91 | 458,24 | 672,38 | 25 |
| 859 | NEHQVFK | 7,74 | 451,23 | 658,37 | 25 |
| 860 | NEHQVFK | 7,74 | 451,23 | 755,35 | 25 |
| 861 | NEHQVFK | 7,74 | 451,23 | 787,41 | 25 |
| 862 | NEITYK | 9,35 | 384,2 | 524,31 | 22 |
| 863 | NEITYK | 9,35 | 384,2 | 621,29 | 22 |
| 864 | NEITYK | 9,35 | 384,2 | 653,35 | 22 |
| 865 | NELLMK | 13,08 | 374,21 | 504,32 | 21 |
| 866 | NELLMK | 13,09 | 374,21 | 601,3 | 21 |
| 867 | NELLMK | 13,09 | 374,21 | 633,36 | 21 |
| 868 | NELPFR | 14,39 | 388,21 | 419,24 | 22 |
| 869 | NELPFR | 14,39 | 388,21 | 532,32 | 22 |
| 870 | NELPFR | 14,4 | 388,21 | 661,37 | 22 |
| 871 | NISSYGNNLVR | 14,36 | 618,82 | 835,44 | 32 |
| 872 | NISSYGNNLVR | 14,36 | 618,82 | 922,47 | 32 |
| 873 | NISSYGNNLVR | 14,36 | 618,82 | 1009,51 | 32 |
| 874 | NISTYGNNLTR | 13,1 | 626,82 | 674,36 | 33 |
| 875 | NISTYGNNLTR | 13,09 | 626,82 | 837,42 | 33 |
| 876 | NISTYGNNLTR | 13,1 | 626,82 | 1025,5 | 33 |
| 877 | NLFNEVHTTGVLVIR | 20,69 | 571,32 | 757,49 | 33 |
| 878 | NLFNEVHTTGVLVIR | 20,7 | 571,32 | 858,54 | 33 |
| 879 | NLFNEVHTTGVLVIR | 20,7 | 571,32 | 995,6 | 33 |
| 880 | NLSTYGNALAR | 14,34 | 590,31 | 764,4 | 31 |
| 881 | NLSTYGNALAR | 14,35 | 590,31 | 865,45 | 31 |
| 882 | NLSTYGNALAR | 14,35 | 590,31 | 952,48 | 31 |
| 883 | NMENLELFGK | 19,08 | 597,79 | 820,46 | 31 |
| 884 | NMENLELFGK | 19,08 | 597,79 | 949,5 | 31 |
| 885 | NMENLELFGK | 19,08 | 597,79 | 1080,54 | 31 |
| 886 | NMLLLEENNGYK | 16,71 | 719,36 | 853,37 | 37 |
| 887 | NMLLLEENNGYK | 16,69 | 719,36 | 966,45 | 37 |
| 888 | NMLLLEENNGYK | 16,68 | 719,36 | 1079,54 | 37 |
| 889 | NMLLLEESNGYK | 18,12 | 705,85 | 939,44 | 36 |
| 890 | NMLLLEESNGYK | 18,13 | 705,85 | 1052,53 | 36 |
| 891 | NMLLLEESNGYK | 18,11 | 705,85 | 1165,61 | 36 |
| 892 | NMLLLEK | 16,99 | 430,75 | 502,32 | 24 |
| 893 | NMLLLEK | 16,98 | 430,75 | 615,41 | 24 |
| 894 | NMLLLEK | 16,98 | 430,75 | 746,45 | 24 |
| 895 | NMTLGDAMK | 14,42 | 490,73 | 521,24 | 27 |
| 896 | NMTLGDAMK | 14,42 | 490,73 | 634,32 | 27 |
| 897 | NMTLGDAMK | 14,42 | 490,73 | 735,37 | 27 |
| 898 | NNGLTEAWLESSLK | 20,61 | 781,4 | 862,47 | 39 |
| 899 | NNGLTEAWLESSLK | 20,6 | 781,4 | 933,5 | 39 |
| 900 | NNGLTEAWLESSLK | 20,62 | 781,4 | 1163,59 | 39 |
| 901 | NQLPFK | 13,49 | 373,71 | 391,23 | 21 |
| 902 | NQLPFK | 13,49 | 373,71 | 504,32 | 21 |
| 903 | NQLPFK | 13,49 | 373,71 | 632,38 | 21 |
| 904 | NQLPFQVEHQR | 14,33 | 698,36 | 796,41 | 36 |
| 905 | NQLPFQVEHQR | 14,33 | 698,36 | 1040,53 | 36 |
| 906 | NQLPFQVEHQR | 14,33 | 698,36 | 1153,61 | 36 |
| 907 | NSAIENTIDNMYLQDLENSTK | 22,77 | 805,04 | 934,45 | 44 |
| 908 | NSAIENTIDNMYLQDLENSTK | 22,77 | 805,04 | 1047,53 | 44 |
| 909 | NSAIENTIDNMYLQDLENSTK | 22,77 | 805,04 | 1210,6 | 44 |
| 910 | NSAIENTIENMYLQDLDNSTK | 23,13 | 805,04 | 920,43 | 44 |
| 911 | NSAIENTIENMYLQDLDNSTK | 23,13 | 805,04 | 1033,52 | 44 |
| 912 | NSAIENTIENMYLQDLDNSTK | 23,14 | 805,04 | 1196,58 | 44 |
| 913 | NSAIENTIENMYLQDLENSTK | 23,7 | 809,72 | 934,45 | 44 |
| 914 | NSAIENTIENMYLQDLENSTK | 23,7 | 809,72 | 1047,53 | 44 |
| 915 | NSAIENTIENMYLQDLENSTK | 23,7 | 809,72 | 1217,55 | 44 |
| 916 | NSAVWVYELFAK | 24,66 | 713,87 | 869,48 | 36 |
| 917 | NSAVWVYELFAK | 24,66 | 713,87 | 1055,56 | 36 |
| 918 | NSAVWVYELFAK | 24,65 | 713,87 | 1154,62 | 36 |
| 919 | NSQVPAYK | 9,78 | 453,74 | 478,27 | 25 |
| 920 | NSQVPAYK | 9,78 | 453,74 | 577,33 | 25 |
| 921 | NSQVPAYK | 9,78 | 453,74 | 705,39 | 25 |
| 922 | NSTVWIYELFAK | 25,64 | 735,88 | 883,49 | 37 |
| 923 | NSTVWIYELFAK | 25,64 | 735,88 | 1069,57 | 37 |
| 924 | NSTVWIYELFAK | 25,64 | 735,88 | 1168,64 | 37 |
| 925 | NSTVWVYELFAK | 24,42 | 728,88 | 770,41 | 37 |
| 926 | NSTVWVYELFAK | 24,43 | 728,88 | 869,48 | 37 |
| 927 | NSTVWVYELFAK | 24,42 | 728,88 | 1055,56 | 37 |
| 928 | NSTVWVYQLFAK | 23,9 | 728,39 | 769,42 | 37 |
| 929 | NSTVWVYQLFAK | 23,91 | 728,39 | 1054,57 | 37 |
| 930 | NSTVWVYQLFAK | 23,91 | 728,39 | 1153,64 | 37 |
| 931 | NTSGALVIQTDK | 13,34 | 623,84 | 816,48 | 32 |
| 932 | NTSGALVIQTDK | 13,34 | 623,84 | 944,54 | 32 |
| 933 | NTSGALVIQTDK | 13,34 | 623,84 | 1031,57 | 32 |
| 934 | NTSGVLVIQTDK | 14,9 | 637,85 | 816,48 | 33 |
| 935 | NTSGVLVIQTDK | 14,9 | 637,85 | 972,57 | 33 |
| 936 | NTSGVLVIQTDK | 14,91 | 637,85 | 1059,6 | 33 |
| 937 | NVDEMFYYYDGSK | 18,86 | 815,84 | 895,38 | 41 |
| 938 | NVDEMFYYYDGSK | 18,86 | 815,84 | 1042,45 | 41 |
| 939 | NVDEMFYYYDGSK | 18,85 | 815,84 | 1173,49 | 41 |
| 940 | NWILR | 16,3 | 351,21 | 414,21 | 20 |
| 941 | NWILR | 16,29 | 351,21 | 527,3 | 20 |
| 942 | NWILR | 16,3 | 351,21 | 587,37 | 20 |
| 943 | NWNAAMDLR | 16,54 | 545,76 | 605,31 | 29 |
| 944 | NWNAAMDLR | 16,55 | 545,76 | 676,34 | 29 |
| 945 | NWNAAMDLR | 16,54 | 545,76 | 790,39 | 29 |
| 946 | NYVDAFHYGNQDISGDK | 15,76 | 648,29 | 933,43 | 36 |
| 947 | NYVDAFHYGNQDISGDK | 15,77 | 648,29 | 1096,49 | 36 |
| 948 | NYVDAFHYGNQDISGDK | 15,76 | 971,93 | 1233,55 | 48 |
| 949 | QADHAILVFDQAR | 16,58 | 495,26 | 523,23 | 29 |
| 950 | QADHAILVFDQAR | 16,61 | 495,26 | 636,31 | 29 |
| 951 | QADHAILVFDQAR | 16,58 | 495,26 | 735,38 | 29 |
| 952 | QAEHALLVFGQER | 16,86 | 499,93 | 636,31 | 29 |
| 953 | QAEHALLVFGQER | 16,85 | 499,93 | 735,38 | 29 |
| 954 | QAEHALLVFGQER | 16,87 | 499,93 | 763,41 | 29 |
| 955 | QAITK | 11 | 280,67 | 361,24 | 17 |
| 956 | QAITK | 11 | 280,67 | 414,23 | 17 |
| 957 | QAITK | 11,01 | 280,67 | 432,28 | 17 |
| 958 | QAMLTEANSDYIIR | 18,26 | 812,9 | 951,49 | 41 |
| 959 | QAMLTEANSDYIIR | 18,25 | 812,9 | 1080,53 | 41 |
| 960 | QAMLTEANSDYIIR | 18,26 | 812,9 | 1181,58 | 41 |
| 961 | QEVQFVSALAR | 17,69 | 624,34 | 763,45 | 32 |
| 962 | QEVQFVSALAR | 17,68 | 624,34 | 891,5 | 32 |
| 963 | QEVQFVSALAR | 17,68 | 624,34 | 990,57 | 32 |
| 964 | QFASIK | 11,66 | 347,2 | 434,2 | 20 |
| 965 | QFASIK | 11,66 | 347,2 | 547,29 | 20 |
| 966 | QFASIK | 11,68 | 347,2 | 565,33 | 20 |
| 967 | QGMPGSIR | 11,4 | 423,22 | 529,31 | 24 |
| 968 | QGMPGSIR | 11,43 | 423,22 | 660,35 | 24 |
| 969 | QGMPGSIR | 11,4 | 423,22 | 717,37 | 24 |
| 970 | QGMSGSIR | 9,44 | 418,21 | 519,29 | 23 |
| 971 | QGMSGSIR | 9,45 | 418,21 | 650,33 | 23 |
| 972 | QGMSGSIR | 9,44 | 418,21 | 707,35 | 23 |
| 973 | QGQTQQSYGNDLAR | 11,16 | 783,37 | 895,43 | 39 |
| 974 | QGQTQQSYGNDLAR | 11,17 | 783,37 | 1023,49 | 39 |
| 975 | QGQTQQSYGNDLAR | 11,16 | 783,37 | 1151,54 | 39 |
| 976 | QIDYGNADPSTIK | 13,41 | 711,35 | 845,44 | 36 |
| 977 | QIDYGNADPSTIK | 13,42 | 711,35 | 902,46 | 36 |
| 978 | QIDYGNADPSTIK | 13,42 | 711,35 | 1065,52 | 36 |
| 979 | QIDYGNVDPSTIK | 15,08 | 725,36 | 873,47 | 37 |
| 980 | QIDYGNVDPSTIK | 15,07 | 725,36 | 930,49 | 37 |
| 981 | QIDYGNVDPSTIK | 15,07 | 725,36 | 1093,55 | 37 |
| 982 | QIGQAR | 2,3 | 336,69 | 431,24 | 20 |
| 983 | QIGQAR | 2,33 | 336,69 | 498,27 | 20 |
| 984 | QIGQAR | 2,32 | 336,69 | 544,32 | 20 |
| 985 | QIMLIEQTPAFTLR | 24,42 | 830,96 | 933,52 | 42 |
| 986 | QIMLIEQTPAFTLR | 24,42 | 830,96 | 1062,56 | 42 |
| 987 | QIMLIEQTPAFTLR | 24,42 | 830,96 | 1175,64 | 42 |
| 988 | QLGSAIDQFW LR | 22,67 | 717,38 | 864,44 | 37 |
| 989 | QLGSAIDQFWLR | 22,68 | 717,38 | 977,52 | 37 |
| 990 | QLGSAIDQFWLR | 22,67 | 717,38 | 1192,61 | 37 |
| 991 | QLPVK | 9,57 | 292,69 | 343,23 | 18 |
| 992 | QLPVK | 9,58 | 292,69 | 438,27 | 18 |
| 993 | QLPVK | 9,57 | 292,69 | 456,32 | 18 |
| 994 | QLSLDVLDK | 18,63 | 515,79 | 589,32 | 28 |
| 995 | QLSLDVLDK | 18,62 | 515,79 | 789,44 | 28 |
| 996 | QLSLDVLDK | 18,63 | 515,79 | 902,52 | 28 |
| 997 | QLVYAR | 11,04 | 375,22 | 508,29 | 22 |
| 998 | QLVYAR | 11,04 | 375,22 | 575,32 | 22 |
| 999 | QLVYAR | 11,04 | 375,22 | 621,37 | 22 |
| 1000 | QMMLTEASTDYIIR | 19,82 | 836,41 | 867,46 | 42 |
| 1001 | QMMLTEASTDYIIR | 19,82 | 836,41 | 1067,54 | 42 |
| 1002 | QMMLTEASTDYIIR | 19,82 | 836,41 | 1168,58 | 42 |
| 1003 | QMSIVEATPDYVLHGK | 18,77 | 894,45 | 1029,54 | 44 |
| 1004 | QMSIVEATPDYVLHGK | 18,77 | 894,45 | 1100,57 | 44 |
| 1005 | QMSIVEATPDYVLHGK | 18,77 | 894,45 | 1229,62 | 44 |
| 1006 | QTLVFAR | 14,65 | 417,75 | 492,29 | 23 |
| 1007 | QTLVFAR | 14,65 | 417,75 | 605,38 | 23 |
| 1008 | QTLVFAR | 14,65 | 417,75 | 706,42 | 23 |
| 1009 | QVVFAR | 12,06 | 360,21 | 492,29 | 21 |
| 1010 | QVVFAR | 12,04 | 360,21 | 545,31 | 21 |
| 1011 | QVVFAR | 12,06 | 360,21 | 591,36 | 21 |
| 1012 | SADEVLPYGGKPQR | 12,96 | 506,26 | 642,37 | 29 |
| 1013 | SADEVLPYGGKPQR | 12,96 | 506,26 | 805,43 | 29 |
| 1014 | SADEVLPYGGKPQR | 12,96 | 506,26 | 902,48 | 29 |
| 1015 | SC[CAM]ATNDLAR | 9,37 | 504,23 | 689,36 | 27 |
| 1016 | SC[CAM]ATNDLAR | 9,37 | 504,23 | 760,39 | 27 |
| 1017 | SC[CAM]ATNDLAR | 9,37 | 504,23 | 920,43 | 27 |
| 1018 | SC[CAM]ATNNLAR | 8,66 | 503,74 | 688,37 | 27 |
| 1019 | SC[CAM]ATNNLAR | 8,66 | 503,74 | 759,41 | 27 |
| 1020 | SC[CAM]ATNNLAR | 8,67 | 503,74 | 919,44 | 27 |
| 1021 | SDIPGGSK | 7,63 | 380,7 | 558,32 | 22 |
| 1022 | SDIPGGSK | 7,63 | 380,7 | 614,28 | 22 |
| 1023 | SDIPGGSK | 7,63 | 380,7 | 673,35 | 22 |
| 1024 | SDWGK | 5,75 | 296,64 | 390,21 | 18 |
| 1025 | SDWGK | 5,75 | 296,64 | 446,17 | 18 |
| 1026 | SDWGK | 5,75 | 296,64 | 505,24 | 18 |
| 1027 | SEDNFHISSQQHEK | 10,36 | 422,19 | 541,27 | 24 |
| 1028 | SEDNFHISSQQHEK | 10,36 | 422,19 | 730,28 | 24 |
| 1029 | SEDNFHISSQQHEK | 10,36 | 422,19 | 756,36 | 24 |
| 1030 | SEMPASIR | 12,02 | 445,72 | 674,37 | 25 |
| 1031 | SEMPASIR | 12,02 | 445,72 | 716,33 | 25 |
| 1032 | SEMPASIR | 12,02 | 445,72 | 803,41 | 25 |
| 1033 | SEMPASTR | 8,2 | 439,71 | 662,33 | 24 |
| 1034 | SEMPASTR | 8,19 | 439,71 | 704,29 | 24 |
| 1035 | SEMPASTR | 8,19 | 439,71 | 791,37 | 24 |
| 1036 | SFAAHNQDQDLR | 10,35 | 467,89 | 531,29 | 27 |
| 1037 | SFAAHNQDQDLR | 10,35 | 467,89 | 871,37 | 27 |
| 1038 | SFAAHNQDQDLR | 10,35 | 467,89 | 888,42 | 27 |
| 1039 | SFAGHNK | 9,4 | 380,69 | 455,24 | 22 |
| 1040 | SFAGHNK | 9,4 | 380,69 | 526,27 | 22 |
| 1041 | SFAGHNK | 9,38 | 380,69 | 673,34 | 22 |
| 1042 | SFAGHNQDQDLR | 10,18 | 694,32 | 888,42 | 36 |
| 1043 | SFAGHNQDQDLR | 10,18 | 694,32 | 1025,48 | 36 |
| 1044 | SFAGHNQDQDLR | 10,18 | 694,32 | 1082,5 | 36 |
| 1045 | SFAGHNQDQNLR | 9,8 | 462,89 | 530,3 | 27 |
| 1046 | SFAGHNQDQNLR | 9,8 | 462,89 | 773,39 | 27 |
| 1047 | SFAGHNQDQNLR | 9,8 | 462,89 | 887,43 | 27 |
| 1048 | SFLESWAK | 18,27 | 484,25 | 491,26 | 26 |
| 1049 | SFLESWAK | 18,27 | 484,25 | 620,3 | 26 |
| 1050 | SFLESWAK | 18,27 | 484,25 | 733,39 | 26 |
| 1051 | SFTAWEK | 14,44 | 434,71 | 462,23 | 24 |
| 1052 | SFTAWEK | 14,44 | 434,71 | 533,27 | 24 |
| 1053 | SFTAWEK | 14,44 | 434,71 | 634,32 | 24 |
| 1054 | SFTTWEK | 14,1 | 449,72 | 462,23 | 25 |
| 1055 | SFTTWEK | 14,1 | 449,72 | 563,28 | 25 |
| 1056 | SFTTWEK | 14,1 | 449,72 | 664,33 | 25 |
| 1057 | SGSGWLR | 13,25 | 381,7 | 531,3 | 22 |
| 1058 | SGSGWLR | 13,25 | 381,7 | 618,34 | 22 |
| 1059 | SGSGWLR | 13,25 | 381,7 | 675,36 | 22 |
| 1060 | SGWGMAVDPQVGWYVGFVEK | 24,65 | 738,02 | 841,45 | 41 |
| 1061 | SGWGMAVDPQVGWYVGFVEK | 24,65 | 738,02 | 1029,45 | 41 |
| 1062 | SGWGMAVDPQVGWYVGFVEK | 24,68 | 1106,53 | 1128,51 | 54 |
| 1063 | SGWGMDVSPQVGWLTGWVEK | 26,32 | 1110,03 | 1144,51 | 54 |
| 1064 | SGWGMDVSPQVGWLTGWVEK | 26,32 | 1110,03 | 1174,63 | 54 |
| 1065 | SGWGMDVSPQVGWLTGWVEK | 26,32 | 1110,03 | 1201,53 | 54 |
| 1066 | SGWGMDVTPQVGWLTGWVEK | 26,61 | 745,03 | 832,46 | 41 |
| 1067 | SGWGMDVTPQVGWLTGWVEK | 26,61 | 745,03 | 1018,54 | 41 |
| 1068 | SGWGMDVTPQVGWLTGWVEK | 26,61 | 745,03 | 1075,56 | 41 |
| 1069 | SIHPASTFK | 10,74 | 494,27 | 650,35 | 27 |
| 1070 | SIHPASTFK | 10,73 | 494,27 | 787,41 | 27 |
| 1071 | SIHPASTFK | 10,73 | 494,27 | 900,49 | 27 |
| 1072 | SISTK | 10,41 | 268,16 | 335,19 | 17 |
| 1073 | SISTK | 10,42 | 268,16 | 389,2 | 17 |
| 1074 | SISTK | 10,42 | 268,16 | 448,28 | 17 |
| 1075 | SLGLSNNLSR | 14,23 | 530,79 | 690,35 | 28 |
| 1076 | SLGLSNNLSR | 14,23 | 530,79 | 803,44 | 28 |
| 1077 | SLGLSNNLSR | 14,23 | 530,79 | 860,46 | 28 |
| 1078 | SLSMSGK | 9,31 | 355,18 | 509,24 | 21 |
| 1079 | SLSMSGK | 9,32 | 355,18 | 563,25 | 21 |
| 1080 | SLSMSGK | 9,32 | 355,18 | 622,32 | 21 |
| 1081 | SMLFIEEK | 17,82 | 498,76 | 518,28 | 27 |
| 1082 | SMLFIEEK | 17,82 | 498,76 | 665,35 | 27 |
| 1083 | SMLFIEEK | 17,82 | 498,76 | 778,43 | 27 |
| 1084 | SNGLTHSWLGSSLK | 16,78 | 743,89 | 877,48 | 38 |
| 1085 | SNGLTHSWLGSSLK | 16,78 | 743,89 | 1014,54 | 38 |
| 1086 | SNGLTHSWLGSSLK | 16,78 | 743,89 | 1115,58 | 38 |
| 1087 | SPTWELKPEYNPSPR | 16,02 | 600,97 | 733,36 | 34 |
| 1088 | SPTWELKPEYNPSPR | 16,02 | 600,97 | 808,91 | 34 |
| 1089 | SPTWELKPEYNPSPR | 16,02 | 600,97 | 959,46 | 34 |
| 1090 | SQDIVR | 8,4 | 359,2 | 502,3 | 21 |
| 1091 | SQDIVR | 8,38 | 359,2 | 543,28 | 21 |
| 1092 | SQDIVR | 8,4 | 359,2 | 630,36 | 21 |
| 1093 | SQQKPTDPTIWLK | 16,6 | 514,62 | 660,41 | 30 |
| 1094 | SQQKPTDPTIWLK | 16,6 | 514,62 | 757,46 | 30 |
| 1095 | SQQKPTDPTIWLK | 16,6 | 514,62 | 785,38 | 30 |
| 1096 | SQVGWLTGWVEQPDGK | 22,27 | 893,94 | 1015,5 | 44 |
| 1097 | SQVGWLTGWVEQPDGK | 22,28 | 893,94 | 1116,53 | 44 |
| 1098 | SQVGWLTGWVEQPDGK | 22,28 | 893,94 | 1229,62 | 44 |
| 1099 | SSSNSC[CAM]TTNNAAR | 16,84 | 685,29 | 907,41 | 35 |
| 1100 | SSSNSC[CAM]TTNNAAR | 16,85 | 685,29 | 994,44 | 35 |
| 1101 | SSSNSC[CAM]TTNNAAR | 16,84 | 685,29 | 1108,48 | 35 |
| 1102 | SVYGELR | 12,65 | 412,22 | 417,25 | 23 |
| 1103 | SVYGELR | 12,65 | 412,22 | 474,27 | 23 |
| 1104 | SVYGELR | 12,65 | 412,22 | 637,33 | 23 |
| 1105 | SWILR | 16,33 | 337,7 | 401,29 | 20 |
| 1106 | SWILR | 16,32 | 337,7 | 500,29 | 20 |
| 1107 | SWILR | 16,33 | 337,7 | 587,37 | 20 |
| 1108 | SYLEK | 9,09 | 320,17 | 389,24 | 19 |
| 1109 | SYLEK | 9,09 | 320,17 | 493,23 | 19 |
| 1110 | SYLEK | 9,1 | 320,17 | 552,3 | 19 |
| 1111 | TAYIPASTFK | 15,43 | 549,8 | 650,35 | 29 |
| 1112 | TAYIPASTFK | 15,43 | 549,8 | 763,43 | 29 |
| 1113 | TAYIPASTFK | 15,43 | 549,8 | 926,5 | 29 |
| 1114 | TDDLFK | 13,48 | 369,69 | 407,27 | 21 |
| 1115 | TDDLFK | 13,48 | 369,69 | 522,29 | 21 |
| 1116 | TDDLFK | 13,48 | 369,69 | 637,32 | 21 |
| 1117 | TDINEIFK | 17,44 | 490,26 | 650,35 | 27 |
| 1118 | TDINEIFK | 17,44 | 490,26 | 763,43 | 27 |
| 1119 | TDINEIFK | 17,44 | 490,26 | 878,46 | 27 |
| 1120 | TFIHNDPR | 18,92 | 500,25 | 751,38 | 27 |
| 1121 | TFIHNDPR | 18,92 | 500,25 | 825,39 | 27 |
| 1122 | TFIHNDPR | 18,92 | 500,25 | 898,45 | 27 |
| 1123 | TGAGFTANR | 9,64 | 447,72 | 461,25 | 25 |
| 1124 | TGAGFTANR | 9,64 | 447,72 | 665,34 | 25 |
| 1125 | TGAGFTANR | 9,64 | 447,72 | 793,4 | 25 |
| 1126 | TGFNDGQK | 7,5 | 433,7 | 561,26 | 24 |
| 1127 | TGFNDGQK | 7,5 | 433,7 | 708,33 | 24 |
| 1128 | TGFNDGQK | 7,5 | 433,7 | 765,35 | 24 |
| 1129 | TGLADSK | 9,7 | 346,18 | 533,29 | 20 |
| 1130 | TGLADSK | 9,67 | 346,18 | 545,26 | 20 |
| 1131 | TGLADSK | 9,7 | 346,18 | 590,31 | 20 |
| 1132 | TGLDLMQK | 15,32 | 453,24 | 634,32 | 25 |
| 1133 | TGLDLMQK | 15,32 | 453,24 | 747,41 | 25 |
| 1134 | TGLDLMQK | 15,32 | 453,24 | 804,43 | 25 |
| 1135 | TGLELMQK | 15,03 | 460,25 | 648,34 | 25 |
| 1136 | TGLELMQK | 15,03 | 460,25 | 761,42 | 25 |
| 1137 | TGLELMQK | 15,03 | 460,25 | 818,44 | 25 |
| 1138 | TGMGYPK | 10,28 | 377,18 | 464,25 | 22 |
| 1139 | TGMGYPK | 10,28 | 377,18 | 595,29 | 22 |
| 1140 | TGMGYPK | 10,28 | 377,18 | 652,31 | 22 |
| 1141 | TGNGR | 0,8 | 252,63 | 330,14 | 16 |
| 1142 | TGNGR | 0,8 | 252,63 | 346,18 | 16 |
| 1143 | TGNGR | 0,81 | 252,63 | 403,2 | 16 |
| 1144 | TGTGSFIDAR | 13,35 | 512,76 | 708,37 | 28 |
| 1145 | TGTGSFIDAR | 13,35 | 512,76 | 765,39 | 28 |
| 1146 | TGTGSFIDAR | 13,35 | 512,76 | 866,44 | 28 |
| 1147 | TGTGSLSDAK | 8,32 | 468,74 | 620,32 | 26 |
| 1148 | TGTGSLSDAK | 8,32 | 468,74 | 677,35 | 26 |
| 1149 | TGTGSLSDAK | 8,32 | 468,74 | 778,39 | 26 |
| 1150 | TGVATEYQPEIGWWAGWVER | 25,49 | 779,04 | 903,45 | 43 |
| 1151 | TGVATEYQPEIGWWAGWVER | 25,5 | 779,04 | 1146,55 | 43 |
| 1152 | TGVATEYQPEIGWWAGWVER | 25,52 | 1168,06 | 1189,57 | 56 |
| 1153 | TGVSYPLLADGTR | 17,4 | 675,36 | 842,47 | 35 |
| 1154 | TGVSYPLLADGTR | 17,41 | 675,36 | 1005,54 | 35 |
| 1155 | TGVSYPLLADGTR | 17,4 | 675,36 | 1092,57 | 35 |
| 1156 | TGWAMDIK | 16,71 | 461,23 | 577,3 | 25 |
| 1157 | TGWAMDIK | 16,71 | 461,23 | 763,38 | 25 |
| 1158 | TGWAMDIK | 16, 72 | 461,23 | 820,4 | 25 |
| 1159 | TGWATR | 9,71 | 346,18 | 517,24 | 20 |
| 1160 | TGWATR | 9,69 | 346,18 | 533,28 | 20 |
| 1161 | TGWATR | 9,69 | 346,18 | 590,3 | 20 |
| 1162 | TGWC[CAM]FDC[CAM]TPELGWWVGWVK | 28,39 | 795,36 | 960,51 | 44 |
| 1163 | TGWC[CAM]FDC[CAM]TPELGWWVGWVK | 28,39 | 795,36 | 1017,53 | 44 |
| 1164 | TGWC[CAM]FDC[CAM]TPELGWWVGWVK | 28,38 | 795,36 | 1028,36 | 44 |
| 1165 | TGWEGR | 9,1 | 353,17 | 531,22 | 21 |
| 1166 | TGWEGR | 9,09 | 353,17 | 547,26 | 21 |
| 1167 | TGWEGR | 9,09 | 353,17 | 604,28 | 21 |
| 1168 | TGWFVDK | 16,08 | 426,72 | 694,36 | 24 |
| 1169 | TGWFVDK | 16,1 | 426,72 | 706,32 | 24 |
| 1170 | TGWFVDK | 16,08 | 426,72 | 751,38 | 24 |
| 1171 | TGYDTK | 2,09 | 342,66 | 526,25 | 20 |
| 1172 | TGYDTK | 2,09 | 342,66 | 538,21 | 20 |
| 1173 | TGYDTK | 2,08 | 342,66 | 583,27 | 20 |
| 1174 | TGYGVR | 8,09 | 326,67 | 478,23 | 19 |
| 1175 | TGYGVR | 8,1 | 326,67 | 494,27 | 19 |
| 1176 | TGYGVR | 8,1 | 326,67 | 551,29 | 19 |
| 1177 | TGYSAR | 2,24 | 327,66 | 480,21 | 19 |
| 1178 | TGYSAR | 2,24 | 327,66 | 496,25 | 19 |
| 1179 | TGYSAR | 2,24 | 327,66 | 553,27 | 19 |
| 1180 | TGYSTR | 2,08 | 342,67 | 510,22 | 20 |
| 1181 | TGYSTR | 2,08 | 342,67 | 526,26 | 20 |
| 1182 | TGYSTR | 2,08 | 342,67 | 583,28 | 20 |
| 1183 | THESSNWGK | 5,36 | 523,24 | 678,32 | 28 |
| 1184 | THESSNWGK | 5,37 | 523,24 | 807,36 | 28 |
| 1185 | THESSNWGK | 5,37 | 523,24 | 944,42 | 28 |
| 1186 | TIC[CAM]TAIADAGTGK | 14,35 | 639,82 | 732,39 | 33 |
| 1187 | TIC[CAM]TAIADAGTGK | 14,35 | 639,82 | 904,47 | 33 |
| 1188 | TIC[CAM]TAIADAGTGK | 14,35 | 639,82 | 1064,5 | 33 |
| 1189 | TIGGAPDAYWVDDSLQISAR | 21,22 | 712,35 | 1004,5 | 40 |
| 1190 | TIGGAPDAYWVDDSLQISAR | 21,22 | 712,35 | 1103,57 | 40 |
| 1191 | TIGGAPDAYWVDDSLQISAR | 21,21 | 1068,02 | 1103,57 | 52 |
| 1192 | TLPFSASSYETLR | 18,73 | 736,37 | 855,42 | 37 |
| 1193 | TLPFSASSYETLR | 18,73 | 736,37 | 1013,49 | 37 |
| 1194 | TLPFSASSYETLR | 18,73 | 736,37 | 1160,56 | 37 |
| 1195 | TLPFSPK | 15 | 395,23 | 478,27 | 22 |
| 1196 | TLPFSPK | 15 | 395,23 | 575,32 | 22 |
| 1197 | TLPFSPK | 15 | 395,23 | 688,4 | 22 |
| 1198 | TLPFSQEVQDEVQSILFIEEK | 28,55 | 827,09 | 891,52 | 45 |
| 1199 | TLPFSQEVQDEVQSILFIEEK | 28,56 | 827,09 | 978,55 | 45 |
| 1200 | TLPFSQEVQDEVQSILFIEEK | 28,56 | 827,09 | 1106,61 | 45 |
| 1201 | TLPFSQEVQDEVQSMLFIEEK | 27,7 | 833,08 | 996,51 | 46 |
| 1202 | TLPFSQEVQDEVQSMLFIEEK | 27,69 | 833,08 | 1124,57 | 46 |
| 1203 | TLPFSQEVQDEVQSMLFIEEK | 27,7 | 833,08 | 1223,63 | 46 |
| 1204 | TLQNGWFEGFIISK | 24,12 | 820,43 | 940,51 | 41 |
| 1205 | TLQNGWFEGFIISK | 24,11 | 820,43 | 1126,59 | 41 |
| 1206 | TLQNGWFEGFIISK | 24,11 | 820,43 | 1183,61 | 41 |
| 1207 | TMQEYLNK | 12,6 | 513,75 | 666,35 | 28 |
| 1208 | TMQEYLNK | 12,6 | 513,75 | 794,4 | 28 |
| 1209 | TMQEYLNK | 12,6 | 513,75 | 925,44 | 28 |
| 1210 | TQTYQAYDAAR | 11,2 | 644,3 | 666,32 | 33 |
| 1211 | TQTYQAYDAAR | 11,2 | 644,3 | 957,44 | 33 |
| 1212 | TQTYQAYDAAR | 11,2 | 644,3 | 1058,49 | 33 |
| 1213 | TTDPTIWEK | 14,39 | 545,77 | 676,37 | 29 |
| 1214 | TTDPTIWEK | 14,39 | 545,77 | 773,42 | 29 |
| 1215 | TTDPTIWEK | 14,39 | 545,77 | 888,45 | 29 |
| 1216 | TTTTEVFK | 12,06 | 463,75 | 522,29 | 25 |
| 1217 | TTTTEVFK | 12,06 | 463,75 | 623,34 | 25 |
| 1218 | TTTTEVFK | 12,06 | 463,75 | 724,39 | 25 |
| 1219 | TWASNDFSR | 13,73 | 542,25 | 638,29 | 29 |
| 1220 | TWASNDFSR | 13,73 | 542,25 | 725,32 | 29 |
| 1221 | TWASNDFSR | 13,73 | 542,25 | 796,36 | 29 |
| 1222 | TWDMVQR | 14,28 | 468,22 | 648,31 | 26 |
| 1223 | TWDMVQR | 14,28 | 468,22 | 761,33 | 26 |
| 1224 | TWDMVQR | 14,28 | 468,22 | 834,39 | 26 |
| 1225 | TYVVDPAR | 12,15 | 460,75 | 557,3 | 25 |
| 1226 | TYVVDPAR | 12,14 | 460,75 | 656,37 | 25 |
| 1227 | TYVVDPAR | 12,15 | 460,75 | 819,44 | 25 |
| 1228 | VAFSLNIEMK | 20,65 | 576,31 | 747,41 | 30 |
| 1229 | VAFSLNIEMK | 20,65 | 576,31 | 834,44 | 30 |
| 1230 | VAFSLNIEMK | 20,65 | 576,31 | 981,51 | 30 |
| 1231 | VANSLIGLSTGAVR | 17,97 | 679,39 | 760,43 | 35 |
| 1232 | VANSLIGLSTGAVR | 17,97 | 679,39 | 873,52 | 35 |
| 1233 | VANSLIGLSTGAVR | 17,97 | 679,39 | 986,6 | 35 |
| 1234 | VELGK | 7,74 | 273,17 | 342,2 | 17 |
| 1235 | VELGK | 7,75 | 273,17 | 399,22 | 17 |
| 1236 | VELGK | 7,74 | 273,17 | 446,26 | 17 |
| 1237 | VFLDSWAK | 18,2 | 483,26 | 606,29 | 26 |
| 1238 | VFLDSWAK | 18,2 | 483,26 | 719,37 | 26 |
| 1239 | VFLDSWAK | 18,2 | 483,26 | 866,44 | 26 |
| 1240 | VFLESWAK | 18,11 | 490,27 | 620,3 | 27 |
| 1241 | VFLESWAK | 18,11 | 490,27 | 733,39 | 27 |
| 1242 | VFLESWAK | 18,11 | 490,27 | 880,46 | 27 |
| 1243 | VFLSSWAQDMNLSSAIK | 23,66 | 948,98 | 978,49 | 47 |
| 1244 | VFLSSWAQDMNLSSAIK | 23,66 | 948,98 | 1106,55 | 47 |
| 1245 | VFLSSWAQDMNLSSAIK | 23,66 | 948,98 | 1177,59 | 47 |
| 1246 | VGFER | 10,32 | 304,16 | 433,21 | 18 |
| 1247 | VGFER | 10,32 | 304,16 | 451,23 | 18 |
| 1248 | VGFER | 10,32 | 304,16 | 508,25 | 18 |
| 1249 | VILVFDQVR | 19,69 | 544,83 | 664,34 | 29 |
| 1250 | VILVFDQVR | 19,69 | 544,83 | 763,41 | 29 |
| 1251 | VILVFDQVR | 19,69 | 544,83 | 876,49 | 29 |
| 1252 | VMAAMVR | 12,3 | 389,21 | 476,26 | 22 |
| 1253 | VMAAMVR | 12,3 | 389,21 | 547,3 | 22 |
| 1254 | VMAAMVR | 12,3 | 389,21 | 678,34 | 22 |
| 1255 | VPLAVMGYDAGILVDAHNPR | 21,61 | 703,37 | 709,34 | 39 |
| 1256 | VPLAVMGYDAGILVDAHNPR | 21,61 | 703,37 | 808,41 | 39 |
| 1257 | VPLAVMGYDAGILVDAHNPR | 21,61 | 703,37 | 921,49 | 39 |
| 1258 | VQDEVQSMLFIEEK | 20,48 | 847,92 | 996,51 | 42 |
| 1259 | VQDEVQSMLFIEEK | 20,48 | 847,92 | 1124,57 | 42 |
| 1260 | VQDEVQSMLFIEEK | 20,47 | 847,92 | 1223,63 | 42 |
| 1261 | VQDGVQSMLFIEEK | 20,26 | 811,91 | 996,51 | 41 |
| 1262 | VQDGVQSMLFIEEK | 20,27 | 811,91 | 1124,57 | 41 |
| 1263 | VQDGVQSMLFIEEK | 20,25 | 811,91 | 1223,63 | 41 |
| 1264 | VSC[CAM]LPC[CAM]YQVVSHK | 14,32 | 526,26 | 569,34 | 30 |
| 1265 | VSC[CAM]LPC[CAM]YQVVSHK | 14,32 | 526,26 | 860,46 | 30 |
| 1266 | VSC[CAM]LPC[CAM]YQVVSHK | 14,31 | 526,26 | 1020,49 | 30 |
| 1267 | VSC[CAM]VWC[CAM]YQALAR | 18,41 | 756,86 | 881,43 | 38 |
| 1268 | VSC[CAM]VWC[CAM]YQALAR | 18,41 | 756,86 | 1067,51 | 38 |
| 1269 | VSC[CAM]VWC[CAM]YQALAR | 18,41 | 756,86 | 1166,58 | 38 |
| 1270 | VSDVC[CAM]SEVTAEGWQEVR | 17,33 | 650,97 | 774,39 | 37 |
| 1271 | VSDVC[CAM]SEVTAEGWQEVR | 17,34 | 975,95 | 1075,52 | 48 |
| 1272 | VSDVC[CAM]SEVTAEGWQEVR | 17,34 | 975,95 | 1174,59 | 48 |
| 1273 | VSEVEGWQIHGK | 13,92 | 456,9 | 582,34 | 27 |
| 1274 | VSEVEGWQIHGK | 13,92 | 456,9 | 768,42 | 27 |
| 1275 | VSEVEGWQIHGK | 13,92 | 456,9 | 825,44 | 27 |
| 1276 | VSFSLNIEMK | 20,65 | 584,31 | 834,44 | 31 |
| 1277 | VSFSLNIEMK | 20,64 | 584,31 | 981,51 | 31 |
| 1278 | VSFSLNIEMK | 20,65 | 584,31 | 1068,54 | 31 |
| 1279 | VSPC[CAM]SSFK | 11,04 | 456,22 | 468,25 | 25 |
| 1280 | VSPC[CAM]SSFK | 11,04 | 456,22 | 628,28 | 25 |
| 1281 | VSPC[CAM]SSFK | 11,04 | 456,22 | 725,33 | 25 |
| 1282 | VSQVPAYK | 10,68 | 446,25 | 478,27 | 25 |
| 1283 | VSQVPAYK | 10,68 | 446,25 | 577,33 | 25 |
| 1284 | VSQVPAYK | 10,68 | 446,25 | 705,39 | 25 |
| 1285 | VVFAR | 11,17 | 296,18 | 393,22 | 18 |
| 1286 | VVFAR | 11,17 | 296,18 | 417,25 | 18 |
| 1287 | VVFAR | 11,17 | 296,18 | 492,29 | 18 |
| 1288 | WDGAK | 4,9 | 288,64 | 302,11 | 18 |
| 1289 | WDGAK | 4,9 | 288,64 | 390,2 | 18 |
| 1290 | WDGAK | 4,9 | 288,64 | 430,17 | 18 |
| 1291 | WDGHIYDFPDWNR | 20,52 | 574,25 | 590,27 | 33 |
| 1292 | WDGHIYDFPDWNR | 20,52 | 574,25 | 687,32 | 33 |
| 1293 | WDGHIYDFPDWNR | 20,52 | 574,25 | 887,37 | 33 |
| 1294 | WDGIK | 12,03 | 309,67 | 359,13 | 19 |
| 1295 | WDGIK | 12,03 | 309,67 | 432,25 | 19 |
| 1296 | WDGIK | 12,03 | 309,67 | 472,22 | 19 |
| 1297 | WDGKPR | 6,36 | 379,7 | 457,29 | 22 |
| 1298 | WDGKPR | 6,35 | 379,7 | 572,32 | 22 |
| 1299 | WDGKPR | 6,36 | 379,7 | 584,28 | 22 |
| 1300 | W DGQTR | 7,41 | 381,68 | 461,25 | 22 |
| 1301 | W DGQTR | 7,41 | 381,68 | 576,27 | 22 |
| 1302 | W DGQTR | 7,41 | 381,68 | 588,24 | 22 |
| 1303 | WDGVK | 10,1 | 302,66 | 359,13 | 18 |
| 1304 | WDGVK | 10,1 | 302,66 | 418,23 | 18 |
| 1305 | WDGVK | 10,1 | 302,66 | 458,2 | 18 |
| 1306 | WDGVNR | 10,39 | 373,68 | 445,25 | 21 |
| 1307 | WDGVNR | 10,39 | 373,68 | 560,28 | 21 |
| 1308 | WDGVNR | 10,42 | 373,68 | 572,25 | 21 |
| 1309 | YAQAK | 12,58 | 290,66 | 363,17 | 18 |
| 1310 | YAQAK | 12,58 | 290,66 | 417,25 | 18 |
| 1311 | YAQAK | 12,58 | 290,66 | 434,2 | 18 |
| 1312 | YFSDFNAK | 14,21 | 496,23 | 681,32 | 27 |
| 1313 | YFSDFNAK | 14,21 | 496,23 | 828,39 | 27 |
| 1314 | YFSDFNAK | 14,21 | 496,23 | 828,39 | 27 |
| 1315 | YGTHLDR | 8,51 | 431,21 | 641,34 | 24 |
| 1316 | YGTHLDR | 8,52 | 431,21 | 687,31 | 24 |
| 1317 | YGTHLDR | 8,51 | 431,21 | 698,36 | 24 |
| 1318 | YLDELVK | 15,52 | 440,24 | 488,31 | 24 |
| 1319 | YLDELVK | 15,53 | 440,24 | 603,33 | 24 |
| 1320 | YLDELVK | 15,52 | 440,24 | 716,42 | 24 |
| 1321 | YLMITEAGR | 15,86 | 527,27 | 533,27 | 28 |
| 1322 | YLMITEAGR | 15,86 | 527,27 | 646,35 | 28 |
| 1323 | YLMITEAGR | 15,86 | 527,27 | 777,39 | 28 |
| 1324 | YLNLFSYGNANIGGGIDK | 22,16 | 639,32 | 773,42 | 36 |
| 1325 | YLNLFSYGNANIGGGIDK | 22,16 | 958,48 | 1015,52 | 47 |
| 1326 | YLNLFSYGNANIGGGIDK | 22,16 | 958,48 | 1178,58 | 47 |
| 1327 | YPVVWYSQQVAHHLGAQR | 18,11 | 535,53 | 544,32 | 30 |
| 1328 | YPVVWYSQQVAHHLGAQR | 18,11 | 535,53 | 681,38 | 30 |
| 1329 | YPVVWYSQQVAHHLGAQR | 18,11 | 535,53 | 889,48 | 30 |
| 1330 | YSNVLAFK | 16,44 | 471,26 | 478,3 | 26 |
| 1331 | YSNVLAFK | 16,44 | 471,26 | 691,41 | 26 |
| 1332 | YSNVLAFK | 16,44 | 471,26 | 778,45 | 26 |
| 1333 | YSPASTFK | 12,22 | 450,73 | 553,3 | 25 |
| 1334 | YSPASTFK | 12,22 | 450,73 | 650,35 | 25 |
| 1335 | YSPASTFK | 12,22 | 450,73 | 737,38 | 25 |
| 1336 | YSVVPVYQQLAR | 18,42 | 711,89 | 778,42 | 36 |
| 1337 | YSVVPVYQQLAR | 18,42 | 711,89 | 974,54 | 36 |
| 1338 | YSVVPVYQQLAR | 18,43 | 711,89 | 1073,61 | 36 |
| 1339 | YSVVWYSQLTAK | 19,75 | 722,88 | 810,44 | 37 |
| 1340 | YSVVWYSQLTAK | 19,76 | 722,88 | 996,51 | 37 |
| 1341 | YSWWYSQLTAK | 19,76 | 722,88 | 1095,58 | 37 |
| 1342 | YSVVWYSQQVAHHLGAQR | 18,61 | 533,02 | 544,32 | 30 |
| 1343 | YSVVWYSQQVAHHLGAQR | 18,61 | 533,02 | 681,38 | 30 |
| 1344 | YSVVWYSQQVAHHLGAQR | 18,61 | 533,02 | 889,48 | 30 |
| 1345 | YTPASTFK | 11,95 | 305,49 | 553,3 | 19 |
| 1346 | YTPASTFK | 11,98 | 457,73 | 553,3 | 25 |
| 1347 | YTPASTFK | 11,98 | 457,73 | 650,35 | 25 |
| 1348 | YTSAFGYGNADVSGEPGK | 15,03 | 607,28 | 673,35 | 34 |
| 1349 | YTSAFGYGNADVSGEPGK | 15,02 | 607,28 | 788,38 | 34 |
| 1350 | YTSAFGYGNADVSGEPGK | 15,02 | 910,41 | 1030,48 | 45 |
| 1351 | YVFVSALTGNLGSNLTSSIK | 23,66 | 691,04 | 906,49 | 39 |
| 1352 | YVFVSALTGNLGSNLTSSIK | 23,66 | 1036,06 | 1165,63 | 51 |
| 1353 | YVFVSALTGNLGSNLTSSIK | 23,67 | 1036,06 | 1190,64 | 51 |
| 1354 | YVFVSALTGSLGSNLTSSIK | 24,04 | 682,04 | 906,49 | 38 |
| 1355 | YVFVSALTGSLGSNLTSSIK | 24,04 | 1022,55 | 1106,61 | 50 |
| 1356 | YVFVSALTGSLGSNLTSSIK | 24,04 | 1022,55 | 1163,63 | 50 |

Les aires obtenues pour chacune des transitions et pour chacun des microorganismes étudiés ont été mesurées. Lorsque les aires des trois transitions d'un même peptide sont supérieures ou égales à 2500 la détection du peptide est considérée comme positive et est noté « 1 ». Lorsque au moins une transition comporte une aire inférieure à 2500, le peptide correspondant est considéré comme non détecté et est noté « 0 ».

### Exemple 25 : Identification d'une résistance aux béta-lactamines de type ACC:

Les échantillons Ech96 à Ech101 sont identifiés selon l'une des méthodes décrites dans les exemples 1, 3 ou 4. L'identification des espèces est reportée dans le TABLEAU 31.

**TABLEAU 31 :**

| Noms | Espèces |
|---|---|
| Ech96 | *K. oxytoca* |
| Ech97 | *S*. *livingstone* |
| Ech98 | *Salmonella spp* |
| Ech99 | *S*. *enterica ssp enterica* |
| Ech100 | *K. pneumoniae* |
| Ech101 | *H. alvei* |

Les échantillons Ech96 à Ech101 correspondent à une espèce pouvant comporter un mécanisme de résistance de type ACC. Chaque échantillon est traité selon l'exemple 5, puis analysé selon l'exemple 6 en détectant les peptides du **TABLEAU 32** en lieu et place des peptides du **TABLEAU 3.**

**TABLEAU 32 :**

| Transition numéro | Peptide | Etat de charge du précurseur | Ion fragment | Temps de rétention (minutes) | (m/z) filtré en Q1 | (m/z) filtré en Q3 | Energie de collision (eV) | Seuil de positivité |
|---|---|---|---|---|---|---|---|---|
| 1 | ANIDESK | 2 | y4 monochargé | 2,45 | 388,69 | 478,21 | 17,9 | 2500 |
| 2 | ANIDESK | 2 | y5 monochargé | 2,37 | 388,69 | 591,3 | 17,9 | 2500 |
| 3 | ANIDESK | 2 | y6 monochargé | 2,41 | 388,69 | 705,34 | 17,9 | 2500 |
| 4 | AWKPADAAGTHR | 3 | y9 dichargé | 10,61 | 427,56 | 448,22 | 20,3 | 2500 |
| 5 | AWKPADAAGTHR | 3 | y4 monochargé | 10,61 | 427,56 | 470,25 | 20,3 | 2500 |
| 6 | AWKPADAAGTHR | 3 | y10 dichargé | 10,61 | 427,56 | 512,27 | 20,3 | 2500 |
| 7 | DEPVHVNMEILGNEAYGIK | 3 | y9 dichargé | 20,81 | 710,02 | 482,76 | 29,1 | 2500 |
| 8 | DEPVHVNMEILGNEAYGIK | 3 | y8 monochargé | 20,81 | 710,02 | 851,43 | 29,1 | 2500 |
| 9 | DEPVHVNMEILGNEAYGIK | 3 | y9 monochargé | 20,81 | 710,02 | 964,51 | 29,1 | 2500 |
| 10 | DTVDDLIQPLMQK | 3 | y5 monochargé | 22,36 | 505,93 | 616,35 | 22,8 | 2500 |
| 11 | DTVDDLIQPLMQK | 2 | y5 monochargé | 22,36 | 758,39 | 616,35 | 39 | 2500 |
| 12 | DTVDDLIQPLMQK | 2 | y10 monochargé | 22,36 | 758,39 | 1200,63 | 39 | 2500 |
| 13 | ILSSLEGNK | 2 | y6 monochargé | 13,47 | 480,77 | 647,34 | 23,1 | 2500 |
| 14 | ILSSLEGNK | 2 | y7 monochargé | 13,47 | 480,77 | 734,37 | 23,1 | 2500 |
| 15 | ILSSLEGNK | 2 | y8 monochargé | 13,47 | 480,77 | 847,45 | 23,1 | 2500 |
| 16 | LSLDQSVSHYVPELR | 3 | y12 dichargé | 20,27 | 581,64 | 715,36 | 25,1 | 2500 |
| 17 | LSLDQSVSHYVPELR | 3 | y13 dichargé | 20,27 | 581,64 | 771,9 | 25,1 | 2500 |
| 18 | LSLDQSVSHYVPELR | 3 | y14 dichargé | 20,27 | 581,64 | 815,42 | 25,1 | 2500 |
| 19 | MGIVMLANK | 2 | y5 monochargé | 18,15 | 488,77 | 576,32 | 23,6 | 2500 |
| 20 | MGIVMLANK | 2 | y6 monochargé | 18,15 | 488,77 | 675,39 | 23,6 | 2500 |
| 21 | MGIVMLANK | 2 | y8 monochargé | 18,13 | 488,77 | 845,49 | 23,6 | 2500 |
| 22 | MQQALTATHTGYFK | 3 | y9 dichargé | 15,01 | 532,93 | 513,26 | 23,6 | 2500 |
| 23 | MQQALTATHTGYFK | 3 | y11 dichargé | 15,01 | 532,93 | 605,32 | 23,6 | 2500 |
| 24 | MQQALTATHTGYFK | 3 | y12 dichargé | 15,01 | 532,93 | 669,35 | 23,6 | 2500 |
| 25 | NNIPGMSVAVTVNGK | 2 | y5 monochargé | 17,45 | 750,9 | 518,29 | 38,5 | 2500 |
| 26 | NNIPGMSVAVTVNGK | 2 | y12 dichargé | 17,43 | 750,9 | 580,31 | 38,5 | 2500 |
| 27 | NNIPGMSVAVTVNGK | 2 | y12 monochargé | 17,43 | 750,9 | 1159,61 | 38,5 | 2500 |
| 28 | NTTQLMAYLK | 2 | y5 monochargé | 19,34 | 591,81 | 625,34 | 29,5 | 2500 |
| 29 | NTTQLMAYLK | 2 | y6 monochargé | 19,34 | 591,81 | 738,42 | 29,5 | 2500 |
| 30 | NTTQLMAYLK | 2 | y8 monochargé | 19,36 | 591,81 | 967,53 | 29,5 | 2500 |
| 31 | NYIYNYGLAAK | 2 | y7 monochargé | 16,98 | 645,33 | 736,4 | 32,5 | 2500 |
| 32 | NYIYNYGLAAK | 2 | y8 monochargé | 16,98 | 645,33 | 899,46 | 32,5 | 2500 |
| 33 | NYIYNYGLAAK | 2 | y9 monochargé | 16,98 | 645,33 | 1012,55 | 32,5 | 2500 |
| 34 | NYSIDQR | 2 | y3 monochargé | 10,93 | 448,22 | 418,2 | 21,3 | 2500 |
| 35 | NYSIDQR | 2 | y4 monochargé | 10,91 | 448,22 | 531,29 | 21,3 | 2500 |
| 36 | NYSIDQR | 2 | y5 monochargé | 10,91 | 448,22 | 618,32 | 21,3 | 2500 |
| 37 | QPQQPVTENTLFEVGSLSK | 3 | y5 monochargé | 20,77 | 701,36 | 491,28 | 28,8 | 2500 |
| 38 | QPQQPVTENTLFEVGSLSK | 3 | y7 monochargé | 20,77 | 701,36 | 719,39 | 28,8 | 2500 |
| 39 | QPQQPVTENTLFEVGSLSK | 3 | y8 monochargé | 20,79 | 701,36 | 866,46 | 28,8 | 2500 |
| 40 | SLGVSYEDAI EK | 2 | y6 monochargé | 16,42 | 655,83 | 704,35 | 33,1 | 2500 |
| 41 | SLGVSYEDAIEK | 2 | y8 monochargé | 16,42 | 655,83 | 954,44 | 33,1 | 2500 |
| 42 | SLGVSYEDAIEK | 2 | y10 monochargé | 16,42 | 655,83 | 1110,53 | 33,1 | 2500 |
| 43 | SVATPIVPPLPPQENVWINK | 3 | y10 dichargé | 22,31 | 733,74 | 612,82 | 29,8 | 2500 |
| 44 | SVATPIVPPLPPQENVWINK | 3 | y13 dichargé | 22,29 | 733,74 | 766,42 | 29,8 | 2500 |
| 45 | SVATPIVPPLPPQENVWINK | 3 | y10 monochargé | 22,35 | 733,74 | 1224,64 | 29,8 | 2500 |
| 46 | TFAATLASYAQVSGK | 3 | y6 monochargé | 19,93 | 505,6 | 589,33 | 22,8 | 2500 |
| 47 | TFAATLASYAQVSGK | 2 | y8 monochargé | 19,93 | 757,9 | 839,43 | 38,9 | 2500 |
| 48 | TFAATLASYAQVSGK | 2 | y9 monochargé | 19,93 | 757,9 | 910,46 | 38,9 | 2500 |
| 49 | TGSTNGFGAYIAFVPAK | 3 | y3 monochargé | 21,68 | 567,63 | 315,2 | 24,7 | 2500 |
| 50 | TGSTNGFGAYIAFVPAK | 3 | y6 monochargé | 21,68 | 567,63 | 632,38 | 24,7 | 2500 |
| 51 | TGSTNGFGAYIAFVPAK | 2 | y3 monochargé | 21,68 | 850,94 | 315,2 | 44,2 | 2500 |
| 52 | TLLPQLGMHHSYLK | 3 | y11 dichargé | 17,99 | 546,63 | 655,84 | 24 | 2500 |
| 53 | TLLPQLGMHHSYLK | 3 | y12 dichargé | 17,95 | 546,63 | 712,38 | 24 | 2500 |
| 54 | TLLPQLGMHHSYLK | 2 | y11 dichargé | 17,97 | 819,45 | 655,84 | 42,4 | 2500 |
| 55 | TTSSDLLR | 2 | y4 monochargé | 13 | 446,74 | 516,31 | 21,2 | 2500 |
| 56 | TTSSDLLR | 2 | y5 monochargé | 13 | 446,74 | 603,35 | 21,2 | 2500 |
| 57 | TTSSDLLR | 2 | y6 monochargé | 13 | 446,74 | 690,38 | 21,2 | 2500 |
| 58 | VPADQMENYAWGYNK | 3 | y4 monochargé | 17,53 | 595,94 | 481,24 | 25,6 | 2500 |
| 59 | VPADQMENYAWGYNK | 3 | y5 monochargé | 17,53 | 595,94 | 667,32 | 25,6 | 2500 |
| 60 | VPADQMENYAWGYNK | 3 | y6 monochargé | 17,53 | 595,94 | 738,36 | 25,6 | 2500 |
| 61 | VYSNIGTGLLGMIAAK | 3 | y6 monochargé | 24,59 | 536,63 | 590,33 | 23,7 | 2500 |
| 62 | VYSNIGTGLLGMIAAK | 3 | y7 monochargé | 24,59 | 536,63 | 703,42 | 23,7 | 2500 |
| 63 | VYSNIGTGLLGMIAAK | 2 | y11 monochargé | 24,59 | 804,45 | 1031,59 | 41,6 | 2500 |
| 64 | YVQANMGQLK | 2 | y4 monochargé | 13,88 | 576,3 | 445,28 | 28,6 | 2500 |
| 65 | YVQANMGQLK | 2 | y7 monochargé | 13,88 | 576,3 | 761,4 | 28,6 | 2500 |
| 66 | YVQANMGQLK | 2 | y8 monochargé | 13,88 | 576,3 | 889,46 | 28,6 | 2500 |

Les autres paramètres machine utilisés sont les suivants :
- Type de balayage: MRM
- MRM planifié: non
- Polarité: Positive
- Source d'ionisation:Turbo V™ (Applied BioSystems)
- Réglage Q1: Filtrage avec résolution unitaire
- Réglage Q3: Filtrage avec résolution unitaire
- Pause inter-scan: 5.00 msec
- Vitesse de balayage: 10 Da/s
- Gaz rideau: 50,00 psi
- Tension de cône: 5500,00 V
- Température de source: 500,00 °C
- Gaz de nébulisation: 50,00 psi
- Gaz chauffant: 50,00 psi
- Gaz de collision induisant dissociation : 9,00 psi
- Remplissage dynamique: activé
- Potentiel d'orifice : 100, 00 V
- (en anglais declustering potential (DP))
- Potentiel d'entrée avant Q0 (EP): 6,00 V
- Potentiel en sortie de cellule de collision : 15 V
- (en anglais cell exit potential (CXP))
- Temps de cycle total: 1,32 sec

Les aires obtenues pour chacune des transitions et pour chacun des microorganismes étudiés ont été mesurées. Lorsque les aires des transitions sont supérieures ou égales au seuil de positivité décrit dans le **TABLEAU 32,** la détection de la transition est considérée comme positive et est notée « 1 » dans le **TABLEAU 33.** Lorsqu'une transition a une aire inférieure au seuil de positivité décrit dans le **TABLEAU 32,** la transition est considérée comme non détectée et est notée « 0 » dans le **TABLEAU 33.**

Pour un peptide donné, lorsque au moins 3 transitions sont notées « 1 », le peptide est considéré comme détecté.

**TABLEAU 33 :**

| Transition numéro | Ech96 | Ech97 | Ech98 | Ech99 | Ech100 | Ech101 |
|---|---|---|---|---|---|---|
| 1 | 0 | 1 | 1 | 1 | 1 | 0 |
| 2 | 0 | 1 | 1 | 1 | 1 | 0 |
| 3 | 0 | 1 | 1 | 1 | 1 | 0 |
| 4 | 1 | 1 | 1 | 1 | 1 | 1 |
| 5 | 1 | 1 | 1 | 1 | 1 | 1 |
| 6 | 1 | 1 | 1 | 1 | 1 | 1 |
| 7 | 1 | 1 | 1 | 1 | 1 | 0 |
| 8 | 1 | 1 | 1 | 1 | 1 | 0 |
| 9 | 1 | 1 | 1 | 1 | 1 | 0 |
| 10 | 1 | 1 | 1 | 1 | 1 | 0 |
| 11 | 1 | 1 | 1 | 1 | 1 | 0 |
| 12 | 1 | 1 | 1 | 1 | 1 | 0 |
| 13 | 1 | 1 | 1 | 1 | 1 | 0 |
| 14 | 1 | 1 | 1 | 1 | 1 | 0 |
| 15 | 1 | 1 | 1 | 1 | 1 | 0 |
| 16 | 1 | 1 | 1 | 1 | 1 | 1 |
| 17 | 1 | 1 | 1 | 1 | 1 | 1 |
| 18 | 1 | 1 | 1 | 1 | 1 | 1 |
| 19 | 1 | 1 | 1 | 1 | 1 | 0 |
| 20 | 1 | 1 | 1 | 1 | 1 | 0 |
| 21 | 1 | 1 | 1 | 1 | 1 | 0 |
| 22 | 1 | 1 | 1 | 1 | 1 | 1 |
| 23 | 1 | 1 | 1 | 1 | 1 | 1 |
| 24 | 1 | 1 | 1 | 1 | 1 | 1 |
| 25 | 1 | 1 | 1 | 1 | 1 | 1 |
| 26 | 1 | 1 | 1 | 1 | 1 | 1 |
| 27 | 1 | 1 | 1 | 1 | 1 | 1 |
| 28 | 1 | 1 | 1 | 1 | 1 | 0 |
| 29 | 1 | 1 | 1 | 1 | 1 | 0 |
| 30 | 1 | 1 | 1 | 1 | 1 | 0 |
| 31 | 1 | 1 | 1 | 1 | 1 | 0 |
| 32 | 1 | 1 | 1 | 1 | 1 | 0 |
| 33 | 1 | 1 | 1 | 1 | 1 | 0 |
| 34 | 1 | 1 | 1 | 1 | 1 | 0 |
| 35 | 1 | 1 | 1 | 1 | 1 | 0 |
| 36 | 1 | 1 | 1 | 1 | 1 | 0 |
| 37 | 1 | 1 | 1 | 1 | 1 | 0 |
| 38 | 1 | 1 | 1 | 1 | 1 | 0 |
| 39 | 1 | 1 | 1 | 1 | 1 | 0 |
| 40 | 1 | 1 | 1 | 1 | 1 | 1 |
| 41 | 1 | 1 | 1 | 1 | 1 | 1 |
| 42 | 1 | 1 | 1 | 1 | 1 | 1 |
| 43 | 1 | 1 | 1 | 1 | 1 | 1 |
| 44 | 1 | 1 | 1 | 1 | 1 | 1 |
| 45 | 1 | 1 | 1 | 1 | 1 | 1 |
| 46 | 1 | 1 | 1 | 1 | 1 | 0 |
| 47 | 1 | 1 | 1 | 1 | 1 | 0 |
| 48 | 1 | 1 | 1 | 1 | 1 | 0 |
| 49 | 1 | 1 | 1 | 1 | 1 | 1 |
| 50 | 1 | 1 | 1 | 1 | 1 | 1 |
| 51 | 1 | 1 | 1 | 1 | 1 | 1 |
| 52 | 1 | 1 | 1 | 1 | 1 | 0 |
| 53 | 1 | 1 | 1 | 1 | 1 | 0 |
| 54 | 1 | 1 | 1 | 1 | 1 | 0 |
| 55 | 1 | 1 | 1 | 1 | 1 | 1 |
| 56 | 1 | 1 | 1 | 1 | 1 | 1 |
| 57 | 1 | 1 | 1 | 1 | 1 | 1 |
| 58 | 1 | 0 | 0 | 0 | 1 | 0 |
| 59 | 1 | 0 | 0 | 0 | 1 | 0 |
| 60 | 1 | 0 | 0 | 0 | 1 | 0 |
| 61 | 1 | 1 | 1 | 1 | 1 | 1 |
| 62 | 1 | 1 | 1 | 1 | 1 | 1 |
| 63 | 1 | 1 | 1 | 1 | 1 | 1 |
| 64 | 1 | 1 | 1 | 1 | 1 | 1 |
| 65 | 1 | 1 | 1 | 1 | 1 | 1 |
| 66 | 1 | 1 | 1 | 1 | 1 | 1 |

Les échantillons Ech96 à Ech101 comportent au moins un peptide caractéristique des protéines ACC. Les bactéries présentes dans les échantillons Ech96 à Ech101 expriment donc une béta-lactamase qui leur confère une résistance aux pénicillines et aux céphalosporines excepté les céphalosporines de quatrième génération.

### Exemple 26 : Identification d'une résistance aux béta-lactamines de type CMY:

Les échantillons Ech102 à Ech108 sont identifiés selon l'une des méthodes décrites dans les exemples 1, 3 ou 4. L'identification des espèces est reportée dans le **TABLEAU 34.**

**TABLEAU 34 :**

| Noms | Espèces |
|---|---|
| Ech102 | *P. mirabilis* |
| Ech103 | *S*. *senftenberg* |
| Ech104 | *P. mirabilis* |
| Ech105 | *K. oxytoca* |
| Ech106 | *E. coli* |
| Ech107 | *S. enterica ssp enterica* |
| Ech108 | *P. mirabilis* |

Les échantillons Ech102 à Ech108 correspondent à une espèce pouvant comporter un mécanisme de résistance de type CMY. Chaque échantillon est traité selon l'exemple 5, puis analysé selon l'exemple 6 en détectant les peptides du **TABLEAU 35** en lieu et place des peptides du **TABLEAU 3.**

**TABLEAU 35 :**

| Transition numéro | Peptide | Temps de rétention (minutes) | (m/z) filtré en Q1 | (m/z) filtré en Q3 | Energie de collision (eV) | Seuil de positivité |
|---|---|---|---|---|---|---|
| 1 | AALLHFYQNWQPQWTPGAK | 22,2 | 752,72 | 372,22 | 30,4 | 2500 |
| 2 | AALLHFYQNWQPQWTPGAK | 22,1 | 752,72 | 442,74 | 30,4 | 2500 |
| 3 | AALLHFYQNWQPQWTPGAK | 22,2 | 752,72 | 884,46 | 30,4 | 2500 |
| 4 | ADIANNHPVTQQTLFELGSVSK | 19,9 | 790,41 | 427,27 | 31,6 | 2500 |
| 5 | ADIANNHPVTQQTLFELGSVSK | 19,9 | 790,41 | 719,39 | 31,6 | 2500 |
| 6 | ADIANNHPVTQQTLFELGSVSK | 19,9 | 790,41 | 866,46 | 31,6 | 2500 |
| 7 | ADSIINGSDSK | 11,3 | 553,77 | 493,23 | 27,3 | 2500 |
| 8 | ADSIINGSDSK | 11,3 | 553,77 | 607,27 | 27,3 | 2500 |
| 9 | ADSIINGSDSK | 11,3 | 553,77 | 720,35 | 27,3 | 2500 |
| 10 | ANIGGVDDK | 10 | 444,73 | 533,26 | 21,1 | 2500 |
| 11 | ANIGGVDDK | 10 | 444,73 | 590,28 | 21,1 | 2500 |
| 12 | ANIGGVDDK | 10,1 | 444,73 | 703,36 | 21,1 | 2500 |
| 13 | ASWVHK | 9,3 | 364,2 | 383,24 | 16,5 | 2500 |
| 14 | ASWVHK | 9,3 | 364,2 | 569,32 | 16,5 | 2500 |
| 15 | ASWVHK | 9,3 | 364,2 | 656,35 | 16,5 | 2500 |
| 16 | DYAWGYR | 15,6 | 465,71 | 395,2 | 22,3 | 2500 |
| 17 | DYAWGYR | 15,6 | 465,71 | 581,28 | 22,3 | 2500 |
| 18 | DYAWGYR | 15,6 | 465,71 | 652,32 | 22,3 | 2500 |
| 19 | ESGSQVLFNK | 14,5 | 554,79 | 408,22 | 27,4 | 2500 |
| 20 | ESGSQVLFNK | 14,5 | 554,79 | 521,31 | 27,4 | 2500 |
| 21 | ESGSQVLFNK | 14,5 | 554,79 | 620,38 | 27,4 | 2500 |
| 22 | GAMQLDDK | 11,4 | 439,21 | 490,25 | 20,8 | 2500 |
| 23 | GAMQLDDK | 11,4 | 439,21 | 618,31 | 20,8 | 2500 |
| 24 | GAMQLDDK | 11,4 | 439,21 | 749,35 | 20,8 | 2500 |
| 25 | GIGIVMLANR | 19,9 | 522,31 | 604,32 | 25,5 | 2500 |
| 26 | GIGIVMLANR | 19,9 | 522,31 | 703,39 | 25,5 | 2500 |
| 27 | GIGIVMLANR | 19,9 | 522,31 | 873,5 | 25,5 | 2500 |
| 28 | IGDMYQGLGWEMLNWPLK | 27,9 | 717,69 | 357,25 | 29,3 | 2500 |
| 29 | IGDMYQGLGWEMLNWPLK | 27,9 | 717,69 | 657,37 | 29,3 | 2500 |
| 30 | IGDMYQGLGWEMLNWPLK | 27,9 | 717,69 | 901,5 | 29,3 | 2500 |
| 31 | IPGMAVAVLK | 19,3 | 499,81 | 600,41 | 24,2 | 2500 |
| 32 | IPGMAVAVLK | 19,3 | 499,81 | 788,47 | 24,2 | 2500 |
| 33 | IPGMAVAVLK | 19,3 | 499,81 | 885,52 | 24,2 | 2500 |
| 34 | LAHTWITVPQNEQK | 16,5 | 832,94 | 743,37 | 43,2 | 2500 |
| 35 | LAHTWITVPQNEQK | 16,5 | 832,94 | 943,48 | 43,2 | 2500 |
| 36 | LAHTWITVPQNEQK | 16,5 | 832,94 | 1242,65 | 43,2 | 2500 |
| 37 | LLHLATYTAGGLPLQIPDDVR | 22,8 | 755,09 | 526,79 | 30,5 | 2500 |
| 38 | LLHLATYTAGGLPLQIPDDVR | 23 | 755,09 | 601,29 | 30,5 | 2500 |
| 39 | LLHLATYTAGGLPLQIPDDVR | 23 | 755,09 | 1052,57 | 30,5 | 2500 |
| 40 | LSDPVTK | 11,1 | 380,22 | 444,28 | 17,4 | 2500 |
| 41 | LSDPVTK | 11,1 | 380,22 | 559,31 | 17,4 | 2500 |
| 42 | LSDPVTK | 11,1 | 380,22 | 646,34 | 17,4 | 2500 |
| 43 | NLGIVMLANK | 19,6 | 536,81 | 576,32 | 26,3 | 2500 |
| 44 | NLGIVMLANK | 19,6 | 536,81 | 675,39 | 26,3 | 2500 |
| 45 | NLGIVMLANK | 19,6 | 536,81 | 845,49 | 26,3 | 2500 |
| 46 | QAMASYAYGYSK | 14,1 | 670,3 | 454,23 | 33,9 | 2500 |
| 47 | QAMASYAYGYSK | 14,2 | 670,3 | 617,29 | 33,9 | 2500 |
| 48 | QAMASYAYGYSK | 14,1 | 670,3 | 688,33 | 33,9 | 2500 |
| 49 | QWAPVYSPGSHR | 14,7 | 692,84 | 553,28 | 35,2 | 2500 |
| 50 | QWAPVYSPGSHR | 14,8 | 692,84 | 640,32 | 35,2 | 2500 |
| 51 | QWAPVYSPGSHR | 14,8 | 692,84 | 999,5 | 35,2 | 2500 |
| 52 | QWQGIR | 13,1 | 394,21 | 345,22 | 18,2 | 2500 |
| 53 | QWQGIR | 13,1 | 394,21 | 473,28 | 18,2 | 2500 |
| 54 | QWQGIR | 13,1 | 394,21 | 659,36 | 18,2 | 2500 |
| 55 | SSVIDMAR | 14,3 | 439,72 | 492,22 | 20,8 | 2500 |
| 56 | SSVIDMAR | 14,3 | 439,72 | 605,31 | 20,8 | 2500 |
| 57 | SSVIDMAR | 14,3 | 439,72 | 704,38 | 20,8 | 2500 |
| 58 | SYPNPVR | 11,6 | 416,72 | 371,24 | 19,5 | 2500 |
| 59 | SYPNPVR | 11,6 | 416,72 | 485,28 | 19,5 | 2500 |
| 60 | SYPNPVR | 11,6 | 416,72 | 582,34 | 19,5 | 2500 |
| 61 | TEQQIADIVNR | 15,8 | 643,84 | 616,34 | 32,4 | 2500 |
| 62 | TEQQIADIVNR | 15,8 | 643,84 | 687,38 | 32,4 | 2500 |
| 63 | TEQQIADIVNR | 15,8 | 643,84 | 800,46 | 32,4 | 2500 |
| 64 | TFNGVLGGDAIAR | 17,9 | 645,84 | 602,33 | 32,5 | 2500 |
| 65 | TFNGVLGGDAIAR | 17,9 | 645,84 | 659,35 | 32,5 | 2500 |
| 66 | TFNGVLGGDAIAR | 17,9 | 645,84 | 772,43 | 32,5 | 2500 |
| 67 | TITPLMQEQAIPGMAVAVIYQGK | 24,9 | 820,44 | 617,34 | 32,5 | 2500 |
| 68 | TITPLMQEQAIPGMAVAVIYQGK | 25 | 820,44 | 778,45 | 32,5 | 2500 |
| 69 | TITPLMQEQAIPGMAVAVIYQGK | 24,9 | 820,44 | 1233,67 | 32,5 | 2500 |
| 70 | TLQQGIALAQSR | 15,5 | 643,37 | 574,33 | 32,4 | 2500 |
| 71 | TLQQGIALAQSR | 15,5 | 643,37 | 645,37 | 32,4 | 2500 |
| 72 | TLQQGIALAQSR | 15,5 | 643,37 | 815,47 | 32,4 | 2500 |
| 73 | TLTATLGAYAVVK | 19,7 | 654,38 | 707,41 | 33 | 2500 |
| 74 | TLTATLGAYAVVK | 19,7 | 654,38 | 820,49 | 33 | 2500 |
| 75 | TLTATLGAYAVVK | 19,7 | 654,38 | 921,54 | 33 | 2500 |
| 76 | VALAALPAVEVNPPAPAVK | 21,1 | 609,7 | 644,87 | 26 | 2500 |
| 77 | VALAALPAVEVNPPAPAVK | 21 | 609,7 | 679,41 | 26 | 2500 |
| 78 | VALAALPAVEVNPPAPAVK | 21 | 609,7 | 793,46 | 26 | 2500 |
| 79 | VEAAWR | 12 | 366,2 | 432,24 | 16,6 | 2500 |
| 80 | VEAAWR | 12 | 366,2 | 503,27 | 16,6 | 2500 |
| 81 | VEAAWR | 12 | 366,2 | 632,32 | 16,6 | 2500 |
| 82 | VLQPLK | 13,1 | 349,23 | 357,25 | 15,6 | 2500 |
| 83 | VLQPLK | 13,1 | 349,23 | 485,31 | 15,6 | 2500 |
| 84 | VLQPLK | 13 | 349,23 | 598,39 | 15,6 | 2500 |
| 85 | VNPGMLADEAYGIK | 18,8 | 739,37 | 632,82 | 37,9 | 2500 |
| 86 | VNPGMLADEAYGIK | 18,9 | 739,37 | 795,39 | 37,9 | 2500 |
| 87 | VNPGMLADEAYGIK | 18,9 | 739,37 | 866,43 | 37,9 | 2500 |
| 88 | WVQANMDASHVQEK | 13,4 | 548,26 | 615,28 | 24,1 | 2500 |
| 89 | WVQANMDASHVQEK | 13,4 | 548,26 | 679,31 | 24,1 | 2500 |
| 90 | WVQANMDASHVQEK | 13,4 | 548,26 | 728,85 | 24,1 | 2500 |
| 91 | YWPELTGK | 17,8 | 497,26 | 305,18 | 24,1 | 2500 |
| 92 | YWPELTGK | 17,8 | 497,26 | 418,27 | 24,1 | 2500 |
| 93 | YWPELTGK | 17,8 | 497,26 | 644,36 | 24,1 | 2500 |

Les autres paramètres machine utilisés sont les suivants :
- Type de balayage: MRM
- MRM planifié : oui
- Polarité: Positive
- Source d'ionisation:Turbo V™ (Applied BioSystems)
- Réglage Q1: Filtrage avec résolution unitaire
- Réglage Q3: Filtrage avec résolution unitaire
- Pause inter-scan: 5.00 msec
- Vitesse de balayage: 10 Da/s
- Gaz rideau: 50,00 psi
- Tension de cône: 5500,00 V
- Température de source: 500,00 °C
- Gaz de nébulisation: 50,00 psi
- Gaz chauffant: 50,00 psi
- Gaz de collision induisant dissociation : 9,00 psi
- Remplissage dynamique: activé
- Potentiel d'orifice : 100, 00 V
- (en anglais declustering potential (DP))
- Potentiel d'entrée avant Q0 (EP): 6,00 V
- Potentiel en sortie de cellule de collision : 15 V
- (en anglais cell exit potential (CXP))
- Temps de cycle total: 0,04 sec
- Fenêtre de détection : 120 sec

Les aires obtenues pour chacune des transitions et pour chacun des microorganismes étudiés ont été mesurées. Lorsque les aires des transitions sont supérieures ou égales au seuil de positivité décrit dans le **TABLEAU 35,** la détection de la transition est considérée comme positive et est notée « 1 » dans le **TABLEAU 36.** Lorsqu'une transition a une aire inférieure au seuil de positivité décrit dans le **TABLEAU 35,** la transition est considérée comme non détectée et est notée « 0 » dans le **TABLEAU 36.**

Pour un peptide donné, lorsque au moins 3 transitions sont notées « 1 », le peptide est considéré comme détecté.

**TABLEAU 36 :**

| Transition numéro | Ech102 | Ech103 | Ech104 | Ech105 | Ech106 | Ech107 | Ech108 |
|---|---|---|---|---|---|---|---|
| 1 | 0 | 0 | 0 | 1 | 0 | 0 | 1 |
| 2 | 0 | 0 | 0 | 1 | 0 | 0 | 1 |
| 3 | 0 | 0 | 0 | 1 | 0 | 0 | 1 |
| 4 | 0 | 0 | 0 | 0 | 1 | 0 | 0 |
| 5 | 0 | 0 | 0 | 0 | 1 | 0 | 0 |
| 6 | 0 | 0 | 0 | 0 | 1 | 0 | 0 |
| 7 | 1 | 1 | 1 | 1 | 1 | 1 | 1 |
| 8 | 1 | 1 | 1 | 1 | 1 | 1 | 1 |
| 9 | 1 | 1 | 1 | 1 | 1 | 1 | 1 |
| 10 | 0 | 0 | 0 | 0 | 0 | 0 | 0 |
| 11 | 0 | 0 | 0 | 0 | 0 | 0 | 0 |
| 12 | 0 | 0 | 0 | 0 | 0 | 0 | 0 |
| 13 | 1 | 1 | 1 | 1 | 1 | 1 | 1 |
| 14 | 1 | 1 | 1 | 1 | 1 | 1 | 1 |
| 15 | 1 | 1 | 1 | 1 | 1 | 1 | 1 |
| 16 | 0 | 1 | 0 | 1 | 1 | 1 | 0 |
| 17 | 0 | 1 | 0 | 1 | 1 | 1 | 0 |
| 18 | 0 | 1 | 0 | 1 | 1 | 1 | 0 |
| 19 | 0 | 0 | 0 | 1 | 0 | 0 | 0 |
| 20 | 0 | 0 | 0 | 1 | 0 | 0 | 0 |
| 21 | 0 | 0 | 0 | 1 | 0 | 0 | 0 |
| 22 | 0 | 0 | 0 | 0 | 0 | 0 | 1 |
| 23 | 0 | 0 | 0 | 0 | 0 | 0 | 1 |
| 24 | 0 | 0 | 0 | 0 | 0 | 0 | 1 |
| 25 | 0 | 0 | 0 | 0 | 0 | 0 | 0 |
| 26 | 0 | 0 | 0 | 0 | 0 | 0 | 0 |
| 27 | 0 | 0 | 0 | 0 | 0 | 0 | 0 |
| 28 | 0 | 0 | 0 | 1 | 0 | 0 | 0 |
| 29 | 0 | 0 | 0 | 1 | 0 | 0 | 0 |
| 30 | 0 | 0 | 0 | 1 | 0 | 0 | 0 |
| 31 | 0 | 0 | 0 | 0 | 0 | 0 | 0 |
| 32 | 0 | 0 | 0 | 0 | 0 | 0 | 0 |
| 33 | 0 | 0 | 0 | 0 | 0 | 0 | 0 |
| 34 | 1 | 1 | 1 | 1 | 1 | 1 | 1 |
| 35 | 1 | 1 | 1 | 1 | 1 | 1 | 1 |
| 36 | 1 | 1 | 1 | 1 | 1 | 1 | 1 |
| 37 | 0 | 0 | 0 | 0 | 0 | 0 | 0 |
| 38 | 0 | 0 | 0 | 0 | 0 | 0 | 0 |
| 39 | 0 | 0 | 0 | 0 | 0 | 0 | 0 |
| 40 | 1 | 1 | 1 | 1 | 0 | 1 | 1 |
| 41 | 1 | 1 | 1 | 1 | 0 | 1 | 1 |
| 42 | 1 | 1 | 1 | 1 | 0 | 1 | 1 |
| 43 | 1 | 1 | 1 | 1 | 1 | 1 | 1 |
| 44 | 1 | 1 | 1 | 1 | 1 | 1 | 1 |
| 45 | 1 | 1 | 1 | 1 | 1 | 1 | 1 |
| 46 | 0 | 0 | 0 | 0 | 0 | 0 | 0 |
| 47 | 0 | 0 | 0 | 0 | 0 | 0 | 0 |
| 48 | 0 | 0 | 0 | 0 | 0 | 0 | 0 |
| 49 | 0 | 0 | 0 | 0 | 0 | 0 | 0 |
| 50 | 0 | 0 | 0 | 0 | 0 | 0 | 0 |
| 51 | 0 | 0 | 0 | 0 | 0 | 0 | 0 |
| 52 | 1 | 1 | 1 | 1 | 1 | 1 | 1 |
| 53 | 1 | 1 | 1 | 1 | 1 | 1 | 1 |
| 54 | 1 | 1 | 1 | 1 | 1 | 1 | 1 |
| 55 | 1 | 1 | 1 | 1 | 1 | 1 | 1 |
| 56 | 1 | 1 | 1 | 1 | 1 | 1 | 1 |
| 57 | 1 | 1 | 1 | 1 | 1 | 1 | 1 |
| 58 | 0 | 1 | 1 | 1 | 1 | 0 | 1 |
| 59 | 0 | 1 | 1 | 1 | 1 | 0 | 1 |
| 60 | 0 | 1 | 1 | 1 | 1 | 0 | 1 |
| 61 | 1 | 1 | 1 | 1 | 1 | 1 | 1 |
| 62 | 1 | 1 | 1 | 1 | 1 | 1 | 1 |
| 63 | 1 | 1 | 1 | 1 | 1 | 1 | 1 |
| 64 | 1 | 1 | 1 | 1 | 1 | 1 | 1 |
| 65 | 1 | 1 | 1 | 1 | 1 | 1 | 1 |
| 66 | 1 | 1 | 1 | 1 | 1 | 1 | 1 |
| 67 | 1 | 0 | 0 | 0 | 0 | 1 | 0 |
| 68 | 1 | 0 | 0 | 0 | 0 | 1 | 0 |
| 69 | 1 | 0 | 0 | 0 | 0 | 1 | 0 |
| 70 | 1 | 1 | 1 | 1 | 1 | 1 | 1 |
| 71 | 1 | 1 | 1 | 1 | 1 | 1 | 1 |
| 72 | 1 | 1 | 1 | 1 | 1 | 1 | 1 |
| 73 | 0 | 0 | 0 | 0 | 0 | 0 | 0 |
| 74 | 0 | 0 | 0 | 0 | 0 | 0 | 0 |
| 75 | 0 | 0 | 0 | 0 | 0 | 0 | 0 |
| 76 | 1 | 1 | 1 | 1 | 1 | 1 | 1 |
| 77 | 1 | 1 | 1 | 1 | 1 | 1 | 1 |
| 78 | 1 | 1 | 1 | 1 | 1 | 1 | 1 |
| 79 | 1 | 1 | 1 | 1 | 1 | 1 | 1 |
| 80 | 1 | 1 | 1 | 1 | 1 | 1 | 1 |
| 81 | 1 | 1 | 1 | 1 | 1 | 1 | 1 |
| 82 | 1 | 1 | 1 | 1 | 1 | 1 | 1 |
| 83 | 1 | 1 | 1 | 1 | 1 | 1 | 1 |
| 84 | 1 | 1 | 1 | 1 | 1 | 1 | 1 |
| 85 | 0 | 0 | 1 | 0 | 1 | 1 | 1 |
| 86 | 0 | 0 | 1 | 0 | 1 | 1 | 1 |
| 87 | 0 | 0 | 1 | 0 | 1 | 1 | 1 |
| 88 | 1 | 1 | 1 | 1 | 1 | 1 | 1 |
| 89 | 1 | 1 | 1 | 1 | 1 | 1 | 1 |
| 90 | 1 | 1 | 1 | 1 | 1 | 1 | 1 |
| 91 | 1 | 1 | 1 | 1 | 0 | 1 | 1 |
| 92 | 1 | 1 | 1 | 1 | 0 | 1 | 1 |
| 93 | 1 | 1 | 1 | 1 | 0 | 1 | 1 |

Les échantillons Ech102 à Ech108 comportent au moins un peptide caractéristique des protéines CMY. Les bactéries présentes dans les échantillons Ech102 à Ech108 expriment donc une béta-lactamase qui leur confère une résistance aux pénicillines et aux céphalosporines excepté les céphalosporines de quatrième génération.

### Exemple 27 : Identification d'une résistance aux béta-lactamines de type CTX-M:

Les échantillons Ech109 à Ech118 sont identifiés selon l'une des méthodes décrites dans les exemples 1, 3 ou 4. L'identification des espèces est reportée dans le **TABLEAU 37.**

**TABLEAU 37 :**

| Noms | Espèces |
|---|---|
| Ech109 | *E. aerogenes* |
| Ech110 | *E. coli* |
| Ech111 | *E. coli* |
| Ech112 | *E. coli* |
| Ech113 | *E. coli* |
| Ech114 | *K. pneumoniae* |
| Ech115 | *K. pneumoniae* |
| Ech116 | *P. mirabilis* |
| Ech117 | *Salmonella spp* |
| Ech118 | *Salmonella spp* |

Les échantillons Ech109 à Ech118 correspondent à une espèce pouvant comporter un mécanisme de résistance de type CTX-M. Chaque échantillon est traité selon l'exemple 5, puis analysé selon l'exemple 6 en détectant les peptides du **TABLEAU 38** en lieu et place des peptides du **TABLEAU 3.**

**TABLEAU 38 :**

| Transition numéro | Peptide | Etat de charge du précurseur | Etat de charge du fragment | Ion fragment | Temps de rétention (minutes) | (m/z) filtré en Q1 | (m/z) filtré en Q3 | Energie de collision (eV) | Seuil de positivité |
|---|---|---|---|---|---|---|---|---|---|
| 1 | AGLPK | 2 | 1 | y4 | 8,4 | 243,16 | 414,27 | 9,6 | 3000 |
| 2 | AGLPK | 2 | 1 | y3 | 8,4 | 243,16 | 357,25 | 9,6 | 3000 |
| 3 | AGLPK | 2 | 2 | y3 | 8,4 | 243,16 | 179,13 | 9,6 | 3000 |
| 4 | AGLPTSWTVGDK | 2 | 1 | y9 | 18 | 616, 32 | 990,49 | 30,9 | 2000 |
| 5 | AGLPTSWTVGDK | 2 | 1 | y7 | 18 | 616, 32 | 792,39 | 30,9 | 2000 |
| 6 | AGLPTSWTVGDK | 2 | 2 | y9 | 17,9 | 616,32 | 495,75 | 30,9 | 2000 |
| 7 | AIGDETFR | 2 | 1 | y6 | 13,5 | 454,73 | 724,33 | 21,7 | 2000 |
| 8 | AIGDETFR | 2 | 1 | y5 | 13,5 | 454,73 | 667,31 | 21,7 | 2000 |
| 9 | AIGDETFR | 2 | 1 | y4 | 13,5 | 454,73 | 552,28 | 21,7 | 2000 |
| 10 | ALAETQR | 2 | 1 | y5 | 7,5 | 394,72 | 604,31 | 18,2 | 2000 |
| 11 | ALAETQR | 2 | 1 | y4 | 7,5 | 394,72 | 533,27 | 18,2 | 2000 |
| 12 | ALAETQR | 2 | 2 | y6 | 7,5 | 394,72 | 359,2 | 18,2 | 2000 |
| 13 | ALGDSQR | 2 | 1 | y5 | 3,5 | 373,69 | 562,26 | 17 | 2000 |
| 14 | ALGDSQR | 2 | 1 | y3 | 3,6 | 373,69 | 390,21 | 17 | 2000 |
| 15 | ALGDSQR | 2 | 2 | y6 | 3,5 | 373,69 | 338,18 | 17 | 2000 |
| 16 | AMAQTLR | 2 | 1 | y5 | 10,7 | 395,72 | 588,35 | 18,3 | 2000 |
| 17 | AMAQTLR | 2 | 1 | y4 | 10,7 | 395,72 | 517,31 | 18,3 | 2000 |
| 18 | AMAQTLR | 2 | 1 | y3 | 10,7 | 395,72 | 389,25 | 18,3 | 2000 |
| 19 | APLILVTYFTQPQPK | 2 | 1 | y10 | 24,6 | 858,49 | 1208,63 | 44,7 | 2000 |
| 20 | APLILVTYFTQPQPK | 3 | 1 | y6 | 24,6 | 572,66 | 698,38 | 24,8 | 2000 |
| 21 | APLILVTYFTQPQPK | 3 | 1 | y4 | 24,6 | 572,66 | 469,28 | 24,8 | 2000 |
| 22 | APLVLVTYFTQPQQNAESR | 3 | 1 | y8 | 23,6 | 721,38 | 929,44 | 29,4 | 2000 |
| 23 | APLVLVTYFTQPQQNAESR | 3 | 1 | y6 | 23,6 | 721,38 | 704,33 | 29,4 | 2000 |
| 24 | APLVLVTYFTQPQQNAESR | 3 | 2 | y8 | 23,6 | 721,38 | 465,23 | 29,4 | 2000 |
| 25 | AQLVTWLK | 2 | 1 | y6 | 20 | 479,79 | 759,48 | 23,1 | 2000 |
| 26 | AQLVTWLK | 2 | 1 | y5 | 20 | 479,79 | 646,39 | 23,1 | 2000 |
| 27 | AQLVTWLK | 2 | 1 | y4 | 20 | 479,79 | 547,32 | 23,1 | 2000 |
| 28 | AQLVTWMK | 2 | 1 | y6 | 18,5 | 488,77 | 777,43 | 23,6 | 2000 |
| 29 | AQLVTWMK | 2 | 1 | y5 | 18,6 | 488,77 | 664,35 | 23,6 | 2000 |
| 30 | AQLVTWMK | 2 | 1 | y4 | 18,5 | 488,77 | 565,28 | 23,6 | 2000 |
| 31 | DILAAAAK | 2 | 1 | y6 | 14,1 | 386,73 | 544,35 | 17,8 | 2000 |
| 32 | DILAAAAK | 2 | 1 | y5 | 14,1 | 386,73 | 431,26 | 17,8 | 2000 |
| 33 | DILAAAAK | 2 | 1 | y4 | 14,1 | 386,73 | 360,22 | 17,8 | 2000 |
| 34 | DTTSPR | 2 | 1 | y5 | 1 | 338,67 | 561,3 | 15 | 2000 |
| 35 | DTTSPR | 2 | 1 | y4 | 1 | 338,67 | 460,25 | 15 | 2000 |
| 36 | DTTSPR | 2 | 1 | y3 | 1 | 338,67 | 359,2 | 15 | 2000 |
| 37 | DTTTPLAMAQTLK | 2 | 1 | y9 | 19,4 | 695,87 | 972,56 | 35,4 | 2000 |
| 38 | DTTTPLAMAQTLK | 2 | 1 | y7 | 19,4 | 695,87 | 762,42 | 35,4 | 2000 |
| 39 | DTTTPLAMAQTLK | 2 | 2 | y9 | 19,4 | 695,87 | 486,78 | 35,4 | 2000 |
| 40 | DTTTPR | 2 | 1 | y5 | 1 | 345,67 | 575,32 | 15,4 | 2000 |
| 41 | DTTTPR | 2 | 1 | y4 | 1 | 345,67 | 474,27 | 15,4 | 2000 |
| 42 | DTTTPR | 2 | 1 | y3 | 1 | 345,67 | 373,22 | 15,4 | 2000 |
| 43 | DVLAAAAK | 2 | 1 | y6 | 12,2 | 379,72 | 544,35 | 17,4 | 2000 |
| 44 | DVLAAAAK | 2 | 1 | y5 | 12,2 | 379,72 | 431,26 | 17,4 | 2000 |
| 45 | DVLAAAAK | 2 | 1 | y4 | 12,2 | 379,72 | 360,22 | 17,4 | 2000 |
| 46 | DVLASAAK | 2 | 1 | y6 | 11,4 | 387,72 | 560,34 | 17,8 | 2000 |
| 47 | DVLASAAK | 2 | 1 | y5 | 11,4 | 387,72 | 447,26 | 17,8 | 2000 |
| 48 | DVLASAAK | 2 | 1 | y4 | 11,4 | 387,72 | 376,22 | 17,8 | 2000 |
| 49 | DVLASAAR | 2 | 1 | y6 | 11,9 | 401,73 | 588,35 | 18,6 | 2000 |
| 50 | DVLASAAR | 2 | 1 | y5 | 11,9 | 401,73 | 475,26 | 18,6 | 2000 |
| 51 | DVLASAAR | 2 | 1 | y4 | 11,9 | 401,73 | 404,23 | 18,6 | 2000 |
| 52 | FAMCSTSK | 2 | 1 | y7 | 11,6 | 466,2 | 784,33 | 22,3 | 2000 |
| 53 | FAMCSTSK | 2 | 1 | y6 | 11,6 | 466,2 | 713,3 | 22,3 | 2000 |
| 54 | FAMCSTSK | 2 | 1 | y5 | 11,6 | 466,2 | 582,26 | 22,3 | 2000 |
| 55 | FPMCSTSK | 2 | 1 | y7 | 12,3 | 479,21 | 810,35 | 23,1 | 2000 |
| 56 | FPMCSTSK | 2 | 1 | y6 | 12,3 | 479,21 | 713,3 | 23,1 | 2000 |
| 57 | FPMCSTSK | 2 | 2 | y7 | 12,3 | 479,21 | 405,68 | 23,1 | 2000 |
| 58 | GNTTGAASIQAGLPASWVVGDK | 3 | 1 | y9 | 21,1 | 700,7 | 958,5 | 29,1 | 2000 |
| 59 | GNTTGAASIQAGLPASWVVGDK | 3 | 1 | y7 | 21,1 | 700,7 | 790,41 | 29,1 | 2000 |
| 60 | GNTTGAASIQAGLPASWVVGDK | 3 | 2 | y9 | 21,2 | 700,7 | 479,75 | 29,1 | 2000 |
| 61 | GNTTGAASIQAGLPTSWVVGDK | 3 | 1 | y9 | 21,3 | 710,7 | 988,51 | 29,1 | 8500 |
| 62 | GNTTGAASIQAGLPTSWVVGDK | 3 | 2 | y9 | 21,3 | 710,7 | 494,76 | 29,1 | 8500 |
| 63 | GNTTGAASIQAGLPTSWVVGDK | 3 | 1 | y3 | 21,3 | 710,7 | 319,16 | 29,1 | 8500 |
| 64 | GNTTGAASIR | 2 | 1 | y7 | 9,3 | 474,25 | 675,38 | 22,8 | 2000 |
| 65 | GNTTGAASIR | 2 | 1 | y6 | 9,3 | 474,25 | 574,33 | 22,8 | 2000 |
| 66 | GNTTGAASIR | 2 | 1 | y4 | 9,3 | 474,25 | 446,27 | 22,8 | 2000 |
| 67 | GNTTGSASIR | 2 | 1 | y8 | 8 | 482,25 | 792,42 | 23,2 | 2000 |
| 68 | GNTTGSASIR | 2 | 1 | y7 | 8 | 482,25 | 691,37 | 23,2 | 2000 |
| 69 | GNTTGSASIR | 2 | 1 | y6 | 8 | 482,25 | 590,33 | 23,2 | 2000 |
| 70 | HLLNQR | 2 | 1 | y5 | 8,1 | 390,73 | 643,39 | 18 | 2000 |
| 71 | HLLNQR | 2 | 1 | y4 | 8,1 | 390,73 | 530,31 | 18 | 2000 |
| 72 | HLLNQR | 2 | 1 | y3 | 8,1 | 390,73 | 417,22 | 18 | 2000 |
| 73 | LAALEK | 2 | 1 | y5 | 11,3 | 322,7 | 531,31 | 14,1 | 2000 |
| 74 | LAALEK | 2 | 1 | y4 | 11,3 | 322,7 | 460,28 | 14,1 | 2000 |
| 75 | LAALEK | 2 | 1 | y3 | 11,3 | 322,7 | 389,24 | 14,1 | 2000 |
| 76 | LAELER | 2 | 1 | y5 | 11,7 | 365,71 | 617,33 | 16,6 | 2000 |
| 77 | LAELER | 2 | 1 | y4 | 11,7 | 365,71 | 546,29 | 16,6 | 2000 |
| 78 | LAELER | 2 | 1 | y3 | 11,7 | 365,71 | 417,25 | 16,6 | 2000 |
| 79 | LGVALIDTADNTQVLYR | 3 | 1 | y6 | 21,5 | 621,34 | 779,44 | 26,3 | 2000 |
| 80 | LGVALIDTADNTQVLYR | 3 | 1 | y5 | 21,5 | 621,34 | 678,39 | 26,3 | 2000 |
| 81 | LGVALIDTADNTQVLYR | 3 | 1 | y4 | 21,5 | 621,34 | 550,34 | 26,3 | 2000 |
| 82 | LGVALIDTADNTQVLYR | 3 | 1 | y3 | 21,4 | 621,34 | 451,27 | 26,3 | 2000 |
| 83 | LGVALINTADNSQILYR | 3 | 1 | y6 | 21,4 | 621,01 | 779,44 | 26,3 | 2000 |
| 84 | LGVALINTADNSQILYR | 3 | 1 | y5 | 21,4 | 621,01 | 692,41 | 26,3 | 2000 |
| 85 | LGVALINTADNSQILYR | 3 | 1 | y4 | 21,4 | 621,01 | 564,35 | 26,3 | 2000 |
| 86 | LGVALINTADNSQILYR | 3 | 1 | y3 | 21,4 | 621,01 | 451,27 | 26,3 | 2000 |
| 87 | LIAHLGGPDK | 3 | 1 | y6 | 13,2 | 340,87 | 586,32 | 17,6 | 2000 |
| 88 | LIAHLGGPDK | 3 | 2 | y9 | 13,2 | 340,87 | 454,25 | 17,6 | 2000 |
| 89 | LIAHLGGPDK | 3 | 2 | y8 | 13,2 | 340,87 | 397,71 | 17,6 | 2000 |
| 90 | LIAHVGGPASVTAFAR | 3 | 1 | y5 | 17,7 | 522,96 | 565,31 | 23,3 | 2000 |
| 91 | LIAHVGGPASVTAFAR | 3 | 1 | y4 | 17,7 | 522,96 | 464,26 | 23,3 | 2000 |
| 92 | LIAHVGGPASVTAFAR | 3 | 1 | y3 | 17,7 | 522,96 | 393,22 | 23,3 | 2000 |
| 93 | LIAQLGGPGGVTAFAR | 2 | 1 | y11 | 20,3 | 764,44 | 989,52 | 39,3 | 2000 |
| 94 | LIAQLGGPGGVTAFAR | 2 | 1 | y9 | 20,3 | 764,44 | 875,47 | 39,3 | 2000 |
| 95 | LIAQLGGPGGVTAFAR | 3 | 1 | y5 | 20,3 | 509,96 | 565,31 | 22,9 | 2000 |
| 96 | NLTLGK | 2 | 1 | y5 | 12,3 | 323,2 | 531,35 | 14,2 | 2000 |
| 97 | NLTLGK | 2 | 1 | y4 | 12,3 | 323,2 | 418,27 | 14,2 | 2000 |
| 98 | NLTLGK | 2 | 1 | y3 | 12,3 | 323,2 | 317,22 | 14,2 | 2000 |
| 99 | QLGDETFR | 2 | 1 | y6 | 13,6 | 483,24 | 724,33 | 23,3 | 2000 |
| 100 | QLGDETFR | 2 | 1 | y4 | 13,6 | 483,24 | 552,28 | 23,3 | 2000 |
| 101 | QLGDETFR | 2 | 1 | y3 | 13,6 | 483,24 | 423,24 | 23,3 | 2000 |
| 102 | QLTLGHALGETQR | 3 | 2 | y11 | 15,6 | 475,26 | 591,82 | 21,8 | 2000 |
| 103 | QLTLGHALGETQR | 3 | 1 | y5 | 15,6 | 475,26 | 590,29 | 21,8 | 2000 |
| 104 | QLTLGHALGETQR | 3 | 2 | y10 | 15,6 | 475,26 | 541,29 | 21,8 | 2000 |
| 105 | QSESDK | 2 | 1 | y5 | 0,8 | 347,16 | 565,25 | 15,5 | 2000 |
| 106 | QSESDK | 2 | 1 | y4 | 0,8 | 347,16 | 478,21 | 15,5 | 2000 |
| 107 | QSESDK | 2 | 1 | y3 | 0,8 | 347,16 | 349,17 | 15,5 | 2000 |
| 108 | QSETQK | 2 | 1 | y5 | 0,8 | 360,68 | 592,29 | 16,3 | 2000 |
| 109 | QSETQK | 2 | 1 | y4 | 0,8 | 360,68 | 505,26 | 16,3 | 2000 |
| 110 | QSETQK | 2 | 1 | y3 | 0,8 | 360,68 | 376,22 | 16,3 | 2000 |
| 111 | QSGGR | 2 | 1 | y4 | 0,7 | 252,63 | 376,19 | 10,1 | 2000 |
| 112 | QSGGR | 2 | 1 | y3 | 0,7 | 252,63 | 289,16 | 10,1 | 2000 |
| 113 | QSGGR | 2 | 2 | y4 | 0,7 | 252,63 | 188,6 | 10,1 | 2000 |
| 114 | SDLVNYNPIAEK | 2 | 1 | y8 | 17,3 | 681,85 | 948,48 | 34,6 | 2000 |
| 115 | SDLVNYNPIAEK | 2 | 1 | y6 | 17,4 | 681,85 | 671,37 | 34,6 | 2000 |
| 116 | SDLVNYNPIAEK | 2 | 1 | y5 | 17,4 | 681,85 | 557,33 | 34,6 | 2000 |
| 117 | SESEPNLLNQR | 2 | 1 | y7 | 14,6 | 643,82 | 854,48 | 32,4 | 2000 |
| 118 | SESEPNLLNQR | 2 | 2 | y7 | 14,6 | 643,82 | 427,75 | 32,4 | 2000 |
| 119 | SESEPNLLNQR | 3 | 1 | y3 | 14,6 | 429,55 | 417,22 | 20,4 | 2000 |
| 120 | SLGDETFR | 2 | 1 | y6 | 13,9 | 462,73 | 724,33 | 22,1 | 2000 |
| 121 | SLGDETFR | 2 | 1 | y5 | 13,8 | 462,73 | 667,31 | 22,1 | 2000 |
| 122 | SLGDETFR | 2 | 1 | y4 | 13,9 | 462,73 | 552,28 | 22,1 | 2000 |
| 123 | SSGGR | 2 | 1 | y4 | 0,7 | 232,12 | 376,19 | 9 | 2000 |
| 124 | SSGGR | 2 | 1 | y3 | 0,7 | 232,12 | 289,16 | 9 | 2000 |
| 125 | SSGGR | 2 | 2 | y4 | 0,7 | 232,12 | 188,6 | 9 | 2000 |
| 126 | SWVVGDK | 2 | 1 | y5 | 13,9 | 395,71 | 517,3 | 18,3 | 2000 |
| 127 | SWVVGDK | 2 | 1 | y4 | 13,9 | 395,71 | 418,23 | 18,3 | 2000 |
| 128 | SWVVGDK | 2 | 1 | y3 | 13,9 | 395,71 | 319,16 | 18,3 | 2000 |
| 129 | TEPTLNTAIPGDPR | 2 | 2 | y12 | 16,3 | 741,38 | 626,34 | 38 | 2000 |
| 130 | TEPTLNTAIPGDPR | 2 | 1 | y5 | 16,3 | 741,38 | 541,27 | 38 | 2000 |
| 131 | TEPTLNTAIPGDPR | 3 | 1 | y5 | 16,3 | 494,59 | 541,27 | 22,4 | 2000 |
| 132 | TGSGDYGTTNDIAVIWPK | 2 | 1 | y8 | 20,9 | 947,96 | 941,55 | 49,8 | 2000 |
| 133 | TGSGDYGTTNDIAVIWPK | 3 | 2 | y6 | 21 | 632,31 | 357,22 | 26,7 | 2000 |
| 134 | TGSGDYGTTNDIAVIWPK | 3 | 2 | y5 | 20,9 | 632,31 | 321,7 | 26,7 | 2000 |
| 135 | TGSGDYGTTNDIAVIWPQGR | 3 | 1 | y6 | 20,5 | 703,34 | 756,42 | 28,9 | 2000 |
| 136 | TGSGDYGTTNDIAVIWPQGR | 3 | 1 | y5 | 20,5 | 703,34 | 643,33 | 28,9 | 2000 |
| 137 | TGSGDYGTTNDIAVIWPQGR | 3 | 1 | y4 | 20,5 | 703,34 | 457,25 | 28,9 | 2000 |
| 138 | TGSGGYGTTNDIAVIWPK | 2 | 1 | y3 | 20,9 | 918,96 | 430,25 | 48,1 | 2000 |
| 139 | TGSGGYGTTNDIAVIWPK | 3 | 1 | y6 | 20,9 | 612,98 | 713,43 | 26,1 | 2000 |
| 140 | TGSGGYGTTNDIAVIWPK | 3 | 1 | y5 | 20,9 | 612,98 | 642,4 | 26,1 | 2000 |
| 141 | VMAAAAVLK | 2 | 1 | y8 | 15,7 | 437,27 | 774,45 | 20,7 | 2000 |
| 142 | VMAAAAVLK | 2 | 1 | y7 | 15,7 | 437,27 | 643,41 | 20,7 | 2000 |
| 143 | VMAAAAVLK | 2 | 1 | y6 | 15,7 | 437,27 | 572,38 | 20,7 | 2000 |
| 144 | VMAVAAVLK | 2 | 1 | y7 | 18,2 | 451,28 | 671,45 | 21,5 | 2000 |
| 145 | VMAVAAVLK | 2 | 1 | y6 | 18,2 | 451,28 | 600,41 | 21,5 | 2000 |
| 146 | VMAVAAVLK | 2 | 1 | y5 | 18,2 | 451,28 | 501,34 | 21,5 | 2000 |
| 147 | VTAFAR | 2 | 1 | y5 | 11 | 332,69 | 565,31 | 14,7 | 2000 |
| 148 | VTAFAR | 2 | 1 | y4 | 11 | 332,69 | 464,26 | 14,7 | 2000 |
| 149 | VTAFAR | 2 | 1 | y3 | 11 | 332,69 | 393,22 | 14,7 | 2000 |

Les autres paramètres machine utilisés sont les suivants :
- Type de balayage: MRM
- MRM planifié: oui
- Polarité: Positive
- Source d'ionisation:Turbo V™ (Applied BioSystems)
- Réglage Q1: Filtrage avec résolution unitaire
- Réglage Q3: Filtrage avec résolution unitaire
- Pause inter-scan: 5.00 msec
- Vitesse de balayage: 10 Da/s
- Gaz rideau: 50,00 psi
- Tension de cône: 5500,00 V
- Température de source: 500,00 °C
- Gaz de nébulisation: 50,00 psi
- Gaz chauffant: 50,00 psi
- Gaz de collision induisant dissociation : 9,00 psi
- Remplissage dynamique: activé
- Potentiel d'orifice : 100, 00 V
- (en anglais declustering potential (DP))
- Potentiel d'entrée avant Q0 (EP): 6,00 V
- Potentiel en sortie de cellule de collision : 15 V
- (en anglais cell exit potential (CXP))
- Temps de cycle total: 0,04 sec
- Fenêtre de détection : 120 sec

Les aires obtenues pour chacune des transitions et pour chacun des microorganismes étudiés ont été mesurées. Lorsque les aires des transitions sont supérieures ou égales au seuil de positivité décrit dans le TABLEAU 38, la détection de la transition est considérée comme positive et est notée « 1 » dans le TABLEAU 39. Lorsqu'une transition a une aire inférieure au seuil de positivité décrit dans le TABLEAU 38, la transition est considérée comme non détectée et est notée « 0 » dans le TABLEAU 39.

Pour un peptide donné, lorsque au moins 3 transitions sont notées « 1 », le peptide est considéré comme détecté.

**TABLEAU 39 :**

| Transition numéro | Ech109 | Ech110 | Ech111 | Ech112 | Ech113 | Ech114 | Ech115 | Ech116 | Ech117 | Ech118 |
|---|---|---|---|---|---|---|---|---|---|---|
| 1 | 0 | 0 | 0 | 0 | 0 | 0 | 0 | 0 | 1 | 0 |
| 2 | 1 | 1 | 0 | 0 | 1 | 0 | 0 | 0 | 1 | 1 |
| 3 | 1 | 1 | 0 | 0 | 1 | 0 | 0 | 0 | 1 | 1 |
| 4 | 0 | 0 | 0 | 0 | 0 | 0 | 0 | 1 | 0 | 0 |
| 5 | 0 | 0 | 0 | 0 | 0 | 0 | 0 | 0 | 0 | 0 |
| 6 | 0 | 0 | 0 | 0 | 0 | 0 | 0 | 1 | 0 | 0 |
| 7 | 0 | 1 | 0 | 0 | 1 | 0 | 0 | 0 | 0 | 0 |
| 8 | 0 | 0 | 0 | 0 | 0 | 0 | 0 | 0 | 0 | 0 |
| 9 | 0 | 0 | 0 | 0 | 0 | 0 | 0 | 0 | 0 | 0 |
| 10 | 0 | 0 | 0 | 0 | 0 | 0 | 0 | 0 | 0 | 0 |
| 11 | 0 | 0 | 0 | 0 | 0 | 0 | 0 | 0 | 0 | 0 |
| 12 | 0 | 0 | 0 | 0 | 0 | 0 | 0 | 0 | 0 | 0 |
| 13 | 0 | 0 | 0 | 0 | 0 | 0 | 0 | 0 | 0 | 0 |
| 14 | 0 | 0 | 0 | 0 | 0 | 0 | 0 | 0 | 0 | 0 |
| 15 | 0 | 0 | 0 | 0 | 0 | 0 | 0 | 0 | 0 | 0 |
| 16 | 1 | 1 | 1 | 1 | 1 | 0 | 1 | 1 | 0 | 1 |
| 17 | 1 | 1 | 1 | 1 | 1 | 0 | 1 | 1 | 0 | 1 |
| 18 | 1 | 1 | 1 | 1 | 1 | 0 | 1 | 1 | 0 | 1 |
| 19 | 0 | 0 | 0 | 0 | 0 | 0 | 0 | 0 | 0 | 0 |
| 20 | 0 | 0 | 0 | 0 | 0 | 0 | 0 | 0 | 0 | 0 |
| 21 | 0 | 0 | 0 | 0 | 0 | 0 | 0 | 0 | 0 | 0 |
| 22 | 0 | 0 | 0 | 0 | 0 | 0 | 0 | 0 | 0 | 0 |
| 23 | 0 | 0 | 0 | 0 | 0 | 0 | 0 | 0 | 0 | 0 |
| 24 | 0 | 0 | 0 | 0 | 0 | 0 | 0 | 0 | 0 | 0 |
| 25 | 1 | 1 | 0 | 0 | 1 | 0 | 1 | 0 | 1 | 0 |
| 26 | 1 | 1 | 1 | 0 | 1 | 1 | 1 | 1 | 1 | 0 |
| 27 | 1 | 1 | 0 | 0 | 1 | 0 | 1 | 0 | 1 | 0 |
| 28 | 0 | 0 | 0 | 0 | 0 | 0 | 0 | 0 | 0 | 0 |
| 29 | 0 | 0 | 0 | 0 | 0 | 0 | 0 | 0 | 0 | 0 |
| 30 | 1 | 1 | 0 | 0 | 1 | 0 | 1 | 0 | 0 | 0 |
| 31 | 0 | 1 | 1 | 0 | 1 | 0 | 0 | 0 | 1 | 1 |
| 32 | 0 | 0 | 0 | 0 | 0 | 0 | 0 | 0 | 0 | 0 |
| 33 | 0 | 0 | 0 | 0 | 0 | 0 | 0 | 0 | 0 | 0 |
| 34 | 0 | 0 | 0 | 0 | 0 | 0 | 0 | 0 | 0 | 0 |
| 35 | 0 | 0 | 0 | 0 | 0 | 0 | 0 | 0 | 0 | 0 |
| 36 | 0 | 0 | 0 | 0 | 0 | 0 | 0 | 0 | 0 | 0 |
| 37 | 0 | 0 | 0 | 0 | 0 | 0 | 0 | 1 | 1 | 0 |
| 38 | 0 | 0 | 0 | 0 | 0 | 0 | 0 | 1 | 1 | 0 |
| 39 | 0 | 0 | 0 | 0 | 0 | 0 | 0 | 0 | 1 | 0 |
| 40 | 0 | 0 | 0 | 0 | 1 | 0 | 0 | 0 | 0 | 0 |
| 41 | 0 | 0 | 0 | 0 | 1 | 0 | 1 | 0 | 0 | 1 |
| 42 | 0 | 0 | 0 | 0 | 0 | 0 | 0 | 0 | 0 | 1 |
| 43 | 1 | 0 | 0 | 0 | 0 | 0 | 0 | 0 | 1 | 0 |
| 44 | 1 | 0 | 0 | 0 | 0 | 0 | 0 | 0 | 1 | 0 |
| 45 | 1 | 0 | 0 | 0 | 0 | 0 | 0 | 0 | 1 | 0 |
| 46 | 1 | 0 | 0 | 0 | 0 | 0 | 0 | 0 | 0 | 1 |
| 47 | 1 | 0 | 0 | 0 | 0 | 0 | 0 | 0 | 0 | 1 |
| 48 | 0 | 0 | 0 | 0 | 1 | 0 | 0 | 0 | 0 | 1 |
| 49 | 0 | 1 | 0 | 0 | 1 | 0 | 1 | 0 | 0 | 0 |
| 50 | 0 | 1 | 0 | 0 | 1 | 0 | 1 | 0 | 0 | 0 |
| 51 | 0 | 1 | 0 | 0 | 1 | 0 | 1 | 0 | 0 | 0 |
| 52 | 1 | 0 | 1 | 1 | 0 | 1 | 0 | 1 | 1 | 1 |
| 53 | 1 | 0 | 1 | 1 | 0 | 1 | 0 | 1 | 1 | 1 |
| 54 | 1 | 0 | 1 | 1 | 1 | 1 | 1 | 1 | 1 | 1 |
| 55 | 0 | 0 | 0 | 0 | 1 | 0 | 0 | 0 | 0 | 0 |
| 56 | 0 | 0 | 0 | 0 | 1 | 0 | 0 | 0 | 0 | 0 |
| 57 | 0 | 0 | 0 | 0 | 1 | 0 | 0 | 0 | 0 | 0 |
| 58 | 0 | 0 | 0 | 0 | 0 | 0 | 0 | 0 | 0 | 1 |
| 59 | 0 | 0 | 0 | 0 | 0 | 0 | 0 | 0 | 0 | 0 |
| 60 | 0 | 0 | 0 | 0 | 0 | 0 | 0 | 0 | 0 | 1 |
| 61 | 1 | 0 | 0 | 0 | 0 | 0 | 0 | 0 | 0 | 0 |
| 62 | 1 | 0 | 0 | 0 | 0 | 0 | 0 | 0 | 0 | 0 |
| 63 | 1 | 0 | 0 | 0 | 0 | 0 | 0 | 0 | 0 | 0 |
| 64 | 0 | 1 | 0 | 0 | 1 | 1 | 1 | 0 | 0 | 0 |
| 65 | 0 | 1 | 0 | 0 | 1 | 1 | 1 | 0 | 0 | 0 |
| 66 | 0 | 1 | 0 | 0 | 1 | 1 | 1 | 0 | 0 | 0 |
| 67 | 0 | 0 | 0 | 0 | 0 | 0 | 0 | 0 | 1 | 0 |
| 68 | 0 | 0 | 0 | 0 | 0 | 0 | 0 | 0 | 1 | 0 |
| 69 | 0 | 0 | 0 | 0 | 0 | 0 | 0 | 0 | 1 | 0 |
| 70 | 0 | 0 | 0 | 0 | 0 | 0 | 0 | 0 | 0 | 0 |
| 71 | 0 | 0 | 0 | 0 | 0 | 1 | 0 | 0 | 1 | 0 |
| 72 | 0 | 0 | 0 | 0 | 0 | 0 | 0 | 0 | 1 | 0 |
| 73 | 1 | 1 | 0 | 0 | 1 | 1 | 0 | 0 | 0 | 1 |
| 74 | 1 | 1 | 0 | 1 | 1 | 1 | 1 | 0 | 0 | 0 |
| 75 | 1 | 1 | 0 | 0 | 1 | 1 | 1 | 0 | 0 | 1 |
| 76 | 0 | 0 | 1 | 1 | 0 | 0 | 0 | 1 | 0 | 1 |
| 77 | 0 | 0 | 1 | 1 | 0 | 0 | 0 | 1 | 0 | 1 |
| 78 | 0 | 0 | 1 | 1 | 0 | 0 | 0 | 1 | 0 | 1 |
| 79 | 0 | 0 | 0 | 0 | 0 | 0 | 0 | 0 | 0 | 0 |
| 80 | 0 | 0 | 0 | 0 | 0 | 0 | 0 | 0 | 0 | 0 |
| 81 | 0 | 0 | 0 | 0 | 0 | 0 | 0 | 0 | 0 | 0 |
| 82 | 0 | 0 | 0 | 0 | 0 | 0 | 0 | 0 | 0 | 0 |
| 83 | 0 | 0 | 0 | 0 | 0 | 0 | 0 | 0 | 0 | 0 |
| 84 | 0 | 0 | 0 | 0 | 0 | 0 | 0 | 0 | 0 | 0 |
| 85 | 0 | 0 | 0 | 0 | 0 | 0 | 0 | 0 | 0 | 0 |
| 86 | 0 | 0 | 0 | 0 | 0 | 0 | 0 | 0 | 0 | 0 |
| 87 | 1 | 0 | 0 | 0 | 0 | 1 | 0 | 0 | 1 | 0 |
| 88 | 1 | 0 | 0 | 0 | 0 | 1 | 0 | 0 | 1 | 0 |
| 89 | 1 | 0 | 0 | 0 | 0 | 1 | 1 | 0 | 1 | 0 |
| 90 | 0 | 0 | 0 | 0 | 0 | 1 | 0 | 0 | 0 | 0 |
| 91 | 0 | 0 | 0 | 0 | 0 | 1 | 1 | 0 | 0 | 0 |
| 92 | 0 | 0 | 0 | 0 | 0 | 1 | 1 | 0 | 0 | 0 |
| 93 | 0 | 0 | 0 | 0 | 0 | 0 | 0 | 0 | 0 | 0 |
| 94 | 0 | 0 | 0 | 0 | 0 | 0 | 0 | 1 | 0 | 0 |
| 95 | 0 | 0 | 0 | 0 | 0 | 0 | 0 | 0 | 0 | 0 |
| 96 | 0 | 0 | 0 | 0 | 0 | 0 | 0 | 0 | 0 | 0 |
| 97 | 0 | 0 | 0 | 0 | 0 | 0 | 0 | 0 | 0 | 0 |
| 98 | 0 | 0 | 0 | 0 | 0 | 0 | 0 | 0 | 0 | 0 |
| 99 | 0 | 0 | 0 | 0 | 0 | 0 | 0 | 0 | 0 | 0 |
| 100 | 0 | 0 | 0 | 0 | 0 | 0 | 0 | 0 | 1 | 0 |
| 101 | 0 | 0 | 0 | 0 | 0 | 0 | 0 | 0 | 0 | 0 |
| 102 | 0 | 1 | 0 | 0 | 1 | 0 | 1 | 0 | 0 | 0 |
| 103 | 0 | 1 | 0 | 0 | 1 | 1 | 1 | 1 | 0 | 1 |
| 104 | 0 | 1 | 0 | 0 | 1 | 1 | 1 | 0 | 0 | 0 |
| 105 | 0 | 0 | 0 | 0 | 0 | 0 | 0 | 0 | 0 | 0 |
| 106 | 0 | 0 | 0 | 0 | 0 | 0 | 0 | 0 | 0 | 0 |
| 107 | 0 | 0 | 0 | 0 | 0 | 0 | 0 | 0 | 0 | 0 |
| 108 | 0 | 0 | 0 | 0 | 0 | 0 | 0 | 0 | 0 | 0 |
| 109 | 0 | 0 | 0 | 0 | 0 | 0 | 0 | 0 | 0 | 0 |
| 110 | 0 | 0 | 0 | 0 | 0 | 0 | 0 | 0 | 0 | 0 |
| 111 | 0 | 0 | 0 | 0 | 0 | 0 | 0 | 0 | 0 | 0 |
| 112 | 0 | 0 | 0 | 0 | 0 | 0 | 0 | 0 | 0 | 0 |
| 113 | 0 | 0 | 0 | 0 | 0 | 0 | 0 | 0 | 0 | 0 |
| 114 | 0 | 0 | 1 | 1 | 0 | 0 | 0 | 1 | 1 | 1 |
| 115 | 0 | 1 | 1 | 1 | 0 | 0 | 0 | 1 | 1 | 1 |
| 116 | 0 | 0 | 1 | 1 | 0 | 0 | 0 | 1 | 1 | 1 |
| 117 | 0 | 0 | 0 | 0 | 0 | 0 | 0 | 0 | 0 | 1 |
| 118 | 0 | 0 | 0 | 0 | 0 | 0 | 0 | 0 | 0 | 1 |
| 119 | 0 | 0 | 0 | 1 | 0 | 0 | 0 | 0 | 0 | 1 |
| 120 | 0 | 0 | 0 | 0 | 0 | 0 | 0 | 0 | 0 | 0 |
| 121 | 0 | 0 | 0 | 0 | 0 | 0 | 0 | 0 | 0 | 0 |
| 122 | 0 | 0 | 0 | 0 | 0 | 0 | 0 | 0 | 0 | 0 |
| 123 | 0 | 0 | 0 | 0 | 0 | 0 | 0 | 0 | 0 | 0 |
| 124 | 0 | 0 | 0 | 0 | 0 | 0 | 0 | 0 | 0 | 0 |
| 125 | 0 | 0 | 0 | 0 | 0 | 0 | 0 | 0 | 0 | 0 |
| 126 | 0 | 0 | 0 | 0 | 0 | 0 | 0 | 0 | 0 | 0 |
| 127 | 0 | 0 | 0 | 0 | 0 | 0 | 0 | 0 | 0 | 0 |
| 128 | 0 | 0 | 0 | 0 | 0 | 0 | 0 | 0 | 0 | 0 |
| 129 | 1 | 1 | 0 | 1 | 1 | 1 | 1 | 1 | 1 | 1 |
| 130 | 1 | 1 | 0 | 1 | 1 | 1 | 1 | 1 | 1 | 1 |
| 131 | 1 | 1 | 0 | 1 | 1 | 1 | 1 | 1 | 1 | 1 |
| 132 | 0 | 0 | 0 | 0 | 0 | 0 | 0 | 0 | 0 | 0 |
| 133 | 0 | 0 | 0 | 0 | 0 | 0 | 0 | 0 | 0 | 0 |
| 134 | 0 | 0 | 0 | 0 | 0 | 0 | 0 | 0 | 0 | 0 |
| 135 | 1 | 1 | 0 | 0 | 1 | 0 | 1 | 0 | 0 | 0 |
| 136 | 1 | 1 | 1 | 0 | 1 | 0 | 1 | 0 | 0 | 0 |
| 137 | 0 | 1 | 0 | 0 | 1 | 0 | 1 | 0 | 0 | 0 |
| 138 | 0 | 0 | 0 | 1 | 0 | 0 | 0 | 0 | 0 | 1 |
| 139 | 1 | 0 | 0 | 1 | 0 | 0 | 0 | 0 | 0 | 1 |
| 140 | 1 | 0 | 0 | 0 | 0 | 0 | 0 | 0 | 0 | 1 |
| 141 | 1 | 1 | 0 | 1 | 1 | 1 | 1 | 1 | 1 | 1 |
| 142 | 1 | 1 | 0 | 1 | 1 | 1 | 1 | 1 | 1 | 1 |
| 143 | 1 | 1 | 0 | 1 | 1 | 1 | 1 | 1 | 1 | 1 |
| 144 | 0 | 0 | 1 | 0 | 0 | 0 | 0 | 0 | 0 | 0 |
| 145 | 0 | 0 | 1 | 0 | 0 | 0 | 0 | 0 | 0 | 0 |
| 146 | 0 | 0 | 1 | 0 | 0 | 0 | 0 | 0 | 0 | 0 |
| 147 | 0 | 0 | 0 | 0 | 0 | 0 | 0 | 0 | 0 | 0 |
| 148 | 0 | 0 | 0 | 0 | 0 | 0 | 0 | 0 | 0 | 0 |
| 149 | 0 | 0 | 0 | 0 | 0 | 0 | 0 | 0 | 0 | 0 |

Les échantillons Ech109 à Ech118 comportent au moins un peptide caractéristique des protéines CTX-M. Les bactéries présentes dans les échantillons Ech109 à Ech118 expriment donc une béta-lactamase qui leur confère une résistance aux pénicillines, aux céphalosporines et aux monobactames.

### Exemple 28 : Identification d'une résistance aux béta-lactamines de type DHA:

Les échantillons Ech119 à Ech124 sont identifiés selon l'une des méthodes décrites dans les exemples 1, 3 ou 4. L'identification des espèces est reportée dans le **TABLEAU 40.**

**TABLEAU 40 :**

| Noms | Espèces |
|---|---|
| Ech119 | *E. coli* |
| Ech120 | *K. oxytoca* |
| Ech121 | *K. pneumoniae* |
| Ech122 | *K. pneumoniae* |
| Ech123 | *K. pneumoniae* |
| Ech124 | *K. pneumoniae* |

Les échantillons Ech119 à Ech124 correspondent à une espèce pouvant comporter un mécanisme de résistance de type DHA. Chaque échantillon est traité selon l'exemple 5, puis analysé selon l'exemple 6 en détectant les peptides du **TABLEAU 41** en lieu et place des peptides du **TABLEAU 3.**

**TABLEAU 41 :**

| Transition numéro | Peptide | Etat de charge du précurseur | Ion fragment | Temps de rétention (minutes) | (m/z) filtré en Q1 | (m/z) filtré en Q3 | Energie de collision (eV) | Seuil de positivité |
|---|---|---|---|---|---|---|---|---|
| 1 | AAQAILSALEMK | 2 | y8 monochargé | 21,45 | 623,35 | 904,52 | 31,3 | 2500 |
| 2 | AAQAILSALEMK | 2 | y7 monochargé | 21,45 | 623,35 | 791,43 | 31,3 | 2500 |
| 3 | AAQAILSALEMK | 2 | y6 monochargé | 21,45 | 623,35 | 678,35 | 31,3 | 2500 |
| 4 | ADLLNFYQQWQPSR | 2 | y3 monochargé | 22,92 | 883,44 | 359,2 | 46,1 | 2500 |
| 5 | ADLLNFYQQWQPSR | 3 | y5 monochargé | 22,92 | 589,29 | 673,34 | 25,4 | 2500 |
| 6 | ADLLNFYQQWQPSR | 3 | y3 monochargé | 22,92 | 589,29 | 359,2 | 25,4 | 2500 |
| 7 | AGNADLEMAMYLAQTR | 3 | y6 monochargé | 21,53 | 585,61 | 751,41 | 25,2 | 2500 |
| 8 | AGNADLEMAMYLAQTR | 3 | y5 monochargé | 21,53 | 585,61 | 588,35 | 25,2 | 2500 |
| 9 | AGNADLEMAMYLAQTR | 3 | y4 monochargé | 21,53 | 585,61 | 475,26 | 25,2 | 2500 |
| 10 | EMALNDPAAK | 2 | y8 monochargé | 13,12 | 530,26 | 799,43 | 26 | 2500 |
| 11 | EMALNDPAAK | 2 | y6 monochargé | 13,12 | 530,26 | 615,31 | 26 | 2500 |
| 12 | EMALNDPAAK | 2 | y4 monochargé | 13,12 | 530,26 | 386,24 | 26 | 2500 |
| 13 | GKPYYFNYGFADIQAK | 3 | y8 monochargé | 19,85 | 627,98 | 849,45 | 26,5 | 2500 |
| 14 | GKPYYFNYGFADIQAK | 3 | y6 monochargé | 19,85 | 627,98 | 645,36 | 26,5 | 2500 |
| 15 | GKPYYFNYGFADIQAK | 3 | y5 monochargé | 19,85 | 627,98 | 574,32 | 26,5 | 2500 |
| 16 | NYPNTER | 2 | y5 monochargé | 7,63 | 447,21 | 616,31 | 21,2 | 2500 |
| 17 | NYPNTER | 2 | y3 monochargé | 7,63 | 447,21 | 405,21 | 21,2 | 2500 |
| 18 | NYPNTER | 2 | y5 dichargé | 7,63 | 447,21 | 308,66 | 21,2 | 2500 |
| 19 | QPVTENTLFELGSVSK | 2 | y8 monochargé | 20,9 | 874,96 | 866,46 | 45,6 | 2500 |
| 20 | QPVTENTLFELGSVSK | 3 | y8 monochargé | 20,9 | 583,64 | 866,46 | 25,2 | 2500 |
| 21 | QPVTENTLFELGSVSK | 3 | y5 monochargé | 20,9 | 583,64 | 477,27 | 25,2 | 2500 |
| 22 | QVAIVILANK | 2 | y8 monochargé | 18,75 | 534,84 | 841,55 | 26,2 | 2500 |
| 23 | QVAIVILANK | 2 | y6 monochargé | 18,75 | 534,84 | 657,43 | 26,2 | 2500 |
| 24 | QVAIVILANK | 2 | y5 monochargé | 18,75 | 534,84 | 558,36 | 26,2 | 2500 |
| 25 | TFTGVLGAVSVAK | 2 | y11 monochargé | 20,03 | 625,36 | 1001,6 | 31,4 | 2500 |
| 26 | TFTGVLGAVSVAK | 2 | y8 monochargé | 20,03 | 625,36 | 744,46 | 31,4 | 2500 |
| 27 | TFTGVLGAVSVAK | 2 | y7 monochargé | 20,03 | 625,36 | 631,38 | 31,4 | 2500 |
| 28 | TGATTGFGAYVAFIPEK | 2 | y3 monochargé | 22,12 | 865,44 | 373,21 | 45,1 | 2500 |
| 29 | TGATTGFGAYVAFIPEK | 3 | y6 monochargé | 22,12 | 577,3 | 704,4 | 25 | 2500 |
| 30 | TGATTGFGAYVAFIPEK | 3 | y3 monochargé | 22,12 | 577,3 | 373,21 | 25 | 2500 |
| 31 | VSPGQLDAESYGVK | 2 | y8 monochargé | 15,43 | 725,37 | 868,41 | 37,1 | 2500 |
| 32 | VSPGQLDAESYGVK | 2 | y13 dichargé | 15,43 | 725,37 | 675,83 | 37,1 | 2500 |
| 33 | VSPGQLDAESYGVK | 2 | y12 dichargé | 15,43 | 725,37 | 632,31 | 37,1 | 2500 |
| 34 | WAEMNMEPSR | 2 | y8 monochargé | 15,78 | 625,77 | 993,41 | 31,4 | 2500 |
| 35 | WAEMNMEPSR | 2 | y6 monochargé | 15,78 | 625,77 | 733,33 | 31,4 | 2500 |
| 36 | WAEMNMEPSR | 2 | y3 monochargé | 15,78 | 625,77 | 359,2 | 31,4 | 2500 |
| 37 | YQPELALPQWK | 2 | y4 monochargé | 20,97 | 686,87 | 558,3 | 34,9 | 2500 |
| 38 | YQPELALPQWK | 2 | y9 dicharqé | 20,97 | 686,87 | 541,31 | 34,9 | 2500 |
| 39 | YQPELALPQWK | 3 | y4 monochargé | 20,97 | 458,25 | 558,3 | 21,3 | 2500 |

Les autres paramètres machine utilisés sont les suivants :
- Type de balayage: MRM
- MRM planifié: non
- Polarité: Positive
- Source d'ionisation:Turbo V™ (Applied BioSystems)
- Réglage Q1: Filtrage avec résolution unitaire
- Réglage Q3: Filtrage avec résolution unitaire
- Pause inter-scan: 5.00 msec
- Vitesse de balayage: 10 Da/s
- Gaz rideau: 50,00 psi
- Tension de cône: 5500,00 V
- Température de source: 500,00 °C
- Gaz de nébulisation: 50,00 psi
- Gaz chauffant: 50,00 psi
- Gaz de collision induisant dissociation : 9,00 psi
- Remplissage dynamique: activé
- Potentiel d'orifice : 100, 00 V
- (en anglais declustering potential (DP))
- Potentiel d'entrée avant Q0 (EP): 6,00 V
- Potentiel en sortie de cellule de collision : 15 V
- (en anglais cell exit potential (CXP))
- Temps de cycle total: 1,17 sec

Les aires obtenues pour chacune des transitions et pour chacun des microorganismes étudiés ont été mesurées. Lorsque les aires des transitions sont supérieures ou égales au seuil de positivité décrit dans le **TABLEAU 41,** la détection de la transition est considérée comme positive et est notée « 1 » dans le **TABLEAU 42.** Lorsqu'une transition a une aire inférieure au seuil de positivité décrit dans le **TABLEAU 41,** la transition est considérée comme non détectée et est notée « 0 » dans le **TABLEAU 42.**

Pour un peptide donné, lorsque au moins 3 transitions sont notées « 1 », le peptide est considéré comme détecté.

**TABLEAU 42 :**

| Transition numéro | Ech119 | Ech120 | Ech121 | Ech122 | Ech123 | Ech124 |
|---|---|---|---|---|---|---|
| 1 | 1 | 0 | 1 | 1 | 0 | 0 |
| 2 | 0 | 0 | 0 | 0 | 0 | 0 |
| 3 | 0 | 0 | 0 | 0 | 0 | 0 |
| 4 | 0 | 0 | 1 | 0 | 1 | 0 |
| 5 | 0 | 1 | 1 | 0 | 0 | 0 |
| 6 | 0 | 1 | 1 | 0 | 0 | 0 |
| 7 | 0 | 0 | 0 | 1 | 0 | 0 |
| 8 | 0 | 0 | 0 | 1 | 0 | 0 |
| 9 | 0 | 0 | 0 | 1 | 0 | 0 |
| 10 | 1 | 1 | 1 | 1 | 1 | 1 |
| 11 | 1 | 1 | 1 | 1 | 1 | 1 |
| 12 | 1 | 1 | 1 | 0 | 1 | 1 |
| 13 | 1 | 1 | 1 | 0 | 1 | 0 |
| 14 | 1 | 1 | 1 | 0 | 1 | 0 |
| 15 | 1 | 0 | 1 | 0 | 0 | 0 |
| 16 | 0 | 0 | 1 | 1 | 0 | 1 |
| 17 | 0 | 0 | 1 | 1 | 0 | 1 |
| 18 | 0 | 0 | 1 | 1 | 1 | 1 |
| 19 | 1 | 1 | 1 | 1 | 1 | 1 |
| 20 | 1 | 0 | 1 | 1 | 1 | 1 |
| 21 | 1 | 1 | 1 | 1 | 1 | 1 |
| 22 | 0 | 1 | 1 | 1 | 1 | 1 |
| 23 | 0 | 0 | 1 | 1 | 1 | 1 |
| 24 | 1 | 1 | 1 | 1 | 1 | 1 |
| 25 | 1 | 1 | 1 | 1 | 1 | 1 |
| 26 | 1 | 1 | 1 | 1 | 1 | 1 |
| 27 | 1 | 1 | 1 | 1 | 1 | 1 |
| 28 | 0 | 0 | 1 | 1 | 0 | 0 |
| 29 | 0 | 0 | 1 | 1 | 1 | 0 |
| 30 | 0 | 0 | 1 | 1 | 0 | 0 |
| 31 | 1 | 1 | 1 | 1 | 1 | 1 |
| 32 | 0 | 1 | 1 | 1 | 1 | 1 |
| 33 | 0 | 1 | 1 | 1 | 1 | 1 |
| 34 | 1 | 1 | 1 | 1 | 1 | 1 |
| 35 | 0 | 1 | 1 | 1 | 1 | 1 |
| 36 | 1 | 0 | 1 | 1 | 1 | 1 |
| 37 | 1 | 0 | 1 | 1 | 0 | 1 |
| 38 | 1 | 1 | 1 | 1 | 1 | 1 |
| 39 | 1 | 1 | 1 | 1 | 1 | 1 |

Les échantillons Ech119 à Ech124 comportent au moins un peptide caractéristique des protéines DHA. Les bactéries présentes dans les échantillons Ech119 à Ech124 expriment donc une béta-lactamase qui leur confère une résistance aux pénicillines et aux céphalosporines excepté les céphalosporines de quatrième génération.

### Exemple 29 : Identification d'une résistance aux béta-lactamines de type FOX:

Les échantillons Ech125 à Ech130 sont identifiés selon l'une des méthodes décrites dans les exemples 1, 3 ou 4. L'identification des espèces est reportée dans le TABLEAU 43.

**TABLEAU 43 :**

| Noms | Espèces |
|---|---|
| Ech125 | *E. coli* |
| Ech126 | *E. coli* |
| Ech127 | *K. oxytoca* |
| Ech128 | *K. oxytoca* |
| Ech129 | *K. pneumoniae* |
| Ech130 | *K. pneumoniae* |

Les échantillons Ech125 à Ech130 correspondent à une espèce pouvant comporter un mécanisme de résistance de type FOX. Chaque échantillon est traité selon l'exemple 5, puis analysé selon l'exemple 6 en détectant les peptides du **TABLEAU 44** en lieu et place des peptides du **TABLEAU 3.**

**TABLEAU 44 :**

| Transition numéro | Peptide | Etat de charge du précurseur | Ion fragment | Temps de rétention (minutes) | (m/z) filtré en Q1 | (m/z) filtré en Q3 | Energie de collision (eV) | Seuil de positivité |
|---|---|---|---|---|---|---|---|---|
| 1 | AHYFNYGVANR | 3 | y3 monochargé | 14,08 | 437,88 | 360,2 | 20,7 | 2500 |
| 2 | AHYFNYGVANR | 3 | y4 monochargé | 14,08 | 437,88 | 459,27 | 20,7 | 2500 |
| 3 | AHYFNYGVANR | 3 | y5 monochargé | 14,08 | 437,88 | 516,29 | 20,7 | 2500 |
| 4 | AMGEQR | 2 | y5 dichargé | 1,08 | 346,16 | 310,65 | 15,5 | 2500 |
| 5 | AMGEQR | 2 | y4 monochargé | 1,08 | 346,16 | 489,24 | 15,5 | 2500 |
| 6 | AMGEQR | 2 | y5 monochargé | 1,08 | 346,16 | 620,28 | 15,5 | 2500 |
| 7 | ESGQR | 2 | y3 dichargé | 0,8 | 288,64 | 180,6 | 12,2 | 2500 |
| 8 | ESGQR | 2 | y3 monochargé | 0,76 | 288,64 | 360,2 | 12,2 | 2500 |
| 9 | ESGQR | 2 | y4 monochargé | 0,82 | 288,64 | 447,23 | 12,2 | 2500 |
| 10 | FAVPK | 3 | y4 monochargé | 12,95 | 187,79 | 414,27 | 12,9 | 2500 |
| 11 | FAVPK | 2 | y3 monochargé | 12,95 | 281,17 | 343,23 | 11,8 | 2500 |
| 12 | FAVPK | 2 | y4 monochargé | 12,93 | 281,17 | 414,27 | 11,8 | 2500 |
| 13 | GGFELDDK | 2 | y6 dichargé | 14,31 | 440,71 | 383,69 | 20,9 | 2500 |
| 14 | GGFELDDK | 2 | y4 monochargé | 14,34 | 440,71 | 490,25 | 20,9 | 2500 |
| 15 | GGFELDDK | 2 | y5 monochargé | 14,31 | 440,71 | 619,29 | 20,9 | 2500 |
| 16 | GIAIVMLANR | 2 | y4 monochargé | 20,15 | 529,31 | 473,28 | 25,9 | 2500 |
| 17 | GIAIVMLANR | 2 | y5 monochargé | 20,13 | 529,31 | 604,32 | 25,9 | 2500 |
| 18 | GIAIVMLANR | 2 | y6 monochargé | 20,13 | 529,31 | 703,39 | 25,9 | 2500 |
| 19 | IPGMAVAVLK | 2 | y9 dichargé | 19,17 | 499,81 | 443,27 | 24,2 | 2500 |
| 20 | IPGMAVAVLK | 2 | y8 monochargé | 19,17 | 499,81 | 788,47 | 24,2 | 2500 |
| 21 | IPGMAVAVLK | 2 | y9 monochargé | 19,15 | 499,81 | 885,52 | 24,2 | 2500 |
| 22 | NYPIEAR | 2 | y3 monochargé | 12,04 | 431,73 | 375,2 | 20,3 | 2500 |
| 23 | NYPIEAR | 2 | y4 monochargé | 12,04 | 431,73 | 488,28 | 20,3 | 2500 |
| 24 | NYPIEAR | 2 | y5 monochargé | 12,06 | 431,73 | 585,34 | 20,3 | 2500 |
| 25 | SWSPVYPAGTHR | 3 | y9 dichargé | 14,97 | 453,23 | 499,26 | 21,1 | 2500 |
| 26 | SWSPVYPAGTHR | 3 | y10 dichargé | 14,97 | 453,23 | 542,78 | 21,1 | 2500 |
| 27 | SWSPVYPAGTHR | 3 | y6 monochargé | 14,97 | 453,23 | 638,34 | 21,1 | 2500 |
| 28 | TGSADLLK | 2 | y5 monochargé | 12,97 | 402,73 | 559,35 | 18,7 | 2500 |
| 29 | TGSADLLK | 2 | y6 monochargé | 12,95 | 402,73 | 646,38 | 18,7 | 2500 |
| 30 | TGSADLLK | 2 | y7 monochargé | 12,97 | 402,73 | 703,4 | 18,7 | 2500 |
| 31 | TGSTGGFGAYVAFVPAR | 3 | y5 monochargé | 21,03 | 553,28 | 589,35 | 24,2 | 2500 |
| 32 | TGSTGGFGAYVAFVPAR | 3 | y6 monochargé | 21,03 | 553,28 | 660,38 | 24,2 | 2500 |
| 33 | TGSTGGFGAYVAFVPAR | 2 | y3 monochargé | 21,03 | 829,42 | 343,21 | 43 | 2500 |
| 34 | TGSTGGFGAYVAFVPAR | 2 | y6 monochargé | 21,03 | 829,42 | 660,38 | 43 | 2500 |
| 35 | TLTATLGAYAAVK | 2 | y7 monochargé | 18,68 | 640,37 | 679,38 | 32,2 | 2500 |
| 36 | TLTATLGAYAAVK | 2 | y8 monochargé | 18,68 | 640,37 | 792,46 | 32,2 | 2500 |
| 37 | TLTATLGAYAAVK | 2 | y9 monochargé | 18,66 | 640,37 | 893,51 | 32,2 | 2500 |
| 38 | VSEQTLFEIGSVSK | 2 | y5 monochargé | 20,34 | 762,4 | 477,27 | 39,2 | 2500 |
| 39 | VSEQTLFEIGSVSK | 2 | y7 monochargé | 20,34 | 762,4 | 719,39 | 39,2 | 2500 |
| 40 | VSEQTLFEIGSVSK | 2 | y8 monochargé | 20,34 | 762,4 | 866,46 | 39,2 | 2500 |
| 41 | VSQHAPWLK | 3 | y6 dichargé | 14,1 | 355,87 | 376,22 | 18,1 | 2500 |
| 42 | VSQHAPWLK | 3 | y8 dichargé | 14,1 | 355,87 | 483,76 | 18,1 | 2500 |
| 43 | VSQHAPWLK | 3 | y4 monochargé | 14,1 | 355,87 | 543,33 | 18,1 | 2500 |
| 44 | VTPGVLAAEAYGIK | 2 | y12 dichargé | 19,37 | 694,89 | 594,84 | 35,3 | 2500 |
| 45 | VTPGVLAAEAYGIK | 2 | y7 monochargé | 19,37 | 694,89 | 751,4 | 35,3 | 2500 |
| 46 | VTPGVLAAEAYGIK | 2 | y8 monochargé | 19,37 | 694,89 | 822,44 | 35,3 | 2500 |

Les autres paramètres machine utilisés sont les suivants :
- Type de balayage: MRM
- MRM planifié : non
- Polarité: Positive
- Source d'ionisation:Turbo V™ (Applied BioSystems)
- Réglage Q1: Filtrage avec résolution unitaire
- Réglage Q3: Filtrage avec résolution unitaire
- Pause inter-scan: 5.00 msec
- Vitesse de balayage: 10 Da/s
- Gaz rideau: 50,00 psi
- Tension de cône: 5500,00 V
- Température de source: 500,00 °C
- Gaz de nébulisation: 50,00 psi
- Gaz chauffant: 50,00 psi
- Gaz de collision induisant dissociation : 9,00 psi
- Remplissage dynamique: activé
- Potentiel d'orifice : 100, 00 V
- (en anglais declustering potential (DP))
- Potentiel d'entrée avant Q0 (EP): 6,00 V
- Potentiel en sortie de cellule de collision : 15 V
- (en anglais cell exit potential (CXP))
- Temps de cycle total: 1,38 sec

Les aires obtenues pour chacune des transitions et pour chacun des microorganismes étudiés ont été mesurées. Lorsque les aires des transitions sont supérieures ou égales au seuil de positivité décrit dans le **TABLEAU 44,** la détection de la transition est considérée comme positive et est notée « 1 » dans le **TABLEAU 45.** Lorsqu'une transition a une aire inférieure au seuil de positivité décrit dans le **TABLEAU 44,** la transition est considérée comme non détectée et est notée « 0 » dans le **TABLEAU 45.**

Pour un peptide donné, lorsque au moins 2 transitions sont notées « 1 », le peptide est considéré comme détecté.

**TABLEAU 45 :**

| transition numéro | Ech125 | Ech126 | Ech127 | Ech128 | Ech129 | Ech130 |
|---|---|---|---|---|---|---|
| 1 | 0 | 0 | 1 | 1 | 0 | 0 |
| 2 | 1 | 0 | 1 | 1 | 0 | 0 |
| 3 | 1 | 1 | 1 | 1 | 0 | 1 |
| 4 | 1 | 1 | 0 | 1 | 1 | 1 |
| 5 | 1 | 1 | 0 | 1 | 1 | 1 |
| 6 | 1 | 1 | 1 | 1 | 1 | 1 |
| 7 | 0 | 0 | 0 | 0 | 0 | 0 |
| 8 | 0 | 0 | 1 | 0 | 0 | 0 |
| 9 | 0 | 0 | 0 | 0 | 0 | 0 |
| 10 | 0 | 0 | 0 | 0 | 0 | 0 |
| 11 | 0 | 0 | 0 | 0 | 0 | 0 |
| 12 | 0 | 0 | 0 | 0 | 0 | 0 |
| 13 | 1 | 1 | 1 | 1 | 1 | 1 |
| 14 | 1 | 0 | 0 | 1 | 0 | 1 |
| 15 | 1 | 0 | 0 | 1 | 0 | 1 |
| 16 | 1 | 0 | 1 | 1 | 1 | 1 |
| 17 | 1 | 1 | 1 | 1 | 1 | 1 |
| 18 | 1 | 1 | 1 | 1 | 1 | 1 |
| 19 | 1 | 1 | 1 | 1 | 1 | 1 |
| 20 | 1 | 1 | 1 | 1 | 1 | 1 |
| 21 | 1 | 1 | 1 | 1 | 1 | 1 |
| 22 | 1 | 1 | 1 | 1 | 1 | 1 |
| 23 | 1 | 1 | 1 | 1 | 1 | 1 |
| 24 | 1 | 1 | 1 | 1 | 1 | 1 |
| 25 | 1 | 1 | 1 | 1 | 1 | 1 |
| 26 | 1 | 1 | 1 | 1 | 1 | 1 |
| 27 | 1 | 1 | 1 | 1 | 1 | 1 |
| 28 | 1 | 1 | 1 | 1 | 1 | 1 |
| 29 | 1 | 1 | 1 | 1 | 1 | 1 |
| 30 | 1 | 1 | 1 | 1 | 1 | 1 |
| 31 | 1 | 0 | 1 | 1 | 1 | 1 |
| 32 | 0 | 1 | 1 | 1 | 1 | 1 |
| 33 | 0 | 1 | 1 | 0 | 1 | 1 |
| 34 | 0 | 1 | 1 | 1 | 1 | 1 |
| 35 | 0 | 0 | 0 | 0 | 0 | 1 |
| 36 | 1 | 1 | 1 | 0 | 1 | 1 |
| 37 | 1 | 0 | 1 | 0 | 1 | 1 |
| 38 | 1 | 1 | 1 | 1 | 1 | 1 |
| 39 | 1 | 1 | 1 | 1 | 1 | 1 |
| 40 | 1 | 1 | 1 | 1 | 1 | 1 |
| 41 | 1 | 1 | 1 | 1 | 1 | 1 |
| 42 | 1 | 1 | 0 | 0 | 1 | 1 |
| 43 | 1 | 1 | 1 | 1 | 1 | 1 |
| 44 | 0 | 0 | 0 | 0 | 0 | 0 |
| 45 | 0 | 0 | 0 | 0 | 0 | 0 |
| 46 | 0 | 0 | 0 | 0 | 0 | 0 |

Les échantillons Ech125 à Ech130 comportent au moins un peptide caractéristique des protéines FOX. Les bactéries présentes dans les échantillons Ech125 à Ech130 expriment donc une béta-lactamase qui leur confère une résistance aux pénicillines et aux céphalosporines excepté les céphalosporines de quatrième génération.

### Exemple 30 : Identification d'une résistance aux béta-lactamines de type SHV:

Les échantillons Ech131 à Ech144 sont identifiés selon l'une des méthodes décrites dans les exemples 1, 3 ou 4. L'identification des espèces est reportée dans le **TABLEAU 46.**

**TABLEAU 46 :**

| Noms | Espèces |
|---|---|
| Ech131 | *E. aerogenes* |
| Ech132 | *E. coli* |
| Ech133 | *E. coli* |
| Ech134 | *E. coli* |
| Ech135 | *E. coli* |
| Ech136 | *K. pneumoniae* |
| Ech137 | *K. pneumoniae* |
| Ech138 | *K. pneumoniae* |
| Ech139 | *K. pneumoniae* |
| Ech140 | *K. pneumoniae* |
| Ech141 | *K. pneumoniae* |
| Ech142 | *K. pneumoniae* |
| Ech143 | *K. pneumoniae* |
| Ech144 | *K. pneumoniae* |

Les échantillons Ech131 à Ech144 correspondent à une espèce pouvant comporter un mécanisme de résistance de type SHV. Chaque échantillon est traité selon l'exemple 5, puis analysé selon l'exemple 6 en détectant les peptides du **TABLEAU 47** en lieu et place des peptides du **TABLEAU 3.**

**TABLEAU 47 :**

| Transition numéro | Peptide | Etat de charge du précurseur | Ion fragment | Temps de rétention (minutes) | (m/z) filtré en Q1 | (m/z) filtré en Q3 | Energie de collision (eV) | Seuil de positivité |
|---|---|---|---|---|---|---|---|---|
| 1 | AGAGER | 2 | y3 monochargé | 0,9 | 280,64 | 361,18 | 11,7 | 2000 |
| 2 | AGAGER | 2 | y4 monochargé | 0,9 | 280,64 | 432,22 | 11,7 | 2000 |
| 3 | AGAGER | 2 | y5 monochargé | 0,9 | 280,64 | 489,24 | 11,7 | 2000 |
| 4 | ATTTPASMAATLR | 3 | y5 monochargé | 16,2 | 431,23 | 531,33 | 20,5 | 2000 |
| 5 | ATTTPASMAATLR | 2 | y9 dichargé | 16,2 | 646, 34 | 459,25 | 32,6 | 2000 |
| 6 | ATTTPASMAATLR | 2 | y9 monochargé | 16,2 | 646,34 | 917,49 | 32,6 | 2000 |
| 7 | CIISLLATLPLAVHASPQPLEQIK | 3 | y18 dichargé | 27,4 | 871,5 | 957,05 | 34,1 | 2000 |
| 8 | CIISLLATLPLAVHASPQPLEQIK | 3 | y21 dichargé | 27,4 | 871,5 | 1113,65 | 34,1 | 2000 |
| 9 | CIISLLATLPLAVHASPQPLEQIK | 3 | y22 dichargé | 27,3 | 871,5 | 1170,19 | 34,1 | 2000 |
| 10 | DMPASMAER | 2 | y7 dichargé | 12,4 | 504,22 | 381,18 | 24,5 | 2000 |
| 11 | DMPASMAER | 2 | y5 monochargé | 12,4 | 504,22 | 593,27 | 24,5 | 2000 |
| 12 | DMPASMAER | 2 | y7 monochargé | 12,4 | 504,22 | 761,36 | 24,5 | 2000 |
| 13 | DSPASMAER | 2 | y7 dichargé | 10,5 | 482,21 | 381,18 | 23,2 | 2000 |
| 14 | DSPASMAER | 2 | y5 monochargé | 10,5 | 482,21 | 593,27 | 23,2 | 2000 |
| 15 | DSPASMAER | 2 | y6 monochargé | 10,5 | 482,21 | 664,31 | 23,2 | 2000 |
| 16 | DTLASMAER | 2 | y5 monochargé | 14 | 497,24 | 593,27 | 24,1 | 2000 |
| 17 | DTLASMAER | 2 | y6 monochargé | 14 | 497,24 | 664,31 | 24,1 | 2000 |
| 18 | DTLASMAER | 2 | y7 monochargé | 14 | 497,24 | 777,39 | 24,1 | 2000 |
| 19 | DTPASMAER | 2 | y7 dichargé | 10,5 | 489,22 | 381,18 | 23,6 | 2000 |
| 20 | DTPASMAER | 2 | y5 monochargé | 10,5 | 489,22 | 593,27 | 23,6 | 2000 |
| 21 | DTPASMAER | 2 | y7 monochargé | 10,5 | 489,22 | 761,36 | 23,6 | 2000 |
| 22 | DTPASMAK | 2 | y6 dichargé | 9,1 | 410,7 | 302,66 | 19,1 | 2000 |
| 23 | DTPASMAK | 2 | y5 monochargé | 9,1 | 410,7 | 507,26 | 19,1 | 2000 |
| 24 | DTPASMAK | 2 | y6 monochargé | 9,1 | 410,7 | 604,31 | 19,1 | 2000 |
| 25 | DTTTPASMAATLR | 3 | y5 monochargé | 16,5 | 445,89 | 531,33 | 20,9 | 2000 |
| 26 | DTTTPASMAATLR | 2 | y9 dichargé | 16,5 | 668,33 | 459,25 | 33,8 | 2000 |
| 27 | DTTTPASMAATLR | 2 | y9 monochargé | 16,5 | 668,33 | 917,49 | 33,8 | 2000 |
| 28 | DTTTPASMAGTLR | 3 | y4 monochargé | 15,3 | 441,22 | 446,27 | 20,8 | 2000 |
| 29 | DTTTPASMAGTLR | 2 | y9 dichargé | 15,3 | 661,32 | 452,24 | 33,4 | 2000 |
| 30 | DTTTPASMAGTLR | 2 | y9 monochargé | 15,3 | 661,32 | 903,47 | 33,4 | 2000 |
| 31 | DTTTPASMTATLR | 2 | y9 dichargé | 15,9 | 683,34 | 474,25 | 34,7 | 2000 |
| 32 | DTTTPASMTATLR | 2 | y7 monochargé | 15,9 | 683,34 | 779,41 | 34,7 | 2000 |
| 33 | DTTTPASMTATLR | 2 | y9 monochargé | 15,9 | 683,34 | 947,5 | 34,7 | 2000 |
| 34 | FPMISTFK | 2 | y7 dichargé | 20,2 | 485,76 | 412,22 | 23,4 | 2000 |
| 35 | FPMISTFK | 2 | y5 monochargé | 20,2 | 485,76 | 595,35 | 23,4 | 2000 |
| 36 | FPMISTFK | 2 | y6 monochargé | 20,2 | 485,76 | 726,39 | 23,4 | 2000 |
| 37 | FPMMSTFK | 2 | y7 dichargé | 19,3 | 494,74 | 421,2 | 23,9 | 2000 |
| 38 | FPMMSTFK | 2 | y5 monochargé | 19,3 | 494,74 | 613,3 | 23,9 | 2000 |
| 39 | FPMMSTFK | 2 | y6 monochargé | 19,3 | 494,74 | 744,34 | 23,9 | 2000 |
| 40 | GIVALLGGNIK | 2 | y5 monochargé | 21,1 | 527,84 | 488,28 | 25,8 | 2000 |
| 41 | GIVALLGGNIK | 2 | y6 monochargé | 21,1 | 527,84 | 601,37 | 25,8 | 2000 |
| 42 | GIVALLGGNIK | 2 | y8 monochargé | 21,1 | 527,84 | 785,49 | 25,8 | 2000 |
| 43 | GIVALLGPDNK | 2 | y5 monochargé | 18,9 | 548,82 | 530,26 | 27 | 2000 |
| 44 | GIVALLGPDNK | 2 | y6 monochargé | 19 | 548,82 | 643,34 | 27 | 2000 |
| 45 | GIVALLGPDNK | 2 | y8 monochargé | 18,9 | 548,82 | 827,46 | 27 | 2000 |
| 46 | GIVALLGPNHK | 3 | y9 dichargé | 17,6 | 373,56 | 474,79 | 18,7 | 2000 |
| 47 | GIVALLGPNHK | 3 | y5 monochargé | 17,6 | 373,56 | 552,29 | 18,7 | 2000 |
| 48 | GIVALLGPNHK | 3 | y6 monochargé | 17,6 | 373,56 | 665,37 | 18,7 | 2000 |
| 49 | GIVALLGPNNK | 2 | y5 monochargé | 18,6 | 548,33 | 529,27 | 27 | 2000 |
| 50 | GIVALLGPNNK | 2 | y6 monochargé | 18,6 | 548,33 | 642,36 | 27 | 2000 |
| 51 | GIVALLGPNNK | 2 | y7 monochargé | 18,6 | 548,33 | 755,44 | 27 | 2000 |
| 52 | GIVALLGPNNNAER | 3 | y8 dichargé | 18,8 | 479,93 | 436,2 | 22 | 2000 |
| 53 | GIVALLGPNNNAER | 2 | y7 monochargé | 18,8 | 719,39 | 814,38 | 36,7 | 2000 |
| 54 | GIVALLGPNNNAER | 2 | y8 monochargé | 18,7 | 719,39 | 871,4 | 36,7 | 2000 |
| 55 | GIVALR | 2 | y4 dichargé | 14,1 | 314,71 | 229,66 | 13,7 | 2000 |
| 56 | GIVALR | 2 | y3 monochargé | 14,1 | 314,71 | 359,24 | 13,7 | 2000 |
| 57 | GIVALR | 2 | y4 monochargé | 14,1 | 314,71 | 458,31 | 13,7 | 2000 |
| 58 | GPNNK | 2 | y4 dichargé | 0,8 | 265,14 | 236,63 | 10,8 | 2000 |
| 59 | GPNNK | 2 | y3 monochargé | 0,8 | 265,14 | 375,2 | 10,8 | 2000 |
| 60 | GPNNK | 2 | y4 monochargé | 0,8 | 265,14 | 472,25 | 10,8 | 2000 |
| 61 | GTTTPASMAATLR | 2 | y9 dichargé | 16 | 639,33 | 459,25 | 32,2 | 2000 |
| 62 | GTTTPASMAATLR | 2 | y7 monochargé | 16 | 639,33 | 749,4 | 32,2 | 2000 |
| 63 | GTTTPASMAATLR | 2 | y9 monochargé | 16 | 639,33 | 917,49 | 32,2 | 2000 |
| 64 | HLADGMTVGELCAAAITMSDNSAAK | 3 | y6 monochargé | 21,3 | 845,39 | 605,29 | 33,3 | 2000 |
| 65 | HLADGMTVGELCAAAITMSDNSAAK | 3 | y7 monochargé | 21,3 | 845,39 | 692,32 | 33,3 | 2000 |
| 66 | HLADGMTVGELCAAAITMSDNSAAK | 3 | y9 monochargé | 21,3 | 845,39 | 924,41 | 33,3 | 2000 |
| 67 | HLLQWMVDDR | 3 | y3 monochargé | 19,6 | 438,22 | 405,17 | 20,7 | 2000 |
| 68 | HLLQWMVDDR | 3 | y4 monochargé | 19,6 | 438,22 | 504,24 | 20,7 | 2000 |
| 69 | HLLQWMVDDR | 3 | y5 monochargé | 19,6 | 438,22 | 635,28 | 20,7 | 2000 |
| 70 | IHYLQQDLVDYSPVSEK | 3 | y6 monochargé | 19 | 678,68 | 646,34 | 28,1 | 2000 |
| 71 | IHYLQQDLVDYSPVSEK | 3 | y7 monochargé | 19 | 678,68 | 809,4 | 28,1 | 2000 |
| 72 | IHYLQQDLVDYSPVSEK | 3 | y8 monochargé | 18,9 | 678,68 | 924,43 | 28,1 | 2000 |
| 73 | IVVIYLR | 2 | y3 monochargé | 19,3 | 438,29 | 451,27 | 20,7 | 2000 |
| 74 | IVVIYLR | 2 | y4 monochargé | 19,3 | 438,29 | 564,35 | 20,7 | 2000 |
| 75 | IVVIYLR | 2 | y5 monochargé | 19,3 | 438,29 | 663,42 | 20,7 | 2000 |
| 76 | LCIISLLAALPLAVHASPQPLEQIK | 3 | y17 dichargé | 30,7 | 899,19 | 906,52 | 35 | 2000 |
| 77 | LCIISLLAALPLAVHASPQPLEQIK | 3 | y18 dichargé | 30,7 | 899,19 | 942,04 | 35 | 2000 |
| 78 | LCIISLLAALPLAVHASPQPLEQIK | 3 | y22 dichargé | 30,7 | 899,19 | 1155,18 | 35 | 2000 |
| 79 | LCIISLLATLPLAVHASPQPLDQIK | 3 | y19 dichargé | 30,2 | 904,52 | 1006,58 | 35,1 | 2000 |
| 80 | LCIISLLATLPLAVHASPQPLDQIK | 3 | y22 dichargé | 30,2 | 904,52 | 1163,18 | 35,1 | 2000 |
| 81 | LCIISLLATLPLAVHASPQPLDQIK | 3 | y23 dichargé | 30,3 | 904,52 | 1219,72 | 35,1 | 2000 |
| 82 | LCIISLLATLPLAVHASPQPLEQIK | 3 | y15 dichargé | 30,2 | 909,19 | 814,46 | 35,3 | 2000 |
| 83 | LCIISLLATLPLAVHASPQPLEQIK | 3 | y17 dichargé | 30,2 | 909,19 | 921,53 | 35,3 | 2000 |
| 84 | LCIISLLATLPLAVHASPQPLEQIK | 3 | y19 dichargé | 30,2 | 909,19 | 1013,59 | 35,3 | 2000 |
| 85 | LCIISLLATLPLAVHSSPQPLEQIK | 3 | y15 dichargé | 29,3 | 914,53 | 822,46 | 35,4 | 2000 |
| 86 | LCIISLLATLPLAVHSSPQPLEQIK | 3 | y18 dichargé | 29,3 | 914,53 | 965,04 | 35,4 | 2000 |
| 87 | LCIISLLATLPLAVHSSPQPLEQIK | 3 | y21 dichargé | 29,3 | 914,53 | 1121,64 | 35,4 | 2000 |
| 88 | LCIISLLATLPLAVHTSPQPLEQIK | 3 | y15 dichargé | 29,4 | 919,2 | 829,47 | 35,6 | 2000 |
| 89 | LCIISLLATLPLAVHTSPQPLEQIK | 3 | y21 dichargé | 29,5 | 919,2 | 1128,65 | 35,6 | 2000 |
| 90 | LCIISLLATLPLAVHTSPQPLEQIK | 3 | y23 dichargé | 29,4 | 919,2 | 1241,74 | 35,6 | 2000 |
| 91 | LCIISLLATLPLTVHASPQPLEQIK | 3 | y15 dichargé | 29,8 | 919,2 | 829,47 | 35,6 | 2000 |
| 92 | LCIISLLATLPLTVHASPQPLEQIK | 3 | y17 dichargé | 29,8 | 919,2 | 936,53 | 35,6 | 2000 |
| 93 | LCIISLLATLPLTVHASPQPLEQIK | 3 | y22 dichargé | 29,8 | 919,2 | 1185,19 | 35,6 | 2000 |
| 94 | LCIISLLATLPLVVHASPQPLEQIK | 3 | y19 dichargé | 31,3 | 918,54 | 1027,6 | 35,6 | 2000 |
| 95 | LCIISLLATLPLVVHASPQPLEQIK | 3 | y21 dichargé | 31,3 | 918,54 | 1127,66 | 35,6 | 2000 |
| 96 | LCIISLLATLPLVVHASPQPLEQIK | 3 | y23 dichargé | 31,3 | 918,54 | 1240,75 | 35,6 | 2000 |
| 97 | LCIISLLATLSLAVHASPQPLEQIK | 3 | y18 dichargé | 31,2 | 905,85 | 952,04 | 35,2 | 2000 |
| 98 | LCIISLLATLSLAVHASPQPLEQIK | 3 | y22 dichargé | 31,3 | 905,85 | 1165,18 | 35,2 | 2000 |
| 99 | LCIISLLATLSLAVHASPQPLEQIK | 3 | y23 dichargé | 31,2 | 905,85 | 1221,72 | 35,2 | 2000 |
| 100 | LCIISLLATMPLAVHASPQPLEQIK | 3 | y18 dichargé | 28,9 | 915,18 | 966,03 | 35,5 | 2000 |
| 101 | LCIISLLATMPLAVHASPQPLEQIK | 3 | y19 dichargé | 28,9 | 915,18 | 1022,57 | 35,5 | 2000 |
| 102 | LCIISLLATMPLAVHASPQPLEQIK | 3 | y23 dichargé | 28,9 | 915,18 | 1235,71 | 35,5 | 2000 |
| 103 | LCIISLLAVLPLAVHASPQPLEQIK | 3 | y17 dichargé | 31 | 908,54 | 920,54 | 35,2 | 2000 |
| 104 | LCIISLLAVLPLAVHASPQPLEQIK | 3 | y22 dichargé | 31,1 | 908,54 | 1169,2 | 35,2 | 2000 |
| 105 | LCIISLLAVLPLAVHASPQPLEQIK | 3 | y23 dichargé | 31,1 | 908,54 | 1225,74 | 35,2 | 2000 |
| 106 | LLISQR | 2 | y3 monochargé | 13,4 | 365,24 | 390,21 | 16,6 | 2000 |
| 107 | LLISQR | 2 | y4 monochargé | 13,4 | 365,24 | 503,29 | 16,6 | 2000 |
| 108 | LLISQR | 2 | y5 monochargé | 13,4 | 365,24 | 616,38 | 16,6 | 2000 |
| 109 | LLLATVGGPAGLTAFLR | 3 | y4 monochargé | 26,9 | 557,34 | 506,31 | 24,4 | 2000 |
| 110 | LLLATVGGPAGLTAFLR | 3 | y5 monochargé | 26,9 | 557,34 | 607,36 | 24,4 | 2000 |
| 111 | LLLATVGGPAGLTAFLR | 2 | y11 monochargé | 26,9 | 835,5 | 1059,6 | 43,4 | 2000 |
| 112 | LLNSQR | 2 | y3 monochargé | 8,4 | 365,71 | 390,21 | 16,6 | 2000 |
| 113 | LLNSQR | 2 | y4 monochargé | 8,4 | 365,71 | 504,25 | 16,6 | 2000 |
| 114 | LLNSQR | 2 | y5 monochargé | 8,4 | 365,71 | 617,34 | 16,6 | 2000 |
| 115 | LLTNQR | 2 | y3 monochargé | 9,3 | 372,72 | 417,22 | 17 | 2000 |
| 116 | LLTNQR | 2 | y4 monochargé | 9,3 | 372,72 | 518,27 | 17 | 2000 |
| 117 | LLTNQR | 2 | y5 monochargé | 9,3 | 372,72 | 631,35 | 17 | 2000 |
| 118 | LLTSQR | 2 | y3 monochargé | 9,5 | 359,22 | 390,21 | 16,2 | 2000 |
| 119 | LLTSQR | 2 | y4 monochargé | 9,5 | 359,22 | 491,26 | 16,2 | 2000 |
| 120 | LLTSQR | 2 | y5 monochargé | 9,5 | 359,22 | 604,34 | 16,2 | 2000 |
| 121 | LNIISLLATLPLAVHASPQPLEQIK | 3 | y17 dichargé | 30,8 | 893,87 | 921,53 | 34,8 | 2000 |
| 122 | LNIISLLATLPLAVHASPQPLEQIK | 3 | y18 dichargé | 30,7 | 893,87 | 957,05 | 34,8 | 2000 |
| 123 | LNIISLLATLPLAVHASPQPLEQIK | 3 | y21 dichargé | 30,8 | 893,87 | 1113,65 | 34,8 | 2000 |
| 124 | LSASSQR | 2 | y4 monochargé | 1,2 | 374,7 | 477,24 | 17,1 | 2000 |
| 125 | LSASSQR | 2 | y5 monochargé | 1,2 | 374,7 | 548,28 | 17,1 | 2000 |
| 126 | LSASSQR | 2 | y6 monochargé | 1,2 | 374,7 | 635,31 | 17,1 | 2000 |
| 127 | LSESQLSGR | 2 | y8 dichargé | 11,6 | 488,76 | 432,22 | 23,6 | 2000 |
| 128 | LSESQLSGR | 2 | y6 monochargé | 11,6 | 488,76 | 647,35 | 23,6 | 2000 |
| 129 | LSESQLSGR | 2 | y7 monochargé | 11,6 | 488,76 | 776,39 | 23,6 | 2000 |
| 130 | LSESQLSGSVGMIEMDLASGR | 3 | y3 monochargé | 23,7 | 723,02 | 319,17 | 29,5 | 2000 |
| 131 | LSESQLSGSVGMIEMDLASGR | 3 | y4 monochargé | 23,7 | 723,02 | 390,21 | 29,5 | 2000 |
| 132 | LSESQLSGSVGMIEMDLASGR | 3 | y6 monochargé | 23,7 | 723,02 | 618,32 | 29,5 | 3000 |
| 133 | MVVIYLR | 2 | y3 monochargé | 19,5 | 447,27 | 451,27 | 21,2 | 3000 |
| 134 | MVVIYLR | 2 | y4 monochargé | 19,5 | 447,27 | 564,35 | 21,2 | 3000 |
| 135 | MVVIYLR | 2 | y5 monochargé | 19,5 | 447,27 | 663,42 | 21,2 | 2000 |
| 136 | NEALPGDAR | 2 | y5 monochargé | 10,9 | 471,74 | 515,26 | 22,6 | 2000 |
| 137 | NEALPGDAR | 2 | y6 monochargé | 10,9 | 471,74 | 628,34 | 22,6 | 2000 |
| 138 | NEALPGDAR | 2 | y7 monochargé | 10,9 | 471,74 | 699,38 | 22,6 | 2000 |
| 139 | NQHIAGIGAALIEHWQR | 3 | y13 dichargé | 20,1 | 638,68 | 711,39 | 26,9 | 2000 |
| 140 | NQHIAGIGAALIEHWQR | 3 | y14 dichargé | 20,1 | 638,68 | 767,93 | 26,9 | 2000 |
| 141 | NQHIAGIGAALIEHWQR | 3 | y15 dichargé | 20,1 | 638,68 | 836,46 | 26,9 | 2000 |
| 142 | NQQIAGIGAALIEHWQR | 3 | y13 dichargé | 22,3 | 635,67 | 711,39 | 26,8 | 2000 |
| 143 | NQQIAGIGAALIEHWQR | 3 | y14 dichargé | 22,3 | 635,67 | 767,93 | 26,8 | 2000 |
| 144 | NQQIAGIGAALIEHWQR | 3 | y15 dichargé | 22,3 | 635,67 | 831,96 | 26,8 | 2000 |
| 145 | NQQIAGLGAALIEHWQR | 3 | y13 dichargé | 22,7 | 635,67 | 711,39 | 26,8 | 2000 |
| 146 | NQQIAGLGAALIEHWQR | 3 | y14 dichargé | 22,7 | 635,67 | 767,93 | 26,8 | 2000 |
| 147 | NQQIAGLGAALIEHWQR | 3 | y15 dichargé | 22,6 | 635,67 | 831,96 | 26,8 | 2000 |
| 148 | NTTTPASMAATLR | 3 | y4 monochargé | 16 | 445,56 | 460,29 | 20,9 | 2000 |
| 149 | NTTTPASMAATLR | 3 | y5 monochargé | 16 | 445,56 | 531,33 | 20,9 | 2000 |
| 150 | NTTTPASMAATLR | 2 | y9 monochargé | 16 | 667, 84 | 917,49 | 33,8 | 2000 |
| 151 | NVLTSQR | 2 | y3 monochargé | 10,2 | 409,23 | 390,21 | 19,1 | 2000 |
| 152 | NVLTSQR | 2 | y4 monochargé | 10,2 | 409,23 | 491,26 | 19,1 | 2000 |
| 153 | NVLTSQR | 2 | y5 monochargé | 10,2 | 409,23 | 604,34 | 19,1 | 2000 |
| 154 | QIDDNVTR | 2 | y4 monochargé | 10 | 480,74 | 489,28 | 23,1 | 2000 |
| 155 | QIDDNVTR | 2 | y5 monochargé | 10 | 480,74 | 604,31 | 23,1 | 2000 |
| 156 | QIDDNVTR | 2 | y6 monochargé | 10 | 480,74 | 719,33 | 23,1 | 2000 |
| 157 | QIGDK | 3 | y4 monochargé | 1,1 | 187,44 | 432,25 | 12,9 | 2000 |
| 158 | QIGDK | 2 | y3 monochargé | 1,1 | 280,66 | 319,16 | 11,7 | 2000 |
| 159 | QIGDK | 2 | y4 monochargé | 1,1 | 280,66 | 432,25 | 11,7 | 2000 |
| 160 | QIGDNVTR | 2 | y3 monochargé | 10,2 | 451,74 | 375,24 | 21,5 | 2000 |
| 161 | QIGDNVTR | 2 | y4 monochargé | 10,2 | 451,74 | 489,28 | 21,5 | 2000 |
| 162 | QIGDNVTR | 2 | y6 monochargé | 10,2 | 451,74 | 661,33 | 21,5 | 2000 |
| 163 | QIGENVTR | 2 | y3 monochargé | 10,3 | 458,75 | 375,24 | 21,9 | 2000 |
| 164 | QIGENVTR | 2 | y4 monochargé | 10,3 | 458,75 | 489,28 | 21,9 | 2000 |
| 165 | QIGENVTR | 2 | y6 monochargé | 10,3 | 458,75 | 675,34 | 21,9 | 2000 |
| 166 | QLLQWMVDAR | 2 | y5 monochargé | 22,1 | 630,33 | 591,29 | 31,7 | 2000 |
| 167 | QLLQWMVDAR | 2 | y6 monochargé | 22,1 | 630,33 | 777,37 | 31,7 | 2000 |
| 168 | QLLQWMVDAR | 2 | y7 monochargé | 22,1 | 630,33 | 905,43 | 31,7 | 2000 |
| 169 | QLLQWMVDDGVAGPLIR | 3 | y4 monochargé | 25,7 | 637,68 | 498,34 | 26,8 | 2000 |
| 170 | QLLQWMVDDGVAGPLIR | 3 | y5 monochargé | 25,8 | 637,68 | 555,36 | 26,8 | 2000 |
| 171 | QLLQWMVDDGVAGPLIR | 3 | y6 monochargé | 25,7 | 637,68 | 626,4 | 26,8 | 2000 |
| 172 | QLLQWMVDDR | 2 | y4 monochargé | 21,7 | 652,33 | 504,24 | 32,9 | 2000 |
| 173 | QLLQWMVDDR | 2 | y5 monochargé | 21,7 | 652,33 | 635,28 | 32,9 | 2000 |
| 174 | QLLQWMVDDR | 2 | y6 monochargé | 21,7 | 652,33 | 821,36 | 32,9 | 2000 |
| 175 | QLLQWMVDGR | 2 | y4 monochargé | 21,4 | 623,32 | 446,24 | 31,3 | 2000 |
| 176 | QLLQWMVDGR | 2 | y5 monochargé | 21,4 | 623,32 | 577,28 | 31,3 | 2000 |
| 177 | QLLQWMVDGR | 2 | y6 monochargé | 21,4 | 623,32 | 763,36 | 31,3 | 2000 |
| 178 | QLLQWMVEDR | 3 | y4 monochargé | 21,7 | 439,89 | 518,26 | 20,7 | 2000 |
| 179 | QLLQWMVEDR | 2 | y5 monochargé | 21,7 | 659,34 | 649,3 | 33,3 | 2000 |
| 180 | QLLQWMVEDR | 2 | y6 monochargé | 21,7 | 659,34 | 835,38 | 33,3 | 2000 |
| 181 | QQDLVDYSPVSEK | 3 | y5 monochargé | 16 | 503,25 | 559,31 | 22,7 | 2000 |
| 182 | QQDLVDYSPVSEK | 2 | y5 monochargé | 16 | 754,37 | 559,31 | 38,7 | 2000 |
| 183 | QQDLVDYSPVSEK | 2 | y8 monochargé | 16 | 754,37 | 924,43 | 38,7 | 2000 |
| 184 | QQHLVDYSPVSEK | 3 | y6 dichargé | 14,2 | 510,59 | 323,67 | 22,9 | 2000 |
| 185 | QQHLVDYSPVSEK | 3 | y5 monochargé | 14,2 | 510,59 | 559,31 | 22,9 | 2000 |
| 186 | QQHLVDYSPVSEK | 3 | y6 monochargé | 14,3 | 510,59 | 646,34 | 22,9 | 2000 |
| 187 | QSESQLSGR | 2 | y3 monochargé | 7,2 | 496,24 | 319,17 | 24 | 2000 |
| 188 | QSESQLSGR | 2 | y8 dichargé | 7,2 | 496,24 | 432,22 | 24 | 2000 |
| 189 | QSESQLSGR | 2 | y4 monochargé | 7,2 | 496,24 | 432,26 | 24 | 2000 |
| 190 | QSESQLSGSVGMIEMDLASGR | 3 | y3 monochargé | 22,3 | 728,01 | 319,17 | 29,7 | 2000 |
| 191 | QSESQLSGSVGMIEMDLASGR | 3 | y6 monochargé | 22,3 | 728,01 | 618,32 | 29,7 | 2000 |
| 192 | QSESQLSGSVGMIEMDLASGR | 3 | y8 monochargé | 22,3 | 728,01 | 878,4 | 29,7 | 2000 |
| 193 | SQLQLLQWMVDDR | 3 | y4 monochargé | 24,9 | 544,61 | 504,24 | 24 | 2000 |
| 194 | SQLQLLQWMVDDR | 3 | y5 monochargé | 24,9 | 544,61 | 635,28 | 24 | 2000 |
| 195 | SQLQLLQWMVDDR | 3 | y6 monochargé | 24,9 | 544,61 | 821,36 | 24 | 2000 |
| 196 | SVLPAGWFIADK | 2 | y9 dichargé | 23,1 | 652,36 | 502,76 | 32,9 | 2000 |
| 197 | SVLPAGWFIADK | 2 | y10 dichargé | 23,1 | 652,36 | 559,31 | 32,9 | 2000 |
| 198 | SVLPAGWFIADK | 2 | y9 monochargé | 23,1 | 652,36 | 1004,52 | 32,9 | 2000 |
| 199 | SVLPAGWFIADR | 2 | y9 dichargé | 23,4 | 666,36 | 516,77 | 33,7 | 2000 |
| 200 | SVLPAGWFIADR | 2 | y10 dichargé | 23,4 | 666,36 | 573,31 | 33,7 | 2000 |
| 201 | SVLPAGWFIADR | 2 | y9 monochargé | 23,4 | 666,36 | 1032,53 | 33,7 | 2000 |
| 202 | SVLSAGWFIADK | 2 | y7 monochargé | 22,3 | 647,35 | 836,43 | 32,6 | 2000 |
| 203 | SVLSAGWFIADK | 2 | y8 monochargé | 22,3 | 647,35 | 907,47 | 32,6 | 2000 |
| 204 | SVLSAGWFIADK | 2 | y9 monochargé | 22,3 | 647,35 | 994,5 | 32,6 | 2000 |
| 205 | TGAAER | 2 | y3 monochargé | 1 | 302,66 | 375,2 | 13 | 2000 |
| 206 | TGAAER | 2 | y4 monochargé | 1 | 302,66 | 446,24 | 13 | 2000 |
| 207 | TGAAER | 2 | y5 monochargé | 1 | 302,66 | 503,26 | 13 | 2000 |
| 208 | TGAAK | 2 | y4 dichargé | 0,8 | 224,13 | 173,61 | 8,5 | 2000 |
| 209 | TGAAK | 2 | y3 monochargé | 0,8 | 224,13 | 289,19 | 8,5 | 2000 |
| 210 | TGAAK | 2 | y4 monochargé | 0,8 | 224,13 | 346,21 | 8,5 | 2000 |
| 211 | TGAGER | 2 | y3 monochargé | 1 | 295,65 | 361,18 | 12,6 | 2000 |
| 212 | TGAGER | 2 | y4 monochargé | 1 | 295,65 | 432,22 | 12,6 | 2000 |
| 213 | TGAGER | 2 | y5 monochargé | 1 | 295,65 | 489,24 | 12,6 | 2000 |
| 214 | TGAGK | 3 | y4 monochargé | 0,7 | 145,09 | 332,19 | 11,6 | 2000 |
| 215 | TGAGK | 2 | y3 monochargé | 0,7 | 217,12 | 275,17 | 8,1 | 2000 |
| 216 | TGAGK | 2 | y4 monochargé | 0,7 | 217,12 | 332,19 | 8,1 | 2000 |
| 217 | TGASER | 2 | y3 monochargé | 1 | 310,65 | 391,19 | 13,4 | 2000 |
| 218 | TGASER | 2 | y4 monochargé | 1 | 310,65 | 462,23 | 13,4 | 2000 |
| 219 | TGASER | 2 | y5 monochargé | 1 | 310,65 | 519,25 | 13,4 | 2000 |
| 220 | TGASK | 2 | y4 dichargé | 0,8 | 232,13 | 181,61 | 9 | 2000 |
| 221 | TGASK | 2 | y3 monochargé | 0,8 | 232,13 | 305,18 | 9 | 2000 |
| 222 | TGASK | 2 | y4 monochargé | 0,8 | 232,13 | 362,2 | 9 | 2000 |
| 223 | TGASR | 3 | y4 monochargé | 0,8 | 164,42 | 390,21 | 12,2 | 2000 |
| 224 | TGASR | 2 | y3 monochargé | 0,8 | 246,13 | 333,19 | 9,8 | 2000 |
| 225 | TGASR | 2 | y4 monochargé | 0,8 | 246,13 | 390,21 | 9,8 | 2000 |
| 226 | TLTAWCADER | 2 | y5 monochargé | 15,5 | 611,78 | 650,26 | 30,6 | 5200 |
| 227 | TLTAWCADER | 2 | y6 monochargé | 15,5 | 611,78 | 836,34 | 30,6 | 5200 |
| 228 | TLTAWCADER | 2 | y8 monochargé | 15,5 | 611,78 | 1008,42 | 30,6 | 5200 |
| 229 | TLTAWHADER | 3 | y6 dichargé | 13,1 | 400,53 | 407,19 | 19,5 | 2000 |
| 230 | TLTAWHADER | 3 | y8 dichargé | 13,1 | 400,53 | 493,23 | 19,5 | 2000 |
| 231 | TLTAWHADER | 3 | y5 monochargé | 13,1 | 400,53 | 627,28 | 19,5 | 2000 |
| 232 | TLTAWR | 2 | y3 monochargé | 14,6 | 374,21 | 432,24 | 17,1 | 2000 |
| 233 | TLTAWR | 2 | y4 monochargé | 14,6 | 374,21 | 533,28 | 17,1 | 2000 |
| 234 | TLTAWR | 2 | y5 monochargé | 14,6 | 374,21 | 646,37 | 17,1 | 2000 |
| 235 | TVGGPAGLTAFLR | 2 | y5 monochargé | 22 | 630,36 | 607,36 | 31,7 | 2000 |
| 236 | TVGGPAGLTAFLR | 2 | y7 monochargé | 22 | 630,36 | 777,46 | 31,7 | 2000 |
| 237 | TVGGPAGLTAFLR | 2 | y11 monochargé | 22 | 630,36 | 1059,6 | 31,7 | 2000 |
| 238 | TWIYLR | 2 | y3 monochargé | 17,5 | 432,27 | 451,27 | 20,4 | 2000 |
| 239 | TWIYLR | 2 | y4 monochargé | 17,5 | 432,27 | 564,35 | 20,4 | 2000 |
| 240 | TWIYLR | 2 | y5 monochargé | 17,4 | 432,27 | 663,42 | 20,4 | 2000 |
| 241 | VAGPLIR | 2 | y4 monochargé | 13,8 | 363,24 | 498,34 | 16,4 | 2000 |
| 242 | VAGPLIR | 2 | y5 monochargé | 13,8 | 363,24 | 555,36 | 16,4 | 2000 |
| 243 | VAGPLIR | 2 | y6 monochargé | 13,8 | 363,24 | 626,4 | 16,4 | 2000 |
| 244 | VALCGAVLAR | 2 | y8 dichargé | 16,4 | 515,3 | 430,25 | 25,1 | 2000 |
| 245 | VALCGAVLAR | 2 | y6 monochargé | 16,4 | 515,3 | 586,37 | 25,1 | 2000 |
| 246 | VALCGAVLAR | 2 | y7 monochargé | 16,4 | 515,3 | 746,4 | 25,1 | 2000 |
| 247 | VDAGDEQLER | 2 | y5 monochargé | 11 | 566,27 | 674,35 | 28 | 2000 |
| 248 | VDAGDEQLER | 2 | y7 monochargé | 11 | 566,27 | 846,4 | 28 | 2000 |
| 249 | VDAGDEQLER | 2 | y8 monochargé | 11 | 566,27 | 917,43 | 28 | 2000 |
| 250 | VDAGDK | 2 | y3 monochargé | 1 | 302,65 | 319,16 | 13 | 2000 |
| 251 | VDAGDK | 2 | y4 monochargé | 1 | 302,65 | 390,2 | 13 | 2000 |
| 252 | VDAGDK | 2 | y5 monochargé | 1 | 302,65 | 505,23 | 13 | 2000 |
| 253 | VGMIEMDLASGR | 2 | y6 monochargé | 19,3 | 639,81 | 618,32 | 32,2 | 2000 |
| 254 | VGMIEMDLASGR | 2 | y7 monochargé | 19,3 | 639,81 | 749,36 | 32,2 | 2000 |
| 255 | VGMIEMDLASGR | 2 | y8 monochargé | 19,3 | 639,81 | 878,4 | 32,2 | 2000 |
| 256 | VGMIEMDLASR | 2 | y6 monochargé | 19,2 | 611,3 | 692,34 | 30,6 | 2000 |
| 257 | VGMIEMDLASR | 2 | y7 monochargé | 19,2 | 611,3 | 821,38 | 30,6 | 2000 |
| 258 | VGMIEMDLASR | 2 | y8 monochargé | 19,2 | 611,3 | 934,47 | 30,6 | 2000 |
| 259 | VGMIEMDLASSR | 2 | y6 monochargé | 18,8 | 654,82 | 648,33 | 33,1 | 2000 |
| 260 | VGMIEMDLASSR | 2 | y7 monochargé | 18,8 | 654,82 | 779,37 | 33,1 | 2000 |
| 261 | VGMIEMDLASSR | 2 | y8 monochargé | 18,8 | 654,82 | 908,41 | 33,1 | 2000 |
| 262 | VLLCGAVLAR | 2 | y6 monochargé | 18,2 | 536,32 | 586,37 | 26,3 | 5400 |
| 263 | VLLCGAVLAR | 2 | y7 monochargé | 18,3 | 536,32 | 746,4 | 26,3 | 5400 |
| 264 | VLLCGAVLAR | 2 | y8 monochargé | 18,3 | 536,32 | 859,48 | 26,3 | 5400 |
| 265 | WLCGAMLAR | 2 | y6 monochargé | 17,7 | 545,3 | 618,34 | 26,8 | 2000 |
| 266 | WLCGAMLAR | 2 | y7 monochargé | 17,7 | 545,3 | 778,37 | 26,8 | 2000 |
| 267 | WLCGAMLAR | 2 | y8 monochargé | 17,7 | 545,3 | 891,45 | 26,8 | 2000 |
| 268 | VVLCGAVLAR | 2 | y6 monochargé | 17,2 | 529,31 | 586,37 | 25,9 | 2000 |
| 269 | VVLCGAVLAR | 2 | y7 monochargé | 17,2 | 529,31 | 746,4 | 25,9 | 2000 |
| 270 | VVLCGAVLAR | 2 | y8 monochargé | 17,2 | 529,31 | 859,48 | 25,9 | 2000 |
| 271 | VVLCGTVLAR | 2 | y6 monochargé | 16,9 | 544,32 | 616,38 | 26,8 | 2000 |
| 272 | VVLCGTVLAR | 2 | y7 monochargé | 16,9 | 544,32 | 776,41 | 26,8 | 2000 |
| 273 | VVLCGTVLAR | 2 | y8 monochargé | 16,9 | 544,32 | 889,49 | 26,8 | 2000 |
| 274 | WETDR | 3 | y4 monochargé | 8,2 | 236,11 | 520,24 | 14,4 | 2000 |
| 275 | WETDR | 2 | y3 monochargé | 8,2 | 353,66 | 391,19 | 15,9 | 2000 |
| 276 | WETDR | 2 | y4 monochargé | 8,2 | 353,66 | 520,24 | 15,9 | 2000 |
| 277 | WETELNEAFPGDAR | 3 | y5 monochargé | 19,1 | 545,59 | 515,26 | 24 | 2000 |
| 278 | WETELNEAFPGDAR | 3 | y6 monochargé | 19,1 | 545,59 | 662,33 | 24 | 2000 |
| 279 | WETELNEAFPGDAR | 2 | y5 monochargé | 19,1 | 817,88 | 515,26 | 42,4 | 2000 |
| 280 | WETELNEALPADAR | 3 | y5 monochargé | 18,5 | 538,93 | 529,27 | 23,8 | 2000 |
| 281 | WETELNEALPADAR | 2 | y5 monochargé | 18,5 | 807,89 | 529,27 | 41,8 | 2000 |
| 282 | WETELNEALPADAR | 2 | y7 monochargé | 18,5 | 807,89 | 713,39 | 41,8 | 2000 |
| 283 | WETELNEALPGDAR | 3 | y5 monochargé | 18,2 | 534,26 | 515,26 | 23,6 | 2000 |
| 284 | WETELNEALPGDAR | 2 | y5 monochargé | 18,2 | 800,88 | 515,26 | 41,4 | 2000 |
| 285 | WETELNEALPGDAR | 2 | y7 monochargé | 18,2 | 800,88 | 699,38 | 41,4 | 2000 |
| 286 | WETELNEALSGDAR | 3 | y5 monochargé | 18,9 | 530,92 | 505,24 | 23,5 | 2000 |
| 287 | WETELNEALSGDAR | 3 | y6 monochargé | 18,9 | 530,92 | 618,32 | 23,5 | 2000 |
| 288 | WETELNEALSGDAR | 2 | y5 monochargé | 18,9 | 795,87 | 505,24 | 41,1 | 2000 |
| 289 | WETELNEVLPGDAR | 3 | y5 monochargé | 20,1 | 543,6 | 515,26 | 23,9 | 2000 |
| 290 | WETELNEVLPGDAR | 2 | y5 monochargé | 20,1 | 814,9 | 515,26 | 42,2 | 2000 |
| 291 | WETELNEVLPGDAR | 2 | y6 monochargé | 20,1 | 814,9 | 628,34 | 42,2 | 2000 |
| 292 | WETER | 3 | y4 monochargé | 9 | 240,78 | 534,25 | 14,5 | 2000 |
| 293 | WETER | 2 | y3 monochargé | 8,9 | 360,67 | 405,21 | 16,3 | 2000 |
| 294 | WETER | 2 | y4 monochargé | 8,9 | 360,67 | 534,25 | 16,3 | 2000 |

Les autres paramètres machine utilisés sont les suivants :
- Type de balayage: MRM
- MRM planifié: oui
- Polarité: Positive
- Source d'ionisation:Turbo V™ (Applied BioSystems)
- Réglage Q1: Filtrage avec résolution unitaire
- Réglage Q3: Filtrage avec résolution unitaire
- Pause inter-scan: 5.00 msec
- Vitesse de balayage: 10 Da/s
- Gaz rideau: 50,00 psi
- Tension de cône: 5500,00 V
- Température de source: 500,00 °C
- Gaz de nébulisation: 50,00 psi
- Gaz chauffant: 50,00 psi
- Gaz de collision induisant dissociation : 9,00 psi
- Remplissage dynamique: activé
- Potentiel d'orifice : 100, 00 V
- (en anglais declustering potential (DP))
- Potentiel d'entrée avant Q0 (EP): 6,00 V
- Potentiel en sortie de cellule de collision : 15 V
- (en anglais cell exit potential (CXP))
- Temps de cycle total: 1 sec
- Fenêtre de détection : 120 sec

Les aires obtenues pour chacune des transitions et pour chacun des microorganismes étudiés ont été mesurées. Lorsque les aires des transitions sont supérieures ou égales au seuil de positivité décrit dans le TABLEAU 47, la détection de la transition est considérée comme positive et est notée « 1 » dans le TABLEAU **48.** Lorsqu'une transition a une aire inférieure au seuil de positivité décrit dans le **TABLEAU 47,** la transition est considérée comme non détectée et est notée « 0 » dans le **TABLEAU 48.**

Pour un peptide donné, lorsque au moins 3 transitions sont notées « 1 », le peptide est considéré comme détecté.

**TABLEAU 48 :**

| transition numéro | Ech131 | Ech132 | Ech133 | Ech134 | Ech135 | Ech136 | Ech137 | Ech138 | Ech139 | Ech140 | Ech141 | Ech142 | Ech143 | Ech |
|---|---|---|---|---|---|---|---|---|---|---|---|---|---|---|
| 1 | 0 | 0 | 0 | 0 | 0 | 0 | 0 | 0 | 0 | 0 | 0 | 0 | 0 | |
| 2 | 0 | 0 | 0 | 0 | 0 | 0 | 0 | 0 | 1 | 0 | 0 | 0 | 1 | |
| 3 | 0 | 0 | 0 | 0 | 0 | 0 | 0 | 0 | 0 | 0 | 0 | 0 | 1 | |
| 4 | 0 | 0 | 0 | 0 | 0 | 0 | 0 | 0 | 0 | 0 | 0 | 0 | 0 | |
| 5 | 0 | 0 | 0 | 0 | 0 | 0 | 0 | 0 | 0 | 0 | 0 | 0 | 0 | |
| 6 | 0 | 0 | 0 | 0 | 0 | 0 | 0 | 0 | 0 | 0 | 0 | 0 | 0 | |
| 7 | 0 | 0 | 0 | 0 | 0 | 0 | 0 | 0 | 0 | 0 | 0 | 0 | 0 | |
| 8 | 0 | 0 | 0 | 0 | 0 | 0 | 0 | 0 | 0 | 0 | 0 | 0 | 0 | |
| 9 | 0 | 0 | 0 | 0 | 0 | 0 | 0 | 0 | 0 | 0 | 0 | 0 | 0 | |
| 10 | 0 | 0 | 0 | 0 | 0 | 0 | 0 | 0 | 0 | 0 | 0 | 0 | 0 | |
| 11 | 0 | 0 | 0 | 0 | 1 | 0 | 0 | 0 | 0 | 0 | 0 | 0 | 0 | |
| 12 | 0 | 0 | 0 | 0 | 1 | 0 | 0 | 0 | 0 | 0 | 0 | 0 | 0 | |
| 13 | 0 | 0 | 0 | 0 | 0 | 0 | 0 | 0 | 0 | 0 | 0 | 0 | 0 | |
| 14 | 0 | 0 | 0 | 0 | 0 | 0 | 0 | 1 | 0 | 0 | 0 | 0 | 1 | |
| 15 | 0 | 0 | 0 | 0 | 0 | 0 | 0 | 0 | 0 | 0 | 0 | 0 | 0 | |
| 16 | 0 | 0 | 0 | 0 | 0 | 0 | 0 | 0 | 0 | 0 | 0 | 0 | 0 | |
| 17 | 1 | 0 | 0 | 0 | 0 | 1 | 1 | 1 | 1 | 1 | 1 | 1 | 1 | |
| 18 | 0 | 0 | 0 | 0 | 0 | 0 | 0 | 0 | 0 | 0 | 0 | 0 | 0 | |
| 19 | 1 | 0 | 0 | 0 | 1 | 1 | 1 | 1 | 1 | 0 | 0 | 1 | 1 | |
| 20 | 1 | 0 | 0 | 0 | 1 | 1 | 1 | 0 | 1 | 0 | 0 | 1 | 1 | |
| 21 | 1 | 0 | 1 | 0 | 1 | 1 | 1 | 0 | 1 | 0 | 0 | 1 | 1 | |
| 22 | 0 | 0 | 0 | 0 | 0 | 0 | 0 | 0 | 0 | 0 | 0 | 0 | 0 | |
| 23 | 0 | 0 | 0 | 0 | 0 | 0 | 0 | 0 | 0 | 0 | 0 | 0 | 0 | |
| 24 | 0 | 0 | 0 | 0 | 0 | 0 | 0 | 0 | 0 | 0 | 0 | 0 | 0 | |
| 25 | 1 | 1 | 1 | 0 | 1 | 1 | 1 | 0 | 1 | 0 | 1 | 1 | 1 | |
| 26 | 1 | 1 | 1 | 0 | 1 | 1 | 1 | 0 | 1 | 1 | 1 | 1 | 1 | |
| 27 | 0 | 1 | 1 | 0 | 1 | 1 | 1 | 0 | 1 | 0 | 1 | 1 | 1 | |
| 28 | 0 | 0 | 0 | 0 | 0 | 0 | 0 | 0 | 0 | 0 | 0 | 0 | 0 | |
| 29 | 0 | 0 | 0 | 0 | 0 | 0 | 0 | 0 | 0 | 0 | 0 | 0 | 0 | |
| 30 | 0 | 0 | 0 | 0 | 0 | 0 | 0 | 0 | 0 | 0 | 0 | 0 | 0 | |
| 31 | 0 | 0 | 0 | 0 | 0 | 1 | 0 | 0 | 0 | 0 | 0 | 0 | 0 | |
| 32 | 0 | 0 | 0 | 0 | 0 | 0 | 0 | 0 | 0 | 0 | 0 | 0 | 0 | |
| 33 | 0 | 0 | 0 | 0 | 0 | 0 | 0 | 0 | 0 | 0 | 0 | 0 | 0 | |
| 34 | 0 | 0 | 0 | 0 | 0 | 0 | 0 | 0 | 0 | 0 | 0 | 0 | 0 | |
| 35 | 0 | 0 | 0 | 0 | 0 | 0 | 0 | 0 | 0 | 0 | 0 | 0 | 0 | |
| 36 | 0 | 0 | 0 | 0 | 0 | 0 | 0 | 0 | 0 | 0 | 0 | 0 | 0 | |
| 37 | 0 | 0 | 0 | 0 | 0 | 0 | 0 | 0 | 0 | 0 | 0 | 0 | 0 | |
| 38 | 0 | 0 | 0 | 0 | 1 | 0 | 0 | 1 | 0 | 0 | 0 | 0 | 1 | |
| 39 | 0 | 0 | 0 | 0 | 0 | 0 | 0 | 0 | 0 | 0 | 0 | 0 | 0 | |
| 40 | 0 | 0 | 0 | 0 | 0 | 0 | 0 | 0 | 0 | 0 | 0 | 0 | 0 | |
| 41 | 0 | 0 | 0 | 0 | 0 | 0 | 0 | 0 | 0 | 0 | 0 | 0 | 0 | |
| 42 | 0 | 0 | 0 | 0 | 0 | 0 | 0 | 0 | 0 | 0 | 0 | 0 | 0 | |
| 43 | 0 | 0 | 0 | 0 | 1 | 1 | 1 | 0 | 1 | 0 | 0 | 1 | 1 | |
| 44 | 0 | 0 | 0 | 0 | 1 | 1 | 1 | 0 | 1 | 0 | 0 | 1 | 1 | |
| 45 | 0 | 0 | 1 | 1 | 1 | 1 | 1 | 0 | 0 | 0 | 0 | 1 | 1 | |
| 46 | 0 | 0 | 0 | 0 | 0 | 0 | 0 | 0 | 0 | 0 | 0 | 0 | 0 | |
| 47 | 0 | 0 | 0 | 0 | 0 | 0 | 0 | 0 | 0 | 0 | 0 | 0 | 0 | |
| 48 | 0 | 0 | 0 | 0 | 0 | 0 | 0 | 0 | 0 | 0 | 0 | 0 | 0 | |
| 49 | 1 | 1 | 1 | 1 | 1 | 1 | 1 | 1 | 1 | 1 | 1 | 1 | 1 | |
| 50 | 1 | 1 | 1 | 1 | 1 | 1 | 1 | 1 | 1 | 1 | 1 | 1 | 1 | |
| 51 | 1 | 1 | 1 | 1 | 1 | 1 | 1 | 1 | 1 | 1 | 1 | 1 | 1 | |
| 52 | 1 | 0 | 0 | 0 | 0 | 0 | 0 | 0 | 0 | 0 | 0 | 0 | 0 | |
| 53 | 0 | 0 | 0 | 0 | 0 | 0 | 0 | 0 | 0 | 0 | 0 | 0 | 0 | |
| 54 | 1 | 0 | 0 | 0 | 0 | 0 | 0 | 0 | 0 | 0 | 0 | 0 | 0 | |
| 55 | 1 | 1 | 1 | 1 | 1 | 1 | 1 | 1 | 1 | 1 | 1 | 1 | 1 | |
| 56 | 1 | 1 | 1 | 1 | 1 | 1 | 1 | 1 | 1 | 1 | 1 | 1 | 1 | |
| 57 | 1 | 1 | 1 | 1 | 1 | 1 | 1 | 1 | 1 | 1 | 1 | 1 | 1 | |
| 58 | 0 | 0 | 0 | 0 | 0 | 0 | 0 | 0 | 0 | 0 | 0 | 0 | 0 | |
| 59 | 0 | 0 | 0 | 0 | 0 | 0 | 0 | 0 | 0 | 0 | 0 | 0 | 0 | |
| 60 | 0 | 0 | 0 | 0 | 0 | 0 | 0 | 0 | 0 | 0 | 0 | 0 | 0 | |
| 61 | 0 | 0 | 0 | 0 | 0 | 0 | 0 | 0 | 0 | 0 | 0 | 0 | 0 | |
| 62 | 0 | 0 | 0 | 0 | 0 | 0 | 0 | 0 | 0 | 0 | 0 | 0 | 0 | |
| 63 | 0 | 0 | 0 | 0 | 0 | 0 | 0 | 0 | 0 | 0 | 0 | 0 | 0 | |
| 64 | 0 | 0 | 0 | 0 | 0 | 0 | 0 | 0 | 0 | 0 | 0 | 0 | 0 | |
| 65 | 0 | 0 | 0 | 0 | 0 | 0 | 0 | 0 | 0 | 0 | 0 | 0 | 0 | |
| 66 | 0 | 0 | 0 | 0 | 0 | 0 | 0 | 0 | 0 | 0 | 0 | 0 | 0 | |
| 67 | 0 | 0 | 0 | 0 | 0 | 0 | 0 | 0 | 0 | 0 | 0 | 0 | 0 | |
| 68 | 0 | 0 | 0 | 0 | 1 | 0 | 0 | 0 | 0 | 0 | 0 | 0 | 0 | |
| 69 | 0 | 0 | 0 | 0 | 0 | 0 | 0 | 0 | 0 | 0 | 0 | 0 | 0 | |
| 70 | 0 | 0 | 0 | 0 | 0 | 0 | 0 | 0 | 0 | 0 | 0 | 0 | 0 | |
| 71 | 0 | 0 | 0 | 0 | 0 | 0 | 0 | 1 | 0 | 0 | 0 | 0 | 0 | |
| 72 | 0 | 0 | 0 | 0 | 0 | 0 | 0 | 0 | 0 | 0 | 0 | 0 | 0 | |
| 73 | 0 | 0 | 0 | 0 | 0 | 0 | 0 | 1 | 0 | 0 | 0 | 0 | 0 | |
| 74 | 0 | 0 | 0 | 0 | 0 | 0 | 0 | 0 | 0 | 0 | 0 | 0 | 0 | |
| 75 | 0 | 0 | 0 | 0 | 0 | 0 | 0 | 0 | 0 | 0 | 0 | 0 | 0 | |
| 76 | 0 | 0 | 0 | 0 | 0 | 0 | 0 | 0 | 0 | 0 | 0 | 0 | 0 | |
| 77 | 0 | 0 | 0 | 0 | 0 | 0 | 0 | 0 | 0 | 0 | 0 | 0 | 0 | |
| 78 | 0 | 0 | 0 | 0 | 0 | 0 | 0 | 0 | 0 | 0 | 0 | 0 | 0 | |
| 79 | 0 | 0 | 0 | 0 | 0 | 0 | 0 | 0 | 0 | 0 | 0 | 0 | 0 | |
| 80 | 0 | 0 | 0 | 0 | 0 | 0 | 0 | 0 | 0 | 0 | 0 | 0 | 0 | |
| 81 | 0 | 0 | 0 | 0 | 0 | 0 | 0 | 0 | 0 | 0 | 0 | 0 | 0 | |
| 82 | 0 | 0 | 0 | 0 | 0 | 0 | 0 | 0 | 0 | 0 | 0 | 0 | 0 | |
| 83 | 0 | 0 | 0 | 0 | 0 | 0 | 0 | 0 | 0 | 0 | 0 | 0 | 0 | |
| 84 | 0 | 0 | 0 | 0 | 0 | 0 | 0 | 0 | 0 | 0 | 0 | 0 | 0 | |
| 85 | 0 | 0 | 0 | 0 | 0 | 0 | 0 | 0 | 0 | 0 | 0 | 0 | 0 | |
| 86 | 0 | 0 | 0 | 0 | 0 | 0 | 0 | 0 | 0 | 0 | 0 | 0 | 0 | |
| 87 | 0 | 0 | 0 | 0 | 0 | 0 | 0 | 0 | 0 | 0 | 0 | 0 | 0 | |
| 88 | 0 | 0 | 0 | 0 | 0 | 0 | 0 | 0 | 0 | 0 | 0 | 0 | 0 | |
| 89 | 0 | 0 | 0 | 0 | 0 | 0 | 0 | 0 | 0 | 0 | 0 | 0 | 0 | |
| 90 | 0 | 0 | 0 | 0 | 0 | 0 | 0 | 0 | 0 | 0 | 0 | 0 | 0 | |
| 91 | 0 | 0 | 0 | 0 | 0 | 0 | 0 | 0 | 0 | 0 | 0 | 0 | 0 | |
| 92 | 0 | 0 | 0 | 0 | 0 | 0 | 0 | 0 | 0 | 0 | 0 | 0 | 0 | |
| 93 | 0 | 0 | 0 | 0 | 0 | 0 | 0 | 0 | 0 | 0 | 0 | 0 | 0 | |
| 94 | 0 | 0 | 0 | 0 | 0 | 0 | 0 | 0 | 0 | 0 | 0 | 0 | 0 | |
| 95 | 0 | 0 | 0 | 0 | 0 | 0 | 0 | 0 | 0 | 0 | 0 | 0 | 0 | |
| 96 | 0 | 0 | 0 | 0 | 0 | 0 | 0 | 0 | 0 | 0 | 0 | 0 | 0 | |
| 97 | 0 | 0 | 0 | 0 | 0 | 0 | 0 | 0 | 0 | 0 | 0 | 0 | 0 | |
| 98 | 0 | 0 | 0 | 0 | 0 | 0 | 0 | 0 | 0 | 0 | 0 | 0 | 0 | |
| 99 | 0 | 0 | 0 | 0 | 0 | 0 | 0 | 0 | 0 | 0 | 0 | 0 | 0 | |
| 100 | 0 | 0 | 0 | 0 | 0 | 0 | 0 | 0 | 0 | 0 | 0 | 0 | 0 | |
| 101 | 0 | 0 | 0 | 0 | 0 | 0 | 0 | 0 | 0 | 0 | 0 | 0 | 0 | |
| 102 | 0 | 0 | 0 | 0 | 0 | 0 | 0 | 0 | 0 | 0 | 0 | 0 | 0 | |
| 103 | 0 | 0 | 0 | 0 | 0 | 0 | 0 | 0 | 0 | 0 | 0 | 0 | 0 | |
| 104 | 0 | 0 | 0 | 0 | 0 | 0 | 0 | 0 | 0 | 0 | 0 | 0 | 0 | |
| 105 | 0 | 0 | 0 | 0 | 0 | 0 | 0 | 0 | 0 | 0 | 0 | 0 | 0 | |
| 106 | 0 | 0 | 0 | 0 | 0 | 0 | 0 | 0 | 0 | 0 | 1 | 0 | 0 | |
| 107 | 0 | 0 | 0 | 0 | 0 | 0 | 0 | 0 | 0 | 0 | 0 | 0 | 0 | |
| 108 | 0 | 0 | 0 | 0 | 0 | 0 | 0 | 0 | 0 | 0 | 0 | 0 | 0 | |
| 109 | 0 | 0 | 0 | 0 | 0 | 0 | 0 | 0 | 0 | 0 | 0 | 0 | 0 | |
| 110 | 0 | 0 | 0 | 0 | 0 | 0 | 0 | 0 | 0 | 0 | 0 | 0 | 0 | |
| 111 | 0 | 0 | 0 | 0 | 0 | 0 | 0 | 0 | 0 | 0 | 0 | 0 | 0 | |
| 112 | 0 | 0 | 0 | 0 | 0 | 0 | 0 | 0 | 0 | 0 | 1 | 1 | 1 | |
| 113 | 0 | 0 | 0 | 0 | 0 | 0 | 0 | 0 | 0 | 0 | 0 | 1 | 0 | |
| 114 | 0 | 0 | 0 | 0 | 0 | 0 | 0 | 0 | 0 | 0 | 0 | 0 | 0 | |
| 115 | 0 | 0 | 0 | 0 | 0 | 0 | 0 | 0 | 0 | 0 | 0 | 0 | 0 | |
| 116 | 0 | 0 | 0 | 0 | 0 | 0 | 0 | 0 | 0 | 0 | 0 | 0 | 0 | |
| 117 | 1 | 0 | 0 | 0 | 0 | 0 | 0 | 0 | 0 | 0 | 0 | 0 | 0 | |
| 118 | 0 | 1 | 1 | 1 | 0 | 0 | 0 | 0 | 0 | 0 | 0 | 1 | 0 | |
| 119 | 0 | 0 | 0 | 0 | 0 | 0 | 0 | 0 | 0 | 0 | 0 | 1 | 0 | |
| 120 | 0 | 0 | 0 | 0 | 0 | 0 | 0 | 0 | 0 | 0 | 0 | 1 | 0 | |
| 121 | 0 | 0 | 0 | 0 | 0 | 0 | 0 | 0 | 0 | 0 | 0 | 0 | 0 | |
| 122 | 0 | 0 | 0 | 0 | 0 | 0 | 0 | 0 | 0 | 0 | 0 | 0 | 0 | |
| 123 | 0 | 0 | 0 | 0 | 0 | 0 | 0 | 0 | 0 | 0 | 0 | 0 | 0 | |
| 124 | 0 | 0 | 0 | 0 | 0 | 0 | 0 | 1 | 0 | 0 | 0 | 0 | 0 | |
| 125 | 0 | 0 | 0 | 0 | 0 | 0 | 0 | 1 | 0 | 0 | 0 | 0 | 1 | |
| 126 | 0 | 0 | 0 | 0 | 0 | 0 | 0 | 1 | 0 | 0 | 0 | 0 | 0 | |
| 127 | 1 | 0 | 0 | 0 | 1 | 0 | 0 | 0 | 0 | 0 | 0 | 1 | 1 | |
| 128 | 1 | 0 | 0 | 0 | 1 | 0 | 0 | 0 | 0 | 0 | 0 | 1 | 1 | |
| 129 | 1 | 0 | 0 | 0 | 1 | 0 | 0 | 0 | 0 | 0 | 0 | 1 | 1 | |
| 130 | 0 | 0 | 0 | 0 | 0 | 0 | 0 | 0 | 0 | 0 | 0 | 0 | 0 | |
| 131 | 0 | 0 | 0 | 0 | 0 | 0 | 0 | 0 | 0 | 0 | 0 | 0 | 0 | |
| 132 | 0 | 0 | 1 | 0 | 0 | 0 | 0 | 0 | 0 | 0 | 0 | 0 | 0 | |
| 133 | 0 | 0 | 0 | 1 | 0 | 0 | 1 | 0 | 0 | 0 | 0 | 0 | 0 | |
| 134 | 0 | 1 | 0 | 0 | 0 | 0 | 0 | 0 | 0 | 0 | 1 | 1 | 0 | |
| 135 | 0 | 0 | 0 | 1 | 0 | 0 | 1 | 0 | 1 | 1 | 1 | 1 | 1 | |
| 136 | 0 | 0 | 0 | 0 | 0 | 0 | 0 | 0 | 0 | 0 | 0 | 0 | 0 | |
| 137 | 0 | 0 | 0 | 0 | 0 | 0 | 0 | 0 | 0 | 0 | 0 | 0 | 0 | |
| 138 | 1 | 0 | 0 | 0 | 1 | 1 | 1 | 0 | 1 | 1 | 1 | 1 | 1 | |
| 139 | 0 | 0 | 0 | 0 | 0 | 1 | 0 | 0 | 0 | 0 | 0 | 0 | 0 | |
| 140 | 0 | 0 | 0 | 0 | 0 | 1 | 1 | 1 | 1 | 1 | 1 | 1 | 0 | |
| 141 | 0 | 0 | 0 | 0 | 0 | 0 | 0 | 0 | 0 | 0 | 0 | 0 | 0 | |
| 142 | 0 | 1 | 1 | 1 | 1 | 1 | 1 | 0 | 1 | 1 | 1 | 1 | 1 | |
| 143 | 0 | 1 | 1 | 1 | 1 | 1 | 1 | 0 | 1 | 1 | 1 | 1 | 1 | |
| 144 | 0 | 1 | 1 | 1 | 1 | 1 | 1 | 0 | 1 | 1 | 1 | 1 | 1 | |
| 145 | 0 | 1 | 1 | 1 | 1 | 1 | 1 | 1 | 1 | 1 | 1 | 1 | 1 | |
| 146 | 0 | 1 | 1 | 1 | 1 | 1 | 1 | 1 | 1 | 1 | 1 | 1 | 1 | |
| 147 | 0 | 1 | 1 | 1 | 1 | 1 | 1 | 1 | 1 | 1 | 1 | 1 | 1 | |
| 148 | 0 | 0 | 0 | 0 | 0 | 0 | 0 | 0 | 0 | 0 | 0 | 1 | 0 | |
| 149 | 1 | 1 | 1 | 1 | 1 | 1 | 1 | 1 | 1 | 1 | 1 | 1 | 0 | |
| 150 | 0 | 0 | 0 | 0 | 0 | 0 | 0 | 0 | 0 | 0 | 0 | 1 | 0 | |
| 151 | 0 | 0 | 0 | 0 | 0 | 0 | 0 | 0 | 0 | 0 | 0 | 0 | 0 | |
| 152 | 0 | 0 | 0 | 0 | 0 | 1 | 0 | 0 | 0 | 0 | 0 | 0 | D | |
| 153 | 0 | 0 | 0 | 0 | 0 | 0 | 0 | 0 | 0 | 0 | 0 | 0 | 0 | |
| 154 | 0 | 0 | 0 | 0 | 0 | 0 | 0 | 0 | 0 | 0 | 0 | 0 | 0 | |
| 155 | 0 | 0 | 0 | 0 | 0 | 0 | 0 | 0 | 0 | 0 | 0 | 0 | 0 | |
| 156 | 0 | 0 | 0 | 0 | 0 | 0 | 0 | 0 | 0 | 0 | 0 | 0 | 0 | |
| 157 | 0 | 0 | 0 | 0 | 0 | 0 | 0 | 0 | 0 | 0 | 0 | 0 | 0 | |
| 158 | 0 | 0 | 0 | 0 | 0 | 0 | 0 | 0 | 1 | 0 | 0 | 0 | 1 | |
| 159 | 0 | 0 | 0 | 0 | 0 | 0 | 0 | 0 | 1 | 0 | 0 | 0 | 1 | |
| 160 | 1 | 0 | 0 | 0 | 1 | 1 | 1 | 0 | 1 | 0 | 0 | 1 | 1 | |
| 161 | 1 | 1 | 1 | 0 | 1 | 1 | 1 | 1 | 1 | 0 | 0 | 1 | 1 | |
| 162 | 0 | 0 | 0 | 0 | 1 | 1 | 1 | 0 | 0 | 0 | 0 | 1 | 1 | |
| 163 | 0 | 0 | 0 | 0 | 1 | 0 | 0 | 0 | 0 | 0 | 0 | 0 | 0 | |
| 164 | 0 | 0 | 0 | 0 | 0 | 0 | 0 | 0 | 0 | 0 | 0 | 0 | 0 | |
| 165 | 1 | 0 | 0 | 0 | 0 | 0 | 0 | 0 | 0 | 0 | 0 | 0 | 0 | |
| 166 | 0 | 0 | 0 | 0 | 0 | 0 | 0 | 0 | 0 | 0 | 0 | 0 | 0 | |
| 167 | 0 | 0 | 0 | 0 | 0 | 0 | 0 | 0 | 0 | 0 | 0 | 0 | 0 | |
| 168 | 0 | 0 | 0 | 0 | 0 | 0 | 0 | 0 | 0 | 0 | 0 | 0 | 0 | |
| 169 | 0 | 0 | 0 | 0 | 0 | 0 | 0 | 0 | 0 | 0 | 0 | 0 | 0 | |
| 170 | 0 | 0 | 0 | 0 | 0 | 0 | 0 | 0 | 0 | 0 | 0 | 0 | 0 | |
| 171 | 0 | 0 | 0 | 0 | 0 | 0 | 0 | 0 | 0 | 0 | 0 | 0 | 0 | |
| 172 | 0 | 0 | 0 | 0 | 0 | 0 | 0 | 1 | 0 | 0 | 0 | 0 | 0 | |
| 173 | 0 | 0 | 0 | 0 | 0 | 0 | 0 | 0 | 1 | 0 | 0 | 0 | 0 | |
| 174 | 0 | 0 | 0 | 0 | 0 | 1 | 0 | 0 | 1 | 0 | 1 | 0 | 0 | |
| 175 | 0 | 0 | 0 | 0 | 0 | 0 | 0 | 0 | 0 | 0 | 0 | 0 | 0 | |
| 176 | 0 | 0 | 1 | 0 | 0 | 0 | 0 | 0 | 0 | 0 | 0 | 0 | 0 | |
| 177 | 0 | 0 | 0 | 0 | 0 | 0 | 0 | 0 | 0 | 0 | 0 | 0 | 0 | |
| 178 | 0 | 0 | 0 | 0 | 0 | 0 | 0 | 0 | 0 | 0 | 0 | 0 | 0 | |
| 179 | 0 | 0 | 0 | 0 | 0 | 0 | 0 | 0 | 0 | 0 | 0 | 0 | 0 | |
| 180 | 0 | 0 | 0 | 0 | 0 | 0 | 0 | 0 | 0 | 0 | 0 | 0 | 0 | |
| 181 | 0 | 0 | 0 | 0 | 0 | 0 | 1 | 0 | 0 | 0 | 0 | 1 | 1 | |
| 182 | 1 | 0 | 0 | 0 | 1 | 1 | 1 | 0 | 1 | 0 | 0 | 1 | 1 | |
| 183 | 0 | 0 | 0 | 0 | 1 | 1 | 1 | 0 | 0 | 0 | 0 | 1 | 1 | |
| 184 | 0 | 0 | 0 | 0 | 0 | 0 | 0 | 0 | 0 | 0 | 0 | 0 | 0 | |
| 185 | 0 | 0 | 0 | 0 | 0 | 0 | 0 | 0 | 0 | 0 | 0 | 0 | 0 | |
| 186 | 0 | 0 | 0 | 0 | 0 | 0 | 0 | 0 | 0 | 0 | 0 | 0 | 0 | |
| 187 | 0 | 0 | 0 | 0 | 0 | 0 | 0 | 0 | 0 | 0 | 0 | 0 | 0 | |
| 188 | 0 | 0 | 0 | 0 | 0 | 0 | 0 | 0 | 0 | 0 | 0 | 0 | 0 | |
| 189 | 0 | 0 | 0 | 0 | 0 | 0 | 0 | 0 | 0 | 0 | 0 | 0 | 0 | |
| 190 | 0 | 0 | 0 | 0 | 0 | 0 | 0 | 0 | 0 | 0 | 0 | 0 | 0 | |
| 191 | 0 | 0 | 0 | 0 | 0 | 0 | 0 | 0 | 0 | 0 | 0 | 0 | 0 | |
| 192 | 0 | 0 | 0 | 0 | 0 | 0 | 0 | 0 | 0 | 0 | 0 | 0 | 0 | |
| 193 | 0 | 0 | 0 | 0 | 0 | 0 | 0 | 0 | 0 | 0 | 0 | 0 | 0 | |
| 194 | 0 | 0 | 0 | 0 | 0 | 0 | 0 | 0 | 0 | 0 | 0 | 0 | 0 | |
| 195 | 0 | 0 | 0 | 0 | 0 | 0 | 0 | 0 | 0 | 0 | 0 | 0 | 0 | |
| 196 | 0 | 0 | 0 | 0 | 0 | 0 | 0 | 0 | 0 | 0 | 0 | 0 | 0 | |
| 197 | 0 | 0 | 0 | 0 | 0 | 0 | 0 | 0 | 0 | 0 | 0 | 0 | 0 | |
| 198 | 0 | 0 | 0 | 0 | 0 | 0 | 0 | 0 | 0 | 0 | 0 | 0 | 0 | |
| 199 | 0 | 0 | 0 | 0 | 0 | 0 | 0 | 0 | 0 | 0 | 0 | 0 | 0 | |
| 200 | 0 | 0 | 0 | 0 | 0 | 0 | 0 | 0 | 0 | 0 | 0 | 0 | 0 | |
| 201 | 0 | 0 | 0 | 0 | 0 | 0 | 0 | 0 | 0 | 0 | 0 | 0 | 0 | |
| 202 | 0 | 0 | 0 | 0 | 0 | 0 | 0 | 0 | 0 | 0 | 0 | 0 | 0 | |
| 203 | 0 | 0 | 0 | 0 | 0 | 0 | 0 | 0 | 0 | 0 | 0 | 0 | 0 | |
| 204 | 0 | 0 | 0 | 0 | 0 | 0 | 0 | 0 | 0 | 0 | 0 | 0 | 0 | |
| 205 | 1 | 1 | 1 | 1 | 1 | 0 | 1 | 1 | 0 | 0 | 0 | 0 | 0 | |
| 206 | 1 | 1 | 1 | 1 | 1 | 0 | 1 | 1 | 0 | 1 | 1 | 1 | 0 | |
| 207 | 1 | 1 | 1 | 1 | 1 | 1 | 0 | 1 | 0 | 1 | 1 | 1 | 0 | |
| 208 | 0 | 0 | 0 | 0 | 0 | 0 | 0 | 0 | 0 | 0 | 0 | 0 | 0 | |
| 209 | 0 | 0 | 0 | 0 | 0 | 0 | 0 | 0 | 0 | 0 | 0 | 0 | 0 | |
| 210 | 0 | 0 | 0 | 0 | 0 | 0 | 0 | 0 | 0 | 0 | 0 | 0 | 0 | |
| 211 | 0 | 0 | 0 | 0 | 0 | 0 | 0 | 0 | 0 | 0 | 0 | 0 | 0 | |
| 212 | 0 | 0 | 0 | 0 | 0 | 0 | 0 | 0 | 0 | 0 | 0 | 0 | 1 | |
| 213 | 0 | 0 | 0 | 0 | 0 | 0 | 0 | 0 | 1 | 0 | 0 | 0 | 1 | |
| 214 | 0 | 0 | 0 | 0 | 0 | 0 | 0 | 0 | 0 | 0 | 0 | 0 | 0 | |
| 215 | 0 | 0 | 0 | 0 | 0 | 0 | 0 | 0 | 0 | 0 | 0 | 0 | 0 | |
| 216 | 0 | 0 | 0 | 0 | 0 | 0 | 0 | 0 | 0 | 0 | 0 | 0 | 0 | |
| 217 | 0 | 0 | 0 | 0 | 0 | 0 | 0 | 0 | 0 | 0 | 0 | 0 | 1 | |
| 218 | 0 | 1 | 0 | 0 | 0 | 1 | 0 | 0 | 0 | 0 | 1 | 1 | 0 | |
| 219 | 0 | 0 | 1 | 1 | 0 | 0 | 0 | 0 | 0 | 0 | 0 | 0 | 0 | |
| 220 | 0 | 0 | 0 | 0 | 0 | 0 | 0 | 0 | 0 | 0 | 0 | 0 | 0 | |
| 221 | 0 | 0 | 0 | 0 | 0 | 0 | 0 | 0 | 0 | 0 | 0 | 0 | 0 | |
| 222 | 0 | 0 | 0 | 0 | 0 | 0 | 0 | 0 | 0 | 0 | 0 | 0 | 0 | |
| 223 | 0 | 0 | 0 | 0 | 0 | 0 | 0 | 0 | 0 | 0 | 0 | 0 | 0 | |
| 224 | 0 | 0 | 0 | 0 | 0 | 0 | 0 | 0 | 0 | 0 | 0 | 0 | 0 | |
| 225 | 0 | 0 | 0 | 0 | 0 | 0 | 0 | 0 | 0 | 0 | 0 | 0 | 0 | |
| 226 | 1 | 0 | 0 | 0 | 0 | 0 | 1 | 1 | 0 | 0 | 0 | 0 | 0 | |
| 227 | 0 | 0 | 0 | 0 | 0 | 0 | 0 | 0 | 0 | 0 | 0 | 0 | 0 | |
| 228 | 1 | 0 | 0 | 0 | 0 | 0 | 1 | 0 | 0 | 1 | 0 | 0 | 0 | |
| 229 | 0 | 0 | 0 | 0 | 0 | 0 | 0 | 0 | 0 | 0 | 0 | 0 | 0 | |
| 230 | 0 | 0 | 0 | 0 | 0 | 0 | 0 | 0 | 0 | 0 | 0 | 0 | 0 | |
| 231 | 0 | 0 | 0 | 0 | 0 | 0 | 0 | 0 | 0 | 0 | 0 | 0 | 0 | |
| 232 | 0 | 0 | 1 | 0 | 1 | 1 | 1 | 0 | 0 | 0 | 0 | 1 | 1 | |
| 233 | 0 | 0 | 1 | 0 | 1 | 1 | 1 | 0 | 0 | 0 | 0 | 1 | 1 | |
| 234 | 0 | 0 | 0 | 0 | 1 | 1 | 1 | 0 | 0 | 0 | 0 | 1 | 1 | |
| 235 | 0 | 0 | 0 | 0 | 0 | 0 | 0 | 0 | 0 | 0 | 0 | 0 | 0 | |
| 236 | 0 | 0 | 0 | 0 | 0 | 0 | 0 | 0 | 0 | 0 | 0 | 0 | 0 | |
| 237 | 0 | 0 | 0 | 0 | 0 | 0 | 0 | 0 | 0 | 0 | 0 | 0 | 0 | |
| 238 | 0 | 0 | 0 | 0 | 0 | 0 | 0 | 0 | 0 | 0 | 0 | 0 | 0 | |
| 239 | 0 | 0 | 0 | 0 | 0 | 0 | 0 | 0 | 0 | 0 | 0 | 0 | 0 | |
| 240 | 0 | 0 | 0 | 0 | 0 | 0 | 0 | 0 | 0 | 0 | 0 | 0 | 0 | |
| 241 | 1 | 1 | 1 | 1 | 1 | 1 | 1 | 1 | 1 | 1 | 1 | 1 | 1 | |
| 242 | 1 | 1 | 1 | 1 | 1 | 1 | 1 | 0 | 1 | 0 | 1 | 1 | 1 | |
| 243 | 1 | 1 | 1 | 1 | 1 | 1 | 1 | 0 | 1 | 0 | 1 | 1 | 1 | |
| 244 | 0 | 0 | 0 | 0 | 0 | 0 | 0 | 0 | 1 | 0 | 0 | 0 | 0 | |
| 245 | 0 | 0 | 0 | 0 | 1 | 0 | 0 | 0 | 0 | 0 | 0 | 0 | 0 | |
| 246 | 0 | 0 | 0 | 0 | 0 | 0 | 0 | 0 | 0 | 0 | 0 | 0 | 0 | |
| 247 | 1 | 0 | 1 | 0 | 1 | 1 | 1 | 0 | 1 | 0 | 1 | 1 | 1 | |
| 248 | 1 | 1 | 1 | 1 | 1 | 1 | 1 | 1 | 1 | 1 | 1 | 1 | 1 | |
| 249 | 1 | 1 | 1 | 1 | 1 | 1 | 1 | 0 | 1 | 1 | 1 | 1 | 1 | |
| 250 | 0 | 0 | 0 | 0 | 0 | 0 | 0 | 0 | 0 | 0 | 0 | 0 | 0 | |
| 251 | 0 | 1 | 1 | 1 | 0 | 0 | 0 | 0 | 0 | 0 | 0 | 0 | 0 | |
| 252 | 0 | 0 | 0 | 0 | 0 | 0 | 0 | 0 | 0 | 0 | 0 | 0 | 0 | |
| 253 | 0 | 0 | 0 | 0 | 1 | 0 | 1 | 0 | 1 | 0 | 0 | 1 | 1 | |
| 254 | 0 | 0 | 0 | 0 | 1 | 0 | 1 | 0 | 1 | 0 | 0 | 1 | 1 | |
| 255 | 0 | 0 | 0 | 0 | 1 | 0 | 1 | 0 | 1 | 0 | 0 | 1 | 1 | |
| 256 | 0 | 0 | 0 | 0 | 0 | 0 | 0 | 0 | 0 | 0 | 0 | 0 | 0 | |
| 257 | 0 | 0 | 0 | 0 | 0 | 0 | 0 | 0 | 0 | 0 | 0 | 0 | 0 | |
| 258 | 0 | 0 | 0 | 0 | 0 | 0 | 0 | 0 | 0 | 0 | 0 | 0 | 0 | |
| 259 | 0 | 0 | 0 | 0 | 0 | 0 | 0 | 0 | 0 | 0 | 0 | 0 | 0 | |
| 260 | 0 | 0 | 0 | 0 | 0 | 0 | 0 | 0 | 0 | 0 | 0 | 0 | 0 | |
| 261 | 0 | 0 | 0 | 0 | 0 | 0 | 0 | 0 | 0 | 0 | 0 | 0 | 0 | |
| 262 | 0 | 0 | 0 | 0 | 0 | 0 | 0 | 0 | 0 | 0 | 0 | 1 | 1 | |
| 263 | 0 | 0 | 0 | 0 | 0 | 0 | 0 | 0 | 0 | 0 | 1 | 0 | 1 | |
| 264 | 0 | 0 | 0 | 0 | 0 | 0 | 0 | 0 | 0 | 0 | 0 | 1 | | |
| 265 | 0 | 0 | 0 | 0 | 0 | 0 | 0 | 0 | 0 | 0 | 0 | 0 | 0 | |
| 266 | 0 | 0 | 0 | 0 | 0 | 0 | 0 | 0 | 0 | 0 | 0 | 0 | 0 | |
| 267 | 0 | 0 | 0 | 0 | 0 | 1 | 0 | 0 | 0 | 0 | 0 | 0 | 0 | |
| 268 | 0 | 0 | 0 | 0 | 0 | 0 | 0 | 0 | 0 | 0 | 0 | 0 | 0 | |
| 269 | 0 | 0 | 0 | 0 | 0 | 0 | 0 | 0 | 0 | 0 | 0 | 0 | 0 | |
| 270 | 0 | 0 | 0 | 0 | 0 | 0 | 0 | 0 | 0 | 0 | 0 | 0 | 0 | |
| 271 | 0 | 0 | 0 | 0 | 0 | 0 | 0 | 0 | 0 | 0 | 0 | 0 | 0 | |
| 272 | 0 | 0 | 0 | 0 | 0 | 0 | 0 | 0 | 0 | 1 | 0 | 0 | 0 | |
| 273 | 0 | 0 | 0 | 0 | 0 | 0 | 0 | 0 | 0 | 1 | 0 | 0 | 0 | |
| 274 | 0 | 0 | 0 | 0 | 0 | 0 | 0 | 0 | 0 | 0 | 0 | 0 | 0 | |
| 275 | 0 | 0 | 0 | 0 | 0 | 0 | 0 | 0 | 0 | 0 | 0 | 0 | 0 | |
| 276 | 0 | 0 | 0 | 0 | 0 | 0 | 0 | 0 | 0 | 0 | 0 | 0 | 0 | |
| 277 | 0 | 0 | 0 | 0 | 0 | 0 | 0 | 0 | 0 | 0 | 0 | 0 | 0 | |
| 278 | 0 | 0 | 0 | 0 | 0 | 0 | 0 | 0 | 0 | 0 | 0 | 0 | 0 | |
| 279 | 0 | 0 | 0 | 0 | 0 | 0 | 0 | 0 | 0 | 0 | 0 | 0 | 0 | |
| 280 | 0 | 0 | 0 | 0 | 1 | 0 | 0 | 0 | 0 | 0 | 0 | 0 | 0 | |
| 281 | 0 | 0 | 0 | 0 | 0 | 0 | 0 | 0 | 0 | 0 | 0 | 0 | 0 | |
| 282 | 0 | 0 | 0 | 0 | 1 | 0 | 0 | 0 | 0 | 0 | 0 | 0 | 0 | |
| 283 | 0 | 0 | 0 | 0 | 1 | 1 | 1 | 0 | 0 | 0 | 0 | 1 | 1 | |
| 284 | 0 | 1 | 1 | 0 | 0 | 1 | 1 | 0 | 0 | 0 | 0 | 1 | 1 | |
| 285 | 0 | 0 | 0 | 0 | 0 | 1 | 1 | 0 | 0 | 0 | 0 | 1 | 1 | |
| 286 | 0 | 0 | 0 | 0 | 0 | 0 | 0 | 0 | 0 | 0 | 0 | 0 | 0 | |
| 287 | 0 | 0 | 0 | 0 | 0 | 0 | 0 | 0 | 0 | 0 | 0 | 0 | 0 | |
| 288 | 0 | 0 | 0 | 0 | 0 | 0 | 0 | 0 | 0 | 0 | 0 | 0 | 0 | |
| 289 | 0 | 0 | 0 | 0 | 0 | 0 | 0 | 0 | 0 | 0 | 0 | 0 | 0 | |
| 290 | 0 | 0 | 0 | 0 | 0 | 0 | 0 | 0 | 0 | 0 | 0 | 0 | 0 | |
| 291 | 0 | 0 | 0 | 0 | 0 | 0 | 0 | 0 | 0 | 0 | 0 | 0 | 0 | |
| 292 | 0 | 0 | 0 | 0 | 0 | 0 | 0 | 0 | 0 | 0 | 0 | 0 | 0 | |
| 293 | 0 | 0 | 0 | 0 | 0 | 0 | 0 | 0 | 0 | 0 | 0 | 0 | 0 | |
| 294 | 0 | 0 | 0 | 0 | 0 | 0 | 0 | 0 | 0 | 0 | 0 | 0 | 0 | |

Les échantillons Ech131 à Ech144 comportent au moins un peptide caractéristique des protéines SHV. Les bactéries présentes dans les échantillons Ech131 à Ech144 expriment donc une béta-lactamase qui leur confère une résistance aux pénicillines. L'échantillon Ech142 comporte un peptide spécifique du phénotype 2be. L'échantillon Ech142 est donc résistant aux pénicillines, aux céphalosporines et aux monobactames.

Aucun peptide spécifique des phénotypes 2b et 2br n'est observé, aucun échantillon testé n'est identifié comme appartenant uniquement à ces phénotypes.

Les méthodes de détection décrites dans les exemples 6 à 30 sont particulièrement avantageuses car elles permettent de doser un grand nombre de peptides et de détecter simultanément la présence d'un ou plusieurs mécanismes de résistance induit par une ou plusieurs béta-lactamases.

En outre, la détection est réalisée en un temps court, inférieur à une heure. En effet, seule la partie du gradient comprise entre 3 et 34 minutes est utile à l'analyse. Les temps de rétention des peptides dosés sont d'ailleurs tous inférieurs à 34 minutes.

De surcroit, les méthodes de détection décrites dans les exemples 6 à 30 sont plus avantageuses que les méthodes de biologie moléculaire car elles détectent le produit de l'expression des gènes et non les gènes eux-mêmes. La détection d'un gène de résistance peut ne pas avoir de sens clinique si ce gène n'est pas exprimé, ou s'il est trop faiblement exprimé pour conduire à une résistance effective. La détection d'un peptide caractérisant une protéine caractéristique d'un mécanisme de résistance n'a pas cet inconvénient.

De façon surprenante, les exemples ci-dessus montrent qu'il est possible d'atteindre par spéctrométrie de masse la sensibilité nécessaire à la détection spécifique de l'existance d'un mécanisme de résistance à au moins un antimicrobien d'un microorganisme contenu dans un échantillon, et ce, sans mettre en oeuvre de méthode d'amplification comme c'est usuellement le cas lorsque des méthodes de biologie moléculaire sont utilisées.

### Références bibliographiques

[1] J. Anhalt & C. Fenselau, 1975, Anal. Chem., 47(2) :219-225.
[2] A. Fox et al, ed., 1990, Analytical microbiology methods : chromatography and mass spectrometry, Plénum Press, New York, N.Y.
[3] M. Claydon et al, 1996, Nature Biotech. 14 :1584-1586.
[4] T. Krishnamurthy & P. Ross, 1996, Rapid Com. Mass Spec., 10 :1992-1996.
[5] P. Seng et al. 2009, Clin. Infect. Dis., 49 :543-551.
[6] C. Fenselau et al., 2008, Appl. Environ. Microbiol., 904-906.
[7] S. Hofstadler et al., 2005, Int..J Mass Spectrom., 242:23-41.
[8] D. Ecker, 2008, Nat. Rev. Microbiol., 6(7):553-558.
[9] Bush and Jacoby, 2010, Antimicrobial Agents and Chemotherapy ; 54 (3) : 969-976
[10] W.-J. Chen et al., 2008, Anal. Chem., 80 : 9612-9621
[11] D. Lopez-Ferrer et al., 2008, Anal. Chem., 80 :8930-8936
[12] D. Lopez-Ferrer et al., 2005, J. Proteome res., 4(5) : 1569-1574
[13] T. Fortin et al., 2009, Mol. Cell Proteomics, 8(5) : 1006-1015.
[14] H. Keshishian et al., 2007, Mol. Cell Proteomics, 2212-2229.
[15] J. Stal-Zeng et al., 2007, Mol. Cell Proteomics, 1809-1817.
[16] Gaskell, Electrospray: principles and practise, 1997, J. Mass Spectrom., 32, 677-688).
[17] V. Fusaro et al., 2009, Nature Biotech. 27, 190-198.
[18] J. Mead et al., 15 nov 2008, Mol. Cell Proteomics, E-pub.
[19] F. Desiere et al., 2006, Nucleic Acids Res., 34(database issue) : D655-8).
[20] L. Anderson & C. Hunter, 2006, Mol. Cell Proteomics, 573-588).
[21] B. Han & R. Higgs, 2008, Brief Funct Genomic Proteomic.,7(5):340-54).
[22] K.-Y. Wang et al., 2008, Anal Chem, 80(16) 6159-6167).
[23] J. Bundy & C. Fenselau, 1999, Anal. Chem. 71 : 1460-1463.
[24] K-C Ho et al., 2004, Anal. Chem. 76 : 7162-7268.
[25] Y.S. Lin et al., 2005, Anal. Chem., 77 : 1753-1760.
[26] S. Vaidyanathan et al., 2001, Anal. Chem., 73 :4134-4144.
[27] R. Everley et al., 2009, J. Microbiol. Methods, 77:152-158.
[28] P. Seng et al., 2009, Clin. Infect. Dis., 49 :543-551.
[29] Manes N. et al., 2007, Mol. & Cell. Proteomics, 6(4): 717-727.
[30] R. Nandakumar et al., 2009, Oral Microbiology Immunology, 24 :347-352).
[31] L. Hernychova et al., 2008, Anal. Chem., 80 :7097-7104.
[32] J.-M. Pratt et al., 2006, Nat. Protoc., 1:1029-1043.
[33] V. Brun et al., 2007, Mol. Cell Proteomics, 2139-2149.

## Revendications

1. Procédé de détection, par spectrométrie de masse de type MS/MS en mode MRM, pour au moins un microorganisme compris dans un échantillon, un marqueur de résistance dudit au moins un microorganisme, à au moins un antimicrobien, qui est une céphalosporine, le ou lesdits marqueur(s) de résistance étant une (des) protéine(s) ou un (des) peptide(s), compris parmi les protéines ou peptides de bêta-lactamases de type TEM.

2. Procédé de détection selon la revendication 1, dans lequel les protéines dudit microorganisme sont digérées en peptides.

3. Procédé de détection selon la revendication 2, dans lequel la digestion est réalisée par une enzyme.

4. Procédé de détection selon la revendication 3, dans lequel l'enzyme est la trypsine.

5. Procédé de détection selon l'une des revendications 1 à 4, dans lequel le(s) marqueur(s) de résistance sont des peptides de type TEM, choisis parmi les peptides de séquence SEQ ID N°166 à SEQ ID N°218, SEQ ID N°220 à SET ID N°230, SEQ ID N°232 à SEQ ID N°241, SEQ ID N°243 à SEQ ID N°252, SEQ ID N°254 à SEQ ID N°257, SEQ ID N°259 à SEQ ID NH261, SEQ ID N°1923, SEQ ID N°1927 et SEQ ID N°1928.

6. Procédé de détection selon la revendication 5 dans lequel le(s) marqueur(s) de résistance sont des marqueurs de type TEM, choisis parmi les peptides de séquence SEQ ID N°166, SEQ ID N°167, SEQ ID N°168, SET ID N°169, SEQ ID N°170, SEQ ID N°171, SEQ ID N°172, SEQ ID N°173, SEQ ID N°174, SEQ ID N°180,SEQ ID N°181, SEQ ID N°186, SEQ ID N°187, SEQ ID N°188, SEQ ID N°189, SEQ ID N°190, SEQ ID N°191, SEQ ID N°192, SEQ ID N°193, SEQ ID N°194, SEQ ID N°195, SEQ ID N°196, SEQ ID N°197, SEQ ID N°198, SEQ ID N°199, SEQ ID N°200, SEQ ID N°201, SEQ ID N°202, SEQ ID N°203, SEQ ID N°212, SEQ ID N°213, SEQ ID N°214, SEQ ID N°215, SEQ ID N°216, SEQ ID N°217, SEQ ID N°219, SEQ ID N°220, SEQ ID N°223, SEQ ID N°224, SEQ ID N°225, SEQ ID N°226, SEQ ID N°227, SEQ ID N°228, SEQ ID N°229, SEQ ID N°230, SEQ ID N°241, SEQ ID N°242, SEQ ID N°243, SEQ ID N°250, SEQ ID N°251, SEQ ID N°252, SEQ ID N°253, SEQ ID N°254, SEQ ID N°255, SEQ ID N°259, SEQ ID N°260, SEQ ID N°261, SEQ ID N°1927, SEQ ID N°1928.

7. Procédé de détection selon l'une des revendications 1 à 6-, dans lequel la résistance est de type béta-lactamase à spectre étendu (BLSE) et les peptides marqueurs de résistance sont, choisis parmi les séquences SEQ ID N°168, SEQ ID N°169, SEQ ID N°179, SEQ ID N°181, SEQ ID N°186, SEQ ID N°199, SEQ ID N°200, SEQ ID N°205, SEQ ID N°206, SEQ ID N°208, SEQ ID N°212, SEQ ID N°213, SEQ ID N°214, SEQ ID N°215, SEQ ID N°216, SEQ ID N°217, SEQ ID N°222, SEQ ID N°225, SEQ ID N°226, SEQ ID N°227, SEQ ID NH230, SEQ ID N°234, SEQ ID N°236, SEQ ID N°237, SEQ ID N°238, SEQ ID N°239, SEQ ID N°240, SEQ ID N°249, SEQ ID N°254, SEQ ID N°255, SEQ ID NH255, SEQ ID N°259, SEQ ID N°261, SEQ ID N°1927, SEQ ID N°1928,

## Patentansprüche

1. Verfahren zum Nachweisen mittels MS/MS-Typ-Massenspektrometrie im MRM-Modus für mindestens einen in einer Probe befindlichen Mikroorganismus, einen Marker für Resistenz des mindestens einen Mikroorganismus gegen mindestens eine antimikrobielle Substanz, bei der es sich um ein Cephalosporin handelt, wobei der oder die Resistenzmarker ein Protein bzw. Proteine oder ein Peptid bzw. Peptide aus der Gruppe der TEM-Typ-beta-Lactamase-Proteine oder -Peptide ist bzw. sind.

2. Nachweisverfahren nach Anspruch 1, wobei die Proteine des Mikroorganismus zu Peptiden abgebaut werden.

3. Nachweisverfahren nach Anspruch 2, wobei das Verdauen mit einem Enzym erfolgt.

4. Nachweisverfahren nach Anspruch 3, wobei es sich bei dem Enzym um Trypsin handelt.

5. Nachweisverfahren nach einem der Ansprüche 1 bis 4, wobei es sich bei dem Resistenzmarker bzw. den Resistenzmarkern um TEM-Typ-Peptide, ausgewählt aus den Peptiden mit der Sequenz SEQ ID Nr. 166 bis SEQ ID Nr. 218, SEQ ID Nr. 220 bis SEQ ID Nr. 230, SEQ ID Nr. 232 bis SEQ ID Nr. 241, SEQ ID Nr. 243 bis SEQ ID Nr. 252, SEQ ID Nr. 254 bis SEQ ID Nr. 257, SEQ ID Nr. 259 bis SEQ ID Nr. 261, SEQ ID Nr. 1923, SEQ ID Nr. 1927 und SEQ ID Nr. 1928 handelt.

6. Nachweisverfahren nach Anspruch 5, wobei es sich bei dem Resistenzmarker bzw. den Resistenzmarkern um TEM-Typ-Marker, ausgewählt aus den Peptiden mit der Sequenz SEQ ID Nr. 166, SEQ ID Nr. 167, SEQ ID Nr. 168, SEQ ID Nr. 169, SEQ ID Nr. 170, SEQ ID Nr. 171, SEQ ID Nr. 172, SEQ ID Nr. 173, SEQ ID Nr. 174, Nr. 180, SEQ ID Nr. 181, SEQ ID Nr. 186, SEQ ID Nr. 187, SEQ ID Nr. 188, SEQ ID Nr. 189, SEQ ID Nr. 190, SEQ ID Nr. 191, SEQ ID Nr. 192, SEQ ID Nr. 193, SEQ ID Nr. 194, SEQ ID Nr. 195, SEQ ID Nr. 196, SEQ ID Nr. 197, SEQ ID Nr. 198, SEQ ID Nr. 199, SEQ ID Nr. 200, SEQ ID Nr. 201, SEQ ID Nr. 202, SEQ ID Nr. 203, SEQ ID Nr. 212, SEQ ID Nr. 213, SEQ ID Nr. 214, SEQ ID Nr. 215, SEQ ID Nr. 216, SEQ ID Nr. 217, SEQ ID Nr. 219, SEQ ID Nr. 220, SEQ ID Nr. 223, SEQ ID Nr. 224, SEQ TD Nr. 225, SEQ ID Nr. 226, SEQ ID Nr. 227, SEQ ID Nr. 228, SEQ ID Nr. 229, SEQ ID Nr. 230, SEQ ID Nr. 241, SEQ ID Nr. 242, SEQ ID Nr. 243, SEQ ID Nr. 250, SEQ ID Nr. 251, SEQ ID Nr. 252, SEQ ID Nr. 253, SEQ ID Nr. 254, SEQ ID Nr. 255, SEQ ID Nr. 259, SEQ ID Nr. 260, SEQ ID Nr. 261, SEQ ID Nr. 1927, SEQ ID Nr. 1928 handelt.

7. Nachweisverfahren nach einem der Ansprüche 1 bis 6, wobei die Resistenz den Breitband-beta-Lactamase-Typ (BBBL-Typ) aufweist und die Resistenzmarkerpeptide aus den Sequenzen Nr. 168, SEQ ID Nr. 169, SEQ ID Nr. 179, SEQ ID Nr. 181, SEQ ID Nr. 186, SEQ ID Nr. 199, SEQ ID Nr. 200, SEQ ID Nr. 205, SEQ ID Nr. 206, SEQ ID Nr. 208, SEQ ID Nr. 212, SEQ ID Nr. 213, SEQ ID Nr. 214, SEQ ID Nr. 215, SEQ ID Nr. 216, SEQ ID Nr. 217, SEQ ID Nr. 222, SEQ ID Nr. 225, SEQ ID Nr. 226, SEQ ID Nr. 227, SEQ ID Nr. 230, SEQ ID Nr. 234, SEQ ID Nr. 236, SEQ ID Nr. 237, SEQ ID Nr. 238, SEQ ID Nr. 239, SEQ ID Nr. 240, SEQ ID Nr. 249, SEQ ID Nr. 254, SEQ ID Nr. 255, SEQ ID Nr. 256, SEQ ID Nr. 259, SEQ ID Nr. 261, SEQ ID Nr. 1927, SEQ ID Nr. 1928 ausgewählt sind.

## Claims

1. A method of detection, by mass spectrometry of MS/MS type in MRM mode, for at least one microorganism contained in a sample, of at least one marker of resistance to at least one antimicrobial, which is a cephalosporin, said marker(s) of resistance is (are) a (one of the) protein(s) or a (one of the) peptide(s), amongst beta-lactamase proteins or peptides of TEM type.

2. The detection method according to claim 1, wherein the proteins from said microorganism are digested into peptides.

3. The detection method according to claim 2, wherein the digestion is performed by an enzyme.

4. The detection method according to claim 3, wherein the enzyme is trypsin.

5. The detection method according to one of claims 1 to 4, wherein the resistance marker(s) is/are TEM peptides chosen from the peptides of sequence SEQ ID No. 166 to SEQ ID No. 218, SEQ ID No. 220 to SEQ ID No. 230, SEQ ID No. 232 to SEQ ID No. 241, SEQ ID No. 243 to SEQ ID No. 252, SEQ ID No. 254 to SEQ ID No. 257, SEQ ID No. 259 to SEQ ID No. 261, SEQ ID No. 1923, SEQ ID No. 1927 and SEQ ID No. 1928.

6. The detection method according to claim 5, wherein the resistance marker(s) is/are TEM markers chosen from the peptides of sequence SEQ ID N°166, SEQ ID N°167, SEQ ID N°168, SEQ ID N°169, SEQ ID N°170, SEQ ID N°171, SEQ ID N°172, SEQ ID N°173, SEQ ID N°174, SEQ ID N°180, SEQ ID N°181, SEQ ID N°186, SEQ ID N°187, SEQ ID N°188, SEQ ID N°189, SEQ ID N°190, SEQ ID N°191, SEQ ID N°192, SEQ ID N°193, SEQ ID N°194, SEQ ID N°195, SEQ ID N°196, SEQ ID N°197, SEQ ID N°198, SEQ ID N°199, SEQ ID N°200, SEQ ID N°201, SEQ ID N°202, SEQ ID N°203, SEQ ID N°212, SEQ ID N°213, SEQ ID N°214, SEQ ID N°215, SEQ ID N°216, SEQ ID N°217, SEQ ID N°219, SEQ ID N°220, SEQ ID N°223, SEQ ID N°224, SEQ ID N°225, SEQ ID N°226, SEQ ID N°227, SEQ ID N°228, SEQ ID N°229, SEQ ID N°230, SEQ ID N°241, SEQ ID N°242, SEQ ID N°243, SEQ ID N°250, SEQ ID N°251, SEQ ID N°252, SEQ ID N°253, SEQ ID N°254, SEQ ID N°255, SEQ ID N°259, SEQ ID N°260, SEQ ID N°261, SEQ ID N°1927, SEQ ID N°1928.

7. The detection method according to one of claims 1 to 6, wherein the resistance is of Extended Spectrum Beta-Lactamase (ESBL) type and the resistance-marking peptides are chosen from the sequences SEQ ID N°168, SEQ ID N°169, SEQ ID N°179, SEQ ID N°181, SEQ ID N°186, SEQ ID N°199, SEQ ID N°200, SEQ ID N°205, SEQ ID N°206, SEQ ID N°208, SEQ ID N°212, SEQ ID N°213, SEQ ID N°214, SEQ ID N°215, SEQ ID N°216, SEQ ID N°217, SEQ ID N°222, SEQ ID N°225, SEQ ID N°226, SEQ ID N°227, SEQ ID N°230, SEQ ID N°234, SEQ ID N°236, SEQ ID N°237, SEQ ID N°238, SEQ ID N°239, SEQ ID N°240, SEQ ID N°249, SEQ ID N°254, SEQ ID N°255, SEQ ID N°256, SEQ ID N°259, SEQ ID N°261, SEQ ID N°1927, SEQ ID N°1928.
